# EUROPEAN PATENT SPECIFICATION

(11) **EP 2 893 024 B1**
(45) Date of publication and mention of the grant of the patent: **04.12.2019**
(21) Application number: 13835630.8
(22) Date of filing: 09.09.2013
(51) Int. Cl.: C12N 15/82, C12Q 1/24, A01H 5/00

(54) **FLUORESCENCE ACTIVATED CELL SORTING (FACS) ENRICHMENT TO GENERATE PLANTS**
FACS-ANREICHERUNG ZUR ERZEUGUNG VON PFLANZEN
ENRICHISSEMENT PAR TRI CELLULAIRE PAR FLUORESCENCE (FACS) POUR GÉNÉRER DES PLANTES

(30) Priority: 07.09.2012 US 201261697890 P; 06.09.2013 US 201314020694
(43) Date of publication of application: 15.07.2015
(73) Proprietor: Dow AgroSciences LLC, Indianapolis, IN 46268 (US)
(72) Inventor: SPANGENBERG, German, Bundoora, Victoria 3083 (AU); SAHAB, Sareena, Mernda, Victoria 3754 (AU); MASON, John, Preston, Victoria 3072 (AU)
(74) Representative: Beck Greener LLP
(86) International application number: PCT/US2013/058766
(87) International publication number: WO 2014/039970

(56) References cited:
- EP-A1- 2 455 454
- WO-A2-2008/021207
- US-A1- 2003 219 763
- US-A1- 2010 257 638
- BASTIAAN O. R. BARGMANN ET AL: "Fluorescence Activated Cell Sorting of Plant Protoplasts", JOURNAL OF VISUALIZED EXPERIMENTS, vol. 36, 18 February 2010 (2010-02-18), pages 1-4, XP55241429, DOI: 10.3791/1673
- KAGEYAMA YASUSHI ET AL: "Plant regeneration from patchouli protoplasts encapsulated in alginate beads", PLANT CELL, TISSUE AND ORGAN CULTURE, SPRINGER, NL, vol. 41, no. 1, 1 January 1995 (1995-01-01), pages 65-70, XP009090822, ISSN: 0167-6857, DOI: 10.1007/BF00124088
- LARKIN P J ET AL: "Nurse culture of low numbers of Medicago and Nicotiana protoplasts using calcium alginate beads", PLANT SCIENCE, ELSEVIER IRELAND LTD, IE, vol. 58, no. 2, 1 January 1988 (1988-01-01), pages 203-210, XP025220322, ISSN: 0168-9452, DOI: 10.1016/0168-9452(88)90010-6 [retrieved on 1988-01-01]
- , 1 January 2000 (2000-01-01), XP55250039, Retrieved from the Internet: URL:http://www.sid.ir/en/VEWSSID/J_pdf/973 20000202.pdf [retrieved on 2016-02-15]
- David Roger ET AL: "lmmobilization of Flax Protoplasts in Agarose and Alginate Beads' Correlation between lonically Bound Cell-Wall Proteins and Morphogenetic Response", Plant Physiol, 1 January 1996 (1996-01-01), pages 1191-1199, XP55250031, Retrieved from the Internet: URL:http://www.plantphysiol.org/content/11 2/3/1191.full.pdf#page=1&view=FitH
- BRIAN K. HARPER ET AL: "Patterns of green fluorescent protein expression in transgenic plants", PLANT MOLECULAR BIOLOGY REPORTER., vol. 18, no. 2, 1 June 2000 (2000-06-01), pages 141-141, XP055250412, NL ISSN: 0735-9640, DOI: 10.1007/BF02824023
- BARGMANN, B. 0. R. E ET AL.: 'Fluorescence activated cell sorting of plant protoplasts' JOURNAL OF VISUALIZED EXPERIMENTS vol. 36, 18 February 2010, pages 1 - 4, XP055241429
- VIJAYALAXMI, G. ET AL.: 'Plant Regeneration from Protoplasts of Indica Rice cv tellahamsa' PROC. INDIAN NATN. SCI. ACAD. vol. 63, no. 6, 1997, pages 631 - 638, XP055241722

### SEQUENCE LISTING

<110> Spangenberg, German Sahab, Sareena Mason, John
<120> ENGINEERED TRANSGENE INTEGRATION PLATFORM (ETIP)
<130> 2971-P10992.1US (72292-US-NP)
<160> 480
<170> PatentIn version 3.5
<210> 1
   <211> 47493
   <212> DNA
   <213> Brassica napus
<400> 1
   ttcccaaaca acacaataag ttattgtcaa taactaatct tatgtccaaa gaatataaaa      60
   cattaggtcc agtcttttta ataacttttt atttatattt tttaaattat ttgatttaaa     120
   tccactcaaa taatttatac gaagttgaaa ataactataa ctatttttgc gggtttagat     180
   actacagaaa agcatttaac ttgaaattaa ttaaattaaa attttatttt gtttgtattt     240
   tcagaaataa aaaatcgaat tccttttaac ttaataaata ttagattgtt ctttgtaaat     300
   tatttttatt aattcataag aacatattat tatatatgct atattatgct tggtttaagt     360
   taaaattaaa tagttttata gaaataaaat gttagaatca ataaattaac atcaaataag     420
   ttttataatt aaaattttat caaataagca tatattaaaa atttaatcca acaaattaaa     480
   gatgttttct taaaacctcg taatgataca ggttgaagta attaaaaaaa aattaagtaa     540
   tattttagat aattttcctc tttaataaaa taatttatta tgtatgttta aatacactaa     600
   aataaaaatg ttaaaacatt acttatataa taatgttaaa atatattgat attataaatt     660
   agattttttt ttaaagatat tttcgcacat ttaatcgtgg gttatagtct agtatatttt     720
   aaaacctaac tattaaatgc tgaattttat gaagttataa cataaagtga ttaatgaaca     780
   aacgtcttat caattaatta tgcttccgca aagcatcatg ttcattgccc tataaaatca     840
   tctctcgttc actgatacca atcaaacatc aaccaatatc aaaaactaaa gcaaatatgg     900
   caaagtctct ttacattgca atgtttgtat ctatagtaat gttcttcatg gcaaattcaa     960
   tttcttccaa ggaaattggt caatattcac aagaggcacc aggagatgta aagatatctc    1020
   ccacatcgga ttttgatatt tacgtcgaat ctcccgatga atctctattt gaagaagtcg    1080
   attcacccgc aatggaatat gagatgaagt ctggacatca ttacacacac aaacaacttg    1140
   gttttcttga ggcttgcttt caaaatctaa actcattaga ctgtggagat aatattttca    1200
   agaacatgtt agatgaggca gcacaagtat tatcaaatga atgttgtcat gatctattaa    1260
   agattagcaa agattgttac ctaggaatga ctcaaagcat tttatcgagt tatgagtata    1320
   gatttattgc gtctaaggct attcccaaaa gtaaacagac atggaatgat tgtgttctta 1380
   gagttgggaa cctgattggt agcccaatcg cttttgagga actacactaa ttgatgttcc 1440
   ggtgtgctga tgttttatat atgtgatttt gtaactcagg cgttatgaga cttaccatta 1500
   tataaataaa gctaaagatc ttttacagat cttatattag tatacgagac tcattagagt 1560
   aacatgaaag ctgaatcaac tatcaacatt ggtggtattt gtctttaggt ttctttaatg 1620
   gatatagatg tttggggaca acgctagcag ctcttagtaa ttccgcatat ttatggacta 1680
   tgagctcttc gctctaaatt acacaatccg acccaccatt tcagatatca atactcgcaa 1740
   acccagtaga gttacagtct ctattgtact tctccttgca ttccttcagc ctgattctct 1800
   tatccacaat ggttatactt gtctctgaca acgatcatgt tcctcaagag caaaaaacca 1860
   tctaggtctc cgcagctcag tggcgagcct ctcacacaac cattgaacac atctaccaaa 1920
   gcaaatgacg tggcattctt gggaatgaaa cctttgatac agttacatgt cagtatcgtg 1980
   ctaatgttat agtaaacatt ttattatcat atttttaata ctaatgaggc atgatttgat 2040
   tttaatttgc atattttatc tcttaatcct ggattatata tagttttcag cagacaagaa 2100
   aatgttattt ccacattcta tgacttgttt ttagactttc atcaattcat tacaacatat 2160
   tattgttatt agtcgataac tttgacttaa ccacgtgaat tttatcccaa aaaaaactta 2220
   accacctaaa atctaatttt ttttataaag aaattagttt ttgatccgcg ttttgcagat 2280
   ttatttttca tttttatatg tttatatctt tgagaatgcc atgaaattga ccgtgtgttt 2340
   ctattgtgtt tagtttttgg tctttctaat ttttgagatt attttttctt taaacgatgc 2400
   ccgggttatt attaatttta tttgataaat taaacattga aatataatta gaattcgaag 2460
   tatactttta ggttttatca gttttaaaga tggtcatttg atttgaaatt tgtataaaaa 2520
   ttccaacttt tattttggaa tttttaaaag aaatatatgt ttactgatgt gtggtatatt 2580
   ttaataagat aaatatatta atataatatg gcatagtatt atgtagataa ttaaaattta 2640
   tatttacaat tatgtaatat aaaactatag atattattga aaactataat atttgttgta 2700
   atatgaatta gtttaaatta tgtacaacta tctgtgcaaa attacgtagt ttgtaaatgt 2760
   gtatatgtga taaaactata ataatacata ctatatttta actaatattg gtttatatat 2820
   ctaatatatt taattgtgta tttcatatta atcaaaaata taaaagattt aaattagaca 2880
   gaaaccgttc aaaaataaat atagtttttc atttgacccg caatttcaaa atgcgagatg 2940
   ttttataaat aattaacata atctttaatt ttggaaatga tatatgtttt tgaattattt 3000
   tacgtttttt atattaccat taacatttat tttaaatttt aaatgagaaa tgattttgta 3060
   attgtaacta aaatttatat aaatgtaact tatgaaaatt aaaaaaaatg taaccaggat 3120
   aattaccaaa aatagcattt tgaatatacc acttttcact tctactttat tcaactttgc 3180
   cattaaactt ttaattggca aatgactatt atatccctaa ttaattgaac ctaaactatt 3240
   caattagata ggttatatat tcttcctcca tgtgagatcc ggcgagctcc ggcaaaggac 3300
   gatgattgga gattattggg gatactgtga acaagaacga ggaacatatc tggaaatatg 3360
   taaatgggaa gagatttcta cacagattag attaatgaac atgatatcag attcattgac 3420
   atcttgtact gattccaatt ttgtctcgtg cttgttattc ctggctttta atgttttggt 3480
   gttgatgtat ctgatagagc aagtggaaag aggagctgga acaagcggaa agaccgagaa 3540
   aataaaagta accaataaac atggacacgt taaggaagaa actgactcca atgattcaga 3600
   gttttggatg cttaagtcaa gttttggttc agagtcagag taggagagtg atgaagaaaa 3660
   ctgcaaaggt acctcttttt ggtgtttttt gttggttcta ctttcatctt tctatcgaaa 3720
   cttaaactag atttgtgtct gatcttggta caggttctca ttgtgatgcc agataatatg 3780
   aggagctctc agaaaggtaa aggtaaatgt atacatttac tcttatttct gtgtatttct 3840
   tacaaaattg cggattggtt ttgatgttca cctctagttg ttttgtctct tctacagagc 3900
   aaagaggatg tcaatagaga ttggaccaag tatgatcatt acggcagcgc cactatgatc 3960
   atgaggcagt ctttggaaga gcagatgagt tctgttacgg agaagagtgc taagtatgct 4020
   cagattgctg ctgaggaagt gcccaagagt ctttactgcc ttggtgttcg tctcactact 4080
   gagtgatttc agaactcgag ttttagggga agctcgcgga gagaatctat gttgtggctt 4140
   ctaagctcac cgataacagt ctttaccatg tctatgtgtt ttctaataac attgttgcta 4200
   cttcggttgt ggttaactcc actgctctca aatccaaggc ccctgagaaa ttcgtccttc 4260
   atcttgtcac taatgcgatg aagtcatggt ttgctatgaa tatgaacttc ttaattgatt 4320
   ccggcttggt tgattcagta tgtggaaatt gcaggtgacg agtgtgcaat cttcgctgtt 4380
   tttagaagca gcatttgcgt caaatgatat gcagcatttg gctgcaatgt taagggtctt 4440
   gtcggaattg attccaggat tcaaagaaac taccgagtat tacacattct aaggtcttct 4500
   taatgagacc agttccccag ttgctgtctg agatttaaca tctcttacct tatttatgtt 4560
   caataactta gataataaga aatttcgttg ataatatttc aatttatgta tgtttcattc 4620
   aaagaaaaca atgattagtt ttctcaaatc agtcttctta tttgtttgag ttaagatctt 4680
   ccaaacttat gtttttttgt aattcaggag taacaaaatg aatatagtct aaatctggat 4740
   ctaattttga aattttttta actggatttt ctttcaaatt aaatccttat ttactcttta 4800
   agaatttttt taaaagttta ggtttgacat ctctaaccta tacgttgcga acttggacaa 4860
   aaccaaaaca agattaagca ttgttttcag aatatttaat ttttaatttt gttgggtttg 4920
   atttggatct ttccagtgta catatgttta aacaccatcc tcttcaaaac ggttgtttaa 4980
   gtgttcattt ttgggtaggt ccaagacgtg actgggacaa ttcaaagcgc tagttatctc 5040
   cgtaacacat catatttccc cttacattaa gataaagtat actcgtgaat ctcaattgga 5100
   tgcaataaat aaaaacaaaa tcccttccca tcaaggcatc aaactgccaa agtcaattct 5160
   agaaaaatat taaaaactat tcttttttta ttttatttta gagacttctc ttccttttta 5220
   ttctgtagga agaaaaaaca aaaaaagaag aagaagagga gaatcgtctc tttctttctc 5280
   ttagaccatc tacaatgcta cgctaaaatt tactctatat ttcactctaa aatagagtaa 5340
   ctctattata gagtgaaata tagagtaaat tttagtgttg cattggagat gcccttaact 5400
   gtatccgtga aggcaaaaag atacgtataa acaaatacat ttacacataa atatgtatat 5460
   gtatgtattt atactcttct agctctctct ctctcacctg tacctttaat atggtgtttc 5520
   tgtttacgag aaaccaaacc cacaccaatt gaaaacgcga cttgttgtta ttcaatcgca 5580
   atcaacgagt aatctgctac ctcgtctcct ctctgagatc tctcttcctc ccattagatt 5640
   cgcctgagga ggtactcatt cttattcctt ttttctctgt ctttttatgg aattttctaa 5700
   aaatgctgta aggtttcaga catatccggt aataactgga ctctgaacta atgacttgtt 5760
   caagtgttcc agctcttatt acatagtacc gatttggatt gtgttttttt ttttgtttga 5820
   ccttcttgca attgtgatgc tctgatttgt tttcacgttc gtagcttatg ctttaatttt 5880
   tttttgtcat ttatcttctt ttccccctaa tttttttttg tttaattgca aacctttttg 5940
   tgtgtgcttc ggcatgaact taggggccct actactggcg taagtttagg cttctttttt 6000
   ggtgcttttt tagctttact gcttgttggt ggaattcact tttcttttta agttaatttc 6060
   agagacatcc ttattgtaac tgctcacttt tcagtgttgt tagtttaagg aatgatgaaa 6120
   gaaagaaagc tttctataac tgcttgattt agcttcagtt gttagtaata tgggttagaa 6180
   ctgttagcct tgagtagata tcactgtctt tattttttgg tttattgttg atttattttt 6240
   gtggtcttgt tttgattcag tgttctcgag gttttgactt cgacttgtga tgatggcatc 6300
   atctaaacaa ggatcaaagt cgagaaaaac agggtttagc aatttcaagg gtgctgattc 6360
   tactgcttcc tcaacaacct cttcttcaaa gctttatcaa gagacatcta tcgatgatgg 6420
   acatagctct cctgcttctt catctgctca aagcaagcag cacttcttct caccggactc 6480
   cgcaccgcaa agtgctcagc gttctaaaga gaacgtcaca gtgacagttc gctttcgtcc 6540
   actcaggttc acagtgatgt gcttttttat tagggcttgc aaatattagg ttgcctgttt 6600
   gttctgtgaa tttgaaataa cttttccgtg acagtccaag ggaaatccgc caaggggagg 6660
   aggttgcttg gtatgcagat ggcgaaacaa tcgtacggaa tgagtataat ccgacgatag 6720
   cttatgcata tggttagtct taatagtttc attaatgtca cagtggttcc ataatcatct 6780
   taaatattgt atgtcggcat atcactcgag aataacaaac taaggatgat agtgttgttc 6840
   ttatgtggta taatcttcct tttttgatgt ctgggtctct atgtctgtta acgattggtt 6900
   ctctttatga cagatcgtgt ctttggacct acaaccacaa cacgcaatgt ctacgatgtt 6960
   gctgcacacc atgttgttaa tggggctatg gaggggatta acggtatgaa gtgaccatat 7020
   ttaatggcag tctattttgt atttagaact caatttatga tatcaactct tttatcgaaa 7080
   agcatacttt cttccaaact ctatcaacaa ggctgactgt tcctaattta tgatgaattc 7140
   gtcagaacca attttgactt tcgatgtact taccagttga tttgtatata actttgaatg 7200
   ttaatccatc tacagggacc atttttgcat atggagtgac aagcagtgga aagactcaca 7260
   ctatgcatgt aagatacctt catctggata gcttttggct tgttttgtac gatgtagttc 7320
   aatttgtcat aaagatatag tgctttgtca gagaatcatg tatttctttt aatttcaact 7380
   ccacaagctt aactaacaca tgtaagaatc tttctgcaat agttatagat aaacaacctt 7440
   gcgatagata agttttctac cctaatactg ttaggtttct gtgcacccta ctgttgaaac 7500
   catatctgat agatttcgat tgggtttttt tttgggttca aatgtcaggg tgaccagaga 7560
   tctcctggta ttataccatt agcagtgaaa gatgctttca gcattatcca agaggtactt 7620
   cttaggatat tgattttgtc agattccgtg gcctaagtac tagcaacctc acatatctat 7680
   tttcttacag acaccaagcc gagaatttct cctgcgtatt tcctacatgg aaatttataa 7740
   tgaggtgcta ttatttatcc actttctttc tctgctccat tacctatatt cagcttcaag 7800
   agtagtggag tgatagaata ctctgccttt aaaatcttcc tttgagatta attcccttaa 7860
   gatccaccgt atttcaggtt gtcaatgatt tattgaatcc agcaggacat aatttgagga 7920
   tcagagaaga taaacaggta tgtgtttcag cttcagatca tcttttgatt acacgggctg 7980
   ctgctctcac ttacttgtaa tattttcttt tattacattg ctcgtctaaa atcccaaaat 8040
   gtttacatca tattcttaaa ttagtaggaa cccatgtcta ggcttgtttg gtacctgaaa 8100
   actatactgg ttccattttt ttcttgtgaa atttgatctc tctgagaact ctgctgttac 8160
   aatgatgata tgtgggtcac attgctttca agcagacttg tagatgatcg ctatgagttc 8220
   ttttcttcca tgtgttttaa ttgaacgcat ccttttaatt tattctaggg aacctttgtc 8280
   gaagggatta aagaagaagt tgttttatca cctgctcatg cgctttctct tatagcagct 8340
   ggagaaggta tgtggtgctt ccacttttcc ctttagctaa agaaataggt ttactataca 8400
   tcctcataac gccagcaaag actgttgcag tcattgtaca tttgttaaag atattgtcat 8460
   caggaaagtg gttctcctct acctgcttgg ggctgtacaa aacgttaaaa attgtgtttg 8520
   ttttttcttt tatttaatat gagagagtag ttctctctct ctcatgattc cagtatacat 8580
   atatcatcct taaaatttgt catgatctag taaatttaca tttaattcaa ttcacattga 8640
   ttggagtttt gtccttatga cagagcaacg ccatgttgga tccacgaact ataatctgct 8700
   cagcagccgg agccatacaa tatttacgtt ggtatgtaaa tactctcttt gaaatattgg 8760
   tagctatatc ctttttcttt ctccgcacgt atatacactt gtattgtgga tggatgcacg 8820
   caaaaccaac gttcttttct attactataa ttggttaaaa ggtaaaaatg atagtttttt 8880
   gtcttataat cacgatagta tttgaaaata gaataccgaa attctatttc ttattcagaa 8940
   taagttgtgg accacgttct tcttttcacg tgttttcttc cgttcaagga atttctgttt 9000
   gtcttttatt ctaattctag ttgtttagtc gtgtcaagtg ttccaccggt tatggtacta 9060
   ctttcttttc attgtaactc atatagctgt accgtttcta aaagatgata aactcatttg 9120
   gatgcagaca atagagagta gtcccttagg caacaagatt aaaggtgaag ctgttcacct 9180
   ctctcagctg gtaagcttct cctgtgccag tgatattatg tatggttatg atcaacactt 9240
   tcttcaacac taagagcaac tgacattaac tacccataat ctcttccgca cccgcagaac 9300
   ctcgttgatc tggcaggttc cgagagttca aaggttgaaa ccagtggttt aagacgcaag 9360
   gaaggatcat acataaataa aagtttgctg acattaggca ctgtgagttt tttcttacca 9420
   cagttttctc ttagtcaaca ataagttttg gagtcagtta aatcgaagga cttcattttc 9480
   ttttaaatcc ggtcaatctg tgcttgtcca aacctaaagt taaatagttg tctattcttg 9540
   tttatattct ggtcctgttt gaacattctc ctgttggctc aattcaggta attctgttgt 9600
   gatattgtag gtgatatcaa agctcacgga tgtgaaggct tcgcatgtac catacagaga 9660
   ctctaagtta accaggatcc ttcagtcctc attgagtggc catgaccgag tatctgtaag 9720
   ttattagatc ttttctccat cctcttgcta ctataaatat aaaatccaca acatgatcat 9780
   cagcagagtg atgtacttta ctgattagtt gtcccttctg gatatggtag ctcatttgta 9840
   cagtgactcc tgcatcaagc agctcggaag aaacacacaa cacattgaaa tttgctcatc 9900
   gtgcaaaaca tattgagatt caagccgaac aaaacaaggt ttgcctcata tcttcttcta 9960
   tcagacctta ttttattgta ctaaagttct tgcttggttt ctaaatctat attactgcag 10020
   atacttgatg agaaatcatt aatcaagaag taccaacacg agattcggca gctgaaggag 10080
   gagttggaac agattaaaca ggacattgta ccaattcctc agctgaatga tattggcaca 10140
   gatgatatcg ttctcctgaa acagaaggta tgagttatat gctcgattag acaagtggaa 10200
   ggatgttttg tgtctttaca ttgagagaat ctgtagtaac cttgtctttt tttttgtttc 10260
   tggctatata acgtgatgat attagctaga agatggtcaa gtgaaactgc aatccagact 10320
   cgaagaagag gaagaagcta aagcagctct attgagtcga atccaacggt tgacgaaatt 10380
   aattttggtg tcgactaaaa cttcacaaac atctcgatta cctcatcgct ttgagcctcg 10440
   gaggagacat tcatttgggg aagaagaggt agaatcactg attatagcct tgatattata 10500
   aattgtttcc atggtcgttt ctgagaatat tttgctggag caagtacaca aaattgctag 10560
   ttttgtttgg ctaatcttgg ttgataatat gcgtgtgcag cttgcttacc taccatacaa 10620
   gaggcgggac atgatggacg atgagcacct tgatctgtat gtctctgcgg agggaaataa 10680
   tgagattaga gatattgcgt ttagagaaga aaagaagacc aggaagcatg gattgttaaa 10740
   ctggttaaag cctaaggtat gcaaacgatt gaattttctt attagcactg tggtttattc 10800
   tggttcgttt cagaactgca cctagcaact ataggtttgt actattgcgc actatagatt 10860
   tatagagttc tcatgttctt caaagaacaa tgatataagt aaagagtgct aatcatatga 10920
   ccttgtgtgc ttatttgatt atttggtgcc tctcttgtcc ccttttttgt aacaatctta 10980
   tttttctcct atcagaaaag agataacagt tcaagtgcca gcgaccagtc gagtgtggta 11040
   aaatccaaca gcacaccatc gactcctcaa ggaggaggaa ataatctgca tgcagagtca 11100
   agattttcag aaggatcgcc tttgatggaa caattctcag agcctaagga agacagagaa 11160
   gctctagagg acacttccca tgaaatggag acgccagagg tacgaagaga cattttcaca 11220
   tatgttatgg ttccaaagta actacatctt tatcttttcc acttcctgcc ctgttcccag 11280
   actagcaata aagtgatcga tgagttggat cttctgaggg aacagaaaaa gattttatct 11340
   gaggaggcgg cgttgcaatc aagttcatta aaacggctat tagatgaagc tgcaaagtct 11400
   cctgaaaatg aagagattaa agtaatataa ccacttggtt catgatttgt atctagcttc 11460
   cgtttaaatc taccaaacat tcactttcct ggttatctgt gttacttagg aggagatcaa 11520
   agtcctcaat gatgacatca aggctaagaa tgacgagatt gcaacgttgg agaaacaaat 11580
   cttggacttt gttatcacat cacatgaggc gttggacaaa tccgacatcg tgcaggtaag 11640
   ttcattgtta agtattatgc agtgactttt tttttctgtg gttcatttct caagctattt 11700
   ctctataaat atcttcaggc acttgctgag ctgagagatc aagttaatga gaagtctttt 11760
   gaactcgagg taaagaattg tttctgctac agcacctaga gatgtcttgc tactgcgtgg 11820
   atgataatta tttctcttag ctccagtata atatctactc atgcctgcat aaagttgctt 11880
   ctctgcaaat aatgatgaaa gctaattgca tctataacgt ttttttttct ttctcattct 11940
   tgatcttgtt tacaggttaa agctgcagat aataacatca ttcaggaaca actcaatcaa 12000
   aaggttgaga gattttttct tttatcgatt tgtcttaagt ttataatctg taaataattt 12060
   aataaccaac gaagtatttt gctgcagaca tgtgaatgtg aagcgtttca agaagaagtt 12120
   gcaaacctaa agcaggaact ctctaatgcc ctggaactag cacaggttct caaacttttt 12180
   atatgaaaaa agcaacacct aatagctaaa tgattctcaa actaaagtca cttgcctttc 12240
   tgacatgaca ggaaaccaag atcgaagagc tgaaacagaa agctaaggag ctaagtgaat 12300
   cgaaggagca attagaacat cgtaacagga aactcgcaga agagagttca tatgcaaaag 12360
   gtcttgcatc agcagctgca gttgagctca aggcattatc tgaagaagtc gcaaaactca 12420
   tgaatcacaa cgaacgacta gcatctgagc tagcaacact caagagctca gtcccacagc 12480
   acggtaataa gccaggaaca acaacaacaa ccaatgcaag gaacaatggg agaagagaga 12540
   gtcttgcaaa gagacaacaa gagcaagaga gctcgtcgat cgagctgaag agagaactga 12600
   ggatgagcaa agagcgggaa cgatcatacg aagctgcact tgttgataga gaccaaagag 12660
   aagccgagct tgtgaggata gtagaagaat cgaagcagag agaagcgtat ttggagaacg 12720
   agcttgctag tatgtgggtt cttgtttcta agctgagaag gtctcaagaa ggtggttctg 12780
   agatctctga ttctgtatcg gagacgctac agaccgatcg atcgttttga gacgtgaagt 12840
   agtaggatat gtttgtgcag tgattccaag agtttgtgtt tgtgtaagta tgataaaaca 12900
   taaagtaatg atttatttga aaatcatcag attgtttaaa attcaagaaa aaaaaacact 12960
   gtttaaatta agaggtaaga gaagtgaaaa gtgggtaaaa gaagaaaata atgatagttc 13020
   tggggagctt agagcatgat gattatcccg gtctcttata actcatgatt ttaatatatt 13080
   ttttttacac tttttggtta aaaaacatct cttatatatt ttatttaaaa aatgttctta 13140
   gttttttaat taaaaactaa gaaacgatta gctaaaaaac tcagttaaga aaccagggtt 13200
   aatcatggtc ttagtaaagt tcttaaaatt ttcactttca cccaatacct ttccactaat 13260
   ccacatttgc cccaattgat ttccttcttt gtgtatacaa cccaaattaa taattgttaa 13320
   caaaacagta aaacactata caaacaccaa gaagtgtact caatcagatg tctgaaaaca 13380
   agaagtgcaa ctgaagagtc ttggattgaa gttagagagg cttaagagtg ttcttaggag 13440
   cacttatcca cggagtgtct ccaatggcaa atctccacaa ggcattgaca tgttgaggca 13500
   atgcgtaatc tattccaacg cgaggaccaa ctagtacttt ctccacatct tctcctccat 13560
   ccagaacctc caatcctcct gtttttcata catcatttat tacaacaaag cttaaactct 13620
   cttaaagaat cagaaaaaac cagaaactcc attaactgat tttggtttat ttgtctgact 13680
   aacccggaga atagagggga tgatgagacc actctgttga aagtccaagc gcctgcccga 13740
   cctgcaagag gcacatatga acagatttca tgcattgtgg atttgcaagg tgcattctgt 13800
   ttgatgcaga tcagactaac ctttcctggt ccatttagaa gaacaggttt gtcggttttc 13860
   tggccacgac gctcctgtat ggtctccagc cctgcaatac catcacaccc ttttaagcca 13920
   acgtaagatc tatgtaatca ttcttaacgc aaaaacagta tcaataaagc aattatttaa 13980
   gtagaaacat gcatcgataa gagagagtgt aatgaacagt ctcacagaac tgatgtggag 14040
   agattattac cggtaacagg agaacaagat cgtatcaaaa cagcagctcc aactccatcc 14100
   ttatcagcaa caatattgag catcatatga agaccgtaac aaagataaac atatgcatgt 14160
   cctcctggtc caaactaata aacaaaaaaa acacacattt tagagaatgt tatcatgtgt 14220
   atttaactaa atgataacaa agcagaatga agaaactcac aataggtgcg gtccgtgggg 14280
   tattcccgaa ccgtccatgg caagctgagt catttggtct ataagcttcc acctgccaaa 14340
   ggaggaacaa tgttagagag tattaacttc gttaatccaa aatcaaaaat tttttaccaa 14400
   caattatagt cagaatatca aaaatcccaa gaaaacaaca aaaaaaacta aattctttta 14460
   atgaggaaac aagaacaaaa ctttgtatct gacctctgtg atccgtagga caacattgtc 14520
   tctcctcagg aacttcccga gcaaacgtgg cgctagatca agcgcgtcta tttgaaagaa 14580
   ctcaggaggc attatcttca tctcgggggt ggagcgggtc agtgggtact cgggtctgac 14640
   ccgaaccgcc cgagccttgg aacagtgctt cttctctcgt gctgcacgta gagccactgg 14700
   agttgtaacc ttgggtagtt ccgattcctg atcaactcgt ttggaacgac gaggcggcgt 14760
   tttcattgac ggacagaacc aaaagatttt tgactttttc gaatgtgttc gactctttga 14820
   cttttcttcg ttttgttatt tggcgggtga ctgttacgct gtccgaaggt taagccttaa 14880
   acataaactt tccttattat gaccgtcata tcaatttttt cctccgtttt tcaatatttg 14940
   gcgttttaaa aaaaaatttg agaaaaattg attttttttt gtttatccga tttaaattat 15000
   atggttacta ataatccggt taaaacttaa aagtaaaact atagtttata gataattaag 15060
   tttgtaattc tcaatttgaa gagaaataaa ataatttatg gagagctagc ctgaaattat 15120
   aatcggtgga gatattttga ctttatatga tagtggggaa ataaaacaca aaataaataa 15180
   tgttttcaaa taaacattta ttaattttat attaattcct attctgtaat tttgaattat 15240
   ttttgaaatt attaatgaat aaatgattaa gaatattttt ttaaaagaaa aattagatat 15300
   accactctaa aataatactt cctccatttc agaataaatg atgttttata aagtttttgt 15360
   tgtttcataa tagattgatg atgttttgat atatttatgt tatttttaat tttattgaaa 15420
   aattgtgtaa ctaattagat attagagtaa tttatgtagt tggttgaatg atttttaaat 15480
   tatattctta aaactaactt ttagttataa aagtaaaatt tttaaaacat catgtgaaat 15540
   agatggagta ggatttaaca taatttgtac gttctagaca cttccaaagc gtttcaaaag 15600
   ttaattagtt tttctttttc tggcataagt attattttat atttttccat ttttcagtag 15660
   gaaaaaaata aataaaaaaa aaacattttc tagaggcttc gtttacatat atctaggttt 15720
   acctcctctg cttcttccca cacatctcat cgaattcgac tagctcccca ctcaatcact 15780
   cctcgttagt tcaatctcga atccctaatc cactcaccat ggcgaagttc ggcgaaggcg 15840
   acaagcgatg gatcgtcgaa gaccgccccg acggcaccaa cgtccacaac tggcactggg 15900
   ccgaaaccaa ctgcctcgag tggtcccgca gcttcttcac caaccaattc tccaacgccg 15960
   tcatcctctc cggcgaaggc aacctcttca tcaaaatcaa gaaactggag aagctcgaag 16020
   gcgaggcgta cgtgaacgtg cgcaagggga agatcatccc cggctacgag ctcagcgtct 16080
   ctctctcctg ggaaggcgag gcgaaggatt cggaagggaa gacgatctcg aaggcggagg 16140
   gggccgtgga tatgccgtat atctccgatg agaatgcgga tgaggatccg gaggttaggg 16200
   tttcggttaa ggacgagggg gcggttggga aggcgttgaa ggaggcgatg gtgaagaagg 16260
   ggaagggggt tgttttggag aaggttaggg tttttgtgga ggctatggcg aaaggagggc 16320
   cttgtaggga tgaattggag aataagaagg tggctcctaa gtgggtggcg gcggcggcag 16380
   cggctgtgga gaagactagt gttttgcctg ctgtggtggt gaaggagaag aagaaggtga 16440
   agacgaagga ggggttcaag acgattagta tgactgagaa gttcagttgt agagctaagg 16500
   acttgtatga gatcttgatg gatgagaata ggtggaaggg attcacgcag agcaatgcta 16560
   agattagtaa agatgtgaat gggcctatta gtgtttttga tgggtcggtt actggggtga 16620
   atgtggagct ggaggaaggg aagttgattg tgcagaagtg gaggtttggg agttggtctg 16680
   atggtcttga ttctacggtt agtttagttg ttatttttct ttgtgacctt tggtttctat 16740
   gtcattgtgg ctgatgtatt gtgctttgac attttcaggt gaagataact tttgaggaac 16800
   ctgaaccagg agtcaccatt gtcaatctta ctcacaccga cgtccctgaa gaagacaggt 16860
   tagtgtcact gcttgaaatc tttcttataa tagtatggta gtatgcaatg aatatttatg 16920
   gtgtttggag tgcttgatag cttggtctta tatataactt tcatattgat gtactatctt 16980
   gagggaattg taaccacctt tagcctgttt tgttctcaat gacagacact aattctgacc 17040
   ttagtatctg tgctaatcct tgcgactgtt tagcttgctg tatgtagttg ttggactcat 17100
   ccagtctttg ttctagtgaa tgaactcttt tggaatttgg gttttgcttt atttgttaat 17160
   atatgataga acatttctat ctctaaatcg ctcaatggcc tacacgtctt taaatcttct 17220
   acctatgcta cctttccact aactagcttt aggttatgct gatagagcgc ttctttgttc 17280
   cttgtgaaca cctgttggct tattcctgaa agtatttgtt tgtccacatt ctcttactcg 17340
   tcctgttgtg aagtgatttt gtccgttact ggatgtgact tatttttctc tgacgtttct 17400
   actggctgat gcaggtatgg gaatgcgact gtggtggaaa acacggagag aggatggaga 17460
   gacctgatct tccataggat ccgtgctgtt ttcgggtttg gaatgtgatt tgtgattttg 17520
   attatcaagt aatcaaaatc agcattgctt catccataag ttcgattatg atttcaattc 17580
   acaaggagac ataaagacga tgcagaaatt tggtttttac ttgtagttta tgcattttcc 17640
   atgaactctt tggtcttttg ttaacactat ttggattaat ggcacacaac aaactattca 17700
   catgagaata agttgtttta agtgttttat tctaaaagtg gtgtccatag tcaaccacgg 17760
   catcaagatt gcctaaggaa cattgtctaa agactgatga aaactaggaa caagaagctt 17820
   tggtttggaa ctataagctt tttgctaatc gcaatgccta accaacaagt tacacatcaa 17880
   ccggtaatta accagttacc agaacctgct ttgaccaaaa tattacacat cacagcaagc 17940
   tctttggatc gtttatctaa aagggttttg gtttagcctg gaaccacgtg gatgaaatat 18000
   ttattaacca caacatcaac agaaacgttt ttactagctt tgtcaggtat agacgtgggc 18060
   acaaacaaga cctacttgaa atcagcacct acaaaataaa accaaaaaac atcttactct 18120
   taatgtatgg cggtctttaa gacaacaaaa atttcttcta ccacttttaa tcatcaagaa 18180
   gagacttgaa atgttacaga ttaaggtatc aagctcagac tcttaaaatg tcatattgtc 18240
   agtgcatatg atttgaacta gattgtaata tttaacatta agaatttagt tatggtatac 18300
   agagtataac ggtttgtgga tctgtcattg tgttatcatt aagctttgta aagatagtca 18360
   ttgttttggt tttgttaaaa tgatgttagg tagaaaatag taataatttc aaatcagcat 18420
   ttagtataat tatgaaactt cacaaaatcg tgtggaaaac tattaaagac gagttttaaa 18480
   tatggttgac cggatagtta attttcttac atcagatcca agattagtat tccgaagatt 18540
   gttgcggtta ctggtatcaa tttcctggaa gatttaaaat atcatcatgt cacgtacttc 18600
   attactagta accttcgttg tttaaagcgg aaatgtcgca actcgtttgc ttcattttgt 18660
   tttctttttg gatccttcaa atgatccgca acacttccgt tggagttgat gccatagaga 18720
   gtgcgtcgag ttatttaacg tataataaat attcttgcgt ccttgtacac atcatacgtt 18780
   aattaagcca tcaagatgta gcattactgg cgttgtttca gtaaaaaaaa ttattggtaa 18840
   aatattaaat tttaattacg cagaaacaac aaagaagaca ggaaaaaaaa ctacaaccag 18900
   agactatgtt acaacgaatt caaagagaaa aagaactgag aagaaaagca cacatcttcg 18960
   tcattcaaaa attaaaccgt ttattgacaa agaaattaat cgtgactaat aagctccttt 19020
   gtcggcccca tgtaacccat cttttacgtg taatctgatg cttgcttcca cggttccacc 19080
   ccgacttaac tgccacatat acactttggt cattcaaact taccaaacac aaagcgaatt 19140
   cgtttttgtt tcatgctttt aagaatcaca atctcaacat tccaaatata tataaaacac 19200
   aatctcaaca ttccaagcat ggggtggcat tctcgtagtt atctccatga caaggggcac 19260
   tttacaatag aaaacactcg ggatactttt ttatcgacga ttcctaagca aaatattacc 19320
   gaacagacac ctctctgtct gtctctttta tatgtcttct caaaaacgaa aaagtctctt 19380
   ctccgtcaac atttcacttt ccctctttcc ctcttgttaa tctctctctc tctctctaca 19440
   ctcaaagaaa acacagagac tcttcacgcg ccaaaaaaaa aaactcacca ccttcctctc 19500
   tcccttacca tgacttcaga ctctgtcaag catacttcta tccacggcgg aacaaccatc 19560
   tccgccgcat ccttcgaatt aaaaagcttt atctccgccg cgaaaccaag aaaagcctcg 19620
   acttttgtat acgccttcgt cataagcttc gttgccttca ctgttctctt agtcttcaca 19680
   ccttctccca tcaccgtctc tcattccatt ccttcataca tcctccctaa tgtcactgcc 19740
   tccttgactt caccgtccag tttcaccgga aacaccccat tgccggaaaa tctcactccg 19800
   gcgccggaaa atctcgctcc ggctactaaa aacgcaacct ttgagtctcc catcgctaat 19860
   ggagcaaatt cacttgcttc tcagccccgg accgaccatg cattggacaa catgttgtct 19920
   ccggacaaca agactaatga tactgctcca agttccgaca aacttggatc cgcggaagca 19980
   cctctgtccg aaaatctaac cgtcaattcc tctgctttaa agaagagaaa acagaggagg 20040
   aagtcgtgga tgagacgaga gataaagtct ttaaagaact gcgagtttta cgagggagag 20100
   tgggtgaaag acgattcgta tccgctttac aaacccggtt cgtgtaatct catcgatgaa 20160
   cagtttactt gtatctccaa cgggagacct gacgctgagt ttcagaaact caagtggaag 20220
   ccaaagcaat gcactttacc acggtaaagg ataaaacttt gctttttaat tttgacttta 20280
   aaatctattt ctttgtctta tatggttggt tgatgttttt ttttttttga aaaattataa 20340
   aaggttgaat ggaggcaaat tgctggagat gattagagga agaagacttg cgtttgttgg 20400
   agattcactg aacaggaaca tgtgggagtc tttggtttgt attctcaaag gatcagtgaa 20460
   agatgagagt caagtctttg aagctcatgg acggcatcag ttccgttggg aggctgagta 20520
   ctctttcgtc ttcaaagtag gtttctttta gtaatctaaa tcagtttcat tagttgttgt 20580
   ctctcgggac ttgatttgta tgtggttgat aggattataa ctgcactgtg gagttctttg 20640
   catcaccttt cttggttcaa gaatgggaac ttacggacaa gaacgggact aagaaggaga 20700
   ctttgaggtt agatgtggtc gggaagtcgt ctgagcagta caagggagct gatattcttg 20760
   tgttcaatac aggacattgg tggactcatg acaaaacttc caaagggtaa tagagttctg 20820
   tcagctactt tgatccttga tttgagggat ctgtctcatt tgttatgttt ggatgctttc 20880
   attagggagg attactatca agaaggaagc aatgtacacc cgaaactcga cgtggatgaa 20940
   gcttttaaga aagcattaac aacttggggt cgatgggttg ataagaatgt gaatccaaag 21000
   aagtctcttg tcttcttccg tggatactca ccttcacatt tcaggtatat acagatctca 21060
   ttttgtttca taatataggt agttgtaggc ttgtagctaa aagcacaaag attaagaacg 21120
   ttattatttt ttttaaagta tcatctaatt ttatagttta gatatatctt aaaccaatca 21180
   taaactagtc tatataattt gactggtcac actatatcca ataattcaat aaatataaaa 21240
   gttaagtaga aatgtaaaaa ctacataatc ttgaaaagaa aaaaaatatt cactaaaact 21300
   acttataata tgtgaaatag agggagtata aaaagagtgt ttctaaaagt tggtttggtt 21360
   tggttgcagt ggagggcaat ggaatgcagg aggggcatgt gatgatgaaa cagaaccgat 21420
   caagaacgag acttacctaa cgccttaccc ttctaaaatg ttaatacttg aaacagttct 21480
   aaagggaatg aaaacgccgg tcacgtatct caacatcacg aggctaacag attacaggaa 21540
   ggacgctcac ccatctgttt ataggaaaca taaactatct gcaaaagaaa ggaaatcacc 21600
   attgttgtac caagactgta gtcactggtg cctcccaggt gtgcctgatt cttggaacga 21660
   gattctctat gctgagatgc ttgtaaagct ccaccagctt cgtggcaata gaaggcggaa 21720
   acctaaaagt ttataggagt tagaatcctt ttcttaagat gatgaataca gatcttttag 21780
   gaaacactta gaatcaattt tcacttttca gatttgtgcc actgaaggtg tagagaaagt 21840
   aagagatggg agtcacatta gtgtttcatg atgtacgtac gtaagagatg agttaatcac 21900
   cttatgttgc tgcatttgta gtcaactaca actagtgact catttttttg tgaaaataaa 21960
   agattgagcc aacatttgta taaattccaa tgcatacccc tttacttata ataatgatca 22020
   atcgattggt caaacgaata catttagaat taagaaccct gcaacgactt tggatttaaa 22080
   ttgacacaga tcttttgctt caaagtaaca gtttgagcta agaacataaa gaatgcacca    22140
   gtcctttaat tacagtatta tgataaaact cactagagcc atctagatac aatctgaatg    22200
   ataatgtcac aggtggaaag aaagcatgaa catactcagt cgtccttcat gagactcctc    22260
   cggtcctctt ggtgtgcaac caaaaccacc accatccacg tctttcgtat ctcacaatgt    22320
   tatcagcagc cttatccaag tcctcctcct tcgcgtcagt tccctctctc tttctttctt    22380
   tctttctttc tttctttctt tcttgagatt gcaagtttga aattaagctc ttatagattg    22440
   tttattttga tgttgttatg tcggtctctg tgtaaatgtt tagttatttt tttcgattct    22500
   ttctcagaaa agtgtctcga ccttaagtat tttcttgtta gagacttaga gctgctactc    22560
   tttgattctc gtaagattta gtttcctcag ttattgcttt aagcaagcga gagactactc    22620
   tcttgactgt tttggccttc tgatttgttg aatccatggc aatatttgtg gtttttgtct    22680
   cctgctctct ctctctctct ctatgtggat gggctttgtt tcatgttact attattagct    22740
   ccaccttcga gattattatt cttttttttt ttttttttga atgaatgtta aattttattc    22800
   aatccaaaaa aacccttgtt acatttacaa tgtttcctac tccaattgtt ttataacatt    22860
   atcaccccta tttgaattta tctactcaag aaactggtct tgtggagaac cagtgttgta    22920
   aaccctcctg ccatctatca tcccctgctc tttgaataga cagaaatcta ttcctcactc    22980
   ctctatcaat cagccttatc atcgtagtag tcggctgggg agcttcacca tgccttctac    23040
   cattcctctc caaccacagc ccatgtatag tagcttggaa cacatacttc aatgtgaatg    23100
   tcttgatagt attctggttt gtagatatca gagctactat ttctgaccag tcacaagtaa    23160
   actcatttcc catcaagcct ctcaccaagt ttccccaaac ttctgccgaa tatccacaat    23220
   caaagaataa atggtttctg gtttccaatg gatcaccgca gagagagcat gctgtattta    23280
   cacttcctcc ccatccttgc attctctctc ccgttgataa tctgttcttg attgtaaccc    23340
   acgcaagaaa agcaaactta ggtgtcgcaa aagtgaacca gattccttta ctccactcac    23400
   agcttactcc tctagttcgc aattgactcc atgtttgctt agtagaaaac ttccgcttga    23460
   atttgtcttc cttatacctc cataatccaa catcatcttc ccttgatgcc atcattttac    23520
   atttatctat ctcatcctca atcaagttca aaacagggat tctatgtctt ctccttcgat    23580
   gagtgctcat tacttcctct acagtagcgt ctttatggat ccccatatcc atgtatcctc    23640
   tttcaccagc tctttctacc agacaaccca aatctgacca agcttcatgc caaaacgaag    23700
   tttgcttccc actcttcacc ctcaccttat agaaaccttt tgccttgtct caaagagatt    23760
   attatctctt ccatatggtt cagtactgtt tagtttgact tgctttgtcc gttgatattg    23820
   ttagctagct aggttagagg actaatttat aacccccaca tctttaactg ttttctctac    23880
   aaactgttat ataggcctgg ccataatatc cgtaacccga aatccgaacc gaacccgaac    23940
   cgaaaaacac gatccgtatc cggtccgaaa tgtaaaaaat atctgaatga gtcttgtaag    24000
   gtggcacaaa acatatccga acccgaagtg ttattaaccg aacccgaacg gataacccga 24060
   aaaactgaaa aaaccgaaaa ttccgaaaaa tatccaaaaa aaccgatctg aatgtccaaa 24120
   ataatataca atataattat ataaaacatg aatatatact tcaaatattc aatttcatat 24180
   ttattttgat atgttatcta acaataagta tttaaaattt aaataactac cttaaatact 24240
   tgattatata taaataaata tatattttta tatttacctt taaattttag attttatttc 24300
   gggtatatcc gaaccgatcc gatataaccc gaatccgaat gatatatgat tactttatgg 24360
   gttgtgatac aaaaccgacc cgaacccgat gtgttatatc agaacccgac cgtacttgca 24420
   aatttactag aatggaacct aagaagtatt ataagagaga accaaaatcc gaaaaacccg 24480
   atccgaacgc caacgggtac ccgaacgccc aggcctactg ttatatatca ataatttccg 24540
   ctttcaaatt tttgtttgat gcagatgaat ttgaaatttc tttgccttac gactccaaaa 24600
   cggtgactgg atttgttgga ttaatcaaca atcaatgtgg tgaaaacgca tatgcgagtg 24660
   ctctcattca aatgatgttt cacattccat gcttttgaaa agctatctta gaaattccag 24720
   agactatccc tgtcaagagt ctcttccact gtcttgagag cagcaagact actgtttcat 24780
   tagagagccc agacataacc agtaataccc tgaaaaaggg tttattttga aacagtgaat 24840
   gacaagatga aagtaagtta ttttttgcat aaccttatag ttcacactgt agatgctttg 24900
   atgtaaatta ttttgtaatt acaaggctca cattggaaga tttgtaattc tcggtttgag 24960
   tgaaatatgt gtattttctg caactaatga ctactccaaa actaatactt tgtgtccaac 25020
   ttgtgcacaa tgtaatggta attagtcgtt gaaaactaac atatgtgatt taacaataaa 25080
   ttcatatatt aacaattcaa aattaattta aagctttcgg atcccaaact atgctgtcaa 25140
   taatcatgtc caagtcatca atccatttgc gtaaggttaa tgagatatca agaaaaatca 25200
   aattcgtaaa tttttttaaa atatggttgt tacttctcac acactagagc atcgtccaca 25260
   aattccttag aaaaatttag tgttctgatc gtaatttgag tctcattgca atcaggaaca 25320
   agctgaaatc tacttatttt ttattgatgc tacctcatgt acttcatttt gtttggcagg 25380
   tgaaccagat gtatgagtgt atccttatga aatagatctc gagaagtttc ttctaaccac 25440
   tgatgctgat gggacaaatc atttcactta caaattgaaa aggtaaactg tgtgttttgt 25500
   ttgatgtggt tgagaaatag tagacacgca ccagattcat tctcaagaca cagcttcatc 25560
   ttcagaaaca gcttccctag aaagtctaat gtggtcatcg agagtattca ccattagttc 25620
   tcagatgatt gatccatcat cacaaactca atcaaaactc tcttctttgt ttctggcatg 25680
   ttgatataaa ccaaaacaag agactgaaaa ggaaaaaatt caaatgccag ctatagtgaa 25740
   catagacaag agcatgagga agttgaatga tatagctttg tagcatattt tctgccatca 25800
   gaatgtaccg gacatgcttg cctgccatca tatcatcagc attttaattt agacccaact 25860
   tgcttcacac cagactctct tccacatgat ttttatatat atcttctaac caaaaaatag 25920
   gacagaacaa attaaaaaaa aagtttcatc gggctgttct tgttaaatgc aatgaaaaca 25980
   acaaaatcta acagttcccc attgtcactc tttaaggtga cttgtttagc ctttactcac 26040
   ctgaaggaaa aaaacaacta ctataacggt gatgaagcaa gttggataaa caaaaaaaag 26100
   attggaaaag ggcaaaactt atagtgaaca ttgcacccaa aaaaaaaaag ttatagtgaa 26160
   catagacact aaaatgaagt atctagcttt cttacctttt ctaccgttag aatgtacggg 26220
   acagcttgtc agctacgagc ttgtctgcca aactttacaa tgtatcgttc tattggttac 26280
   ataacatata aaaatgaata gacagagaga catagaaccg agcatatgaa gatgagagaa 26340
   acgaactaac ctaaattaaa ccagaatgtt aataaatata atccaggaaa aacccgaact 26400
   aaactaaatt gaaccggtag agaactcaaa aaccagaaac cccgagttta tggaaactta 26460
   accggatgaa tcgaatccgg ttcggtataa taaaacccag aagaagaaga taaacctttt 26520
   cgcagtttgc ttctttctct gctcaaacac gaacaatggc gagtctactt gactcactca 26580
   caaccagaaa cttcttctct aaacccataa tctctaggat ctcctctcct tcatcttcct 26640
   ttgcttcttc ttcttcttcg aatatctcac ccttttctcc tccctccgtt ctctcttact 26700
   ctcacaaaag gtcgcattct cgcttccctt accctgtcgc agcaactctc gatggtccct 26760
   ccgttgaaga agacgagcta gagttcgagg aatccgaaga agacagctac cctgatgagt 26820
   cggatgaaga agatgacctc tccatagata tctcaattct tgagaaagaa gcgagagata 26880
   tcgttagaga ctacgctact actctgtctc gcgagctcaa actcggtaaa agattgtgtc 26940
   tttctttttg cattatgctc cattgactgt tgaataatga tcgtagcttg atgttttaca 27000
   gaggatgatg tagttgaagg gaaggagtca cgtagaaagg ggaagaggca agccaaaaat 27060
   gttagtcttt tttcagtaaa gtctagtcct ttgagcttga gatcttcttt aagtagataa 27120
   agttttgatt ttttttgttt ggttactgac atttactcta aaaaaaaaag aaccagacgc 27180
   agataccaga gcatcttctc caaagagttg ctatcgttgg aaggcccaat gtgggcaaat 27240
   cagcattgtt caaccgtctt gttggggtaa aagagtttga ctgttttctc cttacccatt 27300
   caagttttag agagttgtta acttgtcctt aatataattg caggagaata aagcaatagt 27360
   ggtggatgag cctggagtta ctagggatag actgtacggt agatcctact ggggcgacca 27420
   agagtttgtg gtggtggaca ctggtggtgt tatgactgtt tctaagtcgc cagctggtgt 27480
   tatggaagag cttaacgttt cgaccaccat tggcatggaa ggtataccat taagctccag 27540
   ggaggcagct gttgcgagaa tgccttccat gattgagaag caagctacag cggctgttga 27600
   agagtcagat gttattgttt tcgttgttga tggccaggtt cggttcttat cttcaatctt 27660
   ctctacgttt atgtctttgg cttattaatt attttggctg tcatgcaatg ttgttgatgg 27720
   gtggtaatga ttttttggta ctcgatcaat acgcagacag ggcctacagg tgctgatgtg 27780
   gagattgcag actggttgcg gaagtattac tcacataaga atatcatcct cgcggtgaac 27840
   aaatgtgaat cgccacgtaa aggactcatg caggcttcag agttttggtc tcttgggtaa 27900
   tttcactttc atcctaccct cagaatcatg tttgtgcaca ttcatagttt tcattgatat 27960
   ttcagttgcc tacaaaaata aaactcatgg ataaacgtca tttggtactt ttttatcaga 28020
   tgatttgaat atttgttgct ttgttgtgtt cacatttaac aaaaaatttg ttctaatttt 28080
   acggatttag tattgttatt ttgatgagtt tgtagacctg acgatacttg taagcatcat 28140
   aaaatacttt cctccgttgc tatacatttc tttgtcaatt ttggagatta tttggcattg 28200
   agcttaacaa gcatgtcatg atggaacttc caggttttca cccatcccta tttccgcatt 28260
   gtcgggaact ggaacaggag agctacttga tcttgtttgt tctggactaa acaaactcga 28320
   ggtttgtatg taacctttta gtatgttcaa ctggcatcag tttaccaatt atatatcaaa 28380
   accaatgatt ttttttttct agatcatgga gaccatggaa gaggaggaag aagaaaacta 28440
   catacctgcc attgcaatta taggcaggcc aaatgttggg aaaagtagca ttttgaatgc 28500
   acttgtccga gaggatagaa caattgttag ccctgttagt ggcactaccc gtgatgctat 28560
   cgatgctgag tttaccggac cagatggaga ggttagttca attttttctc gtgtttgttg 28620
   agctgcttag ttttcttgcg gcttctatga tacagtgcct gctgtttcgc tatatgctaa 28680
   ttggcatcta tttgatgcgc tttccatgtt aactcctacc attttttctc aatttggttt 28740
   ggctactatc atcaatgact agcattgtcc aagttgaata ttgctgaact ctgaatatga 28800
   gcagtgttca aaggtgcatt ggatccgtat aacactgaaa atgatttatg aactgtgttg 28860
   cagaagttta ggctaataga tacggctggg atcaggaaaa aggcagctgt ggcgtcatca 28920
   gggagcacta cagaggccat gtcagtgaac cgtgcattcc gagcaattcg tcgttctgat 28980
   gtggttgctc ttgtcattga agccatggca tgcataacag agcaggtatc tcactagttc 29040
   taaacaatgt gggaaacgaa ctcatctttc tctccttcct taatggtttc ttatttggaa 29100
   acacaggaca tgaagatcgc agaaagaata gaaagagaag ggaaaggatg tctggtagta 29160
   gtaaacaaat gggatacaat accaaacaaa aaccaacaga ctgcagcaca ctacgaggat 29220
   gatgttaggg agaagctccg ttccctcaaa tgggcaccca ttgtttattc tactgctata 29280
   actggccata gcgttgacaa gtacgtctcc ttccaaagtt ttataaacta attcaacctt 29340
   tacattttta actttattct gttggagaaa aatgtagtat tgtggttgct gctgcgacgg 29400
   ttcaaaagga gagatcaaga agacttagta ctgctacatt gaaccaagtg attagagaag 29460
   ctgttgcgtt taaatcccct ccaagaaccc gaggaggcaa acgaggccgc gtttattatt 29520
   gcactcaggt gaataacgat aagatctccc aatgtttttt tttattagca gtgaaggcaa 29580
   atctttggag actaattgat gtgattaatg caggcagcaa taaggccacc gacatttgtg 29640
   ttctttgtaa acgatgcaaa gctgttttcg gatacgtaca ggagatatat ggagaagcag 29700
   ctacgcactg atgcaggctt tgctgggact cctattcggc ttctttggcg cagccgtaag 29760
   agatctgaca aaaatggagg aggtacgtgt tttgctagcg taaacttgtg attcattttg 29820
   aaaaatatga caatgttttg tatgtataac tttaaatgaa atttgcaggt ggaggtacaa 29880
   tgagaatgtc aagtctttca cgtgagagaa atcttgcaac aaaaaggtca taatgttaag 29940
   tcatctactc atctttgtta aattttgtgt attttttgag aaataatgta ttgaaattcg 30000
   atattatata aattcatagt ttgttcaaaa aaaaaaataa attcatagtc tctgactctc 30060
   cattgatctt ccatgtatgt tccattttgt ttctttctga gaatcaaaac aagatattac 30120
   taatcttctt gacagtttat agaaaacaga actacaacgt ttctttatgt ttatgttttc 30180
   ttaatctcaa gctgataaaa aaaagataaa cagaccaaag cacaggcgat gagtctctta 30240
   gtaaagctgc tctttctttt catgccacca tctccacctg taatagaaca aagaaacacg 30300
   aaactatctt tgtcaaaaac attagaaata ttcatttttg tcagaagaaa aaagtgaaat 30360
   gagatgatat atgataatga tataagtcta cgtagtatac gtaccagtaa tagtcccaac 30420
   gaggacggac tttcgtcatg cggttatcat cagaaggatg ataaggatga gcggcgtggc 30480
   gagtgttgtg gaaggcgcag caaccagctg catagacagc gatgaggagg acaaggacca 30540
   gaatattaac gaccgacaac tttttccagt caagacggat ctcctcaaga acaccagctt 30600
   tgcaagcatc acactcgtag cataacgttt caataccatt gttccatcta tagcaatctt 30660
   ctcctcctac tattactccg gtaacgtacg tgcacgccgt cggtggctta caacatcccg 30720
   actataaatc aaacacacac atacaaagtt gatgaagatg ctagcttaca ccatagggca 30780
   atgaatatga gaaaatatta atgaggatca tggcagtctt ttagcatgtg taaatggaaa 30840
   gattgaaaat ggtttgattt tttttttaat gtataaattg gagtaaaatt taaaatttaa 30900
   aatatattta taaattaata cttataattt caaaaaaata acatatataa atggagttat 30960
   taatacttat aaaaaaaatc tacagttaat aattttattg aacatggttc taggcactga 31020
   aaatggtacc tgaacagaag tcatgtctct ttggaagtaa tcaagtgtag tccaagattc 31080
   aatctgagca caagtcttgg aactcaagat acagcttctt atagagatcc aatactgagg 31140
   atctctaact ctctctctca accatggatg ataatcaccg agcctatact ctttataaac 31200
   cctccctggt acttccacac cgcctccttg gctcgtcacc accaggccaa acagagttag 31260
   acccataaga gtcgcgatga ggaagatcat gaccactagg taaacccaaa gagcccatgc 31320
   cacgttgaaa caggctccta tgaatccggc gagagatact aagagtatga tgaaacctat 31380
   aacgagtaga ggagtctgga ggaagttttc gcaagttgtg ctgcttcttg ccttccatag 31440
   agcggctcct atgattggta ttgaagctag taagctgagg aggtttaaga ctccaatcac 31500
   tgtgttgctg aatctgtaca tagtagtagt gttatcaaga agaaaccaga gacttagcag 31560
   tgccttctct tatatctcgc tcttttgtaa taagcagatt ctcttcttgc aatgtgaact 31620
   tcatcagctt tatcttacaa tcttctttgt tttttagtta ttatattggt taactctacc 31680
   ttttttttgc ttttgattct ctcttatcaa ttgggaattt tttaagcgaa aaagtttaga 31740
   gttaatggtt tgatgattca tgctcatatt ctctggtcgt ctcttgattt gaatcatcca 31800
   aacaataaca ttagaaatag tatttttttc aaataaaagt gtctttttgg acaattggtt 31860
   cttgtttatt attctatttt atacgtttaa ctaacaataa agcagaccaa aagtagtgcc 31920
   ttaattaaaa tatgatatac catattatta gatacatttt tctagcttag agtccaaaaa 31980
   atgcaatcat ggattatata gtggatcata tcatatgtgg gtattgtata ctttgaaatt 32040
   gttgactcgt tacgggaaat aattgagata ctttttatat atactagatc ctttctccgc 32100
   gctacgcgcg gataatatat ttaaatttgt tacatttatc atttttattt gtatgtaaat 32160
   ttttctatat taaattatat ataactaatt tttaaatttg agttttttta tatttttagt 32220
   ttgaagtaaa tatttctatt atatgtaaca caattaacta ataaaatatg aagaatcaag 32280
   tctgagattt taaagttatg taaaaaatat ataattatgt atagaacaca aattatctta 32340
   gtttaaaaga tcggaatctc atctcgaata aatttacgaa aaaaaaagta ctccaataac 32400
   ctacttaaaa tataaaaact ccttttaaaa aaaaagcgta cttaataata tttttttaca 32460
   tgtaatagtt gaaaactaac aattgaatat cgatctagaa tattatttta atatatctaa 32520
   tgtaaaatat taattgtaat aaacaaattt tgaattttgt aatattacat gaattagaag 32580
   tctttaaaat ctaaaatcta ttttattaac atactatatt ttttattcat ttattatttt 32640
   gacgttacaa aatattgctt tagttttatt tgtatattat ttttttaata atttagtttc 32700
   tttttaagta attttaaaat tatcaagaat gtaatatatt taaaattatt tatttaaata 32760
   tatccaaata taatgtctca tttttatgtg tgtttgcgtt gagcatattt aatttgtaat 32820
   ttgtatattt aataacacct tattgcgtgt cgttctatgg ttttaataaa tgtgttgtca 32880
   tctcattttt attactacta acatgatttt ttagtgatca atgataatgt attttaaacg 32940
   ttatgtatta tattttattg tatatttttt aactcttcgt gctggcactt tttttagaat 33000
   cattttaatt agataatagg ttttaagatg taaataaaac tgtgtatgtt gtaaatttag 33060
   acttcttagt gtagctgagc actatcaatt atggaaatta tattatgatt ttattttaca 33120
   aaatcttttt ttctgtgtat tttttttgtt ttattttgta tttttaaaat tttattgcat 33180
   tattctttaa ctgcagagaa ttgatatttc taatttttgt ttcatatacc ttaaaagtct 33240
   tcggttgatt tttgtttgtt ttctttctcc aattataatg tatagttcga ccgtttcttc 33300
   tttttttttg gatcaaacta ctaatatcat cagatagaaa agcttaaact ctaagaattg 33360
   agtgaatatt tgtgttagag aaaactgatt taaccaataa tatagtgaga tattataata 33420
   ttagaaggta tggaaactct tctatgttgt cctacgctgg acataattaa gttgttgtca 33480
   attgatttta tatttgtgat aactggaaat gcatctttat gtcttcctta catcatcctt 33540
   aattggtttg cggttcgact atttctaata tactgagagt aacataaaat tagatgttga 33600
   ttatctcctc ccaattagga atgcacagaa tgagagaaat tcaagatgag accagtttac 33660
   aattttgtcc tcatatctat atagagttag tttatagatt actctatttg tttcaaaatg 33720
   taatcaattt ttattaaaat atgtaatatt taaaaattat tactttagaa atctgtcatt 33780
   taatatataa atttaatcaa ttacacagta atttacataa tttaattggc tacacaatat 33840
   ccaataaata taaagttaca ttgaaatata aaaataattt atatagtgaa acaaaaaata 33900
   cttttaaacc attattatat tataaaacaa agagaatatt taaattatat tgaacattag 33960
   aatagtaaaa atcagaaaag ctgaaatctc aacgtcgatc atttacgttg gctatgttat 34020
   taactttgaa gataacgtga atgatcaaga acaaaaaagt taattttagc ttcagtcatt 34080
   tttttataat cattttacca aaaagaaaaa aataacagga agatgactaa taaaaaaaga 34140
   tacaaacaga gtactgtaaa aaggtgttga tcataatagc aaacattata gtctcaaaaa 34200
   ttgtcaactt tatattaaat attagtcaaa aataataaaa tatataatat taataaattt 34260
   tattttaaat ataatttaac ttttaaaaat tttatcatta cacatggtgc agaaaagcac 34320
   ctagtattat ttgaatttca aaaactatct gatgcactaa ctttttgtga caaatcaaat 34380
   ttaaggaact gggcaagaga ttgagaagag ggatgtggag aggtacaaag agtcttcact 34440
   tgatgagtta taatggagca ttgaagtgcc attgttgtag tcgtaagagt tacaccggtg 34500
   aagttgggta gacgaacaga tttggagtcg ctggagacgt ctgaggcaag taggctaaag 34560
   aaaaggaagc tataagttat acaatgggct ggctattttg atggttttag atggttcgag 34620
   acccatcatc taaacattcg cgatgacgtg gcagtttgat tggtagagaa tattttatcc 34680
   tatgtggcat tcctaagaag cttcaaaatt agtagctttt atatagtagg atcttgtgtt 34740
   ttaatttagt taaatccaaa aatttatttt caagttccat taataatgtt catgtgcatg 34800
   tagtcaatta gtaaactaag ttacaatttt gtatgcttac taattgacaa tatgtttacc 34860
   aattttgtgt aacgtttagt aaactacgtt aaatttacaa tgacccatga agcgaacgag 34920
   ggtaagcaaa gaataataga catggacgga tgaatttgct ttttaagatg cttgtgctgt 34980
   cccatcccca taactttcag aatattaggt tttgtaggtt gatgagcaca atatgatctt 35040
   tgattcctcc acttcgcttt tttgtgtaag caagaagaac aaaaaaatct tgtttcagct 35100
   gacaatttta cgtaccaccg ttttgatttt acattttgca gatgattttt tgacaggaaa 35160
   aagggttttt ttttaaaaca acatttgagc caaaagacac acaaacttga aaggctaaaa 35220
   gagtttacaa agattgtggt ccttgtgtgt gtggttcaaa agacaaacgg gaaaaggaaa 35280
   ataaaatgct aaataagagt ataacaagaa aatcgcaagc ttgaaacaaa gtgttatctt 35340
   cctatagaac cataatctct actagtatac atccaaaatc ctcagtattt gaagacatag 35400
   aagcagatgc tgctcagttg tactcagttt gcaaggtcaa gtcttgtaca cccaagtcat 35460
   ggtatacatc atacacatat tgcagtaaag gcctttcatc tatcccgctc ttcacctaaa 35520
   cacaaatacc cgaaataacc aactcaacat cagtgccgtt cttattgagt actcatgttt 35580
   taaccgataa gaaaacagga aggaagctag tacctggatt ctgagtgagc caacagtgtg 35640
   accaggcaca acctcccaaa aacgagcctg taaaacctct atgacatcct ctcgagaagt 35700
   aatctgtaaa taaagagagt tgaagagaaa cccagcttga tcgatctata tgtacacgtt 35760
   caggaaacac tttatggaac agagcgaacc tgacgcaagc atttacttaa tgcggaagaa 35820
   ggaatgtttg gaggtgccat ctgaagcaag acaccaccgg tggctttgaa gagtggcata 35880
   acaagcataa acactgtaac agataccaat cccaaacata ggacctcagc attttcaacc 35940
   ctgaaaaatc caaagaccgg aggagaaaaa tggaaaatgt aagaggtcag gtactaagac 36000
   atttttattc attttgttcc ttttaaagga agcgagcatt acccgagaga gaggagccag 36060
   gatgccagta tcagacctgc actgaaaaga cagaagcaaa gaatatgtca tgtgggatgt 36120
   atataagttt catgcaaaga gaaacatttt tccagctagc tctccgcggc tctgcttgag 36180
   ataaagaaag ccaaagagaa tatcacacct gcggatggaa tctgatatga catgcaagca 36240
   aacggagtgg tagttcatat cttctgcttt tctgtacact gcatcatttt acgaatttaa 36300
   gaaacacatt caagaaaaat gcaatatgta cgaagggaac aattcttggc cagatcagtt 36360
   gctttcatgc tgtctctctg ttttcactaa tcctcctact tattctatct taattggaaa 36420
   cccttgcttc tttgtggctt tacattgaca ggggtggcca gtttttacaa tgaaatatag 36480
   caaagccgcc aaacaaggct ataccatata aagcaccagt atcggtatta gttaattcaa 36540
   gtctaaccag aacttaagca taataataaa acagagaata cataccaata ttcatacgag 36600
   cataattccg gaagaaccaa acaccaagta ggttcaccag cagatttgtt accgctgata 36660
   caataagata atgcctgcat gaagtcgatt tcagtacatc atattaatgc aaagaactta 36720
   gtgtaaactt agtaagtgct agagaaataa ctacaagctc ttaggcatgt cacttgtgga 36780
   aagaaactta cttgtgctct gattcatctt gaacaaatgc atgaagagct tccacagcta 36840
   aggagaacga catgaacata agaaacagct gtctacacat atgtcaatac tgaacatatc 36900
   aatgtgttaa cccaaaagat aaaatgaatg agacagtgaa ggcaaaatga aaagataatc 36960
   aacattttaa acgagctgta agattattct attctatcaa caacagtaaa ggaaagtgtg 37020
   caaacactat cgacactatt ttctgaaaga tattaagcca gttttattgc atggcacatc 37080
   agccattaga ccctcaaatt aaaaccggaa atgacatcca acaaacttaa agaacatact 37140
   tacagcatta gtgaaagcag aaagaacttc aagtcttttg tacctggaag aaaagccaac 37200
   gattactaag tcacaactga ttcagcatta tgcaatgtca gtatcagcag caaagaagca 37260
   aacagacaag gtttcatatt tagtaattat aaaaactgga tcctatgaaa acaacggcgg 37320
   atctacttta ggaatgctcc agagaaagaa aagaaaaagt aatttaacaa attgggaaat 37380
   caaccgtaaa gtaccacaaa ccatgcattc caacatatat ctccagcaaa tacaagcacc 37440
   aactaatcgg atcaaatttg aaaaatttct cacccgtatg agtaagcatg atcaggcttc 37500
   ttccttgaag tcgccattgc aaataaagaa aacgtcagga gaccacatcc aaatgtcaaa 37560
   tggaatgcat cagaaaccaa acctgcaaga gtcacatcaa acatcaaaca tcaaaaaaga 37620
   gagcaagtaa acaacaacag taaacatggg aatctaaaca aatctcaaac caattttgat 37680
   atttacaaaa cctcagctgc aacagaagac aatatcttaa aaattaaaac tccataacca 37740
   aaaggtctct acatacgcat aggatctact atccatgcgg tttagttccc aaatttaaaa 37800
   gattatatta accaaaaaaa aatcaacttt agctgttttg tttcatctaa atctctagtc 37860
   actacccgaa gtaaggatgt gaagctcaca tcgacctgga gtcttattcc tccaacaaat 37920
   ccaactctct ccctctaaca aagttatcaa gaactaaaac caagcaacaa ccaaatcgta 37980
   cctacacgcc cagtcaatag cccaatcaac agctctgtag tagagtacgc cacgttaagc 38040
   gaaatcaaca taaacaatct cttcatctgc cgatttccat ttctcagcac cccaaaaacc 38100
   accaaaacca tacgcaaaac cgacaccttc ccaatcgccg ccgccgcttt cccttccccg 38160
   aagaaaacat catccccgtc gacagagtat atctccggcg gcatatcgat ggtggagacc 38220
   gtcctatcga gaaaaggctt agccgccggc gttcgcgggc cgtgggagtg gtgaaaggag 38280
   cgggagtaag cgagcctccg atcattggcg gcgtatccga cgtcgctgct ccgatcattc 38340
   gggagttcgt tatcgccgcg aagatcgaag gaaacggatc tctccatact tagctttctg 38400
   tttgctcctc aaatgtgtaa ttcaacgagt cttaaaagta cactgcgtct actagaatga 38460
   ccttaatggg ccactgatgt aagcccatat aagagctcaa gtcatgaagc gttgaccgca 38520
   tttaacgcct tattggactt gattgcctct cgagtagggc ttttaacgcc ttattggact 38580
   tgattgcctc tcgagtaggg ctgggacttt taaccgaacc cgaaccggat ccgactcgaa 38640
   atagaccagt tcggttcggt tttggacatg gccatattat ccaatagatc attgcttcta 38700
   atatcacggg tcggttccgg ttattaccca aaacctgatc aggtatttat ttaaaccaaa 38760
   atggatagtg taaaacctag aatattttaa aaagtattat caccacgact caaacccggg 38820
   taaaagtggc tatgttacca ccagaccact tcaacttata tgtattcttc tatattgtaa 38880
   atatctatag tatttcaata ttatttttta taaaattaat attttagaga ttttgaatcc 38940
   gggttggcca cactacaaat agagtatgta accactgaac catttcaatt tacacgtaat 39000
   tattttattt aaaaaatatg tatatgattt acataataaa tgagtacccg aaactgactt 39060
   ggaatcagag atatccgatc cgaaacctga accgaaattt atctagtacc tattggatag 39120
   ataattcatt tatctgaaag atccagaccc gaatggatct tacccgacct gatccggata 39180
   accgaagtcc cggatctact ctcgagtctt gtgatatgtt ttctcccata acaacaaaaa 39240
   tacagtatag tataatactt cagataaata aactttatta aaattagtag ggttaacata 39300
   tcacattcaa gcatttttca gcccgttaat atcaataatt aactttttat tcaagcattt 39360
   ttcagcccgt taatattaat aactaatttt tggttggatt tgtctcttta aggaggattt 39420
   gtctcatgat ttacaaataa caatattcat atttttagtc tcgtaagatt ttatactaca 39480
   aaatattaac tattataatt tataaataat acattattta ttattataat ataattatct 39540
   agactgtttg taaggtaatc acctagagct ttagaacact ttcatagtga tgtggaatca 39600
   ttgtttcagg cattgtgttg gattctggag atggtgtgag tcacactgtt ccaatctacg 39660
   aaggatatgc tctcccacac gccattctgc gtcttgatct cgcaggtcgt gacctcactg 39720
   attacctcat gaagatctta accgaacgtg gttactcatt caccaccaca gcagagcgtg 39780
   agatcgtgag agacgtgaaa gagaaactcg cttacatagc acttgactac gagcaagaga 39840
   tggagacggc aaacactagc tcatcggtcg acaagagcta cgagttgcct gatggacagg 39900
   tgatcaccat cggaggggag aggttcaggt gtcccgaggt tcttttccag ccgtctttgg 39960
   tcggaatgga agctgctggt atccacgaga cgacttacaa ttcgatcatg aagtgtgatg 40020
   ttgatatcag gaaggatttg tatggaaaca ttgtgcttag tggtggaacc acgatgttcc 40080
   ctggaattgc tgataggatg agtaaagaga ttactgctct tgctccaagt agtatgaaga 40140
   ttaaggtggt tgctccaccg gagaggaagt atagtgtctg gatcggagga tccattctag 40200
   catcactcag taccttccaa caggtaaatc atcttttctg cttgttactc gttttgtaag 40260
   ctgactatga tacacaatgt tggtattgca gatgtggata gcaaaggctg agtatgatga 40320
   ggcagggcca tcgatagtcc acaggaaatg cttctaagat tacgctcgcc gttggatgaa 40380
   agattttttc gtattatttt atatgttcaa cgagttggtt tcagacaatt ttttttcttt 40440
   ggtttttcct cactacaatt gtttcttgtt gtcacactct ctttggttgt ttctgccatt 40500
   aatgagaaaa aaaaagattc atttgtctta ttttcttttg ttccctcaca aatctgaaag 40560
   caaataaata tgaagaaatg aatttggctt atgttgacag gtttctacaa attaaaagag 40620
   aagaatagag ttttttacaa agagactgaa ccacatgagt agtaaaggtg atctgccaac 40680
   caaacaacgc aataactcaa actaacttaa ctgtaacgaa gattagattt atggttcact 40740
   tcaggtgagt aaaacatttt tcttaaagca ttggtatgtc ttgaaacgac aagttgcttt 40800
   atgtcaagat tcactagaac ataatagtag tttcagagga agctaagcca ttacactgtt 40860
   cagggatcca cattaagata gttcgagaac aagagttcac tagtccgaaa gtcacatttc 40920
   aacgatccca aacataacag cgatgagaag aagaacaata cagacacaag acattttaga 40980
   cactaacttc caacatcaca taattttagc caaaatgtca gaaaacacaa caaaatggat 41040
   aattaaagat tattgaaaca gaaacatagc ttcttaaaac aaagacaatc atagatgaga 41100
   aaggttcagt ttcttctttg cttcataact tattgttgta ccagaacaca cctttcttct 41160
   caccttgcct gtccggttcc acatagatac actccttcgc ctccctccac atcgccttaa 41220
   ccaccggcgt cccatcgaac tgataatact ctcccagtat cggctttatc gccttcgtag 41280
   cttccatcgc gtgataatgc ggcatggtcg agaacaggtg atgcgccacg tgcgtgtccg 41340
   tgatattgtg gaagaccttg ttcaagattc cgtagtctct gtcaacggtg gccaaagctc 41400
   ccctcaacca atcccactca gacgagtcat agtgaggcag ggaaggatgc gtgtgctgca 41460
   agtaagtgat caaaactaag aacccgttga caatcagaag aggaactccg tagaagcaga 41520
   ccatcgaggc aactccttgg acagcagcgt agcggtagag accgtagcag acggcgagga 41580
   tgccagcgtc ggagatgtat atctggagac gctcacggtc gttgtagatg ggagcgttgg 41640
   ggtggaaatg gcaagcgaag ccgccgtcgt aaggtctccc cgagacgttg aaggctaagt 41700
   acaaaggcca gccgagagtg aactgaaccg ttaacatcac ggtgcgtccc aaagggttgt 41760
   tgaggtactt gccgtaccac ttgatgtctg acttcttctt ggggacaaac acttcgtctc 41820
   tctcgaggga gccagtgttg gaatggtggc gtcgatgact gtacttccag gagaagtaag 41880
   ggacgaggag gaaggagtgg aagatgaggc cgacggtgtc gtccagccac tggtagtcgc 41940
   tgaaggcgtg gtggccgcac tcgtgggcta tgacccagac gccggttagg acgcagccct 42000
   ggcaggccca gtagagaggc caggcgaagt aggagagagg gtgagggagg agagggaagt 42060
   aagtggtggc gacgtagtag aagcaggagg ctatgatgat gtcccagatg aggtaggaga 42120
   aagagcgagg gatcgagcgt ttgaaacagt gcggtgggat tgctttcttg agttctccga 42180
   cagtgaaggg cggtgtctcg cagggtacgc gcttgatgtt gtcggtttca gactttttgg 42240
   agggaggaga cacttgcatt cttccacctg cacccatgtt tctgcataaa ccaaaagcaa 42300
   agactcaaag ttaattaaac caacaaatta atattcagtg ttacgttatt aaagtttcaa 42360
   aaaaatggac taccacaaaa aaaatggata gtacgtggga taaagagcag agaagcggca 42420
   tatagtggca aaccaaagaa agaaataaac gatagcgttg agaatactac tagttattaa 42480
   tgagatttga tacgttacag cttacatctc tttcttgtgg atatcaattt ctttcgtctt 42540
   ttattactaa actacgttag agaagacaag tcataatcac aatgtctctt agaataagca 42600
   acttgacaaa aacataaacc taaatgaaac agttgatagc aactctattg tcaacatata 42660
   gacttttaaa cataaacaaa caaacaaaaa tacaatcttt ttactatcat gaatcctatt 42720
   attttcttca tgcaaatcta ttgcatctga atctatgaag gagcatctaa tccaattaat 42780
   ttgcaagaaa aagtttataa gcatgaagtg agcataggcc aaatgaaact ttttcatgta 42840
   ttaggtaact acagaatcca gagccaatag gaatcggtca aatctctcag caaattttac 42900
   ccagagacat aagtttttac ccaaagattc aacaaatatg tacttacatg tctcaggtcc 42960
   agatctaaaa caaaagtaaa caaatatata taactttgaa taggaaggat atttctaaga 43020
   ccaggacgtg agactgctat ggtgattttt caactccacc aacccatcaa aaatatatat 43080
   ataattttaa aaacgattga aagtcaaaca gtttgtattc ggacaaatga aaatgcaaca 43140
   tttcatattc tccacggtat agacaatata atcagattaa tggttaagac agcatcaaga 43200
   tttcacacat gaaaacagag aatcaagacc agatctatag tttaatgcaa taaatattaa 43260
   cagatctaga aataaatcga ctgaaatgca aaaatgatag atagaacaga aaagcgataa 43320
   gaaaagaaca gaccaagatt gaaaacaacg taaaaatgag aagaaatgga agaagaaagg 43380
   gaccttgagc tggctgacgt agggggggat gaagatttaa cgttaataac gatgaaccct 43440
   acgaagaagc tcctcctcaa actctctctc tgtctcctct ctttctctct ctctctatct 43500
   ctttggtaat gaatctctct ggttctggta agatgcgatt gggcagaagg caggccgttt 43560
   aaattgacgg gggcccgtgt gagacgattg agtgacatct cttttctttc tttttttaca 43620
   agtgggcccc tccattattt cctctcttca tatttctttt ttttttatta tttttctact 43680
   ccaaatatct gatatttaca ccacttatat atatttacgg aaaagtgaaa aagatgccgt 43740
   gatcatataa ttgcttttat aaagtttatc acgtttcttt ttgtattttg tttttaaaat 43800
   gtaaatattg gtttacccaa tcctaatgtt cacaaagact agtttgggtt atgttataga 43860
   actcatccgt tacgttaatt agcattaaga ttatggtatt tgatgtcttt actttaatat 43920
   gaggaaacgt ccttgccgac tcacaagtag aactgtagaa gttagtggta gccggaaaga 43980
   aaagaaaaaa atgctataaa gttgaggatg gaggatcgtg cttggcttct tcttctccca 44040
   tgtgagaaga aaacttaaaa aaatatctgc agacattgtg gttgagatca cgtgaatagc 44100
   cccgagtgtg tgtgatgtac tttggagatt cgtcatatat tataataaat taatttaggt 44160
   ggtacacaag taggcagcga ctgccgacta gtaattaatt taagtacttt attaaagcta 44220
   tcgctctctc ttatgttaag atgattagag tttttctatg cattaccgct tataacgaca 44280
   cacattattt ttaacattct ttaaagatca tcacctacca tgatttcatt tcatttattc 44340
   agctgttact atcagcgacg gctattaata acatgaattt ttcgttatat agtctatcaa 44400
   acattataag agaactataa catcgacaac gattagttat acagtctagc atactcacaa 44460
   aataatctaa gctgataatc acacacacat cagagaaaca catgaacttg ctgaccaaaa 44520
   taaagcagag aaaacatgaa gtattaagta gagacagcac acaaatccta tccactgttt 44580
   tgccaatcat tcatcaagcc tgccccatct caaattcaaa ctagattcta aaaaaacacc 44640
   aaatggggaa aacttacact gacaatgaaa actaaacatt gtagtttcaa aaaacaaaga 44700
   caactaaaaa caagtaggag aagatgatat taggaatcac cacaacaaat gaaagagact 44760
   tgtttcacca gtaactctag gagcaattca tgacctgcgt agctctgctt ccccgtgagc 44820
   aaagtgacat ctatcaccaa atgtacagct tcctttagag aatctctcac acatcttcgt 44880
   cttgaagttg cttcctggat gtggtttccc ttcagaacca agcccacccc cgccaccacc 44940
   gccaccaggt ggtctcctag atgcggaatt aagcctccca atcagctctc taaccattgc 45000
   gctcgcttcg tttatctgct cgaatgttcc ttcaagctca atgttcttca ggttgggatc 45060
   tctctcgtga tcttggatcg atagctttgc tcctgtctga cgacatatct gtttcgaaca 45120
   gactccacct tttccaatga ttgcgccggc caaggaagca tccacactga tcttggctgt 45180
   ggctgaggca ccaaagctag agacatggcc aggccctgac tctcctctcc ccgaaaaccg 45240
   acctccacca ccaccaccac cagatccttg catgtttctg gaaacttgag acatgggtga 45300
   tcccatgttt gtcatctgtg ccataggatt gtatcctccg ggaacaaagt gcaagaaatg 45360
   gcagttctca ccaaaaggac agccagaagt gctgcaacag acgcataata aatatgtcaa 45420
   gagagttaaa aaccattggc attgagaaga cacaagagaa caaacagttg aaacacagaa 45480
   gaaatggcac atagcaacta tgctaactgc ttgtctatga cggacatgtt caccagtatt 45540
   cagtaatgca acaaccttac tagacactat atgaccatga tgagacaacc ccaaaataac 45600
   taatatctta ccaccaaaca gagaattaca acacatatct ttactgccac agctcaaaca 45660
   aaaggtgtgg attgattttc tacgctataa tcaccagttg aggctaatat atttctaaat 45720
   ttataatcga atatcatatt tgttatattg aaactttttt ttaactggtt cagcatgaat 45780
   aataatacat acctgtaata agagctaatc acttacatta gcatacattt tttagttgtc 45840
   aatttgaagc tcagactata aataacaatt ttgaatatta atttagttaa gaaaaaggat 45900
   tataacctga aaaattttgt gcatggcttc gatttgcttc ctaaaccaga ttccatctct 45960
   gttccaaaga ttaagtgttt ttctttagtt aagactttgc tgatgttcaa taataattgc 46020
   ttttagaatc atacatatca ttcataaatt ctcatctata tggaacttca cacttaaatc 46080
   aaagctgcta ctctagaatt ttcaggatta tttaccaaca actaccacat gaataactaa 46140
   cacataaaaa agctaattca atcacagcaa actcaagctt tgggcatggt gataatatgt 46200
   tacaagaggg actaaactaa agcaataatt aaaactcaag ataactataa gcttgattta 46260
   actttaagat cacttatcaa aagctcatcc atcattaaac tgaaaaatta atggacttta 46320
   gatttgcatc agatcaaaga ctgcagcaat ctctctactt agccagagaa gtgagcacga 46380
   aagaaagatc caaacttgaa tttactagag aatacgaagc tcgaattgaa gaaatcatcg 46440
   ctaccccaaa tcaaattgac aacaaaggaa ctattcatcc atcaattaag atctaaagtt 46500
   tcaaccttta acagagcaag tgaagcaaaa cagaaactgt aacagctaaa acactctttc 46560
   gagacaataa agtaagagga agagcgaaag agctaacctt gcttcgactt cttgaatccg 46620
   ccgccattgg agttgaatga gccagcttca ggccgtcccc tcttacgagc atccatggct 46680
   aaaggtgtac agatccgaat tagaaaccct actgtgtgag tgtctagaga gcgaatcgga    46740
   gaagacaaat cgaaatcgga attaaaggga gaaggaacga gtagagagaa gagaataata    46800
   ataagataaa taaaaccttt ttaaccctaa gggtggtgtt gtcttgtcgt cgagggggtg    46860
   agatgagatt ttcctttttt tcctaactct tcccgatttt tttttaattc gatttaaacc    46920
   ggagtggcgt aacaaataaa caaaaaggta aaccggaatg tcctgaaaaa aggataaaag    46980
   ataaccggat tcgcaagatg aggctgtttc gattcctttg atacggttaa catgtataat    47040
   gacgttgatt gttccttatt cgacatctta cgacgtaaga cgaaaaataa attgcgtacc    47100
   ggtctagtaa gttcggattc tattatgttt tccataccga ggagacagac acacaaccaa    47160
   tgtttatcat ttaccttaag gctgggcgtt tttaacttaa accggtgtcc acaccttaaa    47220
   ccgaaccaaa ataatgagtc cggttcggta acgggaccat gcaaataccg aatgggtttt    47280
   tagctctaag cacttcggac accgggtcgg tttggttagg taccaaacca aaaaatttta    47340
   aaagaggaaa atcatcttac aaaattactg tatatttatg tagaaactaa attattttgt    47400
   tgaaaacgaa gcggtgaaag tcagtataaa tattatatat atatatatat atatatatat    47460
   atcttacaag aaataataat tgttctaagt aca                                 47493
<210> 2
   <211> 40174
   <212> DNA
   <213> Brassica napus
<400> 2
   gggggtgggg tatagatttt ggagtttttt ataatttggg tagactttgg aagttgttta      60
   aacgattttg gtgaagtttt gaaaatacat ttaaacaaca ccattgttgc ttctgagtta     120
   tctctaacat tatgaattct aatgatatta tcacctccta ggatgcttac agtaaaagaa     180
   acaacagttt taattttttt cttactggtc ttaataagcc cattcagaaa tatctcccaa     240
   taatctcttc actgaaggtc cctacaggca cacgagacac tgctatatgc ttctgcatat     300
   ctttggcgaa tatagagctc atcacataaa aacaaaaaga tgatgaacct aaatcttcaa     360
   attgcaatgc gatctctcgc ggaagaatca aaaccttgaa gttgctgtac ttttcttttc     420
   tgtatggttc tctgagtggg actaagtatt atttataata tttttttgta attactacta     480
   tttttatttt ttattttttt tattttaaaa acataatata acttgataat attttgtttc     540
   tttttataaa agatatcaaa tttgaaataa cacaatccta ttggttggtg aacctaaaga     600
   ttcaaaataa acccaagaat aagtcaataa agaacaacaa tatttatttg atatatgttt     660
   tcataaatta tatgtagata aaatacaaag cacaacggtg cacaccagca tatgcaacgt     720
   taaagtttag attttaaaat aataaaaaac cataatacat ttttaaccgt agattttaag     780
   ttacataggg ccggcccgct gcggaagcaa cgtaagcgac ggcgtagggc acacaacgtg     840
   tttcaatttt tttttacagc taaatttgtt cactaacttt taaaaaaaaa tcatccagct      900
   gattaaaatg cacattttaa ttttggatta gggcatctaa tagtaatgga actcgtacga      960
   tgaaactttg aattttgtga aagaaaactc caataagtta atataatcat gttattggat     1020
   aatgtacata aattttaaag gtgactgttt atgaggaaac aaatcagata tgcacgattt     1080
   tgtttcctac aaatggctaa ttttaccaat ttatatttaa gttgtttaga ttgttttcct     1140
   tcgagtgtgt attaaaaatt tattcaaaag tatgtggttc attgaatatt gatatttaca     1200
   atgaacacgt ttcatacata gatgaaatat agatattaac ctaaaataac cttaacaaaa     1260
   attaaaatac aataactttc aaatcaatat tcatattatt agtatatata ataatactta     1320
   gtcccactca catttacaat cggctttcca aaactttgtg ctacattatg tttcatcttt     1380
   caatggccaa cactaaacaa aaaccagttt caatcttatt gcttactaca ttttgcttct     1440
   tgtcgttata ctttgtgaaa aatcaagcct tctcaaaagt ctcatatcca tctaccttag     1500
   gtctaaacca agagaaacta acacatcttc acttttactt ccacgacatg tatggttaca     1560
   acccaacatc agtggaagta gcagaagccg cacaaacaaa cacatctaaa acatattttg     1620
   gttccatgtt cgtaatggat tgtcctataa ctacacttcc taatataagc tccaatataa     1680
   tagggtatgc acagggtatg acagcatctg catctcaaac cgaattggga ttgttgatga     1740
   ttctccattt tgtgtttacg gaaagagaat ataatggaag cacgattagc attcttggac     1800
   gtaaccttgt gtttgagaat gttagggaga tgcctgttgt tggaggcagt ggcctgtttc     1860
   gattcgctag aggatatgct gagggaaaga cgtattcatt agatgtgaaa tctggaaatg     1920
   caactcttga atataatgta tttatcttgc atccttgatg tattatacaa taatgttaaa     1980
   gctaaactat tttattatcg ttgattccgc taattttttt gataattaca gtgatgatat     2040
   ttcataattt ataaatttgt tttacgtaaa ttaatgtcac tatgtcctaa tttggagaac     2100
   aatagtcaca ttgagaaaat gtagatcaac ttttttacta cagagttaaa ccgcaatata     2160
   agaactacca catgtaatgt gagagactca aagtttagaa tccaaatgtt tttcgattaa     2220
   tttgtttgtt ttgatatgga tctttacaat acaatttctt aaacactaat catttacaaa     2280
   actggttgtt aacttttcat tttttgttgg tccaagatgt gaccgagaca cttcaaatct     2340
   ctacttatat atctctttaa atttttgtaa tcaaccaagt tttagctatc tttgtaatac     2400
   aattttcact tcatgaatat tcgcattctc gtgaatcttg attggttgca ataaaataaa     2460
   aacaaaatcc ttggcatcaa ctgccaaagt cgatgttaga aaataatttt ttttttttaa     2520
   acgctagaat tttatagaag gataacagaa caaaggttct acaaggatca tcttcaatca     2580
   aaagagacaa agagaggtag aaatattgac ctaacaaaat aggtaactaa gccaacatgg     2640
   atatcctcta gcaatataag attgaaaaaa atcctcctgg atcacacttt ttgcaataag     2700
   tgttgctcct ttaatgttat gataagagtg gaattgagtc ttccaatccg ggaattcatt 2760
   cagtaatggt aagacctttg aagagtagaa ccttatagaa ggccatgcag atggtttaga 2820
   gatggcgcca atcaggtcat gatcatcact agcaaagatg atggagctga aatggagagt 2880
   cttcaatatc tcaataggcc atctctagtt ttctagagag gcatcctgtt tgcacgaagt 2940
   gtcagagaag gatcttcttc catggaggag aattttacct tcgctgctac gaagaatcct 3000
   agagtaacct tgagtcgtca gtaggtatca ctgcttcttt gtgtgttgat tgatttttgt 3060
   actcatcttc ttcattcatt gttctggagg tttaacttcg actttgtgat ggcatctaga 3120
   aaaggatcaa agctgaggaa agcagggtat accaatttaa agggtgctga ttctactgct 3180
   tcctcagcga cctcgtcttc aaagctttat caggagacat ctattgatga tggccatagc 3240
   tcccctgctt cttcatctgc tcaaagcaag cagcagttct tctcatcaga ttcgtttcca 3300
   caaagctcta agccttctaa agaaaacgtc acagtgacag ttcgctttcg cccactcagg 3360
   ttcagagtga aatacttgta tatgcttgca aatataaggt tgatgtttgt tctgtgaatt 3420
   tgacatatat tttttccttg acgtagtcca agggaaatcc gcaatgggga ggaggttgca 3480
   tggtatgcag atggggaaac aatcgtaaga aatgagaata atccgacaat agcttatgcc 3540
   tatggttagt ctttaacaat ttaattagtg tcatggtgaa cacatcactc aaaaacaaca 3600
   aaactaagga caaatagtat tgttcttatg tgatattcat tttcacttgt aatcatgtct 3660
   taaaatagga ggactaagat taaattggat attctttttt ttttgatatt ttgatttccc 3720
   atgttgtttt cttgaagttg tctttcaatc tgttaatgat tgattctctt tctggcagat 3780
   cgtgtctttg gacctacgac cacaacacgc aatgtctacg atgttgctgc acaccaagtt 3840
   gttaatggag ctatggaggg gattaacggt atgaagtgac cagttttaat gataagtcta 3900
   ttttaaaata gaagaaacga agagcagcga cacttgccgg cggcaattct gtaacaacac 3960
   tccactgtaa ttcctctctc atgcttgtta ttttttggtc gctgttctct gacgcgcgtt 4020
   agagaaacga acagggcttt tgtatttttt tttgtcacag attatttgtt gaaagactgt 4080
   tcttagtgtg ttattcttca gaacagttga tttgaatata atgacatcaa tgttaatcca 4140
   ctacagggac catttttgca tatggagtga caagcagtgg aaagactcac acgatgcatg 4200
   taagattccc taataccgtt tgatttcaac acaccctact gttgaaaact tgaaaccatg 4260
   tttgcattta actttagaat gtccatgact tacgattaat attatctgat agttttggtt 4320
   aattttttgg ttcaaatgtc agggtgacca aagatctcct ggtattatac cgttagcagt 4380
   gaaagatgct ttcagcatta tccaagaggt acttttgtag gatattggtt cgtccagatt 4440
   ccttggctta agtactagca gcctgacata ttaatctttt acagacacca aatcgagagt 4500
   ttctcctgcg tatctcctac ttggaacttt ataatgaggt gctattattt acctattgtc 4560
   ttgtctgctt tatattcatt ttcaatagta gcagagagtg atagaaataa ttttcctttg 4620
   agattactat ctttaaggtc caccttattt caggttgtca atgatttatt gaatccagaa 4680
   ggacacaatt tgaggatcag agaagacaaa caggtatgtg actcactttc agatcacctt 4740
   ttgatacatg caactacttc tcttgttgac tcgtaatata tttgttgtgt ttacgagtaa 4800
   tatattttgc ttatcataga atcttttatt gggaaccatg gttaggctat ttggtacctg 4860
   aaaatatata ttggttccat tatgaggact ctgctattag aatgctttca tatgtggccc 4920
   tttgtttcat tgcaatgctc tatgtcacaa tgttttcaag cacatttgaa gttgatcctc 4980
   ctaattatca tttgaaatga gaattgtata atgagcttct catggctttt attcaatttt 5040
   ttttaattga acccattttt gttgattcca gggaactttt gtagaaggga taaaataact 5100
   tcctctttaa gatatttcgt ccttcaactc tgtcatgatt tggtaaattt ccatattatt 5160
   caatgcatgc ttagtaaatc tttttcctca tgacagagca acgccatgtt ggatcaacaa 5220
   actttaattt gctcagcagc cggagtcata caatattaac gttggtatgt taataatctc 5280
   tttgaagcaa gggagaaatt gctatctgtg tgcagttgat atatcatttt tttctcctga 5340
   caagattaaa agtgaagctg tacacctctc acagctggta agctactctt ataaccagtg 5400
   atatagttag ttatgattag ctctttcgtc tacgctaaag cgaattcaaa taatattaac 5460
   tacccataat ttctctcata tttgcagaac ctcgttgatc tggcaggttc cgagagttca 5520
   aaggttgaaa ctagtggctt aagacgcaag gaaggatcat atataaataa aagtttgctg 5580
   actttagaaa ctgtgagttt ttttcttacc acacagtttt ctcttagtca acaataggtt 5640
   ttggagtcaa ttcagatagt acttttgtga tgttgtaggt gatacaaagc tcagggatgt 5700
   gaaggcttcg catgtaccat acagagactc taagctaact aggatccttc actcctcact 5760
   gagtggtcat ggccgagtat ctgtaagtcc ttttagatct gttctcaatc ccctttctta 5820
   tgaaatccac aacatgatta aaaacatctt gataacctta agcacatact ttgctagtta 5880
   tgtactttac tgattagttg tccctttgtg gatatggtag ctcatttgta cagtaactcc 5940
   tgcatcaaga agttcggaag aaacacacaa cacattgaaa tttggtcatc gtgcaaagca 6000
   tattgagatt cagcccgaac aaaacaaggt tttactcgca tatcttctac ctgcctttat 6060
   ttttttggta ctaacactac aaaactgcag ataactgatg agaaatcact aatcaagaag 6120
   taccaacatg agattcggcg actgagggag gagttggaac agcttaaaca ggacattgta 6180
   ccagttcctc cactgaagga tatgggtgca catgatacta ttctcctgaa ccagaaggta 6240
   tgctcgaaag ataccttcca tccccattta tacttttgta gtgattaagg gatgtttttt 6300
   ttctgaatag agttccaatt ttgaaaactt aaaatcttgt ttttaattgt cattcatgca 6360
   ttttcctgtc agtgataaaa gtttaatatg aactgataat agttgaaacc cacttatacg 6420
   ttggattaga caagtaggag gacattttgt gtcattgtgt tttagttaca atcacgcatt 6480
   tgagagaatc tgtagtaatt ttgttgtttc ctttttttgg ctatataacg tgataataga 6540
   agctagaaga tggtcaagcc aagctataat caagactaga ggaagaggaa gaagctagag 6600
   cagctctctc gagccgaatc caacagttga cgaaactaat attggtgtct actaaaactt 6660
   cactgacata tcgcttacct catcgcttta atcctcggag aaaacactca tttgaggaag 6720
   aagaggtaga atcactgata tagcttgaat agtataaatt gtttacacgt tcctgtggag 6780
   ggaagtcctg agattagaga tgttccatgt agagaagaaa agaagagccg gaagcatggg 6840
   ttgttaaatt ggctaaagcc taaggtatac aaatgtctga ctttttgttt aaagaataat 6900
   gctataagta atagtagaaa tcaccacaca gccttgtctc ctcttaggtg cagtttcttc 6960
   ttttaattat tctctggtca actccccttt gttgatccga gcttactttt acctatcaga 7020
   aaagggataa cagttcaagt gctagcgacc agtcgagtgt ggtaaaatcc aacagcacgc 7080
   catcgactcc tcaaggggga ggaagttatc tttacacaga atcaagactt tcagaaggat 7140
   ggcctttgat ggaaggaaca actctcagag tctagagaag acagagaagc tcctgaagac 7200
   atggagactc tggaggtaca taagagatac tttcacatct gtaatggcgc caaagtaact 7260
   aaatcattca tatgtgctct tgttcccaga ctagcaataa aatcatcgat gagttggatc 7320
   ttatgagagt gcagaaaaag attttatctg agggggtggc gcttcaatca agttcattga 7380
   aaaggttgtc agatgaagct gtgctttaaa tctaccaaat attcaccttc ttggttctct 7440
   aatgttactt aggaggagat taatgcccta catgatggca tcaaggcaaa gagtgaccag 7500
   attgccacct tggagaaatt aacaaatctt ggattatgtt atgacaacac atgaggcatt 7560
   ggataaatct gacatcctga aggtaggttg ttgacattgc aaagtattat gcagtgagtt 7620
   ctttttccgt tgctcatttc acaagcggtg ctctattatc ttcaggcagt tgctgagctg 7680
   agggatcaac ttaatgagaa atcttttgaa ttcgaggtga tcgaattgtt tatgtgttta 7740
   catacaatac ttcttcatct attgatccag cacctagagc tagctttgct attgattaga 7800
   taatatatgt tcacacctta gtaaagttgt ttctctgcaa atattagtgg tcaaatgcca 7860
   gactatttaa cgaagaagcc aaatcatatg ctctacgttt cgactcatca ttacatcttg 7920
   tttacaggtt aaaagctgca gaaaatcgca tcattcgggg aaaactcaat caaaaggtca 7980
   gaggctttgt ctttggagaa gcttgtaaac taggtttgta atgtgtagtg gaattgaata 8040
   actgatgaat tattttccgc agacatgtga acgtgaagta ttgcaagaag aagttagaaa 8100
   cctaaagcag cagctctcta attcccgcaa actagcacag gtctatctac atacatttta 8160
   ttcacgcctt acaccaatct tctagcttaa tagcttcatg actctcaaca taaaatctct 8220
   ctactttctg ttatgaaagg aaacaaagat cgaagagctg aaatggaaaa cttaaggaac 8280
   taaacgaatc taaggagcaa ctagaacacc gtaacaagaa actcgcggaa gagagctcat 8340
   attcaaaacg ccttgcatca gcagccgcag ttgagctcag ctcaaggcat tagccgaaga 8400
   agttgcaaaa ctaatgaatc aaaacgagag actatcagct gagctagcaa cacagaagaa 8460
   ctcagtcaca cagcgaagca acaagacagg aacaacaaca acaaatgtaa ggaacggacg 8520
   aagagagagt cttgcaaaga gaaatgaaca agacaactcg tgggagctaa agaaagaact 8580
   aagaatgagc aaagagcgtg aactatcata cgaagccgca ctcgttgata aagatcaaag 8640
   agaagctgag cttgaaagaa tcgtaaaaga ctcaaaacat agagaacgag ctcgctagca 8700
   tgtggattct tgtttcctgt gttttaaaag gcgatcgcct tttgcgccaa ggcgcaaggc 8760
   gcaccggggc gatggcccta atgcctcagt cctctaaggc gagccctagt tactcaaggc 8820
   gctcgccatg gtgcgccatt ggcttaaata taagcgcctt tgaacctctt aaggcgcttg 8880
   tagtttccgt taaacactac tttgacggaa tccttaaaac atcttggaac tctatcacta 8940
   ctttgtcgac catttaaata tcctaaaaac ataatgctta gatcttcaga gttataggtt 9000
   ttgttggatt tgagcaattg aatttagctt tttagtttca agttctttgt tttgatttga 9060
   tgttttcggg tttatatatg tgtacatagc tgactatatt agtgtgcatg actaactcgt 9120
   tgctgactat attagtgtgt ataaataaga cagtttggta tagctgactg tactagtgtt 9180
   atatacgagt ctactctata acttatcttc tgtacgtaac catagaaata gttacacgtc 9240
   tgtagcttac acatacactt ctaacctaag aacatctatc aatgcatatc taagttcctc 9300
   taaagctgta tataactaaa ccacgtttga atgcatccta accataaggc gagcgccttg 9360
   gagcgccatg gcgcaaggcg catggctcca acctcctcgc cttagagcgc catgcgccat 9420
   ttaaaacaca gcttgtttct aagctgagaa gatcacaaag aagctgattc tgttaccata 9480
   tcagagacac cgccacaaaa acttaacaaa gacgaatcca aacaaataca taatcatcac 9540
   aactttaaat tgtttcttat tccgaagaat taaaaataaa acaaaaggat agaacaatga 9600
   tcaatactaa cggatcagtc agttagtctt gggatcaggc atggggaaca aaccagcacc 9660
   agcagcgcca cgtggcggag aaaaccctag aaacttctgc agattcgtcc ttatactaat 9720
   cgaacacagc aaatacaaaa acgccatgga acaatccgtg gcatcatctc ccttcaaccc 9780
   cctgtgactc atcttcttca cgatcgcgat cggatggaac gggagcttcg cgacgacctt 9840
   accttcgaag agcgagttca acagcccgaa gacgacgaag aggacgaggg ccacgacggc 9900
   gccggatttg aacttgaaga gggacagatc tcggctcgac tccttgaggc tcgtctcgac 9960
   gcggtccatc ttcttggtct tcgatttctt gacggtgagc ttggaggaag ggttctcggt 10020
   cttcatcgtc tccagcttct tggcggcttt gtcgattgag tattttaggg atttgtagga 10080
   ggtggtgcgg tagattagga tccacgagat ggcttcgcag actagcgccg tgcagaagga 10140
   gatgccgacg acggttaggc tgtcggcgta cttgaaggag gcgaagagtg ggatcgtcgt 10200
   cgccattggg atttagtttc ttctgctttc gcttctgttc tctctctctc actgtggaga 10260
   tctgattagg aattgggtaa aataaaagtc aatagagggt acaataagta aataaggaaa 10320
   agtttggggg acttttagaa atacttttta attttacttg taaccaatac ctttcgaatt 10380
   attagaattt accccagctg ttttttttat tccttgctat gcaaaaccca aattacaatt 10440
   tttaacatta gcataaacac tgtggtgcac atcaaatagt gtaaacttgt aatcatgcag 10500
   atgtatgttt ttactcacag aaaagaagaa gcaagggaaa aaaagattgg taagtttgta 10560
   ccaaactcat tagttgtatg ggttttatgt actagattat tttttctagg tttgatttga 10620
   atcagagttt atttgggttt tgattagaaa aatgtggagt aaaagagagt aaataataaa 10680
   gtggaaaatg gagaaaactt tggaaattga aatgaaaaga gaacaaaaaa ttagaataaa 10740
   actcgtttca cttataaatc ccaaaataaa tatgtgagtg ttttttttta atttcatccc 10800
   tcaacttaat gatgctacat agttaacatc acgaatttat tttcattcat ctaaaaattg 10860
   atgtagattt tagaaaccgt ttaaatctta tgtttttatt aattaaaata tgatgtacat 10920
   ggcggtaatt taactaaata tgaaagttac ataagatatt atttaatttt ggttttcaat 10980
   tattttaata tgttttctta ttaactctta ataaaatttt tgtaatttat ttgattaatt 11040
   gtgtggaata tacttatttg atcatttttt ttattataaa acttatttca tgtcaaatta 11100
   ctgactctaa cattttattt gtttgatata aaattaaata actaatgaat tattttattt 11160
   atatattcat aaaaaaaaat tgcatacatt ttaaaatgta tcaaataagt atatatcaga 11220
   caattaatca aatgaattac atatttttta ttaaaatttc ataagaaaat ggattaaaat 11280
   aatttaaaaa caaaattaca taatatctta tagaattttc atatttaata taaataaaaa 11340
   taatataata atatattttc atatacacaa aaataaaaat gttaaaacac tacttataaa 11400
   aaaatgttaa aacatattga ttttgtaaaa tcaattatgt taaaaatatt cttacacttt 11460
   taaaatgtgg gtcaaaatct catttagtta aattacggcc gggtcggtca tattttaatt 11520
   gatgaaacga gatatttaaa cggtttctaa catttacatc aatttttgga tgaacgaaat 11580
   taaacttatg atgttaatga gatatgttat catgcataat atataattcg cgtagctagg 11640
   tatacattag tttagtttgc atggttaacc atgtgaatga ctaatacttc aggtagttag 11700
   caaatatttt tttccttctt ttaatcaatg aacctaaatc cggattgaaa tcctgataat 11760
   tgaattcaac ccaatagaac aatagtgtta tcatagttac aactaaaaaa aaacttaaca 11820
   gacaaatcta aaatttagaa atggaaatat gagaaggaat ggaaaaactt aacagacaaa 11880
   gcgtttcaag aagaaaaaaa aattctagag gcttcgtttt gcgttagttc attataatgc 11940
   aagtagagcg aattcgatga tttatttatt ttgaagtacc atctcattag atggtatttg 12000
   tttctagtgg attttgtatg taaaatcaac taggtgatta tccactcaca tgtgtggagt 12060
   aaaatgtata tattctaata attgttgaat atgaactttg tccgtataac cataattata 12120
   ttattcataa gcctatatat gtattaattt gaatatctat taaatgttag tttactcgta 12180
   tggtttttat gatcatttat attttattat aataaaaaat ttaaactata gatcataaaa 12240
   ttttcagtgt gagagtttta acaattttca ttatttatag tcgtttttaa acattcaaaa 12300
   tataatatat acgaaaaatc tattttttat tatatggtta atatgattgt ttaatttatt 12360
   ttaatactat aacattaaaa aataatgaag atatgtgaat tgttgtcaag tctttattat 12420
   taaaattatt aattgtcaaa tatatatttt agtcacgttt ggtaattccg taaattttat 12480
   ttaagaaaaa aaaacaataa ttatatattg ttaattaatt tcatggttat tctaagtaga 12540
   agtatataat acatgtttaa tagaccaaaa tatttcttta gagactttaa gaaacattat 12600
   agtgatgaca cgtgttatag ttaaaatgtt gtaatgctta tcttttaata tatagaagat 12660
   tatctaataa tttccaataa ctcttgataa atactaggaa aagaacccgt gcgatatcgc 12720
   acggtaactc attttgtaaa aaatacaata caaataaaat tacaataatt tttgaagata 12780
   atttttttaa attctttata agagtgtata tatatatata tgaaaataaa tgaaaagcca 12840
   atttcatata ttttgataaa aaatttaaaa acaaaggcaa tttcatgtag tgatctttga 12900
   tgatctaaat aatgaatctc tttctttttt agtttatttg tgattgttgt gattttctaa 12960
   ttttatttgg tgtttattgt ggttgttatt tgtcattttc gacaactatt tatatatatg 13020
   actaaggatt gcaatagttg acaatcaacc tttgcaaagg tttaactaag ggttacactg 13080
   gttaacacca aaagttgcaa cagttcacaa ctaaaaacaa taatcactaa taattgtaat 13140
   ttttttatca tcaaagaatg taatggttca acactaacag ttgcatcatc taaccaattg 13200
   taactattca ctaagacaac atactccact taaacagtct caaagattca cttaaatctc 13260
   taacaattat ttcttattta attattatca gagattcaca aaataaatat cactcgtatc 13320
   aaactcatgc acgaataaaa atgtacgtaa catcaattcg aaaaacatca taaatgtcta 13380
   tttatttgat agataacaaa atggatcata tatttttata aaaaaatagg atcatatatg 13440
   cgttgtacgt atataatttg acatatcatt aaaccttgta tcagatgtga tgtcaaagtt 13500
   actaatatgg tagatagatt attctttgaa aaagtatttg taaaatggga tgaccgatga 13560
   ttaatagtag cttaacagat ctctcttcac aaaaataaat aaattaatag actatttaga 13620
   agaaataaaa tatcagatgt aaaagttgga atcttcgatg tataaatgtt ttcaaagagg 13680
   tgtctttgga agaggtttaa gccgttttga tttgaaggaa tgcattgatt cgagaaaggc 13740
   aaaagcgttc tttggtttaa taaatgatga tgtcatctat ttaaaggaaa tctaattttt 13800
   aaccattaga ttattataaa ataaagcaaa tctaacagtt agatatatct ttttttccta 13860
   taaaaaatga tgatattatc agttaacaaa aaaacaggtt tcctattatg tatagaaaat 13920
   ttaaaaaaga agtaagaaaa acttgctttg atagtagcaa atgtcatttt atttgcataa 13980
   cttcttgtat atctgccaag aagaagcaag gttggtttca aggtgactct ttaataaaaa 14040
   caaataaaca cgaaccaaaa acaaaaaatc agtttaatac ccagcgagtt gattattgat 14100
   catccatggt ctccagttat caataaccga atagttgaga gatcttatcc aagcttgagt 14160
   cccaatgtaa ggtactgtca tatcgtgatc accaatgttc acacaaaacc acacaaatat 14220
   ttacataaag attttgaatc ttggtgggag aaaaaaactc tgacctgaag atgagagaac 14280
   gatagccatt gatgctatta tccatatggt aaggtacatt acttataatg tcgttactgt 14340
   aaggaatatc caaattacat cgtatccatc tccctatgct ctactacaat tatagatctt 14400
   cttaaaaaac ttgacagcgc ttctttacta aactctttga aatgaacagt cttatatacc 14460
   ttcttgattt gaagagcttt gcgtacgctt tcgctttcgt catttgccca gtaggtagct 14520
   agcaaatacc tataaatctg caaaagcaac cttatatatg ctttgtttcc tgaatggttt 14580
   aacggtaaag aaagaaggtt aaaaatttat aacagtagtt attttaaaat tatacttata 14640
   ataatagtca caacttttca ctttttaaaa gatatctata cataatagca aactccacat 14700
   tctgtgtttg atgacatcaa cattttttat agcaaaataa aattgaaatc ggacggttga 14760
   gattattgcg gttatgtaat ctagtggata ttatttaagt ttcatttagt atatgatgtc 14820
   atccgttatt atatctaaac gatgtctttt tctttttgaa acgttgcatt tttgaaacat 14880
   tgcttctgaa gagacacgac cctctcaaaa taataattct tatatctaac ggtgtcttgt 14940
   ttttgaaacg ttgcattttt gaaacgttgc atttttgaaa agatacgacc ctctcaaaat 15000
   aatagttctt aaatcttaac aaatttatat attcattctt tatgacttcc ctacttcccg 15060
   tcatcattgt tcttttttct ccttcactta ccatggattt cttttgttct ggaaataacc 15120
   gtctctctct aagaattttc atgtctgttt tcaccaccac cgccatcctc ttttctgttt 15180
   gttatgttaa tctccatcct cattttctgt ctccccaacc cactactata ttaatttact 15240
   cataaccgtt gaaatattag aacatgtgtt gtcctcatga aaccatttga tgatatttta 15300
   accgggagat tcaagagact gaaatttata aaagggaaat atgatttcat gtttcaagaa 15360
   ggaactgaca atcataatca atttaactta caaattagcg agaaaagctt tagcgagaga 15420
   attccgatga ttctccctcc actgtttcca ggccttatga gaatataatc catcctagga 15480
   aaattaattg taccaaaaat ttaacaaaaa aattcaccga gcatctttcc acaaatcctg 15540
   tatatctgaa gaaaataata attgtgttga attctttata attgtgtttt ttcacaattc 15600
   tttttgcacc atcaaatcat catatatcac tctcactaat acgcatctct tcgctctcaa 15660
   ctggctattt tcagccaatt gttaaacaat ttttttgatt tccggctgac attgatccta 15720
   gcactggttg ttttctcttt ttatttactt ttgtttttaa tacatttaga tctcggttta 15780
   ctaccattaa aatcagtgga agagacttat aattgaaaaa tgcatggcgg agactattta 15840
   tagattttta aaatctattt gaatagtcac atcccttcaa tctgaatagt tgcattcttc 15900
   taatactcac aacttttcac tttttaaaaa atacttatga atagtcacaa cttttaaaca 15960
   atttttagaa aagacacaac cttacaacat agaactttta gaaaagacac aaccctttac 16020
   acatcttttt caaaagatac aacctttcaa tataagtgag attcacaacc tttggaatta 16080
   atgaggattg ctattattag tagattatga gtgaaatctt tgttttccag taaggagaga 16140
   ttcgaaagag ttttaataaa tgattaattc aataagagtg gatttgatag aacttttata 16200
   aatactaaga ggagatttga acaaaaacta gaattctctt aaatcataag ccccctttgt 16260
   tcctatctgt tttttggtca ccattccatt ccagaaaatg cttttaaaga taacaatcac 16320
   aacattccaa atgtagaaaa ggcacaatct caacattcta aacctgcgtc cacctaacaa 16380
   aaaacaattg ccttctcgta gtttactcca aatacaaagg gtgtaatata acaacagaaa 16440
   cactcaacac tttaccgaac acacacgttg ccttctctcc taactcctta aagttcaatc 16500
   ttcataatct ctctctgtct ctctctctct ctctctctct ctctgacaac agagagactc 16560
   ttcacgtgcc aacaaaaaaa aactgagcac cttcctctcg cccatggcca cggactccgt 16620
   caagcacgtg cctacattcg gcggcgcagc catctctgaa atgaaaagct tcttctccgc 16680
   catgaaacca agaaaaacga tcataacttt tgtctacgcc ttcgtcataa cctttgttgc 16740
   cttcactgtt tacttagcct tcgccccttc cctcatcact atctctaatt cagtttcttc 16800
   ctatatcctc cctaatgtca gtgccgtgac ttcagcgtcc agtaacatca cattacaagc 16860
   aaccacgccg gaaagtctca ctccggctgt tataaacaca acctttgagc ctcccctagg 16920
   taatgaaaca aacccacatt ctagaaacaa cgcttcacgg tctcatgcaa gtgtacactt 16980
   atgtcctaac aacaacactg ctcgaaattc ggacaaacaa gcacctctgt ccgtgaattc 17040
   aagtgcttct tctccgatga gaaaacaaag taggaagtca ggggctaaac gagagatcaa 17100
   gtctctgaag gactgcgatt ttttccaagg agaatgggtc aaagacgaat cctacccgct 17160
   ttacaaaccc ggcacgtgta atctcatcga cgaacagttt agctgtttaa ccaacggaag 17220
   accagacgtt gagttttaca aactgaagtg gaaacctaaa gaatgcactt taccaaggct 17280
   gaacggaggc aagttgctgg agatgattag aggaagaagg ctcgtgttcg ttggagactc 17340
   gctgaataga aacatgtggg agtctttggt ttgtattctt aaaggatcag ttaaagatga 17400
   gagacaagtc tttgaagctc atggaaggca tcagtttcgt cgggaagctg agtacacttt 17460
   ggtcttcaaa gtaagtttgc aatctgtttc ggtgaggcct gcgggtttgg ctagtttggg 17520
   ctgggtcgtt cggttcgacc aaaatatcgc ccaactgaac cgaacagagt ttggttcggt 17580
   ttaggtagtt cggtttttca acttctttta ccgaaactaa tcaaagtttt tggtttcaat 17640
   tatatgttgg tttaaaattt ctttaaattc ggggtaattt ggttaattcg gtttgtcggt 17700
   tattttgatt cgaatttttg attagtttag ttagcttttt tttggaaaac ccgagctaac 17760
   caattactga agtgaaaacc gaagtatttt ataagcttgc cgaattgaac caaactaacc 17820
   aaaaattttc gttcggttcg gttatcaacg aggcctagtt tgactaccta gttgttattg 17880
   gagacttgat ttgtgtggtt gatatgatag gaatataatt gcactgtgga gttctttgcg 17940
   tctcctttct tggttcgaga atgggaagtt acggacaaga acgggacgaa gaaggaaact 18000
   ttgcgtctag atatgatggg aagctcatca aagcagtaca taggagcgga tgtacttgtg 18060
   ttcaataccg gagcttggtg gactgatgac ataacatcca aagggtagag ttctgtcagc 18120
   tattttttat ttttttatat ggaggatttg tctcattggc tttttggtgt ttggatggtt 18180
   ttgattagtg aggactattt tcaagaaaga agcactgttt acccgaaact caacgttgat 18240
   gaagctttta gaaaagcatt gactacttgg ggtcgatggg ttgataagta tgtgaatcca 18300
   aagaagtctc ttgtcttctt ccgcggattc tccctgtcac atttcaggta tgtacagttc 18360
   tttcatggta gtcttaagat tctgtttaaa aaaataaata aatgggtttg gtctggttgc 18420
   atgcagtggt gggcgatgga atgcgggagg ggcgtgcgat gatgaaacag aaccgatcaa 18480
   gaacgaggca tacctaatgc cttacccttc caagatggag attcttgaaa gagttctaag 18540
   gggaatgaag acaccggtca cgtatctcaa catcacgagg ttaacagatt acaggaagga 18600
   tgctcacccg tctgtttata ggaaacagaa atttactgca gaagaaagca aatcaccgtt 18660
   gttgtaccaa gactgcagtc actggtgcct cccaggtgta cctgattctt ggaacgagat 18720
   tctctatgcc gagatgctgg taaagctcga ccagctccgt ggcaacagac ggcggaaacc 18780
   tgaagggcta ctataggagg agttgaatca tattcttgtt ttagatgaaa tacacaatat 18840
   atattttcaa tggatgaaaa gaaaagaaac acttagaagc aattatatgt tttcaaaggc 18900
   atagagaaag taagagatga gaatcatatt actgccttgc tcatcacttt tcttggttgt 18960
   aaactatgtt catgggagag gtttgagatt gtaaatggtt aatttttatt ttactcaatt 19020
   taatagtaag gtttgtcaaa tcataactga accggaaatg gaagcaatca tttggttaaa 19080
   agaatccggt tgatgacatc gactggcaaa gcataggcag gatcaatttg gtttgactcc 19140
   aggatactcc agccctgcac cagttttgga tcattaagtt ctctttagct ttgaagacct 19200
   taacagtatg tactaatgag acgcaaattt tagtcacctg caactccaaa gtaagtgtga 19260
   aagatatcaa cagcatcgtc aggtccattc tccatatctt attcaaatta cacctgaaac 19320
   tcagggaagg ctctaaagag atgtgagttt tatcaaccaa tgtttgagaa aaacctgaca 19380
   gtccaagatg aactttacaa gctttgattt gtcaatccaa tggactctgt tggtcatgat 19440
   aaaactcgat aaaacccacc aagagtggca aacctggtga taattaagca agaacaatca 19500
   gtgtggtaag tgttaggata atgacgtatc attatagaga taatcatagt ttaagataac 19560
   tgttgtattt atcttataga taactatgag catgtgataa gatcaacttg actctatcac 19620
   atgatctcga cttgtataaa tagagagctg cagacatcaa taaacttaag ctttccacaa 19680
   tacaaatctt atatggtatc agagcaattc tcgatccaaa tcgtttaaat ttttcttttc 19740
   tcttcacgca aacacaaaca tcgttaacat gtctgatcaa aacagctctc tcacaactgc 19800
   cactgctaca cgcaccgcag tctatgatcc cgcaaaccct gcaaactcgc ttcttgcggt 19860
   taacatgtcg aacgttacac gtttgacaaa caccaattac ctcatgtgga gcagaaaaat 19920
   tcaagccctc cttgaaggtc atgaactcca cacttttctt gagaaaacag aatccactcc 19980
   agaggcggtc ctcatcaaca atggcctcgc agaacctaac ccggcgtatc taccgtggag 20040
   acgtcaggac aggctcctat acagtgccat cattggtgct atatcccttc cagttcaacc 20100
   actcgttgca agtgctacaa ccactcatga agtctggagc acacccaatc tcatctttgg 20160
   cacaccaact cgcggacaca tcaaacaatt gaagttccaa gtcaagagct gcaccaaagg 20220
   aacaaaaacc atcagtgagt atctgcgtct tatcaagacc aaagcagatg aactagcact 20280
   ccttggcaag cccattgacc ctgaagatct gatagagcag attctagcgg gtctctctga 20340
   ggaatacaaa gccgaagttg atgcaatcaa cggccgagat catctgatct ccttctctga 20400
   acttacagag aaacttctta accgagaagc catgattgtc tgcgatcaac cagcaacacc 20460
   gacgtttcca gttacagcta acaacaccac aagaagcaac accaacaaca ataaccgcaa 20520
   taacaacaac aactggcgtc catcctttgt cccacgacaa ggcaacaact cgcctcgccc 20580
   atctcgtccc taccttggaa gatgtcaggc ctgcggaatt caaggacata gcgcccaacg 20640
   gtgtccatca ttcaaagtca ttgcaaccaa ctcaatgcag caaaacaccc aacacgctca 20700
   gtggcgacca catgccaatg caacgtacat gacaaaccaa caccctgatg cttggcttat 20760
   ggacagtgca gcctctcacc atgtcacgag tgatctcaac aacatggctg cacacatgcc 20820
   atatgcagga ccagatggca tagtgattgg gaatggagcc aatcttccca tcacacacac 20880
   cggttcactt tctcttccaa cttcatctaa aagtttcaat cttaatgatg ttctttatgc 20940
   tccatctatg caaaagaatt tgatctctgt taaccggttc tgtaaaacta acaatgcctc 21000
   tgtggaattc tttccaacta tgtttcaggt gaaggatctt ccaacgggga caccggtgct 21060
   gaccgcgcca gttaatggca acctctatga atggcctacc aatgactcac gcactcctct 21120
   tgctttctct gctgtatcat catcgtcctt agactggcat cacagactag gacatccggc 21180
   ttttccgatt ttacagcata tttcttcttg tttttctcct ggtttttctt gtcgttctcc 21240
   aaactctctt cattgcaatg cttgttctat taataagagt cacaaattgc cattccataa 21300
   aacatctatt acttcctctc gtccgttaca aattcttttc tcagatgttt ggtcctctcc 21360
   catcttttct tttgatggtt acaaatacta tttactcata gttgaccact atacaagata 21420
   tatgtggttc tttcctttga aactaaagtc acaggtcgca gccacattca ccagatttaa 21480
   ggagctcgtc gaaacacagt tccaaacaaa gatcacaaca ctctatagcg acactggcgg 21540
   tgaatacatt gcactccgac cattccttgc gcagcacggc atctctcacc tcacaacacc 21600
   accacacaca cctgaacaca atggcctatc cgagagacga catcgacaca ttgtagagac 21660
   aggtctttcg cttctcactc acgcttccat ccctactgaa tattggacat atgcctttgc 21720
   tgcagctgta tacctgatca ataggatgcc aacgaaagta ctttcaatgg acacccctta 21780
   caatcggctc tttggaactg ctcccaacta ctccaaactg aagatctttg ggtgtctctg 21840
   ctacccgtgg ctgcgaccgt acacatccaa taagttggaa ccacgctcca ctccatgtgt 21900
   ctttcttggt tattctctta ctcaaagtgc ttatttctgt tttgatccct ccacttctcg 21960
   agtctttgtc tcacgacatg taacttttgt tgaacataag ttttcctttg tttctttaag 22020
   tgccaatgtc tcttccactc cagcaacaga ggagctagcg tgggttccta cggtggaacc 22080
   tttaggtcag caacaggtac tcgtggagga gccctcaccg gaaactggac ctgcaccaac 22140
   aacggcatct ccaacaccaa cagcacctgc ttcaccaaca gcacctgctt caccaacagc 22200
   gcttgctcag tccacagctg cccctcctgc tacaagctct cagccaacac aacatgccat 22260
   gacaacacga tcacgcaaca acattgtcaa acccaaccca aagtatggct tgacaacagc 22320
   acttgccccg tacgttgagc cacatacaat cacacaagct ctggctgatg agcgctggcg 22380
   gaagtctgca actgcagagt tcaatgctca ggttgtcaac aacacatggg atctagttcc 22440
   agctgaagaa gcgacaaacc tagttggcaa caggtggatc ttccggtaca aatacaaccc 22500
   tgatgggact gagaaatcac tgaaatccag actggtagca aagggatatc atcaacgtcc 22560
   aggaatagat tatcatgaaa cattcagtcc agttatcaag tctccaacaa tccgacttct 22620
   acttgggctc gcagcaaaat atgactggcc tcttaagcaa ctagacatca acaatgcctt 22680
   cctccaaggg actctcaatg aagatgtcta catggtccaa ccagcaggct tcatcgacaa 22740
   ggacaaacca aatcatgtgt gcaaactcaa caaagccctc tatggcttga aacaggcgcc 22800
   acgtgcttgg tacacggagt taaaaacata tcttctcagc cttggattca aaaattctgt 22860
   tgcagatgca tctctgttct ttttacatga tcgagggatc gtcatcttca tgctaattta 22920
   tgtcgatgac attgtggtta ctggtaactc tctttctcgg atccgtgaca tcatcaacaa 22980
   cttgtctagg cgattctctc ttaaagacct cgatgacttg ggatacttct taggcattga 23040
   ggtcatgcgc tcgtctcagg gtattgatct ctctcagaga aagtacattg ctgacctact 23100
   tcatcggacc aacatgacac acgccaagcc agtaccaact cccatgtgcg cctccacgtc 23160
   gctctctata cgagatggta ctaccttgga taatccttca gagtacagaa atgttgtcgg 23220
   cagcctacaa tatcttcttc tcacgcgacc tgatattgca cttgctgtca acaaactctc 23280
   acaattcatg cacaagccct cagatactca ttggatggcg gctaagagag tgctacgtta 23340
   cttggctggc acgtatacct ctggcatctt cctctctcgt cagtgttctc tctccctcca 23400
   tgcctattct gatgcggatt gggctggcaa caaggatgat tatacctcca ctggtgcgta 23460
   tgtcgtcttc tttggtcaac accctatatc atggtctgct aagaaacaaa cgggaatagc 23520
   acgatcgtca actgaggctg aatatcgagc tgtctcagct gctgctgctg aggtacgttg 23580
   gctctactcg ttactgcgag aactacacat tccaatcatc tccaccccga cgatctactg 23640
   cgacaacgtt ggagctacct accttagtgc caaccctgtg tttcattcac gcatgaaaca 23700
   tctagcactt gactttcatt tcatacgtga acaagtgcag aatggagaca cgtctcatca 23760
   aaggatcagc ttgctgatgg tttaaccaaa ccgctaccaa ggaaccggtt tcaactcctc 23820
   tttaccaaga tcggcctcaa taaccgtgct ccgtcttgag gggggatgtt aggataatga 23880
   cgtatcatta tagagataat catagtttaa gataactgtt gtgttttcac tctctaactc 23940
   cagttcgccc tagctggttc tctctcaggc acggagtcca aggcagccgc acagcttgca 24000
   tctccgccag aatgaaggtc tctttccggt gcagctcctc ctgtcctaat cacgagcctc 24060
   tcttccgctg ctgatgacgt ttcgctcacc atcccgaagc tctccctctt ctcctccaat 24120
   gggttcctct gcaactgtcc cgtttggttc tcctcccctg ccgcctgaac caccagatcc 24180
   ggatcttgtc gtggtgtttc cgataaatcc tccagaccct ccaccggtcc tgctggtttg 24240
   tccctttctc cgccagttct cgccgtctta caccgctcct ctcaatcaaa aggaaatcga 24300
   atctcttatg ccatgggatc tctgcttttc aaccggttgc ttgctcaagc ctctctgccc 24360
   agacgtcgat gtgctatcca cgcagcctgc tctgctgcat ctttcgttgg ctggaatttc 24420
   tccctccgga gctctcaact ctggcctgag ttactcctcc tttcgacatt atcccgtttc 24480
   aaagccgata attattacct cttgtgtgga acatgttttg ttaaagtcag ccttaagggc 24540
   atcaacgatt tatcatcaag agtcgtgtgt ggtttcctta tctgttgcaa gattattggt 24600
   atcaatagct gagtgcaagc tgacttccct tcattactca tctcttcaaa gtcttgagga 24660
   ctgggcttgg aaggttgaaa tattggtggt gattttctct ctgttggcag ctcttaatac 24720
   cgcaatgcag cattttgaag tggagctctc tactgcttta tgcagttctc agtcaaggcc 24780
   tatcttctct tgcttcaagc tctcgcaagg tatggtgttt ttaatttgct ggagtgagtc 24840
   acgattgttc gatccttgcc tcctagtaga tcttagttat ctcaatacga accccatttt 24900
   aattcggaat gaagaggtaa tgctatcctg gattagctct gtacctcttt ttgaggatgt 24960
   tacactccct ttgagtttca ggttgaagct ctctttacct cagtatgagg aagttactcg 25020
   ttgtgatact actttgttac ctcagtgtga ggattttatt tggactgctg tctctgtgga 25080
   catggtttca cttatctcag gcgtgtttag gctatggtgg ttctcctcac agctctcagt 25140
   ctcttcaaag aggtgtttgg tcgcctttga gcttgtagct gggtctttcc caattggtta 25200
   ctttcagatt tgcccggcaa agggaatgtg gatgcaaggc cgtgtcctcc atcgcttatt 25260
   gagtagagtg ggctctggac acgtcgtcaa agcggtgatg attcacaaag cctctcaacc 25320
   agcaatatca actggactct caagacttca gattcttccg gactcaatcg tccccttctg 25380
   ttccctgcgc ttagggatgg acttgaatga gattacaggt ttcttgagct tcaaaaacct 25440
   tgttcctctc ttcactccgt tatcatgtgt ttataatcta cgcacagcat tatgtttagc 25500
   tgttgctttt gcaaagggtg ttgtacccag actttgtatt tcaagtactc tgctttgagt 25560
   tggatatgaa ataaaattgt tgacaaaaaa aaaaaagata actgttgtat ttatcttata 25620
   gataactatg agcatgtgat aagatcaact tgactctatc acatgatctc gacttgtata 25680
   aatagagagc tgcagacatc aataaactta agctttccac aatacaaatc ttatagtaag 25740
   aacaattgga ataatgtttt gtgtttgtag agagaagctc cgttccctca aatggacacc 25800
   tattgtttat tcatctgcga taaactggtc atggcgttga caagtacgtt tccttacctt 25860
   tacagttttg ttttaactta ttctatttga tgttatatat gaacatttat acatgtagta 25920
   ttgtggttgc tgctgcgact gttcaaaagg agagatcaag aagactttgt acagccatat 25980
   taaaccaagt gataagagaa ttagctgttg catttaaacc aagtgatatg aaattattct 26040
   cttcttctct cttttgttct tcacattctt cagtttccag caagtaaaaa agctcatatt 26100
   ctcacactcc tattcgtctt ctttggctca gcccttaaaa gatcgacaag aatggaggag 26160
   gtacatagtt ttttagcact aaaactatta ttcttagttt gtattgaaaa aaaaatgttg 26220
   ctggtgaagg aacaatgtca agtctttcac gtgagagaaa tcttgcaacc aaaggggggg 26280
   cataataaac tgagactata taagtttcgt ttagttatgt atttcttgag aaataatgta 26340
   attctttgtt tttttttttg ttaaagggtt ttataattca cgtttgtgtg ttgagcgtaa 26400
   ataaaaggga ccgctttata cgattcagtg atgtttttag tttaatttca gcttctttct 26460
   tttcatgaaa ttcaagctac ttcacaaatc aaagatgcat gcatatttgc gtggggaact 26520
   acatagacac ccattcaaca agaactctat ttataatcac gtcctaatga atcggtcatt 26580
   tgaacaaaaa aaaaccactt cctaatgaat cggtcatact tttattttta aaatagatac 26640
   aaagacaccc attcaacaag aactctattt ataatcacgt ccttatgaat cggtcatttg 26700
   aacaaaaaaa aatcacttcc taatgaatcg gtcatacttt tattttggag attaatgttt 26760
   cataaccaag ttgctttggt ctagtggtat aggagctcca gctggagtgc ccgcccctgg 26820
   gttcgagcct tggccactgc ggaatttaac atatgggctg cagcatccga gaccgaaaac 26880
   cgttacacgg tgagccacat ggtgacgccc tggcagcgtc catgctcact tcggtctcta 26940
   gtctggacca cctcggtgga gccaggatac tcggttagca aaaaaaaaat gtttcataaa 27000
   taaaaacaga tatcagtgta cgtagtatcc tcccttatct ttaggattcg attcccacct 27060
   tcaacattcc acttttttct catttcagtt tgttatttaa agggtttcaa aacatacaac 27120
   aattatccaa aactagattg tttcaaattt ctcaaaagat atttaattcg aagctaatta 27180
   tcacgagaac tacaaattaa ttccaaaaat gaagttaatt tcattgatgt gcatcgcctt 27240
   tgtcatactc ttgacatcat tcccggctac ggctattacg ttcaaccctg cttgcattaa 27300
   aaatcatgat acctgcggcc ccctagttgc tgtaaagggc cggcggtgga gacccgaatg 27360
   ttgtaaaatt tggagcggga atgtacttcc agaaacaaga caatgtgcat gttatgtact 27420
   gaaacattct ttatttggca atggtgttct tccccttatt ttagctaagt gtaaattagg 27480
   tggtatcgaa caattcaaat gttcggaggt ggaaacataa aatagttaac taaccagaat 27540
   cgtgaaaacc aaactggaga taataagata aagtaatcaa ttgggtattt ctttatttct 27600
   ttctaatcat tttaataatg ttactgttca ataaggagag agaaatggtt tgtttttgtt 27660
   catgctcgtg tgaattacga ttccatgttt ttttgtttcc atgtattttc ttatatattt 27720
   agtaaaaaaa tgaaaaggta taatctagtc tggattaagt accataaata aatatttgtt 27780
   cttaatctta gtgctttgga cttgtaacac caactttatg cgtaataagc atggggagaa 27840
   gaagcaaatg aagaattctt cttattcacc aagttttgta atataataat tttcattcta 27900
   ccaaaaaagc ctctctctag tattttctct aacttctttc taaaaaagct tccgaccaaa 27960
   tagatttgct catgtggcca gtgggctctt tgctgccggt cacccataat ctttttgttt 28020
   ttgtttttgc tttgtattat atttcttctc ttcgcggtgg cgataaaccg tggcaaattt 28080
   ttgttgtgta ttcaaaatct taattgaagc aaaacttgat gattgtgttg tgttattctt 28140
   cgattgagat tgatctttta tgtatgcttc tcctctttca cagttgctct ttaaatcccg 28200
   ttcgctttaa tggagttttc ttcttctcta ttggattcgc atgtactctt ttcttaaggg 28260
   agtcaatttt cactctttaa aagatgaagc cgatgaatag gctcttaggt ggaggtgatg 28320
   ttagagtaat ctcgatgaag atttagatga atcggcatcc tctcggagat ggtccgataa 28380
   agatctttaa ttttattgga ttgattgagt aatcccagag ttattaccca atgaagttga 28440
   tggtggctct cattgaagcg acagccggat gaaagatagc agatcgcagt ctcatcccat 28500
   gtcagatcca gaagtttaaa gactcatgca aaggaggatg agggcgcgtg tcacgtcttt 28560
   cccgtgcatg gaactcgaag agcctaatgg accacgaatt aggcctaata aaatttattt 28620
   tatgatgatg agtttcaagc ttgttgcttc tcttgtaatg ttcattggta ggcatggaca 28680
   ttcggggtcc caatcgggtt tcgattttat ccattcgggt ttcggttttt cgggtttatc 28740
   aaaatcaacc ccattcgggt tatataaaag ttcggttcgt gaccggttcg ggttctatcg 28800
   ggttcgggtc ggggttagta aatcttcaaa gaaccggtat aacccattgt actttcgggt 28860
   tcgggtccca atcggttctt cagtttaaaa atacatgatt tgtacctatt ttgtaactaa 28920
   aacataaata aaatcggttc ttcggattta aaatacatga tttgtacata ttttaatagc 28980
   caaaacataa gtaaaatcga ttcaaaaata agaaaaaaca tcaaacgtga tcattcaaaa 29040
   tcaaacgaaa gataaacata gttagtgata taaaaaaaac cttataaatg aaatcataaa 29100
   acaaaatata agttctcatg aaatgagaaa cattattcaa taaaaacaaa accaaaatct 29160
   aaaaactcca ggcatcaacc gccacattcc accatcaacc ttcatgtaac agataattat 29220
   tttagaagtt caataatatc ttaaagtatt ttggatacat attaagaatt aagatcatat 29280
   ttggtagaag ttctttttgt gattttaaat gtttcgggtt ctatcggata tccatttagg 29340
   tccgggttcg gttcggataa tacccataac ccaaaatacc aaaaaacagg atccattcgg 29400
   tatttatgtc gggttcggat cggttcggat tcatttttat cggatcgggt tcggttcgga 29460
   ttttcgggtt cggtttattt gcccagccct attcattggg cctttctaaa ggaaagaaac 29520
   agttgacaaa aaaaaaagaa aaaaattcta cctacttagg cccttcttaa ttcctcaaat 29580
   ccctcttttt ccaatattac aatttcttcc tcttgactat caacaacaga cctcaatggg 29640
   ttcatatcct atggcctctt gggagtcttt tttggatagt catccatact gtgaaggagc 29700
   attggggtgt gataaggaaa ccaaatccct ttgtaccagt caactgctga atggttagct 29760
   tccatgtgag ctaatcatta ctcatcctcg caagttttta gatgactgct tctttttatc 29820
   aggtggttga gtataatcta ttttgaatta gcctgttgga ctagtttctc ttattcaaga 29880
   aatccacttg ctcagtgaca caagagcagc taagcgggtc cgtaactttg ttggtaatat 29940
   aaagggaaaa ctcagatctt ctgttttgat ctttgcttgt gcgcgtctca gacatgtatt 30000
   gcacatcttt gtttcttaca agcgtcaaat cgtttataca tttttagtga aacaaaggtg 30060
   agcaggtagc caggtagtac ctttccttgc aactcacgca tgaatataat aaatcaacta 30120
   tttcattgtt gatgactgga tgattcacaa tcaagttgct gggtatagta tttacactaa 30180
   attttttttt cttttgttta ctcgtgagat catcaaagat gttttagata tatgaaaagc 30240
   acatttcata gtctatattc atcttccatg tatgtttctt tgatgattta aaaaaaacag 30300
   aagtacatca acacttctta tctttgtgtt ttcttaatct caagttggta aatagaaaaa 30360
   aatgatgaaa agaagaagaa aaacaaatta ctacgaggct ctcagtaaag ctgttctttc 30420
   ttttcatgcc accatctcca cctgtaaaca caaaattgct agaaacatta gtgaactttt 30480
   atataatttt atataatata aaaaatatat aaaaaattat tttaaaaaat gaattttaat 30540
   ataattttgt gccaaatctt gcattctgag atcatcaagt caatgtagta tacgtaccag 30600
   taatattccc aacgaggacg gactttggtc atgcggttat catcagatgg gtgataagga 30660
   tgagcagcgt ggtgagtgtt gtggaaagcg cagcaacctg cagcgtagac ggatgttgac 30720
   aaccgataac tttctccagt caagacggat ctcttcaaga acaccagctt tacaagcatc 30780
   acactcgtag cataacgttt caataccatt gttccatcta tagcaatctt ctcctccgac 30840
   tattactcca gtctcgtacg tgcacgccgt tggtggctta caacatcccg actacaatca 30900
   aagacaggtt agttactaaa caaactgatg aagatgctag ttcatatcat aggacacaag 30960
   cggagtttat aaccaaaccg gaaaaaaaaa caaaatccga tatgaacagt tatacaaaaa 31020
   tacctgaacg gacctaaaat cattagatat ttttggattc cgaaataata tctgaaatta 31080
   gctaaatttg ttaaactttt acatatttaa ggtaatttag atattatcga aagataagga 31140
   tgatgatacc tgaacagaag tcatatccct ttggaaataa tcaagtgtgg tccaagattc 31200
   aatcttagaa caagtctttg aagtcaagat acagcttcta atggagttcc aatactcttg 31260
   atctctaact cttgatctca gccacggatg ataatctcca agtctatact ctttgtaaac 31320
   ccacaccacc tccttggcta gtcaccacaa gaccaaagag cgttagaccc atgagagtcg 31380
   ctatgaggaa gatcatgaca actaagtaca cccacagagc ccatgccacg ttgaaacaag 31440
   ctcctatgaa accagcaagg gatactaaca gtatgatgaa tcctatcacg agaagcggtg 31500
   tctggaggaa gttctcgcaa gttttactgc ttcttgcctt ccatagagca gttcctttga 31560
   ttggtattga agctagtaag ctgagaaggt ttatgacccc aatcactgtg ttgctgaacc 31620
   tgtacatagt aggattgtta tcaagaagaa accagagaac tttgcagtgt ttctcttctc 31680
   taatcagatt ctctcttatc aattaggaat cccctggact atatttgcga agtgattttg 31740
   ccacaagtca tttctacaat caatttcaca aaacaagtat gacattgcta ctgaaattga 31800
   tgatatgtct tatagcttaa tatgacatgg acaattgcat ttaatgttaa tttatatttt 31860
   tggtaaactt tttaaaatat ggtaataact catataatac atttaatgtc aatttatatt 31920
   ttagaaattt ttttagaata tgaaaataac tcataaatca tcattaaaat aaatatattc 31980
   aaatatgaca ttataaattt cgaaatataa tataattata tatttttaaa ttatataatt 32040
   ttattactaa aattttcaaa aatgtatata atttttttag aaaattataa aaatttaatc 32100
   gtaaaatcat tattttctta tatatctaca aattttataa atattgttta attttaattg 32160
   ttggtgatta tgcaactttt acaaatgtat ttaatatatt taattaaaat aaatagatag 32220
   aaaaatctat ctaagattat aatttcaaat ataaacatgc atatttttaa atataatttt 32280
   tatgtttaat taaatcaaat ttatattaaa atattgatac gaaaaagaaa atttacaata 32340
   ttaaaaaaac ttattttaaa atataattta tatttatctg ttaaaaaata ttttaatttt 32400
   ttttactgca catggtgcag gaagacacct agttttaatt aagggaaaaa gttaagagtt 32460
   aattgtttaa tgattcatgc tcatattctc ttgtcgtttc ttgatttatt tttatttttg 32520
   tcaagaggtc gtttcttgat ttgaatcaac taaacaacaa cattaaaatt gtattttttt 32580
   tcaaataaaa gcgtcttttt ggacaattgt ttcttgttta atagtatttt atacgcttat 32640
   gtcgtttaaa ccagaccaca agtagtgcct tgaataaaat atgtacataa aattaaacta 32700
   tattgtatat agaatcagat aaattcacaa tgatcaatga aaggtaagca aagaataata 32760
   tagaggacgg atggtgaatt ttctttttaa gatgctttta ctggccccat aacttggcat 32820
   attaggttct gtaggtagag cacaatatga tgtttgattc ccacattcac tattttttaa 32880
   gcaagaaaaa tgctaaaaca tacttaattt aagccaaaat gtcataaaac aacaaaataa 32940
   gacaataata acattactgc aacaaataca tagtttttaa taccgaaagc tagatcgaat 33000
   caaaataaaa aaaacatgag tggcaactct aaggttttgg cgaatggtga gaatagggtt 33060
   caaaagtcgg gagatcagga tgtgaccatg atggatgtgg gggagagagc gagaccaccc 33120
   ggagaccctc cggacatagg gctttcatgg gtagccaagg tagcgggtac gagtgaaggg 33180
   ggtatgtcgg taccagagag cttgattgat gatgctttcg tgtcggaaag gctccgagtc 33240
   gagttcccga atggagagga tggtgaaccg tgtatcacga tcgagacaga ggttttggaa 33300
   gcgatgaacg ggatgtggaa gcagtgcatg atcgttaggg tgttgggaag gagtgttgcg 33360
   atctctgcct tgagcaagaa gttacgagag ttatgggatc cgaagggagc aatgtatgtg 33420
   atggacctgc ccaggcagtt tttcatggtc tgtttcgaga aagaggatga atacttaggg 33480
   gcactgacag gaggcccatg gagagtcttt ggcagttacc tcatggtgag ggcttggtcg 33540
   tcggagtttg atcctttaag agatgacatt gtcacaacac cggtttggtg ggttagatta 33600
   acaaatatac cagtgaatct ctatcatcga tcgatcctta tgggaatcgc caagggattg 33660
   gggcagccag ttcgagtaga catgaccaca ctgaagtttg aaagagcgag gtttgcaaga 33720
   atctgtgtgg aggttaatct agcaaagccg ttgaaaggga cagtgctaat taatggtgag 33780
   agatacttcg tagcttatga agggttatct gaaatctgtt caaggtgtag aatttacggg 33840
   catttggttc atggatgccc aaaaacgatt gcggagaagg tggctaactt ggcaatacag 33900
   acggagacgc cgacacttac caatccagtg cctaaacaag tcccaccaag gcaggaagat 33960
   ggttttactc aagcaaaggg atcaagaaga ggaacgcaag ccccgcgatc ggtgaatgtt 34020
   gtgaccggag aatcaaatgg ggtgactaat cggaagctcc aagagattct taattttaaa 34080
   gaggctaatg agattgcact atcgaataag tatggaagtc tggagatggc tacgaatgtg 34140
   gatggatcaa aggaggatgt ggttgctggt gaagagaata aggagaatca tgatatgaat 34200
   atccagaaca gtaagggaaa gggtctcccg caaggaaaag aagctttaat ctttagtggg 34260
   aaaacaagta cgtcgactag cttgaaaggg atgactaagg agaagtgggc tgcgaacaag 34320
   agaataatgg agggaggtag aggaaagccc aaaagggtaa ataacaggcc cattaggggc 34380
   ttggtgttta gtccgacgaa gggtgaaatc agtccggtaa aagactgaga gtggataaca 34440
   ttgatgcagg gagatccagg ggtgtgttta gtgatggtgt ggaggaagtc agaagtatgt 34500
   ctaaacctct gctactacga gatgaagcgt tggacaacct gatggagagt actatcagcg 34560
   aggtggatca gagagtggct gatatacaga cgagctcaca aggagatggt aggatcgtgc 34620
   cccttgcatg acagcgtgcc ccggtaattc gtatagacct taacagatta attatgatga 34680
   attgcttatt ctggaattgc cggggggcaa aaaaactcaa tttcagacgc tctattcgat 34740
   acatattgaa gaagtttaat actgatgttc ttgcactttt tgagactcat gcgggtggag 34800
   aaaaaacgag aaggatttgc cagaatttgg ggtttgagta ctcctttcgg gtagatgccg 34860
   ttggtcaaag tggtggaatt tggctcttgt ggagagacca agcgggcgtg attacggttt 34920
   tggaatcgtc agagcagttt gttcatgcta gggtggttaa tgggacagag actatccatc 34980
   tcatcgccgt atacgcagca cctacagtta gtcgtagaag tagactttgg gggcagctaa 35040
   aaaggatatt ggagtatata gatgaaccgg tgctggttgg tggagacttt aatactatct 35100
   tgaggttgga tgagaggaca tgggggaatg ggaggctttc atcagactct ctggactttg 35160
   gggaatggat taatgagatt gccttggtgg atatgggttt taaaggcaat acatttacat 35220
   ggaaacgagg aaaagagacg cggaactttg tggcgaagca tttggacagg gttttatgta 35280
   atgcacaggc gcgggttaga tggcaggagg cggtggtgtc ccaccttccg ttcctagcgt 35340
   ctgatcatgc accactctat atgcaactaa aacctgagca gagaagtaac ccaaggagga 35400
   gaccgtttag atttgaggca gcgtggctga agcatgaagg atttaaagag ctgttattgg 35460
   cttcctggaa tggtcaaatg cgtactcctg atgcattggt atctctacaa ctgaaactta 35520
   aaaaatggaa taaagagatt tttggaaatg tgattcaacg taaagaaaaa cttctcggtg 35580
   agatcaaggg tattcaggag cagttggaaa ggaatccgaa tgatgatcta ctgtcgaggg 35640
   agggggtgct tcagaaggag cttgatgtgg tcctagaaca agaggaagta ttatggtatc 35700
   agaagtctcg ggaaaaatgg atagttcttg gagacaggaa tacgaactat tatcatacga 35760
   gtactatagt gaggcggaag agaaatagga ttgagatgct gaaagacgac gatggccgtt 35820
   ggatagatca gtcggaagag ctggagaagc tagcaataaa ctattacaag agactgtatt 35880
   caaccgagga cctcaaccta gacacggaaa agctcccccc gcaaggcttt accgagctgg 35940
   agattttgaa tgaacctttc tcgaaactag atatcgagac ttctgttcga tctatgtgga 36000
   aatataagtc ccctgggcca gatggctttc aacctgtttt ctatcaagat tcatgggatg 36060
   tggtggggga gtcggtaact aggttcgggt tggagttctt tgaatcggga gttctaccgg 36120
   aaggcacgaa tgatgcaatg ttagtcctca taccgaaggt tcttaaacct gaaagaataa 36180
   tgcagttccg gcctataagt ctatgtaatg ttcttttcaa gatcataaca aaggccatgg 36240
   tgttgagact gaagaaacta atgctgaagc tcataggccc agcgcaagca agttttatcc 36300
   ttggtcgact caattctgat aatattgtta ttgttcaaga agcggttcac tcaatgagaa 36360
   ggaagaaagg acgaagagga cggatgctcc ttaaactaga cctcgagaaa gcttatgata 36420
   gaatcaggtg ggacttctta gaggatacac tctatgcagc aaagctacca cggagttgga 36480
   ttaaatggat tatggaatgt gtcacaaatc cgggaatgag tctattggaa tggagagaga 36540
   acataggcgt ttacgcctca acgtggactt cgacagggtg atcctctgtc cccgtaccta 36600
   tttgtgctat gcatggagag actatgtcac caaattgaat tttcggtggc aaacaaggag 36660
   tggaagccga tcagattatc tagaggtgga ccggctctat cacatgtttg tttcatggat 36720
   gatctaattt tgtttgttga ggcctcacta tcgcaaattc gtgtaatacg cagggtactt 36780
   gagtggtttt gtggagcttc tcggcagaaa gttaatctgg agaaatctgt tatcttcttc 36840
   tctgagaatg ttcatcggga cctagtgaac ttaataagca atgagagtgg cattaaagga 36900
   actaaggagt tgggaaaata cctgggtatg ccgatcctgc aaaagaggat taataaggag 36960
   acttttggag aagtgattga gaaggtttcc tcaaaacttg ctggatggaa gaagagattc 37020
   ttgagtttgg cgggaagaat cactctcacc aaatctgttc tctcatctat cccagtccac 37080
   acgatgagta ctatagctct gccggcgtca actctaaacc aactggataa gattgctcga 37140
   gcttttatct ggggcagtag cgaaggtaac agaaagcagc atttggtttc ttgggataaa 37200
   atttgcaaac ctaaaagaga aggggggctt ggtataaggt tggaaaagga aatgaatgtt 37260
   gctttattag caaaacttag atggagattg ctgaatacat atgatactct atgggtcaag 37320
   gtgctgcgga aaaagttccg agtggggagc tatatgaccc aacatggttg atagtccagg 37380
   ggacctggtc accgacttgg agaagcctag tggtgggtat cagagaagtt gttattctgg 37440
   gaacgtgttg gatcttgggg gacggccgcc gggttcgttt ctggaaagac aattggttgt 37500
   tgaacgagcc cctatacgaa tcaagtatgg tgcatatccc agagccgatc ttagaagcaa 37560
   gggcccgaga cttatggcag aatggaacca gttggctact tcaagctatt gagccgtata 37620
   tgtcagtata gaatcagtta agactagctg aagtagtgat tgatgatgtt actggagtcc 37680
   gagatagaat gtcgtgggaa gagagcaaag atggattgtt ttctgttaaa tatgcgtatg 37740
   ccttactgac tagagatgaa gtactgagac caaacatgga atccctatac agcctggtgt 37800
   ggtgtctagt tgctccagaa cgagtccgag tcttcttatg gttggtaaca catcaggtta 37860
   tcatgacgaa catggaacgg aagcgtagac atttgagtga taatgttgtg tgcccgttgt 37920
   gtagagatgg agacgaaacc attctccatg ttcttcgaga ctgtcaggca gcagttggaa 37980
   tatgggtgaa aatcatgatt ccgagtagac aacagcattt ctttagctta cctctattgc 38040
   aatggctcta cgagaatctg gggagggaca agccgggtaa tggagaccaa tggccaacac 38100
   tttttgcctt cactgtgtgg tggtgctgga aatggtgatg tggctatgta tttggtgaaa 38160
   caaggacatg tcgagacaga gtgcagtttg ttaaagaaaa gtccagagaa gtgttggaag 38220
   caaataagca tttgcgagat cgttcttgtg ctaggggccg ggtggagaag caaatcgcat 38280
   ggcagcgacc cgttaatggc tggcttaaat tgaacattga tggagcatct aggggtaacc 38340
   ctggactggc cacagcggga ggagctgtgc gagacgaatt tggaatgtgg aaaggaggtt 38400
   ttgcgattaa tataggtatt tgtttggctc cgttggcaga actgtggggt gtttactatg 38460
   gactgtgcat agcatgagat tgtggtattc ggaggctaga ggtggaagtt gattcagaga 38520
   gtgtggcggg ttttcttcag acagggattc atgattctca tcccatatcc ttcctagtac 38580
   gtttgtgcta tgacttcatt tcaagagact ggatagtcaa aatttctcac gtgtataggg 38640
   aggctaattg tctagcagat ggattagcta actatgcgtt ttctttaccg tttggtttac 38700
   attattttga gtcggctcca gagcatgttg cttcagtttt gttagaagat tgtaacggag 38760
   tgtccagaac tcgacaaatt tgcctgtagt tgttttgttt ttgatttgaa taaaaagtag 38820
   gcctgcagcc taccaaaaaa atacatagtt tctaattaga acaaagacta aaccagacca 38880
   agagaaaagt cgacaacatc ttttaactct gtccttccac tatcatcatc atcatcatca 38940
   tcctcataac ttattgttgt accagaacac acctttcttc tcaccttgcc tatccggttc 39000
   aacatagata cactccttcg cctccctcca catcgcctta accgccggcg ttccatcaaa 39060
   ctggtaatac tctccaaagt atcggcttta tcgccttggt cgcttccatc gcgttataat 39120
   gcggcatcgt cgagaacaga tgatgcgcca cgtgcgtgtc cgtgatgtta tggaacacct 39180
   tgttcaagat tccatagtct ctatccacag tagccaaagc tcctctcaac caatcccact 39240
   cggaagaatc atagtgaggc agcgaagggt gcgtgtgctg caagtaagtg atcaagacga 39300
   ggaaacagtt gacaattaga agcggaactc cgtagacaca gaccacagag gctactcctc 39360
   gcgaagcagc gtaacggtag agaccgtaac atacggagag gacgccagcg tcagagatgt 39420
   atatctggag acgctcgcgg tcgttgtaga tgggagcgtt cgggtggaaa tggcaagcga 39480
   aaccgtcgct gtaaggtctt cccgagacgt tgaaggctaa gtacaacggc cagccgagct 39540
   tgaactggac ggttagcatc accgtgcgtc ctagcgggtt gttgaggtac ttgccgtacc 39600
   acttgatgtc ggatttcttc ttggggacga acacttcgtc cctatcgagg gatccggtgt 39660
   tggaatggtg gcgtcatgga tgtacttcca ggagaagtac gggacgagga ggaaggagtg 39720
   gaagacgagg ccggcggcgt cgtccagcca ctggtggtcg ctgaaggcgt ggtggccgca 39780
   ctcgtgggct atgacccaga ggcccgttag gacgcagcct tggcaggccc agtagagggg 39840
   tcaggcgagg taagggagag ggaagtaggc tgtggagagg tggtagaggg aggagccgag 39900
   gaggagatga tgatgtcgaa gaggagaagg agcgtgggat ggagcgtttg aagcagtgag 39960
   gtgggattgc tttcttgagg tctccgagag tgaatggtgg tgtctcgcag gggacgcgtt 40020
   tgagggtgtt ggttccgggg gagctggagg gaggagagac ttgcattctt ccacctgcgc 40080
   ccatgtttgt ttctgtagag aaaaccaaaa aaaaagaaaa gaaagtaata agttagttag 40140
   taggaagaac ggaaggggta aggttttttt taac 40174
<210> 3
   <211> 28527
   <212> DNA
   <213> Brassica napus
<400> 3
   acacaagtat ttgaaatatt cgaaatctta gatttatcta gtaaaacatt acatacacaa 60
   ttccaaatta tcttaaagta cttaaaacca aaaaactggt atgaaaataa ttcaaattta 120
   taaaatttat aaaatattca aaatatataa atcatgaaaa ctgaataaaa attgggaaat 180
   tctcctaaat tgatcatttt caagttttgg tcacaaaaat agaccacagg gaggaaaatg 240
   accaaaatgt tttatttaat aggtaaaaag accttaatac actggatata taaaaaataa 300
   aaaaagtaaa aaataaaaaa atcataaaaa aatttatagt tttagattat atgttttcaa 360
   attcgaaatt tttataaaaa attatttttt ttgaattttt tttcgaaatt ttcttttttt 420
   tcaaatttta tttttgtaat tcgaaaatat tttttgaaac tattttaaaa atttttattt 480
   ttaaattttt aacatttatt ttttatttta taaaatttta aacctcaatc ccaaatctct 540
   atcccttaac tctaaaccct aaagtttgaa ttagttaacc atacgtgtat aaatgtatat 600
   ttacctcttt catgaaacag tttggtcatt ttgatcatta gagtctatat ttgtgacaaa 660
   ataaaattag tgctatcctc tggtatttct cataaaaatt taataaattt atttaattta 720
   attttttaat attagtttga gtttagaatc ttatccaaaa gccgaattat catattattt 780
   aaatatttcg taaaatcaga tgaatcaact ttttttgtat ggattttggg ttctaaatat 840
   tgtcgcccaa gttgacaaaa aaaaaagtat tgtcgcccaa tccacatgac acaatagtat 900
   tcttttaatc aatgaaccct aaatcccgat tgaaatccta ataattgaat tcaacccaat 960
   agaacaatag taggcctgcg atttcggttt tccgaaaccg aaccgaacca atttttcggt 1020
   tttcggttaa acggtttttt ttggaaaaaa ctccgaagaa aaccgaattt gaattcggtt 1080
   cagtttttgg ttatttccga tttttgattt tattccaaaa aaatcgaaat caaacatttt 1140
   caataaaaaa atcggatcac acgattaatt tcgggtattt acggttactg ttgggtttcg 1200
   gttacctaag gttgtttcgg ttatgcctct attttcacct gccaaatcaa ataatcatgc 1260
   aatcaacaac ggaaaaataa tctccaacat caaaaccaat ttataatcga ttcaccaagt 1320
   taaaacattt aaaagagatc actctcaaac atgaaaatcc aaaaaaagat aacaatctga 1380
   atctatattt cttttgcaaa tatcagactt tgaaagatac attcatacat agattcatga 1440
   agttcaaaaa gataagttgt ccaaaagaga gtgataggct aagttggttt tggcttcctc 1500
   gacattccaa cctacaagtt tatgaagtaa gaaaatcagt aaaaacaggg agaagaacaa 1560
   actacaagaa actatacaaa taatagagca acaaattacc tttaatgatc ggattcactc 1620
   ggaatttgca aagactctac ataatgtcaa aggcagttag gaatatatga ctgtggacat 1680
   ataaaaattg aaggaaaata gttaaatgat tacctaatga ctcatgaaaa tcgagttctt 1740
   caaacatctg aaccaagctt gcaaccttct cagcctggat gttgtttcga agccactgtt 1800
   gtaggcagac aagagcttca atcatgtatg gagtcagaca actacgaaac ggatctaaaa 1860
   tcctaccact tgtactgaat gctgactctg aagcaacaaa ggagacttga acagctagca 1920
   catcttctgc tagttcagac aagattggaa acttacaaga gttcttcctc caccaagata 1980
   acacattgta atccaagccg aagttgtttg agcctcgtgg tactggtttt tccatcaagt 2040
   agatgtcaag ttcactgctt ccttcttcat ttgctgcctc tgaaaccatc tttgaataca 2100
   aagaatctct cctctcaaag tcttcatcat cgtcatcaga gatatcaaac atggttccaa 2160
   cttcaccttc cccattatca ataacgttgc tgagactatc accttgagat gaagagccga 2220
   gcttgctcac atactcctca tacaacgatt tcatcagtgc cctgattgat gttctgagat 2280
   gaccactctc cagactatcc ttcccgtaca gcttatcaaa acatatagaa gcaaattgca 2340
   tcttgttcct tggatcgaac acacttgcaa tgatgaccag tgggttcata ttgataagcc 2400
   catcccagta tttctcaaat ttgttcctca tctcctttgc ttgaatctgt agaatcccat 2460
   ctttgctatt gctcaatgca atcaagttcc tctcgataat cacaatctca ttctaacaaa 2520
   tggtggacgt gactgtcttc gatgctgaga acgctaaagt gcagccaaag aagattttca 2580
   gaaacttcac taacctctgc acctcttccc acccttccgt agtaggaggt ccaactctct 2640
   tctgtccatt ctcctcttct tccagaaaat aatcattata caacatgtct tcggctttga 2700
   gcttctcaaa agcaacctta aacttcaaag cggcagacaa catgagatat gtggagttcc 2760
   aacgagttat gcagtccaaa gacaagctcc ctctactaac cattcttgtc agcaccctta 2820
   actgaaacga ctgcaatctt gtacccgatg atctaacata cttcacagca tttctaatag 2880
   ccattacact atctttgacc tcagataaac catccctaac tatcaagttg agaatatggg 2940
   cacagcaacg catatgcaac aatgcaccat ccttaactaa tgcatttggt cctatctccc 3000
   tacatgtttc tgtaaacagc cgtagggcct tgtcattcgc cttagcattg tctactgtaa 3060
   ccgtaaagac cttctatatt ccccaatcag agagacatag actcaaatgc tcagaaatat 3120
   tgtcaccttt atgatccgtg ataggtttga agctgatgat tctcttctgc atattccagt 3180
   ttcgatcaat ccaatgagct gtgacgacca tgtagctgta ggatgttgta ggagcaaccc 3240
   aaatattggt ggtaagagac actctctgct tctcagaatt aaacaactgc tttaagctag 3300
   ctttctccct tagaaacatt ccaaatatgt cttttgttgt tgttctcctg cagtgaactg 3360
   tgtacatagg caagacgttg tgacagaacc tcctaaaccc ttcagattcg acaaaagaaa 3420
   atggctcctc attcagcact atcatctcat tcactgatct cctaaacaag gcagcatcat 3480
   acttaatggt tgtcaataca ccactactat ctccacctag cacctgctgt tttccaaatt 3540
   ctttgtagag ctggtacatc ttacatcgag caatgtgatt cttcatcgca ctcgtcccac 3600
   tcttcttcga gtcacatcca atcccttggg cacaataatg gcagttgctg atgcttggat 3660
   catcttctct ttgaatgaaa tgttgccaaa cttctgctct gtgagcttgc tttttctttg 3720
   caacaggagg catatcagtg cttgatctct cagaagcaga tcttttcttt cccccacctt 3780
   gatctatata tgcttcaacg tcttctgcga ggaagttacc attatcttca caagctgaca 3840
   tctacaaata caatcagaaa ataaacactt aagatcagac aaatatatat acaaaacgaa 3900
   ccaaatgaac acaaaatcat acgctatttc agaaaactca ttcgctaaac tctgttcaaa 3960
   atcaacagaa atcgttccta accaaagctg ataaattgat aaactataac tgactcatta 4020
   agcgacacac acacaaaccc taacaattaa aatcccaatt cgattatcac acaaagtcac 4080
   aaaccctaaa ttcgatttac acataaactt agaaagaaga gaagacatat acgaaaacta 4140
   accttctacc ttgtgctcca aattatgtaa ggaagaagag aaattggtct ggtctgagaa 4200
   tcagacaagg attgatcgga gatcgtattt gagaaagaga gattaagaga ggcgattagg 4260
   gtaaaccgtt tacgacttta ggtttgtcgg cacttaggtt aaggatgact taggttaggc 4320
   atacatagtt aggttaaaat ttcggttatc cgttcggtta tcacgaaccg aaccgaggaa 4380
   aaaaccgata tatttgaaaa cttgtaccga tcgatttctc tgttatttct gaatcataac 4440
   cgatatccga cgtttttggt tcggttcgga tcggaccgat cggttcggtt cggaatacca 4500
   ggcctaaaaa atagtgttat catagttaca actaaaaaga aagttaacag acgccgtaaa 4560
   aaaagaaaaa aagaaaaaaa agaaaaaaaa gttaacagac aagtctaaaa catagaaaag 4620
   gaaatacgag aaggagacta gacgcttcct ctactaaagc gttttaagag catgtacaac 4680
   ggtataaatc aggaaatcct taggaaagat ccttaatgct ttttgattaa agaaatatta 4740
   aaagaggagg gcaaagttaa ggatgatcct taattaagga ttaaaatacc gaagtcctta 4800
   tcgtgcttgc acactttgat tggtccgttt tgtgttgtct ctcgttaggg gagagaaaaa 4860
   acacgggacc caacctcatt gtatctcgaa cccgaaaaaa aagcagagga gaaggagagg 4920
   cgacagagag gcgatttgcg atcccgactt gtgaaggtaa aatccgatct taatcttttt 4980
   tttccttctc gagtttatgc atgttgattt catcacgttt tagggttcga ttgaggtgtt 5040
   ttataaaatc tagggtttga aatcagattg gggattttta tcgatttagg gttttcgtct 5100
   tggtttgatt tttttttgca ttttcgcatc ggttctttgt tgttatcgtc tggattagag 5160
   gttctggatt gagttcacaa tcgatttagg gttttggttc ttgatttcaa ttttttttta 5220
   ataagaatcg tctcggtttg tttttttttc atttttgctt tggttgtttg ttgttaacgt 5280
   ctcgattaga ggtttaatca attgagttca caatcgatta aggttttggt tcttgatttg 5340
   aaattttttt ttaataagaa tcgtctcggt ttttttttca tttttgcttc ggttgtttgt 5400
   tgtgatcgtc tcgattagag gtttaatcga ttgagttcac aatcgattaa ggttttggtt 5460
   cttgatttga attttttttt ttgcaggttc tgaaatggat gaagaacagc gagatatgaa 5520
   agcacacaaa gcatactacc aaagggttga tttcgtatca aattcgctgc aggggattcc 5580
   ccaactgtgc ccctgtggat caatcacgaa ggaaattgta gatgaagagg atacacatga 5640
   ctacctccct gggaaaacat acttcatctg caaagacttc gaggtatgaa ctttgtttct 5700
   atctctttca tttattacgg gttatatgtt tgctatttga caattttttt ccctttggca 5760
   gaatgacggt ctgcattaca ggaaaccatg ggttattggt gtgcaagaag aggttgagag 5820
   gctcaaactg aaagttctcc gccatgagaa ccttcttaga gagtgagagg cacttaaggt 5880
   gagtttattt tttttcggta tcataaaaat tgtgtttcta ctatctttct aaccaatgtg 5940
   ttttggtttg tcttatgtct gttaaggaac atgttaaaat gttggtcaag cgggtttctg 6000
   aacttgaggt tagtcttaaa actcaaccgc aatagtttcc tggttagaga acatagctgt 6060
   tagaattgaa acggatagga ctgtcggtaa ctgtgtagtt aggaaaaaat tacttcatta 6120
   aatagaccct aactgcattt tggatagtaa aacttcatta aattgtttgt agctgttctg 6180
   ataagacagt tttctactag gattcaatgt agaatctctt atttaatcct agtagaatac 6240
   tagtagagaa tcacacttca tcttaaatgg caagctctag ttttgttaat ctcttagcga 6300
   gccaagggtc agttgacctt aactctgcag agactccatt gtttagtacc caaactccta 6360
   cccaaactcc gcaagaacca agtgtcaaag agaggagaaa gtggtctgtg aaggaggatt 6420
   taatcctggt gggtgtttgg ctcaacacta gcaaagattc aatcgttggt aacgaacaaa 6480
   aaggtgttgc cttctggaag aggattgtag agtactacaa ctccagtcct cttctcgttg 6540
   ggacagtgcc aagagaacta gggcaatgca agcaaagatg ggctaggatc aatgatttgg 6600
   tctgcaagtt tgctggctgc tacgacacga cattgaggga gcacagaagt ggtcaaaatg 6660
   acaacgatgt gatgaaggct gccttggata tcttcaacag tgaccagaac atgaagttca 6720
   acttggaaca tgcgtggagg gagcttaggc atgatgtgaa atggtgctcc acctatcagg 6780
   agaaggacaa ggataagcgc aaaaccgcgg atacttcggt tgcagaacca gaagatagac 6840
   caatatgggt taaggctgcg ggtaagagga agaaaacagg aaaagatgaa gaattaacca 6900
   agcttgaagg gctggtggac attaaaaagc aaatctcaag gcaaagtttg cttgaaagtt 6960
   tgcttgcaaa gcctgagcca ctgtctgata tggaattagc actgaaaaca aaactgttgt 7020
   ctgaaatgtt gtcttgatgg tttgttagct ttagattaac ttgtttgctt tattactttg 7080
   ctttgtttac tttactgact cgtttggttg ttgctttgtt tcaggatcag taaagacatg 7140
   tgcaagatga agacaaaaga tggtgtccct tcctttttca gtcactggtc atttcgtttg 7200
   ctttttctgt atgttggatg tgtcacgggt tgtatgtgtc acgggttgct ttgtttttgt 7260
   tgttgctttc tattttgaac ttctatttat aagtcagtgt ctgttgcttt gtaatttgaa 7320
   cttcccttgt atcatttcat caatgaaatc tctttcctct tattccaagc tttgaagcaa 7380
   acttcccttg tatcatctcc aagcttttct cctcttctac ttccataaat gtcacgaaca 7440
   taaaaccaac aagtgaagaa agttaaacca ttctccaatc tttcctcctc ttctacttct 7500
   ctaaattcac gacaataaaa ccaacaattc aagacacttc aaccattctc caagctctcc 7560
   tctacttcaa gtaatttttt tattctaaaa ttagtaattt ttttatattc taaaacgtga 7620
   ttagtaattt tttttattct aaaattattc taaaacctga ttagtatttt ttttttggca 7680
   gtatgggaga tgaagtcgat cgaagattga atgcggcatt ggataaggct gtcgatgaat 7740
   attttgaaga cacatacaac aacatcgtca agaaccaaac aaagaaacaa accaaacgtg 7800
   catatgtcga acgaaacagc gaagagggcc acagaagtct atggaattac tacttcagtg 7860
   aaaatcctac atttctgcct catttattca gacgacgttt ccgcatgaac aaggcggtgt 7920
   tcatgcgtat cgtcgatcgc ctctcaaaaa attttccctc ctttcaacaa agaaaagatg 7980
   caactgggag gttaggtcta tctccactac aaaagtgtac ggcggctctt cgtatgcttg 8040
   cttatggttg tgctgttgac gccgttgacg agtatctccg acttggagaa agcacaacac 8100
   tttcatgttt aaccaatttc acagaaggtg taatacagtt atttggagat gagtatctac 8160
   gaaggcccac tctagaggat cttcaacgac tactcgatat tggagagata cgcggctttc 8220
   ctgggatgat aggaagcatc gactgtatgc attgggagtg gaaaaattgc ccaaccgcct 8280
   ggaaaggaca gtacacacgt ggatcaggaa agccaaccat tgtcttggag gctgtagctt 8340
   cacaagatct ttggatatga cacgtttttt ttgtcctcca ggtaccttaa acgatattaa 8400
   cgtcctcgat cggtctcctg tttttgatga cattttacaa ggtcgagctc caagggtaca 8460
   atatgtggtc aacgggcacc agtatgattt ggtgtactac ctcacagacg acatatatcc 8520
   aaaatggtaa acatttatcc aatctatctc aaaccctcaa ggtcctgaag cagaattatt 8580
   tgctaaagtt caagaagaaa tccgaaaaaa tgtggagcgt gcttttggag ttttgcaaac 8640
   tcgatttgca atagtgaaaa acccggctat tttgtgggac aagagacaaa tagggatgat 8700
   tatgcgaaca tgtatcatac tgcacaatat gatagtagaa aatgaacgca atggatacac 8760
   tcagtatgat acatcagagt ttgaagaagg agagtcgagt agaagttcac aggtggatat 8820
   gtcatattat ctgaagcctt caaatctcct tactatgctt gacatacgaa gtcgtgtgcg 8880
   tgacccgcac atacatagac aattgaaata tgatttgatt caaaatattt gtaacaagtt 8940
   tggtaatgat gaagatgttt aattattgta tgtttacatt ttgtttttca ataaatgaaa 9000
   attttaaatt tcaaatttta aaattttaaa tttttaagat taaaaaaaaa aaaatcaaag 9060
   tactccttgt tggataacac aattggacct atggttatgt taaaagtcct taactattga 9120
   agaaaaaaga aattataata taactaagga ttccataccc accattggag ttgctataag 9180
   aagaagaaaa aaaattgtct agaggcttcg ttttgcgtta cttcatcata taggtaagta 9240
   gagaaccaca tcttatcgaa ttcgacctct ctgcttcgct ctcagtactt gaaagctcaa 9300
   tcaaccctct cttgtagtag gcaatttcaa atccacggac atggcaaagt tcgtcgaagg 9360
   cgacaagcga tggatcgtgg aagacagacc cgacggcacc aacgtccaca actggcactg 9420
   ggccgaaaca aactgcctcg aatggtctcg caacttcttc aacaaccaat tctccgacgc 9480
   cgtgatcctc tccggcgagt ccaacctctt cctcaaaatc aacaaggtgg agaagctcga 9540
   aggcgaggcc tacgtgaacg tgcgcaaggg gaagatcatc cccggctacg agctcaacgt 9600
   ctctctatcg tggcaaggcg aggcgaagga ctctgaagga agacgatctc gaaggcggaa 9660
   gggttggtgg agatgccgta catctccgat gagaatgccg atgagaatcc agagattagg 9720
   tattagacgg tacttatagg tgtaattcgg tttagtatat accggtagtt atggtggttt 9780
   aagtttactt tagtatatta cggtacatat aggtttaatc cggtttatta tatacagatg 9840
   gttatggtgg ttaagattcc tttagtatat ttcggcatag gtttaatccg gtttagtata 9900
   tacaggtagt tatggtggtt aagtttactt tagtatatta cggtacatat aggtttaatc 9960
   cggtttagta tgtaccggta gttatggtgg ttaagtttcc tttagtatat tacggtacat 10020
   ataggtttaa tccggtttag tatatacagg tggttatggt ggttaagttt cctttagctt 10080
   tccacttgta tttaaacttt gatgtgtaca tttgagaaat atatacattt caatgttagg 10140
   gtttcggtta aggacgacgg ggagattggg aagacgttga aggaagcgat ggtgacaaaa 10200
   gggaaggtgg ttgttcagga gaaggttagg gtttacgtgg aggcgatggc tagaggtggg 10260
   ccgtgtaggg atgagttgga gtttaagaag gttgcgccaa aggcaaagga gaagtctagt 10320
   ggtttaccgg ttgtatctga tgcgaaggag agtaaggtag tgaaagagaa gaaggggaag 10380
   acgaaggaag ggtttaagat gattagtatg accgagaagt ttagttgtag agtgaaagat 10440
   ttgtatgaga ttctgatgga tgagaatcgt tggaaggggt ttactcaaga gtaatgccaa 10500
   gattagtaga gatgtgagtg gtgcgattag tttgtttgat gggtcggtta ctgggatgaa 10560
   tttggagttg gaagaaggga agttgattgt tcagaagtgg aggtttggga gcaggcctga 10620
   tggtcttgat ttaacggttg gtttactgat tgtttttgtt gtggttcaga aacatggtgc 10680
   tttgatttgg atctggttgc gattgatgtg ttggctttct ttgctttgac attttaggtg 10740
   agaatcactt tcgaggaacc tgaaccagga gtcaccattg ttaatcttac ccagtgacat 10800
   tcctgaagaa gataggttag tctcacttac cgaattgatg tatgtagttg ttcgactcat 10860
   taagtctttg ctttagtgaa tggattcttt ggaatttggg tgtgctgtat tggttagtag 10920
   tatatgatat agaatactca acgcacaggc cgagaaccat tggactatta gagattagag 10980
   aggatcttaa atatcttagc cgcccaaacg tctttgacta tctatgttaa tcatttcctc 11040
   taattgactc ttagtatttg ctgcgcattt ggagcttatt tgtttgtcca tgcgctcgtt 11100
   ttcttctttt ttgttaagca aatatactta agagttattc gctcgatatt ggctcctttg 11160
   atgttatctg atgggtttcc attgacatac gggaatgcga ctgtggtgga aaacacggag 11220
   agaggatgga gagaccttat attccacatg atccgtgctg tttttgggtt tggaatatga 11280
   tatgtgattc taattttcaa acaaacaaaa ccagcattgc ttttatctat ggttatgatt 11340
   tcaattccaa aacaaggggg actagtgaca agcagcgcaa atgtatgatt tctaagtttt 11400
   ttgtttatgc attttcatgt aactatttgc tcacttctat atttatttag gaatggacaa 11460
   ttatccgtaa attttgattc gattttttgt ttcgatctga aaaatcttga tataatcaag 11520
   caaaaaacta aaatattata tccgtaaaaa aatgaaacaa atcacaaata ctatttgtaa 11580
   tatatatata tatatatata attatatctt tttctaaatt ttataatatt tttaaagtaa 11640
   ctatttagtt attaattttt taaataatag gtaatttttt aaaaaatcgt aataaaatat 11700
   tattatttat atcagatttt tttattattc ttttttcgga tcaatcggac attcatgtcc 11760
   cgtaaaatac agatttttca gatatctgaa aagttacaga ttaaattgaa tctgaaaatc 11820
   gattatctgt acggagtaga atggatatct taaaaaaatt atatctgatt tgcgccgacc 11880
   ctacttttgt tgagattgtt tagatcaatg gcacacaccg atctatgtta aaactgttgt 11940
   tatcagtatt ctatcaatca ttaaattcag atgggacaag aacttccagt gtaataatac 12000
   tgcaggtcaa ctacgttgaa ataatctatg ctcaaaagat atagttataa accccacaat 12060
   ttgacatgta agagcatatg taagagcatg ttcaacgcag acatggaacc atatgtaact 12120
   ttttttgttt tagtattatt tttttagttt gaaaaaaaaa ttaatttaaa accgaaccaa 12180
   tcgcgggtcg ccacgtgtca gtgaagtccg caaacagtga taaaaacaga gaaagatcga 12240
   tctttatttg cttacttttg tgatcggttt taaaagtttt ggtgggactc ttgcattaag 12300
   aaacccagtt ataaccatgg ataaacatgg tctaagcata tgagcctgat gccttgctaa 12360
   ttattagcat taatttgtta aacctgtagg ttgggcagaa attaagtact ttacaattat 12420
   catatttatt gaaactagca ataaattagt taaacatcgt tcaccaaata atttaatttt 12480
   ctaacatcag atcccaaaat tcaaatttcg aagattataa cctctgtcgg tttcgtgctt 12540
   ccgcgccaga ctaaacagcc acatacacaa atactttgtg gagcttgttg gaatagagat 12600
   ttggaaaaat gttaaaagat ttcatatatg atagaattta gctgaatcta tttagattta 12660
   aggatttaac agagattttg atgagaaatt tggtagattt tggtcatttt ggtctccctc 12720
   tataacctag ctaaagggag tgaactatcc ctaatactaa gcattcacgt tacaacttct 12780
   cttgatcagg aaatagcaaa tatcaaagtt agaagacgat gtttacagtt tcagattgat 12840
   tcttccacaa gctatcgttg gtccgtcagt ttcggcaaga gaagaagcat ttcttcgaga 12900
   gaattggtag gaggttcttt cacaagatca catactttaa tcatgtgtaa gaaaaatttg 12960
   tgatatttgt ctctgttttg tttcaaccat cgttggagag tgaggaagga agataattgt 13020
   tttatttatt ttgaaatacc atctaggtga ttatccgttc acatgcacga aatgaaatgt 13080
   atattctaat aattgttgaa tatgaacttt gtccgtataa ccataattat atcattcata 13140
   agcctatata tattaatttg aatatctatt aaatgttatt ttactcatat ggtttttatt 13200
   atcatttata ttttattata acaaaaaatt taaactatag atcataaaat tttcagtgtg 13260
   agagttttaa caattttcgt tatttatagt catttttaaa cattcaaaat ataatatata 13320
   tatgaaaatc tattttttat tatatggtta atatgattgt ttaatttatt ttaataatat 13380
   aacattaaaa aataatgaag atatgtgaat tgttgtcaaa tctttattat taaaatcatt 13440
   aattgtcaaa tatatatttt attcacgttt ggtaattccg taaattttat ttaagaaaag 13500
   aaagaaaaac aataattgta gattgttaat taatttcatg gttagtccaa gtagaagtat 13560
   ataatatatg tttaatggac caacatattt ctttagagac tttaagaaac attttattga 13620
   tgacacgtgt caaagttaaa atgttgtaat gcttatcttt taatatatag atgattatct 13680
   aataattcca ataactcttg ataaatatta tgagtgaaat ctttgttttc caataaggag 13740
   aaatttgaaa gagttttaat aaatgattaa ttcaataata gtggatttga tagaactttt 13800
   ataaatacta agaggagatt tgaacaaaaa ctagaatttt cttaaatcat aagcctccta 13860
   taagccccct ttgttcctat ctctttttgg tcaccattcc attccagaaa atgcttttaa 13920
   agataacaat cacaacattc caaatgtaga aaagtcacaa tctcaacatt ctaaacctgc 13980
   ttccacctaa caaaaaacaa ttgccttctc gtagtttact ccaaatacaa agggtgtaat 14040
   ataacagaaa cactcgacac tttaccgaac acacacattg ccttctcttc taaataagtt 14100
   caatcttcct aatctctctc tgtctctctc tgacaacaga gagactcttc acgtgccaaa 14160
   caaaaaaaac tgagcacctt cctctcgcca tggccacgga ctccgtcaag cacgtgccta 14220
   cattcggcgg ggcagccatc tccgccgaaa tgaaaagctt cttctccgcc gtgcctactt 14280
   ttgtctacgc cttcgtcgta acctttgttg ccttcactgt ttacttagcc ttcgcccctt 14340
   ctctcatcac tgtctctaat tcagtttctt cctatatcct ccctaatgtc agtgccgtga 14400
   cttcagcgtc cagtaacatc acattacaag caaccacgcc ggaaagtctc actccggctg 14460
   ttataaacac aacctttgag cctcccctag gtaatgaaac aaacccactt tctagaaaca 14520
   acgcttcacg ggatcatgca agtgtacact tatgtcctaa caacaatact gctcgaaatt 14580
   cggacaaaca agcacctctg tccgtgaatt caagtgcttc ttctccgatg agaaaacaaa 14640
   gtaggaagtc aggggctaaa cgagagatca agtctctgaa ggactgcaat tttttcgaag 14700
   gagaatgggt caaagacgaa tcctacccgc tttacaaacc cggcacgtgt aatctcatcg 14760
   acgaacagtt tagctgttta accaacggaa gaccagacgt tgagttttac aaactgaagt 14820
   ggaaacctaa agaatgcact ttaccaaggc tgaacggagg caagttgctg gagatgatta 14880
   gaggaagaag gctcgtgttc gttggagact cgctgaatag aaacatgtgg gagtctttgg 14940
   tttgtattct taaaggatca gttaaagatg agagacaagt ctttgaagct catggaaggc 15000
   atcagtttcg tcgggaagct gagtacactt tggtcttcaa agtaagtttg caatctgttt 15060
   tggtgaggcc tgcgggttcg ggtagtttgg gctgggtcgt tcggttcgac caaaatattg 15120
   cccaactgaa ccgaacagag tttggttcgg tttaggtagt tcggtttttc aacttctttt 15180
   accgaaacta atcaaagttt ttggtttcaa ttatatgttg gtttaaaatt tctttaaatt 15240
   cagggtaatt ttggttagtt cggtttgtca gttattttga tttgaatttt tgattaattt 15300
   agttagcttt tttttgaaaa cccgagctaa ccaattactg aagtgaaaac cgaagtattt 15360
   tataaacttg ccgaattgaa ccaaactaac caaaaaaatt tggttcggtt cggttatcaa 15420
   cgcaggccta gtttgactac ctagttgtta ttggagactt gatttgtgtg gttgatatga 15480
   taggaatata attgcactgt ggagttcttt gcgtctcctt tcttggttcg agaatgggaa 15540
   gttacggata agagcgggac gaagaaggaa actttgcgtc tagatatgat gggaagctca 15600
   tcaaagcagt acataggagc ggatgtactt gtgttcaata ccggagcttg gtggactgat 15660
   gacataacat ccaaagggta gagttctgtc agctattttt tgtttttttt atatggagga 15720
   tttgtctcat tggctttttg gtgtttaaat ggttttgatt agtgaggatt attttcaaga 15780
   aagaagcact gtttacccga aactcaacgt tgatgaagct tttagaaaag cattgactac 15840
   ttggggtcga tgggttgata agtatgtgaa tccaaagaag tctcttgtct tcttccgcgg 15900
   attctccctg tcacatttca ggtatgtaca gttctttcat ggtagtctta agattctgtt 15960
   taaaaaaata aataaatggg tttggtctgg ttgcatgcag tggtgggcga tggaatgcgg 16020
   gaggggcgtg cgatgatgaa acagaaccga tcaagaacga ggcataccta atgccttacc 16080
   cttccaagat ggagattctt gaaagagttc taaggggaat gaagacaccg gtcacgtatc 16140
   tcaacatcac gaggttaaca gattacagga aggatgctca cccgtctgtt tataggaaac 16200
   agaaatttac tgcagaagaa agcaaatcac cgttgttgca ccaagactgc agtcactggt 16260
   gcctcccagg tgtacctgat tcttggaacg agattctcta tgccgagatg ctggtaaagc 16320
   tcgaccagct ccgtggcaac agacggcgga aacctaaagg gctactatag gaggagttaa 16380
   atcagatttt tgttttagat gaaatacact atatatattt tcaatggatg aaaagaaaag 16440
   aaacacttag aagcaattat atgttttcaa aggcatagag aaagtaagag gtgagaatca 16500
   tattagtgcc ttgctcatca cttttctagt ttttagattg taaatggtta atttttattt 16560
   tactcaattt aatagtaaag gtttgtcaaa tcataactga accggaaatg gaagcaatca 16620
   tttggttaaa agaatccagt tgatgacatc gactggcaaa gcataggcag gatcaatttg 16680
   gtttgactcc aggatactcc agccctgcac acagttttgg atcattaagt tctctttagc 16740
   tttgaagacc tgaacagtat gaactaatga gaagcaaatt ttagtcacct gcaactccaa 16800
   agtaagtgtg aaagatatca acagcatcgt caggtccatt ctccatatct tattcaaatt 16860
   acacctgaaa ctcaggaaag actctaaaga gatgtgagct ttataaacca atgtttgaga 16920
   aaaacctgac agtccaagat gaactttaca agctttgatt tgtcaatcca atgtactcta 16980
   ttggtcatga taaagctcga taaaacccac caagagtggc aaacctggtg ataattaagc 17040
   aagaacaatc agtgtggtaa gaacaattgg aataatgttt tgtgtttgta gagagaagct 17100
   ccgttccctc aaatggacac ctattgttta ttcaactgcg ataaactggt catggcgttg 17160
   acaagtacgt ttccttacct ttacagtttt gttttaactt attctatttg atgttatata 17220
   tgaacattta tacatgtagt attgtggttg ctgctgcgac tgttcaaaag gagagatcaa 17280
   gaagactttg tacagccata ttaaaccaag tgataagaga actagctgtt gcatttaaac 17340
   caagtgatat gaaattattc tctccttctc tcttttgttc ttcacattct tcagtttcca 17400
   gcaagtaaaa aagctcatat tctcacactc ctattcgtct tctttggctc agcccttaaa 17460
   agatcgacaa gaatggagga ggtacatagt tttttagcac taaaactatt attcttagtt 17520
   tgtattgaaa aaaaatgttg ctggtgaagg aacaatgtca agtctttcac gtgagagaaa 17580
   tcttgcaacc aaaggggggg gcataataaa ctgagactat ataagtttcg tttagttatg 17640
   tatttcttga gaaataatgt aattctttga tttttttttt tggttaaagg gttttataat 17700
   tcacttttgt gtgttgagcg taaataaaag ggaccgcttt atacgattca gtgatgtttt 17760
   tagtttaatt tcagcttctt tcttttcatg aaattcaagc tacttcacaa ataaagatgc 17820
   atgcatattt gcgtggggaa ctacatagac acccattcaa caagaactct atttataatc 17880
   acgtcctaat gaatcggtca tttgaacaaa aaaaaaacca cttcctaatg aatcggtcat 17940
   acttttattt ttaaaataga tacaaagaca cccattcaac aagaactcta tttataatca 18000
   cgtccttatg aatcggtcat ttgaacaaaa aaaaaatcac ttcctaatga atcggtcata 18060
   cttttatttt ggagattaat gtttcataaa taaaaacaga tatcagtgta cgtagtatcc 18120
   tcccttatct ttaggattgg attcccacct tcaacattcc acttttttct catttcagtt 18180
   tgttatttaa gggtttcaaa acatacaaca attatccaaa actagattgt ttcaaatttc 18240
   tcaaaagata tttaattcga agctaattat cacgagaact acaaattaat tccaaaaatg 18300
   aagttaattt cattgatgtg catcgccttt gtcatactct tgacatcatt cccggctacg 18360
   gctattacgt tcaatacttc gttcaaccct acagattgcc ttaaaaatca tgatacctgc 18420
   ggccccctag ctgctgtaaa gggccggcgg tggagacccg aatgttgtaa attttggagc 18480
   gggaatgtac ttccagaaac aagacaatgt gcatgttatg tactgaaata ttctcttggc 18540
   gatggttatc ttccccttat tttaggtaag tgtaaattag gtggtatcga acaattcaaa 18600
   tgttgggagc tgcgaacata taactaacca gaatcgtgaa aaccaaactg gagataataa 18660
   gataaagtaa tcaattgggt atttctttat ttctttctaa tcattttaat aatgttactg 18720
   ttcaataagg agagagaaat ggttttgttt ttgttcatgc tcgtgtgaat tacgattcca 18780
   tgtttttttg tttccatata ttttcttata tatttagtaa aaaaaatgaa aaggtataat 18840
   ctactctgga ttaagtacca taaataaata tttgttctta attaatctta gtgctttgga 18900
   cttctaacac caactttatg cgtaataagc atggggagaa gaagcaaatg aagaattctt 18960
   cttattcacc aagttttgta atataagaat tttcattcta ccaaaaaagc ctgtctctag 19020
   tattttccct aacctctttc taaaaaagct tccgaccaaa tagatttgct catgtggcca 19080
   gtgggctctt tacagccggt caccataatc tttttgtttt tgtttttgct ttgtattata 19140
   tttcttctct tcgcagtggc gattatgtta tgattatgtt attcgcggtg ccgataaacc 19200
   gtggcaaatt tttgttgtgt attcaatctt aattgaagca aaacttcatg attgtgttct 19260
   gttattcttc gattgagatt gatcttttat gtatgcttct cctctttcac agtttttttt 19320
   ttttggtaat ccagggttcc cagtttcgcg ggtcattccc tgggtccggt caggcagcgg 19380
   gtcggcttca cccgggaggg tatgtcctga gcccgaaggt ccagtacccg cttcgtgaca 19440
   tggatgagca gttcggctcc ggctggcgtc gaacccgcaa gcatgacaat tggccctcaa 19500
   ggttctaact agtagaactg actcatcccg tctcctcttt cacagttgct ctttaaatcc 19560
   cgttcgcttt aatggagttt tcttcttctc tattggattc gcatgtactc ttttcttaag 19620
   ggagtcaatt ttcactcttt aaaagatgaa gccgccgatg aataggctct gagtgaagtt 19680
   tcctggatag aaaggtggag taatctcgat ttgatttgat gaagatttag atgaatcggc 19740
   atcctctcgg agatggtccg ataaagatct ttaattttat tgagtaatcc cagagttatt 19800
   acccaatgaa gttgatggtg gctctcattg aagcgacagc cggatgaaag atagcagatc 19860
   gagaagttca aagcttgttc ggtgaccatg agcagtctct gccgccggcg aggtgtaccg 19920
   acttcctctc atcccatgcg ttggtgcgac agatgcatgt cagatccaga agtttaaaga 19980
   ctcatgcaaa ggaggatgag ggcgcgtgtc acgtctttcc cgtgatgaag acacatgcac 20040
   tattaggcct aataaaattt attttatgtc gagttgtatg atgagtttca agcctgttgc 20100
   ttctcttgta atgttcattg ggcctttcta aaggaaagaa acatttgaca aaaaaaagaa 20160
   aaaaattcta cctttaggcc cttcttatca acaactttga caacagacct caatgggttc 20220
   atatcctatg gcctcttggg actctttttt ggatagtcat ccatactgtg aaggagtatt 20280
   cggggtgtga taaggaaacc aaatcccttt gtaccagtca actgctgaat ggttagcttc 20340
   catgtgagct aatcattact catcctcgca agtttttaga tgattgcttc tttttgatca 20400
   gatggttgag tataatctat tttgaattag cctgttggac tagtttctct tattcaagaa 20460
   atccacttgc tcagtgacac aagagcagct aagcgggtcc gtaactttgt tggtaatata 20520
   aagggaaaac tcagatcttc tgttttgatc tttgcttgcg tctcagacat gtattgcaca 20580
   tctttgtttc ttacaagcgt caaatcgttt atacattttt agtgaaacaa tggtgagcag 20640
   gtagccaggt agtacctttc cttgcaactc acgcatgaat ataataaatc aactatttca 20700
   ttgttgatga ctggatgatt cacaatcaag ttgctgggta tagtatttac actaattttt 20760
   tttcttttgt ttactcgtga gatcatcaaa gatgttttag atatatgaaa agcatatttc 20820
   atagtctata ttcatcttcc atgtatgttt ctttgatgat ttaaaaaaaa acagaagtac 20880
   atcaacactt cttatctttg tgttttctta atctcaagtt ggtaaataga aaaaaatgat 20940
   gaaaagaaga agaaaaacaa attactacga ggctctcagt aaagctgttc tttcttttca 21000
   tgccaccatc tccacctgta aacacaaaat tgctagaaac attagtgaac ttttatataa 21060
   ttttatataa tataaaaaac atataaaaaa ttattttaaa aatgaatttt aatataattt 21120
   tgtgccaaat cttgcattct gagatcatca agtcaatgta gtatacgtac cagtaatagt 21180
   cccaacgagg acggactttg gtcatgcggt tatcatcaga tgggtgataa ggatgagcag 21240
   cgtggtgagt gttatggaaa gcgcagcaac ctgcagcgta gactgtgatg aggaggacta 21300
   ggactagaat gttgacaacc gataactttc tccagtcaag acggatctct tcaagaacac 21360
   cagctttaca agcatcacac tcgtagcata acgtttcaat accattgttc catctatagc 21420
   aatcttctcc tccgactatt actccagtct cgtacgtgca cgccgttggt ggcttacaac 21480
   atcccgacta caatcaaaga caggttagtt actaaacaaa ctgatgaaga tgctagttca 21540
   tatcatagga cacaagggga gtttataacc aaaccggaaa aaaaaacaaa atccgatatg 21600
   aacgggccta aaatcagata tttttggatt ccgaaataat atctgaaatt agctaaattt 21660
   gttaaacttt tacatattta aggtaattta gatattatcg aaagataagg atgatgatac 21720
   ctgaacagaa gtcatatccc tttggaaata atcaagtgta gtccaagatt caatcttaga 21780
   acaagtcttt gaagtcaaga tacagcttct aatggagttc caatactctt gatctctaac 21840
   tctctctctc agccacggat gataatctcc aagtctatat tctttgtaaa ccctccctgg 21900
   tacttccaca ccacctcctt ggctagtcac cacaagacca aagagcgtta gacccatgag 21960
   agtcgctatg aggaagatca tgacaactaa gtacacccac agagcccatg ccacgttgaa 22020
   acaagctcct atgaaaccag caagggatac taacagtatg atgaatccta tcacgagaag 22080
   cggtgtctgg aggaagttct cgcaagtttt actgcttctt gccttccata gagcagttcc 22140
   tttgattggt attgaagcta gtaagctgag aaggtttatg accccaatca ctgtgttgct 22200
   gaacctgtac atactaggat tgttatcaag aagaaaccag agaactttgc agtgtcttct 22260
   ctcttctcta agcagattct ctcttatcaa ttaggaatct attggactat atttgcgaag 22320
   tgattttgcc acatgttctt tctacaatca atttcacaaa acaaatatga cattgctact 22380
   gaaattgatg acatgtatta tagcttaata tgacatggac aattgcattt aatgttaatt 22440
   tatatttttg gtaaattttt taaaatatgg taataactca tatagtacat ttaatgtcaa 22500
   tttatatttt agaaattttt tagaatatga aaataactca taaatcatca ttagaataaa 22560
   tatattcaaa tatggcatta taaatttcga aatataatat aattatatat tttaaaatta 22620
   tacaatttta ttactaaaat tttcaaaaat gtatacaatt ttcttagaaa attataaaaa 22680
   tttaatcgta aaatcattat tttcttatat atctataaat tttataaata ttgtttaatt 22740
   ttaattgttg gtgattatgc aacttttaca aatttattta atatatttaa ttaaaataaa 22800
   tagatagaaa aatctatcta agattataat ttcaaatata tacaagcata ttcttaaata 22860
   taatttttat gtttaattaa attaaattta tattaaaatg ttgatacgaa aaaagaaaat 22920
   ttacaatatt aaaaaaaaaa tttaaaatat aatttatatt tatctgttaa aaaatatttt 22980
   aattttttta cacacctagt tttaattaag ggaaaaggtt aagagttaat tgtttaatga 23040
   ttcatgctca tattctcttg tcgtttcttg atttattttt atttttgtca agaggtcgtt 23100
   tcttgatttg aatcaactaa acaacaacat taaaattgta tatttttttt caaataaaag 23160
   cgtctttttg gacaattgtt tcttgtttaa tagtatttta tacgcttatg tcgtttaaac 23220
   cagaccacaa gtagtgcctt gaataaaata tgtacttaaa attaaactat attgtatata 23280
   tagtggaaca tatcatatat agaatcagat aaattcacaa tgatcaatga aaggtaagca 23340
   aagaataata tagaggacgg atggtgaatt ttctttttaa gatgctttta ctggccccat 23400
   aacttagcat attaggttct gtaggtagag cacaatatga tctttgattc ccccattcac 23460
   attttttttg gtttaagcaa gaaaaatgct aaaacatact taatttaagc caaaatgtca 23520
   taacacaaca aaatgagaca ataataacat tactgtaaca aatacatagt ttctaattag 23580
   aacaaagact aaaccagacc aagagaaaag tcgacaacaa cttttaactc tgtccttcca 23640
   ccatcatcat catcatcatc atcatcatca tcatcatcat catcctcata acttattgtt 23700
   gtaccagaac acacctttct tctcaccttg cctatccggt tcaacataga tacactcctt 23760
   cgcctccctc cacatcgcct taaccaccgg cgttccatca aactggtaat actctccaag 23820
   tatcggcttt atcgccttgg tcgcttccat cgcgttataa tgcggcatcg tcgagaacag 23880
   atgatgcgcc acgtgcgtgt ccgtgatgtt atgaaacacc ttgttcaaga ttccatagtc 23940
   tctatccaca gtagccaaag ctcctctcaa ccaatcccac tccgaagaat catagtgagg 24000
   cagcgaaggg tgcgtgtgct gcaagtaagt gatcaagacg aggaaacagt tgacaatcat 24060
   aagcggaact ccgtagacac agaccatcga ggccactcct cgcgaaccag cgtagcggta 24120
   gagaccgtaa catacggaga ggacgccagc gtcagagatg tatatctgga gacgctcgcg 24180
   gtcgttgtag atgggagcgt tcgggtggaa atggcaagcg aaaccgtcgc tgtaaggtct 24240
   tccagagacg ttgaaggcta agtacaacgg ccagccgagc gtgaactgga cggttagcat 24300
   caccgtgcgt cctagcgggt tgttgaggta ctttccgtac cacttgatgt cggatttctt 24360
   cttggggacg aacacttcat ccctctcgag ggatccggtg ttggaatggt ggcgtcgatg 24420
   gctgtacttc caggagaagt aagggacgag gaggaaggag tggaagacga ggcccacggc 24480
   gtcgtccagc cactggtggt cgctgaaggc gtggtggccg cattcgtggg cgatgaccca 24540
   gaggcccgtt aggacgcagc cttggcaggc ccagtagagg ggccaggcga ggtaagggag 24600
   agggtggggg aggagaggga agtaggctgt ggagaggtgg tagagggagg aggagacgag 24660
   gatgtcgaag aggaggtagg agaaggagcg agggatggag cgtttgaagc agtgaggtgg 24720
   gattgctttc ttgaggtctc cgagagtgaa gggtggtgtc tcgcagggga cgcgtttgag 24780
   ggttttggtt tcgggggagc tggagggagg agagacttgc attcttccac ctgcgcccat 24840
   gtttgtttct gtagagaaaa ccaaaaaata ataataatgt tatggaggac ggagactttt 24900
   tatcctttaa tcaaagttgt atatgcaact cttcatgcat ttgaatctat aaaagaacat 24960
   ctaaaccctg aagtacctac ctactttaaa tcatccgttt tatttgtaca aagttgatga 25020
   gcttataagt caatgaaacg ttcttccatt tattaagaaa taacagtagc aagaaccagt 25080
   aggaatcggt caaatctatc aaaggttcaa caataagtga tttttatatt gaaaaaaaac 25140
   aaaaatagca ctaaaccaag tttttgttcc caaactagca ctcaaggtca aaagtcacaa 25200
   aaatatcact taatatttta tcaaaagtca caaacttatg gtttagagtt aaagggtggg 25260
   gtttagggtt tagggtttag ggtttagagt ttagggttta gggtttatgg ttcagagttt 25320
   agggtttaga gttgagaaat gaggttttgg ggataagatt tcaaattttg aaaaataaaa 25380
   aattaaaatt ttcaaaagat aaaataatat tttggtcatt ttaatttttg agtgctattt 25440
   ttgtgatata aacttagaaa gttgctattt tggagatttg accttttcta tttcaccaac 25500
   ctattgaata aaaagatttc tgaatagata gaaagttgca acatttcata tgctagacca 25560
   tagacagtaa tcattattta acggtgagat gaaagaaaca tgacacaaga tttgtcaaat 25620
   ggaaacagag gaacagatct ttagttgaat gcaataaaga ctggcagatc tatcgattta 25680
   aatgccaaat gctagactgc tagagagatg gacgagatga acacagacca ggaattcata 25740
   aaaataagag gaaaatgaga agaagaagaa gaagaagggg accctgaaag ctgctgacgt 25800
   agggtggggg gagagagatt ttacgttaat aacgatgaac cctacaatga agctcctcca 25860
   aagaatctat ctctctctct tctctttctc tctctctctc tcttgtctcg gcacttctct 25920
   caatctgttg tggtttgtct ggtcagatgc gagtggcaat aggcagggcc gtttttaaat 25980
   tgatgagaga caggaaggca tttgaatgac atctcttatt cttacaactg gcccctcttt 26040
   tctcttattt ttacaactgg gcccctctgt tcaaaatatc tgatatttaa ctcaaggaac 26100
   gaccattaat ttagaattta ccaaaatgta aaagttagag tgatgaaaag gtgatattac 26160
   tacgacgttc tctctttttc tcgtccacgt tatcttggtt atgtatgtaa tgtaataaaa 26220
   aaggtcgtat catgttattg acttaatcac tttggaacgc aatgacacat gcggtacttg 26280
   tttgaatcag tttttctcta cgtaagtcaa tcatgtatca tttcttaata tgacattaga 26340
   aacaacttga taaagaagaa gaagaagaag atggtagttg atgcctgaaa atggagtatc 26400
   gtgtttggct tcttctcact tttgcccatg tgaacacaca agaacttaca gaaatatgta 26460
   acagacaaat tgtggtgatg ctcacgtgag gatttctctc tcttttcttt ttcttattac 26520
   aatttgttat gtatgtgtaa tcatctttga tacttataat aatcaaattt gttggcagac 26580
   gaaaagaaac tagtaattga ttagattgag atctcttgta ttatactaga cttagaagat 26640
   gattagagtt tgatttgtct aagcattacc tctaacaaca acacactttt ctttcaaaat 26700
   tttttgttta agatcacaca cccaccatta ggaatgttat atttattcag ctgattgtta 26760
   ctaccagtga cgggttttat aacatgtttt tttggtatca aagatgatgt gtctgactca 26820
   cgcacaacag acaaatcaga gcaagcatga ctgtaagaaa attaaggcag catacaaatc 26880
   ctaaccaccg tctgtcaatc catccaccaa gtctctcatc aattcaaact caatttaaca 26940
   gcaaaaaaac tatattctaa gaatcaccac acagagagaa agaaaactta caaagacaat 27000
   gaaactaaca ttgtattttc ataaaacaaa agcaaaatag atgaagaaga tggatgatat 27060
   tagagatcac caccacacac aaaagaaaag acagcaaact ccacaaacag caacttaggc 27120
   aattcctgac ctgcgcagct ctgcttcccc gtgcgcaaag tgacacctat ccccaaacgt 27180
   acagttccct ttcgcgaacc tctcacacat cttcgtcttg aagttgctcc ctggatgtgg 27240
   tttcccttcc gaaccaaccc caccaccaat cccaccacca ccaccaggtg gtctcctact 27300
   agctgcagag ttaagcctcc caatcagctc tctaaccatc acactcgctt cgtttatctg 27360
   ctcaaacgtc ccttcaagct caatgttctt caggttcggg tccctctcgt gatcttgaat 27420
   cgacagcttc gcccccgtct gacgacatat ctgcttcgaa ctgactcctc ctttaccgat 27480
   gatggctccg gccaacgaag cgtccacgct gattttcgca gtggctgagg cgccaaagct 27540
   agacacatgg ccaggtccag actctcctcc tctccctgag aatctccctc ctccaccacc 27600
   accaggtcct tgcatgtttc tggaagcttg aggcatgggt agtgccatgt ttgtcagctg 27660
   tgctacagca ttgtatcctc cgggtacata gtgcaagaag tggcagttat ctccaaaagg 27720
   acagccagaa gtgctgcaat agagcataat aaagacatca gatagttaaa accatttccc 27780
   ttaagatgac acaagagaag ttgagaacaa acggttaaaa tacagaggaa gtatcaattg 27840
   ctatacacaa ggaaccaatt agtcaccacc agtaatgtaa taaacttacc tgaacctgac 27900
   catatatgac catgttataa acacaacatc actaacacct taccaccaaa cagaaaatta 27960
   tagcaatttt ctaaacaagt tatagtttga tttcgaacgt tatgatcacc aattgagttg 28020
   aacgtttcta tgattataat ccagatgcac ttatctctta ttgaaacttt tttttaactt 28080
   gtttaggatc aatgatttat aagtacctgt aataacaatt aaccacttca ttagcataca 28140
   gttttacttg ttaatttgac aattaacaat tttcaatatt aatccagtta agaaaaaaaa 28200
   ggattataaa tttataacct gaaaaattta gtgcaaggct tggatttgct gcctaaacca 28260
   gttgaatatg attccatctc tgttccaaac atttaagtgt gtttttattc tttagttaag 28320
   agttttctca agttcaataa tatgacacca ttcatacttt tcatccatat gaaacttgag 28380
   acttaagaat taaagctgca tgataccttc tgtaaatttt caggacacat caatccgaca 28440
   aaggaaaaga catgggctgt cttactcaac tgaacatggt ttactaaaag caaaattgga 28500
   actaaagtaa cttcgaaaaa aaaacat 28527
<210> 4
   <211> 26095
   <212> DNA
   <213> Brassica napus
<400> 4
   acatcttcct cggaatatac cgagggacac ctttccttgg aatataccga gggacatgtt 60
   cctcggaata ttttgaggaa gatgtccctc ggtatatttc gaacgttttt ttataaacgg 120
   atcgatcgat ggatatatgt ccaaaaacgc atcgatcgat gaacttccga ggaaatatcc 180
   cgacgaagtt ctccctcggt atattccgag gagattttcg acaaactagt gatcctcgga 240
   atttcctcgg aaatttgttt cctcggaatt ccgtcggaaa attccgaggg atttccgagg 300
   aaagaagaaa ttccgaggaa ttatttccga ggacttgttt cgtcggtatg tcgtcggaat 360
   aacgttattc cgacgaaatt ccgacgattt tttccctcag tatccttgct gttttcttgt 420
   agtgcaagaa agctaataga tattagcaag taagaagaca taagtacttg atggccaagc 480
   aaaagcctag aattggaata tcacgacatt caagcaaaag gagaagacat attccaacag 540
   agccagagcc atgttcaaga tgagacaaac aaatgtgggt ctataagggg acataaagca 600
   aaaactcaat aaaaagttag gctagtgcct atatcagctg gacacatggg agaaccaggt 660
   ccatgctata taacaatatt atcaaaagca gtgttttcat ataagtagga gtaactttct 720
   tccctagtcc actctgtggt ctaaataacc accagaggca ctacacagga tacaaacaaa 780
   agttgaaaac acacacagaa tctatgtata gagagtgaca aagaaaagga taagaaggta 840
   cttccattga aagtacggag tgtctgatat atattgaatg tataactatt gtaattatca 900
   gtcaacaaag tcctcacaaa gtcaagcctt tccatagagt cttttttcgg tagactcttg 960
   ttagcaatgg atgaatcttc caagtgccct ggcgcaatat gtttcgagat cgcttgccaa 1020
   acccaacact ccagctgatc caccatgact ctctccatcc tccactctac aaccctacaa 1080
   cccgctcgac accgtctccg acgttctcaa cgccgctttc ttccgttaaa tctcccgttc 1140
   ccgtctttac ccaaccaaaa gcaatctcaa gtgctgtcca atctcaaacg gaggcgaata 1200
   cttcatgagt atctcaatcg gtactcgacc aacagccttc agatcgtcaa ataacataag 1260
   agagttagac gacgcttgga ttaagccgct tctatcatgt tctatgcttg gactcagaca 1320
   gagtatatga cgactatttc ttatcaatac atataggccc aaacaattta aactcaacaa 1380
   ctgagcccaa taaaaaactt cgaataaatg aaacgatgag tttcataata ctgggacacg 1440
   tggcacaatg atgtaaagtg actttcctaa ctggaatcct atgtgtcaca atgaggaggg 1500
   agtaaaacac ttatttatat aattagataa ctatgtatat aacacataat cggttgaatc 1560
   gaatgttttg gttcagatct cagtacataa ccaaaccgac ccggtgtccg aagtgcttag 1620
   agctaaaaac ccattcggta tttgcctggt cccgttaccg aaccggaccc attattttgg 1680
   ttcggtttaa ggtgtggact ccggtttaag ttaaaacccc cagccctagg gtaaatgata 1740
   aaacatttgg ttgtgtatct cctcggtggt atggaaaacg taatagaatc cgaacttact 1800
   agaccggtac gcgatttatt tttcgtctta cgtcgtaaga tgtcgaataa ggaaaaagca 1860
   acgtcattat acatgttaat ccggttatct ttgatccttt tcgaggacat ttcggtttac 1920
   ctttttgttt gttcaatacg ccactccggt ttaaaaaaaa aatcgggaag agttaggaaa 1980
   aaaggaaaat ctcatctcac cccctcgacg acaagacaac caccaccctt agggttaaaa 2040
   aggtttattt atctgattat tattctctct actcgttcgt tctcccttta attccgattt 2100
   cgatttcgat ttgtcttctc cgatttgctc tctagacact cacagtaggg tttctaattc 2160
   ggatctgtac accttttgcc atggatgctc gtaagagagg acggcctgaa gctggctcat 2220
   tcaactccaa tggcggtgga ttcaagaagt cgaagcaagg ttagctcttt cgcctcgtcc 2280
   tcttactttt ctatctcaaa agagtgttat agttctgttg tgcttcactt gctctgttaa 2340
   aggttgaaac tttagatctt tattgcttga tggatagttt ctctgtgtca attggatttg 2400
   gggtagcgat gatttcttca attcgagctt cgtaatattt tttgctcaac aaaagttttt 2460
   gtttttagtt atgagattct ctaggattca agtttggatc tttctttcgt gctcacttgc 2520
   tgcagtcttt gatctgatgc aaatctaaag tcaattaatt tttcagttta attgtggatg 2580
   agcttttgat aagtgatgtt aaagttagtt tgagtgctgt tcgtagcaag cttattgtta 2640
   tttgactttc tcttgagttt cattattgct ttagtttaat cccttttgca ttagctagta 2700
   acatcttatc accatgccca aagcttgagt ttgctgtgat tgaattagcc tttttatgtg 2760
   ttagttattc atgtgtctag ttgttggtaa ataatcctga aaattctaga gaatatatct 2820
   tgcagctttg atttatgtgt gaagttacat atagatgaat ttgatatgta tgattctaaa 2880
   agcaaatatt attgaacatc agcaaagtct taactaaaga aaaacactta atctttggaa 2940
   cagagatgga atctggttta ggaagcaaat cgaagccatg cacaaaattt ttcaggttat 3000
   aatccttttc ttaactaaat taatatttaa aattgttatt tatagtctga gctttaaatt 3060
   gacaagtaaa aatgtatgct aatgaagtag ttagttgtta ttacaggtat gcatcatcct 3120
   tcatgctaaa caagttaaaa aaaagtttca gtataacaaa tatgatattc actaggaacg 3180
   gaaaaacaaa tctggattat aaatttagaa atattaagct caactggtga ttatagctta 3240
   gaaaatcaat ccacaccttt tgtttgagct gtggcagtag ttaagatatg tgttgtaatt 3300
   ctctgtttgg tggtaagata ttagttattg tgtttgtatc atcatggtca tatagtgtct 3360
   agtaaggttg ttgcattact gaatactggt gaacatgtcc gtcatagaca agcagttagc 3420
   atagttgcta tgtgccaatt cttctgtgtt tcaactgttt gttctcttgt gtcttctcaa 3480
   tgccaatggt ttttaacttt ctgacgtatt tgttatcctt cttttgcagc acttctggct 3540
   gtccttttgg tgagaactgc catttcttgc actttgttcc cggaggatac aatcctatgg 3600
   cacagatgac aaacatggga tcacccatgt ctcaagtttc cagaaacatg caaggtggtg 3660
   gtggtggtgg gggccgattt tcggggagag gagagtctgg acctggccac gtctctagct 3720
   ttggtgcctc agccacagcc aaaatcagtg tggatgcttc cttggcaggc gcaatcattg 3780
   gaaaaggtgg agtctgttcg aaacagatat gtcgtcaaac aggagcaaag ctatcgatcc 3840
   aagaccacga gagagatccc aacctgaaga acattgagct tgaaggaaca ttcgagcaga 3900
   tcaacgaagc gagcgcaatg gttagagagc tgattgggag gcttaattct gcatctagga 3960
   gaccacctgg tggtggtggc ggtggacttg gttcagaagg gaaaccgcat ccagggagca 4020
   acttcaaaac gaagatgtgt gagagattct cgaaaggaag ctgtacattt ggtgatagat 4080
   gtcactttgc acacggggaa gcagagctac gcaggtcatg aattgcgcct agagttgctg 4140
   gtggagttag agagtttgct ggcgaaacaa gtctctttca tttgttgtgg tgattcctaa 4200
   tatcatcttc tcctacttgt ttttagttgt ctttgttttt tgagactaca atgtttagtt 4260
   ttcattgtca gtgtaagttt tccccatttg gtgttttttt agaatctagt ttgaatttga 4320
   gatgggggga tgcttgatga atgattgaca aaacagtggt taggatttgt atgctgtttc 4380
   tacttaatat ttcatgtttt ctctgcttta ttttggtcag taagttcatg tgtttctctg 4440
   acatgtgtgt gattatcagc tttgattatt ttccgagtat gtagatgtta tagttctctt 4500
   atgatagaca atataactaa aaattcatgt taataatagc cgtcgctgat agtaacagct 4560
   gaataaatga aatgaaatca tggtaggtga tgatcttaaa aaaaatgttg aaaataatgt 4620
   gcgttgttac aatagcatct cctaaccact tttatatatg tctctataat agcatttaga 4680
   tttagaagta aaatcactgc aatcctactt tatttcttcc tctaaaataa aaattgttat 4740
   tttcacggaa atacattcct ttataataaa aacatacttt tttattcaca aaataatctt 4800
   ttaatttttt attttaacaa ttataaccaa aataaatatt ttttaatgaa aatgtactgt 4860
   ttatataaat atataatcat actttttatt tacataatag tttctataaa aatattcagt 4920
   ataaataata tcatagtttt atgaatgtta cactaaattg gattggtttt caactttcac 4980
   aaataaaaag tactatttat aaaattagaa aaaaatatat caagactatt cttttttaga 5040
   ggaagaaata gaagaataca ttggaaacaa atctatctct attatatagt tttcctattt 5100
   tagaaaaaaa aatagagaaa tacattggag atggtttaag cggtagtaac acaaagaaaa 5160
   actctaaata tcttaagagc atctctaatg tacacttctg taatttcttc taaaatagag 5220
   atctctatta tagaggtgaa aatgctccaa tgtatgcctc tataatagaa ttcatctatt 5280
   ttaaaagaaa atatagagaa aaattacttt ttgcttttat atttaaaggt ggaaataaaa 5340
   tatctctata taaataaata aactctatta tacatgtata cattggagca ttttcacttt 5400
   tataatagag tttttttatt ttaagaaaaa atatagagat agaaatagaa atagaaatag 5460
   agatgagttg gagattagaa atagagatga gtttgagatg ttgttacgta agaaagagct 5520
   agagctttaa taaagtactt aaattaatta ctagtcggca gtcgctgcct acttgtttac 5580
   cacctaaatt aatttattat aatatatatt acgaatctcc aaagtacaca tcacacacac 5640
   tctactcacg tgatctcaac cacaatgtct gcagatattt tttatagttt tttctcacat 5700
   gggagagaag aagccaagca cgatcctcca tcctcaactt tatagcattt ttttcttttc 5760
   tttccggcta ccacttgtga gtcgagtcgg caagggcgtt tccttatatt aaagtaaaga 5820
   catcaaatac catcgtctta atgctaatta acgtaattga tgagttctat aacataatcc 5880
   aaactagtct ttgtgaacat taggattggg taaaccaata tttacatttt aaaaacaaaa 5940
   tacaaaaaga aacgtgataa actttataaa agcaattata tgatcactgc atcttttcca 6000
   cttttccgta aataaataca taaaagtgcc gtaaatatca gatatttgga gtagaaaagt 6060
   aataaagaaa agaaatatga ggagagggaa taatggaggg ggcccacttg taaaaaagaa 6120
   agaaaagaga tgtcactcaa tcgtctccca cgggcccccg tcaatttaaa cggcctgcct 6180
   tctgcccaat cgcatcttat cagaaccaga cagattcatt accaaagaga tagagaagag 6240
   agagagagag agagagagag agagtgagtt tgaggaggag cttcttcgta gggttcatcg 6300
   ttattaacgt taaatcttca ccccctacgt cagccagctc aaggtccctt tcttcttcca 6360
   tttcttttca ttctacgttg ttttcaatct tatgaaactt tctggtctgt gcttttctta 6420
   tcgcttttct attctatcta tcatttttgc atttcagtcg atttaattct agatctgtta 6480
   atattaaact atagatctgt tcttgattct ctgttttcat gtgtgaaatc tgatgctgta 6540
   ttaatctgat tatattgtct ataccgtgga gaatatcaaa tgttgcattt tcatttgtcc 6600
   gaatacaaag tgtttgactt tcaatcgttt ttaattatat atatatatat attttttgat 6660
   gggttggtgg agttgaaaaa tcaccatagc agtctcacgt cctggtttta gaaatatcct 6720
   attcaaaatt atatatttgt ttacttgttt tagatctgga cctgagacat ataagtacct 6780
   atttgttgaa tctttgggta aaaacttatg tctctgggta aaatttgctg ggagatttga 6840
   ccgattccta ttggctcttg attctgtagt tacgtaatac atgaaaaagt ttcatttggc 6900
   ctatgctcac ttcatgctta taaacgtttt cttgcaaatt aattggatta gatgttattt 6960
   catagattca gtcattcaga tacaatggag ttgcatgaag aaaataatag aattcgtgac 7020
   agtaaaaaag attgtatttt tgtttgtttg tttatgttta aaagtctata tgttgacaat 7080
   agagttgctc tcaactgttt catttagctt ctttttttgt caagttgctt attcttagag 7140
   acattgtgat tatgacttgt cttctttaac gtagtttagt aataaaagac gaaagaaatt 7200
   gatatccaca agaaagagat gtgagctgta gcgtatcaaa tctcgttcat ttactagtag 7260
   tattctcaac gctatcgttt atttattttt ctttcgttgg tttgccacta tatgccactt 7320
   ctctcctctt tgtcccacgt actatccatt ttttttgtgg tagtccattt tcttgtaact 7380
   tataataacg taactctgaa tcttttgtct gtagattaat ttgttggttt aattaacttt 7440
   taagtctttg cttttggctt atgcagaaac atgggtgcag gtggaagaat gcaagtgtct 7500
   cctccctcca agaagtctga aaccgacacc atcaagcgcg taccctgcga gacaccgccc 7560
   ttcactgtcg gagaactcaa gaaagcaatc ccaccgcact gtttcaaacg ctcgatccct 7620
   cgctctttct cctacctcat ctgggacatc atcatagcct cctgcttcta ctacgtcgcc 7680
   accacttact tccctctcct ccctcaccct ctctcctact tcgcctggcc tctctactgg 7740
   gcctgccaag ggtgcgtcct aaccggcgtc tgggtcatag cccacgagtg cggccaccac 7800
   gccttcagcg actaccagtg gcttgacgac accgtcggtc tcatcttcca ctccttcctc 7860
   ctcgtccctt acttctcctg gaagtacagt catcgacgcc accattccaa cactggctcc 7920
   ctcgagagag acgaagtgtt tgtccccaag aagaagtcag acatcaagtg gtacggcaag 7980
   tacctcaaca accctttggg acgcaccgtg atgttaacgg ttcagttcac tctcggctgg 8040
   ccgttgtact tagccttcaa cgtctcggga agaccttacg acggcggctt cgcttgccat 8100
   ttccacccca acgctcccat ctacaacgac cgcgagcgtc tccagatata catctccgac 8160
   gctggcatcc tcgccgtctg ctacggtctc ttccgttacg ccgccgcgca gggagtggcc 8220
   tcgatggtct gcttctacgg agtcccgctt ctgattgtca atggtttcct cgtgttgatc 8280
   acttacttgc agcacacgca tccttccctg cctcactacg attcgtccga gtgggattgg 8340
   ttgaggggag ctttggctac cgttgacaga gactacggaa tcttgaacaa ggtcttccac 8400
   aatattaccg acacgcacgt ggcgcatcat ctgttctcca cgatgccgca ttatcacgcg 8460
   atggaagcta ccaaggcgat aaagccgata ctgggagagt attatcagtt cgatgggacg 8520
   ccggtggtta aggcgatgtg gagggaggcg aaggagtgta tctatgtgga accggacagg 8580
   caaggtgaga agaaaggtgt gttctggtac aacaataagt tatgaggata tgatgatggt 8640
   gaaagaacaa agaagatatt gtcacgaacc tttctcttgc tgtctctggt cgtctttgtt 8700
   ttaagaagct atgttttcgt ttcaataatc ttaactatcc attttgttgt gttttctgac 8760
   attttggcta agttatgtga tgtgggacac gttagtgtct aaaatgtctc tgtgtctgta 8820
   ttgttcttct catctgtgac tttcggacaa ctaaactctt gttctcgaac tacctcaatg 8880
   tggcattaat gaaagtgtta ttgttgattt taatctgaaa ctgctattat ttagtgaatt 8940
   tttacatcag ccaacttgtt tgtttaagac ctaccaatgg tataagaagg tttgtgtact 9000
   aatgttcacc atgtccatag tgttaagaca taaccatgat cttctgtcca attaatttgc 9060
   gtcgagttat cgtgttattt ggcaccttta ctatgttttt ttgtaaagaa ctccttacag 9120
   aatagctttt tgtaaagaac tacgttttat ctttttgtaa gaacctttta acaaaagcca 9180
   aattcattat tacctggcac aagaaaaaac tctggtttct tcctctttct ctgtttttag 9240
   atttgaggag gaacatgaag atgaagaaaa agaaacaaat aaataacaaa tctctttttt 9300
   tccattaacg gcagaaacac caaaacagag tgacaacaag aaacaaatgt agtgaggaaa 9360
   aaccaaagaa aaaagaatat tctgaaacca actcgttgaa catattcaaa tacgaaacaa 9420
   tctttcatcc aacggcgagc gtaatctaga agcatttcct gtggactatc gatggccctg 9480
   cctcatcata ctcagccttt gctatccaca tctgcaagac caacattgtg tatcatagtc 9540
   agcttaaaaa cgagtaacaa gcagaatcga caattttacc tgttggaagg tactgagtga 9600
   tgctagaata gatcctccaa tccaaacact atacttcctc tccggtggag caaccacctt 9660
   aatcttcata ctactcggag ccaaagcagt aatctcctta ctcatcctat cagcaatccc 9720
   agggaacatc gtggtaccac cactaagcac aatgtttcca tacaaatctt tcctaatatc 9780
   cacatcacat ttcatgatcg aattgtaagt cgtctcgtgg ataccagcag cttccattcc 9840
   gaccaaagac ggctggaaaa gaacctcagg acacctgaac ctctcccctc cgatggtgat 9900
   cacctgtcca tcaggcaact cgtagctctt gtcgacggat gagctagtgt tcgccgtctc 9960
   catctcttgc tcgtagtcaa gtgctatgta agcgagtttc tctttcacgt ctctcacgat 10020
   ctcacgctct gctgttgttg tgaacgagta gccacgctcg gttaaaatct tcatgaggta 10080
   gtcagtgagg tcacgacctg cgagatcaag acgcagaatg gcgtgtggaa gagcatatcc 10140
   ttcgtagatt ggaacagtgt gactcacacc atctccagag tccaatacaa tacctgaaac 10200
   aatgattcca catcaataaa agtgttctac ctttttttta tcaacaaaag tgttcttcta 10260
   ccttataaac tctctagata attataacat aataataata aataatttat aattataata 10320
   attaatattt tttagtataa aatcttatga gaataaacaa tataaatatt attatttgta 10380
   aatatttaaa ctcttatatt attatttttt tccaaccact acactcttat attacatata 10440
   ttgttataat tggtaaacaa ctaggtcgaa gataggcatt gagttaccgt ctcagcgcta 10500
   aaatgtctac taaaattata ttacattaca ttgagaaagc taagatgaac atcataaacc 10560
   aatggtgttt gaagatctta ccagtagtac gaccactggc gtagagggac aaaacagctt 10620
   ggatagcgac atacatagcg ggggtgttga acgtttcaaa cataatctga gtcattttct 10680
   cacgattagc tttaggattg agaggagcct ctgtgagaag aacgggatgc tcttcaggtg 10740
   caacacgcag ctcgttgtag aaagtgtgat gccaaatctt ctccatgtca tcccagttgc 10800
   tgacaatacc atgctcaata gggtacttga gagtcaagat acctctcttt gactgagcct 10860
   cgtcaccaac gtaagcatct ttttgcccca taccaaccat cacaccagtg tgacgtggtc 10920
   taccaacaat gcttgggaac acagctcttg gtgcatcgtc tcccgcgaat ccagcctttc 10980
   atatagagat ttggaggtaa gaaaattaaa agattttcag acagcattat attaaacaaa 11040
   gttgttacgt ttatgattga ttaccttaac cattcctgtt ccattgtcac acactagtgg 11100
   ctgaatgtcc tctccatctg ccattttcta atgattctga aactatatat atatatatat 11160
   atatatatat atgtcaatga ttcaattgat tacaaaaaca caataacatt cttgaaaaaa 11220
   atcaaatgaa catgaactca aacaaagatc tctgattcac tcacattgtt acaaaataca 11280
   aaaaatcaat ttaacatttt accaaagaaa aacaaaaaat cgagaaaagt ctggtaattt 11340
   atttttacct gatagtttgc gaggagagga aaaatagtac gaagagaaca aagagaagag 11400
   cgaacgaaga agagaatata taggaagagt ctttctgaga aaagaagttt tattttattt 11460
   taatggtgga agaagatccg agccgttgat atttgtaaga tgtgaccgaa gaaggacccc 11520
   acgacgagtc atgttgatgg tggatacagc tgtctcataa agagtaacga cactccattt 11580
   aattatttta ttaatctttc gaaatttggt aacgtaactg aaagtatcta ctctgtaaag 11640
   tattaaatgg gctggaaaat gttccttaag ggacaaatcc aaccaaaaat tagttattaa 11700
   tattaacggg ctgaaaaatg cttgaataaa aagttaatta ttaatattaa cgggctgaaa 11760
   aatgcttgaa tgtgatatgt taaccctact aattttaata aagtttacta gattctgcca 11820
   gtatatttgt ttttattcag aaactagggc tggcccgccc tacggacgga atgaatattt 11880
   aaaaataatt taaattgtta aaataagtat ttaatgaaaa tttttattaa ttaaaaatat 11940
   aaatattagt tatatttctt tttcttgggg tggcattaca tataataact tatgtggtgc 12000
   attattaata ttgtgtaagt tgtgattgag atgtaagagt gaagttgtga tcgagaagtt 12060
   attaatattg tgtaagtgag aagttattaa tattgataat ttatattatt tatttaaaat 12120
   cttagggggt tttaacttgt ttttgttttt tccatttttg ttatgttttt aactttaaaa 12180
   gtgtttataa attataaact gctaaattcc cattgaaatt ttgtgattga aatttaaata 12240
   tttataacaa aatacaaata attacaaaaa catacgtaag atatatttgt attgtatttt 12300
   ttaataacat ataccatata aaaccaacta attatttaaa tttagatttt aatagctgca 12360
   ttgtattttt taataacaat tatgaattac taaaaacata tactatttga atttttatta 12420
   atatatagtg atttccaaca gaatatgcga atgattattt ttatctgaaa atgagaaaca 12480
   ttttgactaa aatattgtgt ctcgatacat gtgaccacgt caatttaata tatcggatca 12540
   ttaagtgtcc aaatattttg taatgttatt ttctcagttt cataagataa tttgaatata 12600
   taactttatt tctaatgtta ttggttagtg tattttaata gatttagaaa tccaaattaa 12660
   atatattatt attagttata taattgtaaa atatatattg aaatcatgtg ttactggtgg 12720
   catgatttaa aaattctaat tcaaaacaag tgttattcaa tcatactatt tattaataga 12780
   tttgatttca taatggattt gaatagattt gtatatattt ctttgttaaa atataaattc 12840
   tcaaatctga aggtaacccg aaagaaaacc atcggatttg taaatactaa tttttttttt 12900
   ttttttgaca gcaagaaatt tacagactca tgttgactct gtaaaccata ttggtaactc 12960
   cgcatccatg tgaacgacaa aggacagttg cttgcgtgca ctacgtgcta agctatccgc 13020
   ccgaaggttc gccgtccgag gtacatggac gatgtctgag ttgaggaagt ttcctttgag 13080
   gagtttgata tcttccaagt agcttccaaa tgctggccat tcttctggtt ccgaaaccat 13140
   cttcaccaat tgagaacaat ccgttgcaaa cgtaacctgg acctgtctta aattcctcat 13200
   acatttcatt gcccaaatca aagcctctat ctccgaatgc aggggagaga ggcatgccct 13260
   tacattcctt gcccccagta gaccctcgaa ccccggtaaa gtactatgcc agccttgccc 13320
   tgacattaat tctttatcct tccatgatcc atcaataaaa caccatcgac ctgatgtctc 13380
   taaaggagga atggtttgta ccgaaagccc cctccttggt tcatttctca cttgtgcgtc 13440
   tgcccagagt gatgattcca cttcagccaa tttaagagta tcccgcggat caatatccaa 13500
   attactataa actttgttat ttcgggcttt ccatatatac cataaaatcc atgcaaaatg 13560
   gtggtcatcc atttgcggtt gtactctcca aaaaagatga tccatattaa caaataaaga 13620
   gctgataggg aagatattat gcggtgaagg aatcttggat agtgcccaca cttgacgtgc 13680
   aggaggacat tcaaaaaata catgatttat tgattcttcc ggatctccac aacgagcaca 13740
   acatatatct cctctgatcc ctcgtgcctt taaatttttc atcaccgata tacatcctga 13800
   taccatttgc cacaagaaat gccgtatctt tggtggacac cgcactttcc agcagaaagc 13860
   tttaagtata tccactgtgg ggccattaaa aacaggaggt tttaacctat cagggtaaat 13920
   acgttcaacc tgataacctg attggaccga atattttcca ttattagtaa aatgccatcc 13980
   atccttatcc tccatctgaa tcctacttaa agggatactt tcaataattc ttacatcctt 14040
   cggatccact agagccctga ttgcctgtat attccatgaa cgagattcct gattgatgag 14100
   ggaatccact gtgagttccg gataaaagtt gtgaaatttt ttatttgctg gtctcgggcg 14160
   agtggctggg atccaaggat cattccatac agagatagat gatcctgttc ccaccctttt 14220
   aattagtcct ttacaaacca gagatctagc agaagtaata ctcttccagc catatgacgg 14280
   ggagtaagat cggatcggtt ccaggggtga agcattcctg taataccgtc ctttgaaaac 14340
   tcttgaaaaa agagtatttg gtttctcaat tagcctccat agttgcttac caagcattgc 14400
   tgtattaaaa tccataagat ccttaaagcc caaaccacca ttatctttgg tttcacacac 14460
   tttatcccat gatttccaat gcatacctct tgcactaccc cctggactcc accaaaattg 14520
   tgctacagca cccgtcagct tcttaactgt agcttttggt aacctataca cagacatcac 14580
   atggtttggt aaggccgtaa tcaccgattt aataatcacc tcctttccac cttttgtaaa 14640
   aaaacgaaag gtccatccat taaccctttt attcaaccgc tcttgaacaa atccaaacac 14700
   ttgtaccttt atgtatattt aaatttgata ctaatttaaa tttgtatatt aaattttaac 14760
   tttatgaaca aatccaaata ctaatttaaa tttgtatatt aaattttaac atatgtatat 14820
   tttactttga tttgtaaata ttatttggat ttctgaatca actaaaatac ataaactaaa 14880
   taataatata ttttctttgg aaattttaaa tgtatggact attaaatcat gcaaatacta 14940
   tgaaaaacaa attgatctac aattggtata aaactatttc catgtatgag atacaatatt 15000
   gttacgaacc taaaattata gtttattata ctatcaccta ttactgttat ttttgtaaaa 15060
   caatatttta attttagata gaacttcaag attactcttt tggtaactgt tgccttaata 15120
   ttcccggttc tattacatca gccattgaaa taaaatgtta ctaataaagt aagttactgc 15180
   tggtaactaa aattcagaaa ccgagatgac ttaactcttc tattataacg tagatttata 15240
   catttataaa acagatctta cataatcaac ttcttcacat cagactcact tacgtgacct 15300
   ttttctagga tttccttgtg agattgggtt tgttcagttc ctagactcag tttcttcgcc 15360
   tttgaccctt ctattcttct tccataaatt ctcctttcca ttttctcaac tcttgttcta 15420
   cgcctaactc ttcttcttta gctttctcaa tttcttctgc aagtgttttt tgcttcttcc 15480
   tctccaagtt accttgtcta tttctctagc ttcctcgatc tcagaacaac tgttgcaacc 15540
   attgtattgg cttcttcttc agcatcatga gcacgctagc taagcagacg gcggctaatg 15600
   ccaacctctc tgaaccctta gcagccttag tttattccta agttgcaaat aaaatggtct 15660
   ccattgtact tgccccagcc tttgcttgct ctgcttcttc ttgagacttt cgaagctctt 15720
   cagtgaatga ttttgcactg tgacagtgaa aattttttct tcatcagcct ttgtgttgct 15780
   ttctgcggct gcttcggtag ctccgctatt tatcttatgg cttcttttcc ttggactcag 15840
   aaatagctat tctcatgtta tacgaccatc actcgccagc aaaacaaacc tggataaaca 15900
   taaggcattg tttactaaaa gcatgagaaa tgaaactaaa aatcatcttt cttggctttt 15960
   tttcaaaaaa aaatatggta acaaaaccat aaactccgaa ttatcacaat atgcttaatt 16020
   ctatggaggg acatcaagag aatctacgaa tgatatcttt tttgaattct ctgtaatccg 16080
   taaccataag ctatcagtat tgtgaaacac taacatctaa caacctaaaa tcagacaaca 16140
   accccacaaa gttgtagttt tatgacaata caatgaagca tttctctccc accaaccatc 16200
   aaagagtatg caaacctaaa gccaactgaa gcatacacct taagattaaa acttgggaga 16260
   actgtaaagt agacctctgg ttttggggtg actttaacag caacctcctg acccttgagc 16320
   tctctttctt gaacttaggc tagcagagca agtgcaacca aaatgacctc tcccgagctc 16380
   tcccatctga aactgcatac ctctgtattc agaaaacata ctgacaacac gttagtttgt 16440
   agtaactaaa ataaagagag cccagcaaat aattattatt ttacagaacc agacaacatc 16500
   aaaatcagat atattttttt gttaggtaga acttgttgca gttttgaaaa atagtaataa 16560
   gaatttgctc actctttatg atgaagatgt actaagacac cgtccctctg caaactgctg 16620
   ttgatgtcca taatacttgt attgtatcca tccttcaccg tgaagtttgt ttatgtttgg 16680
   cagaatgacg gttagaatgg aaaagtgttt ggttattcaa actctaaggc agctgtatct 16740
   tctttttagg cttgttgagc tttttcttct tctttctaca aaaacatgtc acagactcag 16800
   agttagccaa gttcggtatt actaaagcaa gaggcctaaa caacacagaa ctgcagataa 16860
   gttaattgac ccaccaactt ccactcatct gcctagcact cctcatcctt tactcgtgtg 16920
   tagcttcttc agcctatgcc agacagtatg aagaaatttt tattaggaaa acgttgccac 16980
   gtactaacgc tacaatctta aactttcaca gttagggcct gtaactgttc gactgatatt 17040
   catgtagttt cccatccttg ctacttggtt acatatgtga attagataga acaatcaaca 17100
   aacaaataaa taatcaacaa aaaaataaaa aatctgtaaa acttggttaa atgattcaac 17160
   acaacgcata agttcaataa cataatcaag gataaaaaaa gttggtaagt ggattcacac 17220
   ggcggagtcg ttacaaatat aatcaaattt tttttaatag attgatagga tgctttagat 17280
   tgtagggatg ctaaccttat tatagtggaa cctccactga ttgatttcta gattggatca 17340
   gtaggaaaaa gatgaataaa ttgctccaaa ctctagattc gctactgcgc agattgattg 17400
   atggatcagt gaatggatag aaactttaga atttcttact gcggagaaga gagagcgtga 17460
   aacgataaaa taagaaaagc ggcgtttcaa acccatcata ttatgcttta tatcgatatg 17520
   ggctttaata aagtaaaata cacatacgaa gccaagccca acggaatccg atgaaaaaac 17580
   aaatgaaacg gagcgtttaa taaggtggac acatgttaac gcgagagagc tcgactttcc 17640
   tagctggaat ctgacgtgga gccctcagga gtgaggtgac tctactttat atataaagat 17700
   ttagaaattt aattttcatg tttgtctttt tctttgtaat catatttgtg tttttctttg 17760
   agatcatatt tgtgtataaa ttttaatcaa aatctattta taaaataata tcaatttaaa 17820
   agttgatctg acatacgctc gtatttttgt aatcatatat gttcgtatgc tcgttttttt 17880
   gtaaccatct gtgtctattc tagattttga tctccacttt taaagtgtat atttatttgc 17940
   taaacaaatc aaatttattt gatataaatt tgtatttttg atttttaatt acatttaaat 18000
   gttacaaata aagcattata attgtgcaga ttgtaatatg ttttctaata gaaataattt 18060
   tgatgatgaa atatgtaacc aagttttact aaaatcaaga tatttttgca tttttagtat 18120
   tttgaattat taacgtaatt tataatattt gtactcgaaa acctatgtag catctacctg 18180
   taagtctagc cccgaatccg attaaatacg acctatatac tcaaaaatat tggtttcttg 18240
   tattgatcta aattttgcta aatttaattt ataatataat tttcatatat tttctcgcta 18300
   tttttatatt tgatcataat ccacgataaa aatggtaaat taagtgatct gcatataatt 18360
   tatctcagat tttggattaa attataaaat ttatttttaa aaactccata aaattcggtt 18420
   aaacccaaaa aaaattattg acatgtgatc cgataatggt tgacccgaaa aaaactagat 18480
   aaatctaatg gtcacctata tgaaaatatt atcactacaa gaaaacataa cattaacgac 18540
   ggcgaaattc gtagtaaatt cgtcgtaaaa caggttttac gaggaattag cgaggaaaca 18600
   agtttcgtcg ttattcgttc gtcgtaacgc atatttcctc gctaattcgt cgtaaaatag 18660
   cgagaaacac aattcgtcgt aaagacgaag aacaatattc gtcgtaaaaa ccatgtaacc 18720
   tttccacgta aggaggacgc tagatttcct cgtaaatacc tcgaaagtaa ttcctcgtaa 18780
   attacacata aacctttcca cgtaatatac tcgttaagct ttcctcgtag tgttgccgta 18840
   aaagttttcc tcgtaacttc ttcgcaaact ttccacgtaa cgtagtcgtg ctttaggcgg 18900
   atttgaatgc taccagcaaa tttatagatt ttaagtgttg ttatacatac gtagacatgt 18960
   tctctattta ttaaataaat agtaacaatg tcaattaggt atagaccatg gccatatttt 19020
   tagctaacag ataaaaaaaa tatttgagaa aaataatata tatgtatttc tggtcatgag 19080
   aaaataatat gtgggttaaa tcatttatca tatagtagaa gggagtgggt tccgccggtt 19140
   acaaggaaaa tgatcacctc gtttgtttcg agtaaaaaag ttaggtaact gtcatacctt 19200
   ttataatgtg gtggttacat tcggaaatta aaaaaaggtt gcagttatca tataaaaatg 19260
   tatgtgttgt tgaaataata gtttgaccct acgtttatca attggttaca ctaatagaat 19320
   agatttatca agagtagtat actgtatttt tgttgttacg tttgtttccc gacaaactta 19380
   aattttatta atacgaaagc ataatatctg aatacaaagt tggagttaac cacttaggtg 19440
   gataaacttt acaattttca aaaatagaat ttgttggaca atgtcgaaca attttatgga 19500
   cggcttgatt tccattacgt ttgacaaaaa aaaatgttgt tgtagaaaac ttattcctca 19560
   agctagtaat ttcttcaaga agattttgaa tggttgaatt cgttagttga gcattgatca 19620
   ttttaaccaa gatttctgag tctccttcga aaatcatatt gctatatcct cctatccaaa 19680
   cattctgcat tgccacaagt aaagccaact aaactggaat tagagaaccc caaattagct 19740
   gaaccccaca tataaggatg accataacag ttccaaataa tccatgcacc tcgaacttgg 19800
   ttcgaattta aatcatatgc tgcatcgtaa ttacatttta gaaaaccttg atctcgtcgt 19860
   gtccatgacg ataaagcagg aaaagaagaa attgtgcttg tattgcttac attaggagat 19920
   gataaacata aatgagatat ccattctcta acgtcagcac aagcattgtc cactgtgaca 19980
   tttgggggca gtatttgttt cctaaagaga agaaaattcc tctttttcca tatacgccat 20040
   agcaaccaaa aaggtaacaa ccgttgataa agcgaaagag attgtatgct ttgaatatgc 20100
   agcagaaacc gattttattg tccatataat catttgttaa taatggagcc gtaaaaggta 20160
   gatatgaaaa tctccaaata catattgaat ctgagcacgt acaaaaacaa tggtcaattg 20220
   tctcttctgc caaactacat ctctgacaaa tagaatctaa attcattcct cttgaattta 20280
   gtcgtgtagt tgttccaata gcctttgata gaatacgcca aaaaaatggt taagtttggg 20340
   cattatattt aatttcctaa tcttatcctt tagaatagga tcaccataag gaattggagg 20400
   agcttcaatc aaaaactctg gtgcatgcct agcaacctta tatccagaag acacaggata 20460
   gttttcatct cgtgtatgac tccaaatcaa cttatcttct ttttttgttt ggcttaaata 20520
   tatctggctt acgagacgtt gatcctcatc gcataaataa gactgtagcg ccaccgtatt 20580
   ccaatgtttg taaggtgata ctgtaatcat attactaaca cgtaaatcac aattaattgt 20640
   tgtagaagca ggtcgaggtg gaataacagg taaccaatta tctttggaag ctcgtatatt 20700
   tttgccattt cccacaatat agctacatcc tttctttaaa acctcaatac cagcgagcag 20760
   agttgcctat ccatatgact gcttgcgaca tgtttttcct tctagaaatt gttttccttt 20820
   aagatatcga gctttataaa gtttggaaaa gaggcaattt ggttttgagt agattcacca 20880
   tgcttgtttg gcaagcaaag catcattaaa ttttggtaaa tctttgaatc caaatcttcc 20940
   ttcttttttc gtatactgta gtttttttca agtcatccat ggtaaacctt taagatcatt 21000
   tttaccccac cagaatccca ttaagatggc atctatcttt tgtgttgttg caacagggag 21060
   tttaaaatgt gacatagaat agataggggt tgatacagca accgatttga tcgtaacttc 21120
   tttaccagct gatgaaaaca tacataatta caagactcga gagccaatca agtccaataa 21180
   ggcgtttaat gcggtcaacg cttcttgact tgaggctctt atatgggctt acatcagtgg 21240
   cccattaagg tgattcttgt gtacttttaa gacttgttga atttacacat ttgaggatca 21300
   aacagaaagc taagtatgga gagatccgtt tccttcgatc ttagcggcga taacgaactc 21360
   ccgaatgatc ggagcagcga cgtcggatac accgccaatg atcggaggct cgcttactcc 21420
   cgctcctttc accactccca ctcccacggc ccgcgaacgc ccgcagctaa gccttttctc 21480
   gataggacgg tctccaccat cgatatgccg ccggagatat actctgtcga cggggatgat 21540
   gttttcttcg gggaagggaa agcggcggcg attgggaaag cgtcggcttt gcgtatggtt 21600
   ttggtggttt ttggggtgct gagaaatgga aatcggcaga tgaagagatt gtttctgctg 21660
   atttcgctta acgtggcgta ttctaccaca gagctgttga ttgggttatt gactgggcgt 21720
   gtaggtacga tttggttcca gcttttaaca ttttgttaca cttagttttt agttcgtggt 21780
   aactttgtgt gtgtgggaga gacttttgga tttgttgaag aactaaggtt ttagtagtgg 21840
   ctagagatat agatggaaag tgaaatgaat cagctaaagc cccaagattt tgattccaag 21900
   tcttaatttg atataatctg aatcatgaaa ctaaaccacc acatggatag tagatcttgt 21960
   gcgtgtatcc agggaacttg gctatgcagc tgtaatttct gattatttat taaccttact 22020
   ctcttttttt ggctgtttta tttgactctt acaggtttgg tttccgatgc attccatttg 22080
   acatttggat gtggtctctt gacgttttct ttgtttgcaa tggcgacttc aaggaagaag 22140
   cctgatcatg cttactcgta cgggtaagaa atatgtaaaa tttgatccgc ttagtttgtt 22200
   ttgtattagg gatataggtt ggaatgcatg ctttgtggta gttaacggtt gatttctcaa 22260
   tttgtcaaat tattttttct tttctttctc tctggagcaa ttcctaaagt agattcgcta 22320
   atgttttcgt aggatatagt atttatagtt gctgaatatg aaaccttttc tgtttgattt 22380
   ttttgctgct aatactgtaa ttgcataatg ctgaattggt tggaacttag aaatcgttgc 22440
   cttttttggt taccaggtac aaaagacttg aagttctatc tgctttcact aatgctgtaa 22500
   gtatgttctt aaagtttgtt ggatcatgga tgtcatttcc agttttaatt tgaaggtcta 22560
   atggctgatg tgccatgcaa taaaactggt ttaatatcgt atagaaatta gtgtcctgag 22620
   cttatctcag tgtttgcaca ctttccttta ctgttgttgg tagaatagaa taatcttaca 22680
   gctcgttaaa aatgttgatt atcttttcat tttgccttca tcactgtctc attcatttta 22740
   tcttttgggt taacacattg atatgttcag tattgacata tgtgtagaca gctgtttctt 22800
   atgttcatgt cgttctcctt agctgtggaa gctcttcatg catttgttca agatgaatca 22860
   gagcacaagt aagtttcttt ccccaagtga catgcctaag agagctcgta gttatttctc 22920
   tagcacttac taagtttaca ctaagttctt tgcattaata tgatgtattg aaattgactt 22980
   catgcaggca ttatcttatt gtatcagcgg taacaaatct gctggtgaac ctacttggtg 23040
   tttggttctt ccggaattat gctcgtatga atattggtat gtattctctg ttttataatt 23100
   atgcttaagt tctggttaga cttgaatgaa ctgatgccaa tactggtgat ttatatcata 23160
   tagccctgtt tggcggcttt ggtatatttc gtttgtaaaa actggccacc cctgtcaatg 23220
   taaagcaaca aagaagcaaa gggtttctaa ttaagataga ataagtagga ggattagtta 23280
   aaatagagag acagaagcat gatgactgaa tctggccttc tgtacatatt caaaactcac 23340
   atttttcttg aatgtgtttc ttaaattcat aaaatgatgc agtgtacaga aaagcagaag 23400
   atatgaacta ccactccgtt tgcttgcatg tcatatcaga ttccatccgc aggtgtgata 23460
   ttctttttgg ctttctttat ctcaagcaga gccgcagata gctagataga aaaatgtttc 23520
   tctttgcatg gaatttacat acatcccaca tgacatattc gctgcttctg tcttttcagt 23580
   gcaggtctga tactggcatc ctggctcctc tctctggggt aatgctcact tcctttaaaa 23640
   ggaacaaaat gaataaaaat gtcttattac ctgacctctt acattttcca tttttctcct 23700
   ccggtctttg gatttttcag ggttgaaaat gcagaggtcc tatgtttggg attggtatca 23760
   gttacagtgt ttatgcttgt tatgccactc ttcaaagcca ctggtggcgt tttgcttcag 23820
   atggcacctc caaacattcc ttcttccgca ttaagtaaat gcttgcgtca ggttcgctct 23880
   gttccataaa gtgtttcctg aacgtgtaca tatagatcga tcaacctggg tttctcttca 23940
   actatcttta tttacagatt acttctcgag aggatgtcat agaggtttta caggcacgtt 24000
   tctgggaggt tgtgcccggt cacactgttg gctcactcag aatccaggta ctagcttcct 24060
   tactgttttc atatcggtta aaacacgaat ccataataag aattgtactg attttgagct 24120
   ggttatttgt gtttaggtga agagcgggat agatgaaagg cctttactgc aatatgtgta 24180
   tgatgtatac catgatttgg gtgtacaaga cttgacgctg caaacagact acagctgagc 24240
   tgcatctact tctatgtttt caaatactga ggactttgga tgtatactag tagagattat 24300
   ggttctataa gaagatagca cttggtttca agcttgcgag tttcttgtta catttgttta 24360
   gttttatttt ttttaattcc ttttcccatt cgtcttttga accacacaca caaggaccac 24420
   aatctttgta aactctttta gtctttcaag tttgtgcgtc tttttggctc aaattctcct 24480
   ttttaatcct ttttcatgta aaaaaatcat ctgcaaaatg taaaatcaaa acggtagcac 24540
   gtaaaattgt cagctgaaac aagattttat tgttcttctt gtttacacag aaaaaactaa 24600
   gtggaggaat caaagatcat attgtgctcc tcaacctaca cagcctaata ttctgcaagt 24660
   tatggggaca gtacaagcat cttaaaaagc aaattcatcc gtccatgtct atgttattct 24720
   ttgcttaccc ccgtttgctt cgttgatcat tgtaaattta acatagttta ctaaacgata 24780
   caaaaatttg taaacataaa aaaaatgata caaaaattta atatagttta ctaattgact 24840
   acgtgcacat gacaaaggaa tttttattaa tggaacttga aaatatattt tggatttaac 24900
   tgaactacaa aaacgcaaga tatatatgaa aagtatctca attatttccc gtaacgagtc 24960
   aacaatttca aagtatacaa tacccacata tgatacgatc cattatataa tccatgattg 25020
   cgttttttgg actctaagct agaaaaatgt atattaataa tatggtatat catatttaat 25080
   taaggcacta cttgtgtggt ctgctttatt gttagttaaa cgtataaaat agaataataa 25140
   acaagaacca attgtccaaa aagacacttt tatttgaaaa aatactattt ttaatgttat 25200
   tgtttggatg attcaaatca agagacgacc agagaatatg agcatgaatc atcaaaccat 25260
   taactcttaa cctttcccct taaaaaaaat tcccagttga taagatagaa tcaaaagcag 25320
   aaaaaaggta gagttaaaca atataataac taaaaaacaa agaagattgt aagataaagc 25380
   tgatgaagtt cacattacaa gaagagaatc tgcttattac aaaagagaga gatataagag 25440
   aatacactgc taagtctctg gtttcttctt gataacactt tcactatgta cagattcagc 25500
   aacacagtga ttggagtctt aaacctcctc agcttactag cttcaatacc aatcatagga 25560
   gccgctctat ggaaggcaag aagcagcaca acttgcgaaa acttcctcca gactcctcta 25620
   ctcgttatag gtttcatcat actcttagta tctctcgccg gattcatagg agcctgtttc 25680
   aacgtggcat gggctctttg ggtttactta gtggtcatga tcttcctcat cgcgactctt 25740
   atgggtctaa ctctgtttgg tctggtggtg acgagccaag gaggcggtgt ggaagtacca 25800
   gggagggttt ataaagagta taggcttggt gattatcatc catggttgag agagagagtt 25860
   agagatcctc agtattggat ctctataaga agctgtatct tgagttccaa gacttgtgct 25920
   aagattgaat cttggactac acttgattat ttccaaagag acatgacttc tgttcaggta 25980
   tcatcctcag tgcctagagc catgttaaaa aaaattaacc gtagattttt atatatgtct 26040
   aaagttttta ttgatttata agtattaata actccagtta tatatgtttt ttttt 26095
<210> 5
   <211> 1161
   <212> DNA
   <213> Brassica napus
<400> 5
   atgggtgcag gtggaagaat gcaagtgtct cctccctcca aaaagtctga aaccgacaac      60
   atcaagcgcg taccctgcga gacaccgccc ttcactgtcg gagaactcaa gaaagcaatc     120
   ccaccgcact gtttcaaacg ctcgatccct cgctctttct cctacctcat ctgggacatc     180
   atcatagcct cctgcttcta ctacgtcgcc accacttact tccctctcct ccctcaccct     240
   ctctcctact tcgcctggcc tctctactgg gcctgccagg gctgcgtcct aaccggcgtc     300
   tgggtcatag cccacgagtg cggccaccac gccttcagcg actaccagtg gctggacgac     360
   accgtcggcc tcatcttcca ctccttcctc ctcgtccctt acttctcctg gaagtacagt     420
   catcgacgcc accattccaa cactggctcc ctcgagagag acgaagtgtt tgtccccaag     480
   aagaagtcag acatcaagtg gtacggcaag tacctcaaca accctttggg acgcaccgtg     540
   atgttaacgg ttcagttcac tctcggctgg cctttgtact tagccttcaa cgtctcgggg     600
   agaccttacg acggcggctt cgcttgccat ttccacccca acgctcccat ctacaacgac     660
   cgtgagcgtc tccagatata catctccgac gctggcatcc tcgccgtctg ctacggtctc     720
   taccgctacg ctgctgtcca aggagttgcc tcgatggtct gcttctacgg agttcctctt     780
   ctgattgtca acgggttctt agttttgatc acttacttgc agcacacgca tccttccctg     840
   cctcactatg actcgtctga gtgggattgg ttgaggggag ctttggccac cgttgacaga     900
   gactacggaa tcttgaacaa ggtcttccac aatatcacgg acacgcacgt ggcgcatcac     960
   ctgttctcga ccatgccgca ttatcacgcg atggaagcta cgaaggcgat aaagccgata    1020
   ctgggagagt attatcagtt cgatgggacg ccggtggtta aggcgatgtg gagggaggcg    1080
   aaggagtgta tctatgtgga accggacagg caaggtgaga agaaaggtgt gttctggtac    1140
   aacaataagt tatcttgcta a                                              1161
<210> 6
   <211> 1134
   <212> DNA
   <213> Brassica napus
<400> 6
   atgggtgcag gtggaagaat gcaagtctct cctccctcca gctcccccgg aaccaacacc      60
   ctcaaacgcg tcccctgcga gacaccacca ttcactctcg gagacctcaa gaaagcaatc     120
   ccacctcact gcttcaaacg ctccatccca cgctccttct cctcttcgac atcatcatct     180
   cctcctcggc tcctccctct accacctctc cacagcctac ttccctctcc cttacctcgc     240
   ctgacccctc tactgggcct gccaaggctg cgtcctaacg ggcctctggg tcatagccca     300
   cgagtgcggc caccacgcct tcagcgacca ccagtggctg gacgacgccg ccggcctcgt     360
   cttccactcc ttcctcctcg tcccgtactt ctcctggaag tacatccatg acgccaccat     420
   tccaacaccg gatccctcga tagggacgaa gtgttcgtcc ccaagaagaa atccgacatc     480
   aagtggtacg gcaagtacct caacaacccg ctaggacgca cggtgatgct aaccgtccag     540
   ttcaagctcg gctggccgtt gtacttagcc ttcaacgtct cgggaagacc ttacagcgac     600
   ggtttcgctt gccatttcca cccgaacgct cccatctaca acgaccgcga gcgtctccag     660
   atatacatct ctgacgctgg cgtcctctcc gtatgttacg gtctctaccg ttacgctgct     720
   tcgcgaggag tagcctctgt ggtctgtgtc tacggagttc cgcttctaat tgtcaactgt     780
   ttcctcgtct tgatcactta cttgcagcac acgcaccctt cgctgcctca ctatgattct     840
   tccgagtggg attggttgag aggagctttg gctactgtgg atagagacta tggaatcttg     900
   aacaaggtgt tccataacat cacggacacg cacgtggcgc atcatctgtt ctcgacgatg     960
   ccgcattata acgcgatgga agcgaccaag gcgataaagc cgatactttg gagagtatta    1020
   ccagtttgat ggaacgccgg cggttaaggc gatgtggagg gaggcgaagg agtgtatcta    1080
   tgttgaaccg gataggcaag gtgagaagaa aggtgtgttc tggtacaaca ataa          1134
<210> 7
   <211> 1161
   <212> DNA
   <213> Brassica napus
<400> 7
   atgggcgcag gtggaagaat gcaagtctct cctccctcca gctcccccga aaccaaaacc 60
   ctcaaacgcg tcccctgcga gacaccaccc ttcactctcg gagacctcaa gaaagcaatc 120
   ccacctcact gcttcaaacg ctccatccct cgctccttct cctacctcct cttcgacatc 180
   ctcgtctcct cctccctcta ccacctctcc acagcctact tccctctcct cccccaccct 240
   ctcccttacc tcgcctggcc cctctactgg gcctgccaag gctgcgtcct aacgggcctc 300
   tgggtcatcg cccacgaatg cggccaccac gccttcagcg accaccagtg gctggacgac 360
   gccgtgggcc tcgtcttcca ctccttcctc ctcgtccctt acttctcctg gaagtacagc 420
   catcgacgcc accattccaa caccggatcc ctcgagaggg atgaagtgtt cgtccccaag 480
   aagaaatccg acatcaagtg gtacggaaag tacctcaaca acccgctagg acgcacggtg 540
   atgctaaccg tccagttcac gctcggctgg ccgttgtact tagccttcaa cgtctctgga 600
   agaccttaca gcgacggttt cgcttgccat ttccacccga acgctcccat ctacaacgac 660
   cgcgagcgtc tccagatata catctctgac gctggcgtcc tctccgtatg ttacggtctc 720
   taccgctacg ctggttcgcg aggagtggcc tcgatggtct gtgtctacgg agttccgctt 780
   atgattgtca actgtttcct cgtcttgatc acttacttgc agcacacgca cccttcgctg 840
   cctcactatg attcttcgga gtgggattgg ttgagaggag ctttggctac tgtggataga 900
   gactatggaa tcttgaacaa ggtgtttcat aacatcacgg acacgcacgt ggcgcatcat 960
   ctgttctcga cgatgccgca ttataacgcg atggaagcga ccaaggcgat aaagccgata 1020
   cttggagagt attaccagtt tgatggaacg ccggtggtta aggcgatgtg gagggaggcg 1080
   aaggagtgta tctatgttga accggatagg caaggtgaga agaaaggtgt gttctggtac 1140
   aacaataagt tatgaggatg a 1161
<210> 8
   <211> 1137
   <212> DNA
   <213> Brassica napus
<400> 8
   atgggtgcag gtggaagaat gcaagtgtct cctccctcca agaagtctga aaccgacacc      60
   atcaagcgcg taccctgcga gacaccgccc ttcactgtcg gagaactcaa gaaagcaatc     120
   ccaccgcact gtttcaaacg ctcgatccct cgctctttct cctacctcat ctgggacatc     180
   atcatagcct cctgcttcta ctacgtcgcc accacttact tccctctcct ccctcaccct     240
   ctctcctact tcgcctggcc tctctactgg gcctgccaag ggtgcgtcct aaccggcgtc     300
   tgggtcatag cccacgagtg cggccaccac gccttcagcg actaccagtg gcttgacgac     360
   accgtcggtc tcatcttcca ctccttcctc ctcgtccctt acttctcctg gaagtacagt     420
   catcgacgcc accattccaa cactggctcc ctcgagagag acgaagtgtt tgtccccaag     480
   aagaagtcag acatcaagtg gtacggcaag tacctcaaca accctttggg acgcaccgtg     540
   atgttaacgg ttcagttcac tctcggctgg ccgttgtact tagccttcaa cgtctcggga     600
   agaccttacg acggcggctt cgcttgccat ttccacccca acgctcccat ctacaacgac     660
   cgcgagcgtc tccagatata catctccgac gctggcatcc tcgccgtctg ctacggtctc     720
   ttccgttacg ccgccgcgca gggagtggcc tcgatggtct gcttctacgg agtcccgctt     780
   ctgattgtca atggtttcct cgtgttgatc acttacttgc agcacacgca tccttccctg     840
   cctcactacg attcgtccga gtgggattgg ttgaggggag ctttggctac cgttgacaga     900
   gactacggaa tcttgaacaa ggtcttccac aatattaccg acacgcacgt ggcgcatcat     960
   ctgttctcca cgatgccgca ttatcacgcg atggaagcta ccaaggcgat aaagccgata    1020
   ctgggagagt attatcagtt cgatgggacg ccggtggtta aggcgatgtg gagggaggcg    1080
   aaggagtgta tctatgtgga accggacagg caaggtgaga agaaaggtgt gttctgg       1137
<210> 9
   <211> 20890
   <212> DNA
   <213> Brassica napus
<400> 9
   aattgttgta ttttttaaat ataattctca aaaattctat tttaagaaag ttttcatttt 60
   tacttaaaaa tattgtagat ttgaagttgt ggtttagtaa tttggataac aataattttt 120
   gttgatagat gaaaaacaga agaagatcac gattcgttca cacattccca actcacatta 180
   cactaacacc tttgagtaaa gtgagccatt ttattatatt catgcctacc aggctaccaa 240
   tatctgtaaa gctctcctca aataaatagc aagcataaga ttttgattat atcccagtag 300
   aaaaactaga ctttatcttc taaataatca ttaagcatgc taatgactta gttacaaaga 360
   gggtagatca aaagaaatgg atttgatgaa gctctgggaa gcttagtaag agcattggtg 420
   attctctggt agttcttgtt aacatttttg tcttctcggt gactctcaac ttcaacatca 480
   accttgacag tttcaagaca tttcaaattt cccaagaaat gcttctctga ttcagctctc 540
   tacgagttcc tctataccct gaaaccttta gcaccttcac ttgacatgtc gataaacaac 600
   atacttcctc ctccttattc ttcctagcga cgcacacaca ggcgtccccg catctatctg 660
   taactcggtg cacaagaccc tgaaaagcaa aattcatgta acaacaacaa tcaaacgaat 720
   tgtgtgtgtg tctatgtatc aaaacgaaac tggtacctcc tactttgatg acaagagttt 780
   ctaggtttgg agagttgttg agaagaagtg gcaccacttg ccatcctttt tctttgtcac 840
   tctcaaaaga cagagtaagg agcttgtgaa agaccggcat tgatttacag tataggtgaa 900
   acacctggag atttattact attatttatc acaaaccaaa aaaaaaatgc aataactaat 960
   aacactaaga ctttgcactt cagattgaca caactagcag aaggaaagat aacaaaacta 1020
   acctcaagag acgtaggaga caagtcaagg actttgtttc tataacgtgg atgtactcgc 1080
   tttctaagtg aagctccgag aagcttcttc gttccaggac agtgcggatc cagttgtcca 1140
   ctcgagaagc gtcgtgagtg tgctcacagc gcaaagagaa tcttttgatg attgaagagt 1200
   tggttaggag agctagtgtt ttgtcgacga agtcagggaa gccacgtgga tcaccagttg 1260
   cagttgcgtc gtcgctcaga tcgaggctgt ctacgaggga aagcagattc ctccaccttt 1320
   tggacagaac caatgtggaa gctgcttgat ttgtcggaag caaggacagg actttgccaa 1380
   gaacctcatc tgggagactg cttattgaat ctcgttgggg agacatatat taaggtttaa 1440
   gatcgaacca gaaacttgtc gattaaaggt cacaagttca gaacaatcga agaaaggagc 1500
   aaacgaacga aggtaggtga acttacaatt agaaaggaac cgacgacgag ggagcgaaac 1560
   gcagcgtttg acgtggtatt tctaattgtg taatatttat ttttaaaaaa tgtgatttct 1620
   tttaaaaaaa gttttacaaa agttgatagg tttcggggca taataattgg gttaattgca 1680
   gtgaggatgg gagtaaaatt gagtttgcaa aagtgaggcg gtaaatttgt atggttctgc 1740
   atagttgaaa ataaataagt ttatcatgtg tttataattg tttagttata aagtagcgac 1800
   taaataaaat aaaaatgatc attttataat atatagctat aaaatagtaa aattagaata 1860
   ttatacttag aatataagat atattaattt gatataacta gtaataaatt atttgtataa 1920
   tgtttgttta ttttgaaaat tttggtttat cccactatat aaaagaagct aaatttgagc 1980
   ttcataaggc tatccacatg tgcacaaata ttcaggacca accaaagtgc catgtcatct 2040
   ttgtgagctt gcaattttaa aaaaatttgt cacctacgtg gcccgtatga cccatctctc 2100
   ccgagcctct cttcatacca tattggtcgc agcccattac ccatctcttg atacggttcg 2160
   ggttatatcg ctgtcctctc tgaaatatca aaatcactaa ccctaatcac cgttctcgat 2220
   ctctttgtcg attctcttcc tcccccaaac tcatcccgat ctctttgtcg attctctttc 2280
   tctccccaac tcatcccgca taacgtcccc gatgagagtg ctggtacttc aatgtgctct 2340
   cataaagcct tcaatgtttt cttcaaccat gctgttgcct gttggtgatg ttttcaattt 2400
   aatatgcgga gaggatgaga tcgacttggg gagaggaggt tattgttcgg aattgaaacc 2460
   cgaaaatgga tttcacaatc gtaagctctc acttctttgg gctcgcttcg tcttcttaaa 2520
   gaggctttat cggctgcgtt gaggctgtat cgtccggtgc cggagttctt caaccccggc 2580
   ttgaagggcg acaagataca acatgggact tttgtttcgc agtgatgttt ttgatttact 2640
   tggctgggag gatgagatcg actctgggag attttgtttc gagttcaaac cggagggatt 2700
   gactttgaaa tcgtacgctc ttagtataca taattatggg ccaatacaca cagattacga 2760
   tacaaacaca aacacgaagc tcagcattag agtttcagcc ccggagattc aacagcaact 2820
   aaagtaagat tccaaattcg tcctctgttc agcctccagt caatttcttt tactttttta 2880
   atctttgcct aatgttcatt actgtgatca aaaataactc gcttgcatat gtcttctttt 2940
   tcaggttgtt acttggcttt ctatttcatg aagcacagaa cgtagtatat aaaaggaaac 3000
   aggaatacac tttgcaaata ttctctgtgt ctttggatta atctataatc ttgtgatgta 3060
   gatagataca aaagcttctt acggatctcc atggagaatt catgaaggta acctgaaaca 3120
   actctctatc tcttgcaagt ggatgccaga ctaatgtcat agtttggtaa aattccagat 3180
   taagttttgg tgaatgactt tgtgttttgt acagaagata agaactcatg ttcgttatgg 3240
   gaagcagtta tcacaataca accttactta tcgaattttc atcaagtaat attacatgat 3300
   ttataattag ttgtgtattt tatgacattt tataagtgtg gttgacgata aaaatgacaa 3360
   ggctatcaca aaagatacct caagttcagg tattttagat atgggactct ctggaccttt 3420
   aatatgtgat aatgtatcga agttttaagt cttcttccaa caatactcta attcgatttt 3480
   gtggtgtatc gatacatttc ctgaagggct tactcggaag cttccagtta ccaacaagta 3540
   tgtgaagcca atatgtatag gatttggagg ggcggaggac cacgaccttg aaaatctgaa 3600
   gaaacagctt gaagatgatg atctcatcag aggtacaata actgcggaac atcaaggcag 3660
   tgaaggtaca attttacctg tgcatgtcaa aaccgaactc tgtagccatc tccctacacc 3720
   ggtttagtca taactgtcat ttgattaaca aacagagtct ggtgttaatt agctgataca 3780
   aaagacaatc gcgcatacag ctgagagggt cacgtggtct aagtcttgaa ttaacgtttg 3840
   agttgttctg ttcagtgaca aaggcttctg tccattccaa atcaagcagg tacacatatg 3900
   aatccggtcc tgtgtttaga atcaagaaac aaagttcctt cgcgtcaaag gcttgtgtgc 3960
   gagtctcttc agtgctctct ttggctttct tatgttcgat tcacacaagt attggtcttc 4020
   cacaacaaag actcatccac attattacat cttctgctat aaaccttttc ttttacctct 4080
   aggctcattg tcaataccaa aatacagctg cgttttgacc ttgattaggt gtgattgtga 4140
   ctctctttca cttcctcgat gcacatggct acacttttct ttgcggtggt tgagatgtcg 4200
   atagacataa tcactcttgg gaaaatcaag ggactgctca gcatgggtcg cctcttttgc 4260
   ttgaaatatt ggagaccaat gagttagagt ttagagacat caattggtag attcatacaa 4320
   tataagctta gagttttgtt tcttctttgt ttttccggtt gattggtttt aagaaatgga 4380
   atcctttctc tcaaaagact ataagcatat ttagtgtcag atggcttgat gattcttcga 4440
   ttttgaaacc agaaatctat tttcctgcca aatgcttctt tgttattgtt acatagtgga 4500
   gtgtttaaaa cattactaaa ccaattccgt caaattttaa tagaacgaag caaaacgatt 4560
   agaaccagtt gtatttttat atctttgtaa aactcagctt ctcaggatca atcttatcac 4620
   tacgaatcat cattctataa aagaagatga agtcggattt ggaaagcgtt tggtaatttt 4680
   tagaagtttg agagaaggta atagaagttg tattaaatag tggatatagt ggacgtttga 4740
   attaagtttg tacacttctc ggattgatac atttattcac gttttgaaat tgaacacgtc 4800
   tattcattaa acacgttccc aaagtcttag aaacaaatac attatcaatt caaatcccat 4860
   tagaataagt tattgttcat acgttctaaa tatttaataa taaattaaac aacaaatttt 4920
   ttatatctac aaaattttca tcataacata agtattttta tcacgtaaat taaattgaaa 4980
   tgcatttgaa atatttagta agaattaaat atccagtttt ttaatatcac aaaaaaatat 5040
   cttttatcac gtaaaaactt gaaaacatcc atgtataaaa ttatatacaa tctgtataga 5100
   gatttatctc ttttgaaaaa atattaaaaa ttatatgatg taaaatatat tttaatgata 5160
   acacaataca aactatatat aatgataatt atcaaatcaa taaaattcat ttctaattta 5220
   tggttaagta tatattaaca aatttaatta tttattaaag ttaataaaga ctttgtaaca 5280
   cagtataatt tagttttgga caatgataat tatcaaatta atattttaaa aattttatgg 5340
   ttacttatat attaacaaat ctaattattc attaagaata ataaatattt tagccgctct 5400
   acattttaaa gtgaaagttt agaagatgaa aaaactcact ccataaataa tattataaat 5460
   tatttaaaat aaacataaat aaatgattaa atataagttt gattataaca aacaatccgc 5520
   gcagggcgcg gataaaagat ctagtaatta gtaataagtt atttgtataa catgaaattg 5580
   agtatttgaa acaaatattt atgttttaga tatttatatt tattaactac ataaatatgt 5640
   attccaaata ctcaatttca tacttaaata tgtatgttaa atgcccagtt agatgtaaat 5700
   acacattttc ccttatgtgt tgcttttttt tttaacttat gctatatccg caatggccgt 5760
   atatattttt caaagttttg ctaattagta aaacttttga aatataaata aattttaaga 5820
   taataattta aattaaagta atatatatat cgaattttaa tttattatat taaagttttt 5880
   ggtttaaatt tccagcgttt aatttttttt tggtaaagta acagttaaaa cccattaatg 5940
   gaaagtattt tcaccgcctt tgagatcttt tcctcagtat taatttccct agacgaagca 6000
   attccaaaac caaaaacata ataacacata ttcattgctt ttaccaaaaa aaaaaacaca 6060
   tattcattgc atgctttaat taccagaaaa cgaataaaaa tctcatttac gttccaaaaa 6120
   caaagtacac acaaaaagaa cttctagaag aaaaaacgta taaacacgtg tctctataca 6180
   gagtgagaac aggacaaaca aagctggaca gggttttaag taccgtataa accctcgact 6240
   acgaacacaa aacagtttca aaagtaaggg taatattgtc atttagttag ccttcaaata 6300
   atgttgcccc ggggatcatg gacgctttat attcagctta cacatattta tctaactgaa 6360
   tcactcaaga aaataaatca cacagacgtt ttttaaggag agaaacaaac ctctctctct 6420
   ctctcagatc ggagaaaaga gccatggcgg ctgcgtggaa cgggagtgag tatttcgaca 6480
   tcgacgttga gaccggtaga caatcgttcg cgcggccgtc gaacgccgag actgtcgagc 6540
   aagacgaaga agatctgaga tgggcagccg taggaaggtt accgtcgcag agacaaggga 6600
   gccatctatc ggttctgcgt cggtcgcaaa cgtcgcaggc gcagacttct ggctacgcag 6660
   acgggaacgt cgtgcagacc attgacgtta ggaagcttga tcggtctgat cgtgagatgg 6720
   ttgttcgtca ggcactcgcc actagcgatc aggataatta caagctcctc tccgccatta 6780
   aagaacgtct cgataggttt gtttctattt ttataggttt gttttgatta ttgatattcg 6840
   atggatcttt gatataatct tggtgttgtt ttatttgtag agttggaatg gaagttccca 6900
   agattgaagt ccggtttgag catttgaatg ttgaagctga tgttcaagct ggtacaagag 6960
   ctttacctac tttggttaac gtatctcgtg atttcattga ggtttgtctc ctcttttttt 7020
   gactatcttg ttccacacgt aaccttttgt ttctaatatt gtatctcttt gtttgtgttg 7080
   ttgcagcgtc tcttaagcag cttgaggata atgaagacta gaaaacacaa gctaacaatc 7140
   ttgaaagata tcagtgggat tatcaaacca ggaaggtgaa tgaaatacaa tgttttgatt 7200
   attataacta tgtaacacaa acactaacag tttatatatt ttgctgttct tgaaggatga 7260
   ctttgctact aggaccaccc ggttcgggga agtcgacttt acttcttgct ctcgcaggga 7320
   agcttgataa aagtttgaag gttagttaat taacccgtga aattatctaa tatgctcata 7380
   tatatatcac atgtttgata tctcttttgt tagtattcac atgtatcttg agattcatct 7440
   ttttatttgt tataaattta tttttatttt tacagaaaac gggtaacatc acttacaatg 7500
   gagagaatct tgatgagttc catgttaaaa ggacttcagc atatattagt caaacagata 7560
   atcacattgc tgaactcact gttcgtgaga cacttgattt tgctgcgaga tgtcagggtg 7620
   caagcgaagg atttgcaggt tagtatttac actttactat attaacttct gaaattgacg 7680
   tgtcctcaag tgtttcttgt ttacattata ggttacatga aagatctaac ccgattagag 7740
   aaagagaggg gtatacatcc ttcttctgaa attgatgctt tcatgaaggt cagcatcata 7800
   tacctcctaa cttcctttta ctagtttata atttataagc cacaatcacc aacactttct 7860
   tcaaatttgt tataggctgc ttctgtcagt ggtagtaagc atagcgtttc cacggattat 7920
   gtgcttagag tgcttggtct tgatgtatgt tcagatacaa tggttggtaa tgatatgatg 7980
   agaggtgttt caggaggtca aaggaaaaga gtgacaacag gtctctttca ctctctttaa 8040
   acctctctat tttcacttat ccattagtct aacttataaa tcttgatgca ggggagatga 8100
   ctgttggtcc aagaaagact ttgtttatgg atgaaatatc tactggtctt gatagctcaa 8160
   caactttcca gattgtgaaa tgtgttagaa actttgtcca tctaatggat ggaactgttc 8220
   ttatggcact tcttcagcct gcaccagaaa catttgatct ttttgacgat ttgattcttc 8280
   tatcagaagg ttacatggtt tatcaaggtc ctcgagaaga tgtggtggga tttttcgagt 8340
   ctctaggatt ccgtctccca ccacgtaaag gtgttgcaga ttttctccaa gaggtatcat 8400
   acatcctaat ccttttcttt ggttatattc atgacaagat ctgagttttt ggaaattata 8460
   aacattttta aataaattta ataaaaaaga aatatatatt ttttaatttg agaacctata 8520
   ctatgtaaaa aacttcctaa aactttggag gccaaggcct ggttatattg ttacatggta 8580
   gtccaaaaat atattcttat gttttataat gttgttatgc atgcaggtga cgtccaaaaa 8640
   ggatcaagct cagtactggg cagatccttc taagccttac cagttcattc ctgtctcgga 8700
   catagcagct gctttccgca actcgaatta cgggcatgct gcagattcaa aactggcaac 8760
   accatttaat aagtcatctg cggatccttc agctttgtgc cgaacacagt ttgccatatc 8820
   aggatgggag aaccttaaag tttgcttcga acgagagata ctattgatca accgtcacag 8880
   gtttctttac acgtttagga catgtcaggt attataataa ctctacgtat tttgattttc 8940
   attacatcta tttgttgcat aacttctatg tttctgacat ggaacatctt gtatgaaggt 9000
   tgcatttgtg ggatttgtta cagccacggt gtttttgaga actagattac acccaacaaa 9060
   cgaagcatat ggaaacgagt atctgtcttg tcttttcttt ggcctagtac acatgatgtt 9120
   caatggtttc tctgaactgc ctctcatgat atcgcgtctc ccagttttct acaagcaaag 9180
   ggataactcg tttcatccag cttggtcctg gtctattgct agctggatct tgcgtgtgcc 9240
   ttactctatc cttgaagctg ttgtctggac ttgtgtcgta tactatagtg tgggacttgc 9300
   tccctcagca ggcaggttgg tcatttttct agacatcctt ctttttattt tatggtttca 9360
   atgtcagaaa ataaaaaaaa tctttttgtt cttttaggtt tttccgatac atgttactcc 9420
   tcttctcggt gcatcaaatg gctctaggtt tgtttcgtat gctggcttct gtagcaaggg 9480
   acatggtcat tgctaataca ttcggatctg catcaatctt ggcagtgttc ttgcttggag 9540
   gattcgttat tccaaaaggt tggttattac tactttactt catacataat aagaattgct 9600
   atactaaaac cctcgcattt tttgacagat gatattaaac cctggtggac ttggggcttt 9660
   tggatatcac ctttatcata tgggcaacgt gccattgcgg tcaatgaatt cacagccacg 9720
   aggtggatgc aggtgtgctc aataatctca tatctaagtt aatataatac ttaagagtat 9780
   atacaaatgc ttaacaatag actttttctt gcacatcaag cagccatcag ctatatcgaa 9840
   tactacaatt ggattcaact ttctcaagct acgaagtttc ccaacaaatg acaactggta 9900
   ttggattgga gttggtgtac tcatttgtta tgcacttctc ttcaacaaca ttgtcactct 9960
   cgccttggct taccttaacc gtgagattct ttctattatt atctaatgat catttcttgt 10020
   atatatatca ctgtagcaat atattgtgaa gctttttgtc tttttttctt actcttgcag 10080
   ctctaaaaaa ggctcgagca gttgttttag aagatctcaa tgaagaaacc caaactgctt 10140
   cagtatcaaa tgcaagacaa ggtagaagtg agaagaaagg aatgattctt ccgttcaaac 10200
   cattaacaat gactttccac aacgttaact attatgttga catgccaaag gttacattca 10260
   cttcctttgt atataacagt cctaatatat ggttacataa ttatattttt tttggaatgt 10320
   caggaaatgc gttctcaagg tgtaccagag actagactac aactgttatc aaacgtgagt 10380
   ggagtcttct cccctggcgt tcttacagct ttggttggat caagtggtgc tggaaaaact 10440
   acattgatgg atgttcttgc gggtcgaaag acgggtggat ataccgaggg agatatcaga 10500
   atctctggtt accaaaaaga acaacaaaca tttgctagaa tctctggata cgttgagcaa 10560
   aacgatatac attctcctca agtcacagtt gaagagtccc tttggttctc tgctaggctt 10620
   cgtcttccta aagatatcag caaagaaaag aaaaaggtaa gtatgaaaaa agattaactc 10680
   attttgttcc tatttaaaca gttttactag taatatgttt ttgtgtgttt gttaggaatt 10740
   tgtggaggaa gttatgagac tagtggagct tgatagtcta agatatgcat tagtaggttt 10800
   acctggtaca acaggactgt ctacagaaca aaggaaacgt ctaacaatag cggttgagtt 10860
   agttgcaaat ccatcgataa ttttcatgga tgaaccaaca tctggacttg atgcaagagc 10920
   agctgcaatt gttatgagaa ctgttaggaa cactgttgac actggtagaa cagtggtttg 10980
   caccattcat caacctagta ttgacatttt cgaggctttt gacgaggttt gccctaagat 11040
   ttcttgggtt acaagaaata ttatcaaccg gtgatcttaa cgtgtgttct tttttgccta 11100
   cagctgcttc taatgaaacg aggaggacag gttatatatg gcgggaaatt aggtgaacac 11160
   tcgcaggtta tggtagacta ctttcaggta ctttgtcttg gccttctcta catagttgct 11220
   tgtcacccaa gaaaactatt atttcaaacc ctaaactttc tacagggtat taatggagtc 11280
   cctggaatct caagtggcta caacccagca acatggatgc ttgaagtaac cacacctgct 11340
   ttggaggaga aatatagcat ggactttgca gatttataca aaaaatctga acagtttagg 11400
   taactatcac attacctaca ttttccaatc tcttttaaaa attattataa taaactgatc 11460
   tttaaccatt tacagagaag tggaggcaaa catcaagcaa ctcagtgttc caccagaagg 11520
   ctcagagcca ataaagttcg actcaatata ttcacaaaac caactctctc agtttctact 11580
   ctgcctctgg aaacagaacc ttgtctactg gagaagtcca gaatacaatc ttgtgagact 11640
   gatcttcaca acggtcgctg ctattatact cggcacggtc ttctgggaca ttggtaccaa 11700
   gagaacttcc acacaagatt tggtcactat aatgggagct ctttactcgg cttgcttgtt 11760
   tcttggagtt agtaatgctt catcagtaca accgatcgtt tcgatcgaaa gaacggtttt 11820
   ctatagagag aaagcggcgg gaatgtatgg tccaatccca tatgcagcag ctcaagggct 11880
   tgtggagata ccttacattc tcacccaaac cattctctat ggtgtcatca catacttcac 11940
   cattggtttt gaaagaacgt tgagtaagtt tgttctctac ttggtgttca tgttcctcac 12000
   tttcacctac ttcaccttct acggcatgat ggcggttggt ctcaccccga atcagcactt 12060
   agctgctgtg atctcctctg cgttttactc tctatggaat ctcctatctg gtttcctcgt 12120
   ccaaaaacct gtaagtatat tccactctat caagtgaaaa tgtagttaag atggagaaat 12180
   gagtgatcag ttgtgtataa tgttgttgtt gtttcagttg attccagtgt ggtggatatg 12240
   gttctattac atatgtccag tggcgtggac acttcaagga gtgatcctct cacagcttgg 12300
   tgacgtggag agcatcatca aggagccaat gttccatggc acggtcaagc agtttattga 12360
   acagtacttt gggtttaagc cagatatgat aggtgtatcg gctgcagttc ttgtcggatt 12420
   ttgcgctctc ttcttctctg gattcgcact ttcagtcaaa ttcctcaatt tccagagaag 12480
   atagaagaca agaacaaagg atattttgac tctttcttat gttagcatca ctcacgtgac 12540
   aaacttttca tgtttttggc tctttctcac attttagtta gctttctttt ctattttacc 12600
   actgatttag agttagtttt gttgacattg acgtaaaata aacctaaata tatatataaa 12660
   gaaactgttt ttctctgttt agaaatttct ttgcttttgt aattttttgt ttagttgtta 12720
   aaagccttgt ctcaaatact atatgagaaa cggctaaaaa gaatctctgt catcttactt 12780
   actccacacg aaattgttta tatacaagtt taaccgatat gctaaaccta gatacacaat 12840
   tttataataa aggaatgtag atatgttact ctatgattct tacatgagtc tccctaataa 12900
   tactatgttt attatgcctt gctttctttg tttatctctg ctcttagaac aaacaacctt 12960
   gatttgttgg gtctccttta gagggacgtc gttgtttttt ttggccaagg agactttttt 13020
   ttttgaacta ccggctcaag gagacttaac acagctaaca gagtgtctat gaatagcaat 13080
   gagtgtaaag tgatgtcttt gcaaatggta gcctcaagag ccctagcatc tccaatggga 13140
   cacaaaaatt tactctatat ttcactctaa aatagagtaa ctctattata gagttgaatt 13200
   tgcttcaata gttcactcta taatagagta actctattat agagtgaaat atagagtatt 13260
   tttgtttttt tactctatat ttggagtaaa aaagcaacaa tactctatat ttcactctat 13320
   tatagagtaa ctctattata gaataaacca ttggagcaaa ttcaactcta taatagagtt 13380
   actctatttt aaagtgaaat atagagtaaa tttttgtgtc ccattggaga tgctctaagt 13440
   ggtagcctca tttgagaata gaatatgctg tcttggtgtt tccactttgt taatatctct 13500
   tgtggaggtt ttgaatatac aaatgtcaga gctgttactc ttattttatt tttaatttat 13560
   tttatcattt tgttgtattg agcgaccaac ctataagagt acgattatga tttggagtct 13620
   gacactcgtt ttctctcttg catcaaataa aactaggaat acaaatttga aaatactgta 13680
   ttgaaagaac caaaatctct atttaaatcc aacataggac gaatgaaaat tttctaaaat 13740
   tatgtaggaa cagttttacg agcaacacta atagtaatat ctttattatt atttggtcaa 13800
   atgatacata ctaaagggtc aatttgtaat taaaaaaaaa gaaactaaaa agaacttcaa 13860
   aatcttttta gatatatttt tagattgtgc aaaaaaaata tatttttttt agatatatca 13920
   cagtcatgcg catcagaaag gcttatatat atttgggccg taaagtattg tccatcactt 13980
   aaaaaagcga caactccgtg acattattgt tgtgctggga cccaaaaacg gcgtgcattt 14040
   tgtcgactct ctcagtcgaa ctttttcttt tgtccccacc aacaaaaagt ttttaagacc 14100
   tttatttatt gtaactaaaa acataaagaa aacgaacaaa aacttgattt gtaatgtaaa 14160
   tacatttaat taaaaaaagt ttcacgagta catttaactt aaaaacaacc agaaataagt 14220
   aaaaaccaaa ggactgtttt attcctaaat agagctagga agaaaggtta gttgattttg 14280
   gatttgtcag aagcataaac gtagagatct ggatctgtct cgtagaagac aatatcacca 14340
   gtgtcactga cgtaatgatc tttcttaata cttgccacca aactttccac caagtggatc 14400
   ggtattgctc ctgacgtctt tggttctctg tagtatcttc ccaacacatg tttagctgat 14460
   ttcgtctgtc gcatatcatt aattaagatc actaatttag taattaatca ccctttaatt 14520
   ttaatcaaat gaaactagag agagagcgag atcactcacg gcatcaacca agtgatagtg 14580
   agggatttgt gggaaaagat gatggatcac gtgagttcca atatcgtgat gaatgttgtt 14640
   gaagatcccg taatctctat caatagttgt taatcctcca cgtaaataac tccattccta 14700
   ttattgtaca caaaacatca ataatttaga ttaatcaaat actaatcatt gttgcttctt 14760
   ataaattaat gttgatctac ttaccttgcc tctgtaccaa ggcaacttat catcgtgacc 14820
   atgatgatgc aagtacgtga cagcgtccaa ccacattaca aagatctgaa atttttccaa 14880
   aactttatgt caaaaacaaa ttatattagc aatgatataa taatgaaata tatgaaactt 14940
   acaatgtaag gaacaccata gacttttaga actgtgactg gaccaacgag gaatgataga 15000
   taaacaagag tggccaacat gatcgaccag caagtagttg aagttgcaat aagctttctc 15060
   tcgcttgggg caaataaact actgtatggg ttataatgtg acccttcttt accaggactt 15120
   ctgtaccact gtagtcatcc ccaaacaaat ttaatttata tttagttaat actcaaaatc 15180
   taaaaattca aaattgtaat tataatcagg aagaaaaatg aggaattagg atttaccaga 15240
   tagagagggt aagcgagcat ggggagaggg acagtgtatc tgagcatccg tgtactgtgg 15300
   gacaaattct tgtataattt ttctggcaac tggaatgcaa aattaagatt aaaatgttaa 15360
   ttaatattta acagtatggt tatatattcg aatttattca ttgcatgtgg tgtgtttata 15420
   agtttttttc tttttattag ttctacgtaa actacaaaac tgaaaaatac taagaaaagt 15480
   aaacgaattt cgagaagaat cattttatgc caatggctcg aatataagtg gcccgttgtt 15540
   aaagttaact acagtaccat aaacaattta aatcagttgt ttactacagc taaacgacaa 15600
   atctgacaag tggtcgtcca agcctcacac tggaaaaagg attgattaaa ataaatacat 15660
   agaattctaa gaaaattaaa atgaaagagt ttcaaaaaaa gaaaaaaaaa taatgagagg 15720
   gggattaccg gaacccaaga ctcgtcgttt tcaacatggc catggttctg gtggtgtgtc 15780
   cgatggctta ttctcctgca accaccccca attataaaat aaactattat tttattttca 15840
   taaaaatgaa attggaattg tcaataacat atcgttttcg aggcagatgc taagaatctc 15900
   actcgtttaa ctacgttatt cattttttga gcaacaaaca aatgtatcta ggaaaatgat 15960
   gcatgttcgt agatatttca agctgatgta tccatttaac aataaaataa gccattaaaa 16020
   caaatatata taaatattat attaaactta tacattaatt tattcaagga catgtcatat 16080
   gataatagct aattggacca taaataggcc catagcatta aataaaagtt tggttctttt 16140
   ttcttcgatg ctaaagattt tgatgctttt agtcacatgc attattttac tatgaaaaat 16200
   taatatattt tcagttatca gattactgtt tgctaacatg caccaagaat gacaaggaaa 16260
   atgtaagaaa tacgaaaaca agaataaatt tgcatgaaaa aagaagttaa aataaatgac 16320
   ttaccaacca tggtatggaa cgagaatgaa ggaatgaaga atatgaccaa ccgcagtatt 16380
   cagaagagga atgtctgaga agctcccatg tccactgtat tattcaaatg aaattttaca 16440
   tcataaacac atttatcatt tattgcacaa tgttaactga actttcctca attcaaacgt 16500
   ttcaacaagg taacaaaaat agaatatgac gtgtcacatg actatatttc gaaaatagat 16560
   tggaacaaca cacaataatt aaaagaacca ataaacagta attaaattgt tacagaaaac 16620
   aataaaatgt gttttattga aaatttcaaa cgtagatcca taaaacgcgg aaacaacaat 16680
   aattatagga aagaaaaaga tgtttagtta ggagtgttac gactgatgaa aaagaaccaa 16740
   aaaaaaacaa aagaattaaa aatcttagat ccccttttgc ttttaaaata ggccaaattg 16800
   gatgaacata ataattaaaa ttgaaaaaag taaacctgaa gagaatcaaa tcttgaagtc 16860
   agtgaaaatc tcatatcgaa cgtacggtca agaaatcaaa gacaatgcaa aaaacgaaaa 16920
   aaacatataa acatatcaaa attaagaagt tgaaagaaaa attaaattac cagtcgtggc 16980
   cgagtacgaa gatagcccag aacagggttc cttgggcggc ccaataaaga ggccaaaaga 17040
   accagctatc aaaatacacg gcggcgacgg caagagccac gacggcgaaa atgtctctgg 17100
   cgacatagct catggatctc aaaggactct ttacccaaca atgcttagga atggccgccc 17160
   ttatatctcc gatcttgaac ggtggttgtg cgctcggatc aaacctttcg tctccgttcg 17220
   cattgctacg ctggtccata gcgacaacca tcgccggaga aagagagaga gctttgaggg 17280
   atgtttctct ctctctctaa aactgtgtgg gctctgagtg aaatgtggtg aagaaagggt 17340
   ctgatggact ttggggtatg tgtggtttgt ttatatagag ggagaagatg tgtagagaca 17400
   ccaaactgtt ttctattttt cttaatttaa gaaacttatt tatttctttg aagaataaaa 17460
   agtgtatttt tgcggtaacc tgtgcgcaat gtatctttgt tacgtcgttc atttcgatga 17520
   aaactaagtt agagaaatgt gttacaaaaa aaaaggcaat gctataaaat ttccagaaga 17580
   ttagaaattg cgttattaag tataaggatt ataccaaatt gcattatttt ccttagaaat 17640
   aaggattata ccaaatgaat tgttaatgtt tcgtactttt actggatatt tatgcactga 17700
   aatggtagtc ctttttggga cttaaacaac ttgtatgatt tttacaattt agcaaaagaa 17760
   aaatacatgt agtcgaaaat atttttttta gtcttcaata tatagttttt tgctaaaatt 17820
   tcctcgatta tgtattaatc ataaaaaacg atctatatcg atatcatata gacagtagat 17880
   atgacaacat ttatatggat ttaaaaaaac gtattaatgt gagggaaaat agttgccaca 17940
   tcactgtgat gtatttgact taagaaacag acttccatca gtttatttat ttgagacgac 18000
   ttgattaaat tggcagtcta tacaatagta caatgtatag gtaactttaa ttttatcaaa 18060
   aaatttgtgt aaccaatcaa atttaatatt agttatattt tatagttggt tgaataattt 18120
   ttaatttata attttaataa tatattttag ataaaaaata ttttttaata aacgtgtttt 18180
   atctatagaa tatcttatat ttagaaacgg agagagtata acatatgtat atgagaatca 18240
   gttggattta acaaattcac tagatccgga aataacacca ccaaaataga acaagatcaa 18300
   aacatgatgt agggtctgaa tgaattgctt aaaaatggta taatattcca attatgttta 18360
   ttagtactta taaaattagt gatcggttta actttttaac atataactaa ctttgactgc 18420
   tgaatatggt gtcttgatca aaaaagacat ttgtggttag tcaatgagac atcatatttt 18480
   agaaatgcag gcaagatggc gtttcctcta cctctttttc tctcttaaat caatttccca 18540
   acacgtcttt acgagttaag catcaactaa ttgctacaat tgtatacaga tttgacctac 18600
   ttgcctccat taactacatt tcaggctata tgttagtgta tatgtaggca ttaattataa 18660
   atacgcattt caactgagct tcaatgcata tattcaaatt ttttgttgga atgatttccc 18720
   catctttaag aatcgggtag tgaagactga ggacgtgaac cgtgggttta ctgttttatt 18780
   aactctacct atatcagttt ttaatattca attttatatg agaaatcgat taatattact 18840
   ataatacaaa cattgttttc ctccgttata ttatggtttt tgtcactgaa tttgaacatg 18900
   atttgagaca gagaccaaac aatatatgac gtctgtatac ttaatcaaaa tatgagaaga 18960
   ttatatgcac tctatcttta aacgtgagat ctccaaaact gtcataaaaa cgtgaactcg 19020
   tttcttcttc caataacaaa tatcaatatt gttcatccaa ttccttcctc cataaaaacg 19080
   tgaacacctt tcttcttcca atcgtaatat catgtgttgt tcatccagtt ccttcctcca 19140
   caagctttct atcgaacgga acagtctgaa accgtgttaa acaaatcacc ctggaagatg 19200
   taatccagct tctgtgagag ttttgaagaa ggaagatctc tttttgtaac gcaaacattt 19260
   aattttcctc atatgtgatt cgatgatgtt tgataattaa aaatgtgatg gccttaatga 19320
   ataatcttgg tcatgttttt agtaaccact atttcttcta gcagtcatca aaacaatttt 19380
   tttttataat gttgatttat tatgatatta attatgaaaa atattacata gacgattcga 19440
   caaccgacaa tactacatgt cttatgagga tctacttcta actgtattat ctgagccgtc 19500
   ctacgaatat ccactcctga ctagatttac ttgcaccatg ttgaagattc cttgtaagtt 19560
   tttcttctgt agtctgcatt aataaatcgt tatattcgga acttgaaaca tggatttcct 19620
   gtaatctgca aataattgca tagtctggga ctcgaactcc aaacctgact gtataagtct 19680
   ttaaacctta actaataggc tatggtgctt ccacgatcat caaaacagtt taccacatga 19740
   gattatatat gacgttggat aacatgtatg attaatttat taaagactca ttaataaaaa 19800
   tttaactgta gttttttttt tgaataaaca tagtttcctc gatctcaaag aatctacaat 19860
   ttaaaattca aatgtttctc taaaaaatga agtaattcca caatataatt gagtttactc 19920
   aaatcgtaat tcattgttag agtgaaaata aagtaataaa taaaaaatac ttttttattt 19980
   gaaatgccat tttaaagtaa attacgaagt tggatggaaa atattttaat tactcaaaat 20040
   tttataacta tatatctagg tgagccatgg aaaaggaaag gtacaaaatg atgagtgtgg 20100
   gcgtagacat gaagcctgca cgtgagagtt gtagctattc gacaaacata tactaatttg 20160
   ttgcgtacca tttccacttt atatatattt atatatttgt gtgtgttgag ctgagatatg 20220
   agaataaaaa ttgagaatat acctcaaaaa tgcaaagaga agtatgtgtt tgttatttag 20280
   cagacgcaca tggtggagga catcctcgtg agttccgaag ggctaagtta tacagcttta 20340
   accgagctaa ttaattcatc gtccttacat aatttgagca ctatttgaag aagacagagt 20400
   atatatacat attagttaat acagttatat atgatccaat tttctttgtt tgacaacaat 20460
   gtgttttcaa acaaagaccc tgtaactttt tttgacccgg ttctgatata tgtatgtgaa 20520
   tatgtgattc atatatttct ctaactacga gtacgactaa atgtgcttat caattatcat 20580
   acacgtctct acgtgcttct ctatcttata ttcttggtat taaccattcg tattttatga 20640
   acattcgtgt acgttgaaag gaatcattac gtagatgccc acgatgttac ccaagttgga 20700
   gaattatgtt atttagaaaa cccattttta attacgctaa ttaccaaaaa taatatgaag 20760
   aatggggccg tgggaatatg ctttcggtag gttttgcgtt ctaaatttac atagcatagg 20820
   cagtcaacag ataagaggtt aaatgtatat tagaccgaaa tatttttaac gtgttggggg 20880
   gtgggggggg 20890
<210> 10
   <211> 105998
   <212> DNA
   <213> Brassica napus
<400> 10
   acaactaata ctatatctat tcaacaaaaa aaaaaaaact acccaataaa aaacatttca      60
   attgcaataa tgaagataat gatatgactt cagaacaaca tcgtcccatc ttaaacccaa     120
   tatgatgtca tctttagatg ataaaatatt ttgattttta tttttagcct tttattagaa     180
   agaaaattaa ctgtaataaa ttatacaaat tgaaaaatat ccttacaatt ttattaagct     240
   tcaatcagac attaaatttt ttgtggttac aaatttttgc agattatttc aaggaacatg     300
   caaaatattc atcaactaat aaattattta aacaaacata ttaactcatc tattatatta     360
   aattaggaac atgatctatt aatatatgtt ttgtgctact attttgatat aattattaaa     420
   acattttact aatcacttaa aaaaatattt tacaaaaata taattataaa aaaacaccaa     480
   tacgattaaa cacacaaata gaaaaattag tttaaaaaat gtaaaacaga aaatataccc     540
   gctctttgaa gagcgggtca gaatctagtt cagtgataaa ttagaatatg ttacttttga     600
   cgttaaaaca aacagaatat tttttgaaca ctagaatatt tgaatatgtt aattggtatt     660
   gatgtttttt tgaaaaaata aaaaaaataa aaattaactc tgtgcttttc tacagtaata     720
   gaatcgatgc ctcgagtcga gatacaacca tctggtgcac taaaacttgg gaccattaag     780
   ataagaacca agcaaattgc atattacatt atatatatat ataaatatat atatatatat     840
   gtatataaaa attacacaga agttgtcact attaattaaa gatatctttg tcaatatttt     900
   aatggtgaca tcaataattc ttttttgggg ttcttcatcc agctgccagt attcaaaaaa     960
   aaaaatctct gaaaatattt cattgattag aaagaaatat acatcagcaa aattagcata    1020
   tcaacaaaag aataatcaag gctaatggaa cataacatca aaacaaagaa ctagctaaaa    1080
   cagaggagga tctctcattg tcaaaaagat aagagtgccc agaaggatgg ctgagagcaa    1140
   cataagatcg cattcggttt tgaaaaatca catgttgaac gatgagatga gcttctctgt    1200
   tagcagtatg atgttcgcat acaaccttcc attgatccaa attttccaaa aggagattaa    1260
   gctccaccac tttgaagcta aaggatgacc acgctcttgg tctattaatt gcacccacta    1320
   aggttcttcc catctaagtc aaaccaaacc ttcaaaagct tatgactaaa tactttcaat    1380
   atatagtcca cacaggagag atgaaatgag cttcattttc cgaacccacg gacctgaaag    1440
   cccctcggct atgcatagac aagtcaagat attatcggca tcatgccatc ttttgcatac    1500
   cacacgtcgg caacaaaata tttccaaata aacatattta agcacaataa ttacatcttt 1560
   ttggttaata acgagaatct gttgatagta tggttgaatt agaataaatc ttgcttttct 1620
   tcttacattt tctcaccaaa attcaacatg cacgcataaa gtgtgtaaac tgtttagtat 1680
   aaaatttcac gcgaagttcg tgtgaaattg gaaaacagac ctaactctgt cttaattctt 1740
   gcaaatgcag cttgattttg aagctaaatc ctttaacttg tggttaacgt tgattaccaa 1800
   aaaaaacatg tggttaacgt gtaaattaac aacccattta gtggtgaacc taactcagcc 1860
   atgtcgcttt ataattaggt caatattaat tacagaactt caattcactt ggttcactta 1920
   cctttgattt tccttgttcc acgactcttt ttttttgttt tttttttcat acaagaaact 1980
   cagatggcac atttttaaag aagagttgaa aggaaaatga acaagcataa atttggtttt 2040
   tttcaaccga agaacattat aagtcaagtt ttgaattatc attaacatgt ttcttattat 2100
   ggaatagcca tacacattcg gagttcggtc atatgtatca tacatgcgtg ggaacaagaa 2160
   tattcgtaga caactaattt taaaaatgtg acgtaaatgt caaactatta gggtatgaat 2220
   ggtgaccaag gaatgacgag gaacaaatgc attccctaac attccttaca aaaatcacca 2280
   ttcataagga ataatttttc cttctcattc cctaccattc ctttttatgt agagaaataa 2340
   agaacaaatt aattccttgt taaatatgag atggaacaac cattcccttt cattcctgca 2400
   attttattcc tctacattcc tttcctattc gttcctcttg tttccagaat ggttaccagt 2460
   cggaccctta gaaaaaatct tacgatattt tttattgaaa gatgacgttt cttttttctg 2520
   gagcatgaat attcatatat ctataggact cctgttgaca attaaaaact atcttaggcc 2580
   ggggtatttt gatcggattt aagtctatgt atttttattt ataccaatgg gccgggttac 2640
   cgtttaagtt taggacaatt tattaaaaat aatccaaaat ttgaaaaatg aaaatctata 2700
   catgaaattc aatcaaaagt aaaattaatc taaaataata gtgcaattac aactattttt 2760
   atgactaaaa atatataaag acttttaaaa ttatagtctt gaaaattttg cacgggacaa 2820
   tgaaatcttc tgagaagtct agttataaat ctaattataa ttactttata aatttttgag 2880
   aaaaaaatac tttgattgga aataaattga aatcatgtga atataaaaaa ttctaaataa 2940
   tgtaaattga aagttaataa gaataaataa ttatgataca tataagtgaa tatatattgt 3000
   aagtcattat tgtccattgg ttagaatttt tgttacatat tcaagtaata tttatatagt 3060
   tatattttgg attcttaaat attttttgaa attaaaattt tgcatataag tgttaaattt 3120
   tgtgtatatt aaacatttta caaattgatt ttattttaat aaagaggtat ttctagttat 3180
   ttcaattatt tgattttagg ctctaaggat aagagacttc gtaaagaatt cggctaagtt 3240
   attttgtttc ctatccgttt tgatttttta aatctctttg ttgtgaaatc tgttattaaa 3300
   tatagcataa tttaaaaata attgtaaata taaaacaagt aacaagtatg aaaaaattgc 3360
   tacctggcta tatattactc gaagacatta ttattgctat gaaaagtaaa taatattcat 3420
   atatagaaac atccatgttt ttgctcatta ttatcttctg taatgtgcac aaatattact 3480
   tttagagtga ttcctgtact cttatagtag agtaaacaaa aaaaatcata tttttttaat 3540
   agtattattt aacaaataat tttcaaaaac tctatatact tctaaataac tgacaagtat 3600
   ttacaaagca agtaacagaa catgacataa tgtacaagta aatcattatt aagcaagtgc 3660
   ttgaaataac aaatgattgt ttttaacaaa caaaatacaa atcaaaattt tgatgactcg 3720
   tgtaaaacaa gtcaaatatc aaatcaacac aataattagg tttggttttc tccagagcat 3780
   gacacctcag attttaaaat agatgtggga ttgacatgtc atcaaatgaa aaaagttgag 3840
   taaccaatct aattattttg tacgtccaca tcagtttcgc tcagccgtaa tgagaaaaaa 3900
   aaattacgaa gattacttta cctatctctt ctcctctgtc tctcctcttg ctctgtttcc 3960
   tcctctcctt actcaatttt tttcagacgt gggctggtca aaccccaacc cttgcaaatg 4020
   ggacaccgtc caatgagacg ggaacagctg cgtcacgagg aaccagctca gacagaaggg 4080
   gatccgcagc actctccctc cggatctcca taaactctcc gagcttgtcg tcctcgagac 4140
   tcacgtcaga cggtcagaga agacagagca gcggcgtcgg agaagacaga gcagcggggt 4200
   cggagccgag ctcgtcgtcc tcgagactca cgagtcagac gtcggagaaa acagagcagc 4260
   ggggtcggag ccgagctcgt cgtcctcgag actcatcaga cagacgtcgg agaagacagt 4320
   gcagcggcgt cggcgaagaa aaagagcagt ggcgtggtcg gcgaagaaga gcagcagcgt 4380
   cggagatggt actgaagcgg ggctgattga cggcgtaggt tgaagaagag ctttgtttga 4440
   cagctgaatt aggtttaatc aattggttta gttaataaac caatttgtaa ttgtaaccaa 4500
   tttttaattg taaaccatgt atccaaattt cgtatcgtaa agaaatacca atttataatc 4560
   cgatttagtt acaaataaac ttttatttta tgtttttttt aaaataatta aaagaagtaa 4620
   atactataaa attaataatt ttaaaataaa taataacaaa aaaaatgata ttaaataata 4680
   tttaatggca gacaaaaaag agaattacac tatgatatca ctaaaaaaag tttctgtcac 4740
   aaataaaaaa tatagactct aaagattgaa atgatcaaaa tgtttcatta aagagttaaa 4800
   tatacattta tatctctagg gttaactaat tcaaatttta gagtttaaag ttaaaagtgg 4860
   agatttgaga ttgagattta aaattttata aaacaaaaaa taaatattaa aaataaaaaa 4920
   tttaaaaata gtttcaaaaa ttattttcga attacaaaaa gaaaatttca aaaaaaaatt 4980
   aataaaaaaa ttcgaatttg aaaacatata atctaaaact ataacaaaat ttttttttaa 5040
   atttttttaa tttattttaa ttttattttt tatatatcta tggtgttagg gtccttttac 5100
   ctattaaata aaatattttg gtcattttct tccttgtggt ctatttttgt gaccaaaaat 5160
   tgaaaatgat ctttttagaa gaattgctct acaaaaaatg ctatattaat cataaaaaat 5220
   taatcataaa aagtaatgct tatcattata ggataaattt ttataatatc tattagtgtg 5280
   tatgcttttt gaaattgttt aagcaaattt gatacactat caagagctgc agaaattaca 5340
   ttatcaaatt agattaatac taattctgta aaaatacaaa atttatataa tatctttgtc 5400
   cgcggcgtag cgcgggtatt aacctagtat aagtaaaata gcaaatatca ggtctgtgtg 5460
   tgttcccata ttagatggtt ggtccatctg actttgaaag gtactggatt tgattttgat 5520
   acccagatgg cgatgtttta aaataattga attattaatc tagttcctaa taattaatat 5580
   acgaaatttt gttattcagc taataattaa tcaagtttct ggaagatttt ctttcgggaa 5640
   gattgcacat aatgactttc actattaaaa actcgtttca tagctagaac attttttata 5700
   tattttgtac ttttattcat ggctgattta caacatgttt tctatatatt ttgaaaacta 5760
   acttttatag caaattacta actagtttat tatttctttt tccaaatata ttgagaaaat 5820
   ttgatcaaaa tgtaaactag ttttccagaa tcatatataa catcggaaat atatcagaat 5880
   atatatagtg ttacaaattt aattataaat tttcaaaaac taattagtct ttctatggga 5940
   atagaaaaac agacaagtcc caaaggtttt tttttttgac aaagggttaa gtcccaaagt 6000
   tacgatgata aataatgtta caaatgtgtc accaaatttg tgagaaacat tgctaaggca 6060
   tcagcagtct accaattatc aaacgcatga aaactattct catgatctta aaaatggcga 6120
   acaaaatgaa ctcaacaaga ttttgattgt ggcctgaaac gatcagcttt tatgatactc 6180
   ttataatatc acaagatttt gattgtgggt gatcgtactg atctaatatt agttttatca 6240
   agtttgcaga acttttctta tcgatttcgc tattgagttt acacttacct gctgtatagt 6300
   attcacatcg ccgagaaggt aaataaacag tactcttatg tttttttgtt tttttggtaa 6360
   aatcagtact ctatgttgca aaaatgtgcg actgattcat gtttggcttt acatttttgc 6420
   ttcggtagaa atcagaaagc aagtgaatag taaaaaatgg ttcgtatcaa gttggtgtaa 6480
   aattttgtga ttgattgaac aatttaattc tgtcgagttc acattgctgc tagcctggta 6540
   caaactctcc aataatttaa agaaacgtaa atggactgga catatgcatg cacacggttg 6600
   ggaattattt aagaaaatgt aactcaacaa ccaaatcttg taagtgtcct gtcatttggt 6660
   ggggtccatc cgtaccatct cttaaaataa aactcaaagt acatgcatgt aaaagtggat 6720
   cggaataatt gcactcccaa aacaaacaaa ggctactaga aaatatatgc aaaaataaaa 6780
   gaacagaaag aagaaaaagt gagattgcgt gtgtaaaaag taaagtagcc agaaaaaaaa 6840
   gaagaaaaaa gtacaaaagc gtccctttga tagatgtatt gtgttcaaag ttctgtatga 6900
   tgtttctatg aaatttctag atttgatacc ataatcaata tactcggatc cgatagacct 6960
   cataggaagg ttctctgaac actttaacaa ctagtataag aaatagagtt caatacaaaa 7020
   tattaaatta caaataaggt ttattgttta gtttcgtttt agacattcgt atctaattat 7080
   aatttatagt ctttggtaga ttgatcaggt taaaaggcct acatgtgaca aatcagcatc 7140
   atgcattaat gggttcccaa tttttgcgat ccagtttagt aaaagtcaga ttaaagccaa 7200
   tgccactatc accccaagaa taccatcatg ggtcctgtca actaatgtga cacatgaccg 7260
   aacctgaatc gttatttgtc ccattgtaat aattcacaat ctagagggct tatccatacc 7320
   atatctaagc cggtcttgtc gcttcattcc atttttacca ttttactgac taattataag 7380
   agttctatct accccctaat ttttttttta aattgatcat ttattaggcc gattgtaacg 7440
   ttatgaacat tccaacccgg tccatcctga tctgatcaga tagctaggtg tcggtcatat 7500
   cacaactagt gcttggttgt ggatcaaacc cgacaaccca cagattgagt gatttttttt 7560
   attcaagaaa tttgacttgt ttaacccgca acaagaaaat ataaaatcta catccgtccg 7620
   cttaaacttg cggatgaccc acaagtaatt ttaataataa taaaattgtt attttaaata 7680
   ttttttaaaa taataacaaa aatcaattta taattaaaat taaatatttt ttattaataa 7740
   tattttcttc ctaatttttt gtggttcaat ttcggctgac ccgcataaaa aactcttgac 7800
   ccttacccgc atccgccaat caactttttt tcaaatcact cgaccgcaac aaccacgcgg 7860
   cggatccaac agggcagaac ccgccaataa tgactcaaat atctctagcc caatattgat 7920
   cggatgatcc ggttttgaag ttcttataga actgcaatac actaatctaa tgtaacacgc 7980
   cttgtttaat aaaaaagaca caatccaatg catacatttt gaaaaatcaa aacaaaggga 8040
   tattctttcc acataacgaa tcccaaaaca accccagaac ctctcatatg tgtcacatgt 8100
   gatactcttg tgacactaac atataaactc gacacgactc agaaagtgaa catgatgaca 8160
   ctgacagaac atgtatcaat ttcaagaaaa agaaaagaaa gccaagttat gcgatggatt 8220
   taaaacatat caggctgtaa attaactagc ctcgtgtgtt tgttgtatca atgcatgcat 8280
   cttacgcaga gggcacagac tcgtcggttt tcttttcttc aacagctttc tctgcctctg 8340
   ctgatgctac cttctcgact ggtttctctt ccttcttctc ctcggtgtcc ttctcttcca 8400
   ctgtcaactt ctcaagaaga ccagcagtat cagaagcctc tttactctct tctttctctt 8460
   cttcagaatc ggtaacttcc ttgaactttt gcataaatgc tttgcagtct gataacggta 8520
   ttgtaaacaa gacttgatta tttatacaag gtagaaagat ttttaaaaac acaaacaata 8580
   aatcacatga aacagtttcc aatactacac gtctactaca ccagctcatt cccaagctac 8640
   actgagacag ccaatataaa cactagagct ttcgactgct tttagtctat ccagatcata 8700
   cacttgaacc tacacaaatg tccagatacc tctagttgga ttgccacctt taaaaggcta 8760
   ccattaagta aacaaccatg atacccttca acaattactg attagtgcaa agggaaaata 8820
   actcaaaaac aagtaaaaac tctaaaatgg gtgatactat aagcttgtag aactgaacat 8880
   ccaaaacttg tgaccatatg caaatatcac caagttcaag atcctccagt gacctaagcc 8940
   ataaatttac attatctaca ctctagaccc cggaaaaaaa ggattagtca tgctcttaag 9000
   gtttaagcta ctataagtat gccacacata cacaagggaa aaatccagac tcactctcaa 9060
   ctgacgcaaa gtggattaaa aaaaggttta agctttgggg aacatcctaa agatttaagc 9120
   tactataaac tatgctacaa tgagaacagt gctaccacaa gcaacaatga gatccaaaca 9180
   cacacaatga gactgattcg aactcactct caaccgaagc aaaccggata cagaaaagct 9240
   catccttcaa ctccccatcg gagaaatcac gagcgtgcca cacacaagac ttatcattcc 9300
   cagcgtgttc ctgaacactc atccccgacg taactgcaca taaggagaag aatcaaacaa 9360
   tgagaataac acaaacacag atccatcatt aaaaaatagt cacgatcggt accgagatga 9420
   ttagcacaga tcttgagagt tttggactgc ctcataacga gacggatctt cccagactcc 9480
   ttatgcttca agaacttgac cgtaccagcg cctctctcct tccactgact cccatcttta 9540
   tcgaacctat acagcttcga tttcctaaca gattcgcaaa aaaaaacaat aatcaacaac 9600
   cacgatcaga tctagaaccg atctagtagg aggagtagag tttacagatc gaggattgcg 9660
   tcttcgtttt cttcgccggt agtgacggcg acttcttcga gtttgatgat gggagcgacc 9720
   tgagcgccgg tgtcttcgtc ctcgttggct ccggactctt cttcgtctct gtgctcgcgc 9780
   tccggctcgt tgctgatgct cgccatctta tcagatcaga tcgaagcttt gctggttgtt 9840
   gttgttggat tacagagtgg gcgtaggagc tagctagatt ggaggagaga atgttgggag 9900
   agtttctgtt gacggaaaat gatttgtttt tttataagag agagagacgg cgctttgttg 9960
   gaaatggatc tttgatttaa atgggcctac gtcacgttta ttccggagag ctgaatatgg 10020
   tggactgtac tggatccatt ctggaaagct gagtatgcag agctcaaatt gaattaattt 10080
   gattaggggg catatgcatt tgtcttttca aatcggaatt tgagttagtt cttatcaaag 10140
   aaagttcaag aaatctgtaa gagatagttc tgcgtttctt aagaaattat tgattatgta 10200
   aattagaccg atttttttaa tatttaaacc atttttttag gaaaaaatgt ttcgtttaaa 10260
   tactgcttag gcggacgtcc aactgttgcg aacataattt tttagaaaac tggttcttat 10320
   catatatgat tttcagatag aaacgttttg aatacattcc atggaatttc cgattggttg 10380
   tactcaggtt tcaaatcagt tccaattttt tttatacatg taaatatttg aaaaacatat 10440
   tagtcctttt tcttggatac tttgggaaat tctttaaatt tatcttgtta caattatttt 10500
   gttacaacta gatcataaat aaaaataatc atgagtatac ggattttggg actgaatgtt 10560
   tcaaacaaaa aaaatttaga ttaatatttg ttcacaaaat ttcaaacaca agactaaact 10620
   tgacattttt tttcctaacc gaatccagtt aaaccagtat gctaaagtca aatatgacac 10680
   aacaagaaca tcatgtgtcg aaagattcag gtagtccagt ttaaaactaa gataatatat 10740
   ttcatgaaga tagtgtttca aaaggggaaa acaaaagaaa aagtcgctag gaaagttgaa 10800
   aatgtccaaa atgttccaaa cccaaattga gaaaaaacca catccacatt ccctcagata 10860
   gaccaccaaa ccagcctgag aaaaacgatt cttttgaaag aagactttaa ttcagtaaag 10920
   gaaacagcga aacatatcat tccagaacgg tgacttgttc ccactctcca tcaacagcct 10980
   ctttccacac cgtcacgttg ttgttcccat cggacacggc caacatgtta cctgtcaacg 11040
   accacgacac ccgccacact ggagtcataa agtccttcag aatcttacct tcccattgct 11100
   caccttcttt ccccacagtc catatgatca ctttcccatc ctgtgagcca ctggctatgg 11160
   tggacttagg gagacccaag ttcggtgccc aagccacatc acgaacccaa tcagtatgct 11220
   tctgaagagc cggaaagcaa tccatcttcc acgacccgtt tgagagcttc cacactttca 11280
   cagtattatc acacccaccg gaagccagct tgtaaaccgg atcaagcaag ccagagctga 11340
   caagagcacc aggggaagtg gcaggtgccc atgagacaga agtgactcca acaggatgcg 11400
   cttggtcaat cttcgtcgtg tcccagccac catcagcacg gcctgtgaat accgaaatgt 11460
   ttccgtcgga tgacccacaa gccaaggata gtccgaggtc atgaggagcc caagcgatgg 11520
   agttgacaga agatttatgg tccgtgaaga catgagcttg ggtccactgg ttttggctgc 11580
   cttctttcca gagtatgacc tgaccgtcat aggagcatga agcaaggaat gatccaaact 11640
   tagggtgggc ccacgcgacc tgccagacag gaccacggtg gccggttaat gtagctaggt 11700
   gctgggatcc accgttgttg ctgactccgg ttatcttgat ggtgcagtca gatgaggcag 11760
   ttgcaactct ctttccgtag tagtccattt gcacatcatg gaccatgtct tcatgacctg 11820
   tttcgatctt ctgacccggc atgtttccgg attgactttc tctgcttctt aaaagaaaac 11880
   acagcgaaac agctcgtaaa cacacagttc aatttcaatg aagtataata acattttaca 11940
   cgttgaggat gttcgcttaa ccacgtgttc tcataggctc acacatgtaa tcaagaaaga 12000
   ttatataata tgattatgaa cagaatgaag tttcagtcag agaccactaa caatgtacca 12060
   ttcaatcctc agagatcaat ttcaacctca actaagaaaa ttacgattga tcaaacgtca 12120
   caggggccaa ttgcacaata ctgtataaga gattaacaat agatccgagt agtaaatcct 12180
   cagaacgaaa ctctagccgc agatcgactc gattcaaaca caaagatcta agctaagatc 12240
   tcgaatccaa agcagaatca aatcgattca aatgttgaga gatagctgta gaaatgagat 12300
   tcaattagac ggatcacgag gtcagagtca cgatacaaac cagatcaaac gaagattaat 12360
   cacgctgaca aaatcaatca cagattcgaa cagaaaccta gcttagattt accgagacag 12420
   cgcagaaaat cgagaaaacg aaattcgcag aagtagctca gggaagagat agcgtacctg 12480
   aaggagcggt cggtcgacta agagacgccg gagtgtgagt tggagaagaa gatcgacaga 12540
   gaagaaaacg ctagggggaa gcgatggata gttttttcct gtttctaaag aaaaagaaaa 12600
   atagatctaa cagagtgatc taaaccgtag tccagactct aaaccgggtg ggtagactag 12660
   agatatttta ttataaagcg gttatcagcg cagcttaatt atctaactat tttcttctcg 12720
   accttggttt gacccttttt tggttctaga gtttgtataa accgatctca aaactaatta 12780
   cagagtaatc taaaccgtgg ccatccagat taaaccggac gttcaaatag atgagagtca 12840
   actcccatgt tttttttctg aacctttttg gctatttttt tcttttcttt tttttaatca 12900
   tctgattata gatgaaatac agagctaacg gaacatacga agcccccgaa tcaaaagcct 12960
   aaaacaaggc agcatagagt ttcatttcta cggaatttct atagcataat gcgtttagtc 13020
   aattgttttt ttcttttttt gctaaaagta gtcaattgtt agtctcattt aacaaaaatc 13080
   atatcttata ttctcacgga tctatattgt aactcttaag tatcatcaat gaatttgatc 13140
   tcttctacgt tactttggtt gatgtgcact tgcaatatag tagtattata taggttaata 13200
   cgttgtcgtc aacttccact gtttaccatg ttcttgttca tggaaacgca caaaccattc 13260
   gattcgcctt tcggaaagtc cccatataag tgattcctcg ctgaatgatc tcgttggggc 13320
   caacctaaaa gtgcattttg tttactccct agcagtcaaa catttcattc ctgagttcaa 13380
   caaaatccag taaattcaat gttttaattg tttggcattc ccgaagaatt ttcccaaatt 13440
   gtattatcat tggacattgg ctctcttatt aaatactact atgggtcaaa ccttcattca 13500
   actacgaagc tttctcacgt ttacatgctt ctttttttat atatggataa cctacaaaag 13560
   agtcgtaaaa tgaaaagggt tgctggactg cactacccta cccacctagg tattggctaa 13620
   gttggccaag tatacataat atgtaaatgt attaaacata aactacaata caaatatgat 13680
   caactcgtaa agaaatcaaa tatttaatat cgatgcaaaa atatataata ttggaatttt 13740
   aagtacaatt atccactaaa aagcaaagaa agtgttgcac aaaaataaaa tagaaaatga 13800
   aaaaaggata tgcgatgaag agagtggaat actctaaaag gtagcgtata atctatgttg 13860
   atacctttct ccaaattgaa aacttgtgga gttgtggcaa tccaacattg cccaccactt 13920
   catagtcata ttccatttgc tcctccttat ttctttgttt attgtctggt ttttaaacat 13980
   tgatcaacgt ttatagttca cagactatgc gacctaacaa gtttatctac accaacacca 14040
   aaattaaaga gaggctggca atttcaggtt ggcccctaat cacttacttt agtaggccta 14100
   actacactac ttgcatggtc ttagttcgtc tctaacgacc ttcaatatat aataaaaata 14160
   ataatacttg gtcaagaagc taccactacc aaatcaagat gggattgtgt aaacgagagt 14220
   tatcaacaaa aagaggcaac agttgagagt taggacgctc atcacaccac gtaaaagagc 14280
   tttcaagaaa tagatagacc gatccgaatc acatgcatta ccgaataaaa agttaaggct 14340
   gagaatgaaa gagatttttt ctcgcaactt cttcttatta ttatattcat gatgataaca 14400
   aaaatatata acacgaataa taatgctgta aaacttgaca tatatctgaa tattctctac 14460
   cacaagtaac agcaatagtt cacacgtcat cgccgacgtg gattcttcat ttcccggcgg 14520
   tctaacggac gtgttcaatt ccgattctac ccttgctgaa actagatatt ccccttgtgc 14580
   ccctgactct tcgaaagcat tggctctcac ctcaatccaa ccgtttgatt cccattttgc 14640
   ccctccggtc gtcgctaatt tactcatcct tgccatcgcc gaaaccgtag actcaactct 14700
   agctacatcg ctcacttcat cttcttcgaa atccgagttg acccagtcca atacgcaacg 14760
   cggagacgac tcggtgacaa acgagtgatg atcttgacac agaaacggat gctccagaag 14820
   ctggccgcag ctccatctct gactccgatc tcgtctcaag catttgtcca agaaatcgcg 14880
   accgagctcc gaaactcccg ccggaataaa cggcagctcg tttgaatacc cgatccgact 14940
   cagcgagtcg aatccgttat cttcccacgc tggctttctg gtgagcatct cgatgacggt 15000
   gcaaccgaga gaccacacgt cactctccgg cccttgatac tctctcctta tcacttccgg 15060
   agccatccaa agcggacttc cacgcggcgc aatcccagcc gtcggttttt taaattccat 15120
   cgccgatccg aagtccgcca gcttaacgga gcttccgccg ttaacgacca gaacgttctt 15180
   cgatttaacg tcgcagtgaa cgattccgtt agagtgaacg tgaccgagag cggagacgag 15240
   acaccatacg taacggcgta tgagagtttc gtcaactacg gttccaccgt ttgacaggtc 15300
   accttccggt aaatattcca aatggagatt cctgaacgac gtcgttcctt ctttggacac 15360
   gtcatcgccg aggaacctca cgatgtgtgg gtgggacttg agagagcgga ggattgtgat 15420
   ttcgttctcg agggactcgg attgagaagg aagacacgtg gcgagatcta ctgacttaac 15480
   ggcgaaaact ccaccgtcga tcttactcac ggctttggtt accgttccaa agcatcctct 15540
   cccgatacaa gaacctcgaa tccaaggaga tgaagaagtg tttgtgatgc tctgtttctc 15600
   catgtgtttt tgtttgctaa ctaactttgg tgtgtaaaat tatgaagtac acacgacggt 15660
   atataactat atatacgtgt gcgaaagtgt caaatgtgaa gcacaaataa agttgggagt 15720
   tttattaatt tccgacgtgg acgtttcttt tctacttgtc tttctgacat ttgaaatcgt 15780
   gaagccattt taagccattt taaaatacaa taaaaagttt cccacttggg aattcagaac 15840
   taactctcga attattgatt ataatatttt aaaattagac aaatggataa ttgggagaac 15900
   ggtttgatga agtcagttcg acacttggtg atgttcttgg gatgttctgt aagaaaaccg 15960
   agtactttcc atattatcct tatccataat aagattcaag ttgcggtttg atcaggtccg 16020
   ttgatctgtt acctcttaga cactgttatt ttatttgagt gtcatataga aaaggtaaca 16080
   tatatctttg taaaacgcaa cttcatttta aatcatttat ttactaagaa cagaggaaat 16140
   attattttga tttactatta ttttataaat gcaccatttt tatgaatttt tataaaattt 16200
   tatatgctga atatgtaaga tgttttcata ttttatatgt aacttttaat tttataaaaa 16260
   aatgtaagat tagtgatatt ttataatcta tttataatta gttaaataat ttaaatttaa 16320
   attttaataa ttatttttat ataaaaatat atatttttaa tagttgttca ttgacgtaaa 16380
   atttcatata ttttagaaca aatggaatgt acaattaagt gtttaaattg ttatttttta 16440
   tgttttaata gtttttagta ttaatttgta cctttaaatt tgatatacga gtttaatggg 16500
   tattgggtac cctttgataa ttatcatgtt cttttttgtg acaagataat tatcatgttt 16560
   aagtatcact aggttttgac ccgtgcgccc gcacgggtgt atattttgca taattatata 16620
   tttttgttag ttgtagactt gtaagttaat gttttgttat tgagttctta tatatagtgt    16680
   atcttgttca ttttgcttgg tgatgaattt taaactatta gttgtattta ttttcaattg    16740
   tacttttttt tacctttact tggtaaatta aacaattaag tgtaaaatat tggaatattt    16800
   tgtttagatt aggtgtgttt tattaaatta tactataaaa tttttgtgat ttttagagat    16860
   aagcattact tggttgacaa gttttttgaa agataattat gtgattgcgt tagttatttg    16920
   atcctttttt aaatgctgac tgcgtacaat taagaaacaa tattctttgt tgatttgtct    16980
   tttaataatc ataaatttat gagtcgtttt tggaatattt tctcatatgg aagaaaataa    17040
   gtttaattag gtacgatttt atatgtaaaa tcttaactaa tatgatattt aaggagcata    17100
   ctatacgcat atacaaagta taccaaattg ataaacaata aaaaatattt gactttagga    17160
   accaaaatct aaaccataaa acaaccaaac cgtaccttta ttatagaatt aatatactaa    17220
   atgttggtat gcatagtcat aaagaatatt attctctgtt tatatcatgc atatgtaata    17280
   gaaaacgtga atataatggt atatatacgt tttgatatga aagatatttt gtaaatatat    17340
   gttcaatcga ttggtttgca acgggttaac agattttgaa acatttggtt attgattttt    17400
   tgtgttcggt tgataaaatt ctaaatttag cattgatctg ggcaattaac aatttctaag    17460
   cccaaagcaa tgttatgggt gggtaagaaa gacgaaaagg caaaaatatt tcaaaaaaaa    17520
   agaagtaaaa tgacagaatt tgatggcagt ggcatagaga tgtaattttt gtgcaactct    17580
   aaggggtaat tactgtttgt acttctgctt taatagttta gatgtttact ttacaaatgt    17640
   catacttaca aaaatattaa aatggataag tcaacggctc ttgtttttat gctatctcat    17700
   ttcctttttc aaccataact tggaaaaaaa atacagtata tgtgtatata tatatattta    17760
   tttatttttt tgatcaaata tatatatata tatatatata tatatatttt atttttttga     17820
   aaaatatatt tctcaacaaa taaaaagttt gttgacattt actgttgagg ccattaggtt    17880
   aggggcgaca agtgatgaga tctctccgac gaatcctggg aacggcaagg caaactaaaa    17940
   cgtgtcgatt gattttcggt catttgtttc cgttgacttc tgttgatatg cattacagtt    18000
   ttctttttct tttgttaaca cgatcagaca tggaatattc ggtggtaatc accaatcaag    18060
   tactcactat tcttagaatc gtgatactaa agtatatcac gtaataagcc aatcatatac    18120
   gtagaacttt tagcctataa ttacaaaatg acatcaacta taatttataa gcgattgttt    18180
   tgtgtcactg tcaagtgtca acaacttaca tgtaaatact tcgattatag ttcagtattt    18240
   ttgatagttt tggctcaatt tggaagtcca gtttagtccc agcagaaaag aaaagaaaaa    18300
   tctcaaagaa ctttaaaatt ttcaataaac caatcagttc cctttaccaa accggactaa    18360
   attgattacc aattttatat aaaaatttgc ccagtggatt ccagcttaaa accgaaccca    18420
   aactgaatta actaaacata gactttgctt gatatggtta cgtagtctgt caagccctat    18480
   tgcctaatac caatacacat ctcgtcatca taattttaag ttaagactta agacacaata    18540
   cgctttgtat acgattaact agagtcgtaa aatatgttta aaatacgcaa ctttttgaat 18600
   tgttagcgct taaatcattg tcaacaatca atgtagacga gagtgtatcg gtacactgca 18660
   agtacgtgta gcgacagagt tagttgtcca acgaagtttg atccaagaca tgagaaagag 18720
   aggttcatca cttaaaactt ttaagcacct aaaaaactac tttggtgggt ctactttgtg 18780
   aatctaacgt gtcaagaagc tgttggtcca cgttctccaa cagagcacca gagactccag 18840
   ttgtccgaac tcacttatgt ttctgctcga agattgcact attgacgtgc cttcaccacc 18900
   tccaccacca catctacttt aaataacatt tttttgttct ttcgttagga aacaaaacat 18960
   agaaatgtac tcaacgtgat ccttgaggaa atgagaatga aaaatgctag tttaataatg 19020
   ttgaccaaga aaaaagataa tcaaggattt attcaatata cttatcagtt actagcaaac 19080
   tcatgagttg acaaaaaaag caagtgaata aataagaact tcaaatactt ctgctgctta 19140
   tatagactag ccatctatta tgctatttac atattaagaa aacgtcattt tctgaaagaa 19200
   aatccgccgc aactatcata tataaaaggg tggatatatg gagtatgttg ttaaataagt 19260
   ttattttgtt tgttagcttc tgggagagat ctgcccctcc atgaacatga agtactatat 19320
   caacggtcca ccacttgtag gttatccttt cgcttagagt tcaaaaataa gatatcattt 19380
   tgagatttaa tggacccata ccatattacc aagttacaga tcgagcaatc ccacttggaa 19440
   aacatattag acaatgcaag tgaaagtgca acatgccatt cgctggtact aactttaatg 19500
   tcactttaat gttcttttct aatggaaatc gactcagaca tatgtatagt aatatactcg 19560
   gagaagagaa aagtaacaag gtcatgtatt tactcggaaa agagaaaagt atgaaaataa 19620
   agttaagata atcaggaact atttgaaatt aagtcgcgtg gttttagaga caatatgttg 19680
   atttgcttta ataatttctt taaataaaat aaatagtata tttgggtact ataagatgca 19740
   tggcaaagag caaaacacaa tatagacaaa agttgctgtt tatgttgata agtgtcgtgg 19800
   gagaagaaga caaaaacgaa gcagaaataa ctctaaacta aggtggccga caatacaaca 19860
   tgcttatgtt attgtaactc gggagaaacc tctaaaaaca taatcttcga ctttttttat 19920
   ataggatcgg ttcaagatca tgcacactga tcctcgtatc acaaggataa cgatcctatc 19980
   gatcggtagt aagaggtgca gtatcatggg tattttactc ataacaaaat tgtggaatct 20040
   gaacggtgga agcattataa gcgtagttga ggaggtatgg accgtcacaa tgttatgtga 20100
   actgctattg cagtttaata aagttgaatg taaaagtttt atgtatttat gcaaagttta 20160
   atatgatgat tttttaaaaa tatgtaagca aaattgtaag gttttgactg taacttcaaa 20220
   tgtcaaaagt ccaattaatg atcagtgaag actgcacatt acttcaaatt aataatggaa 20280
   gcagcctagt gattctaaaa gtgaataaat cttttttgat acaaagtttt aggcaaatgc 20340
   attttagatt aagacattaa actaatgctt agaatcagat aattcgaatt tcagaaacag 20400
   gctactataa aaaatgtatt tcttcatgat ttgattgtaa acaatgagat gagaacaaca 20460
   aaatgatcaa caaacattta ttagtttagt tacattgaat tggtggaaca catgatgtgt 20520
   gtgtgggact gaactgctaa attggcggag actttgtttg gtaaagtaaa ataaccaaca 20580
   ataaaaagag aaaagcttaa caacgtgtcg ttttggaatc cattgagaaa acaaagaaac 20640
   agcaacgtat tctccgccta caacacaaaa acatgagttt atatttcacg tgttgctttt 20700
   tcgttttcac tttgaccatt gtcttcttcc tcttcgtgtc ggtaatcatt atcagcgcac 20760
   aaattttaaa tttactttga ataaagttga gttttcaatc tatgaaaatg tttatgacaa 20820
   tctcatagtg ttgattcaaa gtaacgtaag tgtccatcat cgatatggtt gaaagtctaa 20880
   tgtgaatacg taaaatgtgg acgatgtgat aaatactact actagactaa aaggaccaac 20940
   aaaagacaca accaaaagta gtaacggttc tcagttcaag ggttttaatt caaccggtgg 21000
   acgaattaat ttagaggctt aacaaagcaa acaaagacta caagaaacag agacttgttt 21060
   tggcgcggtg gaggatcttg cttttggttg ttataagtca tacaaggttt ttgtcttctt 21120
   aagtaataaa aacaaacgtt tgtggatgat ctcatgtcga agcgtgagaa actaaacatt 21180
   ctctaatagt gatatattgg aaatgagttc ttggtcaaaa tataattaag gtatatatac 21240
   cagagcccat cccaagttca aacaaagaaa gcttgagctt gtctgcttgt gctttcaatt 21300
   caaataaata tttagaggcc gttgaatact gcattatttt tatagtctag ttgtgatggt 21360
   ttgtaaatgt gtttaaattg ctgaggagtc gcagctcttt tttacctccc atatccatta 21420
   attttgtttg cttccgcggc tttcaaatac ttaggccggc tctagacctt tcatattgat 21480
   aaatttgaca taaacctttt ttatgtttgt tccacataat ttctaatcta ttttaactct 21540
   tgttgatatg aaatgcatcg aaagttaagg ggttaaatcc atgtcaacat tcaacaacat 21600
   tgcttgcata tgtgttctat gtgatgtcag cgtcctaaac ctttgctcag atacatatct 21660
   taggtcaaaa agactcccat gacatgttcc agagtccata gggtgaggga aggttccaat 21720
   ttatcaatgc aaactgctat tcgcatagta ggctaggaac tcgcatcaag catctggtcg 21780
   agagacgaac caacgaccat tatgccaaaa gacgggccac atgaagactt ggtcggccca 21840
   aatggaaagt taaccaaaaa atttacccaa ctaaacctcc ataagcctca aactagaaca 21900
   tgcaccaaag cttcaggatg accacatggt cgaccatgaa gccaatagga agtaaatgga 21960
   ccaagaagat gttttgatca tcaagaacgt ggaagagctt aaagactcga gccaagaaaa 22020
   ctctgaggat gatactacta caccaaggac tactcaccaa ataaaccaga acgcatcaaa 22080
   acagccaagc accaacctgg atcaagatac atctaaacta ggtattttca atttaaacga 22140
   tttatgcaga taagatggac catcctagta gttcctaacg atcattcatc ccatctgaca 22200
   caccatagtt ttaggccgca agatagttta tataaatttt cttccttttt tcttgttttt 22260
   ttcccgtttt ggtcttaaac cacaaatgtt agttttttgt tttcttttct ttgcaaaagt 22320
   ctttttgtct tgaatatacc tctgtgagcg taataataag ggcatctcca accctactcc 22380
   attttttact ccaaactcaa ttatggagta aaatcttctc caaccccact ccatatttaa 22440
   ctccaaaatg gagtaatagc tagggttact ccatttatgg agtaatctta ctcattactc 22500
   cattttggag ttgaattttt tatatttatg aaatggttct tttaattttt aatgttttta 22560
   tttcatactt aaaataatat aataacttta aaaaatataa tactccgaaa aagattactt 22620
   tatagtttac agaaaatatg cataaactca taaaagtcaa aactaagaat aaataatata 22680
   aaataaatat aatataatat gaataagtaa tttaataatt aattcggtaa attgttttcg 22740
   aaactaccaa aatcggtgaa tattattcaa acggaataga tgagtttttt aatcttgtgg 22800
   gtcaaaattt tgattgataa catttgtact tgttgagctt gatatatgca caaacaaaca 22860
   ataagaccca atacataatt caaattacaa aacaaaactt tgtttttttc tttatgttcg 22920
   tttaatgcat aaaaatattt ttgaattaga aaaattgcat atgataaaat ctgcacgaat 22980
   tgaaattgga agataatctc tagttgtatt tttaatgata aatatttagt ttaaataaaa 23040
   tatattatta tggaaatttt gtaaacataa aatagttggg ttaaatgtta attttttata 23100
   agttgaaggt actaataaca attattaact aaataaaaaa aagaatcttt ttgtttggag 23160
   taaaaaatgg agtaatacat tggagtaaaa tccaactcta ttttggagtt acaccatttt 23220
   aaagtaaaat ttggagtaat acattggaga tgctctaagg ctctgcgtag ctttgtacaa 23280
   cacactttta cactagatca ataaaataac agagttcaac ctaaggtcgt cttgttcttg 23340
   agttttggga ctttgttctt cgggtgagat tcacctagag ttaagtcttg tgcagtatca 23400
   aatatccttt catcattttt gtggtgtcat tcgatccact agcaatctcg tcaaccgttc 23460
   cagcaaaaaa atgagagtca acttgttaga tctcattcca caagttttgt ccaaaaaatc 23520
   ttgtgtccgt ctttcatcca tccaactgcc acgagaaaga gcatagtagc cagcttatgt 23580
   gttccatttc actattttca aaggctcacc accgagtctt atttcacaat gaattttatt 23640
   tcttaggtgg tttcattagt ttcaatgtct aaaggattga aagtagagag cacgaatgaa 23700
   taaacagatt caacgacatt ccaacaacta gacaaaatca aaacacatat tacctttaca 23760
   tggaaactag tttgagatac aaatacaact gataatcaaa attaaactac ttgtgtggaa 23820
   ataattgatt tccagtttgg cccaatgctg gtgaaatttt ttagaaattg tttaccggaa 23880
   tagcttgggt cctttcattc tttataaatt ctaaggtaaa gagcaaatta agcttaaaca 23940
   catccccaat acacacgtct acaccacaaa tcatgttcta attttcagat acgatccaca 24000
   acaaactcac ccacaaatca gaatacacat actcattgtt tttcgttcaa actttcatat 24060
   acgttgccat cattcttctc taactattct ttctatccac cccgtgtttg gatttaacat 24120
   agacaaattc ggaggataat aataataagg aactgataat tagattaaat tcgaccaaat 24180
   gctcgtttca tacaagtacc tcttcaagtt agaaagaatg aataaatgaa ttatatcaaa 24240
   agtcaaatta ataaaggtaa atggacgcaa gcccttcaga tttctatcta aaatatctaa 24300
   ggatctctct tatatgaact ggtccaaagg gatcagcatc acactaatat catccaatga 24360
   gcctcgtgaa gccgaaagat cgacaagctt cttacagccc aacaacaatg gcttctcctc 24420
   ggttcctagg cagaagggac gagcaatgtc tactgcttct tggttactca ctttgtccca 24480
   tagaccgtca gatgccaaga tcaagaactc atggtcctgc tcgattctca acgtctttgt 24540
   ctctggttcg gctataaccc atttcttgag atgagcatca ccgatccctc ttgacacagc 24600
   caaagatcct tcaactctcc atacacctcg aaacgtatca acgtatccac cctgcacaaa 24660
   aaaaaaattg aatctacttt tagaaactat actttccatt tgtataaaac attaaaaacc 24720
   gagattctca ccgtggtttc aattcttgtc cgttcatcgt ccctagacgg gcggtggtcg 24780
   gaagaaagag cctccgcgac tcctccaaca ctcatgacgg cgcgacaatc gccggcattg 24840
   gcaaccacga ggttcccgtc gctgaacata gccgtgacgc agcaggaacc gcctttaacg 24900
   tcattctcgt tgagaaacgc agcgtctgtg gtcaagtaac cgcgtttcac cgcgtctgcg 24960
   atcgctgact cgtcgttttt accagcaacc gcttccaaaa cgttcttgtc taagttcttg 25020
   gccgcaaact cagccgcttt agctcctccg tgaccatcgt aaacaccgaa gatggcatgt 25080
   ttgcgatctc cttggagatt ggttacggca gagaagcgat cctccatagc ttctctcctt 25140
   cctctcttgc aataaacaga atagccatcg ccttccctct ccacctctct accttcctcc 25200
   ctcggcgtcg ccggagcaac gaacccggtg gtaccgatcg gtatatcaag cctcgtgggg 25260
   cgtttgcgtt tcagaacccc tccgggaggt gattggccgg tacacgacgc cggagagaaa 25320
   ccggtcggag gtttctgtaa acggagacgg aacggcgagt tgagggacgc cgcggcggcg 25380
   gaaggagagg aaggtttgag atgagaaaga gtgagagaga tggtttcttg cggcgaagag 25440
   aggatgatag aaggtttgct gcagaaaaga gacgacgacg gagaaaaaac cggagagtta 25500
   cagacggaac aagacatcgt gtaagagaat tctgagttcg aagattgatg tgtttctttc 25560
   tctctacctt tgagatattt gttttaggag aggaaaagag gtttctatta atataaagag 25620
   agagagagag agagaggtaa tgaatgttga agactttcaa agtggtaata atggagtccg 25680
   tgagggtaat acggacattt aaaagtaagt caaaaacacg tctaaaagga aagaggaaga 25740
   gagtgttaag gaaataaaca aagaatttgg gcatgtggtg gtttaacgta tcagtgttaa 25800
   agaagtgttt ggttgatgac tcttcacgtt ttttcaattt attcttttgt ttttaataat 25860
   aaaaacagat tctatgaacg ttgtcggtcc gttagagctt atgagttgta ttttgatggg 25920
   attactttct tttgtttctt ggtggtccca gtcgcagctt cttagcaagt gaccgttgtg 25980
   tggactgaga ggagccttct ttctttcttt ggtttttcat ttgatgacct ttgtaaaatt 26040
   atctatctca attcactcga ggtcttctaa ttaaaactaa caagtcttct aatgacatca 26100
   ataataacgg ctacttcttt ttccttataa aacggtattt gtttatggat ttatgtgctg 26160
   actgctgaag atcaaacatt ggcatccata aggatcaaat tatcttattc tctactagca 26220
   acttttaaaa caatgcttta acaagctact gattttgagt acccaataca tattttctgt 26280
   ggtttttttc taacactgaa actaatccat tatttagttt gaatcatgta ttatcaagga 26340
   tctcaataag caaaagtatg aataaaattt atgattctat tcaaaatata ttttttagat 26400
   attctctttt cgttcaggaa ttccaactac tattacagac ttgtgatggg gattcagtgg 26460
   gtttttttat aaatagcaat catatgtata acatcattat ttgctgcgaa ttgtaccgtc 26520
   attagtttga gtatttacat taataagtaa tggtatgatt ttctgttgtg ttcaaatact 26580
   gtttatgaag aatgagtcat atattttacc ctacttttac gattagactt ggtcattaag 26640
   atagttgacc agataaaatg attaatcaag caaagaagct tcccactcca taattattgt 26700
   ggtcacttgg ttcactttga agttgtcttc gaacgtcttt ttaatagtac taggggttat 26760
   ctgtgtttta cgcatgaatt tttttattga catttatttt tagtttaagg ggttaattat 26820
   ataattgtga accattattt ttgtgtgagc tttttttata ctccatatgt tttaaaatgt 26880
   tgtatatttt agatttttca cacattttaa taaaacacat taaatttcta ttttttttgt 26940
   gattatcttt tttttcataa aagattagtt aataaaatat acattgaaaa tgtaaaaaaa 27000
   tagatcttct tgatacaaaa tttttctcta taagtaactt tataaaacgg aagaaatata 27060
   agaagacata agaatgtgtt taaaaaaaag acataagact atcgagagcc gaactcgttg 27120
   tagttgagta aattgcataa tgttatagtt gttaatttta atggtataaa ttttatttag 27180
   aggacttata atttgtttga tattttaaga tcatccacta ttatgtgatt tttgtcagtt 27240
   tattacaatc atattctgct atttaaaaaa aaaattgaat tcactttttt ttagttttcc 27300
   acaattattt ggacaaaata atcttacatc ccattgttaa attgtgagaa caaaactttc 27360
   atctatctac aatagtagca agcgtcgcat ttgtttctga tgttcttaca ggtggatgtt 27420
   atcgttacgt catcgtgtga ttgttgtttt tgtatttcta taaattctta aataccgtgg 27480
   tatgattact ctagtttcat gaaatgattt cctgaaacgt ttccagcatc agtccccttt 27540
   atacattacc catgcatgtc taaatataca ttaccaatct ctaaatatac attacccttt 27600
   gttattatgt gaagatgttg tagttcaatg cctactccgt aatgttgata catccatgga 27660
   cttgagaacg ggcagaaagc accagcacct tttgttataa cagtatattt tccagaggaa 27720
   tttggtggta aacataaata catcattaat cctaatcaaa acgactaatt attattgtca 27780
   aaagatcatg cgactagtcg tatgtcaact tacgaagcct gcaacaaaag aatatctttt 27840
   tgtaaatgtg gattatttca aggggtgggt cggacacatg aacagtaatg ttaagaagta 27900
   aacctattgg gccaaatgag accgacgtag gcccatagaa aacccatgac gacccgtaca 27960
   ctagataaat gcatattaac ttcgaagaca ataaatacag aggggtccac gtttccgttg 28020
   caaattgggc agtagacatc ctccccgtga agcaaattca aagtcttacc agtagcgaag 28080
   aacacaaaaa ctaactcgct acaatcttct cttttttctt tttttttcaa tgttctctct 28140
   cgacgaaggt acaaagatct tgtcttaaat agatatatat ttttatttaa tctaagcata 28200
   gttattacac agcccctcag ccagagagag aactaagatg tacaacgtgc atctttatca 28260
   gggggttaaa ctgacatagg ttatttgtta attatatgtt tttaatttcg attaaccgcc 28320
   gagtaaaggt ggttaattaa gctgcgaaaa gtaaaaccta cataggttat ctttagatta 28380
   tatgtttctt attttgatta accgccgagt aaaggtggtt aattaagcag cgggagagtt 28440
   acaggaagat tgatgtcgga gatttgtcca gatgacatct ctatattatc agcttcggct 28500
   ccctcaagat ttttcttctc aatctgtaaa gataattata ggtcaaaaac atattcactt 28560
   ctctttttgc cttctatgac ttattaatac aagagaatat tttttcccta ccaaccatca 28620
   tatatatagc aatctggatt tcaaaattta gtttttcttt tcctaccaac catcaaatat 28680
   atagcaatct ggatttcaaa atttagtttg tcttgtttct gactttcaag cttctaggtc 28740
   tttcaagttt aaagaacatg catctttctc caaagcaggt atctagcttt tccagtttat 28800
   aatcaacctg gctgaactag ctaggaaagc tatggctaga tacttgaacc taccaatctt 28860
   acccaagcca acctaaacca tataacaaca aaaaccccaa tcggttccat aaatctcaaa 28920
   ccaaattacc aaaacccaat tatgtccaac aacagggaag agattttact atatcaaaat 28980
   tatatatcta tcaaaaccca ggcttcattg gatatataat tggggaaaac ctcaaaaccc 29040
   caaagtataa aaagactgaa tcatttatat ttaaagaact ataccacttc ttaatttttg 29100
   gggtaacagt atcaaggttt atgaaatagt tttgaattta cttttagggt tcagggttag 29160
   gttttaaact gttgtgctca tctaaaataa ttttattatt actttacttt aaaatttaga 29220
   ctattttcat aaacgttctt atatatatat acacaatatt agaaccgaat cgagattttc 29280
   ctcaaccaat accagaccaa agaaaaagag aataaacatt ggcacacgaa aacagaataa 29340
   acaaaatcta atcaaaaacc aaaatgctct tattaatctc gagatgtttt tttatattaa 29400
   aagtgcttat atatgtatat atctgcaagt aagtgtgtgt atatgtgcaa gaagtgctta 29460
   ttagcttttg catatttata aagaatgtag cttttcgtta cctgactagc caaaacctgg 29520
   ttctcttctt tcagcagatt ctcctgtttt taaaaggaca acatgtacat caattaattt 29580
   cagattttgg tatccaaaac atttgcaaca tccctaatag acaagatgca ttgtgaaatg 29640
   tgaagaagtt tgatgtagac ctgcgagctc ataaagcaca cttagagaaa ctaatacaac 29700
   aattgtgcca aaaaaaaaaa aactaataca acacacaagt gttttgaggc tctcaacacg 29760
   agaatatatc tataagtgct ataaaatcac aaatctaacc ttttctttga gactttcaac 29820
   aagctttaac attagttctg cctgcaacaa attaaatata agtgcacaat cgttttcgac 29880
   aaacaaagta aacaaagaga acacattatg gagtcgatgt accttccgag ctctagttac 29940
   agagagggca gtctcaaggt gatcttctag ctcaacgagg gaatccacgc ttacatcatc 30000
   aattgattcc acaagcttgc tgcatatatg cacagattga caggaaaccc taatatattt 30060
   gtaatgaaag gaggagagca aaaatagtct cagttagtac taacctttcc acaagttcta 30120
   gtagctcatg gtgtgaacta tagttcagag attttgactg aagatcctgt tcacggagaa 30180
   ggataaaaga ggcggtaaaa gaccagtgta tttaagaaat ttgatatgca cgtggaccaa 30240
   taacctcaca caggatcatt tggcgaataa tagttcataa caaactcaag ttttaagaaa 30300
   tttgttctca acatgtgttt acattatagt tcaaaaaaaa aaaacatgtg tttacatatg 30360
   tttatcgaaa aactactcta aaggaagcat cgatagataa cctcttccat gttgaaaaca 30420
   atatttgaca actacagctc tcaaatagga atgatacacg cagtttaaat gcccgtgagc 30480
   catgcccaca aaaagtaagt tgaaacatga cgctgcgagt tggttctgac cagtatatat 30540
   ggagctctct aagccttact tatatcctta aatactgcat gaggaaatat tcaaaatgtt 30600
   tgtattacca gggcattgag atcatcagca tgttttttcc catatcgatc aaggatcttc 30660
   tccagtctgg agaaaagata aataaaaatg ttaactgaga tcaaaagtca aaactacttg 30720
   ttaacccttg aagacaccac tggaatctat caaaacatta aaagagaaat gaaacaaaag 30780
   gttaattttc gcaccctaga atttgctaac aattcagaaa aaaccgtagt aatcaggttt 30840
   aagcagcagc acatttggat ttccatttct tatttttaac atgctctgat cctttctaag 30900
   caatctcaat aataataaaa aagtcctcta aaattaccaa gtatttcaag tttaattttc 30960
   caatagttgt gttaggttac atgtatttat ttatatactt gatagactga aaatggcatt 31020
   cttttgacaa agaaaagtca taatctatac tatattaaaa gggttatatg agctccatac 31080
   agcatgtcca cgtaggacaa ttaaatcgac caatcacgtt gaagcgttta gccatgtcac 31140
   taatatgttg ggctcacggt ttttcttttg tgtatttgtt acgattgggc tcaagcccat 31200
   gaaaccatta taacaaacaa tcgcactctt tcacgttttt tcgaaaccaa aatcagaacg 31260
   attctcatcc cctttcctct tcctcttctt tgatccgttt cacgatctga ttcatgagca 31320
   attgattcat cctccacttc gttctccctt tactctcatt tatggattcg tttttctctt 31380
   cttttgttta taaaactctt gaacggagtt tcgtttcgat taagcttcgt cgtaaatttt 31440
   cattcgtaca ttgcaatgag tttcaccgga aaatccaact cggagaaacc acaacgcgtt 31500
   gagggtgact cctttcccgg accgatcaat cccatcggcg atccccactc gaagcaagcc 31560
   aaagtcgaag cgtcgttctc ctccggtctg acgaaattaa aggctgacac ctttcccgga 31620
   ccgattaagc ccatcggcga tccccactcg aagcaagcca aagccgtagc ctcgatctcc 31680
   tccggtctta cgaaattaaa ggatgactcc tttcccggac cgatcaagcc catcggcaca 31740
   cctgattcga agaactgcaa aggtaagaaa ccttatcgtt tcttatcaaa ttatatagtc 31800
   cgtttgattt ccaaaaaaaa atctgaagct ttgagattta aaaccatgag acgaacaaat 31860
   ttttttttta atagataatt tttatacaac cgcaaaggta agaaccctta tcgtttctta 31920
   tcaaattata tagtccattt gcttacgaaa aaaaaagtct gaagctttga aatttaaaac 31980
   catgagatga acaaaatact tttttttata atagattatt tttatactat agaaaataga 32040
   aaatcatata aattgtggta cggagtttag tatccttttt tgatgatgag aacgttcgta 32100
   ttcctatgca ggtacgatca atcacaacac gaagactggt ttctcttcag gcgttagagg 32160
   caaagccgct gtctcctctg ccgtcaaggg aaaagccatt gtctccgcca aagtaatggc 32220
   tttcaaagat gtgaaatacg gacttcatga cggcgagctg aggtttcggt tgatccattt 32280
   ttgggaagct cgaaatgttg tgacgaaggt gcttctcggt ctcaagatgc ttctcatcga 32340
   ctaagaggta taaaccgaat tcttgattgc gatttagttt agaaattgtt cagacaagat 32400
   gctgataaac attttagatt caaacttatc gctctattat ttatagcaag tgtttgcttt 32460
   tgtttgacag gagggagctc agatatttta ggaaagatgg gcacaaccgg aggaagaaga 32520
   aagatggtaa aacgatgaaa gaggatcacg aaagctcaag gttaataata tatttacatt 32580
   catgttacct cagtttttaa taaatatttt agagtaatat tgtttaagta tatatctagg 32640
   ttggaagcat tgatgtgtta cattgttact aggcacatgg ggaagacaat gagaatttcg 32700
   agaggctttg ctactggatg cttgaacagt gagtcctatg ttttccatat ttcacatttt 32760
   taccggttta gtgtaccgta aatgttactt tgagaacaga aggaaaatga aatgtgaatt 32820
   gttactttta gtgcttatgt cctctgtttt tttatgggta cagggaactg atgcatattg 32880
   tttttgttca atacttggag gttaaggtgt agcttttctc ttggtgctgt actaatatat 32940
   tctttagaat ataaacatcc attcactcaa cattatattg tttcttttta aagtgggttc 33000
   tctttttttt aatggtggct ttgataagtt cacgctattt atacagggta ataggataag 33060
   ctccagtgga ataaaagaaa acaattcaaa ttctgtgagt ggctccactt ctgtgaatat 33120
   tgattcaatg gcaaacacat ccagaacatt gtcaccacta tgtgaagatg ctgattagta 33180
   tttgccggag ctttaccgtg gagtccgttg tggccgtgtt attgacgttt tagatttgaa 33240
   tcagcctaca cggacctaac agcaacaggg attccagcgg actaacagaa gcagccgtgg 33300
   agaggcaagg cagggacgag attagcggcc agaaatttga aaggaaatct tcagaagaat 33360
   cagatagtaa gatagttggt gaagaagaga aggaagatat gtcgcttggt aatgatagtg 33420
   gtggctctat caaggcggct acacatgaca aagacagaga tacttctcct tcccatgaag 33480
   ggataaagct ttctctgtga ttgtgctgtt taaaatgatc atttatgcat agccttggtt 33540
   tagtattttt ggtttataaa ggtcatgact acaattcaac agggtattgg aacgagctac 33600
   tatgggcagc ttcgtgttct ctctggaggt caagcacctg gtgggcacac tgctatatat 33660
   ggaccattcg gtaagtgttt aaaatacatt tttgtgtgtt ttaaaatgat gctttacatc 33720
   ttagtcatat acttaattaa gaaagcagag tagcaggatc actcttattt caaaatatgt 33780
   gcgcttttta gaagtttaca tttgatgtca taattttgtg aatcagtgtc tcgagtaatc 33840
   aaatttgatg tcaccctaac ttcaaacttt gctattcaca tccctcaaaa cttattctct 33900
   cagtgtttgc atgcagggtg atccgaagat tatcacctgc aagttttcat gctgctgtag 33960
   caaagacatg ttttgaggta cggtaatatt ctcaaaacac cagactttga ctccctttgc 34020
   ataacaaatt cttctgcagt tttctgattc taagtatctc cactttgtta ttcactcagc 34080
   tgaagaatca actcatcact gctagctatg ttgatgatga aacatccatg tagatggcta 34140
   agaaatactt ggattatgat tcttggtgaa gtgacatcca caacttggat aattgagaat 34200
   gaggctcata tcagcaaggg gaaagtgaag tctttcacgc tcttcctgtg gtagcattcc 34260
   cttttcttct gcagcagata atttattagc acagattgaa ttacttcaat ctgatgtatt 34320
   caaatcatgc aataatgtga tcaagagctc acattcacct tccactttgt aatatttatt 34380
   atgtatcttt ttttttggga atcataataa gcaataatat tttacagtac atatgcatta 34440
   caattacaac caaaagaaaa ttaaagaaaa acagtgaaca tataaagtta gcttaaaaag 34500
   ggaccccata gagaaaatac atttaaaata tatagtaaat tattaaataa acaaaataaa 34560
   attggaacaa atttaaatag taaatatatt taatgtataa ttttaaatag taatgaccac 34620
   tattaaattt ttatgtagtt tacctgattt aattatattt tcagttaaaa tggattaaac 34680
   ttcacaactt attacttata acttcattaa actcatcagc aatttttaaa aactaattct 34740
   aactattaat atagttaaac taaaacaaac attaaaatga atagtcaaat aaacagaatt 34800
   tttttatagt aaaaaacatg tcggacgttc gacaactgtc tcgcaacgag cttctaccaa 34860
   gagatataaa tgttacttgc acagctcgtg aagtcctctg aaaccgactt acgttacgca 34920
   atggcgtcga ccccggttct ggggttggca gcaggagact cacgtatata caaggacctg 34980
   gatctccaga cgcatacatt ttcctgattg gcttctatca accgtttctg tgatcttatg 35040
   gatcgatgtc ttgctagcgg taacatccag gcacactacg tgcaaggaat tcatgaatat 35100
   ttttgcaaca acacaatcaa tggcatgcac catttacgcg tctcagcagg tggttcttac 35160
   gcagatggtg taatcatgtt gtgcagaggt gagcgagctg tcggtcatgc ctacatatac 35220
   atgcttggtt ggagggagtc cccaactaaa ttagacgaat actggagaag aattaaaact 35280
   tcgcttcatg gtattgttgt tgcgagactc ccggtttaca tgacgacgta ccaagaaaca 35340
   agagctgcta ttactagcct ttgccaaagg aacctgcgga agctcgagcc accggaaaga 35400
   tgccatgtca atgacatgga caattactgc gagctttgct tatgctacaa gtaaatcaag 35460
   cagttcattg ctatcctttg agatcacatt agtgttttgc agttcgttgt tatatcgaat 35520
   cgtattccgt accaaatcca tggtaatcgc aggaaaattt gatttccggt tttggctgga 35580
   agtttgcttt ttgtggtgga aaaattgatt tcgtggtttt gactgcaaat tttgattttc 35640
   tcggctttgg caaaaatatt cgattttgtg gttctggcgg agaaaaagaa tttgctgttt 35700
   tagcgggctg gaaattttgt gtttacgatt ttggcgggaa gattcaagtt cacggctttg 35760
   gcgaaaattt taattttgtg attttggcgg aaaattttgc tcttgcggtt gtgacgggaa 35820
   aaaaacaatt ttttattttg gcagtaaatt tcaattttat tttattttat cgagaaaata 35880
   caatttgtgg ttttagagga aaaatctaat ttgctgtttt agatggaaat ccgatttgcg 35940
   gtttagagga aaattttaat tttacgggtt tgccgaaaaa atcgactttg cgttttttga 36000
   aaaaaaacaa ctaaaacctc attttccata atcaatcttt aaatattttt ataatatttt 36060
   taaaaagtgt ttttttcttc caaatagtct tacattaaaa ataaatatta aaaacagaag 36120
   atcatatatc attttaaatt ggtcaaaaca agtttaaatg agtcaatgta atatttgagg 36180
   gtctaaatga aaaattctaa tagatctatt ttaaaattaa tctaacggca tagctattga 36240
   atggggtgag tcttaatttt ttttttgaca acatgggatg tgtcttaaat ggggtgggtt 36300
   ttcccatttt aacatccata tactccaatg taaagaatat aaccattaga ttattttggt 36360
   ttgacattag aagttcggta gctcatataa atctaacacc atgttatgtt gtcaaaggtt 36420
   tcggacatta gtaaattaat aaaaatgtag caatcaataa tgtgaattta ttatagtata 36480
   tattgttatc agtctaagta taaaaatata tttatattca gatacaaatt ataaagtaat 36540
   ttaaatttaa ttaaaatata tggaaaataa cccgggcgta gcccgggaaa atctctagta 36600
   acattaatac ctgtacatgt tatccattaa tctatcaatt aattcatatt caacgctggc 36660
   ttttgagtca cttaaataaa aattaactaa ggtacataag aaccctatac tcaagtcaaa 36720
   tacactttgt tttgcctctg cacccacaac tgtttctttg cattcaggtt tgtgttccat 36780
   tttataataa tttgatacta taacaggaac gacgactgag gcttaaatga gagtgtatat 36840
   atattacata gaggtaaaat aaagtgtccc aagtgaaaga aactttgttt tgattctcac 36900
   ttggtgcata tagaaaagta ttccataaaa cgaagacata caaaataagg ggaaacaaat 36960
   actacatttt ctatttatga ggttacagag acgtctaacg catttcgaaa aaaattacca 37020
   acgcagttaa cagtttgtat taataggttc agagttccat tgtgaagtta atcttttgca 37080
   cattttcatg tgcaaaacta ggagtttgac tactccaagg ctgaacctag cattcagtct 37140
   aaggcgaaca aatcctagca aagtatgcat cgagtgagaa tcaacgatct tcaccaaacc 37200
   actagtacga cttggttata ctagagggtg ttagtacaaa cttcatcgat taatttgaca 37260
   atgtaggatc atactgaatc tagagagata ctaaaggggt tggtattttt tatgaagacg 37320
   aatgtttttt gtggggttgt cgatttccag gaggagccaa agaaacaggg cgtgtgttgc 37380
   tgcacttccc aagacaaaag acccaagatt tcatatacca aagcgacaac gttaatttaa 37440
   tatagttcag agagaatagg aacaaagtgt tgatatataa tgcagacaat gacaatgaaa 37500
   ataagaaaga tgaacgaata tccagtcaca ttaatatgta gcatataaat gtatatcaca 37560
   ctgcgaggat gggaaactaa aaagtagaaa ctagtagaaa cttgtaggat agaaattcct 37620
   tgaaaccata tatccacatt aatcttagtc catagataca caatctatca tacatttgaa 37680
   aaaagttaat gatctatttt accatcaact ccatactatt tttataacaa atctcccgga 37740
   agtgcattgc aatgtatagg aatccaagag aacaatgaat taaaattagt ctaaagttag 37800
   accaactgat gcacattacg tgctgcacaa agtatcaaat atatgcacac acacaacgat 37860
   tgccgattaa agaatcgaag tcgatactgc atcacataaa taatatagca tatgtgagtt 37920
   aattaagaac taattgtgag gttaaatttt cttaaataaa aaaatactat atatatatat 37980
   atatatatgt atgtgtgtgt ctgtgtgtga aaatcaagaa tcagttagaa accttaaaat 38040
   tcggataatc ttaagtaata attcatggta aaaaggataa aaattttgaa gatctaaaat 38100
   tatcttttag tcaatagctg cacaatgtcg catacattca aatcaaaact cttgatctag 38160
   tatccagcat taaccccaca tcagttttaa ggatctcttc gcagtgcatt gcatacagat 38220
   ctgagcgaac caatgattgt agttacgata aaggcagaca gattaattaa ttttctgatg 38280
   caaaatccct aaagccaatg aactaaaaga tggcttgaaa cattactaaa gaacatcaag 38340
   tattcatcac aacgtatcga gaagatcttg acggacatgc aaatttttaa caaataaaat 38400
   aaaatccatg cagaaaaata gaaaaaaaac gaagaaaata agagagaggg agattgagta 38460
   agaagcttta gctcatcaca acattgttct tcctcaattt tggttatatc atgtatagag 38520
   gaaacaataa caaacaaaac cgaaaaatcc ataagagatt cgccgggtaa atctaagtgt 38580
   cttttaatta ttttatttta attattttat tagtaaagta aagagaagaa agcttaaagg 38640
   gcaaaaaaat aagagagaga ggagaagtcc tacctatccc cggaggagaa gctgtagagt 38700
   ttgtcggagg cggagacaac gagaagccca acagatgcct cgcagagaac agaaagctga 38760
   cgagctttct cgatgagacc gttgcgtcgt ttgcagaagg tgacttgtct gctactgttt 38820
   ttctcgattc gcttgatttc tagttttttt cttcccatag cttctgtctc cgagaggtct 38880
   ctgtgcccta atttgattct gaggtacggt taaagtcgcc ggagagacta agcgttttat 38940
   tctttcttct ttttcttcct ttttttttct ttaatttcta cctatttttc ctcgggtagg 39000
   gtttttttgg gcgggggtaa acgagagaaa taaaaataaa aatatgaaag ttaaaacgat 39060
   gcgttttaac gacaagacag ccacgtgcac cgcaggacga actccctgtg gacgcgttgc 39120
   gtttgactac tcgactctac catacataca cacaggtagt gctgggaata tgaatcatta 39180
   tccgcgggcc ccgccccatt tgatccgctg cggggcaggt gcggatcgag tgatttgaaa 39240
   aatttggttc gcgggtgcgg gtgcggattg agtgattttt atgcggagcg ggtgcggatc 39300
   agccaaaatt cagtgcgggt acccgccaac ccgcaaaaac taaaaaagaa aagatttttt 39360
   taaaaaaata ttatttttaa atagaaaatt tttaaaaaat aatttaattt taattataaa 39420
   tagattaata tttattattt taataaaaat atttaaaata ttaaatttta ttgttatttt 39480
   taataaaaaa atatttaaaa tattaaattt tattgttatt ttaataaaaa atatttaaat 39540
   taataatttt taatattatt aatattatcc gcgggtctag cggatcaccc gcgggtttta 39600
   gcggggcggg tgcggatttc atattttttt cttgcgggtc aagcgggtca aattttttga 39660
   gtaaaaaaaa tcagtttatc cgcgggttgg cgggtcagcg gggcgggttt gacccgcaat 39720
   ccagctctac acacagggtg tgagtgaaag gtattaatag agtaaaagtt tattctatat 39780
   caatatttac tctaggttga gctaaaatgt taccaatcaa gtggaacata aatttttgtt 39840
   atttactcta tgtactgtac acaaagagaa aaagagacac taaatattac tcttgattat 39900
   gctagaggta tatgatttta ctctatatag atttttcttc tcatattttc atcttttaca 39960
   tttttcactg agtttcactt tttcatttcc tcctttattt accaatcaca ctcatatagt 40020
   aatttgcagc acttaaatat ataaaaatca aaatcaattt tcatgttttt tttgtatagg 40080
   tgatatatta gtttttaagt aaaactcata taattctaaa atatgtggcg caattttaga 40140
   gcatatattg ataatttctc gaaaagcctt ccataaataa aaaattaaga aaaatataag 40200
   aattgtagac attactactc ttgctcattt cacatttatc aaatatgtta taactgaatt 40260
   tattttaata ttattaattt ttttgtcttg agaaactaag agtatcatta ctggtcagtt 40320
   agctaagaat cgtttctagt ataacaacaa tagaatttga tatttgtaat taatttttaa 40380
   acacaaaaaa gtttaatcag tgatattgtg acaagtaagc aaattagttt ctcaattttt 40440
   ttacgtctct ttccattgat aatttggtat tctatccatt ggaattgaca tttactatcc 40500
   aagaaaatta ttctaaacac attaaacaac caaaccctag cttatatgta cgttatgctt 40560
   ttgaacagat acactcaggt gatatatggt tagatctaga taactagaga ataaccctct 40620
   tgaatttctg caaaaaataa taatttatgt attttttaga gttaatttac tttaaagtag 40680
   gaatttagtc aactatccta aacagtaaaa ctagacaatt ggatgtaggt actggacact 40740
   taaaatgaca attatatcct tgacaagcaa tttcaattct agttcaaaaa acttcgtcca 40800
   aaaaacatta aaaaaaaaca caacttgtga gtaaagagag aagacggtta aacggaagga 40860
   ggaaagaaaa atatccacgt cggagagaga agaaagctgg gagaggagct tggggtggcg 40920
   agagacaacg tcgtggcgga gctgggcatc gaggaggcgg atcgcttggt ggagacgctc 40980
   gacggtggag gcagaggatc cggaagcgag tgccatccgg aggcgagtgc ggaggaaaaa 41040
   gaagtggaga ggaatggaga gacggtgggt tcgaaggagt ctagaggtga tgaaaaagat 41100
   gatggttgtg ggatgataca tgtaggggat aggagtatta gcgtcattag attttaaaca 41160
   tagttcgtaa tagtgtcgaa tggtagatca ctaattagga aatattgttt gtgtatacaa 41220
   tgcaaatgct ctatctatta tattaaaatc gaagtataaa ataatacttg attattttaa 41280
   atggtttttt acggttttca ttttaaaaaa ctaactttaa cacttctttt gttatctttt 41340
   ctaattaatt tgaaaaattt atcatacatt atgaatcaaa acttacttat ttaaagtgtt 41400
   tattacatat tttacattta tttccacttt tcttaactat ttcattaatt gtctttatca 41460
   taatattttg ataacattta gtttacatgt caaccataat aatttgacat gtttgaataa 41520
   aattatttca tttgtgacaa aaattcaaaa aggttaacaa ataatttttt tattttctta 41580
   aagaaatagt taatcatggg tgttcggatg ccagtttggg tatatatcgt ttctttcgcg 41640
   tatcaagttt tttgggttca aaattaggct ctgatcacgt attataaatt tttgggtgta 41700
   tttcaagtcg tgttctccta tgtccagatg gattcggttc tgatgtataa aaactttaag 41760
   atatccaaac aaccaaaagt gatttcatat tacggttcaa gtattttgta ctaaaaataa 41820
   tcatattacg atttgagtat ttttttattc aaactaaaaa taatttaaaa ataaccaaat 41880
   aactaaaagt aaccatatta tctgattgga tttgagttta attctaattt gtaaaaacta 41940
   gttatccaaa taatcattat acaattattt atacggtgac acatgtaaaa tatataacgc 42000
   tatacatgaa aataaatatc aatttataaa agaggataaa aaccaacact agtcaacaat 42060
   taaatagatg ataagtaaca gatttttttt caaaaacgaa atcgggtctc ttgtttgagg 42120
   gacaattatt tattcctctt tattcctctt tctccacttt ttttaatttt tgtattttag 42180
   tatgagaaac ttgtaaaaag actgtatgtg attgttgtac ccctaaaact ttttctcagt 42240
   atactttttt ggctggctgt ggagagaacc atttttctcc atacgtttaa tcaaataaat 42300
   gttattgaaa atgtttatat taacaaatgc aaatgaaatt agctcattgt acactactct 42360
   tacttctaca taatcgacac atataaacct cgatttaaaa ttcaatcatt tgttcttcca 42420
   tctcctctcg aaactcaaac tcttgttata atttacgtat taagttttta ataatccttc 42480
   catatacgaa tgagtcttaa actttcagct agacaagata atatgagtct gataagataa 42540
   tacctgtagt ttatgatata acaattcgat tcacggattc tgccatctcc acttgttttt 42600
   tttaaagcta ctgataaagt ggaaacaaat aaatgcccaa taagaaaaca gcagcatcta 42660
   gtccattact cattacaatt cattgtcatt tactgcttcg ccacctgcag cattaagtac 42720
   tattacagga ataatcattc gtcacttcat cttgagatat ttttatttct tggcttgttt 42780
   agacagagta taattccact ccgtttttta ttaaatggag taaaagttaa aatagagtaa 42840
   aaattaattt aactcaactt taaatctcat tctataataa aatttatttc ataaatagaa 42900
   taatttattt tttgtttgtt catttagagt agggttgaaa tatttttact ttatttttac 42960
   ttttattcta ttttaaaaga aagaatagag tattgtattg tttttctccc tcttcatctc 43020
   cttagccata tgaacaatct catattttaa agttagtcac tttaaaaaga tactcggtta 43080
   caatcattat cgccaagatt attcgagaat atatgtttac aaaccactga accagcatct 43140
   cctcgatcaa cgagttgaat cgccgttacg cgagtacgtc cgtatttgta tcccgtgggt 43200
   attacgtggg ctaagtgtcc ttgtataaac gcccattcta gactcaacaa aaaaaggccc 43260
   atatagttat ccaatttcac accattattt cggttgctaa gccctttcaa agccccttcc 43320
   ttcaacagct tttggttgct ataagggacg ccacgcgcgc attttgcttt cataatcctg 43380
   taaataaggc atgcaaaagt ctttggagaa gagccaaggc ttatgatatg ttaggtttgc 43440
   taaacaaatt ttgttattag cgattatgat caacacattg tctaatttca gtttagttaa 43500
   tagtttttgg tgccaatatc tatggatttt gttcaggtat gaggcataga cacggcataa 43560
   acctacctga agggcaaatg tgaagaatcc ctagaacaat ggatacaaca aggtccttga 43620
   aagaagttga gaagacaaga aagaaatgtt tttttttttt ttgcttttaa cacagacagg 43680
   aaatgtcttc gtatggggtt ttaatcaact tgaaggagta aagatgaaag gaagatattt 43740
   ttttttgtat gggtgtgtat gttactaaag gacttttcgt agtggaaagc gggtataatt 43800
   tgcctcggta cggcttgaaa tattttttga ttaaacaaat gacatttcac ctgcagagaa 43860
   aataatatta catgcacccg cactatttaa ttctgtggat gactcgtggg attatcatgt 43920
   tttttttttg ctaaactaaa ggaaataatc gtgggattat catgttatat caatatttat 43980
   aaaaataatt tagtaatatt tataattcat ttttacaaaa aaaaaaaaaa tatttataat 44040
   tcaatatata tttttgaaag tttttagttt actgatgggc aagtatcata acttaaatcc 44100
   ggccgtccta cacttgcctc gcataaaata aatcaacatg cacgcgcatt tcaaatattt 44160
   aaaattgttt gattcaaaca tgctcagtgg caggtttaac ctgcgggttt tgagtcaatt 44220
   ttccaactct tgtcgttagg tttgtctatc tattatcttt atttagatga ctcttaaagt 44280
   gttgttcatg agtgttcgtt tctttatcta gttggttcat ttgttgtgat gaattgtttt 44340
   ggttgaaaat atttttacat gaggttttaa gcagcaacca aaaattgatg tttggtggcc 44400
   catcgtggcg acaaggtgat aatcggcgtg gttagatggc gaggaaattt attccttaat 44460
   aaactgcgta ttgagaaaat tggggcctaa cggtaacatt aaacattgaa tgcaacacta 44520
   actacagaat aagtttgcta agcaaatttg tttaaaagct tcgcaaactt tctattgatt 44580
   cgctgactca tctgggcgta tgctttacgt gatgcataca tatgtccttt tttaatcgtc 44640
   catgtagaac gcttacgcac agtttgctca acttcctcac ttcctctatg catttcagct 44700
   tttgctttct gttatgtagg aaccaatgtt tcaagttaga gttgagtgtg gaaattttta 44760
   agatctaaag aaatctaacc cacaagatta ctatttattt tcactaccaa aaccaaaaat 44820
   aatatcttcc taacatatat atgctgacaa caaaaaaacg tctctctcgt tgctagtcat 44880
   tctcatctct ctcacgtttt tttcttcgga gaaaaaacaa ggcggcacaa atagaggtgg 44940
   gaaagtttgg tgatgcaaat aaaattacac aaataatatg cgtttcttaa gaagaaagta 45000
   aaacttgaaa atgacgtgac gtgacgtgac acatgtcata tattgtacgg aactgacagt 45060
   ggaaccacgt cggggaccag tgctagggat ggcgttttat tacgctgtta agccacggtg 45120
   ttacgatatt ttgatggggc cacgagctct gctcaattat ataagagacc catctttttt 45180
   ttttgaaact aagaccaacc atctttcttg ttttggaata accgtttggg tttctattaa 45240
   gtttgcggtt tgctaaaaac ggttgtttcg gttttatact caacttttgg aaacttctta 45300
   tgacagtttt ttttataatg ccaatgccag tgttgacact cgtccgaaga gttacataaa 45360
   gctttatcag actaatagaa ttctctcact aatctgattt acttttgttt tcttgattag 45420
   aacatccgca aaaaaacttt ataacttcaa atttgctcta aaaaaagttt caaaattagt 45480
   ttaacaaact tcaaaaagaa acttcaaatt tgctcttcaa aaagaaacaa ccgtttagca 45540
   aaaactacta tatagagttt ttcctttcta aaaataaact tcaaattttg aaatttgaag 45600
   tttttagaaa tgaaacttta tatttgaagt ttcactactc aaaatttcaa atttgaggtt 45660
   tcatattttt atttacattt taaaataaag agaaacattt cttactttga aattgatcat 45720
   atacgagagc cttatgaaaa taattttatg aaataatatg atattttgct cgtattttaa 45780
   tatttaataa tgtaatttta tttataattt tatatattag tgtaatatct tttaattaaa 45840
   attgatgtaa tatttttata tatgtgttag ttatttataa aatatttcta tatttaatta 45900
   actttgacaa atataagaac catattataa aatacaaata atttaaagtt aaatttaaag 45960
   ttttaatttt ggaaaaaaac acatttaaac tttcgatata aaatcttgca aacttcaaaa 46020
   tagatagtct ttttggagat actgttagca gttgatatgt attaagtttt actctcctgc 46080
   taacttgtta ttgtaaaatt actccaagga aaaggtttgg ttattgattc gatccgatat 46140
   gtgaacccac gttttgttta cctggtttgt attaaaggaa acagtaccaa aactttaggt 46200
   tctcaatggt gataataaaa cagttttagt aatataaaca ataaggaata tgagtatact 46260
   gtaatccaac caagatttag gcgttacacc caataagtaa aattttcata aaataagcgg 46320
   tacggaataa tggtgattag atatattttt tggtacaaaa taaatatttg attaaaagaa 46380
   tatcaaaatt gttcgaacat tcacgaaact cacataaatt ttatttttgt ttgtttgatt 46440
   tggtttaggt aataataata gaaatatata ttttgtttga attaaaaata tgaaatagta 46500
   aatatctttc ttagtgaact attctttcaa aagtatatta tttttgtaaa gatatttata 46560
   tgtttttcaa atcaaagtta tctaaattta tatataaatt ctaaattatt tttaaataaa 46620
   aatataatat aatacatgta agataatata tctttagttg tatttaattt aataatctgt 46680
   tttctctagt aatatagtaa ttagtttttt ttgttaatta ctctatatgc taaaatagag 46740
   tataattgaa atatagtcca attctattat aaaattatct taaagaaaaa aaaatgaatg 46800
   tgtcattgga gatagaatta aggtatcatt ggtagagtat atatctagaa aagtttccta 46860
   ccattattat tatattgata tttaacagta accttttata tgttttaatc ttaatcaaaa 46920
   actagattat gacctggtat taaaaaatat tttttttaaa aaattcattt tactaattaa 46980
   tatgttttaa catttatttt attgtatttg aaaatattat tttgtattta ttttatatat 47040
   atgaaattat atatatatat atatatatat tacttaattt tgtttttcag ttatctcaac 47100
   cttattcgtt atgatttttt taataaaacc tctccaaatt atttagataa ttatatgata 47160
   tatattttga catattttaa gttatacaac ttttttaatg tcaagttatt gactttaata 47220
   ttttatttat atacaaattt taatttaata tgaaatattt ttaaatttaa atgaatgtat 47280
   ttttatttat taaaatataa ataaattaat tcattgattt aatttatttc atgtataaaa 47340
   gaattattat ttttaaaaaa tatagttgta cttattttca aaattttctt gaattatttg 47400
   agtgttttaa tcaattattt gatttactaa ataattaaaa aacaatatta ataaaaagtt 47460
   attaaaaagg taagatataa tttttttggt cacaaaatca attagtatct tatttgaaaa 47520
   caaatttatt agtatgatgt tattttccat agctatcttt aacgaagttc taatgttttt 47580
   ttttttaagt tttaatgttt ttattaatta ctaataacat taaaaataat atattgtatg 47640
   acgaaaaatt agattcaaat gaatgtgtct attttaataa gatagattat ctaagaaacg 47700
   acacatgaca tgttggtgac tttttaataa gaagagattc ttttatgtca tctctcatac 47760
   tttaaaaaat aaaattattg tggttaagag attcaaagtt ttttacacca ctgctgggtg 47820
   tgctctttaa gttgttgatt aatgacgatg tctagagttt taattttacc tcaaagaaaa 47880
   ggtttgggct gtggatttga tgtgatgtga ccccacgttt tgtttatctg gttcacgttt 47940
   ttgaaatcca tcgatataac ttataagcag cagcatgcat cgactgtagt ctttagctgt 48000
   catcaagacg tttaccactc acggaagtgc tcagagatct tttgctactc ttttttcttt 48060
   gttcaacgga tcttttgcta ccaaaagaga aaaaatatca aagcatagca acttttgcaa 48120
   tttgaaaatg cacccaaatt ttctattatt taccaaagag cttcagagaa ttttttggct 48180
   atttatggct ccgaatggta acagcggatt gagcggtgcg agacaagcgg tttgactgca 48240
   gtgcagttct gacaattata aaaacgtata gatatatggt atatgtagag atttttgtta 48300
   ctgtggactg cagtgcggtg cgggacgaat gttaccattc gaagcctatg aagcatccaa 48360
   aaaaatttct gtacctaaat ttgttgtctt tcaaaataat tttgggaaac tcgtatatta 48420
   accatacaat agtcaccttt gaactataca tgaaaatttc atacatatca ggacaaatca 48480
   tggccttgta agaacgagaa ttatacataa tgaaacataa acaaattaaa attaacaact 48540
   aacaactcta caaacataaa aaacattatt caaagtttaa tataaaataa cattgtttaa 48600
   acttcaaaaa atattcataa ttgagcgtca agtgcagttg tgaagacata aaaacatcaa 48660
   tattgaccaa ataccaaaaa tagttattaa agtaagtatt ttaattaatt atttaagtaa 48720
   aacataatta ttttaggcat atgataatat catagtatac tttggataca tattaaggat 48780
   tgagattgag tttggtataa attttttttt cggattttga aattttcaag ttttttttcg 48840
   gatatccatt cgggttcata gtcgaatctg gtaaaattca taacttgaaa taccagagaa 48900
   catgatccat tcagtattta tattgggttt ggatcggttc aaatttattt ctatcgagtc 48960
   gggtttgatt tggattttcg gattcagttt agttgtccac cactaatttt ctatctaaat 49020
   ttgaaatatt ttcaattatt gaactgccta ataccttcac tatttacaaa aggttttgaa 49080
   acttatcgcc tatacttgtt gttctattca cacacacaca aaaaagcccg gaatttttat 49140
   tttgttttct aactgtactt aacttttgat atttactatt ttaccaagag gtttccctat 49200
   aatttgtgct attcataatg atgcacataa atttttctat ttattagata ccccgtaatt 49260
   tttgctaaaa gaagtaacaa ctgaagtgtt ccattccata tagtttctat atacaataat 49320
   acccctgctc taaaattcat ttaactcggc tgcctagggg gcgagtacac atgaatcggc 49380
   caaccactgt ggtgaatcaa cataattggc catattactg gagtattttc atttttgttt 49440
   ggattttttg ttaaaactca atgtacttta gttacatttt tactgtgaaa aattacgaaa 49500
   agctgtacga aattttttca gagtttgagc ctaagttcaa ttggacgtaa gtcactcagt 49560
   taccacttag accaaagagt ttttttgtat tagctacgca aataccctta tataaatata 49620
   aaaagttgaa aaactaatcc cgactaattt ctgatttttt gataactcac cgaaactaat 49680
   ctcatgactt ggtattcaca aaagatttat aaacttttca catttaccga gtcgcaacat 49740
   tgttacagat acactcgctg tcacaacacc aacaaaatat acaaaaaata aaggatgtcc 49800
   tataaaaagg aagaatccaa aaatccaaaa aaagtactgt attatacaaa aaccggaaat 49860
   aaaaaatctc tgtattaata actccaaagg gacacgtcgg tatctccgtc aaagtcatag 49920
   ttatattcga attgtacgga cggtggcggc aaaagcatcc cttcggccat gctagccaac 49980
   aaggtcggca tcccgaacat ggactcctcc tcgtccatat aaaacccatc gctgttttcc 50040
   tccgtaaaaa tagcctccac gatcgtctcc tccacgtcca agccatgatc cttcgtcgta 50100
   tcatttatct cagcctgaaa agccaccgcg gcttcagcag ccgccttctg gatatccttg 50160
   gggcatgttg tctccgggat acggagccgc caagccgagt cggcgaaatt gaggcaggcg 50220
   gatttgccac ggagggctat ggcggcgacg tcgtgagcac gagctgcgat ctcggcggtt 50280
   aggaaagtac cgagccaaat cctagacttt ttgtttggct ccctcacctc acacacccac 50340
   ttacctgagt ttctcagacg tactcctctg taaattgggt gacgcgtctc ccgaaacttc 50400
   ttccgacccg caggtttctt cggacagctc gcggccagcg tcggacaata ctccccgctt 50460
   aatgtaggag actcgtactc ggagcccaac atttcagaga aggcagaaaa tgaggtcatt 50520
   gttaactgga taaggttgag tatagtaagg aactagaaag atctcggttc tgatgggttg 50580
   ataaatgttt attttatctc tcaggtggat tctaaagttt gtagttcgat aaaaagttgg 50640
   gagtgagagt tggtgtttat attggcctct ggaactagag gcgaacaaac atggagtttc 50700
   tggttcctgg agtgacaagt gcgagtgtgt gacttgacac ggctaagcca tcccacggtt 50760
   agtgtatgcg ctgtttttat tcactaagaa tctcacacgt gttctactga cccacaagaa 50820
   atgacttttg agttttgact cttcattcgt tattaagtat ataaatttat taattttgaa 50880
   aataatggac ataaacaatc tgcaaagaag atatttttag tgttctcttt ttgttttctt 50940
   ccttgatatt tttaatgctc tccctatgcc ttttttttgt caataatctc catgatttta 51000
   tttttcattg atattgaata tcctgtctat gtgttttttc ttgatacatt acaaatatat 51060
   aaatttatca atcgtggatc tagaaattat tttaactaga agcatatata tattaagata 51120
   ataaactatt taaataaact atttttataa aaataatata actatgtaat gttttttgga 51180
   cgaaatttat taaaataatt ataaaaaatt agtttatagt attaaactat atttaaactt 51240
   agtatctaca acatacacat acagtaatat aatatcaaat cattcacaca tcacagagta 51300
   gaaaacgaat gattttatag tatattgaaa acaagagagt tttctttcaa aacatttcta 51360
   tctttctctt tctttttatg tcgtttcaat ggaaaaaaaa actagatgaa atatttcgtt 51420
   tgaacttgcc ctagatcttt tcatttattt ttcaatatac aatacaaaat cacttatttt 51480
   cttttctcat gatactggtc caatagtaat ttggagtagg taaagaacaa tttgtaaaat 51540
   atacagttat gattagttct gttcaaataa aaacatagta ttcgattgcg tttctcttat 51600
   ccagtcagat ggttcttaaa gtactaggta gtataatata ataatataag ttaatctaag 51660
   atgaattgag cgaataattg agcgaaaata attgaccaca ggattagacc gatagaaaag 51720
   caaaaaaaaa catcactctc atttgctaaa aaaaaacatc actctcaatc tcaaaatata 51780
   tcgataaaat atctgaaatc aaaataatat ctttttcttt tttttgaaca catcaaaata 51840
   atatctatga aaaaaatcgt ggtctaaacc taaatcacgt ggtgtgagta tttaaagccg 51900
   gacgatcgat caaacttaca agattttata ttcttactat aaatccagaa agtagtttat 51960
   attcctaagt ataatggaaa caagaactta accaaaccaa aaaaaaactg aataatcttt 52020
   tttctgtaaa ctaaatacaa aactgtgtca aattttatac atatctattt ttttaaaaaa 52080
   tatccaaaat ttagaagaat tgaatcaaaa accaagtgga atatcaaaga ttttattagt 52140
   atagatatct ttatcaacat gtatctaaaa tttcttctaa ttaaattaat aacaagggat 52200
   gataaaaaca tgggaaatgg tgggaatgca accattatca tgagagtaac tgagatctta 52260
   ttatggtaag tttaagaata ggtataatta taagattaat ggtttattaa gtagtgatat 52320
   aattatataa gatttgaatg gtacatgtga gaattatata acatgaagca acattgttat 52380
   aatttacggt gtcgggtcca gactcttccg gcgtttaaag cagataaaaa aactgatgcc 52440
   ccttaactat agtaaaattt tactatattt taaatttata atcaaaataa tgctaaatat 52500
   tattacaatt tatgatattt ttaaagaaat aaaatgcaaa acatcaaaac attttgcagc 52560
   tcctctagac tgtttttcct tctcattgtg ttcataaaat ttcacaaaaa ttgtttatat 52620
   atgggtttat tcagttgaac tcatcagagt attattatca tagtccaacc accaagcatg 52680
   aatcttgtgc attcttttca aacttataat ggtttataca ccatctttta tattatatta 52740
   tttcgaagct ttttttaccg taagtttttt tctgactcta catctagctt attcagtttc 52800
   ggaatcaaaa agataaaaac gttttctttt ctaaaatagt agtgtttttt aaaccagacc 52860
   ggtctgatgg ttgaaccggg tttgaccatg aaccggttgc atagcagggt tggaactaat 52920
   aattggtttg accatgaatc ggttacgtag ccggattcga tctcaaagtt attaaactga 52980
   taaaaatcat taaaactatc aaaaatcaat ataccattca tttaaacata aaacaagttt 53040
   atatttttaa tattttatca tatttcattt atatttttaa ttatgtatca tatttactaa 53100
   cattaatttt aaacttatac actaaaacat agaaagatta tagaaaacaa actattaaat 53160
   ttttttgaca cacacaaaag aaaaggatta taccaacatg ttttattatt tctggtatca 53220
   ttcataaggt gaaaacaaaa atcaaatata accataatag ttgtaaaata tactagttaa 53280
   atacgtttta attataccaa ttataccgat tgtaatagct atattcgttt tagttgtact 53340
   agttatatta tttttttgtc ataacaacca atgaaaaatt attgattgaa gaagattatg 53400
   agttaatata ttttcgttga attgtatttt tttggtgaat catgtttttg aaagtattat 53460
   aagatgaaga agatgaaaat agattttttt ttgatttaat gtaaaaaata tccagaaatg 53520
   aactggtttg gtgatagata gcaaaaataa atttaacaat gtatcacctt tcgttgacaa 53580
   aaaaaaaaaa aacaatgaat cacctttctc atttaaaaat aataaaaata ataagaaata 53640
   taagtattgt agaattttaa taagccacta cgggcacata agaatttgat cccacacctt 53700
   tgtgacaacg cctcggcgct ctggaacttt ctcgtcgcaa cattctcttg actggctcaa 53760
   gtttgacctc ctgttaatcg taagatcttt ttcatgaata cgattcctct agatttgttt 53820
   tcgtttcctt tttgtttctt gattttgttg ctacgaactc ttagggtctg cgatgcttgt 53880
   gctttgcgat agctctctat atctcttaga ttcttttcaa gaaagttgat agcttcatag 53940
   attaagtatt agatctctga aaaatttgca actttggaat aacagtgttt cggcttaaat 54000
   tgctgcacat aagatgttcg acgatattcc tctgagaaga taactactag acatgctttt 54060
   gttttccaag tttcggtttg attttactga acagtaatca catacgcatc tctttatgga 54120
   tgagacccac cacatgtata aggaagtgac cattttattt tggcaggttc actgtttcag 54180
   tagccatggc aaagcatcac cctgatctga tcatgtgccg gaaacaaccc ggcattgcca 54240
   tcggacgact gtgtgagaaa tgcgacggga aatgcgtggt gtgtgattct tacgtgcgtc 54300
   cctgcactct ggtgcgtatt tgcgacgaat gcaactacgg gtcgttccaa ggacggtgta 54360
   ctatttgcgg aggggttggg atctcggatg cttactactg caaagagtgt acgcagcagg 54420
   agaaagacag agatggttgt cccaagattg tcaaccttgg gagtgccaag acggatctct 54480
   tctatgaacg taagaagtat ggattcaaga aacgatgaag atgtattggt ttgcccgatt 54540
   gctggatctc ttatgctatg tctgttgcat gataaaacta atatgtattg ggtataaaaa 54600
   acccatacat tatgctttct ttttcttgat aatctagact ttattggact tatcttagtg 54660
   tctaaatagt ctcttgcgtt gtgtatcgtg tttgatttca tcacaccaca gtagaagtag 54720
   gcatgttctt ggactcttaa tcatgttttg attgaataca aaattactaa actacatgta 54780
   ccgctcaaat gcaatcatgt taaaacataa taaattttag tttatccaaa ctgtgcgagt 54840
   ttaaataaat aaaaatgtta ctaaatactc aatccgttcc acaaagatcg atttttttag 54900
   tatttttacg tatattaaaa aaatacatta aaccgtcata attagtgtat cattttcaaa 54960
   aaaattaatt gattttattg aattatcatt ggttaaaagt tattaaaaca taaaacaaat 55020
   tttttttcta aaaagtctat catgacggat ggagtaatcg aaaggactgg tgtaacaaac 55080
   aagagtgttt gaggaattgt tgtgatcact tgattagcgg atgcagtagt ggttgactga 55140
   tcattttctt atataaactt gggtctgttt caaatgtaaa tcgtgggtct atttatttgc 55200
   agtggtttaa aaatgaaaga tcatcgcatg aactaatttg atgattatgg gctatctctt 55260
   ttttctaaac ccagaaaagt ttataagata gatgggccca aagcctgtta agaatcgtat 55320
   tatattattt taaaaataga agcaagaaaa gaagaaagat gaaacttctc cttcagctga 55380
   tacagatctt ctagacagag acatattcaa atgcttccaa agctcaggga aaattcctaa 55440
   atcagattcc atcactttga ccaaatacta agaagaagaa agatgttctt gatcaagaac 55500
   ctcagacgaa tctcgccgac aacctcctcg gccctgatcg gcttccgaaa caccggatca 55560
   ccccctctct cctcccgttt ctgcaccact ctgaatcaac cccaacaggt ccagactccg 55620
   gctcccaatg gattggatcg gagccgttac gaaggtttgg caccgacgag agaaggagag 55680
   aaaccgagag tggtggttct cgggtcgggc tgggcgggtt gtcgtttgat gaaagggatc 55740
   gatacgagca tctacgacgt cgtttgcgtt tcccctagga accacatggt cttcactcct 55800
   ctcctcgctt ctacctgcgt aggcactctc gagttcaggt ccgtcgctga gcctatctct 55860
   cgtatccagc ctgccatctc gagagagccc ggctcgttct tcttcctcgc taattgctct 55920
   cgccttgatg ctgattctca tgaggtatta ttactgtggg aatcatctga atctcagcat 55980
   ttgtaactga accggaaaat tcgaattgaa ccgatccata ccgaaattga ttttgtaggg 56040
   ttgtatttgg gatgtcccaa aaaaaaccaa acaggaaaac ccaaaaaaac tgaacctata 56100
   taaatactct ttttttagga acacctatat aaatgcttta aatattcaat cttataagtt 56160
   attttgatgg attttgtaat aatatccgaa tccgaagtat tattaatcaa acttgaaaag 56220
   gttcagatct tagacaatgt tataaaattt actagaatcc gaagtattat taaccgaatt 56280
   atgatccaaa cgtatatttt ttccgtttct aaaaaattca tattttagga ttttcacatt 56340
   tattaagaaa atatatcaaa ttttagttac ttatacatta ttttccgtaa ccaactattt 56400
   cccacaagtt ttcaccaata gaattttaat aaatacaatt atgttttttg aagtttacaa 56460
   tttacattta atttatgcat tgaaaatatg aaaatctatc tttttgaaac aatttttttt 56520
   tctaaaacat ggatatttta ggaacggaga gagtataaaa attcttctgg aaccgaaccc 56580
   gaaagctcat gcacttttga tgaaaaatat ctttgcacgc tttcttaaat gtttgtcatt 56640
   ggggataggt tcactgtgag actttaactg atggcttgaa cacattaaag ccgtggaagt 56700
   tcaagatagc ttatgacaag cttgtggtag cttgcggtgc agaggcctcc acttttggaa 56760
   tccaaggagt tctagaaaac gccatctttc tccgtgaggt tcaccatgct caggagattc 56820
   gcaggaagct tcttctaaac ctcatgctct ctgatactcc tggtaagtga taaacaaata 56880
   atgttatatt tctcatgaag aatcaaaatt attagcacag aacactttgt tttaaattag 56940
   gaatatcgaa agaggagaaa cagaggctgc tccattgcgt tgtggttgga ggtggaccaa 57000
   ctggggtgga gttcagcggt gaactcagtg acttcatcat gaaagatgtt cgtcaacggt 57060
   atgctcatgt gaaggacgat gttcatgtta ctttgataga ggtttgtttt caagaagctg 57120
   cttcttcagg ttcctcctta tgtgtgtttc atcacttcac aattgtctct gttttatgtg 57180
   attatttaca ggccaaggat atactttctt cattcgatga tcgtctcaga cgctatgcta 57240
   tcaagcagtt gaacaaagtg agttcattaa tggttttaaa aatcaatcta ggcggcaaat 57300
   cgtagtcgaa acattttttt tttaaatccg attatacgat tcaaaccagt ataaaccatt 57360
   cttaatcggt ttaaattgat ttaaaatagt ttaaatctgt taaattaaat aatcatgtta 57420
   gtacagattc acaacttgtc ttaatttttt tgttttgtat tatctaattt tgataataca 57480
   tcgaaataat tatataatta aatccaaaaa ctaagtatct tatataaata taaaataaat 57540
   caataattca cttaatcatt agttttctac attataccgc ctagcgattt cttgtggtta 57600
   atttataaga cgtgaaatgt ttctgtgctc attattatgc tgcattcata tacattatta 57660
   gtctggagtg cggtttgtgc gtgggattgt gaaagatgtg aagccgcaga agctaatcct 57720
   tgacgatggc acagaagttc cctacggact cttagtatgg tccactggtg taggtccttc 57780
   tccttttgtt agttctcttg atcttccaaa agctcctggt ggaaggttag ctcatcaaca 57840
   tcactacatt agaccctttt ttttttgcga aaaatattcc acatcggcta agactttttc 57900
   tatctttttg tccctgtata gaattggtat tgaccaatgg atgcgtgtac cttctgtaca 57960
   agacgtgttt gccattggtg actgcagtgg atatcttgag accactggaa aaccaaccct 58020
   tcctgctctt gctcaggtaa actttttaga tagataagct tcataatcgt ctataccttc 58080
   tcatgccttg ttatactacg ttactgctca attaaggtag ctgagagaga aggcaaatac 58140
   ttggcgaatc tactaaatga gattgggaaa gccaatggag gacgagccaa cagtgcaaag 58200
   gagatagcac ttggagttcc ttttgtgtat aagcaccttg gaagcatggc aacaatcggt 58260
   agatacaaag ccctagtgga cctccgcgag agcaaggtaa caaatatttg actatgattc 58320
   acctcgtaaa acaatgtggg gttgagagag attacttggg caggacgcaa aagggatatc 58380
   aatgactggt ttcgtgagct ggttcatatg gagatccgct tatctgactc gagtcatcag 58440
   ctggagaaac cgcttctatg ttgctattaa ctggttcact actttcgtct ttggccgtga 58500
   cattagccgt atctgatgtg tccgaatcca ccagtgtgtt ttgacctcgg tttactttac 58560
   acgtcgtcgt tttttgtaca aaattacaat aacacaatct tctgaagact gagaaggttt 58620
   taaattatcc tctttttttt ttgttgttac taataatatc tttggttgtt gcgatttcgt 58680
   ttgaagaaaa aagaataatt cagggttaaa tatttttttc agggttaaac aataagtatc 58740
   tggaaaataa ttatcagtta tggattagac agatgcccta aagagtttat atttaaagtt 58800
   tctattttga ttgaattaga aaatattatt tatagtttta atatgatatc ttaaacaatt 58860
   ttttgcatca aagtaggata gttgctgttt taatttttaa tgtaaaatca agttggtctg 58920
   caagggaaga catccaagcg accgcttagg acatataatt ttaaaagaca tatttttata 58980
   tatttatttt tattcagaac ttcgatagtg tttatatgta aaaatattta taatattttt 59040
   gataataata atatttgtaa gaatttttac cctcgttaat agaactctca ctaacaaata 59100
   aattggaaaa atgtattgat aaataatgat tattttaaaa tgtaaaattt tgcgtgaaat 59160
   atttatggta atgttaacta atattgatgt gcagttaatt tattaaaaat atgtttacca 59220
   attagtagtt gaccaaattg gtttatcaag ttttaatgtg atttatatca tatagatatg 59280
   atattagata aaacataaac atatatatta tttgcagaaa ggctaaccta aaaagaaaat 59340
   ggataaggat catgatgact atcccaccat gcttgttgag atagtacctc ttaagatatt 59400
   ttgaatttca atttatcaaa tagatacttt attgattgaa aatagcaatg ttagtagctt 59460
   aaggtatagt attaaagatc aaatgggctt tgaatcattc ggactacgta tgtccaatag 59520
   aggtttatcg gctctatacg ctgaaatgaa aggactatta tgacagtatc atgcatgaga 59580
   gacgagaggg ttcctttggt ctggtttcaa atggattgct cagatttagt ggatatgact 59640
   acgagatcga tagactggct gttttttgct ttggatattg gtgtgtttcg gagtttacat 59700
   gatgattttg agagcatgag catgtacttt ttagaaaaac gtctcatcta atccatatat 59760
   ggtctagtga tatgaatagc tgataaaaaa aaagataaat tgatttttaa ttttaatctt 59820
   tctggttctg aaccggattg tagatttatt tatttattta ttttagttgc tttttctttt 59880
   tttccacaaa tttttttatt ttaataccaa aaaatttaac atatctaatt tgaaaacttt 59940
   tgtcaaaaaa atcttagggt atccaaagat gttagatcaa cactatgtaa atttacacga 60000
   tttatattag gtttgttttg tagatagatt ccctaaggct aaaacatcag aaaataaagg 60060
   taatatttag ttgcccaaaa aaaaaggtaa tgttaaatat tggatcttat atattcacat 60120
   gttcatgtca gttgccacgc atgctcatgt actactatgt gtgtgcttgc aattcaataa 60180
   acaatgtcgt cgtatttaat atttctgaaa agtctttgta gtttgttatt cttaaaacta 60240
   tataaaaaga tgtttttttt ccaaatcgtt ttacacggaa acataatgca aagtaatatt 60300
   attttaagaa aaggtctcat gtacagttaa cgaaaggaca agatagggaa taaagtgaga 60360
   aaatacaata ataaacaaag aaatgaatat tgaaatattg gtctataaaa tctcaggacg 60420
   gctacggtga caatgtctaa aactcatttg gtctctctta tgtccaaatc agattttttt 60480
   tctctgaaag aaggtctacc aaatcaaact tcttctctac cgattgctaa acgactcaca 60540
   ttcatcacgt acataactaa tattttctct gtttcaaaaa aaagatgcat gttttataat 60600
   tcttatacat attaaaaaaa atatgaaatt ttgattacta atatagatta atttttgtaa 60660
   ctaactattt ctcctaattt ttaatcaata gaattttaat aaacacaatt atatttttca 60720
   aagtttataa tttatcatta attaatacat tgaaaatata aaaaatacat ttttagacaa 60780
   tttttttcta aaacatgaac ttttttggaa cagaagaaat aattgtcttc gtaaatatct 60840
   ttttgcctaa tcgttataaa actttaaata tataaatggg agaatatatc gtttagatcc 60900
   gataccaaag gggtttgtca attatttacg aacgaaaatg gcatgaaaat gcctatgtat 60960
   ttcaatcaag gcccttaaat caactgtttt ctctcagcaa aagtaagaaa aaacgatttc 61020
   aagactccag actcatgatg ctatttgaga aaataattac ctcttattca tctagttcat 61080
   gtttttaatg catatatgta aaaagatgaa agtgaccaaa tgtgccagca aaaacaggac 61140
   tatgacttta cctttcagct ctattattta aactttgctt atctttcccc caaccaacta 61200
   agaaaccttt gtctactttt ttgtagacat ttgcgcaaga gtcagtgtga acttattgat 61260
   tcggggaagc aaactcatta ctaaaggcat cattatcagt ggatttctac agctgagtat 61320
   ttagacattc gtttattaat attttaaaat aaaagaattt ttataatcat tctacagctg 61380
   agtttatgaa cattaacagt agatttctac agagaagttt gaaatagtct tgtatcagtg 61440
   acaaaatgcc taatgaattt atggtttctc aatatctcta aagagtttct cagcaaaaag 61500
   acaattctca ttttttactt ttatgatatt tttaatacaa aaaactcatg aaagaaatgc 61560
   caataaaaca aggggcaatt tgttggataa ccatagtagg aaaacaatta acaggtaata 61620
   aaagaatata aactctgaaa cgtttggttg attgaagcaa tgtagtaaat ctgaaactta 61680
   tttggttgat ccaaaccgaa acctgttctc tctaatggga gtatgcgtcg ttgtaagata 61740
   ttcaccgtga tcatttacaa gttgacagaa acaaaaactt tttcctaggg aaaatattga 61800
   tgaatcgaaa aaaggagaaa gcctcgaacg agatgtcatt gtttagggcc aaataattaa 61860
   ctggataatt agagatttgt tagaaagtaa agccattgct tctttaggaa tagaagacaa 61920
   cgtgtttcgt cgtttacacg tgcacgtaca acatcccatc tttctttttc ttgtccaaag 61980
   ccatcactct ttttttctga acaactcttt gaattgttta atttacatct aattatcttc 62040
   aaaaattggc ttgattaatc acatgagatt ggtctaatgg tatgtagact acagagagat 62100
   ccgggttcac taaacctgta taatcataag gatatggacc attgtttaca acccatttaa 62160
   aatatgaaag aaaatcaatc catgacttcc ccttagaaaa ttaatatgga ctcttccata 62220
   atagtatctt tgaaaaatat atactctgtt agatataaac catacatata aatggtttgt 62280
   gatgctgaag agacatgtat tcatgaagtg atcgtgatct ttaactgttc tttttcttaa 62340
   tggttgtatc tttaactgtg ctttgtattg atagattagc cacgttttta ttcaacgcac 62400
   acatattatg acgaatatta agggctttaa tgtacgcctt tcacctttgg tggaccacta 62460
   atccatgtta atgattttgt tatgagaagt atagaagcaa ttcacttatg acaaattgac 62520
   aatatagggt ttcggaactt cggttccgcg cgaatctcct ccaaaacaat gaaaaaaact 62580
   cagtttgtat gggcctagct agaaacaatg gtctctggtg ctatgaattc gaacattctg 62640
   gtgctatgaa ttcgaacatt cttttgaatt catatgatcc tctacaaggt ctgaaccaag 62700
   ctactctacg gtccatgact ggcttgcgca actttagtgt agtccagggt tttttttgtc 62760
   gtggtgatga gtctatatgt ggttggaagg ttcatgcagt acaataaatc tttgttttag 62820
   cgagctgttg tatatgtggt acacgaaaag acatcattct tacgacgtgt tctataccaa 62880
   ctacattccc tcaacacttg tattggtttg ttcgtctgaa tcaacaattg tgtcttttaa 62940
   atgattttta tgattagttc aaaacccaaa atagttaact aacggggcaa aaatggtaac 63000
   gaatagctta actgattata ttttccttta taaccctaca cattagagat atttcagtgt 63060
   aatatataag ttactagata ataacccgcg cattgtgcgg gatgtgatta ttagttttct 63120
   tatttttaat aaaaagacat taaatctatt taatctagat attagttcgg ttttaagttt 63180
   ttttttggat tttaatcttc taaaataaac tattatttta aattaatatt cattttagtt 63240
   tattcggtta aaatgtttga ttttttttta tccggtaaaa accaaaaatt aatattattt 63300
   atttattttc atgttatgaa ttttagatag tcgtcatgtc aaaccaatag attcatatta 63360
   ttgtttctaa acagataata gttaagaaaa ttattaagac aaattatttc actacaattt 63420
   ggttggtagt gaaagaagca ttaagaaaaa atattttaac tttcaaaaaa aaattagata 63480
   cttcagttgt ggtgaatact tagttataag gtgctcacat caaaatgcac atgtatgtgt 63540
   atgtaaaagt atatataaat agttgacaaa tatataaaga tattgttagt taataataaa 63600
   tgacattttt ttttcaaaac aatacatgaa agataaaatt aaaattaatt taaaataaaa 63660
   aggcattgac gttagtcatt tttttatata aataaattaa aattggatcc gtaaatagag 63720
   gtggacacat atcgaatatc tgggtatttg gaaacattcg tgtcgattcg atctttagcc 63780
   acctagatat tcggtgactc ggatatccaa aatattttag aattttaaag aatatccgat 63840
   ttgatccgta aataaaataa aattttaaaa ataattttaa taataaaatt ttattacaaa 63900
   aataaaacat tatttaactt tttaaattat agtacctaat ataataaatt taattcatta 63960
   aaatattgta aaactaatat aaagtataat atataacgta tatatataat tctgtacata 64020
   tatgtatata tatgcatata acatagcaaa ttagatattt gttcctaaaa atattggtat 64080
   ttgtgatttg cttctttttg gatattgtat tttagtattt gatttatttc ctagagttaa 64140
   gtatatccag attttttggt tcaaatcaaa acggataaca aatcgaatcg aaatttatga 64200
   atattttgct caattttatc tgtaaacaat aaaaataaca tatatatatg gtttggcttt 64260
   tgatttgtta tctattttta ttcgaaccga aaaatctaga gttttattga aaccatgtat 64320
   gtgagattta tgttaaaaaa aatgcaaaat acatagtgtg cacacattta tgaatatagt 64380
   atgaacgcgt tagtatattt attatcaaat cattgtgagg ctgccacgtg tctattatag 64440
   tgtgaatgta tttattacaa tgcttctctt ttaatataca agggattttc attgtaattt 64500
   gcaaatttat aacaggcagc atattccccg ggcctactct tcatattatt tttggtgagt 64560
   agcgtaatca tagatagttt tcttaattct tgaacttggg taacatcgtg ggtatctacg 64620
   aaatgattcc tttcgacgta cacgatttat agataaacac gtagagacgt gtataataag 64680
   cgagaaactt atttagcagt gttagagaaa tatttgagtt aacagactat agaaccttta 64740
   taaattagta ttcaataaat taatattttt aatattcaat aattaatatt ttaatcttca 64800
   gtaaaaaaat ataatattcg ataacttagt attcaataaa ttaatatttt caataaatta 64860
   atattcaaaa aattaacatt tataaaaaat cattaaatta tattgtctca ttacaattgt 64920
   aaattaataa ctgatgtata aaaattatat aaacataaca aaatattgtt atgtatggtt 64980
   tttatttaaa atgaaactaa ttctaatttt ttcaacactt caaagtattt tataattata 65040
   tatttaaaaa tattaacatt atgtgattca tattatatat atgtcaaata atttaataaa 65100
   cactatgaaa gctaagttta caaaacttaa ttaatatata attcacgaaa aaatctattc 65160
   cttttatttt acatataaac atattttaaa atatataaat ctaagtatga tattttgata 65220
   aattactaat tttataaatt aaatattata gttcattaag tattttgaat aattattgga 65280
   tctttaagta ttttgaataa ttattcaaaa ttgactcatt ttgtttttta agatttttaa 65340
   aaaattgagt ttttttttcg atttccgtta gaatttgatt tgggtaaaaa ctaaaatctg 65400
   aaataccata gaataataac catttggata cttatgtcga attcaaaaca gtttaattct 65460
   caggttcaaa ttttcatatt gttttttcat accatagaat aatagccatt tggatactta 65520
   tgtctaaaag taatataatc tgagacaaaa tataaaaata taaggattta tatatttcaa 65580
   ccatatggat atggttgtgt gatacgaaag tgttagacat tatcgatttg aaatctatca 65640
   ttcagatttg tcttttacat ggttaaaggg tgtgtgaata taaaactttc acgtagaaca 65700
   acggatttat ctgttgcctg aaaaacaggc taaacactct attatgatta gtcttagatt 65760
   taggacaccc ctggtccata aaaaaggtct tacatattta ctttcgcata catatttttc 65820
   taatttaatt tcactgaata gaacgatgta acaaagtaac aaacccattg catttaaaat 65880
   tacagcaaat tatccttttt ttaaatatat aattatttct ttaaatatat atatattttt 65940
   ttattttttt ttcaacaaat atataattat taaaaaaaac agttttgagt atctcaatca 66000
   attctacaga cttacacatc ctccttcccc tttatataaa gaaacttcag acctcaaaat 66060
   acatcgaacc ctttcttcac cacattccac ttcccacact ctcttttttt ttgaattata 66120
   gagagagaat cctcctccaa atctctctct ctcccaggat ggttgttgct atggaccaac 66180
   gcaccaatgt gaacggagat gccggtgccc ggaaggaaga agggtttgat ccgagcgcac 66240
   aaccgccgtt taagatcggg gacataaggg ctgcgattcc taagcattgt tgggtgaaaa 66300
   gtcctttgag atctatgagc tacgtagcca gagacatttg tgccgtcgcg gctttggcca 66360
   ttgccgccgt gtattttgat agctggttcc tctgtcctct ctattgggtc gcccaaggaa 66420
   cccttttctg ggccatcttc gtcctcggcc acgactggta aagtttcttc cattttgcat 66480
   tgcatcgatt tattgaatgc acgttctacg agtattgttt gtcagttact tcgtaaaatg 66540
   attcttttga tgttcatttt ttgaagatct aagatttttt ttttagattt tctttttaaa 66600
   tcattgttcc accaccacct ttcatcggtc gtacgactcg ttacaacacc acatctttat 66660
   tttctataat tactactgct tccgcatttt atggatctct caacttataa ttaaagtata 66720
   atatcaagaa tatctattat ttttcttaaa caagaaagat aatattgttt ctttgttatt 66780
   ttggtgtatt tccaatctat ttcgagattt agaaatgtga cacgtcatta ccttgttgaa 66840
   gtgtttaaaa caaacatgga aagtttaaat aaatagtgca ataaatgata tatatgtata 66900
   tgatgaataa tgatgtgaaa tataattgaa taatggcagt ggacatggga gtttctcaga 66960
   cattcctctg ctgaatagtg tggttggcca tattcttcat tccttcatcc tcgttcctta 67020
   ccatggttgg taagtcagct tatcaaccct ttttactata ttattaatta ttaaacttgc 67080
   atttgtatac ttggtgcaag ttggtaaatg taatctgata actgaaaatc tattcattgc 67140
   tcgttctatt ttttttttgg ctagagacaa ttttataatt aaataatgca tgtgagaata 67200
   tgactattta tgtgaggtag cttttcttat tcctgtcgaa aagcatcaaa tctttagcaa 67260
   cgaaggaaaa aggaatcaaa ttttttatta aatgcaatgg gtctatgtct tggtcattag 67320
   ttttttgcat ataatttatt tatatttttt tcttaacagc agctaattta attataatta 67380
   aatattcatt ttataaataa tattagacca attattaaag gttagatatt ttaagaatta 67440
   ttcatgactt tgtttattgg aactcctttt atcttttaat cttttctatt tctccatttt 67500
   taataatgag aaactgactt caaatctcca ataaagatgg tcttatgtag taacagtata 67560
   attttttgtt tggtaaatgt aacatcatct tcaaatatct ttgaaaatag acttacatgc 67620
   attattttgc tgcgacatta ttgtcactta ttcctggcaa taaattagtt tattactgaa 67680
   cttttttttg gtcaatttat tactagtaac tttaaactta aaagagtgag attgtttgat 67740
   caaaaaaaat aaaaatagag tgagatagtt agaatctgcc atgaaagcaa cactatatag 67800
   acaatttaat ttttatgaaa acacatttaa taatttgagg ctgcaggaga ataagccatc 67860
   ggacacacca ccagaaccat ggccatgttg aaaacgacga gtcttgggtt ccggtaacat 67920
   ttccctcttt aataatttct atttttctgt caaaataatt agtttttcga aatttgaggc 67980
   cagaacgacc acttgtcaaa tttgattttt agctgtagta aaaacagttt gctagtgtca 68040
   cagttaaccg gtaattgatt ctttttaacg atttatagaa gtaacatttt tgtaaaataa 68100
   aatatacatt atggtatgtg acaacggacc acgcttattt gtattggtga atcttttaat 68160
   tactccctcc aatttatttt agttgcagat ttagatttat gcacatagat taataaaaat 68220
   attttgcaca ttttcaaaat aaaaacacca ttacttatac aactaaccat atttcaacca 68280
   ataaaaataa attagaaaat attatttata aattttgtat tgaaattata aaataatact 68340
   tattttaaaa cgaaattaat ttacaacgac aattaaactg aaacggaaag aaattattaa 68400
   tacttaatta aagagttttt agaaaaattg aaagacatgt ttatgcgaaa ctcatgtgaa 68460
   agtctttgaa ataatagatt ttggtataaa tatttcaaat tttcttaaaa taataattat 68520
   atattaatat aatttgtgat aaaatctcgt caaaaactca ctaatgcaaa tgcttttatt 68580
   ttgaatttct tactcctcta aatgcattta cttttatact aatattattt tctttctcta 68640
   atttggcgtt tcgtaatagt ttgtctgtat tttgaaaact aacaaaaaat aataaaaaca 68700
   aaagcttata aacacatagc atgcaatgaa tatgtacgaa tatatatacc aatacatatc 68760
   taagtactat ttttccaagt acttaatctt gattactaaa attcatttta attgttcctt 68820
   tcagttacca gaaaggttat acaagaattt accccacagt actcggatgc tcagatacac 68880
   tgtccctctg cccatgctcg cttacccgat ctatctggta ttttttaatt cctaaaattt 68940
   actacaagtc attttagact gtgttttaaa acaatataat tatttttgtt tggttttact 69000
   gcagtggtac agaagtcctg gaaaagaagg gtcacatttt aacccataca gtggtttatt 69060
   tgctccaagc gagagaaagc ttattgcaac ttcgactact tgctggtcca taatgttggc 69120
   aattcttatc tgtctttcct tcctcgttgg tccagtcaca gttctcaaag tatacggtgt 69180
   tccttacatt gtaagtttct tagtatatca taaagggtat atatttatta ttcaatatat 69240
   atactatatg atttgttttt gtcatatatt tttgaaatat tcagatcttt gtgatgtggt 69300
   tggacgctgt cacttacttg catcaccatg gtcatgatga gaagttgcct tggtacagag 69360
   gcaaggtaat taaattaact attacaagta ttttacaaaa aactaatgat tagtatattt 69420
   gattaatctt aattcttgat gttttgtgat taataatagg aatggagtta cttacgtgga 69480
   ggattaacaa ctattgatag agattacgga attttcaaca acattcatca cgacattgga 69540
   actcacgtga tccatcatct tttcccacaa atccctcact atcacttggt cgatgctgtg 69600
   agtcatctca ctctctggct actttcatca aaaccatttg attaaagggt gattaattac 69660
   taatgtagtg attttaacaa atggaatgtg acagacaaaa gcagctaaac atgtgttggg 69720
   aagatactac agagaaccaa agacgtcagg agcaataccg atccacttgg tggagagttt 69780
   ggtagcaagt attaagaaag atcattacgt cagtgacact ggtgacattg tcttctacga 69840
   gactgatcca gatctctacg tttatgcttc tgtcaaatcg aaaatcaatt aaactttctt 69900
   cccccttttt gtttagcact attatgaata aaccagtttt ttttacttat atattgttgt 69960
   ttttaagtta aaaatgtact cgtgaaactc ttcttaattt agatattatt ccatttacac 70020
   tgaaaaacat acaatttcaa aggttgaaaa gaaagacaaa attttctaga atgaccctaa 70080
   aatccctttt atcacaaata tagtcttcaa ggatcaaaat taccaacata tttcattaaa 70140
   aagtaaatag acacttatac tcttagagtt aaaaaatagc ttcaaaaaat ttttgaattt 70200
   caaaataaaa ttttgaaaca aaattcgaaa aatgtttcat gcacctatgt atatgtgtct 70260
   gtgtctgtgc catcgttgtc caaatgtaag tttgcacgat cagtagtatt cgtgacttga 70320
   gcatctatgt catgctctcc attcccacat gattttagag agttatgttt catgtcacag 70380
   cgggggatct agagtttgca tgggttgatt gcgggttcag aaccttcgtc cagttcccct 70440
   agctgcggtc aaagtagagt tttctctttg gaggaccatg tactctgctt cgagctgagt 70500
   tagtctctaa gcactttatt ctagcggttt ggaatttctt tccatctgct attttaagtt 70560
   ttgaacctct gaggtgactc ttggattgca tgtagtggta ttattgtttg ccgtagctga 70620
   gttcatctct tcaacttact tctccaaggc ttcaagataa gcttggaaaa ttgctcatgt 70680
   attaatctat gtgactatgt ctagcaatgt acgcacaatc ggtataaaat tttaatagtt 70740
   tattttttgg tcaacaaatt tttaatagtt tttttgacca aaatattttt aatggttttt 70800
   aatatgtatt tctaatggaa aaactgatta aaatggtttt ccaaaaacgt caatgaaatt 70860
   attaattttg taaataaaat ataggattat ataaattagc gttatgtgag tattgactta 70920
   gtaataacaa taatcaatta taagtctaag ctcaatgtga tgattttttt tttttgcttg 70980
   aaatgtaatg acgatgatga aaaaaattcg caatataaat aaaaagttaa tactttgtaa 71040
   tcataaattt atctttagaa aatttattgc attgtattaa agctttacat tgttttgtct 71100
   cttcataaaa aaattaccaa atttttttaa gtaatcttat aagaaaagaa aagtctgtaa 71160
   caaatataca aagctggatt atttcaatat attatttgag aaatattaca atatttgagc 71220
   tatgtcatgt gtcattatta gaatgctttt taaattatct agaaacataa gttgatctat 71280
   ctaaacatat attatacttc tcattagact aattatacaa tcaaattaat aatctacaat 71340
   taatattttc attctttcct tagaaaaaac tacggaatta cctaatgtga ttcaaatata 71400
   tatttgacaa ataatgactt ataataataa gtatttgata acaatttgtc tatcctcaat 71460
   cattttgttt aattttatat tattaaaata aagtaaacaa tcacattaac catataataa 71520
   aatttagatt tttagtatat aaccacatta aaatgtgacc agtgatttaa atttcttgtt 71580
   ataagaatat ataaatgatt ataaaaccat atgagtgaaa atttcattta ataatcattc 71640
   agatatatat ctacatatta aactatatac catataaaat aaataaatat tttaatttca 71700
   attgcattga agaagtattg aaaacttaaa attttaattg caaaattttc attgaatttt 71760
   tataaattat taaaactatt aaaaatcaca cattgaaaat ttgttagtat tggttttgaa 71820
   attttgctat aagcatatat aaataattat aaaaatatat aagtagaaag tctgatttaa 71880
   tagatagtca tattaaaata tatattatat atctatgttg ttattatata aatttaatta 71940
   tatatcacat aaaatagata aaagtgattg cttgaattta tttagcataa aattattcta 72000
   aacaaataag agtaattgtt ttggtttatg tgtttgcgct ggtttaaata tatatacaat 72060
   agttaatggt ttctcaatta ttcaatatat atatatatta tttcataata tataaaaaat 72120
   aaaataaata ataatatata aaaataattt gtatatacaa taatcattct gtgaaggaat 72180
   tttaaactag taaattatat tacttcagtt tgactttcct tttcgaggta ttaatagttg 72240
   ttgcttggta aggaatgtca aaagtcaaaa ctaatagtca gagtcaaaaa catatcatct 72300
   ccagtatagt atataatcaa aaaggatcca tatatttaaa gaatatttca aatatatata 72360
   tgaaaggttt tagactcttc atattcataa gaaaaaacta aaacaataaa gacaaaaaaa 72420
   tcaaaatgat atcaataaga aaatgttatt ttttggcgtt cttgtgtttg gcgattctct 72480
   tgactctaag tatgcaatat atgttgatta ttttgtttct atttgttatt atattatata 72540
   tcccttcatg tatgtagtgt aacatattat ataggtttcg gttaaagtat atacatttgt 72600
   ttgttatagg ataagtcttt gagatattga attgtacact aacaaaaaaa tcatgttctt 72660
   aaataactcc ctaatttctt tttaaaaata tatgctcaga tcttgcggaa gctcaagata 72720
   ggagtaagct aattcctata ggtccttgcg cacagattcc gaactgcagt cagacatgca 72780
   aaaattcagg ctttgctaaa ggcggacaat gcatcaaatg gtatcctaat tctattaagt 72840
   atacatgtgc gtgctttgta aacgctgcta caccggctgt ttaagataat aactcttcaa 72900
   atttgaacta aaaagatctc aaatgactat ttaaatagaa tattgaagaa atatgtttta 72960
   tgcaaataaa agtgcatttc aattttaatt atgttctcaa tgtggactgt tatatgatca 73020
   tatatatata tatatatata tattctgtat gaaataaacc gaattaataa agtttagaat 73080
   tgttgtcaag tttgcaatca taaattttca attaataaca acgaattcaa gatatgagtt 73140
   atctagttca cataactaac atgagccccc caaaaaaaca tgagccacac atcttattgt 73200
   tttggttgtt cgattctaca aaaatgaatt ttatttatta acaatataaa caatttaaat 73260
   gaaatttttt gtgaagtact gttttattaa taagatacag aatttcagaa aaagataaca 73320
   aataaaaata aataaaggta ctgctaatca atttataaac cataattatc taaacatgtt 73380
   gatctccttt attgttctgc tcttaaccat tccagaattt gtttgttatc ctattttgta 73440
   tagaaaaaca ttatttatct taatacttgt ataattaaaa aacaaacatt tgattcctta 73500
   tataataagg tcaattatat aatttggggt catcgtcaat gtctacttca taaaatgata 73560
   tgcgcctgat tccaaaattt gaggaaaagt cttttatgta aaattctttt tattttttct 73620
   aatgtgttaa gtttatgttg gatttgaacc aatcaattct agtgataaaa ttatacttga 73680
   cagctaatct ttcactctga atatttttat taaaattttg gaaagaaata gaactatgta 73740
   tattatttta actctatcaa aaataaaaga agtctttcgt gcctccagaa aaattaatgt 73800
   gttttatcac ctacctaaca ccttgtaaca tagaactatg tatattattt taactctatc 73860
   aaaaataaaa gaagtctttc gtgcctccag acaaattaat gtgttttaac accttgtaac 73920
   acatactcca tttgcgatat cgtaaaacta aagtacaaaa aaatttatgt agtgattgta 73980
   aggtcaatac actagtcttc ctaaactcaa agataaatta atgtactgac catcgccatg 74040
   aaattgaccc atatgccaag tgaacaggcg tgaaaaatcc attagcttaa ctgccgatgg 74100
   tcggatatta aaaatttctt tatcatatcc cttatatatt aattaagtaa cattacaaca 74160
   ttgttttgta gcaacgtgtc accgtgaaaa tgaaattcag aattcttata gaaatatgta 74220
   ggttcatctt aacttatact atacttttta ctaaactagc tattaaatta ataaatagtg 74280
   tacaaaagaa tattttagta ctttctttat ataaaaacta cagaattgtc taatatgatt 74340
   aacgtatata tgacaattaa tgattatgaa taatatattt ttgataataa tttttgtatc 74400
   ttagcttttt tttctgttta attttagatt attaaaatat attaaacaat cacattaacc 74460
   atatattaaa aaatattttt ttatatgtta tatttttaaa tttttaaaac gactacaaat 74520
   tattaaaaac gttaaatgtc tcacactaaa attttgtgat caatggttta acttttttgg 74580
   taataacaag aaacaaatga tcataaatcg tatgaatatg aagtctcact cactagacat 74640
   taatattata tattaaatat agcttaaaat tatagtttaa aattaaacta taaaacatag 74700
   aaaaatactt aaatatgata atttctaaat ttgtattgaa aaagtattga aaccttcata 74760
   ttttaatatt gaaatttgca ttcaaaaatt cgcacattaa aaattttgtg tttatcatat 74820
   gattataaat tctcaataat aaatatttat attaaaatat actatatatt tatatccatg 74880
   tcattgaaat ttagttatat accatataaa ataaataaaa ttattgtttt ttaatttact 74940
   aaaaaagtat cgtaaataaa caagatgtat tgttttgatt tatgtgctta atctaattta 75000
   attatatata taatatgtaa atgaatataa ataaataata atatatataa tatttttata 75060
   tataacattc attctgcgca attgcgcggg tcttaagcta gtatatatat taagtcagat 75120
   gatagacaat tgagaatctc tcgacagttt tgttctcaaa aggtgatcaa agtgatccaa 75180
   gaaattcggg gaagatagtt gatggtaaaa atggcagtga aacctttaat tggctctctc 75240
   aatcaatggg tgtgagtgac tctctcaatc aatggtgaac cagaatttct agaatcgcac 75300
   aacaatccta atccagtgat caagagcaac aaatgaataa ctcaaataat aaagacaaga 75360
   tacactcttt gaagaaggaa gcaatttctt ttataaaact ttttggttga ttgaaagtgc 75420
   tttgtacaag gacgaccatg agcttaaata gactctgaag acaaagattg ctaagccaaa 75480
   atcaaataga gatcaaagaa aataaaggga aagagctgtt ggccttaaat ggctttcttg 75540
   gccaaaaata agaaaggtga tgattatttt gcgtcttgag agttttgtgg agaaagccta 75600
   gtgtcttggg acgaccatgt gaattgcaag tgtcttcata aggctgtggt tgaagtgata 75660
   tagccattgg tcatcaaatg gattccagcc caaccattag ccgggggaca atatgataag 75720
   aagccggcca tacaattttc aaaggttgca gcattataag ctgtaaccat ggaggcaaca 75780
   agagatggat atgttctgta tccaaaggtt taaaaatctg agcatgtgct ggacgaagat 75840
   ggtgagtcta tgattggtca taacttaaac cgacatagac caaacttagt atatttattc 75900
   aaccacatta tggttcaata tgtttttcca aaaatttagt aaagttctgc tttaaacgta 75960
   attgttgaaa cttgcatcat gctacaacag tgtactgctt ataaattaca aaactttgaa 76020
   aaactagaga gaaagagaag agaatagaga acgagacgac gcagacaaac atctttctga 76080
   ttctatctac cagtgaaacg gagggaaggt ttgtggaaga ggtacagcct cattaccgga 76140
   tccatcagag actgcactcg cattctcaaa caaaccggac ccataaaaaa cagtctcttg 76200
   ggacaaagaa agtggtggct gtggctgaag caaatgagag gaagatgatg acagaagaga 76260
   gagagcgcaa tcagagtcat ggatgcaact cgtcattctc gagctgtcct cttcttcttg 76320
   caagaaaggg aactgtttcc ctctgctgtt tggagaagaa gatatcggga acgtaatgcc 76380
   tgtctttgca ggaaaagaac caacatagct ctggttctgc ccgtaacttg aaccattggc 76440
   catagctacg cttacaggac cgcttcccca gctcggactc acaacagatg tagttggaaa 76500
   cacgtgtgga gagcttgaaa aatccagcaa tttgctacct gttaacaaga tggtgcttag 76560
   ggactcttaa gtctattgga tccaaaaaaa aaaagaattt atttttattt ttttaaaaga 76620
   agaaaagcaa aaaccttgga agaaagttgc agtacgatcg gtatggtcgg gctgaggctt 76680
   ccgtcttctt cgattgtgtc catcaagacg tttcctacaa cttttcttac cttcatcaaa 76740
   ctcttccaaa ccatgaaacc tatctcaaaa acaaagtgga ccatgagcta aatttttttg 76800
   ttttttggaa ttcatttctt taaataaaat atatattttt aaaaaattag gctttaatct 76860
   aggctaaaat aacacaaaaa aactgtagta tgtgcaaatg caccctcagc aatatataca 76920
   cagatgttaa cattatccaa catatgaata caatcagttt ttacctgctg cattgttgac 76980
   aaaacctctg attattgcca ttgattgtaa ccacaggagt tttagaatgg acatcacaga 77040
   ctttatgtct tttatgatac tctctacagt tactaaaatc agaatcacat ccatcaacca 77100
   gacaaatcgg gatctggttg ttccctcttg tcctcttgga gcttcttgaa gcctcagagg 77160
   cgctctcttt caacttacta agacttatca ctttctctgt cttgccaaaa gcagaggagg 77220
   aggaggaaga agaggagttt acaatgtttc ttccaagttt cagatcaaat ggtaaatttc 77280
   cttttggctg caatcttctt ggtggtgagg atgacccacc gaacgagatc gatgcatcga 77340
   ctggggttaa atccggtaca gattcttggt cgaattcaga gaaataaccg gagctgagtt 77400
   tgaagttcca atccatttaa agcttttttc cttctccttc ttcactcact aaaagaagac 77460
   atacatagaa acaaaaatat caagatttat ccttttggtt ttgttaatct aaattgacag 77520
   gtttaagaaa aggatacaat aaattcaact ttcaaacatg aaatttttta actcgattaa 77580
   tcttgaattt tgaagaattt ttttttaaaa aaattaaaaa atcccaaaaa tgggcaaact 77640
   tactgtactg aaaaaacaaa tgggaagtgc agatatatag aactagaaca gatcccatgt 77700
   aatgggaaga aagcaaaaca aaataaaaat aaagcaaata tataaacttg cagttttgag 77760
   tttcacttca tcataaaacc cctctctctt ttttatttat gtcactcact tgaaagcaag 77820
   aatttaatgc aataaagaga cagagattaa agaaagatga aacatgttat tcataaatta 77880
   taaaataaga aaagcttggt atttgaaggt tgagaaatct gaccaaatcc atgcacctac 77940
   caatggtcag tagaagaaac tcaaaaaaga gaagagcgaa atctacaaaa tattgacaag 78000
   tgagaaagag agttgatggg tttagcgaga gaaagtggag acaacgagag tggctgctgc 78060
   tgcaataatg cacaagagaa agtgaagaaa aaagtaatat attactaagt ttaaaaatga 78120
   agcttaattt aattgtttat ttgctttcct aatataggaa ttgttgatat ccttctttag 78180
   agagagagag agagtggagc aaagggacag ctgttattgt tgtttgttca tttgctaact 78240
   tttgcgtttt tttaattaaa aaattcttta ttagtttgct tacgaaattt ttaattttgt 78300
   aacaagtttg ttattatttt aaaaatttat ccttaattga taattatttt attaaatact 78360
   tcaaattttt gacaaaaaat aaattaactc ttttaaatta tttataatgt ttaaggatag 78420
   tttataaaac atttataaaa atttataaac ctaaatctta aacaataatt actaaaccat 78480
   aaattcaaat gttaaaatat ttttattgaa tataaaattt aaaaatgata gacaacttaa 78540
   tgtatataac caatttttct ttatcaattt gtttgccatc caacaagtct gaatttatga 78600
   taattaaatg aaatgatatg aaaaatacac agcatcatta gttaattttt tatatatttt 78660
   atatgaaaaa acattaaaca tgtaactcat cagtttatgt tagtagtttg gtatctaatt 78720
   tagacctgat atgttgttga gaaaagcaaa ttatactaaa attttaatat cgttgaaaat 78780
   agtatagaat ttaatgtgta tgattaaaca atatttgttc ttcatggaac tagaatttga 78840
   aaattttaag ctgacattta catttttcaa aactgaaaat cttccaaaca taagttagag 78900
   atgatagagc acaacctttt taaaaagtca taagattgtc gttagcctga atttcacttg 78960
   gtgtgaacaa taatttaatt ataccaacta attctgttaa cgtcattata tccaattaaa 79020
   attacaatca atcaaactgt gacaaaaaaa aatcacaatc aatctaaata taaattgtat 79080
   aaagcatctg attatccaaa attttactct gtttttttac tttagtatat ttcaagttca 79140
   tgtagatgtc caaactaatc tctaaacgag tggtatggct tttttttttt tttgacagca 79200
   agaaattcac agactcatga tgactctgta aaccatgttg gtaactccgc atccatgtga 79260
   acgaacgagt ggtatgatct acaaatagac tttcattcta gctattcaaa tggaccataa 79320
   aataaattta tatttgtata gtcacaaagt aaagtgtagt ggaatgccat caactctatg 79380
   ttgattggca attccaaagt tcgcctacaa gatttttatt actaaactat cactttatgg 79440
   ttaaaatttt attttgttcc caataactat caagatcttt attttaattt gttatagtac 79500
   atagcaatcg tttgcaatat atatagcatc tatccaattt taatagcttt caaacatggt 79560
   caccttgttt ctttgaaaat aagaataaga cagacagggt tttctaatat gctcttgtaa 79620
   ataacaaaaa aaaaattgga aagtaataaa taagaggtat atgatgctta tttgcttacg 79680
   gcaaaacata gcatgtgaac gtcgtggttc gcattacaca aacatcttct tctgttttta 79740
   actttttatc atctctttct ttctttcccc gatacgcgct atttcttcga ccaacattta 79800
   ctccttcacg ggtcacaact cacaagtcga caaataatat gtttttttgc caacaactaa 79860
   taaacatatt ttgtttcctt ttcttaaata acatgtcttg tcttcaaaga atcaaactag 79920
   ccttctactt cttctaaaga gtatcatcac tttaacactt ttcatataga ttaaaatatt 79980
   aaaatatatt actattttta ttaattaaat ctatttaacc actagtattt gagataaata 80040
   aaactatttg tagaatcaat atattttata attaatatta aacttcaaat aagtataaat 80100
   tgctttaaaa tataaatgtc aatctttgtg taacaaaaaa gtatcaaatg atactatttg 80160
   taaaacagag aaataattag aaatggctga ttaacaccct cgttaaaaat ttctccaaaa 80220
   tcaatttatt tttgaagaat aagttagttg tagaaataaa aataaaaaat ttagttgcat 80280
   gtttgactat ttaaatatat tgatttatct tgaattcgga tgttgcaact aagcgatgga 80340
   tgttgaatca agtacataca tactggatta catcaaatgt gttatatcaa attgttgtgg 80400
   atgttacacc tgatagtgag tttagttcca tgaggttgta tgtactaaag tattaagatg 80460
   catgatactg gtgtatatat attttgtatt caaaataact tttattttgt actcgataag 80520
   cttaatatcg cctataataa taaaatctca ctttctctgt ggacgtatcc aaattggacc 80580
   acgttaaacc tttttgtctt tgttacatcg ctttatccat ctgtttttgc atatgttcat 80640
   tttcatgtat gtaacaacaa aagtggcatc acagcttcgg gtctatgatt tggtgagaag 80700
   atggctggta taaatgcgaa gatagaaaag tttgatggga gaaataattt caatctctag 80760
   tattgcaaac gtttccgaaa caccatggca tatgcgggcc gctgtcagga aagaagtcta 80820
   atgttgctgt tttagatact taggaagaaa aggcattctc taaattttgg ttgtgtttaa 80880
   cagatgagtt catcatcgaa gtatcggatg agaaaactgt tgctagtttg tgacagaagt 80940
   tagagagttt gtaaacaagt tacttctaaa gcaacgcctc tttgccttgc atatgcaaaa 81000
   atatatatat tgagatttgc gaccatcctg gcaagttaaa tttgatacta ctagagatgt 81060
   gtaacatcga tgttaaggtg gaggatgaag acactacagt aatcatgttg gtatctatgt 81120
   cgaacttatt tgaaaatttc gtgcaatcgt tcattattgg caaagataca atgaaactgg 81180
   aaaaagttag atcatcgctt catagtcaaa aattttatta gaacaatcca gttaaaaacc 81240
   caaaataaaa ataatttagg tattttcttt atatatccca aaagaagagg agtaaagaaa 81300
   aatatttacc tttgaaaatc tttataagat attacttaaa gagatttgaa atgtataaaa 81360
   gaaataatgg ctatgagagt tgaaaagaat ccgcaatatc tgctagttaa gccctctagt 81420
   acaccaagat ttagttttaa acaattcaag gaatataatg ttaaagttta tggtattatt 81480
   tttagaagtg acttgaattt aaagccttgt aaattaagat ctttgtagaa ctaacttgaa 81540
   tataaattct tgtaaagaaa gttttctgga gatcgtcagg cctcaaaact cagatctaac 81600
   cactaaatga gtaaatgtac agccataagt gaattttggc ccttttaggg acgactttgt 81660
   ttgtgttcag aaaaaataga ctggatggct ttttttttag atcaccagtg tgatgatttg 81720
   tttggcattt ttattttaga tcaccagtgt gatgatttga gaataagtga tgcatatggt 81780
   gagaaagtat ggcatactta taaaaagaaa caaaccgagc ataacaattt aaactggtaa 81840
   tatattaaaa ataatatttt tgacgtcaga ctgaactttt cacataggtt caagcagacg 81900
   gctcataaga aatgaaatta caatcatatc atcaacttgt aaacgcattt ttccgtaaat 81960
   taaataggag agaaagacag aagtaaagca tcaaatatta gagactgaag gaaccaacac 82020
   taaagcctct ttgtgtcccg tgcattctct ttttagtcac tcagtctggt gtcgttcctg 82080
   tattccaaac accaaattaa aaaaaaagac cgtcaatata tatacaatag tgtttctttt 82140
   tgtttcacat gtagtattac aaacctagac aaccattcta gtactttttg caaagaaaaa 82200
   aaatctcatt atgaaggaaa gttaatagtt ttcattggta taattattta ttttcccttt 82260
   atgcaaatgc aacctatggt gcttttgttt ccctgaattt gacatcattt tttgaatcaa 82320
   gattatagtg atagattgtt gctccgctgc acttgaacca aatccgtttt gatcacactt 82380
   tagatccagt tcgtttgaac cttaagtatt aaaaaccggt tatcattttg gcacgttaca 82440
   tgcctagtag actctttttt ttttaatgaa aggcccttgc acttacatag tgaagctcaa 82500
   acaaatccgg aaaaatgacc aaaccatatt cgaaggatga taactcagct atcatgtgga 82560
   ccaacctatt taggactagg tttgccctca caaagatttt catcaccacc ataaattttc 82620
   aaatcaagtg gatatgctat atgagttcaa gatatatatt tacgttatag taacctatag 82680
   gaagatagga aaatggttaa agatgaaata gttgacctta ggtttgagga tgccatactt 82740
   cctaaactgt tccctcacga ctctgttgta tatgaaagct gctcctctga attgcggcaa 82800
   aaccaaccat gctaccaaca ctagcttcgc cgtgtaccat atcggtatcc tatatacatt 82860
   ttcacacaaa aattcaattt ttgtttctca cattatttcc aggacaaata aaaatacata 82920
   gttagtatta ttgttaccac tctaggagcg attgaaggat gagttctgag agagttaaga 82980
   aagagtagat aatccaataa gcaagccatt gctcatcatc tgcttttgat gggctctcta 83040
   ttgctagcac cgacgcatat cttaccaata ttataaatat aaaaaaaaca atttgaattt 83100
   aatcattgaa ataaaacgaa acaaaatgtg aatttatcaa agaaacaata gtaagttact 83160
   tacaacggat aaagcagcat caccacagga ctgcatatgg tcgtaaaaag taaacaaaac 83220
   acgaaatcac gttgttattt taataataaa aatgctatta taaaagaaaa acaattcagc 83280
   cataaattga tggagataag tattaaatcg agaaaataat atgaaaagtc aagtaagtac 83340
   ccagcgatgg aatgaagagc agagaggaaa gtccaaagct tagtcattgt aagaggaaca 83400
   caaaaaaaat ccaaaccaat caaaggaaga ataaaagaga agtttcgaaa cccttttgtt 83460
   ttctaaccaa cacgcccaaa gatggaagga gatcttctta tttataatat caaacttaga 83520
   cattaaaaca gtttggcacg tggttcagcc cctggtttaa gccgggacaa ctatatttca 83580
   atattttgga taccaaaccg atgaaaaaag ttttgtgaga gcatctacaa taatgaaata 83640
   acaccaaatt tgttattttg atgttaaaat agttaccatc tctaacaatg acaccaaatt 83700
   ttacaccaaa aataatatta tatattatta atattttaaa ttttaaattt ttttttatta 83760
   tttataatta ataaatatct agaatattat ttatattttt gttattttta agtgataaat 83820
   gataatagtc atttaattat ttattttgaa aaaaattaat tttttaatta tgcgaaaata 83880
   aatttaaaat acaaataata caatatattt atgtctaatt acaaatttta tagtaattaa 83940
   attatattat ttattggtgt gctttacatc aaatttggtg agtgttaatt ttagtatttt 84000
   attgaagatc aaattacacc aaatttgatg gtttagtgag acggccttat tcatataact 84060
   aggcgatcaa aatcgagttt attagtccgg tttacatatt ttggtggctt aagtttcaat 84120
   gagttaccgg acacgtgggc tgaagagaca agaggtatca gattctaact tgagcgtgtc 84180
   cgacatgtca ccggccaata gagtcccgat gtcggtggga ttctctttat tgttattttc 84240
   catgctttcc ctactatatt gatttatcat taattacaca tacaaatatt tttgttgtag 84300
   caacactcgt aaaaatagtt taatatgcta taatatttag aaaaatatct gatatatgct 84360
   aaacactttt gttagaaatt atatacaaaa ttttttcata tacttctttc attttctttt 84420
   gaaagtatta aatattttta tcaactagat acatggaaga aagacacatg aaaccatata 84480
   tctgtaaaca catttgagat atacaatacc gtaaaaaaga caaaaattat tgaagataca 84540
   aatatgcttt tcaaatgaat gaatgttaat aaatatattt tgaaaaacat gttgaaactg 84600
   tatctaaacg ataagccttt tctcaaaaaa aaaaaactgt aactaaacga atgaaaatta 84660
   tattttggaa aaggtgatcg atctttgaga gcatcccatg atgatgtgat agaaaaaatt 84720
   tcttgggaat tcgtaaactc aatgatgtat gactaacctc caggttttct ctatgtttac 84780
   tagttgatat caacgatcag aaaccatcac cgcaagatgt attcgcaacc gtaaaaccaa 84840
   acaatttttt aatagaatgt aacactcaaa tatctcttta atagacaaag cactgcgatg 84900
   agttgcataa gttgtttgga aaatgtgttg agggttttgc agtggaaagc tcaaaatttg 84960
   tgtatattag ttacgaactt ccacacatta aactacatgc aaaacagcaa aagtattttt 85020
   tttttggaac aaaaaagcaa aatccataca tctcaaatgg aggaagcagc gagataagtt 85080
   gcaaaaaaaa aaactgtttg agagtgtttt gaaagttttg caagaaagca caaagatagt 85140
   atataatgta ttaggacatt ttaaacatga cgtagtattt acttttacta tttagagatg 85200
   aagactttta gaaacatgta agtgcattta tattgagttt gtatcaagag tgcttcaaca 85260
   atgagttcct aagaaagttc aaatgaataa gtcgtaaaaa ttgggtattc ttgttttcaa 85320
   gtcagttgtg cgagtgaaac gaattcgtga gattaagcca tcaatataat ttcgtattat 85380
   tggagatcga tttcgaggct caaatctctg catggagaat tttttatgtt acaatactaa 85440
   caataacatg atcatctaat aagcttgaaa taagaaagaa tccatttaac gacataaata 85500
   gagtaaaaat tctaacttct taagcaaacg atttactaca tcatggtaca agcgttgggg 85560
   ttctcgtcac tgaatatctg tggtggataa gcaaacatct ccacaggata cctcggtggc 85620
   tggtattgat attcattctt ctttaaatcc accactttgt catctccctc cgctgcagca 85680
   cctccatctc cggccgccga tttggccact ccaccaccgt cttccttagc ctctttgctt 85740
   tctttgggtt gttcttcttc ctttttcttc tcgtctttgt cttttgtttc tttttctttc 85800
   tccggtggtt ttggcgatgg atcttgcttg acaatcgcag catgcttccc gattttcttg 85860
   ttaacgtact caactagctt ttccggtata aaaactcctt tcacgctcac ttgtgatgct 85920
   ttaaagtctg gttccacaga ctccactcct gtatagtaaa aggtagttgc tttatttttt 85980
   tttaataata caatattcaa ggaattaata atcgaagaaa gtcgaacttt caaattgcat 86040
   tacaaatatc gaatgcgcaa aactaatttc aattcttaag caaccaatgc tattctttgg 86100
   cccttagaac tcgattagtt gatagcattt atgtgtatat atctatcaag cataaaaata 86160
   tccacacttt ctagaaacaa caatttgtac aacttatagt tagcatatac acatacgtac 86220
   tggaatttta gataactccg catacgagga atgtattcac taactaacta aaaaagtgtt 86280
   tagaactttg agatccttgg gaaatataat aggtgaagta aatacaaagc acttgacttt 86340
   agttgactct attcaaaccc actacagttt catgtaacct tgaaatacta aaagaaacaa 86400
   aaaaaaaata ttcgggacaa ttttgttaaa atatatgatt atagtaacaa ataatctggt 86460
   gaatgagttt ctttttaaag gaggaaatgc tctaacggtc taaacgcatg gtcttgtata 86520
   ttgctctttt taaagggcct acatagtaca cacaatttta aagatggaat caacttatga 86580
   catacataag agtccaaaac gtaatgtccc aattaagtga agtcagagaa aacttcgatt 86640
   taataggagt catacccagt tgaggatatt ataattaaaa ttttgaataa gaagatgaaa 86700
   aagaaaacaa acctttcatt ctcatgattc tcttttggat ctccatggca catgcttcac 86760
   aatgcatgtg aactctcaac accactgtca ctacctgttt ttctcagtgc ccacaaaatt 86820
   ttaatgttaa ttacacaaaa ccaacttact ttcttcaaat tacaaatcta tcctcaaaag 86880
   tcttaacctc ttcttttttt tcttgaggtt ttggtttctc ctcttttttc tccggttcat 86940
   ctgaaaccgg tttaggctct gggataggag aaaggagctc cactggacgg tggctctttc 87000
   tttgcagtct ctgcaacact tttagtggat ctgccttctc tcctttcacc acaactttac 87060
   tatttttaca atcagttgtt acatcctcca cccctaatca cattttcaat tatcccaaaa 87120
   ttaactaaac cattacacaa atggatatga aagaaatgtg tttagttgta ccttcaaagc 87180
   ctttaagaca tctatggatt tttttagcac aaccttcaca atgcataaag atcttaagaa 87240
   caatctcttg tggctctttc ttcttctctt cttctttctt atcatctggt tttttcactt 87300
   gtggctcttc agcttttttc tccattttct cttctgattt cttcttatct tcctgaaaat 87360
   ggtaaaaaag gagaaaaaaa gtttaggaat agtgtttgga tttgtgaatc tgaaagtttg 87420
   aaatacaaac ctctcccatt gattttagtg ccagtgttga ctgttgagac ttgagagagt 87480
   tttttagtgg ctcacttatt taagtttttt cctcttcttt ctacggactt gagagagatc 87540
   tggttatata aaagacacat actatttctt ttatttcttt tttttcaccc cacaaccaca 87600
   agtacagaga cttattagta tttttcccat ccaattattc atagattttg aagatctttt 87660
   ataaaatgtt tcttctcagt gttttgtttt aactgatttt tttctcagtg tttttagcta 87720
   ttttgtatat ttgaccaata ttgggtactc tctaatcgta tattcgtatt gtttccaaaa 87780
   tttgaataca gttttaaacc ttatctacca taactcaaac cttatcggtt gaagtaatta 87840
   atcggcttga ttgtcaacat attaagtctt ctacaaaaaa taattgcatt actattcggc 87900
   agaacctaca tatctaactg aaatatactc tttatgtttt acaaagatat cactctgaca 87960
   ttttttttaa ttaaacttct aatactccac atgttttaag acgatccatg ttttagaaaa 88020
   atgtgtttca aaaataaatt tttacatttt taatccatat tttatcacat aataattgtt 88080
   aattataaaa ttcaaaaaaa taattgtgat tatttaattt atgctgactt aaaattgtgt 88140
   caaatagata atcacaatta atacattttc ataaaaaatt atgttttctt aatatatata 88200
   aaaaaattaa acataaataa ttgtgaaacg gatagaatat tattttagtg aaatgacatt 88260
   atgaaatcag tcaatatgtc tctttcttgg aaaaccccaa taaatctagt atttattatc 88320
   atttaatatt gctgatattt aatactccct ctgtttttta aagatggatg ttttaggaaa 88380
   atattttgtt tctaaaagat gtatttttca tgttttcaaa gcatattttg tcaattaata 88440
   atgaaaaatt gtgtgtttca aaaatattaa ttacatttct tttaatccta ttggtttaaa 88500
   aatataggaa atataaagtt acaaaaaact atgcattaat aactaagttt taatatggtt 88560
   tcttaataag tgtgaaaatc ctagaacatt catctttaaa aaacagaggg agtatttaaa 88620
   attattttga ttgttttatt acattatttt cttttttaac tagttattat catttatttt 88680
   cagctaactt tttattatct atacaaataa atattcctct tagttataaa ttcagattaa 88740
   ataattttat acaatctttt caaaaataaa attttctttt ggaaatctat tctatcaggt 88800
   tgcatatgca cattttattg taaaaacaaa agcacttatt tcacccaaaa tatttttaga 88860
   attttctttg tatagtttta tatatatttc ataataaaac tttaagaatg ttttgttagt 88920
   gtattttcat tcattcattc attgtcttgt ttacttgaca aaccacaaag agttatgact 88980
   aattaatttt cagaaaatat tcaaagtttt tcagactgaa ataattgttt ccaacaaaat 89040
   atgataataa taataataat gtagttttat taataattat aacaaagttt aacactaaat 89100
   gtttttacgt taaaatataa cgaaggtcac actattttct tgctttaagc cacaaaaaat 89160
   actgtctggc atgctttttt tttccttatt gctagacttt tgttgatgat gtagacttca 89220
   ttaatgtttg attcaagtca cgactactaa ggctatgtac aataggtggc tttattcaac 89280
   accataattt acgcttacac atcatctttt atttcatcca cctattagtt taatattttc 89340
   ttatttttat atttacgata atttatttaa taaaatacaa cactataatc caccatttta 89400
   tctcatattt tcctttttat aattatattt tgtaagcaaa aaattgaaaa atattttttt 89460
   taaactataa taactaaaac ttaataaatt gtaaattttt aataaaaaat atttatgttc 89520
   cacttaatat aaaagattaa aaatagactt ttatatatca aaataaaaaa acctaatctt 89580
   tatttaagga acacaaaaat aataaatttt aatataattt atttctacaa aaatattatt 89640
   tgatataaaa taattaatct caagttatta ggatgtaaca accaataaaa tagttataca 89700
   tatatcaata tataatcttt tattttttaa aagaaatttg cttatattca tattcgatta 89760
   tgttttttcc cgaacgtagt ttaaagtgaa gcaaaaccaa catagtggat cttacataaa 89820
   atactttcaa catgtagaaa atattcaaca acaaataatc cacctcattt ttttaggttt 89880
   tcaacagatc cattgcaggt attcaatagt tgaaagtaaa attcaacaaa cccattgcat 89940
   atggtataat agtgacattt gtatacaatg gtgcgtgtat attgtatata tatgaaattt 90000
   gttggcccag tgcgtttgta aagtattcta cataatttaa tatatatagg aaatttgaag 90060
   cacatacaaa atgtgatttg aagaaagagt tcataatgct agacgttaac ggctttataa 90120
   ttgagcatga aagtcttgtg agtacactat ttgaaaccta gtcagcgtac atgattatgg 90180
   gtgtgattgt aagtcatgtc tagagtaaat attgaagaaa aaatatcagt tattcttatt 90240
   tattctgaaa tcttatcaat caggtaaaaa cacttttctt cctcctacct ctaattgcta 90300
   tttacaagag aataaaacac gttaatagtt ttactccaat tcaaacaaga gtaaatgtgt 90360
   ttacctagtt tattctctct ctcatttttt tcttttcatt ttcatctttt ttcttttcct 90420
   cttatttact ttatattttg atattttcca tccatgctct atatgtgaca acggtttaaa 90480
   cgttatattc cttacgaata ttttttttgg taaaaatgaa tgatttattt catatagtac 90540
   tatacattag atcaaattta ccccgtcaaa aaaataattt ttctaagaat aattgcagtt 90600
   aatatttggc agacctttca tatttaactg acatataccc tttatgtttt ataaatttta 90660
   tcattttgat attcttttta atatacaaat aacgccactt taaattttta atacaattta 90720
   tactcatttt aaaatattaa ttattaaaaa ttttgattta taaaaaaatt tattcatctg 90780
   aaagattatt aattaaacaa atgtaattac taaaaatata tgcattttaa tcatttcatt 90840
   atttatgtaa aatgttaaaa taatattttc atgaaatgaa aggaacataa ttgtcttttt 90900
   ctttggattt tccaaaatgt ccggcggacc gagactcaac cgactaatcc atgagatata 90960
   tttaccggcg ttaaatagat ctgattgttc acagtggaca gtagatactt ctgttgcatg 91020
   accacacaaa cgacatatct aaaatggtga gtttaaatat gaaatgctta ctattttcca 91080
   agtccccgta ccattcaact acggttgtgt taatataatt gttttgcaaa tggcagaaca 91140
   gaaaactaga tgtaaattca caatgcaagg ggcaatgcga tgatagatgg tattctttcg 91200
   atgtccgaat aagccataat gtaactactg tctccttaag aagattagaa aaatcttaaa 91260
   tgagtaaaat ccatgaattc tactttaaca cttttaactg gagaaaactc tattaaaaac 91320
   aacgaagcta catgagattt actttatttt aacatgcata gccgacatct cgaatatttc 91380
   tggagcggta aataaggcat tcttgcctac gccaatctcc tgtatattat ttgagaagaa 91440
   ttgtaacatt tttttgtagc cagatgtcat cactataatg atttttagaa ttcttagaaa 91500
   aatacgttgg ttcatctaaa tatataataa gccttttatt aaaccacaat aaatacatta 91560
   ttaatgtcat tcattatttc cttaaataag attacagaat tatctaatgt gactagagta 91620
   tataagacaa ttaataattt tgaataataa agatttgata aaaataagtg tgtattctaa 91680
   ttatatttgt ttaattttaa gttattaaaa taaattaaac aatcatagta accatataat 91740
   aaaaatttaa aaaattattt atatattata ttttgaattt ttaaaaacga gtataaatta 91800
   ctaaaactgt taaaagtttc acattcaaat tttgtgatct atgatttaaa atttttgtta 91860
   tgacatgata caaataatta aaaaataata taggttgaaa gtctcattta ataagtatca 91920
   aaaataaaag atatagaaat atatgtaaca ttttaaattt aactatatgt catataaaaa 91980
   tacataaata tcttaatttt aaaatttact ttcaacattt ttttgataaa aaatttgaaa 92040
   aaatattgac aatttaattt tttaaaatat tataaattat ttaaaacatt aatcccacag 92100
   tgaaaatttt ggtatcacta atttagactt tttgctataa cagatacaaa tgataaaaaa 92160
   aatgagcaaa aatcatcatc taataaatat taatattaaa atatatcata tatatgttac 92220
   tatcatttaa atttaattat atatcatatc aaatagaaaa aatatttttt cgatttataa 92280
   gatttattta tatgttcaca ccaatttaat tatataagta gtacataatg acattttaat 92340
   tattcaatat atatttatta tttcataata tgttataaac atataatata taaattaatt 92400
   tatatatata atgttcatcc cgcgcaaggc gcgggtctta acctagttaa caagataaaa 92460
   ggcatatatt tacttctttt ttacagataa cagaaacaga ctaatagtaa aacaaaaaat 92520
   cataaataaa ataaaataca aaattacaca aatttaaaaa aaaattggaa aagtacttcc 92580
   gtttattttt tactttaaaa atatcattgt ttttcatttg tagtactctc agataaatgt 92640
   aatgtacata aatccagtgt acattctgca tattagatta aacaattttt gtttaacttc 92700
   gtttaagact agcgccattg cgcggattaa tgttagcaag accgctttgg acgcaaggga 92760
   gtacaaggaa gaccggttaa agcaaagcga ttaacacgtt cgtgaaccta aagaaagagc 92820
   acaagtgagt tcattggcaa gaagatatgg ttcctttctt ccggaacgtt tgtctctcca 92880
   aaaaacccta caccggtgct agtcctaaga tattttaggt ctaataggaa attaaaaata 92940
   taaactctaa aaaattaaaa tttgataaaa attaatttta cgattaaaaa ttaaaaattc 93000
   ttcaaaagta tacatagcta ccagatttaa aagttatttt cgttttcttt ttatgtaaat 93060
   aagaaactaa acttcaaaaa ttattttgtt aaatgtttga aatatatttt agatccaact 93120
   tttatatttt tctactaaca acaataacaa aattaagcct taaaagcttt aaaaattatg 93180
   ggccgcatac ccatgttttt tagttatagg ctcaggaccg gcctgccacc actagatgct 93240
   atatggagtt gtccaagatg acaaaacagc ttgctaaagt tgatcctttg agaaattggc 93300
   tgatgcaatg atcgcttgga ctgaggcgtg ggaggagctt aacccttcag ttggtggaaa 93360
   agatgtcacg gccaagtgat gaaaaattga cgattataag tgatgcctgt tattgctgca 93420
   tgaataaggt ttgttattgt tgtgatcttc tatttatata tctcattctg gaagtgtgct 93480
   tcgtacaata acgtaatact gtgtgttatt gttgacgtta acgttgctcg acatgtattt 93540
   aagccttatt ggtgaaatga tgtgtgctac tttaaattac atggatgaaa tgattgtttt 93600
   aacaggaaag taccagagga cttgatacca tcctatatcc aagtaatcgc ttgtccggga 93660
   cgtcgaaata ctcgtttatg ttggcctccg acaagcttct aagccttgat tatttcattg 93720
   ataatgatat aaatgttgta atttacaaaa ttatgcttag agatttttta aaatattact 93780
   tgtgatcagt tttaaaacta aattaggttt gattacgaaa attaagagaa aacattaatt 93840
   ttgtgactga gagcatctct aaccccactc tatttttcac tctaaaatag agtttagagt 93900
   aaataatgct ccaatggtac tctatttctc actctataat agagtaataa ataggtttac 93960
   tccaaatata gagtaatttg tttttttatt gttcatcact ttattttcta ctctaaaata 94020
   gagtaccatt ggatcaaact caaactctat tatagagtta ctctatttta tagtaaaaaa 94080
   tagagtaaac cattggagat gatctgagat ggaagacttc atgtgatcca atggtcaaga 94140
   atcaaccact taaggaggca tgtgtcttta actaaagagt tttgtttgtt tgtcaggttt 94200
   aggtggcact aaattggtgg atatttgcct catcatgcat gacatcctta gagcatgatt 94260
   agtgaaggag atccatttgg gattcttaaa ctatgatttg acatttttct gctaaaaata 94320
   tttttattat ttttattaat ttttttttta atatttctta gttaaaaact aaaagatata 94380
   tatttttgct tgtagcctca aaataatctc ggagaaaaca tagctatttt cgaattaatg 94440
   acatatcgtg aaatacaaaa cgtgcctaac catttcaaat tcattattaa gaaaaccacg 94500
   aaatatttac taaaaatgtg acaaaagcag acatgatttt ggtcacgaaa tattcctata 94560
   gcaattaggt tagtcatatt catatagctt ctaagaaata tggcacaatt gtgattaaaa 94620
   tgaattcatg gcaactatga gtgttactgg ccatagaaaa tgctttgaca tttttttttt 94680
   ggctaactaa aatgctttga gttctaaata ctgaatatgg caactcacaa agatcatttt 94740
   tcacttctat ttatgaacaa atgcttgaga cattatctaa ccacccttac tattattttt 94800
   caagtggtta ttcaagtttc tttcaaattc tcatccttct agatgacaac aataagaaga 94860
   tgatattcat ttgtccttat agcacttata cttaccgtag aatatgattt ggcttgtgaa 94920
   atgcaccaac atacgatgtt tatttttact tatccgattg aagatataat ggaagttttc 94980
   aaggaagata ttttcgtcta taagaacaac ttttatgtct atttatcaaa tttatgcagg 95040
   atgttccaac gctgtgaaaa aaaaacattt tctgctaaat cggaggaaat gcactttcac 95100
   ggtgagagat agaatcgtgc tgagacatca gacttcaaaa aatgactgag gtgaacaaga 95160
   caaaaatcaa ggttatgaga actctatgac caacgaattc tattatagcc gacgaatttt 95220
   attaaagaga tttagaggtt ttttgggaca tggtttcttt catcaaggat ttctttaaga 95280
   tcgcaagacc tttcacccag ctgttgtgca agaattgttt ttctgaattt aagagtactt 95340
   ttttttataa atgcaagtta tatgtattta ttttttaaaa taaattttac aacattaatg 95400
   gttgttttcc taaatttgat aaatatatta tacatattta gaaatatata tcttcataaa 95460
   ttttagaaaa tgttatatat tcaatattat ttttctaaaa aatattggtc aaattcaaga 95520
   agattttata cacattcatg aagatttttc ctagaaaatg ttatacatat tcaaaaatat 95580
   tttactaaat atacatctaa ttcaaattta ttaaattatt cttatatatt catgaaaatt 95640
   ttcttacaca catttgagaa ttatttataa atacatattt gagtttacct aaattttatg 95700
   aagatattat atatatgtat taatatattt ttcagtaata cttttataaa tatgcatata 95760
   actcaaattc aggatatcat atatatttag gaatgtcttc ctaagctttt taataaaaaa 95820
   gatttttcaa aaaataaaaa aaaataaaaa tcattttttt aaatatcatc tttgagaaaa 95880
   ttcatttaaa tatttattta ttttatatat ctaaaaaata ttgtcattta cctcattaat 95940
   gaatgctaac ttggtcattt taccgttata ggatcttttt ttttggtagg accgttatag 96000
   gatcatttga gatttgtatt taagaccatt tgaaaccatt tttcaaacta aaatatatta 96060
   tacatatttt atattatgca acataaatat tttaataaac tttctatttt tttttgcgta 96120
   tgacacgagt cattacttaa ttatatatta taaattaggt attagaatac cacataaatt 96180
   tggttgcggg gcgcacattt agtaccggat attccttttt ctcaaaagta tttaataata 96240
   ccaaacatag caagttgctt taaacatagc aagtcgcaag tagaaaattt cctttttaca 96300
   aacatcagcg gtagttagcc agtgacggac gggtgtgtcg aacaatatac aaaaaaaacc 96360
   cggaacatta gaatacaaat ttgataaaaa caaacttcca aacaaagttt atcaatgatc 96420
   tatcgaatat cacaattcac agcaaaatga tatcctaacc tctttgaaat gtatttgttt 96480
   gtaacttgtc attctattat ccattaagac aatgattttt ggtttttgga ataattccgc 96540
   ttttcatgtt ttaagtatat tttatatcag tgatttgtga tatataaaaa tgtctatctg 96600
   tgatagaaat atttaataat ttataatagt tacatttgtt aacaattgtt aagagtttcc 96660
   attaagtaaa tttattgtct tatagtgatc atttttggct caacaaacta attaatttta 96720
   aaacagaact acaaaattat caaaataaaa attattgcat agatattaat tacatgcgcc 96780
   gaatagtaag gtggatacaa ctttaaaaga attagaccca aaaaaaaact ttaaaagaaa 96840
   aaaatggtgg atacaacttt tgtggataca acaaacaaag ttcgcatatg ctttttacaa 96900
   atgttcgtca attcatatga atttaaacaa gtcaacacgc tcacgttatc accttctcct 96960
   tcggtagtgt ctttctaggg tagctgtaat atgaggaagg tttttcacag cagtaatttt 97020
   ttctgtcaac ggataaagta acaaatagaa aaaaaattaa ttatttgaca gatgttgact 97080
   ttttggtatt tatagatgaa cttgtggaaa atgtggaaag cataaacctt tttagtgcct 97140
   ctcccttatt acaaaataat aactataggt atatatatat atatatatat tatttttttt 97200
   taactatagg tatatgattc catattaaat tggactagaa ccaacctcga acattgacgg 97260
   caaaaaattt aaattttttt aacactgata atcgattata tcattatata atatcttttt 97320
   atgtttcata tgataattac aacatatgta attatgatga gaatttcaaa gacaaagatt 97380
   tcacaatata gtttaccctg taacattcga attgattggc ggttctacgt gtactacata 97440
   tgaccataac aaatgattct gtattcagca ctgaaatttc cgataatctt gtgttctata 97500
   actgtaagaa attatttttc tgaaatcgaa ccccaaacat ggtatagaaa cctttaaact 97560
   ttgacaaatg aattacaatg ctttcacaat ttttttttat catctaccaa aaagaaaaag 97620
   agaatataag aagtgttgga ccatagttac acagattcta aggaaaataa agtatataat 97680
   ctttttaata aagtctatac ttatctatca aaaattgtct ttggatactt ttagaatcat 97740
   caaaaaccat ttaaataccc attgaaatgc ttaaatattt taaaaagccc aggagaacag 97800
   atgacgtgtg ttatgtagtt gttagatatt gaaaataata ggtatgcacg aaaggaaaat 97860
   caggtggtat ccatcttgga aaggcgacta aaccctttcc gttgacaaaa ctgaataaaa 97920
   caaacatacc agatcaccaa taaccttgaa tatatatctt tttttttatc aaggactata 97980
   ttataaaaaa aaactcaatt attagaccat gagttcgtat atggtgaagt aagggttata 98040
   ttgaaaagtt aagcccgccc tcttctgatt gtcattgact tcaaagtaaa cctatatctc 98100
   ttcttttcca atcaagattc tctatatata aaagagattc aagaaacata taactacaga 98160
   aagaaaaaaa acaaagaaac aaatggagac atggagaaaa atgaaatctt ttgggcataa 98220
   gagctcttca agcacggctt cgatcaccaa gagcaagtct tggaatggct ctgctcatct 98280
   cgagaatgct aataacaagg aatcaacagg aaagatcaag aaaaaatcgc cgccgccgcc 98340
   accacacgga tgtttcacag tttacgtggg tcccacgaaa gagagagtcg tggtgaaaac 98400
   gaaactgttg aaccatcctt tgctcaagaa cttgttagaa gaagcagagg ctgaatatgg 98460
   atatagacgt gatgggccta ttgttcttcc ttgcgaggtt gacttcttct acaaggtttt 98520
   ggctaatatg aagtttaatg gtgatgagta cgatgaagaa gatgatgatg atgatggtat 98580
   gattaaccct ccgatttgcg gtttgggtag tccctataga tgtgctggtc tcgagtccat 98640
   gggcgtgaga cgtagcggct cgtacaagct tcttcgatct ccatctttgt tcaaattaag 98700
   taggttttga tttttttgtt tggttttttg aaaatgatat ataggttttg attttctttt 98760
   ttcccttctc cataatacta ggtatctaag atcttgttca taccattacc ttatgcataa 98820
   aagaaaaatg cgaggaaaaa aaagaaccct cacatttccc taaattatat tccatttgtt 98880
   tttctgagat tttgatgtct gattttgtat cttaatttac atgtgagtgt ttttggatga 98940
   cgcaaacttt gaattaaaga aattactaaa aacactaacg aaacaaacgc ttgtaaaccg 99000
   aattgtttgt tgttgaactt aaagccacta catcaaagat acaagaacat caaaaataaa 99060
   aagactcctc actaagattt tgattggtag aacctttaca agaacattat attctttatc 99120
   taatcactat ttttattaac ttgatatatt attcaagttt gaggtggtat gaaaaaccag 99180
   aaacagaatc tttacatatt taaaatagca tctattagat gtaaatgctc tttatgtaac 99240
   gatctcttat gcttttgatg agagcattta actttaaaat ataaaatact aaatataaaa 99300
   taaagattat ttaaattaaa ttaaaaatat acttatataa aaattaaatg gtatttaaaa 99360
   taaaatttat aattaatata tttaaatcat ttaaaataat agtattttag attaagaata 99420
   tgatgatttt atttatgaat cacttacccg tactctgcac tcacttataa taaaaaaaat 99480
   ttgtcatcca ctttaataat tttattaatg aaattatata atatttgcaa catagtacac 99540
   ttttatagca tagtgctaga attttatcag caactccata tctatacgga tggtaactgg 99600
   gtcattcgaa cacatcatta tattttgcta gttatataat tgttctttga ataaatttag 99660
   tgcattttta atttagctga cttcaagttt atatttaatc gtatcatatc taattaattt 99720
   taatatgcaa tccttttagc caattaattt tatatttaga ttttctgtaa ataaattatg 99780
   taatttcatt atcctaaaga taaaaataat taaatttcgt atgattcatg aattcaatcc 99840
   tgatttactg agaaaacaac tatgaagatt aatccaattt gggaattcat agattgaatt 99900
   caccttttgc aatcaaactt ttataaagag aaaaaggaat taaatttcgg tatggttcat 99960
   agatttaatt taattttatt ggaaaaaaca actaagatgc tggtccagtt aattctctgt 100020
   taattaggat gttatgaggc aagtattata gaatgaattc accattgcaa tcaaaccttg 100080
   tgacaattca tcttatgttg gaaaagagag caaagccact aatagatttg gggataaagc 100140
   aaaaagtgca ttcaggttat gagattatgt tttgagaaga tccatggatc tcaacaagtc 100200
   ttgttgagtt attacacatg ttgttcatcc aataatgata gtgagtgact taatggagag 100260
   ccaaaaacat ggaagatgga aaaataagga actttaccgc ggaagatgac aatccttgcc 100320
   tataagtcaa ttgaggcatc aagatagttt tgttcgagtt atacgaataa tagtctatgt 100380
   atagtcaaat tgggatattg agtagctaat aatacactta atcatgaagt tgatgtcatg 100440
   aagttaatac agcttcaaac atttgtttag aaaattaatg atatgtcatc ttatgtgact 100500
   tgacaagcac atgaaacctt atgtaactat gaatttaaca aatcactcat gagatgtgat 100560
   aatcattgtc ctcaatgcgg gactaaatat gaatccataa acaataatat ttttgagtgt 100620
   ccatcaactc ttcaaacttg ggttttaaca acatcatttt cttaattttt ggttttctct 100680
   attttaagtt tatataccaa tatgaattat ttattttatc ataagaacaa tattgaagac 100740
   tcaaaaatgg atagatatcc ttacccataa ataatatgat atatttaaaa agtgcaaaag 100800
   gctaaagatc tgatatcaac agtaaaagac ttagtaacgt ggtatagctt tcctacaaag 100860
   ttaaaggagt tatatatcct gaagagtaga tttgtgtaga aataaaagtt gtagattcat 100920
   tagttagaag acataaacat ctcagagatt tatatttcgt ttgttgtttt atttcgatat 100980
   agtttttcaa acgacttcaa gttcaaactt aagtaataaa tgagtcatat gatttgatat 101040
   tttttttaaa aaaaattatg gtaaatgatc tagccatata aaagagaatg gtttagtaca 101100
   attatatgtt aactctttat taaaattgac taacgatcgg ctcggcctct gcctaatgtt 101160
   tgaagtagct ctgcggtttt gtccgaaccg aaccgaacca aaatttttgg ctttcggttt 101220
   agttacggtt ttgggttcgg taagcttttg aaaaataatt tgatttttgg ttcggttcgg 101280
   ttcgttttcg attttcaaaa aaaactaaaa aaaaaacaaa atcactgaaa aaccaaacca 101340
   aaaaacccaa atttaaccga aaatatccaa aaaaaattag aaaactttac cgaaattaac 101400
   cggaaacaaa aaaaaatcgt tatttcagaa ataaaagtga aaaccaaaaa taatcgagaa 101460
   ccaaaccaaa cgaaaccgaa tcaaaatttt gttcagttaa tttcgaaatt ggtttccaaa 101520
   aattcggtta accgaaaacc gacgattcgg ttgggtctct ggcagggcta atttgaagtg 101580
   ctggagagaa agaaaagtaa agaaacggca ccgtttcgta tcattttttt ttcctcggca 101640
   ccgtttcgta tctatcatta agctttttta accttttaat gcagtctcca ttctcgggag 101700
   agatcaatta atactttttc caataaagtt cttttgaaga aaaaacagac tcgccttcct 101760
   cgtcatcagc ctttcttctt taacctaaaa atggatgatg atgatgctat tcgcgtaaag 101820
   ctagagaatc tcccgactcc tacttccgtc aacggaatca aaccctccgt aatcgatctc 101880
   tgcagcagcg acgaagaaga caacgacggc atcgatgctt ccagaaccgt cggcgagaag 101940
   agagcgcgaa gggactgtga tatcaatact ccggcgaaga gggtggcggt agaggaaggg 102000
   cttgggcaat cgtcgtcgat agtggctctc caggctacgc cttgtaacgt cgtgaggcct 102060
   tcttcgtcgg cggcgtcttg caagcagttc tggaaagcag gggattacga aggaacctct 102120
   ggtggtcact gggaagtctc tgcaggtagc gaatctcgaa ccgtgggttg tatacttctc 102180
   tatttatttg gggtaaagtt tgttgagatg ttaattggtt caggtgggtt tgatcatgtg 102240
   agagtacatc ccaagttctt gcattctaat gctacaagtc acaagtgggc tcttggaggt 102300
   atctttttta tattttttaa tcaaagtttt catttttatt atttttagca gtgttttatg 102360
   aagttggttg gaataagaat gtttgtttga ttttttgcag catttgctga gcttttggac 102420
   aatgctctgg atgaggtgtg gaatgttttg tttctatttt taatattttt tctgtttggt 102480
   ggtaatgttt tttttttttt ttctgttttg ggttatcagg tacacagtgg agctacttat 102540
   gttaatgtca acatgctaac caataagaaa gatggaagca ggatgctctt gatcgaaggt 102600
   atataatagt ttatttagta tttttttctt tctgtttgta ttcacgtttt gatgatgttg 102660
   tgtttgggaa attttcagat aatggaggcg gtatgaatcc tgagaagatg cgacactgca 102720
   tgtctttagg atactctgcc aagagcaaac ttgcaaacac tattggacag tgtaaggcaa 102780
   ctcttttacc tgcaagatta tatttttaaa tgcttcttcc atcaagaata cacttaaagt 102840
   tcatatgctt tttttttgaa gatggcaatg gattcaagac tagtactatg agacttggag 102900
   ctgatgttat tgtattctca cgttgccctg gcaaagatgg agataggtta gttggtttta 102960
   atgattttac tgggatatgt gttgtgtatt gaaagagatc aacaaaagct ttacaatgtt 103020
   ctgtttgctt gatgttggtt ttagctttac acagacaatt gggctgttgt catacacgtt 103080
   tctgaagagc acagggaaag aggacattgt tgtacccatg gtaagctaac tgtatgaaat 103140
   aaccattcat ataacccttg ataatctgga atatttgata gcatgtgact gatttgtaag 103200
   cataagaaag ttaggtaggc cagacactgt ttcagagtag cttttgtctg aaatactgtt 103260
   agaaaatagg cgtcaaactc cttgaaacct ttgtctgctt cagagttaac tgtttgtcca 103320
   aatttaatta gaattagcca attttaatct ctcttctaat cttcttctga tggtgattta 103380
   aatgaaagct cgactacgaa agggaaggtt cagaatggag tccaatagta cggtcttcag 103440
   ctagtgactg gaataagaac gtggatacga ttgttcaatg gtccccattc tctactgaag 103500
   acgagcttct ttgccaggta aaaaacaaag gagttgtttc ataatattta tagctacttg 103560
   tttattttga gaatatttcc acttatctgt gctctatggc tgttgtagtt caatctaatg 103620
   aaggagcatg ggacaaggat aatcatatat aacctctggg aagatgacca aggactgcta 103680
   gaacttgatt ttgacacgga tccacatgta tgtgtttttt tttactgtga ttttgatctg 103740
   caacgatgta aaagctttct gtattcgtat actttgacac acgtttggtt gcaggatatc 103800
   caacttagag gggtcaatag ggatgagaaa agtatcagta tggctgctca gtaccctaac 103860
   tctagacact tcctcacata caggcattca ctcagagtat gaatcttcta tccgtctttc 103920
   cttaacagtg gcagttgaaa ttgttttttt ttgttttacg aaattcattt gttaccttgt 103980
   gaattgttgt ctccagagtt atgtatcgat tctatacctg agagttccac ctgagttccg 104040
   tatcattctc cgaggaagag atgttgagca tcacaacatt gtgaatgaca tgatgcacac 104100
   aaaccaaatc acttatcgtc caaaagaagg acccggtgga caatctaatt tctcaaatgt 104160
   aatgtttttc acaacttagt tatactcaaa agacttcttc ctgcaaattt tatttgaaga 104220
   acttgcgcag tttctaaata tggttgtgtg gcaatattta taacagatgt ctgctgttgt 104280
   gacgattgga tttgttaagg atgcaaaaca tcacgttgat gtacaaggct tcaatgtcta 104340
   ccacaagaat cgccttatta aggtttctct cgctcttttc ggcttatatt acctttgttt 104400
   ctgtcagttt tttaactgtc ccactttgtt tttgtcagcc attttggagg atatggaatg 104460
   cagcaggaag tcaaggtcgt gggattatag gtaatcgtta ttttgcagga aggtctataa 104520
   tacatgattg gctcttttaa tgtgaagtct aatgcgttag tttgctaaaa ggtgttttgg 104580
   aagctgattt cgttgagccg gctcatgata agcaaggttt tgagcgtaca acagttttgt 104640
   ctagactcga gacacgtctt cttgtaatgc agaagaatta ttggtttgtt gctctcttct 104700
   cttgcttttt agaaaattgc cgatgcttca ctgaactctt tgcgcttctg atttattcag 104760
   gaggttgaac tgtcacagaa ttggatatgt ttcagcacat ggcaaaaagt ccgctaaaga 104820
   ctctgaagac agaggtacta acatcttctt cttttttttt taaacaatcg gggttttaaa 104880
   acgtgtgcta ataaacaaat ctcttggaca tttgtgtaga atcatcacca gagtatgcag 104940
   tcccaaccag gaaaagagct gctgctgctg catcgttgag ctttaaaact ccaactggtg 105000
   caaggacagt tgtgaatcga ggaggaaaag gaaaaggatc tgttagagat tctaatgggg 105060
   tcggttcatc agagaaaagt ggtaaacatg gaaacacctc ttccaaattt aatggacgag 105120
   caaaggctcg aggagctcct ccagctttag aagatatcaa cagtgatgag gactctgatt 105180
   acgatcctcc gggtgaagaa aatgtcactg agcttcctga gaaggtcctc caatgctctt 105240
   tcttttattt ttctccggta aatagaatta tgaacgtaac cttttgtgta cttgttctcg 105300
   cagagcttcg aaccaccaac caagccacgt tctactgatt cacgtaccct cagtcaacta 105360
   gagcaagaga atgaaacgtt aaaagagagg ttttgttacc ttacgttacc atgttatgat 105420
   tcatgtttct cacttgtttg aacaacactg taagctttgt gttttcctta attctcaggc 105480
   taaataaaaa ggaagctgtt tacttgctgt tgcaagaaga gctgcgacgt gagaaagagc 105540
   ttcgcaaaaa acttgaagct gaggtataaa ttctactctt taacattttt actgtgtctg 105600
   cttgcaaacc tataagcaac aatcagttag tcaccaatgg atgattcctc tttggtattg 105660
   ttaggttcaa agaacaaaag acgagttaga agacgtgaag aaagagcaag agagtttaat 105720
   cgacatattc tcagaggata gagacagacg cgacaaggag gaagaagatc tcagaaataa 105780
   gctagaggtt ccttctttct tcttatcacc tttctctact aaatcttctc tcagattcag 105840
   gaaatgtaaa ctttttcttg tggttgcagg aggcgtcaaa gaggatccaa gcgttgttag 105900
   atgaaaaatc ccgagggaga cgctagaggt ctggagctag ctcggaagga tagtcactgc 105960
   atggaggagg ataccattga ctcgtttagt tttttttt 105998
<210> 11
   <211> 59642
   <212> DNA
   <213> Brassica napus
<400> 11
   gtagataaca aaataaaact agtggttaat agacaaaata atatatatat atatatatat        60
   atatatatat atctatggtt tgttattttc tgtaaatttt tagtttttct aaaacaaatg       120
   atataaaaat aataaagatt tattttaaat tttggttttt tattttagtg tagttttagt       180
   ctaaatatat tttataactt tggtaaaaat gtataatttt ttgtaattta aatattatat       240
   aataaaatta aaagctgatt taaaaaaaaa ttccagtgta ataaatgtat aatacttata       300
   actaaagaaa ttatttaact tttctttaag atttatggta tacatattga ctaaaaatta       360
   taaattaact tacataaaaa ttaaaattac ttaattcata gagaagatta ttatttgtaa       420
   gtttttttgt ttgttttcta gaagggcaga tataattata tatatgaaaa attaatttct       480
   tgaaagaaaa tgaagtcagt cgtttggaaa ccacaaaagc acataaaaag tggattttgg       540
   tttctatgta taaaatagtg taggagttga aaaactgggt tcccaaaaat acagggaatc       600
   tgttttagtt aaatcttgat tgggtaaaaa gtagctttta gtgtctccgc tggtatcggt       660
   ctttctcgtt ttttgggtgt aaaatgttaa gaaaaatgtt tggaaaaact gcggtgagcg       720
   tgtcattctc attttgttat agaacaaaac ttaggttcac tctctagact ggatttttaa       780
   attcaccaca tctcctatca ctaatcaaat tgttacctag ataaataata aaaatattat       840
   ttttaaaaga acaaatctaa aataatagaa aaataataat gacgtcaatt ttaatgacgt       900
   taacgccgtt atctaaaccc taaaccctaa atcttaaact tcaaacctta aatttaaatc       960
   ttaaaatgta aatctaaacc ctaaacctta aactctaaaa ccaaacccta taccctaaac      1020
   cttggatcct aaactcaaat cgtaaatcct aaacctaaac cctataccat aaaggtttga      1080
   gttaatgttg atgtttttct tttagggttt aaggtttggg tttaaggcct aggatttggg      1140
   tttatggtct aggatttaag tttagagttt acgatttgga tttagggtct aggatttagg 1200
   tttagggtat atgatttggg tttcaagtct agagtttggg tttagaattt aggttgaggg 1260
   tatatgattt cggtttcaag tctagagttt gggtttagaa tttagggtat agtatttggg 1320
   tttaggggtt aggatttaga tttaaggttt agaatttagg gtttaaaatt taggatgagt 1380
   tttgttagtg gtgttaacgt tattaaaatt ggcaatcatt attttttatt attattttaa 1440
   tttttataaa aaaatattaa taatattttt attaatttag gtggtacttt gattagtgat 1500
   aagagttgtg gtgaattagg atgaaccaaa attttgttct ttgttatata ctagtggcaa 1560
   acccccgcgg agctttgatt taagttgttc tatttatcct gaagtgtata atagtatgac 1620
   aaatgtgtat cgtattcatg gtagcatatg ccactctcga gctctgaatg agaatgaaaa 1680
   ttttctcgaa aacattgatg aattgatata tgtgagagct tgttagcttg acaatttagc 1740
   aatataagaa tgttattatc agccttcatt tcatttttat agtgaaagaa agaatatgaa 1800
   cggtaattag caataaagaa cggttgcgta gtttccaaat aagttttttt ttgtgattct 1860
   tgaaaaatac agcgaaacat atctacaatg ctagattaac tttagctcat ggttcaattt 1920
   tctggcaaca gttacagcac gagcagaaat ccttaacaaa caaaaataag caaaaccgcc 1980
   accaccggtt ctaaccacta aaatgagtat ctcatttttt aaattagacc gtttgataga 2040
   attgcataac tgtctaacta agcatttgaa accacatggg tgttctcggt ttcttgatct 2100
   ctttctgagc attttgaaat tcaatcttaa aacattgtga ctgagagata ttacttggac 2160
   aggagcaaaa gggatatttc cagagctggt tcatatggaa attggcgtat cttactcgag 2220
   cccaggggcg gatctactaa gggagtagtg ggatctgacc ccattaaaat gaacaaatag 2280
   tttgttttgt ataagaaata ctaaagaatc ttcagctcaa ttgttgttag ttctagttct 2340
   gacctctata aattagggtt cagttcccta attgacactt tttcagtata tttttgcaga 2400
   tttcttttct tatttagtga catctacagt ccacgtaaca tattattaag aagaatcaaa 2460
   gcccatgtaa catttttaaa gcttattaca tttttatata gtgaaaacac tacttattct 2520
   ttaaaaatac ggccactata atacactata accccggtaa aaacatttct agatccgcca 2580
   ctcctcgagc cattagctag acaaacgagt tatatgtcgc tatcaactgg tttactactt 2640
   tcgtctttgg ccgtgacatt ttccgtatct gattgtgttt gattccaagt gttttcttct 2700
   tagtttactt tacacggcgt ggtttataca gaagcacaat aacataactc ctgagggtat 2760
   ttaaagtttc tatttttatt gaattataaa atattagggg tgtgtgaggt ggtgtatttg 2820
   ttgtatttta tgtattttat gtttgttatt attttttgtt atgaaaatgt ttgaaagtac 2880
   agttttatat attttttgct atagttaatt gatgttatag gatgatatta tcttatacca 2940
   atattttgaa tcccggtccg gatccgcggt tgaaccgata aatccagtaa tccgaatata 3000
   attcggtttg agttttatga agaaaccatt atttaaaaac ccaataaaac tcggaactcg 3060
   acaaccagtt gaatactggt caaaccaata agtaactttt atttattttt taaattcttt 3120
   aattatgtta ttaaattttt ttatttaaat taaaaaatga gattttctga ttttacttat 3180
   tttttttctg ctgccggata tcgactatgt atctttttct cttttttttt tcgtacatct 3240
   catttacact atttaatttg tgatttattt gttatttttt aagattttga tgaagatcca 3300
   tgccatttga aaaaaataaa gtgaacgatg ataaggaaaa cctgaattag ttgttgtgat 3360
   ttggtcggta ttagtttttt tggttatcga tattctatta ttatagttta atttttaact 3420
   tttgtttttt tatttaaagt ttaactttac ctttcacatt taaatactat ggatttcaag 3480
   tattaatatt tttttattag atgtcataac gtctaacttg ttaattgtaa aatagtctga 3540
   attaaatttt gatgttttgt atgttaaatg aaataaaaat aagaaaattt aagtgttttc 3600
   taaatattct taaaacataa atatgtatta tttttaatac ctaataagtt attatttaat 3660
   aaatttaatt aatatattga actgcggttc gtccgcggtc catcgagtaa tccgatgatc 3720
   cggtaaggtt cagtgtccgg tttcgggttt caaaacattg cttatacttt atacattatt 3780
   tgtttgtatg aaaatgtgca gtatattttg tattgtattt aattaagatt ttactgctta 3840
   tgatagatat tgttggagct taattggtgc ttacgtagtt atctaatata acgtaagaca 3900
   agagattgac ccgcacgcca gtgcggatgc taatttttac ggttttataa atttttattc 3960
   gttattttat aattagtgta agatgtgtcg tcgtataact aattgtattc aaaagatttg 4020
   gactgaatct ggtaaaaaga ttatttatcg tacaattata gtaacccatt tcagatacat 4080
   tggttattta gatattttta tgtttctaca catcagaatc aaaatcattt agacccggga 4140
   tgatccaact caaacctcat acataaattt ataatatcca agtggcgcct aatttcaaaa 4200
   tccaaaaaaa ttaatcccga aagaaacaac ttgtacctca atgagtattc gaatgtccat 4260
   acttaactga ttctgcaaat aaattaaaaa gaaaactctt tttatatatt aggcaaaaat 4320
   aagaaaaata gaaagaattt tttatttagg aaaatagtta tatgaagtga aacattaagt 4380
   gacaataaat gtaatacttt ttaattattt tgatttaaaa ttattatttt gtttacataa 4440
   actatgtctc gaaactgaga attttgactc ctgatacatg taatatttgt ctatttcttt 4500
   ttttttggtc atattggcta acaactttgc tgcaacaaaa ggcaagcaaa aacacaaaga 4560
   tagaatcaat cattatacat aaacaactaa cattgcagag acgtcctcgt atatcgacaa 4620
   cactaggctt aagcagtgga atgacgccat ggaaaacaaa gaacacaatc aatgatctaa 4680
   tgcttgtgat tggttccaca actagtgcag aaataacaat cttaacactg gctacaatag 4740
   cagaggcgat gtaacaccaa aatgccgagc ttttaaacag tacagagagt aaatcatctg 4800
   tagagcaatc ttccaaccca aaggctgagc ttgcaataca tgagcagcaa cgatacaaaa 4860
   gccatgataa taggaaagtt gaagttggtt caaaagaatg ttaataatca agtctgaaac 4920
   cacaaccaag ctaaaaatct acaatttaac attgagtact ccattctaga ggaagttaaa 4980
   aagatctgaa accaagaagt gatagacaac agacactcta taacagagta gaatgtagta 5040
   attaataagc aaagaacact ctttagcaga gtagaggaag gactctaaaa catgtttgtt 5100
   aatagacata aaatttatat tttgctttag attaatacac atattgatca ggcctgagta 5160
   tgcatgtgag caaatgcata ttaatttatt atcctctgtt atagagtagt tgtaaatgtt 5220
   aaaaagaaat tatgatggta attatttatt aaaaaaagat ctagtttttt ttataattct 5280
   gattaaataa ttcatttagg ctccaaatgt ttccaaccgc tccgtcccac accgcactta 5340
   acagtaacaa aaatctctac atatattata tatctatacg tttttataac tgatataacc 5400
   gcacctcagt tgtaccgctt gtcccgcacc gcttaatccg ctgttaccat tcggagcctt 5460
   agttagacca tatagttttg tattttcttg tgaatgtaca catatatagt acatactaca 5520
   taatgtctag aattagattt attggggttt ttaggtaata aaccataaaa tcttttttag 5580
   tacgtttaat acagaatcat aatgcaaaag aatgatattt taagaaaagg caatgtcatg 5640
   tacagttagc gaaaggataa gagagggaac aaagtgaact gagaagatta agatataata 5700
   atggacaagt gacattgtcg tggctatgtc tttaaaactc atatatggtc tctctttgtt 5760
   tggctttatg tccgattccg atttcttatc tacgttcaca tgcataacta ataatagtct 5820
   tcataaatat cttctccttt cctcattata atttttttta aatacaaaat cgaatttatt 5880
   gggagaatat ttcgtttaga tccgatatct tttctaatta tttatggaac aaacgttatt 5940
   ttatattaac tcaaatccac tatatgaatt atgtatgtaa gaacaaatat gaaaggtgag 6000
   gatgaatata attaaagact tgatgctaag tttggttaaa caaaactaag tgatggtggt 6060
   ttagggggcg actggttttc tcgttaccac ccgcaaacgc agcttttgcg attggtcgcg 6120
   gttgtcggcg atttgtaaca attactcaaa tcgctctaaa ccgcttcaaa ccgttccgaa 6180
   tctcataaat tcaaaagctg gctccagcta gcatttgcgg ttgcgaacgg ttgcgggagg 6240
   gtgaattttt ttttcttttt ttaaaacaat atatatacaa aagtaaaaat gtttaataaa 6300
   aaatttaaaa tttaaaattg acattatgaa aatattaaaa tatatctatt atattttaat 6360
   taaaataata aaattttata ataaaaacaa tttcaataaa ttttcgaaaa ttaaaattat 6420
   aactttctaa atataaattt tatatttatt ataattttat gatttttgat atttttataa 6480
   ttatattaaa tgtaaatatt gttaatttat tatttgattg ttaccgcatt tggtagttaa 6540
   ccagtcataa gtcacccgca aacgcaccaa tttttaaccg cagtacgagt cgtacaaatc 6600
   tcttaaaacc gctagaaacc gcaaccgccc gcatccacaa actcctgcaa ccgcaaccgc 6660
   tacgtttgaa ccagtcaggc ccttagtgat aaaaatgaag atgcagaatg ctgagatgat 6720
   atgtatcagt tcgcgaagta ctagaggagg tactacaggc gtgtgtcatg aagatggagc 6780
   ttaatgtgag tttgggtttt gtgatgtgcg tgggcttgct gagcttggaa aaggaaagat 6840
   gatatgtgtt taaagacata tggacgtttt ccataatgca aaagggagtt tgcttgaaga 6900
   tgaagttttc cattaatgaa aatggaaagt taccttaagt gtatttggaa gacttgagga 6960
   gcaagttaag gacgtggaag gcaagttctg gtctactata taaggaggga cgtgccttct 7020
   gagaaagcta gacctgagag aataaagaga gagaggtttc cttggtgtgt gttactgctt 7080
   ggtgtcgaag gacattctga agcattgtct gatggagtcc gatgtggact tagtttggtg 7140
   gcgttggagt tggcgccttg tgtggtggag ttagccattg tgtatagctc gtgtgagctt 7200
   tgtgtgtgct tgggtgatca agcgttttgg tgtcactggt gtgcgttggg tgctgacgta 7260
   cttggtgaag tacttccgag aagtgaaaga tcgaagcata gactcagggg gagtttagta 7320
   gaggcggttt cattgaagag atcagtggag attgcagctg tagaagacag tgtgctccga 7380
   tgcatcggat ggtgatctat gcatgcgtgc ttgattccta atctttgtag attgcctact 7440
   tagaaaagag tggtagacac tagtgtgtgt gtgtgtgtgt gttgtatcat atagcaattg 7500
   taggttgctc cttgttctaa gtcaatgaaa tctggacgag gtcccgagga tgtaggaaac 7560
   gaaccccgtt aacaaacttt gtgtgtttta ctttctgcac ttgtttattg tcgcctcatc 7620
   tgcactaaca attggtatca gagcgggtca cctaagttac tggtgagatc atggatgatg 7680
   aggacgaaac ttgttcagaa agtaggacaa agtttgattg aagatcgttg aagatggcgt 7740
   gatgggattt cttcctaggt ttggaaggtg atgatcttcg agttggtttt tgaccatgat 7800
   gtgattcata gggggagatg gaagacgtgg ttttcaagtc ggttatgatg agtgcacatg 7860
   catagtcaaa aagagggaga ttgaagatgc agtatgctga gatgatctgt atcagttcgc 7920
   gaagtactac aggcgtgtgt catgaagatg gagcttaatg tgagtttggg ttttgtgatg 7980
   tgtgtgggct tcctgagctt ggaaaatgaa agatgatatg tgcttgaaga catagggacg 8040
   ttttccataa agcaaaagag agtttgcttg aagatgaagt tttccattga tgaaaattga 8100
   aagttacctt aagtgtattt ggaagacttg aaaagcaagt taaagacgtg gagagcaagt 8160
   tctggtctgc tatataaaga gggaaatgtc ttctgagaaa gctaaacctc agagaataaa 8220
   gagagagagg tttccttggt gtgtgttact gcttggtgtc gaaggacatt ctgaagcatt 8280
   gtctgataga gtccgatgtg gacttagttt ggtggcgttg gagttggcgc tttgtgtcgt 8340
   ggagttagcc attgtgtata gctcgtgtga gctttgtgtg tgcttgggtg atcaagcgtt 8400
   ttggtgtcac tgatgtgcgt tgggtgctga cgtacttggt gaagtatttc cgagaagtgg 8460
   aagattaaag tctagactca gggggagttt agcagaggcg atttcattga agagatctgt 8520
   ggagattgca gctgtagaag acagtgtgct ccgatgcgtc ggatagtgat ctatgcatgc 8580
   gtgcttgatt cctaatcttt gtagattgct acttacacta atgtgtgtgt gttgtatcat 8640
   atatcaattg tagattgctc cttattctaa gtcaataaaa tctggacgag gtcccgggaa 8700
   tatagaaaat gaaccccgtt aacaaatttt gtgtatttta ctttctgcac ttgtttattg 8760
   tcgcctcatc tctactaaca aaatatacct tacaacatga tgctactgac tcagttttcc 8820
   tccaggtttg atttttataa aactctttca cacctcttat gggcaagttg aaatggggtt 8880
   atatttcaaa ttcataaaaa aatttattac tcatggttac tctcaccttg aaaaaaataa 8940
   taattgaatt gtgttaaaat ccaaatcaca gaatatatat atatatatat atgtatatat 9000
   atgtaagaac ttatttttca gcaaaacaaa atttgatttc aagattccac ctcatgatat 9060
   taacagagaa aacattacct cttatttaac tggttgatat tttatacgag tatggaagtt 9120
   cctaaaagtg atcaaatgtg tgaaataaat atgccggcaa aaggcagaac tatgacttta 9180
   gctttcagct ctgtttacct ttgcttatgt ttttccccaa ccaactaaga aacatttgtt 9240
   tacttttgtg tgacattact cattaagtga ctgagaattt tctaactccg gcaaacaaaa 9300
   tcatttctaa agaatgctgt attaaactaa agtgattgga cccactagtc aagttacttt 9360
   taccgtgaac tactgtttca ctctattttg gcttcatgct tagtgttcta aaattatgtt 9420
   tgagtgtcct aattaagaac aagagaacta attccacagc cggaaattcc aaactgaaac 9480
   ctgttttctc aaatctccaa atctatgaag ccatatatgt aaatttcgta gtggcgaatc 9540
   ggaatatgtg ttgctctatg gttgtagttg attttcgact tgatcacttt atttaatgac 9600
   aagaacagca atgttttgtc ctaagaaaag gttgatgagc ctgacacaaa aagggaggaa 9660
   gccaagaatt tgttggggtc gaacgaagtg tcatgctaca gaaaagaaat gtcatggttt 9720
   aagggtccgc taattcatta gatagttcga tgtttttata tagtagagag acagtgcctc 9780
   acacgtgcat gtacgtccca tctttttctt gtcctgtaag ccatcctttt aaacactatt 9840
   gttaatccac aaacctaact tttaactatt taaatggttt tagttttcat ctagttatca 9900
   agaagtaact taaaaacatc tccaaaaagt attctataac tttaaatatg aagttttttg 9960
   cattccaaaa aataaatttc aaaactttaa atttgaagtt tcatatattt gtttgcattt 10020
   tactccctac aattacacat cacatttaaa aattcttgtt tattgtttta atcttttaaa 10080
   aaaatatctc ataaatattt tgactttttt ataaatttaa tttttacata taaaattaaa 10140
   taaaacttta aaataagatt taaaatgttt taaaactaga tttaaacaac aacaatatac 10200
   aaaagaaact taaaagaaaa ctttaaaatt acatgaagac ataactacta cacaaattta 10260
   aatattacaa tagttatgta aatttgattc ggaacctcca aaatctttaa aatattgtcc 10320
   aaacaaattt tgtttaacca aaaatggttg ttgttgtttt tgatgttttt cgtacgattt 10380
   tttcttgttc agattgaatt tatgcacgag tattaacatc atcaatagaa gttaagtctt 10440
   ttagcaatat tttatttttc tcttttacat ttttttagat ctaatgtatt tataatatta 10500
   gtttcactag atttaaataa tttttagctc gtattctaca aattacaaat aaagatagtc 10560
   atttttactt caaaatacac tagattatca tatatgcatt acaaaaataa ttttatagaa 10620
   tattatggta ttttccttaa atattaatat taattatgtt atttctattt aaaattttac 10680
   taattaatat tttgtaatac gtttatatat gtgttagtaa aagtttgatg aatttaaatt 10740
   aataataaca aatatagtac tccaaagctc tattcatgca taacatggcg ggtggcaatc 10800
   caaaaaatat tcacatcacg tatgttttca gtgttgacta tacgaggatt cactacaaaa 10860
   aaaatagcca tattgttacg aatttttttg tcacaataaa gaataattcg taacaataaa 10920
   aatattgtga ccagtttgtg acgttcttta aacggtgaca atatgatcgt cacaaatttt 10980
   gttggtaaca aaaaacgtca ctctgtttat gacgatatat attgtaacta tttcgtcaca 11040
   gatagcaact atttactaaa gtaagaaaaa cgttagaatt accaaccaca attaacatca 11100
   caaaattgtc atattatgtg actgtctaca agtcttaatt tcgtctctag ttaccactaa 11160
   aataaagttt caaaccgtcg caagaaaata cgaaaaaata attactaatc catcttcatc 11220
   gtgacttttc gtggccttaa actttgtaac attttattac taaataattg tcactaattc 11280
   atattacttt tttatatttc atcgctaatt tatcatcaaa cttctggctt aactaattat 11340
   taatttgtca tctcaaaagt ttcaacattg atcactatat tattgatgat ttgtagttta 11400
   aatacatagt aatgaaaaca ataccaaaat aaactgataa cataaataat aaaccacaat 11460
   aaaagaatat tctaatatgc gatcagattc agtttagcga tctccagttg tgactcatga 11520
   taaaaggctt cttgattttt agcatcggtt gattcttgca cacgaagcga aaggtacgac 11580
   tgctaacttg tattctttaa agtgaccacc ttgtaactgg cttcttcttc catgatttta 11640
   ttaccaatgg ctcagatctg cttcacctct gctgtcttta agcttaagat ctactctcta 11700
   ttgaactctc cttaatgtcc cagatacgca taccaccaca gcgccagaaa cgactgttgg 11760
   tgacaacacc gtcaacacaa atctctcacg cttttccatt tttacgctct cgctcttcct 11820
   aaggtgcctc tgcggctttt cttttggagg aaatatatac actttttatt tagggtttct 11880
   aggctaatgg gctctaagcc tctacttatt agtctaaggg ttccggctta aaatagagat 11940
   acggtggatt cagattaaag tatttttacc tatattacga tccatcttca atagaaatag 12000
   ttgatatttt ctattgttta tcatttttac atataaatat aatcttctat ctaatacaaa 12060
   ctatatatgt attgtttttt ctttaaactg atttacataa ataataaaat tttagtttat 12120
   acataattta aaacttatca taaaatgata tataaaacat ccatattttc aattagtatg 12180
   agaaaaagta ctttttgaaa tccttcacgt ttatacatag gggcatataa atcgactata 12240
   taaacattgc taaatcaatt tctaaatatt tcatggattt cttacatctt tgagtagaag 12300
   tttattacaa aaaaaatttc atctacgata tgtaccagtt agaaactatt attttgttat 12360
   tataccgtca caaagtaaaa ctaacttttc catcacaagt tcgtaattag tgtgacgaga 12420
   catacagtca taatatggta acagatgtta accaattgtt aatttgtcac aattttgtca 12480
   ttgattgcga caaaacttag ataccatttg aatcatcaca atattgtgtc aaaaaagcta 12540
   ctcatctgtg acacaaaaat ttggtcacca taacgtatct aaaatgtcat aaatttgtga 12600
   caaatatttt tttgtatcaa aatttagtca caatataatc attttctcgt agtgattgga 12660
   ctgaaatgca taagacacta aacctttcgg agatgcgtgg attggggctt atttgccaaa 12720
   taaccaaaac agaggtaatt agatctgtag tgagaagtta gagagatata gaaagagaat 12780
   tgaagagaaa gaggatgttt ttggttagat agtgtatttg tgtttttgta tctttagagg 12840
   ggcaaatttc ctttggttaa ttccggatat gtatcactat cagtccactt ctttaacatg 12900
   ttttttttta acatgttaat tttcagctgt ggtggatcat gtactaatct tctcacatac 12960
   aataaacaaa ttggtaaact agacatagag gatcattagt attaagatgt cttatagtaa 13020
   aataaagaca attttataag agttagatat tagtagttat gagaagtata taattaattt 13080
   atgacggttc ggattggcac gagttcatca caaataaata aaaagctatc agtttattat 13140
   gggattagat aacatagtct tgaaggctat gaactcgaac attctttaac tatggtccac 13200
   tacaaggcct gagtaaagta ttctctacgg ttcatgcaag actagcttgc gcaacttgat 13260
   tgtggtccag gatccttttt tgcttgtagt ggacctattg gtgactcaaa atgtgtttgt 13320
   caggttcatg cagtacaata aatcttttta tttttcaagc aagggtcatg gactatatta 13380
   ttgtgctttg ttactatgca tgagccacgt gaatgcatca atattgatag ggccgtttct 13440
   ttttttttct tttttttttc gagcaacaag ggctgttctt atatatacaa aataagcatt 13500
   agtgttgaaa atcccactca tgagtgattt aatggtagat ggattttggg aaactaaaca 13560
   atccagattc gaatcaaccc cacgatatta aacagtgtag tcacgcagat atgaaactat 13620
   tatttgagtc ccatttgaat attcagaaaa aaaattcata tttagaccat gtatctccac 13680
   ttgagagact agtttgagtt tttctatagg tttgggatac tctcaagtta atcaacaaaa 13740
   ctccaaatat tttagttata taaagtatat aatctcaaat cattaaaaca aaattttata 13800
   tttagcctct aatattaaat gtgaaaatgt atatttgggt taggttgtga atggttaatg 13860
   gccctttcat ttttttccca gctggacgat ccatttctta cgggttttgt ggtctaaata 13920
   aaaatgataa tgtatgtctg catgcacgcc gatggaacat gtaattctta tccgtgtaat 13980
   ggtggcctct atagcactta tatgataaag aaaaattaaa ggtgaattcg aatacttcca 14040
   tgttgcaaat ttatgctaat attttcgaat acattttcca tccgactgag agaaaaagtg 14100
   ttgtggggtt gggttacaaa caaatgcgag gtaggtgcat gttcgataca cgaaaagaca 14160
   tcattattta cgacgtgttc tataccccgt ccattccctc aacacttgta ttgtttaaat 14220
   caagttaaga attgtatttt tatgattttt actatgatta gttggaaatc caaaataatt 14280
   cattaaagat gagaaattag taacgattag ctttcactaa tccatttttt ctttataacc 14340
   ccacacgttg agttatttcg gtctaatacg taagcttcat atgttgtcct ttgaaaattt 14400
   agaaaaccta cggatgcata ttctctcggc cccactcttt gtttttttgg taattagctt 14460
   aatgataaat ggtttttaag aaaactaggt gactgatctg caccctgtgc ggacataaga 14520
   acatgatcgg cccgcaccat gtgttctctt tcggtctgca cctcacgaga gggaacacag 14580
   taacgtcagt atgaagaggt agatattgtg tgtatgtata attgcaacgc cacaaaatat 14640
   tttgtgtgtt tacgaagata ttttcattca aaaaatgaaa taaatagtgt atagttttag 14700
   aaataacata ttttatatta ttattaatta gaattgtatt gtatatgtgt cgtaactctt 14760
   tattttaggt gaataatatt atttttccat aaataataaa caaacattta tgtagacata 14820
   ttaaaagaaa atataataaa aatcaaaata ttatccataa ataatataaa ttatgaagta 14880
   tatttctcga taaagaaagc aaattcaatt agaaacctgc aaaaattaaa taaattttgt 14940
   aagcaattgg acgggttaac attatttgat agatttataa atttttaaat tttattgaac 15000
   atgaaataat attaaattga cataccgtca tcggtctcct aactcatcac aacccatcta 15060
   acaaatacaa aaaataaata attgtaacag tttatatatt ttaaaatttg tatttgaaaa 15120
   aaaagtagat taaatttacg taccgtaact acgaaatatt ctgaaaaact tgtttgtaga 15180
   caacattcaa tgtttttgtc tgtggcttcc cttctttacc agttattagg atttttaatc 15240
   cagatctcga cttaactctt gaaagtgcaa ctttgccttg cattttgtaa gcattttata 15300
   aattatgata aattatgata aatttattct ttaaacaacg ataaataatt taaaaataat 15360
   attaataaac atttttggat tttgtaatat tgaaaatagt tattatataa ttatattcga 15420
   acacaattca tcaagtagag tataaaacta ttataattat cttatataaa atttcgttca 15480
   ttgtatttta tagtttataa atattaaaac aaataaatgg tagagtatgt aacacctgtt 15540
   ttctaccagc gtgagttaga acaccgccgt atttaagaat cataagggcc tctacaggtc 15600
   tttcttcttc gccttcacca tcccacttta gcggcttcag ttaaaccttc ttgtatatcc 15660
   ctgagaaatt tcctccctgc gccattccaa aaggttctct gctgtgagaa aatgacctca 15720
   tgaattaaat aaaaaatgca taatgctagc ttacttataa gatagtatat tcaacaaaaa 15780
   aatgatataa gatagtatta cttgtcccct ggtcacattt atgtattata tacatagatc 15840
   ctttgatcct tcctgtatat tcaacacaaa ctcttcacca actcaagcat gaattcttgt 15900
   cctctggtca caaaaattat aagagtttta taaacgcaga ataagtatat aaacaatgct 15960
   tctaaaaaat gtattttgtg tgatgtatac ttacttcttt atcaacaacc accatttcaa 16020
   tggtgtttcc tgattctttg ttatagttta tccacaagcg aacaatccta atttcttttt 16080
   ttttgacgct gatttattaa gatattacaa ttacgatatg agaaagatta catagacgat 16140
   tcggcaaccg gcaatactac ctgccttatg aagacctacg cctaactgca tcacctgagc 16200
   cgtcctatga aaatccacgc ctggccagat ttatttgcac catgttgaag atcccttgta 16260
   tgtctttctt ctgtagtctg cataattctt taataaactg ctctctccgg gacttgaaac 16320
   ctggatttct tgtaatctgc aataaattgc atagtctgag attcgaaccc cagacctggg 16380
   tgtagaagcc tttaaacctt aaccagtagg ctagggtgct tccacaacaa tcctaacttc 16440
   aatacgcgac gtatctttac ggggttttaa ttctctcaca taagaaacaa tatttttttt 16500
   tgctcgcacc attgccattg ttcttgtaag agctgaatgt ttgtaattta agcattcggg 16560
   tttctcatat atatattaag ccgatttatt tatcatatta atgaccccta ataaatatta 16620
   aaatcgttta aagaaagata ttaattgtgt attgtcataa attgatttgc atatgatatg 16680
   attttaactt gcgcaagtaa tgtaataaat atgataacaa ccggcgcaag caattgattc 16740
   gaataataag gaaagttatt aatatgcaaa ttagttaaca atcttgcgca agttatctaa 16800
   ttattatccc caaatcgaat aaatatatgg gcttaacatc taccgacaat atatttgggt 16860
   ttttctaaac aggctattca catttttctc ataaataaga agaccaaact tgaaaatccg 16920
   aaaaccaaca gaccggaaac cgcaaggaat gaaactgact gactaaatta gtaatgtgaa 16980
   gctctaaatc atgctgcaat tagtgatgca acagactatc gcaattagta atgtgaagct 17040
   caaaatcgtg ctaatgaaac aaaaaccaaa cttgaaaatg tcaagagagg actgtctaaa 17100
   tgcttgttga gttattaagg agaagataat cttacagtcc ttcttcagag actgaagtcg 17160
   aattgccgat tgttgtaaat gtcagttgtc tcttcttgtt ccatcagtag acagtccgca 17220
   aaatacatct ctggtcgtgg caatggtctt gtttaagaga atcaagaact caacaacaag 17280
   aagattaatc agtcaattag atacaaagat tcaaagtgtt aataagcaaa tcgtagttta 17340
   tagcttaaca tatgtcgaat ctaatcagtg aaatccaaaa atctttgtat catagcttaa 17400
   aagtcgaatc caatcagaga aaccgaaaaa tcttcgcatt atagcttaaa agtcgaatcc 17460
   aatcagagaa accaaaaaaa ctataaattc cgagagtatc gacaaacttc acctcgtctg 17520
   gttgtcgctt caattgtttt catcggtttg aaaccatatc tcccttcaat tgatacgcga 17580
   ttgaaaaaaa aaaaaaaaaa aaacttcatg acatacgacg gtgttttcaa atccgtggag 17640
   gggagtgaaa aaaataaatg aaaaagaaaa attccaaaaa atcagccaat agaattataa 17700
   ggattttccc gagaagctct atatgagtgc cacgtcagca gaaatcacta aagtgacttc 17760
   tcttttaatt tttaggagga taattctcta gcttgggtaa aatcgtggat atctacgaaa 17820
   tgattccttt ctacgtacac gacttttcat caaatacgaa tggttagtac aattaatagt 17880
   ccatccgttc ctaaaagatc tatgttttag aattttcaca ctttttaata aaacactagg 17940
   ataagacctg cgccttgcgc agggtgaatt tatttatata tattatcgat aattttttta 18000
   tatattggat cattttattt atacttatat aatgtttttt tgttgttatt atataatttt 18060
   tttccgatga ccggatcaat ttttattaaa aattatggaa ctaaactata aataataaat 18120
   catgggttga tcggattgga cattaagcaa attatgacac aaaaatttta ttttttccac 18180
   cgaacacatt cttgaaaaaa ttcaacagta ttattttcac agttgaatta ttttgacatt 18240
   tatcttccat atggttttga aaggtctcag atcaaccatc gaattgatac atgtcatttt 18300
   aatgttttta atcgtattct taagggaaaa ctaacatttt tgtaatttaa agtggtttta 18360
   aaaaattcaa aatataacat ataagaaaaa aatctaatat ataagaaaag tataacatat 18420
   aaggtttact catttttgta atataaagtc gttttacgaa tttaaaatat aacatataat 18480
   gtctcctcat ttttgtaatt taaagtcatt ttagaaaatt caaaatataa catatgagaa 18540
   aaaaaatcta actttttatt atatggttaa tgtcactgtt tattgttttt taataatata 18600
   aatttaaaca aaaattcaga aggatgtaaa aattgttatc aaatctttat tattcataat 18660
   cattaattat catatttatg ttaatcacat taggtaattt cgtagttttt atttaaggaa 18720
   ataatacact cttcttatat tttagattaa tataatgttt tctagtaatt aaattttgaa 18780
   ccaacatttt ttcaatattg atttttaagc tgtcacgtaa gttaaattat tatcctaatt 18840
   aaatgacacc gaatcagagt cttttttaat tagtacaaac ttagagttat aatttttaaa 18900
   tgattttcaa ttaatatacg tacatgagaa actaaaacag cttgttatat aactaccgag 18960
   atatttgatc ggattagcat aagcaatatt taatagcctt ggccgcaaat tctcaattga 19020
   tacgccctca catataagct ctatatatta tttagtttcc attagttcct taggcttaat 19080
   taatagtctt ggctataaag tctcccacta cgatgaattt ccataggtta atgtgttagt 19140
   ttataaaata tattaataat atattgcctt ggccacaaag acttaacaaa catattttat 19200
   ggatctcaca cgattattaa tatttccatg ggcagctttt ccttgaagaa aaatgagaaa 19260
   taaaaaaaat tgattaaatt cgtttaacat aaataccaaa actggtaatg attgatttaa 19320
   cataacccta aattagtttg tgatatgaac cggttaaatt gtagagcagt actttttgaa 19380
   tcacatgaaa ctcaaaagta atctgccgtt tttatatacc tcacttacag taataattac 19440
   atgattttag aacaaaaatt ctctagaacc aactgaagaa ggactcccca accattgttt 19500
   tacaaaaaaa aggacccccc aaccattcat gcaaacagac atagttatga ccctttaaac 19560
   aatatcatag tacagattat aaagtttttt atcaagtgac tgaatttttc tggtaaacca 19620
   cgtttgctac atatacaata taattaataa agtggatatg agaaaatcag gaagattaac 19680
   tgaaacttgt gtagcatagt tctattacag tggtgaatgt tcttattaat caaggtagat 19740
   aatattaact gacgataatg ttctaacgat aatgttcctg tcaataattt ttgtaagtga 19800
   tgtaggtctg tttattttcg tacataatgc atagaaaatt acatgttcta ttttctacaa 19860
   acttgaagta aaatgagaac atttaatatt tattccctat aaaatgtatt cgtagacgtt 19920
   attacatagt tatgcttaca tgataagaaa aacatacaca ataaataata ctgatggatt 19980
   acactatggt tttacatagc ataggcgcac ctgccgtctt atttttagac tatgtatatg 20040
   tgactgtcaa aaattgtatt tcgctaggga gttaatttat aaactatgct atttcttaat 20100
   gtgttataat tctgacacgt cagattttag aaggcttaaa caactgccac ataggatggg 20160
   gtcttttttt aatttttaca aaattcaggt tataactttt taaaagatcc tcaattaata 20220
   tataggggat attaagactt agttataaat acatagtttt ttttgtaatt ttatatttta 20280
   tatattttta aactaataag attctaaaaa ataaaattaa tgttcttgaa ctttacaatt 20340
   tctcactatt gttgacaaaa ttacattgaa aatataaaat atgtatattt ttaaagcaaa 20400
   agttttctat agaatatgaa tcttttagaa acggaaagag tataagatat gtcaacacgt 20460
   caagacgtgt atgataattg ataagtacat ttactcgtag ttaattaggg aaaatatgaa 20520
   actacatatc atatatacat aacattatta aaatagaata aaactgtaat catatggagg 20580
   tggttcagtg gtaaacggac ttcagaaaac ataaatttga ataaattcgt gtggtcaaac 20640
   agatatgaaa ctatatctta aacttcattc taatatctag aaagacagtc catctatagg 20700
   ttttaccttc atgtttatag caaaaaaaaa gagaatgaaa aatgtcaaaa aaacatcata 20760
   aaaatgtcat tataacctaa gaaatcgtaa tatcattttc atctcgctat caattcaatt 20820
   caacctaagt cataactgta tcaactaatg tgtatatata tattgtctcc ttcaaataaa 20880
   gctcgaaata tgtaacgatt tattcgttaa ttgtttaaag ttcatactta aaacaaagtg 20940
   gcccttcgga aatcacgagg aaatcgaagg atgttctcca ccatgtgcgt atgctaaata 21000
   acaaacacat acttcttttt acattttagg atttattctt aaactattat tctgatatct 21060
   aaacacacat atataaatag tagaaatggt acatagcaag tcgcctacat tagtttctta 21120
   ttcttgaaga gcttcattcg tgaggaaaat taactatagt tctctaagtt tggcaatctt 21180
   tgatgtgaaa aaaactatgg aaaaattccc tctaattagc accagtccca cgtttcacac 21240
   cttcattaag agaaaattgt aatgtgcact caattaattc catagtttat aggaaaatat 21300
   gatagtcttt taagccgggc tacaactaga cgcttgtgga tgtgagcaat ctaagttaga 21360
   tattacccgg cagatactat gacttacaaa gtacatccta tgtttctaat tacttgtaaa 21420
   cggtgcgctt taggttgcca actctggtca tagagtgtca caaccatgtg aaaatgtttt 21480
   atccaaataa agaaaagttg ttacaagtaa ttttaatgag taactagatc tcgatccgcg 21540
   cacatgtgct gatttttatt ttcatttctt tttatataaa tattttgttt taaattctaa 21600
   attagtatat attataatat atatgtgtct atcaattttt aaaacataat aagtttacgg 21660
   tatatttttt cattgaataa tttgtttcaa actttcacat atatttgtat ctttttctat 21720
   atatattttc gaataattat tttattatta aactcgtaac tatatatata aagattacta 21780
   aaatattatt ttattgtcat attcaaagat attgtaacat ttcacaaatt tagaaagttt 21840
   ttaaaaaatt aaactttttc gtttcataga tttatattat cgagtaaata attaaacatt 21900
   tagtttttgt tttaattttt aaaataaact atatagtttg aaatttgttt tcattggttt 21960
   aaggtagtaa atattaatca ttgttagata atatgatttt tgttatttaa attttttttt 22020
   ataattttaa aaattaacat cgacaaatat ttaattattt aacatatgga ggtatagtat 22080
   tataatatta aattatatct attttattta tactatatat aaatccaatg gatcatctat 22140
   tgtttaaatt caattattga tagttcaata aaattttctg gtaggcctaa aattttaata 22200
   ataagattat agattaaatg taatatgact tttttagaat aaattcatta ggtccatttt 22260
   ttaaaaaatt acacatgaat cgaagttgtg acttatgttt taatatatat ataagattgc 22320
   atatagtccg aacgtactta atgcaactaa gtccaatata tatacaatta tattaagtcg 22380
   ttgatgattg aatcgcaaag gcgtgttgga aaacaatcga agagagaaga agaggtatgt 22440
   tcaaaaaaaa gaaaagaaga agaggtagat gaaaccctca attttaaaat tcaatggggt 22500
   gattaggtta gaagtaaaat aaaaaaaaat tgtgtagaat ttagtttgta tgattttttt 22560
   atttaactgt aaggaaagta ttttaaaatt ttattgctgt agcattattt tttctacagc 22620
   taaaaattgt tgttttagaa aatatagttt ttttacatct atttttaatc ttcctgttgt 22680
   agttttcaga actattctaa agcataattg ataattttaa aggttataga taaaaattaa 22740
   aactaaaaac agctactata acacaatcca ccaccccaag tctccaccac tagccacatt 22800
   aaatgaattg attttagttc attcaccatt tataatctta ttatatattc ttaataaaat 22860
   acaaaatata tatattagaa atgatgctat tttttttttg taactggaga aatgatgcta 22920
   tttttaatca accatttaac ccacttgacc cacacaatga atttgttctg tttttgtgtt 22980
   gttatttccg gataaagtga attagttcca tccaactgat tcttctacgt atgataggtt 23040
   tctaagcatc taactagtat gcagtattat attacgtgat gaatgaaaaa caaaaaacca 23100
   ccaactacgt tatgccaaaa atagaacttt tttttccgcg gggggggggg gagaggggta 23160
   acaaatacaa aaaaaaaaag ttattcttgg gttcaccccc tagagtgaac ttctaggttc 23220
   accaaccaat atgattttat tatttcaaat tcgatatttg ttaaaaaagg aaataaaata 23280
   ttgtcaagtt atattatgct tttaaaataa aaaggtaaaa aaaaatagtt acaaaaaaaa 23340
   gaagttttta aaaaaaatac tgttaacgtc gccagcaaaa cactaaactc taaatcctaa 23400
   tccctaaacc ctaaatctga aaccctaaac ctttgggtaa accctaaacc cttgggtaaa 23460
   ccctaaatcc ttggataaat cataaattct aaatcaaaaa cactaaacac taaaatccta 23520
   aacccttgag tgttttagtg tttagtgttt ttgatttaga gtttatgatt tatccaaggg 23580
   tttagggttt cagatttagg gtttaggaat taggatttag ggtttacttt tttcctgacg 23640
   acgttaaaaa tatttttttg taattactac tatttttatt ttttattttt tatcttttta 23700
   ttttaaaaac ataatataac ttgacaatat tttgtttctt tttttaaaag atattgaatc 23760
   tgaaataatg aaattctatt ggttggtgaa cctagaaatt caccctaggg agtgaaccca 23820
   agaataagtc aaaataaaat cgctattaaa gcaagacatc ttccaaaaat ataaaaaaaa 23880
   taaaaaaaac caaagtcatc tcaaataaat aaaaccgctg gatacatgtt tagtaagtca 23940
   aacaaatcat agtgatgtgg caactgtttt ttcctcaact ttcctcaatt taatttgcta 24000
   gcaatttcta ctcaattcaa ttctaagcta ctacccatta actacttcat ttttttttta 24060
   gattttctta tttattggga agttttatta atcactttta tgatgaacta attccttata 24120
   tattatttga gaaaattaca atatttaaaa cgtgtagtgt atggttctca gattacctaa 24180
   agaaataaat tggtcaatct aaatatacac ggtagttctc attaaattaa ctaaaaaact 24240
   aattactaat gtaccaaaag aaattattat ttagtttctt aaataaaagc tacaaaatta 24300
   ttaaatgtga tcaatatata tacatgacaa ctagtgattt tgaataataa aaaattgata 24360
   acaatttgtg tttcttctat attttgtttt atatttttaa aataaattaa ataatcatat 24420
   taatcataga ataaaatttt aaattttttc ttatatgcga tactttgatt ttttttaaac 24480
   aactataaat tattaaaact gtaaaaaata ttacattaaa aattttgtga gtaatggctt 24540
   aaattttttg ttatacaata tataaatata caaatgatca taaaatcata tgaataaaat 24600
   atcttattta atagattttc atattaaaaa tatgttttta ctatcgttta aattaaacta 24660
   tataccatat aagaacataa tagtttaatt tgaaatttgc attgaagaaa tattgagaac 24720
   ttaatattct aattttatat tttgtattaa atttttaaaa acaattataa attactaaaa 24780
   ctattaaaag tatcccattg agaattttat tttcaatatt ttaaaaaata cgaattgtca 24840
   taaaactata taactataaa gcattattta acagatattt taaaatatac ttctatatat 24900
   taatattatt taaatttaat tatataccat agatataatt gaatttttag attttttata 24960
   tcaaaattat tttaagtaaa aagagtgttt gttttgattt atgtgttcgc gtcaacttaa 25020
   ttatatacat aatagttata gacttttcag tttattattt tattatttca tgtaaaaacg 25080
   taaaataaat aataatttat atacacaatg tccatcccgc acatataaaa ataattcatg 25140
   ttgatcttag cctagtcaat aaataatcga caaaatttta gggaacaaaa tatatatgct 25200
   agaggatcgt tatgtttgtc ttccattcca ctgcatctac atatggcatt tgattctaga 25260
   gtaagaaaca caaataaatt tatttggtac aatccttccg tccaaggaaa atctaaaaat 25320
   ataaaagaca tcttagtgaa gttatagatt atggtagcat tctatttata cccaagttta 25380
   aatatgattg tcgtataacg tattgaatag caaatatctt cgaatctcat atatatgaaa 25440
   ttagtgtaaa ttttaaacgt aaacaattta tacgaccaca gttcgaaaat aaaaacaatt 25500
   tatacgacca gaaatggcaa aatgttgttc ttagcatttt tttttttaac tttacttttg 25560
   cgtaaaacac atttctccaa tttggtttca ttgcgttgaa cgacgtaaca aagtaataca 25620
   cctaaccctt ttttttggaa cattatacac ccaacccatt gtacaaaagt tacagctaaa 25680
   ttaccctttt tattcttttg ataaataaaa aataaattat taatcattaa aaaataattt 25740
   ggagtatttt ctcaatgtcc atatatacat cttctccctt tatataagcc aacctcacac 25800
   acccaaaaaa tccatcaaac ctttcttcac cacatttcac tgaaaggcca cacatctaga 25860
   gagagaaact tcgtccaaat ctctctctcc agcaatggtt gttgctatgg accagcgcag 25920
   caatgttaac ggagattccg gtgcccggaa ggaagaaggg tttgatccaa gcgaacaacc 25980
   accgtttaag atcggagata tcagggcggc gattcctaag cattgttggg tgaagagtcc 26040
   tttgagatct atgagctacg tcgccagaga cattttcgcc gtcgcggctc tggccatggc 26100
   cgccgtgtat tttgatagct ggttcctctg gccactctac tgggttgccc aaggaaccct 26160
   tttctgggcc atcttcgttc ttggccacga ctggtaaatt aaattttctg ttttaattat 26220
   tttgactctt tttgttcaat ttattaattt cttgaatgca cgttcgatga gtatcgtcgt 26280
   cactgacttc aagatttaat tcttttgagg ttaccttttc atgttcaatt attaaaaaat 26340
   aaaataaaat ataggatcta agattttttt cttcatcagt tcaagcatca tcactcatca 26400
   gtcgtaagac tcgtaacaaa atatcttctt ttctataatt aatattattt ccgcatttaa 26460
   tggatctacg ttttgatgtt ctcaaatttt gtttctcttt ctctagatcc ccggaacttt 26520
   taattataat tatagtatag tataatatca agaaaatata ctgtttattt tttttggcaa 26580
   caaatatatt actcttgttt ctttgacaag aaaaaaatat attgtttttt tcttcttttt 26640
   gtgttccaat ctattttcga gatttagaca agtgacacgt catataccgg atttgttacc 26700
   ttgttaaaga gtttgggtta aaacaaatgt agaaaagtta aaataaattg tgcaataaat 26760
   gataaatacg tttttatgtt aaacaatgat gtgaaaataa aattgaataa tggcagtgga 26820
   catgggagtt tttcagacat tcctctgctg aacagtgtgg ttggtcacat tcttcattca 26880
   ttcatcctcg ttccttacca tggttggtaa gtcatttatt aactatttcc atgtaaacta 26940
   ttagtacttg ttttcgtatt tcttacattt tcgtttgtca ttcttcttgg gtgcatgcta 27000
   gcaaactgta atcagtatta actgggaact accaactgtt ttttttttgc tagagtagca 27060
   attttataat taaataagaa tcctattaaa caatgcatgt gacaatatga ggttgctttt 27120
   ctgttcaaaa caaatcttta gaagccaatg aaaaagaatc caaaactttt ttttaaatga 27180
   tatgcgccta tctattggtc ctgactcctg agttttctta ctttcttaag tataattaga 27240
   ttttgatttt tttttatagg ttttcactat tgttatttgt ttacatcagc ttcagatatc 27300
   ttcgaaaaag atttacatgc atcaatttca tgaggattta tagtttttct tttacttatt 27360
   tccgacacaa tgtttagtag taaaaagcat taaatgtttt tttgctcaaa aaaaaaagaa 27420
   tgggattgtt agagcactct attgttagtt gttcaataaa tataccaact aaaaaaacaa 27480
   aataaatata aaatgagtga gattgttaaa tcattataga gacaatttca ttttcacaaa 27540
   aataaataaa tacataactt tttataattg gggtttgcag gagaataagc catcggacac 27600
   accaccagaa ccatggccat gttgaaaacg acgagtcttg ggttccggta atctttccta 27660
   ctctcgtagt ttctcttgtc ttttatttat ttgtttgttt ttcggaattt attcttatgt 27720
   ctatgttctt aggattctat atgtttattt tattagttta tgttttcagt ctgaggtcag 27780
   accgaccact tgtcagatct gttttctagc tgtagtaaaa aacaatttgc aagtgtaata 27840
   gttcagcata attgatcttg ttagagcatt tccaaaacaa actttataat tttaaatata 27900
   cagttttttg ttctctaaaa aagaatttaa aaattttaaa gtttgaggga cgaaacttca 27960
   aatttgaact ttcactactc aacttcaaat ttgaaatttc atctttttta tttacatttt 28020
   gatcattata attaattata cattacattt atgattctta agtattttct catttattgt 28080
   tttaattctt aaatttttta tacatcataa atatttccaa tttgttttta taaattcaaa 28140
   ttttacacaa aaaagtaata aaaattttaa ataagattta taatatttta aaactataat 28200
   taggcaaaaa aaatattaca aaaaaatgta ataaaaactt taaaataaga tatatcaaga 28260
   cataattatt agaaatttta aatattataa caatattaat aatctggtaa atttgctcca 28320
   aaacctcaaa aatttctaaa ttattgtcca aacaaatttg tttaaccgaa tatggagcat 28380
   tacaaaaata attttatgga atagtgtggt attttgcttg tagttaatat ttaattatgt 28440
   atttctattt ataattttat atatttaatg taagattttt ttaattaata ttactgtaat 28500
   atttttatat atgtactagt tatttataaa agttttatag atttgtatta gttataacaa 28560
   aaataaggat cattgtgtaa aatacaaata attttgaaat tacgtttaaa gttttggtta 28620
   tgaaaaaaat actttgaaac tttaaattta gagttttgca aactttaaaa tgttagatag 28680
   atagtttttt tggagatgca tttagtggtt atggtagtaa ctcagaaaat gaaaaatcta 28740
   tacttttata ctccctccgt tttttaatat aagtcgtttt acagttatac acgtagatta 28800
   agaaaaccat taatttctta tattttctag acaaaaacat cattaattat ttacctaacc 28860
   acaattcaac caatataaaa atagaagata tattaccatt ggtcatacaa cattaattat 28920
   taataaattt tacatagaaa accgaaaacg acatataatt tggaacaaaa aaatttctct 28980
   aaaacgactt atattaaaaa acggagggag tagtacctaa ctttaacgat ggaccactta 29040
   tattcgagtc cttagcataa aatgattctc ctcgaaatcc gtttactttc ttcattattt 29100
   tttccttttc agttttggcg ttttcgtaat acttttgtct tcaatcttga aagctattag 29160
   tataaaaact tataaacaca tcacatgcaa tgaattaata cgaatacata accagaatga 29220
   caaattttca atgaatattt aataccagta agtactactc cgtaatagta atagtaatag 29280
   tcatattaat ttttttttgt catcaaacaa acagtaatag taatattaat tataattatg 29340
   tatttcagtt gccagaaaag ttgtacaaga acttgcccca tagtactcgg atgctcagat 29400
   acactgttcc tctgcccatg ctcgcttacc cgatctatct ggtaaaaaaa aatacaattt 29460
   caattttttt cttaaaatta caaatggttt tatattttga gttttaagcc aatatataaa 29520
   ttaattttga ttggatttta actacagtgg tacagaagtc ctggaaaaga agggtcacat 29580
   tttaacccat acagtagttt atttgctcca agcgagagga agcttattgc aacttcaaca 29640
   acttgctggt ccataatgtt ggccactctt gtttatctat cgttcctcgt tggtccagtc 29700
   acagttctca aagtctatgg tgttccttac attgtaagtt tcacatatta ttacaagaga 29760
   tttatatatt attaataata aatttgtttt ttgacataaa gttttggaaa attttcagat 29820
   ctttgtaatg tggttggacg ctgtcacgta cttgcatcat catggtcacg atgagaagtt 29880
   gccttggtac agaggcaagg taaataaatc aatttttaaa aagaaatgta cagaaagcaa 29940
   taatggttag tattgattaa tcttaatttt tgatgttttg catacaataa taggaatgga 30000
   gttatttacg tggaggatta acaactattg atagagatta cggaatcttc aacaacatcc 30060
   atcacgacat tggaactcac gtgatccatc atcttttccc acaaatccct cactatcact 30120
   tggtcgatgc ggtgagtgat ctagctttct ctctctctag tttcatttga ttaaatggtg 30180
   attaattact aatttaatta atgaattgtg gacagacgag agcagctaaa catgtgttag 30240
   gaagatacta cagagagccg aagacgtcag gagcaatacc gattcacttg gtggagagtt 30300
   tggtcgcaag tattaaaaaa gatcattacg tcagtgacac tggtgatatt gtcttctacg 30360
   agacagatcc agatctctac gtttatgctt cggacaaatc taaaatcaat taacttttct 30420
   tcctagctct attaggaata aacactcctt ctcttttact tatttgtttc tgctttaagt 30480
   ttaaaatgta ctcgtgaaac cttttttatt aatgtattta cgttacaaaa agtggaagtt 30540
   ttgttatctt tttctctagt tgcaatcaaa aggatcttta aaactttttt gatttggaca 30600
   gaaagaaaaa gacagttcca ctgaaagtcg acaaaatgca cgccgttttt gggtcccagc 30660
   acaacaacaa tatgtcacgg agttgtcgct tttttaagta atgggcaata cttttcggcc 30720
   caaatatata aaagccttct taaattgcgt caggtatctc acgcaggacc taaataatta 30780
   tacaaacatc tcattcgtcc ccatatatta aagagttgat tacctagtag gccacttttt 30840
   gagttttctt tgcacccaaa gctactttcc gcttgtagca taaacattca cggaaactga 30900
   aagagttttt ggattatttt gcccttactg aaacgaaacg gaaaattgga atattgtttg 30960
   tgttgttttt gttcggttag cttttagaca tttattagat taggtttctc gatagttaga 31020
   tttttataag gaccacaaga tcgtaaaaaa aatgttaatc caacaatcac gttaaaatga 31080
   ccagtttagc aagttacagt catccatatt tcatggatgt ggatgctatc atgtccacaa 31140
   atacatgttc ggtggttatg gatgctttcg tgtccatgta aggatgttat ggttactcag 31200
   atttgtggat ggagaaagtt ggataaacat tacttggata gataaacatt atgtggacgg 31260
   acgaacatta tggatacaaa aatagtggac atgtaagttg tgggcagaca aatgttacaa 31320
   gaatgagtta tagacgagaa cacaacatgt aagggaacaa aagttattta acttaacttt 31380
   gtggacaaga ttttatattc tacaattagg cggtaaatta acaaattttg tcggaactgt 31440
   ttatcggaaa gtgatctgat gattccgaag cacttctgag aaatattggc gatgatcata 31500
   taaaaatcaa tattttaaaa taaaaaataa attttggata agaagtataa aacatattgt 31560
   agacaagttt ctgcaagaaa atgtgtgaaa atggcctgcg aaaactaaaa tcaatataaa 31620
   aaaaagactt attcttaggt ccactcccta gggtgaacct ctaccaatag gattgtttta 31680
   ttttatattc aatatctttt aaaaaaagaa acaaaatatt atcaaattat attatgtttt 31740
   taaaattaaa aggtaaaaaa atagtaataa ttacaaaaaa aatattttac gtcgtgagca 31800
   taacattaaa ccctaaaacc taaattctaa tccctaaacc cttaatccta aaccctaaac 31860
   cattggataa accctaaact ctaggataaa tcctaaactc taaatcaaaa tcactataca 31920
   ctaaaacatt caagcgttta ggatttaggg ttttagtatt tttttattta gagtttagga 31980
   tttatccaag ggtttagagt ttacccaagg gtttagggtt tacccaaggg tttagggttt 32040
   acccaaaggt ttagggttta tccaagggtt tagggtttag ggattaggat ttaggattta 32100
   gagttttgtt gagaacatta aaaatatatt atttttttta attctttttt ctgtaactat 32160
   tatctttttt tactttttta ttttaaaaac ataatataat ttgagaatat tttgtttcta 32220
   tttttaaaag atatcaaatt tgaaataata aaatcctatt ggttggtgaa tcaccctagg 32280
   ggtgaaccca agaatgactc aaaaaaaaac tataaagttt cttctgaatg agcttgcatg 32340
   tttttttctc tacgatcagt gatgttaaag ttcttccttg taaagagata atctctccag 32400
   caatttgctt tggctccttc ttgacgcctt atccttcgct gacaacaaag gtcttcctca 32460
   ctatctgaaa aaaaaatcta aacattggtt gagagagttt gatggtgaag ttagagaaag 32520
   aggccaaagt taaaaccttt gatttaatgg ggcgttggat aagagaccac agatctggaa 32580
   ctgaaaaatg aacaaaaccc aatgatgtta gtagctagcc aacgagtaac caccacaagt 32640
   tgctggctct tcaccattat cagcaatgaa ctagggtttt gttcccacca ttggtgaatc 32700
   tgaatcgcag cattgagggg ctccacaacc atggcggtga catggaagaa tttgttacgt 32760
   ttcgtcacac agtctcgtct ccaccctttg attactacat ctcttgaaat catccattgg 32820
   acaagacaac gacagagaaa acagcttcgt tgccacaact gtcatcaagg ttgtgtggac 32880
   aaataaaaat ggagataaca acctttgagc tcatctactc tctgaaactc cagccaacaa 32940
   atcccgaact caaccacatc cgatctcgag ctcacccacg gcgagttcca agctcatcca 33000
   ttctctgaag taaagcgaat ttgggattaa gagagaagaa gagaataaaa gaagcgttga 33060
   ttaggtttta tcaatttggg aatttggtaa ttagagttcc aaaagagatt gtcggtatct 33120
   tccactcctc taaggcaggc cgcaaacgag aaattaggag acttttcgag agagatgggt 33180
   ttcgtggctg agagaaaaga tgaaataagg gattagggtt tgaaagttga ttttgaaaaa 33240
   gtgaagtgaa cagatagaaa aaagatgggc tccattaatt ttgaaaacct aaagtgaaaa 33300
   tagagaagaa agacaggccc cgtgtaactc tagtttggtt gctggaagtt ggttctttct 33360
   ttagttagag ggcactaaga ccatgtttat ccctaaaaca cttagtgggt tttctaattt 33420
   ttattttatt ttattttgtc tgatttaaaa aaaaaaatta aaaagtatac taatcgcggg 33480
   ccgtcacgtg ttggtggggt ccgcgcacag tgctaaaaac ccacaacaat ctctattatt 33540
   aaaagagaag tacccataaa aaataaccct aaaagttaca caatatttac agtcaaatgc 33600
   cattgagaat taaattaatc ttacactaaa aatgattgtc ttttccacat attaattgtt 33660
   tttctaaaat aactcaaaca aactacaaaa gaaagaaaca tattattaat aactcaaaca 33720
   attacatatt attaaataaa ggaataagca taaataattc tcctgcaata tcaacactgt 33780
   aacattcctt attatatgag tcccatcctt tttttttgtc atcatatgag tcccatcctt 33840
   agcttacgta acctgtacga acatcaaatt atataagctt tataagaaat taaactaaga 33900
   aaaactaaca atgattttca tatgagtttg aacaatttca attcacttta tttcacggtg 33960
   gtgtatgtag cttattttta accaccttat tatattgaaa tattccactg acttctatat 34020
   gtccaaataa ttaataatca ttattattaa ttaaaatcta ataattagga aaataactgt 34080
   agttttgaga acactggcga cggcgaatgc gaatttttag ggttttgaga tgtttcatgg 34140
   atgggatccg gggtctagtg gatttttctg gaattgcaag gagctctatc ttaatgagca 34200
   aatcgggaga ttatggaagc aaaatggtct tcttgtcata aggagaaagg gagatctggg 34260
   aattttattt ggctttgatc ttgtactgtc aaagatcgga atcgcggaga ttcgattgag 34320
   aagggagaaa agtaaggcat tcgttaacat caaagcgaga tcttttctac taatattggg 34380
   gatttctttt tgctttttgg tgcttagata tctggtaact ggttatagga aattcgggat 34440
   ttggggtttg atgcgatttg atattcagga aagtcggaat ctaaggatta atggggttag 34500
   agggagtatc aatagagagg atctcctgat tgattttctt tacgatttgt gttattatat 34560
   aaaggagggt gttcagagct tcagtagcac aaacttacaa atctccttct ttctcttacg 34620
   gatttcgttt ctggttgttt tttctttggt atctatgagt cagggacaat tggtgggaaa 34680
   ggggggagcc tcgaaggagg gagaaggagt tcgcaaaaga ttgaagatct ccgttcctca 34740
   tttcgataac tcggacctta tcaagagcta tgcaatgact ctgattggga ggtgtatgaa 34800
   cccggttgcg caaaaagtca actcgttgct ggtgatgttg ccgaagatat ggaaggtgga 34860
   agagagggtg actggtgcag atttgggaaa ggggatgttc cagtttcatt ttgagaagga 34920
   agaagacatt gaagcggttt tggagtcaca gccgtaccat tttgattatt ggatgatctc 34980
   gatagctcgg tggcaaccaa ggatgacaag gagctttcct tcggagatcc ctttttggat 35040
   caaagtggaa ggtcttccaa cagagttttg gtcaactcca gcgcttcaaa gcataggcga 35100
   tgccattgga gagactacgg atgtggatct ggactatgga aagatgcgag tggtgcttga 35160
   tggcttcaaa gagttaacac tggaaacatc cgtggagttc aaaggaggtg aattctatga 35220
   tgaggaagag gtcccggtat ctcttaaata cgataaattg tttggcattt gcaagctctg 35280
   ttctagtcta tgccatgacg aggatcattg tcctcttaat cctaaaagtg tggacaagaa 35340
   aacagatagc agagaggagc tggctaataa gaaagaggac agggcaagga gctacaaagg 35400
   agtggtgatt catggagagg agagtcaaca ggagaggggc acagatcaac ggaattatta 35460
   tggtaagggg aaagggaaaa tgcatgagga ccaggactca aagtgggtac gagttcctga 35520
   aagaggaaac aagaggtact cgtcttacca cgataacaac agaaacgatg agggaaataa 35580
   cagacacaag aacactcgtt gggaacagcc taggagttac gtgcaggaat cgcgggagaa 35640
   ggggcatcgt ggcacaagac gggagaggag tcctccgcat tatgcacgag aggagccaaa 35700
   ggaggaaggg gagctgcaag acacaggcag tgctaacaaa ggatctcaaa tggaaggaaa 35760
   gacttctgca tctaacaacc tgcagattga atcgaatggg gccagggcaa atttgattaa 35820
   gcttcctcct aaatccgtgg aaatggagaa tggtgcaata gctgcgatag tttcaggaac 35880
   ggttggggcg gggaaaggaa cggagccacc attgggtgac aatggaaagg atatggaaga 35940
   gaatgaagta atggacctag ctgagaatgt gattccatct gcaggggaca aaggttgcat 36000
   gggtgaggat gaagctttcg aaaatcttac tgatggagag atggaggaac tgaatggatc 36060
   acaagaagtg gtgctggaga ccgttgagga agaatcacga ccaacggatg tcgaggagaa 36120
   ggaactacaa gttggagagg aggaaaaaaa gaagggcgct cgcaagatac taaagcacac 36180
   aatggcggca ggagcttcaa agaagaagtt cgttcaggca ctcctttcac agaacaaaaa 36240
   tactcaagct agacagggaa agcgtcaggg agacggaagc aaattgcagg aggataaggg 36300
   ttcttcatac cccaaacaaa cttcctcaaa gaactcaact gcatcccatg gttaatacaa 36360
   ttcatataga attgaggagt ggacttctgg ttgcgtcggt ttctgcttac tgttggttta 36420
   tttcaataag ctctaggagc tttcttctac ggttcaattt gatttttgca ttgctggttt 36480
   tatgtttcag tggcaattgc ctatctttta ttgtatttgg ttatggtttt ggtattaaga 36540
   ataataattg tctttctggt ttctatgatc ttgatatggt taaatattgg tatggtgtta 36600
   agacccttta ttcaggtcag atggcgttag tggcttggct gtgtgtaggg atgcatttgg 36660
   ctcacttcat acaatggatg ttggacctga tctctgtaaa acaagttcag tggaagaatg 36720
   atacaaggag ggttttggac aaggtacttg ggtctggttt cattatttgg tataaggtgg 36780
   cacttaatta ctcatttctg gatagtacta agtgcacggt agtttggaga tgtttgttgc 36840
   ttggtctttc taaggaacct agatgctctc gagtggctat gtatttgaac acattatatt 36900
   gttatggtta tgggtttaat ttcagggata gagatctgag atggtcctta attatgggtg 36960
   gagggggaag aggtagagtc atggacacgt cttggatcat tgcaggggag cacactcttg 37020
   gcttggtctc attaaaccaa gtgcagagaa cctttggatc catgatcggt atcaagctgg 37080
   ctttctcagt gccgctgcaa gatggaagtg gatacgacaa atatacggtg tctaactctt 37140
   ggcgtttatt taggacatgt tcaaaatatg caaggttatt gtcctttgga gtcataatat 37200
   gggatataat atggtgggtg aagttttggt ttcctttggt tactggttta tatgtacagg 37260
   tcgaaatgat aatgtgtttt ggtatcctga gtgtacaata tggaactgat gagtattgga 37320
   taattgatct agttcgtaaa aaaattatat cttctcctac aatcttatat atcgttacat 37380
   acattgttaa ttgggcctta tggtttatta ttagaagtgg tgaatgtgat cgtatagtta 37440
   ctggtgggct ggagagttgg ataaattata agatcacatg gccttttttg gtctttcgtt 37500
   tttgtcacca aaatttgagt ttcttaatca agtggataat tttatgggtc ttgggatgcg 37560
   aattgtgttt attggttaca gttggtatgg gaatggttat aggatcatgg gtatgtgatg 37620
   gtgatcaaga gtctttatat ataattgtcc ttacaagcga ttgtgaagta tctgaagttt 37680
   tttcagatag gatgattagg cttattgagg ttaaatcttt tgtcggtatc atcaaaccta 37740
   tcttttctgg ttcgaacgat aaatatatat atatatatga agatattaag ctggaattgt 37800
   agaggtcttg gaagtcactg gacaataagt tatcttcggg agatatggca ccaacacaaa 37860
   ccggagtttt tatttttgtc tgaaacgaaa caggatttcg atttcgtaca aagatttcag 37920
   tctcattttg gctatgatag cctggttact gtggatccaa atgggcggag tggtggttta 37980
   gctctttttt ataataatga gtatcaagtt agagtcatat attctagcaa tagaatgata 38040
   gacgtggagg cggtggttaa aggaaaacaa gtttttctta cttttgtata cggggatccg 38100
   gtaccaaagc taagagaaca ggtatgggag agattaactc gatatggatt agcaagatcc 38160
   gaaccttggt ttattattgg tgatttaaac gagattactg ggaatcatga aaaggatggg 38220
   ggatccctaa gatgtgcaac atcttttatt ccgtttaaca atatgatacg gaacagtggg 38280
   ttactggaat tcccggctcg tggaaataaa ttttcatggc aaggaaggcg tggcaaagga 38340
   aaggatgctg tgacggtcag atgtcgattg gatcgagcct tggcaaatga agaatggcat 38400
   acgttgttcc cgtgctccta cacagaatat ttgaggttag tgggatctga ccaccgtcct 38460
   gtaatcgctt ttttggagga caagttattg aggaaaagga gaggacaatt cagatttgat 38520
   aagagatgga taggtcagga ggggcttatg gaatcaatag tgacaggatg gacggagaat 38580
   cagggtgggc aaattgagga ttttgttaca aaaattagta attgtcggca tgagatttct 38640
   tcatggcgaa aggataatca gccatatggg aaggataaaa ttagggagct tcaacatgca 38700
   ctcgaggaag ttcagacaga taatagcaga tcccaggaag agattctgga agtttccagg 38760
   aagctacaag aggcttataa ggatgaagag gaatattggc atcagaaaag ccggaatatg 38820
   tggtattcat ctggagatct taataccaag ttttaccatg ctctaacaaa gcagcgaagg 38880
   gtccgcaata aaatagtggg tctccacgat gaaaggggta attggattac tgaggacaat 38940
   ggaatcgaga aggtggccgt tgattatttt gaagacctgt ttagtacgac cactccaaca 39000
   gaatttgatg gttttttgga tgagatcgtt ccgtctattt ctccccaaat gaatcaagtt 39060
   ttgttgagaa tagcaacaga ggaagaggtc cgacaagctt tatttatgat gcatccggag 39120
   aaagcgccag gtccggatgg aatgacagcc ctctttttcc agcattcctg gcatgttatt 39180
   aagaaggatg tggtagaaat ggtgaacaat tttttggtta caggtgctat ggattcaagg 39240
   ctaaatacta ctaatatttg tatgattcct aagacagaga gacctacaag aatgacggaa 39300
   ctgaggccga taagtctttg taatgtgggt tacaagatta tctcgaaagt tttgtgtcaa 39360
   cgcctgaaaa tttgtctccc tctcttaata tcagagacac agtcagcttt tgtggaaggc 39420
   aggttaatat cggataatat tctcatagcg caggaaatgt ttcatggatt gagaaccaat 39480
   aagtcatgtc aaaataagtt tatggcgatt aaaacggaca tgagcaaggc ttatgatagg 39540
   atagaatgga gttttattga ggctcttcta tataaaatgg ggtttgatgc acattggatt 39600
   aagctaatgg tggaatgtat atcctcggtt caatatagag tacttcttaa tggtcagccg 39660
   cgaggcctta taattcccca gcgagggtta cgtcaggggg atcctttgtc tccttatcta 39720
   tttattatgt gtactgaggc tttaattagg aacatcaaga aggcggagag agacaaacgg 39780
   ttaaccggta tgaaggtagc aagagcttgt ccagcagtct ctcacttact attcgctgat 39840
   gatagccttt tcttttgtaa ggcaaataag gaagagtgtc aaactattct caggatttta 39900
   aaggaatacg aagcggtttc agggcaacaa attaattttc agaaatcctc aattcaattt 39960
   ggccacaaga ttatagaatc cagtcggcaa gaaatgagag atattttggg tattcaaaac 40020
   ttaggaggaa tgggatctta tttagggttg cccgaaagtt tgggaggatc taaggtacaa 40080
   gtgtttggtt ttgttcaaga acgcttgaat aatagggtta atggatggac ttttcgattt 40140
   tttactaaag gaggaaaaga ggtgattatt aaatcagtgg tcacggcttt accaaatcat 40200
   gtgatgtctg tttatcggct accaaaagca acagtaaaga agttaacaag tgcagtagct 40260
   cagttttggt ggagcccagg aggaagcaca aaaggcatgc attggaaatc atgggataaa 40320
   gtgtgtgtcc ctaaagacaa tggtggccta ggattcaagg atctcatgga ttttaacaca 40380
   gcgatgcttg gtaagcaaat gtggaggcta atagacaagc cacattctct cttctctaga 40440
   gtttttaaag gacggtatta caggaatgct tcacctcttg aaccgatccg ttcttactca 40500
   ccgtcatatg gctggcggag tatcatatct gctagatctc tggtttgtaa aggactaatt 40560
   aaaagggtgg gaacaggttc atctatttcg gtatggaatg atccttggat cccagccact 40620
   cgcccgagac cagcaaacaa aaaccttcaa aatagttacc cggaccttac agtggattct 40680
   ctcattaata tggaatttcg aacttggaac cttcaggcaa ttagggctgt ggtggatcct 40740
   catgatgtaa aaatcattga gagtatgcca ttaagcagaa atctgatgga agatagaaat 40800
   ggatggcatt ttactaacaa tggaaaatat tcggtaaaat caggatatca ggtggaacgg 40860
   gtttatcctg atagagaaaa accaccagag gtttatgggc ctacagtgga tgtccttaaa 40920
   gccttctgtt ggaaaatacg gtgtccgccc aagatacaac attttctatg gcaacttctt 40980
   tcaggttgta tagcggtgtt gaaaaatcta aaggcgagag gaatccatgg ggatatatgt 41040
   tgtgctcgat gtggggatcc ggaagaatca ataaaccatg tatttttcga atgtccccca 41100
   gtacgtcaag tatgggcttt atctaaaatc ccttcgagcc tcagtttatt ccctacagga 41160
   tctttttttg gtaatatgga tcatcttttt tggcgagtta atccaaaaat ggatgatcat 41220
   caatttgctt ggattttatg gtatatatgg aaaggtagga ataataaagt tttcagtaac 41280
   ctggatgtcg atccaaggga aacccttaga ctagcagaat tggaatctac actttgggct 41340
   gcggcacagg tgaacaacga ccaaaaacgg gaattacagg tacataccag acccatattg 41400
   gtaacttcag gacgctggtg ttttatagat ggatcatgga aagataagga tctattttca 41460
   ggacagggct ggtatagtat cctaccgggt ttcgatggct tattaggggc acggaatgta 41520
   agggcatgtc tttcaccact acattcagag gtggaggcgc tgatctgggc aatggaatgt 41580
   atgaggaatt taagacagct tcatgttacg tttgcaacgg attgttctca actggtgaag 41640
   atggtttcgg aaccagaaga atggccagca tttgaaagtt acctgaaaga tatcaaagtc 41700
   ctacaaggaa gcttcaacaa ctcagagatt gttcatgtac ctcggacgga gaataaaagg 41760
   gcggatagct tagcacgtag tgttaggaaa caatcgtctt tcgtcgttca catggatgca 41820
   gagttaccga tttggtttac agagtcaagt tgagtctgtg aatgtcttgt tgtcaaaaaa 41880
   aaaaataatt aaaatctaat atttttgaat tgaaaatctt ttccctcccc caacaatctt 41940
   ctacttagat ttcggaaaaa aaaatagaaa catttgcgga atctactaat ttgtttctaa 42000
   acaagatttc cccttcaatt tcggaacaaa gaagatatat ataaaatttg atccataact 42060
   actaaacaat aaacacaata ttcgaatttc accaatataa tcttactctc tcctattttg 42120
   ttagtttcac aataacacac aataaacaaa gtattctaaa tattaatgca aacaagagat 42180
   gccttgcgag ggtggttaag atatttcctc aactttaggg ttttgtattg cgttaaaaaa 42240
   attgacccac acacttgcgg aacaagcaca agatcttatc atttcctatt tcaaatcata 42300
   accattaaga ttttaccata atttcaaaaa caataaacag aatcaacaaa atattctttt 42360
   catttatttc gcctaatatg tcttgcaaaa taagcaaaga tatttattct caactagggt 42420
   attgtccctc tactatatat tctacccgag tacaaaccca ttctacacat tcttttacca 42480
   cttacgctga tgaaacatta caaatggttt tagctgatga aactgttagt tctataatat 42540
   ttgtattttt tttttgaatt ttataaagta gactttgaac aaaatcatct cttcctattt 42600
   ttgaatgttt ttttgtaact tagtttcatt attatttttg gtttgtctaa ataatgtatt 42660
   tgttttcaaa aatttcaata aaatatttga actttatatt caactttaaa ataaaatatt 42720
   tataatttaa tttaataaaa ccccaaatat acttaaacct ccgatacttt actatttaat 42780
   ttaccaaata aactaaataa aaatacaata aaagaaaaac acaatctcat agtttaaaaa 42840
   tgatggctaa tcatattgaa caagacacac cgaaatcaaa cctgaaaaac atatgaatct 42900
   ataacataat aagtacaaac aattaaattt atcaaatttt caaaagttaa aaatatatga 42960
   ttatgaaaaa caaaatcatc cttttttgaa caagaagaaa gcccccacgt tctgtcttgg 43020
   atggtattac caatatttca cattctttat ctaatggaaa cgaagaaaca acaacaaaca 43080
   tacatcgtga tatcaatcaa gaggataatg attttgttag aggatgatga ttttattcat 43140
   agcctttgaa aaaattaatt tccgtaaaag ttatacctta tttatctatt tcatatatca 43200
   tactaactca taatttttta tttcatcata ttttaatggt tttcaataga aatgtggtcc 43260
   aaattatatt accttatcac agtatgatca attttgttgc caccgtgtga tcaaattatg 43320
   ttacagcaat atttgtatta tgtgatgtat ttttgtcatt atttgtatta aaattttgat 43380
   atattatata atggtgtaaa aaaatttaat tacattaagt aaacagaaaa aaaacacccg 43440
   cccggtcggg cgggaccaga tctagttggt tattatttca tcaactttgt taccggtttt 43500
   tgcataaaac atgggaccca acactgtaag aaaccctata attacctccg ataaacatgc 43560
   cctaagagca tctgcaatag tgagtctcac catgaaattc ttagcattat tataatatac 43620
   tagagatttt ttccgcgctt cgcgcggatt gtatcttata aatttatttt atttataata 43680
   ttatttgttg gtttttttat attaactttt tgtttttccg atgttagttt tttttaattt 43740
   aaatttatat gtttatattt ttatattttt cttgttgtag atggagaatt atatttttta 43800
   ttgatggttt tttgtatgtg acataaactt tttgaaattt taaaataatg ttatatatag 43860
   tacgattaac acattaaaga agagaaacat attcagacac attttacaca ggttttatat 43920
   gcataatttt aaacattata tatgtatata ttataagttt gaaacatgta aatgctttct 43980
   aaagctaaat acttgttctg agtttacata acttatcgag agttttatct ctttttaaat 44040
   ttaaatcaca gaaaaaaaaa tatcaaaaag tcagtataaa tggatttttt gggcttttaa 44100
   atcaacactg aaaaattaca tgaattagat aacaacactt ttataaacaa ctcgataaaa 44160
   tttgaccgag ctaaagattt tcacacaata tgttctttct tcttcaaatt gcgaagagcc 44220
   tataggcaca aggaaaaaaa ttataatttt tgctttcact tatataacat tttttctcct 44280
   ttacacacga agtttattac actgctataa gcaatggaaa actctattca tataagattc 44340
   acatctatgc attttgacaa agaagaattt aagccatctt tagtttcgga atggacaaac 44400
   ttcagtcata tacactatat tttctctatg attcaaatct tacaatttta atatatgtgc 44460
   agatttccat gtaaaaaagc acgcacgcca tctatcattc aacctattac tttttccaaa 44520
   gtaaacacta taatcctcgt ttgattagct ccacaaacta atctctttgg atcagtttac 44580
   taaaaaatat ggatactaat gttagaaaag aatataaaca ctatcaacaa taaatattgg 44640
   cacaagacta tttggttcaa ggaacatatt caacgtaatg cgtttatatc atggctggtt 44700
   ttgcggagaa gactgccaac caaggatcgc ttgaggcgtt gggggttaaa tgtctccgga 44760
   acgtgcgtcc tttgtaatct ggaaatagag actcaccatc atctcttctt tgagtgctct 44820
   ttctctcgct tgatatggga gccttttgct actgaaattt ggatttttcc tccggctgat 44880
   ctacactctg ttgcagcctg gatcaatcaa cctcgcgtca acgcagatgc gcatgctact 44940
   tcagtcatca atctctactt tcagtccgcc atctacctgc tgtggaaaga gcgtaatgct 45000
   cgtatgttca cagctgtctc ctcaccttca tcagtcatcc ttgcctcttt cgaccgtatg 45060
   atgcgtgacc gtctcttctc ttacccggca aattcttctt tctcctattc tctacttctt 45120
   tttatctttc ttgtataaga cctccttaag gctttttcta ccttgagttg ttgttggttg 45180
   tttttgtttc cttgctgtaa caagttgttt aaaaacaaca gtgtaacttt tcagaaaatg 45240
   ataatcttaa catcttacca aaaacaacaa caaatattga cttatttatg tgaatatata 45300
   ttttatttta aatcattata gtggacgaag aaaacaccat aatttgtaca acaaattttc 45360
   ttagattcac ctcatcatac tcaccatttt actattttat ttacataatt ttacatgagc 45420
   ttcttcaccc tccccggtta ttttatcttt atttataact acgatataaa gttataaact 45480
   atattataga ttaataattt atttatcctt gaagtctaac gattaaaaat agaacataat 45540
   ttaatataga tatatgattc tattaataaa ttagtagtta caaatttgaa atttctagaa 45600
   atatcaaaag tcgtatgtta gttaattatc ttcttagtga catttatttt taattttttt 45660
   tggatgaaaa tattttggct gaggtagata ctctcaaaaa ccttgaattt agtccctttt 45720
   atatagtagg atatattttt ttaaatagtt aaagatccta atccaaaagg tacgtacaat 45780
   ggtgttatct aatttagagt cttcaggtct gaagctataa aacatatttc agaaaatggt 45840
   tttgttctaa agaacttggc gatctattaa atttttaatc agagtttgat ctaaaaaact 45900
   tgtattatat tctatcttgt attatattct atcttcttct gtttccatat agtcttagag 45960
   tcagaatagg atgtacaagt tacaaacata tatgcttatt aactaacaaa ttaattttat 46020
   gtgttttggt agtaaccact catcttcttg aagaaccaat gaaggagaat gatagtaagc 46080
   agaaaaacca tgaagatgca gcaagattgt ccacttccac gtcttcttcc ttcacccgtt 46140
   gtagtgtcct ttatttcact acactcctcc tccgtcaacc ttactggaac attcagtgct 46200
   gaagaaccgc agatttcaca tacactacaa aaaagagaaa cagaggtttt acacaatcca 46260
   tatggttact aagctaatga actgaataga gtacctgttt cctcttagct taaaccaagc 46320
   ttcagcgcaa tgaaaatgag caaggccaag ctcatttttg catttgcaac caatctaaat 46380
   caagtctaca cttacaaact tgccagaaac tctatctggc gtttgatcag aaccaaaatg 46440
   acaaatcctg cagattcttt gtccattatc actttcttct cctccaccac tcagatcaat 46500
   catgtgaaac tccttctcct ttgctttttc tgaagcatca ctctcgtgac accatctttc 46560
   cccattcttg aatcattctc tttttgatct tccatcaata catggtttga gagagagagt 46620
   cactggtctc acccttgagg ccagagacta caatcacagc ctcagggaca gatccactcg 46680
   aactttcaca aacagtgatc gaatttgaac aaggtatctg gtccatttca ttcatataca 46740
   caccaaaaac aaaaccagga gtggttgtat caagatcaag aatgattgta tcagacaaag 46800
   agttaaacat aaacccaaaa ctgaaaacct gtaacagcta gaacatactc aaattattgg 46860
   tacgcagagt cctaaagtac aataaagatc gaaactttac cagaatcaag atctagtaga 46920
   gtgacaaggt ttcgttttta tttcagaaga atgataaatc agacaattga atctaaaccc 46980
   tttgccggaa acggatgcgc gccgctacaa gtgctctcta atctgttgct cttcggtttc 47040
   agtttgtgtt ttttctttca taagatgcct cagctagatt ttaggccaga ctcgagaatc 47100
   aatttttttc tctgcatcgg tcgagactcg agtatgacga cttttttttc cccactagga 47160
   aacacaaaaa ccttcccatc cattcacaag tagccacgta ccataaggat caagtcctaa 47220
   aattccttag ttatatatgt tccagtcctt agttttatta agcaaaatat tattattata 47280
   tgtgtattta cctaagatta agccctaagg attggtgatg ttactccgtt gcgggtggtc 47340
   taagaatatg attattgaga gtttttatgg tggattttta gcggaatata agaactccac 47400
   tctaaaaatt tctgctctaa gagcatgatt atccctaaat acacattaga ttagttaatg 47460
   actatttaag tattaaattt tagtgaagga atttagttaa gataggattg gagaaagaaa 47520
   aaacacatta aaagagagga aggattcaag aatgaagaga agtgttaatg gaaggttctt 47580
   catcaatata cacttcagtt cttatcagta tacatatagt ttgtactata taaatcatac 47640
   aaaagagaag tattctcaac catttggtga tgtagttttt attaccatac aaaaacaatt 47700
   ctaatacaag cgtgtctcaa gaacacaaaa atcgtttcag tttttattat ctttcgagga 47760
   gcttgtactg agtgtcgttc aagtaaaacg actgagccgt ctccatgatc catttcgcct 47820
   cctcgtcagt gagtttgctt gtgaacaaaa catcacctcg gataaacacc aaggtgtgtt 47880
   acaagctgtc aaacatactt agatcattaa gcatgatata cacaaaacaa aacaaaaaac 47940
   attgaaaaga gaacaagaaa aacaaaaaac aaaaaacatt gaaaaaattg agaatgaaga 48000
   atatgacgac aatgatacaa aagtttgtat actgataata cactagcata caaaacgtga 48060
   gtgacgacaa tgacatttct tcactaggcc gatgatacaa aacgttactg ctcccacaga 48120
   agcatacaaa acgtctaacg acaaactatc atgaaacagg gagcaaggca tcgactcaaa 48180
   ttggccatca cctctttcaa atcgtctgtt tgtttagtaa ggagaaaata aagagtctag 48240
   acccaaattg gctatgtacc tcctataaaa cgttatttat tttgcaaaac aggaaacatg 48300
   gaacggtggt tatgcaaatg caaaacactt atatactgta taacagtaaa atttcaaagg 48360
   aatgacattg tgaaccattc actatagaaa attcaaattc ataatctcgt aatgctgtca 48420
   acatccatgt aaagctcagt gcgccatcta aacaaaattt cttcataatc cacatttcat 48480
   tagaaatata aaaagggtca agactcaact tcgaactatt aaaaaggaaa aattcatttc 48540
   gtgtagaaac gttgtaataa acaattttgg aatggactta gtgatatcat attagttgcg 48600
   tttttaataa aatccttaat tacttgttaa ttaattgaaa gagagtaaca gaatgggtct 48660
   tcatatacaa attaagcaca ccgaaaaatg cagaatccta atatgaaact gatactcata 48720
   tgataactaa taacgttaca caaaatatac agaaaaccgt aaaatgatag aaagaacaat 48780
   agcaactatg gtaaaaacca actaaaacca aaacatgtgg caatttggcc ctccattaaa 48840
   agctatatac cacagtttag ctcagctata agcttataat aatatacact agggccgggc 48900
   ccgcccttcg ggcgggaagt ttgaataaaa caatttcata tgatttatat ttatttatga 48960
   ataatttata attatgatat agatgatatc atatacaaac aacacaaatg agaactttta 49020
   agttataata tactggttat gagttcaatt ttagtatcat atattactat gagagtaatc 49080
   ttcgctatta tttcaaaagt ttagttttag ctatcctcca ttagactaac ttataaattg 49140
   atttaggtga gtacgaccca aacccccaaa gcatcctttt attattcgag gccttttgtt 49200
   tttttttcat gatgcatata tacacatgtg aattttgtac ggaagaataa tgtataaatt 49260
   ggagaaatct tattatttgt tattaagctt gatgcaaaag tttaatttaa ataatgtttc 49320
   aataaatttg gcgggtttgt ttacggtttc tttgtgcgta tgtagtcaat aaattaaaat 49380
   aataacaatc ttcgcatgcg ctgtccatat catgctggtg acattctgct tcgggctcca 49440
   tcctggctgt atttgctaaa taccttgtct tcaaaataac tttgatcgat ttaagtgaag 49500
   ttttaataat aagtatatta gcttgtggac gacagacgta cactcatgca cgtaaccaaa 49560
   gttttgtaat acataatatg attatggacg tcagtttatg cacacaattt aaagaacatt 49620
   aaatattttc acactcatat acataattat attagaccgt ggactgtata catacactca 49680
   tactaacgta cccaaagttt ttgtagtcca taatatgatt atggacgtcc acttacgtac 49740
   acaattaaaa taaacactaa ctcttttaat aaaataatca ctaacattta ttaactcatc 49800
   ggaatcaaat aaagcatcaa cttgttcctt ttttaaactt atgtcaactc aatataaaaa 49860
   gcattcataa caaccataaa gtagagagtt tgaaaaaaaa acaactggaa tgtagaaaat 49920
   ccataacata gatagaaaaa agatgacaat aaagtagaat gcagaaacat tattaagctg 49980
   cagaatatcg agagatgatt atcgaagatc catcttaagc aatacgcgcc ctcttacgca 50040
   cattaccgac tggctccttt ccagctctct ttgatgtctc acaattgccc ttgccggaac 50100
   cagactccac acggttgtta ggcccgtctg gcgcatcatc atcatcattg tcatcacctt 50160
   cctgttacat tcaaattgtt atacgctgca tatgctaatg gcgaaatatc ataataagta 50220
   ttgcacttac atcatcgaca aattctggag ctggtgcacg ctcaacctca ttgatgatac 50280
   gcgacacggt gaacgtctgg tggttgacgg tgaagttgta gggcgtgaca cggacttgga 50340
   aagtgtaggt cttgcgttcc attcctgcaa cgaacggagg catcacggaa tcctcagggt 50400
   tcactccttc ttcagccttg tcattaaatg gaagcatcga gagtatcaac aacattatat 50460
   aataaccaca taagtaatca aactatatat taccagtaac tggaccgcct cacttgcccg 50520
   gagattatgc aacttcgtca taacaccatc aaagcaaaca aatgtccctt cagcagtatc 50580
   atcagttaca accatctcaa cgcgataact aaaagagaac aaataaaccg aaatggaatc 50640
   agtgtgacgc aagggataaa gaaggcgacc aactatcata ttataacaaa tcgatacata 50700
   ccgtaaagat ccgaccgcat gagggttatt acagcgtgca cattcgaaag aagtgacagt 50760
   gcgttgcaat ttcttgctgc acttagaaca cgcaacatag caccaccctt tgtccgattc 50820
   aacccgagaa actctcgcag tgcataagaa atctatttcc tgctgcttaa acatcaacat 50880
   tgcgtgcagt caaaaaacaa tctttatata taaacacacg aatgagctaa ttgcatatac 50940
   ataactcatt acctgtggcg aatccgtagt gataaaatgg ttaagctctg caattgtcac 51000
   agtctcaacc ttcgcataag acttcaagag aggtgcggca gacggaagac cagtgtctct 51060
   agccaccaat ctgaacaaaa gttcacgaaa ttaacatcac aaactctcaa cgtctgatta 51120
   catactcaga acagaccaaa cttaccggta aaataaagac tctcctgcat gtgtctcctt 51180
   atcataataa acatgtgttc ctgacgttgc gttgaggaat aaacgaccta cacaaaatag 51240
   taaaaatttc tgaaaaagga attacacata ttaatttatg ccacatcttt aaataagcaa 51300
   cgaagtacct ccaaccatct tcgggtttat gcttgtggca acaatcactt taggatcatc 51360
   acgcatgcct ccaagcttct ggtggaataa aacggcttga gcgtcaaaca gactaagagt 51420
   gacagacaca tcactgcaca tatataacaa acgttagtgc agctaaaatg attaagcaaa 51480
   tatgaaagtt gttaaagaaa caaataaatt acttttctaa tttgacggtg accatgacac 51540
   ggttcttatc ctccggaggg tcagacacgg tgctcttcac cgccacgatt tcaccaataa 51600
   tatctacagg ttcaatattg agatcagaga gctcagtgac ttaatacata aagcacaaaa 51660
   cccatacaga taaatataca tatgtacgat acgatcacat ctgtacctgg aagctgagtg 51720
   tttgtattgg ctaaaccaac caactcggtc tggttacgga accggaatcc ctctgccggt 51780
   attggcgaga ccggatcaga taacacgtca aactcggtgg aatcgttaaa ccggatcatc 51840
   aaagaagagt ccacaagctt gaagttctga gcacagcgag ccacgtcaaa gccagaaaca 51900
   gagtacatcg tcccggcggc gagcctatct cggaaccttg gaagccgatt cgcgttgata 51960
   gtagcttgga tcaaagtcga ctgaaacaga taaaatttag aagtcagaaa ataacgtaaa 52020
   cagatctaag gagaccagga agaatcaaac ttacattcac gtccataagt agcatatcga 52080
   cccacatcag ctcgccaccg cgtttgacgt tcctcgcctc ccagaaccgt agaagccggg 52140
   cctcgacgac ggaggagcat ttgccggact tcaggtcaga gaagaagact ctcgaaatag 52200
   acatagcaac aggaatcaga aagtctcaag agaaagaaag agatgatgcg ctggagatct 52260
   atagatactt acatatttat acagatctgc tcgggttcaa atggcgcatc gaagagtctg 52320
   agggagggat tgaatgagcg agggattgaa tgcaattgga ataaagacga cgacataact 52380
   ccggccgttt catcgaagag gaaggaatcg aagacgtcac gcgtcgccgg cgcagaaaag 52440
   ggttacgcga gagtaatgtg tcttagggtt ggagacgtcg tgacatcgtt cgggctgtga 52500
   gtgtaaaggc ccatcacaga aagatcgagc gaggcccaga agataacatg ttcagtttaa 52560
   tgaaacgcag cacctcgtcg tcagtgacac gtgtcgacgc gagaggaagt gaacgtggat 52620
   ggcctaagaa gagattaaac tgtcttttat atatatacat ttagttgaca aagctcaaac 52680
   tcaaaccaag ccgatgacaa aaactctcag gagatctaca tataactatc atcacacact 52740
   atatatatat atatgataaa ataaaaaccg aaatgattag atcacttcaa ctctcgccgg 52800
   taactgtatt cccgccgttt cctcttcagc ggtagaatct tgagaggcga caagtttcac 52860
   agcgaaagaa aaattggaat tatacttttg tctcgcttca gcgaagcttg aagaaagaac 52920
   ggtttgcatc cactgatcaa ccgttttctc ttcatctggt gaccatctca aggcagctag 52980
   aatctgaagg atcgcatcgc tttcgatctc tagacgtgtt tcatgtccaa acgaccgacg 53040
   aaaatgcgac aagatgctcc tttgtatgtt cttcgctaac ccatcaaagt cgatcgtttt 53100
   gaacgtcact ttcccgtcaa cttgctcgaa aaaatcctca agccaagctt tactgttctc 53160
   ggacattgca tgcgcttctt catctgcgtt tgcttctgtc tcatccactg gaagattcag 53220
   atcgagaaac gaacgttgag actttacagc tcgaagctct gtaccctctg ttcctaactc 53280
   ctgtcttcgc ttattcagac cgttcttgtt agcattgtcg gcgagtttta tctggagttt 53340
   acatttttta ggggtgagaa ctcttccctc ggagtattcg acacgctcat caagaatatt 53400
   cgaagtagta gctaaaacta taacattctt cataccgatc tctcttccgt gcgagtcacg 53460
   gagcttacca gttctcacag catcagacag tcttacctga tcaggaaact cagctttgtc 53520
   cacgttctcg atgaaaacaa cagactccac acgcttggac acttctccgg cgatgtagtc 53580
   aacaactgtt ttccctctga atctatcgtc gagccggtcc tgtgccttga aatccacgca 53640
   aacgcagttt tctcgcccgc cgaagaaggc ttcagcgaga gttgttgcta ctttcttctt 53700
   cccgacttga tctggtccaa gaagagcgag ccagacatta cttgtggctg aagctagctg 53760
   gcttctatca tctctgtatc cgcagatgat ctcgctaacg gcgttcacag cttcgttctg 53820
   aaaccctact ttccgagaga gtaattctct gagagacttg aagtctttgc agtaccgtga 53880
   caatggtttc tctttgctca gctcaaagcc tctccggtta agtgataccg gtgtgcttga 53940
   ctcctggtag attgttccta gccctagatc tgttgtaaca cagctcaaag gcgagttcgt 54000
   tgttgtgcga gttgtgtgat cttctatatg ttttggcttc gagattctta cagataaacc 54060
   gggttggtgt ggcggattct cagcttgaag ggttgaaaca agctgaagtg ggaactgtgg 54120
   tctaacaggc tggaagctga gtttaggaaa cgccggagtt tgatggattc gttgacaaat 54180
   gtcgtcccat ttcttctgca cagaagctag tgtgtttgga tcatctttag cctgcactga 54240
   atgagaacta aacactctca agatagtaac tgagagattc gaattcagag aagaagttgg 54300
   ttcataaaag cagcacctgt ctgagaatcc ctttgtcttg ttcagattcc acattacgta 54360
   accaacaagg caacttctct gaacactgat caccggactt acctaaggct gtgacttctt 54420
   gctcacactt ctcgttacag agatgacacc gaggaagacg agactggttc attgagttac 54480
   taaacggtac tctgaaatct gatgttgatg agaagaagcc tccaaatgga acaaatgatc 54540
   ccatcaaact gtagatgaaa ccacaaaacg tcacgtttca atcaaaaata ttttagactt 54600
   tcccaacact aaataccagt gttataaaag ttgggctcag aaagcgccta tgcggcaaat 54660
   catgtataaa gctatttctc taaagcgatt tttttttaaa ttcagtccgt tcgttaaaaa 54720
   attggtctac acgcccgtct aaacattagt ttcttgtaaa atgcataatt atagcttaac 54780
   tattttaaac attgctaaaa acaaaaacat tcatcccgaa aattcggtta aaaaatcggt 54840
   gtagacaccc atctaaacat cagtttcttg taaaatgcat tattatagct taactacata 54900
   ttttaaacac tgctaaaagc aaaaacattt atccggaaaa ttcaaatcat ccgataaata 54960
   aaaaaatctt aattatccaa actttttact gaattgacta taattatata gaaatatatg 55020
   aatctaacca aacgaaatta aatcggagaa tttttcaaac acagtattag agatttctag 55080
   tttcgaaaaa aaacaaaata aaacgaaaat aaagccacta taaaagacat taccttgact 55140
   tgggataaac tccttgattc gaagatgtaa taggaagaag atgaatattc cagtctttgt 55200
   caatcgtggg gaacctctcg atcagtttca aatacatctc gttgctcgac acactcccga 55260
   cgaaccagag cttctcacaa tgaagcttca acagctccga gagcctcgac acgagagcat 55320
   cactggttaa gaccttgagc tctcctagat tcaaaaccgt ccccgtttta gatttcgagc 55380
   agctttgctc cacgattctc cccaactcat cgagtttcat ctcaccgatc tctttcgcta 55440
   cactaacgac gcttaaccca ctaatctcca gaggcagaaa ccctaccttt cctctgttga 55500
   tcgagtcact aaacgttttg agcgctttac cgccgcaagt tccgacaaga agaggattct 55560
   tcttgtcttt ccgacccaac acttccccga ttctccgaca gttctcgtcg aagtcaccgc 55620
   tatacccgaa tctcgcccga ccagagccag attcggttac gttacataga aacagtggag 55680
   gacagcgaga acgcgggaac cgcgtcaccg gaggatgaag cacgtcgagc tttatgtccg 55740
   tgctccgaaa cccggcttcg ccgaacaccc ggctcacgat cgggtcatcg agtatcgaca 55800
   atatgaagta cttaagctca accttcaaaa ccgacgtcgt ttgagtaacc ccaccgtgga 55860
   gctgatggag atggtaagtc tccgggtgcc ttctctgagt cgcctgagag cgtttgatcg 55920
   ccgccatgag ggagttagac accggcggct cttcctccgc ctcgttctcc gtcgtcgtcg 55980
   tcgtcgtcgg agaaggtttc gaggaaggga gtctgtcgag agatacgccg acgcagagct 56040
   cgagcgcgcg gaactggagg cgggaagagt acggcgtgct gtgagcggcg cgtgaaatgc 56100
   aaacttcgcg gagaatcgaa gaaggcatgg ttaagagacc ggagatggcg tggagagacg 56160
   tcgtttgcgc gtggcttctc ctacgcgcga cggctaccgc gtcgtctagt gcgcgtgctg 56220
   tttcttccgt taaacattgc ctcgccgtgg taaccggtgt cggcatcgtc gcccgctttg 56280
   atcaatttca aactacacca accaacaaac aaagatcgta aaagaataag gagaatgctg 56340
   agaaatgtat aaacaaatcg cgcgtgaaat ttctcgaaat ggattttacg acaaagatat 56400
   caaactgagt agtcgctttt tttgaaaaag aagtattttt atttattttt atgttttgtt 56460
   tacttctgtt gctttggttt cagacctcag gcttacgctt agatatgtaa gaaagaagag 56520
   tcgctgttta actggtctat tgtgaatagg tcccactaat atgtaatatt tatgtttttt 56580
   tcttttcaat ttataatcat atttttgtat ttttgttgtt gcccccaatc ctcgtgtata 56640
   ttgaaggagc aaaggcacat gtatagtgag catagattct ctatggccca agtgaaagat 56700
   ctccttttac ttctattggc ttatactctt tcaaatttca attaatttta gatttgacaa 56760
   tcccaacagt ttttcacaat tattctcttt cataattttc tctctaattt ttttaatatc 56820
   ctcttctttt attcttatct ttaagaatct gttcattcag ctgataaaaa tatctaaata 56880
   taagtatcca tgcatatctt cttcttcttt tcgttatttt tccaactttg tatccgtatt 56940
   atacacatta cacttccaca ccaactcaat ataagttttg gtccttcggt ttaagtatct 57000
   tgaatctaga tgcaagtttt attccttttt tgcaagcttt cttttagttt tgttatacca 57060
   ctattcttaa atatttgaga aaataattaa aatgacttaa ttcatgctaa ccaactaaaa 57120
   tcaggtaata aactaagaaa aatatataaa gcatcaacac tcatctaaaa atgaatcgac 57180
   aaagcattaa ccataagatc atattgagtt atacagggaa gcacaaaagc cattataata 57240
   tttcagaatc attacaattc tcacgtcaaa taaagggatc agtcaagatc aatagatgtt 57300
   gtactaatcg attagttttt tttttaaaga gaaaacaaaa catgatgatc atggttaaaa 57360
   aatgtttgct tcaaaaaagt tctgaacttg attgatttga atagaaaatt gatactttat 57420
   gtaaaggatt ttgaattatt atgcatttta aaaagataat aagaatgatt aattagacag 57480
   gtcaacttaa attataatta agaatatatt cagtggtaga cagtgatata attttacttt 57540
   taagagatta gatggatgtg atatggtaaa gaataaaaac aggagtaagt gattaggccc 57600
   caaccccctt tataaattcc accaccacca tttatatgct acttttgtgt cgttgtcatt 57660
   gcaaaagtct tttattaata ataatgaaga agaaaataaa acttcctttg tgttctactt 57720
   tttatattct ccattgcaaa ggcctccttt tgtctttccc cttttggaaa aggagattta 57780
   ctcaacgagc aataattatt accagtgaaa tagtttttga tattatcaca ccagttaagg 57840
   acaaacaaac atcgatcacc ggaacatcgg cttaataaaa ttttttagat ttatttttgt 57900
   ttcaaaataa taaatttttt aaaattaaag tacttttatt agttaatgct taaaaactgt 57960
   atatttttaa gaaacatatt aattgaaaat atttgaattg gttaaatact atcagttgat 58020
   atttattaga aaatatataa taacataaat aataaattta attgtaaata tttattatat 58080
   ttttaatatg cgtgaatact ctagaaaatc tgtttttcag aaacagaggg agtagtaagt 58140
   actacaagtt agtaaattca gttttaaaac taaattgacg gcctatacta tagccagata 58200
   taatttccag acgcatgatc caaaatttcc agaatcgcga acgaacaaca tctgattgtt 58260
   gcatccagtt actgtgcgcg gatgccgcgt ctggaattct catccagttt acgaaacgaa 58320
   cagggccata ttgtgagtca acctcttcat agctccatat tgcttcttgc aagagttgaa 58380
   cttgttccta aactttatcc atggatgcct caccccatat gtatcataaa acttgtctgc 58440
   aattgtcttt ctcgcagtct catggagaac cttatttttc acattgcctt tcagttcttc 58500
   ttcaattctt agttgaagca caaaccgtgt ttgttcatca ctccacaata cagtctatga 58560
   acaacacaag aggaccatct aagtcaagac taaactaaat tcaatgcatt aagaccatat 58620
   ctaagtcaag actaaactaa attcagtgcg taaagaccat ctaagtcaag actaaatgca 58680
   acacagaaac accaaaatag agtgttgaga atgacttaca tcacttccag ggacagatgc 58740
   cattcaaact tgaacagata gtcactgaaa caaaaataaa aacgagttaa tacaagactt 58800
   taagactctt aagcgcaaac aatgaaacaa gaagaccata ctctacaaag acacagcaac 58860
   aaaaacaaga agaccatact aataaaacaa gagcttcctt gtattaagag ctacggttaa 58920
   atgaaggtgg gaaagtaatc attttcttcc ctcagaacac aatgaaacaa gaagaccata 58980
   ctaaaagatt gttaccttac taaaagatct gttctgtctt attttgtttt cttgtctgaa 59040
   tgtaataatg aatatacaaa cgacacaaca cattcattag ctctaagcaa ccttactaaa 59100
   gattgttacc ttactaaaga gagtctgagc ttgagagggt ttgaacttga gagagcctga 59160
   gcatggagag ggtttgaact tgagagagcc tgcgacctgc aagaaaaaaa ataacagacc 59220
   ttttgaagct ggcaaatgac agtacatgtt ttgtgactta aaaccacttg gtaagagagc 59280
   tcaaatgctc aaatatacaa gaaaggacgt attggtgatg taattcagct aattaatcac 59340
   aaactcactg atgagaataa aacaaatgca tcacaaatat acacataacg tcataccgtg 59400
   agagagtgag cttgagaggg tctcgagaga gtgacttgag aggagcaaca gctttacaca 59460
   aacctaagca tcaaaaaccc aatgctatca tcacttcatc aacccaaaaa cccataagac 59520
   tcaaatcaat gtggtatcag agatagttac ctcggagaag agagagctcg agagagatag 59580
   ctcgagagag agaactcggg agagagagcg agagagagag agagagagag agagagagag 59640
   ag 59642
<210> 12
   <211> 28086
   <212> DNA
   <213> Brassica napus
<220>
   <221> misc_feature
   <222> (27461)..(27461)
   <223> n is a, c, g, or t
<220>
   <221> misc_feature
   <222> (27463)..(27463)
   <223> n is a, c, g, or t
<220>
   <221> misc_feature
   <222> (27465)..(27465)
   <223> n is a, c, g, or t
<220>
   <221> misc_feature
   <222> (27467)..(27467)
   <223> n is a, c, g, or t
<220>
   <221> misc_feature
   <222> (27470)..(27470)
   <223> n is a, c, g, or t
<400> 12
   tttttttttt tgcttaaatt ttaaaaaaaa aattaaaaac taaccaactg cgaaccgcca        60
   cgtgtcagta tggtccgcga acagtacaag aactacacca aattcgcctc ttgagaaaag       120
   aagaaaatag tgggttttag ttttttgtgg gcccacactt cttaagaacc ccttaaaaat       180
   cagggataaa gatagctcta agtggtagcc tcatttgaga atagaatatg ctttattggt       240
   gtttccactt tgttaatttc tcttgttctc ttgcatcaaa taaaactagg aatacaaatt       300
   tgaaaatact gttttgaaag aaccaaaatc tctattaaaa tccaacatag gacgaatgaa       360
   aattttctaa aattatgtag gaacagtttt acgagctaca ctaatagcaa tatctttatt       420
   attaactggt caaatgatat acatactaaa agtttgattt gtaaatcaac acgccttggg       480
   ctagtggtat ttgagagata atttcaatac agtgaacccg cagttcgatc tctgttggcc       540
   ataaaataat ttaacattgt acttttgaga tctacagaat aatcggttga tcatattgtg       600
   gttaattcaa aaaaaagttc aatttgtatt taaaaaaaaa acttaaaagg aaaatcaaaa       660
   tcttttaaga tatatcgcag acatgcgcat cagaaaggct tttatctatt tgggccgtaa       720
   agtattgtcc attacttaaa aagtgacaac tccgtgacat tattgttgtg ctgggaccca       780
   aaaacggcgt gcattttgtc gactctcagt cgaacttttt cttttgtccg tcccaccatc       840
   aaaaagtttt taagaccttt ttgattgtaa gtttgtaact aaaaacatag agaaaacgaa       900
   caaaaacttt tacgatttgt aatgtaaata catttaataa aaaaaagttt cacgagtaca 960
   tttttaactt aaaaacaacc agaaataagt aaaaccaaag gagtgtttta ttcctaaata 1020
   gagctaggaa gaaagattaa ttgattttgg atttgtcaga agcataaacg tagagatctg 1080
   gatctgtctc gtagaagaca atatcaccag tgtcactgac gtaatgatct ttcttaatac 1140
   ttgccaccaa actttccact aagtggatcg gtattgctcc tgacgtcttt ggttctctgt 1200
   agtatcttcc caacacatgt ttagctgctt tcgtctgtcg catgtcatta attaagatca 1260
   ctaatttagt aattaatcac cctttaatat aatcaaatga aactagagag agagcgagat 1320
   cactcacggc atcgaccaag tgatagtgag ggatttgtgg gaaaagatga tggatcacgt 1380
   gagttccaat atcgtgatga atgttgttga agatcccgta atctctatca acagttgtta 1440
   atcctccacg taaataactc cattcctatt attgtacaaa aacatcaaaa attcagatta 1500
   ttcaactact aatcattatt gcttcttata aataatgttg atctacttac cttgcctctg 1560
   taccaaggca gcttatcatc gtgaccatga tgatgcaagt acgtgacagc gtccaaccac 1620
   attacaaaga tctgaaattt ttccaaaact tttatgtcaa aaacaaatta tattagcaat 1680
   gatataataa agaaatatat gaaacttaca atgtaaggaa caccatagac ttttagaact 1740
   gtgactggac caacgaggaa tgatagataa acaagagtgg ccaacacgat cgaccagcaa 1800
   gtagttgaag ttgcaataag ctttctctcg cttggggcaa ataaactact gtatgggtta 1860
   taatgtgacc cttctttacc aggacttctg taccactgta gtcatcccca aacaaattta 1920
   atttatattt agttaatact caaaatctaa aaattcaaaa ttgtaattat aatcaggaag 1980
   aaaaattagg aattaggatt taccagatag agagggtaag cgagcatggg gagagggaca 2040
   gtgtatctga gcatccgtgt actgtgggac aaattcttgt ataatttttc tggcaactgg 2100
   aatgcaaaat taagattaaa atgtaaatta atatttaaca gtatggttat atattcgaat 2160
   ttattcattg catgtggtgt gtttataagt ttttcttttt attagttcta cgtaaactcc 2220
   aaaattgaaa aatactaaga aaagtaaacg aatttcgaga agaatcattt tatgccaatg 2280
   gctcgaatat aagtggtccg ttgttaaagt taactacagt actataaaca atttaaatca 2340
   gttgtttact acagctaaac gacaaatctg acaagtggtc gtcctagcct caaactggaa 2400
   aaaggattga ttaaaataaa tacatagaat cctaagaaaa ttaaaatgaa agaatttcaa 2460
   aaaaaagaaa aaaaatatga gagagggaaa gattaccgga acccaagact cgtcgttttc 2520
   aacatggcca tggttctggt ggtgtgtccg atggcttatt ctcctgcaac caccctcagt 2580
   tataaaataa actattattt tattttcata aaaatgaaat tggaattgtc aataacatat 2640
   cattttcgaa gcagatggta agagcatgtt taacggggtt tttaagatgg gattcttatc 2700
   agaatataaa actcaacccc aacatgaggc catgattaaa actgtttttt ggtttcttaa 2760
   tttttttctc cgattaaaaa aataaattaa attaaaaaag aaaccaatcg cggaccacca 2820
   ccagtgggat ccacaaacag tacaagtaaa agaccaaaat cgatccttct ttcgcgactt 2880
   ttgtaaccgg ttttttgttt tttttgggcc cacactatat cttattatta atattttgtt 2940
   aaggaccctt cttagagcac taagagcatg attattgaga agttctcagg gtggagttct 3000
   tagcggaata taagaactcg tctcttgatt tttaactaaa aaaactaaaa aacggttctt 3060
   aaatacgagt tttaaaagcc ggttcttaat ttttttagtt aaaagttaag agatagattc 3120
   ttatattccg gtaagaacct cactctagga acttctcaat aatcatgctc taagaaaccc 3180
   catagaacat gctcactcgt ttaactaagt tattcatttt tgagcaacaa acaagtgtat 3240
   ctaggaaaat gatgcatgtt cgtagacatt tcaagctgat gtatccattt aacaataaaa 3300
   taagccatta aaacaaaaat atataaatat tattaaactc acatatgaag ctacattaat 3360
   ttattcaagg acatgtcata tgataatagc taattggacc ataaataggc ccatagcatt 3420
   aaataaaagt ttggttcttt tttcttcgat gctaaagatt ttgatgcttt tagtcacatg 3480
   cattatttta ctatggaaaa ttaatatatt ttcagttatc agattacagt ttgctaacat 3540
   gcaccaagaa tgacaaggaa aatgtaagaa atacgaaaac aagaataaat ttgcatgaaa 3600
   aagatgttta aataaatgac ttaccaacca tggtatggaa cgagaatgaa ggaatgaaga 3660
   atatgaccaa ccgcagtatt cagaagagga atgtctgaga agctcccatg tccactgtat 3720
   tattcaaatt gaattttaca tcataaacat gtttatcatt tattgcacaa tgttaattaa 3780
   actttactca attcaaacgt tccaacaagg taacaaaaat agaatatgac gtgtcacatg 3840
   actatatttc gaaagtagat tggaacaaca cacaataatt aaaagaatca atatacagta 3900
   attatattgt tactttcaaa caataaaatg tgttttattg aaactttcaa acgtagatcc 3960
   ataaaatgcg gaaccaacaa taattatagg aaagaaaaag atgtttagtt aggacttatg 4020
   agtgttacga tttgatcaaa aaaaaaagtt agcagtgtta cgactgaaaa agagaagaat 4080
   taaaaatctt agatcccctt ttgcttttaa aataggccaa tttgggtgaa cataataatt 4140
   ttttttttta aaagtaaacc tgaagagaat caaatcttga agtcagtgaa aatctcatat 4200
   cgaacgtgcg ttcaagaaat caaagacgat gcaaaaaacg aaaaaacata taaacatatc 4260
   aaaattaaga agttgaagaa aaaataaatt gaaaattaaa ttaccagtcg tggccgagta 4320
   cgaagatggc ccagaaaagg gttccttggg cggcccaata aagaggccag aagaaccagc 4380
   tatcaaaata cacggcggcg acggccagag ccacgacgga gaaaatgtct ctcgcgacgt 4440
   agctcatgga tctcaaagga ctcttgaccc aacaatgctt aggaatcgca gcccttatat 4500
   ctccgatctt aaacggtggt tgtgcgctcg gatcaaacct ttcgtccttg gaatctccgt 4560
   tcacattgct acgctggtcc atagcgacaa ccatcgccgg agaaagagag agctttgagg 4620
   gatgtttctc tctctctaaa actgtgtggg ctctgagtga aatgtggtgg agagagtttg 4680
   atggactttg gggtatgtgt ggtttgttta tataaaggga gaagatgtgt agagacacca 4740
   aactgttttc tttttttctt aatttaggaa acttttttat tctttgaaga ataaaaattg 4800
   tatttttgcg gtaacctgtg cgcaatgtat ctttgttacg tcgttcattt cgatgaaaac 4860
   taagttagag aaatgtgtta caaaaaaaac aatgctataa aatttacaga agattttaaa 4920
   attgcattat cgagtataag taaccatggt aatggtatca aaatttacca agattttctt 4980
   cttttgtttc tctttagttt ttccttagaa gtaaggattg tgcaccgaaa tggtagtcaa 5040
   cttgtatggt ttttcatttt cactgattga tatttacaat ttcgcaaaaa aaatacatgt 5100
   agtcgaaaat attatgttag tcttcgtact ctattttgtt tctgctaaaa tttcctgact 5160
   atgtataaat cataaaaaac gatccatatg gatatcatgt agattgtaga catgccaaca 5220
   tttatataga ttttttttaa aacgtattaa tttgagggaa aatagttgcc acatcactgt 5280
   gatgtatttg acttaagaaa cagacttcca tcagttttta tttattttag acgacttaaa 5340
   ttggcggttt atacaatgta attgttattt tccccagttt gtcattaatt agttaatggg 5400
   aaaatcagtt ggattgattg aaccgattca cttgatcccg aaataacaac accaaaatag 5460
   aaccaatgtg tggggtaggg tttgaaagaa tttcttaaaa aaatggtaca attttttttg 5520
   gactaaaaac atggtataat tccaactata ttttatcggt ttaacttttg acatataatt 5580
   aactttgaat ggtgaataaa gtcataaact aagatcaaaa catttatggt gttttgataa 5640
   taaaagacat ttatgggtta gtcaatgaga catcatattt tagaaatgca ggcaagatgg 5700
   cgtttcctgg ccagcctcga gatttcgggg gcttatgcga tattggtaaa gatttcatta 5760
   aaaaaattta aaaaaatttg gaggtctttt taaaaaattt gggggcctat atttatgtag 5820
   tttttttcaa aaaaattagg ggtcctaaac gaatgtttca tccggctttg cccaggaaca 5880
   gctctgctct acctcttctt ctctcttaaa ttaattttcc aacacgtctt tacgagataa 5940
   gcatcaacta attgctacaa ttgtatacag aatttactta gctgctgcct ccattaacta 6000
   catttcaggt tatatggtag tgtatgtgca ttgattataa atacgcagct tcattgcata 6060
   tattcaaact ttttgttgga atgatttccc catctttaag aatcgggtaa tggacgtgaa 6120
   ccgtgggttt actgtttaat ttattaacta tacttatatc agttttttaa tatttaattt 6180
   tatatgagaa atcgattaat attactaaaa cacaaaaaat tgttttcttg cgttatttta 6240
   tggtttttgt cactgaattt gaacatgata ttttctcttt cattaaaggc aaattaccct 6300
   gttatggttt gagccagaga ccaaatacta tatattacgt ctatatatac ttaatcaaaa 6360
   taagagaaga ttatatgcac tctaccttta aacgtgagat ctccaaaact gtcataaaaa 6420
   cgtgatctca tttcttcttc caataacata tatcaatatt gtacatccaa ttccttcctc 6480
   cataaaaacg tgaacacctt tcttcttcca atcgtaatat caatgttgtt catccagttc 6540
   cttcctccac aagcttttta tcggaagaat ctgcaagcgt gttaaacaaa ccaccatgga 6600
   agatgtaccc cagcttctgt gagagttttg gagaaaggag atctacatgc aatttcttct 6660
   agcaatcttt tttaacgtaa aacatttaat tttctcatat gtgattctat gatgcttgat 6720
   aattaaaata tgatggcctt aatgaataat cttgatgatg tttttagtaa gtcaacagtt 6780
   tagcatatga gattaacttt ttaaatattc atttataaaa tttactgcag tttgtataat 6840
   aactaattac ataacaccat attcttggat ctaaaagcat ctccaatata aaattctatt 6900
   ttttcttcta aaatagaata attcgattgt atagttagtt tactccaatc ctactcattt 6960
   ttggagtgaa agcaatgatg aacaaaaaaa taaaaaaaaa tctatttatt ctattataag 7020
   tggaaaatat aatgtggttg aagcatttat ttactctaaa ctcctttttg aaataaatta 7080
   tgaggtggga ttggaactat tctaattgct caaattctta tgactatata tctaggtaag 7140
   ccatggaaaa ggaaaggtac aaatgatgag tgtgggcgta tacatgaagc ctgcacgtga 7200
   gagttgtagc tactcgacaa acgtatacta atttgttgcg taccatctcc acttcatata 7260
   tatatttata tatctatgtg tgttgagctg agatatgaga ataaaatttg agaatatacc 7320
   tcaaaaatgc aaagagaagt atgtgtttgt tatttagcag atgcacatgg tggaggacat 7380
   ccttcgattt cctcgtgaat tccgaagagc taagttattt tcttttaatt atacagcttt 7440
   aaccgagcta attaattaat cgttacataa tttgagcact gtttgaagaa ggcagcgtat 7500
   atatacacat tagtatagta atacagttat ataggatcca gttttctttg tttgaaaaca 7560
   ctcatatgaa taatatatac ttttaaaaca cgacctgtaa cattttttga cccggtttat 7620
   atgtatgtga ttcatatatt tctctaacca cgatcgagta cgactaaatg tgcttatcaa 7680
   ttatcataca cgtctctacg tgttcatcta tcttttatta tttttatcaa ccattcgtat 7740
   tcgtgtacgt tgaaaggaat cattacgtag atgcccacga tgttaccgaa gttggagaat 7800
   tatgttattt agaaaaccca tttttaatta cgctaattac caaaactaat atggggtcgt 7860
   aagaatatgc tttcggtagg cttcgcgttc taaatttaca aactatagca gtcaacatat 7920
   aagaggttaa atgtattaga ctgaattttt tttaatgtgt ggtgtggggt tacaaagaaa 7980
   taaaaacggg attagtgaag cttattggtt actaatttcg aaataatcat gcatggtaaa 8040
   aaatcatgtt atacattgtt gtatcagacc aaaaaaatgc tatctcggat tttgaatatt 8100
   ttacagtcaa aataagtaga tttaaaagaa tcttgtatta ctgaagttgg aatttagaga 8160
   ttattttgaa aattagatag ttgaaaattg attagatcgt tgtagtgatg agttgacaaa 8220
   aaataaggtg gtctaaatat atggaaattt cgtcctgaag ataacaaagg cctttgatct 8280
   tgcatctagt gcattattaa tagaagaata ttcacaagaa tcttgtgctg tgtgaccatt 8340
   tttgtagaac aatggccaca ggaaatgtta tgtttcttgt atctagaaca atagtatcgg 8400
   gaggactaat tgtcaccaaa actgaaaaaa taacaagtta actaagtgta tcgatacata 8460
   ttcacagtcg aaataattaa tagaggacaa cttgtccatc agttgttaat cttggtggaa 8520
   aaggttgctt gttaattgtg ttaaatgcga gtagagtata agcggattta catgtaggaa 8580
   aatataggaa gaacataaat attggttgaa aaattgcatc acatttttac caaaaaaaaa 8640
   ttgcatcaca tgcatattat tcgcatgaga tgtttaaaga aaggcccacc gcacgcgagt 8700
   ttaatctcca atggaaagac ttacagaaag gtcaaagttc tttatcaaca gacaacagga 8760
   tatgtgtgcg tagattgtaa aacacgtagt tatctataca taaactaatt cttaaattcg 8820
   ttatgtatag tttttttggc aggaaaaaag catagaacca taaagaagaa gaacggttga 8880
   agatcacgat ctattcatga atacgtgtcc tcagctttaa accactcaca tggacggttt 8940
   aatatctaac aaagcattgt ttttccaaag atactttatt actgtactag gcggcaatcc 9000
   agctgataat tagatgaaaa ctaacaccat ttaaataatt taaagttagg tttgtaccaa 9060
   taataatgtc taattggacg gcttagagaa gaaaaagatg ggacgtacgt gcacgtgcgg 9120
   accgacgaaa cacgttgtcc tctgttcaca taagcaatgg ctctcggctt tctaaaaata 9180
   tctctaacta tgcagtgaat tacttgacct aaaccatgtc atttcgtgca accccaacaa 9240
   attcctggct tccttttttt gtggttcatc aatcttttct taggacaaaa cgtttttttt 9300
   gtttatgtca gttaataaat gatcaagtcg agtctcgttg acaactagat atcaacgcat 9360
   atctggtaga tcactataaa actcagatta tgggtgcatg ttttggatat taaagcaaat 9420
   atgtttaggt ttggaatatc agggtatata aaaagatata gtttttgttc ttacggaaaa 9480
   gaaactcaaa ttaatgaaca ttaggcttga agtcatataa tcaaacgtgt aaatgacatt 9540
   ctttagtaat gattttgttt cccgcagttt aaaaagaaat ctcactcatg actaatgtct 9600
   acaaaagtag acaaaggatt cttagttgat tctttagtaa tggttgaata gagctgaaag 9660
   ctaaagtcat agcatacatt tggtcacttt catgaattta catatataga taaaaatatc 9720
   aactagttca ataagatatg attgttttat caaacagaac atcatgagtt ggagtcttga 9780
   aatcatttta acctgttttg ctgagagcaa aaatattgat ttaaataaca attgtgagat 9840
   aggcaaataa tctcacgtct tacttttcac atatataata cacatatagt tcatatagtg 9900
   ggtttgcgtt aaaatagaaa taccattttc atccacaact aattgataaa agaaacattt 9960
   ggtatcggga tctaaacgaa atattcacca atcaaattta attttatata tagttttata 10020
   atgaggagac gagaagatat ttatgaagac aattattaat tatgtatgtg aatatgattc 10080
   gttttctttt ggatttatag agctatagta gcaatccgta gagaagaaat ctgaatcgga 10140
   tataacgcca aaagagagat catatgagtt ctaaaaactt aaccacgaca atgttatctg 10200
   tccatattat ccatcttcgc acttcatttt gttccatctc ttgtccattc tctatctcta 10260
   catgacatta cgtttcctta acatacatgc ttccattatg tttctgtgta aaattaatta 10320
   cggttacatt atttattgat ttgcattaca tgtatgattt ggagatgcat acacttggaa 10380
   ggagtatacg agcatgcgtg acaactgaca tgaacatgtg aatatttaag atccaaactt 10440
   ccaagtatct tataattcaa tcagaataga aactttaaat tataactctt tgttgccaaa 10500
   aaaattataa ctccttcagg gatctatcca caaaatccaa atatagcaca aactaataat 10560
   tagtttatca gaatgcttaa tgcttgacta ttaaatattt cttctgattc ttttcccttc 10620
   aaacaaaacc acagcaacca aaattatcat taaaaaacga caattttaaa accttctctt 10680
   tctccgggaa ggttatgtta ttatattatt gtaaatcaaa ccgagacttt ggtctctggc 10740
   acaagtcagt tatacggcta atgtcacggc caaagaagaa agtggtaatt tagctgatga 10800
   agatagtagg agttttctcc agcttatgac tcgatctcca tatgtaccag ctcacgaagc 10860
   cggtcactgg tattcctttg gcgtcctgac caaataatct atctcaacca cattgcttac 10920
   gagtgaagtt cattcaaaaa gaaatctcga gtcaaagtga tggatttcgt tttaagaatt 10980
   ttccttgagc tcaatgagca tttaaaatgt cccaggccaa aagttctttt cttaataaaa 11040
   tttgtgaacc gaaacaaaac attcttctct taacaggtct ttgggcctgc tgttgaaaga 11100
   aacagatatt taggcccata tatagtaaaa tttttatggg gcttatagaa atcagatatg 11160
   agatattcca taattatcaa attagttcac gagaacctca agtgataggt agaagttgaa 11220
   taagattatc agtccagatg aatgccttaa tcttgggaaa gtcatcactt catatgtctg 11280
   agaagacgtt tactaacttc aaagttttgt ttgtaaaaaa aaaatcaata tgtgaaatca 11340
   aataaactgc atgaacacac acaaagtgaa gtatacaaaa agctgaaatc tagtaagatt 11400
   aaataaagct gaaatcgatg tagaaacaga aaatacaaat aaaggtttta tttttgagtt 11460
   atttttattg ctctctcagt atacatacat tatttgtaag cttgcaagta aaattaagaa 11520
   gacaaaaaag attatcaccc tctcaacgtt tgcgtcctcg gccgccgcga ggtggatcgt 11580
   gtctgccgtt agctgaaggt tcaccgtagt cgttggtgct caccatcaat gaccgttctc 11640
   tcaccaccct catttcattt tctgtcatat atgcatatac gttacaagtt agaacatagt 11700
   gagaatataa aatgttgtac ataagaacct cttattaaca aacgatttat taattaagta 11760
   tctatacaaa cgtcaatacc ctcgttttca ttttgtttta actacatcga catgcattca 11820
   taatctttta actttatttg cacataaatt tataaacgta tattgatata tatgtttcga 11880
   tggttgtgtt ataaacttaa atttataaac atatattgat atctgctaaa aagaatagat 11940
   ttaaacacac ccaaattcga cctttttgtg tgtgttggat gtcggtttca caaatcgaaa 12000
   tctttgcttg gatttttcac agatagtcag atacgatgga ctaagatcca tttcaacttg 12060
   ctattttatg caatttaata ttatctgtaa acttcaatta tatagtcgtg atcttatctg 12120
   tcattgtctt tttcaaataa tgtcaacgct tttgaagtgt gaacacaaat taaatatcaa 12180
   gcttttatat tacatggttg tactttacaa aaactcataa tacttcaaaa aaatatttaa 12240
   aatactttgt tttcttcatt agatttatag tttataattt tatatgacgt tttcttactg 12300
   gattcgtcgt tatcacagat atgttctttt aaaagaacaa gtcatcggcg aaaggaaaga 12360
   caatctcgag catcgtgatt catgtttgct tgaatttgaa tacaaacaag ctggaaacag 12420
   agcgcataaa actaaggata tatccaactt gttttaacaa tatatatttc aacacttatt 12480
   caagtaataa ttgtaataat ttagttgtgg gtttctgtag tgatttaaaa tgaaaggtca 12540
   atgaagttca catgaactaa ttagtgtgtt attcttttgt tatttgtatg ggttcatcat 12600
   gtgttattct tttgttaatc agagtatgta tgcatatcta gggataattg gtatcatgta 12660
   aatacgaagg ataaatatac atacaattat ttattttgct tgtgtaattg agattttctt 12720
   gttttcttta ttaaaaaggt aaaaactgtt aaggctttct tcttctcctg gtgatatatt 12780
   tgaacatact cttaagatat acacagattt acagatatag atcatgtgac taccaccaca 12840
   tatcaccgat cagtgatcca ataattgtgg ttgtaaaata tttgattctg agatctcatc 12900
   caataacaca taaaatagta aactagatta gttttaacgt taaacaaaga tgatatatgt 12960
   agttattagt gaagaaatcc ttatgagttg ttaacaggat atggattatg aagaacttgt 13020
   tagcttatat atagtgcttg gatattagat aaccaataca tattaccata caaaaagcta 13080
   gtaaacactt gaaactaata gagaaacgaa gggagggaag aagagtatac ctggaaatga 13140
   aagactgagg cgagcagaag aagagacgaa agcaaatgtg aagaagagta acaaacataa 13200
   cacaaccgag gaagatgatg cataacccat tctctctata tatatatttc tctctctcct 13260
   cccttcttct atatatatag accacaaaat gtctcatacc ggcccttcgt tttcagcctt 13320
   tctcactatt taatcatttt gatttttatt aatatacccg cttccaaacg tttagttttt 13380
   acataattgc gtttgaaagg aacatattct ctataatcta atggttttgt attcaatgcg 13440
   tgtatatgca tgtgtttgtt gttgacaagc acaaaaacaa gggaacatga ttgcatttac 13500
   atacggtagg tttgacaaga ctgaagtggg atccctttaa accatcaacg aattaaaatt 13560
   cattttttca ttgtattggt tacaacagaa ctcaaatgcc agcttaaaat ccaacccatt 13620
   gctatttttg attttataat agctttagag gcacaatgat tccaaatcca ttactatttc 13680
   ttattctaaa atagaaatta ctattttttg ccaaaaaaaa atagaaatta ttattttgtc 13740
   ctctatttat agaggaagaa ataacagtct ctatttttac tctatatttt gaagattgct 13800
   attataaaga aatacattag agtaaacttc acctttttat aaagattttc tattttagag 13860
   gcaaaaatag caaaatacat tggttttagt aatgggtttt agtagaataa tttaatactt 13920
   tcattgtaca aattaaaaaa ctttgttagt tatcacatac attcaattag gataatcata 13980
   acataaaaac aagtacagac cacccgagtc tagattatca agaacaagaa agcattatat 14040
   gtctggtttt gtacccccat caacttaaga ttctcttgaa cataggcaac acacaagttt 14100
   acacatacat agcataagag atccaagtac ttcaagaaag cataggatcg gataaatcgg 14160
   aaaatacatc atcgtttttt gaaaccatat ttcttacgtt catagaagag atcggtcttg 14220
   gcactcccaa ggttgacgat cttggggcaa ccatctctgt ctttctcctg ctgcgtacac 14280
   tctttgcagt agtaagcatc cgagatccca acacctccgc agataacaca gcggccttgg 14340
   aatgacccgt agttgcattc gtcacagata cgcaccagag tgcagggacg cacataagaa 14400
   tcacaaacca cgcatttgcc gtcgcatttc tcgcacagcc ttccgatggc aatgcctggt 14460
   tgtttccggc acatgatcag atcagggtga tgctttgcca tggctagtga aacacagacc 14520
   tgcacacata agtcacttgt cttgagctca tatgatcgta aagagtacaa aactagaaac 14580
   tgaagaacaa gaagcaactt aaagtcctgt tttcacttgt gtctgaacaa tcaattaaaa 14640
   gaaaaagaga gtaaaaaaat tggaaaataa agtttgtgta gcagtgttaa cttctcagag 14700
   gaatatcatc gaacacctta catgcacaag tctcagccga acattactct ttcaagattg 14760
   cagattctag agacatgatc aatcactcta cgaaatataa ttaataatgg gctgagaaaa 14820
   caaattgaac aaaagaagga aatcaagaag ctatcacaaa ccctaaaaat tcaaaatcaa 14880
   gaaacaaacg aagacgataa ccaatctgga ggagtcctct ttagagataa aaaaaaaaaa 14940
   ccaaagctta cagttaacgg gagatcaaac tcgagcaaat caagagactg ttgcgacgag 15000
   aaatttccag agcgccaaag atcaaccaac caagaaaggt ctggaacgaa cgaggcaagg 15060
   aggaaattta tcacgagtag agctttttaa atcggtccac ttgttatggg ctttttactt 15120
   tgggcttaca aactcttcat caaaccaaac caagccggta agcaatgtaa aatccagggc 15180
   ctaaaccaaa ccaggttaaa cagcaatctg agttgcgact aaaagtgtcg gtctcggtct 15240
   ccgtctccgt ctcagaccca atttttattt catcagccgt tagctttgac ttctgactag 15300
   cataacgtga ctttgttgct acaatggtac acaatatact tcttttttta attgggaaaa 15360
   tcgcattttt aaccttcaaa gtgacatttt ctaacacttt aaacctccaa cctttttcac 15420
   tagcacttca atacctcaac cctcaaaact tatcatatta aaccttgaag tcgtttcccg 15480
   ctcttaagcc tccaggcgat ttgacggtaa tgttcacgcc gtcatcctca ctaaaaacgt 15540
   gtgtcgtttt tttaattaaa aaacaccaga tacgtttttt atctttttta tctgttctaa 15600
   atcgaattgg ggatctaggg tttactcaaa atcaaaatca gaaggagaaa gctcgatact 15660
   tggcgacgag caagagattc gaacagagta cgtcgtctca attgatttgt taagcatctt 15720
   agtatagcaa gttgtttctg ggctttgttt ttcacttcat aaatcatgta tatgtgtaga 15780
   tagcgataat tgtctgagtt agaattggtt tcacttcgtg aatcatgtat atcaagttgt 15840
   gtagaagctc ttttacatgt ttatatcaga taatggtgtt gtatatgtgt agatggcaca 15900
   cagttcaagt tcatcaaacg ttgtgtacaa aaacgaaaaa ggtgtggttt gcaattgtaa 15960
   ctgcttagca aacgttgttc aagcttggac tgatgacaat cccgggagga ggttctatag 16020
   ctgcgaaaaa cgcaagactg gagatgaata tgattgttgt aacttttttc agtggtatga 16080
   tgttgagaag cctcatggat ggcagcgtga tgcattgatt ggtgctagaa atgttaatcg 16140
   ccaacaaaga gaggagatta agagtctgag gaacaagata agagcactta gggaaaacat 16200
   gggaccaaat tcaatagatt tgaaggaaaa aactgaagca tgtgacgcat gtgaagggct 16260
   caaaagggag gtgctgatac taaacgagag gagcagagtg tatcgcaatg ttctcataac 16320
   gtcatcagtt ggattcactg ttgttcttgg tgtgttcatt ggtgtgttga agtggtagaa 16380
   ggttattcaa agttgtttga tgattttatg actatgttat gactatgtaa gctatttgat 16440
   gttatgacta tttatgcttg tttgaaggtg ttaagactaa gatgattatt atgtttcaat 16500
   gttatatttt tgtcatataa agtaaaaaaa catcaagatc ataaaaccga accaaacaaa 16560
   ctacattaag tcatgtcatg agaacaacaa aagacaaatt ttaagtcatg agaacaacaa 16620
   aagacaaatt ccaagtcatg tgaacaacaa aagtcattga cacaaaaaaa gacagattcc 16680
   gagaagacac ataaacaaca tcaagatcat acatagattt aatcactctt gtggaggagg 16740
   ttgtgggttt aggtcggacc tatcataaac tcgatctcca agcacctcaa aaggtcgatt 16800
   tgtgaatgga ctccataatg tcccaacacc atgaggaata ttagttatct tccttacctt 16860
   caaaggaagt cctcttggag ctttcttggc atgaggcttt ggatcagtgg atgaaactga 16920
   tggctgtgga gcagtagagt gagttggtat ctgagaggat gattcagcag cttgaacagg 16980
   ttgagaagag cttgttcttt ttcttcttgt tgaaggaggc ttaggcggac cctgaatcaa 17040
   aaataaatgt taacatagat gcattgtgta taaattaaag agtatacgag taacttacca 17100
   ctggatgtat acgaggtcgt acacgtttgt tctttggacc ctcataaacc acttgttcat 17160
   ttttgcagcc acttttaatg tgacccatct gaaggcaacg gctacatttg ggcacacgtc 17220
   cgtgtcttgt cgattttcca gcgttttcaa ggtcttcaaa tggctctttt ctcctctctc 17280
   ttgttcttgg tctacctctt ggcttcctta actctggtat tcctattgat ggttttccta 17340
   gcctcttcca caagttttca ccattgacgg gcttgatgtt ctcgttgtat gttttcttca 17400
   tcttatgggt gtagtaatac tcggatgtat acttcacagg gtcttcttga ttatcatcaa 17460
   acacacagac atcatgtttg caaggtatac cagtaagatc ccatcgcctg caagcacact 17520
   gatgtgttgc caaattcact gtgtaaccat tatcacactc attaacctca tacaaacttg 17580
   agctgcttcg tagtgttgaa caatatttct tggcaatcct tgctttctcc aataaagcaa 17640
   gtgtgattgg tgtaacaata gtatcccact tatctgccat aaaccaccgc cttgaattcc 17700
   tcttcatagc ttgtcttcga atgtcctcca acatagttat cacgggtttc gcccttgcca 17760
   tctttatggt tctgttgaag ctctcagata agttattatg cacgtcagga cagtgtgaat 17820
   caacactgaa atatgctcta caccacctct tagggtctgt cttgagtaac tcttggtgtg 17880
   ctacaacatt atatgcctct aatagactca acttctcttc atactctcct ttagtgtagc 17940
   tgtaagcaac tccccaaaac aaagatttaa actctgatct cgcaaaccca agcttcttcc 18000
   aattcgcata aatatgtcta gcacacatgc ggtgttctgc atcagggagt tccaactgta 18060
   tggcatgaac aagacctttt tgtttatccg aaatgatggt cagatccttg ccatttccca 18120
   agtcgagatc catctttagc ttcttcacaa accagcccca agtgtctttg ttttcccctc 18180
   ttacaactgc ccaagcaatg ggaaacattc tgttatcagc gtctctacca actgctgcaa 18240
   gcaaatctcc atttaaatcc cactttaaga agcatccatc aagacctatt acaggtctac 18300
   aacaactctt ccatgattca cgtaattcct tgaagcaaat ataaaagcag tcaaacatct 18360
   gaacaccgtt agcctctctt gtgcataatt cagtgcttat accaccattt gatctatgta 18420
   actctgcttc ataatcccat atcttgaata gtcggatttt catcctctga ttcttcaggc 18480
   tcatcgtctt cctttggtct gtcaacatct tcatcttcat cactacacga acgctcgtct 18540
   tgttcggtgt ttggtatgtg ttccacgaac acttcaacaa catctactcc tagcttcccc 18600
   gcagaacgaa gtatacgcat ctcctcatcc aagtaatcat atgcatatct caggtctttc 18660
   atctcctctt tctcgaactt gaaccaaagc agtccaattg gtgctcgtat cagtgaatct 18720
   tccttgcaaa acagactgaa cctctcccat gtgatctcgt caatcttcca ctccacattt 18780
   ttggtgcccg tttcaccaac atacgcatat ccttcaccat ccttcttcat tgaacctcca 18840
   aaatgaatct ttaacttcat ttatgttgct tccctgtaat caattgctta aactttagac 18900
   aatttcgaga gataaaacga atgtaaaact cgaaattttt gaaagaatag atcaaatcga 18960
   tgactcgcgg acccttaccc catatttgct ttgattcacg aaatttccta tcacttaatc 19020
   gagctttctc cttctgattt tgattttgag taaccttaga tccccaattc gatttagaac 19080
   agataaaaaa gataaaaaaa cgtatctggt gttttttaat taaaaaaacg acacacgttt 19140
   ttagtgagga tgacggcgtg aacattaccg tcaaatcgcc tggaggctta agcgcggaaa 19200
   acgacttcaa ggtttaatat gataagtttt gagggttgag gtattgaagt gctagtgaaa 19260
   aaggttggag gtttaaagtg ctagaaagtg tcactttgaa ggttaaaaat gcgattttcc 19320
   cttttttaat tagtatactt tctctatatt tcactccaat agcatctcca atgtacacct 19380
   ctataatttt ttctaaaata tagatttcta ttataaaggt gaaaatgctc caatatatgc 19440
   ctctataata tagttcatct atttatacgg gaaaatatat aaatatattt tttctatatt 19500
   ttcttttaaa atagaagaac tctattatag aggcatacat tagagcattt tcacctctat 19560
   aatagagttt ctctatttta gagaaaaaat atagagatag aattagaggc gggttggaga 19620
   aggtctaata gtataactct ttggatttgt tccatggttc attctaacat aattactaga 19680
   tctcgatccc cgcaaccgcg cagatttttg ttttcattta tttttatata aatattttgt 19740
   tttcaattct aaattggtat atattataat agatgcgtct atcaattttt aaagcataat 19800
   aaatttaccg tatatttttt tctttgaata gattgtttca aacattcaca tgtatttgta 19860
   ttttcttcta tatatatatt tcagattatt atttcattat taaaatcgta actatatatt 19920
   taaagattag taaaatattg ttttattgtc atattcaaag atattgtaac atttcacaaa 19980
   tttagaaagt ttttaaaaaa ttaaaatttt cgcttcgtag atttatatta tcgagtaaat 20040
   aattaaacat ttggtttttg tttaattttt aaaataaact atataattta aaatttgttt 20100
   tcattggttt aaggtagtaa atattaataa ttgttagata atatgatttt tgttatttta 20160
   aaaaaaatat ttataatttt aaaagttaac atcgacaaat atttaaatat ttaacatatg 20220
   gaggtatagt atattacaat attaaattat atatatttaa gttatactat ctataaatcc 20280
   aatggataat ctattgttta aatccaatta ttgatagtcc aataaaaatt tctggtaggc 20340
   caaaaattta aatgatataa ttatacttta aatgtaacat gacttcatag gaataagttc 20400
   attaggtcaa tttttttaaa aatcacatat gaatcaagtt atgacttcta ttttaatata 20460
   taagatattt tcacaaaaga tagagatcat ctttttctgc gctgggaatg gagtgctgat 20520
   ctttggaaca tttgtctgcg aagaatgggg tattccaatg tggagttcca taaatggtta 20580
   gccttctacg aatggtaagg ctgcatgaca aagttgtgcc aaggcttctc agacccttgg 20640
   tagtctcaac aacaatctat ttcatttgct ctcagagaaa cgagcgctat tatgttaata 20700
   tctcatcgca gccaacaatc attttcaagc tgcttgaccg tttcattaca aatgcacttc 20760
   tattcattag aaatcaaaaa cagagctgcg gactgatgca agtatgcaac attggctttc 20820
   caagtatgca acattggctt tccagggaat taaaaccata gtctgaacta tacccatttt 20880
   aatggaattt acatatgtgg caaaaaaaaa atacaagtca agaggcagac atatatactt 20940
   ctttttttta attagaagca agagttttaa ataaactgaa atttttcata aaatttaaag 21000
   taattattta caaaaattaa atttaagcta attattaaaa attaaaaatc aaaattaagc 21060
   atgccactga atataaaact atgtaaatgc taatctaact agatgttgtg gctgatttgt 21120
   tgaactttgt agaaatgatg ctgataaaaa tgttataaat gatctgatgt aactagggat 21180
   tctttttgtt ttattttgta ataaatgaag aaaaatattt ttaccattat aattttttat 21240
   atatcttaga aaataaggtg ttcgttatat cccaacagta gtttttaatt ggactagatc 21300
   tttttgtgtt tgtatatttt tcgtcttttt tatgtattgg tttatttttt tgttatttta 21360
   ttgatttcaa atattttttt gtctcaaatt tcttatttag attagttacc atttttaaat 21420
   tttgtacttc tgaatattta gttatactct ttattttctt agttatttta atatagtatt 21480
   gcatatttag atacaaagga aaatagtacg tgtaaaaatt aaataatgta gacatatttt 21540
   tccttgtctg gttttcttca tcccatgtaa aaatccacct aactttgatg taggtttttg 21600
   tcatgttcca atatacgtat aaagtttttt gttggtaaaa atttacgtat aaagttgttt 21660
   cattattttt tcttggagct tcgaataact tttattgact taccaaaata aaatcttgag 21720
   tgattttaag gtgaaaacta acatttctgt taaacagtta ttattttttt acatcgttaa 21780
   ataattatat taaacattgc atatggttat ttgatatacc aaaatgtatt atttataact 21840
   gagactatga gaaacagaat agatgttaaa tgcattactt gtaacctttg gcatcatctt 21900
   tgctatatac tcgaattata ttatattaag tattttattg gtctttaaca tttattttaa 21960
   tcctgttcta aattgtaatg tattaattat tatttttata tttgtttgtt ttttttttct 22020
   cattgtgttc ttttcttaca tatgttttag attaaatatt tttagcatgt attttaaaaa 22080
   acctgccttt ctaaaattaa agttatgttg aaccaaataa agttatatat gtagtaaatt 22140
   aaaatatact taaagtataa attaaatata atatatatta tttaattgtt gtttaatcta 22200
   ttgtgtttgt tatagttaat aatccacatc taacatattt ttaatgttgg tgggaaaaat 22260
   aaccttacac atgataaaac caattaaata tgaagtacat gatatcaaat gtgccaaaaa 22320
   ctatcctgaa aaactaacat taatcaaaaa ctaaagtaga ttatcttaag tctttactga 22380
   tgaaaaaaaa aaaaaaaaaa aaaagtcttt actatatggt acacgaagct actcttctaa 22440
   aatgattttt ttctaattaa tatcacttta tgaacaattt tagtttactt attattgtat 22500
   tgttttctac tatatcctca aatctaagtt caactggaat ttaattttaa tgaatctttg 22560
   tattttttat tttatttgcg ttggcaaatc ctcctgttaa ttttttttta atgtatattt 22620
   catattctag taaataactt ttagttccac tcattggtca atagaaaaga aatatttatt 22680
   taggaagcta agacgaaatc tgaaccatgc aacaaaaaca aacataaagt cattaaaatt 22740
   cagagacaat ttataagtta atcaacatgc aatagaatca gcaatataac cttggcccaa 22800
   caccaatggt gatgggagtg atctgcttca ctacatccgg agagcctgct tgcgtacacg 22860
   gtgtgaaaat tttcccatat gttggttcct taattatata taacaaaaaa aacatgcagg 22920
   ttcctcattc gttagcgtaa gctttgcagc cacatatgat agatatgtca accaaatgtc 22980
   aaactctgac caaattcgtt tctaaagcac ataacaatta agactggaaa ctggaagata 23040
   tatattcact atcttacaat gactttcata aggtgctcac ttatagaacc aagtgtaata 23100
   taaattactc acatatatgt cttctactca catattctca cctgatccct gagcaggtgg 23160
   tttgtattga tctagcagtg tgtgtgaaac tggatttggt tggttgagtt ggctgacttg 23220
   agacgtctct cacggataaa ctttggcttt gttccaaatc ctattttgta attcatcaca 23280
   caaacctatg aagattgatg gaaaaactat catgaaatat atcaattgat gaaaaagtta 23340
   gattaatctt accgtaatac atttgaatca gattgaaata gatatatccc accatataaa 23400
   ctaaacacta agctgcgtcc cctagcttct gactcctagt gacaataaca aagggaagac 23460
   ataagatgga cattcatata gtgaaatctg taactatacc aatatcaata gcttcagaaa 23520
   ccatttgtag ggtctgtgga tcatatgtat ttgcagctgt taaagatagc aagatgattc 23580
   catggaccac aatcagcttc tggctcactt gtgctccgat tttctcacaa cttttagccc 23640
   gtgaaccata tacgtttgtc ttaattacta tatagaacaa agaaaatcaa tatctgctaa 23700
   aaatatattt tctttttctg ttgattatgt tctaatccat gtatttttag tttataaacg 23760
   ttgaatacaa gatatcttca tatcctaggg atgcttatat aatgcatcct caatgttaat 23820
   ttaaataaac aaattagaga gggaagtaag gcaaacgttt catgaaaaaa aattgtagtc 23880
   atcgcatacc tcttgtcaat cttccagatg caaagtagat ctataccagc cattgatctc 23940
   cactgttctg cgcaagaata attgactcac attccaacac tgagtcctct caacaatacg 24000
   tagaacaaag aacagttcct atgggaaagc ataccactta gaacattatc acgttagttt 24060
   ggcattaatc actttgttac cagcacgtgg tgctttactt acctcaactt atatctttta 24120
   attcgagaag tcatctgtga aaggttgcaa aactgtatga ccagctggca tctttgtgtc 24180
   gaaaagtgtt tgtgggaacc gtagaagttc ttgctgctca ttctcaaact tggccagttt 24240
   atgcaaacca attcccctga atatccacca taactaaaga gttattttgc tattttagct 24300
   cttccacgga cctagaagtg aaaccacaaa ccataacatt gttcagaact accaacctat 24360
   atagtgcaaa ggtcctacac atccttggta ttatagtcga attcaaaaca cgcaccgttt 24420
   cattctgact ctgaaaggga ttcccaggcc tccactgcga accagataca ctatttgagc 24480
   ctgagatttg tgtctttgat atcaaagttt gcttaacaca tccaaacagt gctcacctcg 24540
   gaaatctctt tctattatga tcatcgacat caagaaggaa agatcaatca gtaagggttc 24600
   taagcaattt cagatataaa cagaaacgcc agtggtgttt agatttaatt tagaaactac 24660
   tgaatcagaa aagcgattat taagttaccg gcaatggagg agcaacaacg taaccaatat 24720
   tggaggccat ggcgggaatt tgtgttctga aaattgtcat cgtctgtgaa gaaacattgg 24780
   attttgagtg tagccgtcgt ctatatagtg aggatggcga gaaggatgga aaatgaagag 24840
   gcttcaatat aatgtcaaga aggcttaaaa ggatttgtac ggtgaaagaa aaagagatga 24900
   agagctagat agttatggtc tggttcaaga gaaaacgaat ggaattgatg aaacaaagat 24960
   aaagaaaata agaatgtgat gatgacgtgg caataaactc tgacctaatc ggttgatttt 25020
   ttaatctgag ctggcatcct ctccattcag catatctgct ttttagtatt gttagattat 25080
   aattaaattt aaaattaata aagcatattt agtaaattta aaagttgtaa aaaatattat 25140
   aaagatatca cgtacaatat cattttacat aacattccaa atatcttatt tttggaaagg 25200
   attctgattc aatctggatc ccgcataata agcctcagcc ctgttcctaa caagaaaggt 25260
   ctggaacgaa cgagggaagg aggatattta gcacgagtag cgctttttaa gtcggtccac 25320
   ttattacggg atttttactt ttggcttaca aactcttcat caaaccaaac caaaccaaac 25380
   caaaccggta agcaatgtaa aatccagtgg ccaaaccaaa ccaggttaaa cagcaatttg 25440
   agttacaacc aagtgtcggt ctcggtctca gtctcaggcc catattttat ttcatcagcc 25500
   gttagctttg acatattatg actaatacga ctggacacag attggatatc cagatttttt 25560
   aagatatttt tgatttgatt cgtatgttac agatatctaa tttattgatt tgctttgttc 25620
   caaaaaaata cggatattcg gaaagacgga tatccgaaaa ataaatacat agttgcggat 25680
   atttacgaat acctacggat atctcatcca ttttgattaa tacaaacaat cttaaaaatt 25740
   cgatacaaat ttgtatttaa aaatattttt tgcatgatat ataaaacaaa aattaaaaga 25800
   aatagtgaaa ctatatattt ttaaaatttt aaaacttaat taacaattat aataaaataa 25860
   aacttaagaa aaaattataa ttgttataat tatttctcgt atattttatg taatactttt 25920
   atataagtaa taatgtgaat aaaatttgtc aaatcatatg ttagaataat aattatataa 25980
   atacatttaa aacttttaag tataatcaag atatacatgt atttatatat taccggattg 26040
   gagcggatat ccgcttccca aaattttaat atttgtgatt tacttcgatt ttaacggata 26100
   ttaattttag tatttgtttt ccttcaaaaa tttacggata tcactacaag aaaacataag 26160
   tttaacgacg gtggttttcc tcgtgagttt gtcgtaaaag agagtttacg aggaattagc 26220
   gaggaatcac gtttcgtcgt tatatgttcg tcgtaaatca tattttctcg ctaattcgtc 26280
   gtaaactagc gagaaaacca tttcgtcgta aagacgaaga aaacaaatcg tcgtaaagac 26340
   cacgtagata gtccatgtaa gaatgtcgct agcattcctc gtaaatacca cgaaagcatt 26400
   tcctcgtaaa cgacacgtac atatctcgaa aatatttcct cgtaaaattc acgtaattac 26460
   cttgaaattc tttcctcgta acattcacgt aaataccttg aaagtatttc ctcgtaaaat 26520
   acttgtttac catttctcgt gatttcctcg taaactttca acgtaaataa atcgtagatt 26580
   agctacgaat ctacttcgtt ttattgtttt acagaattta aaaatataat taaaaaattt 26640
   aaaattatta aatttattaa taaaattaaa attttaaaaa aatacgcaaa tattttatat 26700
   ataaataatt tttgaattta taatacaacc acgggaaaaa aaaagaacta agagtcgtgc 26760
   atcgcccgga ggaattcatc actcctcctg tctacatcct cctcggcatg tgtgtcgtcg 26820
   gatggttcct cgcctgaaat gagattttgt tgtcgcatgt tcctcaacat ggtctcccat 26880
   tccggatttg tggccgctat aacgtccaag aagctctcga gtccacccac acgagctctg 26940
   aacgcagatt gcttcgaagc caactcgtta tgcagctgag tgacttcatc atcccgtcgc 27000
   tgaccataag acaatgtcgc tctcggaaca tcgttgacgg aaccaatccc caacgtccat 27060
   cccttttttt aaagacaatc ttaaaacaaa aaataaatat tgttagtaaa aatttaaagt 27120
   taaattaaat gaataataaa aaattaaaat tttagaaaat ttacctcctc gtaaatctta 27180
   tccacttcaa gtgtggataa ggtgacgagt aatccgtcgg tggacagctg gatctggtgg 27240
   tcttcaaccc gagcaaccaa gtcgttgtag atttgcttgg acttgccatc tagaaatacg 27300
   cctgccttgt tcttgtgggt cctctcgtaa agttccataa gagacgggag atgtgccgtt 27360
   tctttggcct taaaaacatt taagaaagtt agaataaaaa tatatatata tatatatata 27420
   tatatatata taataaatat atatatatat atattattat ntntntnttn ttttattatt 27480
   tttttttttt tttttttgaa gaaaacatat ataaaccgaa atcgaatata tacactgcga 27540
   tgcaatcaca cacttacaac acccaatttt tccatttaca cctctagaca cacaggtccg 27600
   tgtctaacca cctaaatgtt ccgatcctac ctaaacagtc ggatgaacca tgtctaccct 27660
   aatctctcca ttgtttttgc acatgtatgc acatataatc agtgtgtaag aatgcatgga 27720
   gatgaaataa aagtgtacgg tgtaggtgtg gtaccaaact attgatgagt ctggccattc 27780
   aggattatta aagagtggta aaatgtggta aagaaaatcc tgaatgtgta tatggtgtac 27840
   cgtttcctga tgttgattcc tggtcaaaag aaattaaatt cattaatggt caaaattatt 27900
   tgagtcgatt acaattcacc gagacttgat aaaagattta aggagaggtt gcttggtcaa 27960
   gtagttcctc ggtttgtctg cgctaacagt cctgaaaaat ggtcaatatg aaatgtaata 28020
   cacaacacac aaggaaatag tctaataatc atcacagggt ctgagaaaaa cacgtagtag 28080
   tttttt 28086
<210> 13
   <211> 10653
   <212> DNA
   <213> Brassica napus
<400> 13
   aggtcgagta tctttgtagt ggtcgagttg cgggcgatca gattgttctt atttgaacca      60
   agagtcaaga atgccactag gccatggttt gacaatgcct tagattgatc agatggatgt     120
   tttggaacca agcacgacgg aatagaagca cgacgggaaa actcgaaatt ggacggaaac     180
   cctaatttcg gtattatgga agtttctgat caagccgaag aatcaagaaa tatttaccgc     240
   caaggtcaga gttcagattg gagtttatta aaaatattca gctcatcaga atgggagtag     300
   aaaaatattt gggattgatc gcgggtcaga aatttaccgg aatgaccgaa atcagaccaa     360
   tggaccgaaa agctcgaggt ggctcgttgc atgggttcag aacgtggtgt aaaccatcta     420
   acaagctgag tgtctacaga agctcgaggt gtcatcgtac atggaagttg tacatgcagc     480
   ctgacatgta gaagcacgag gtggatcgac caagcacgag gtggatcgac caagcacgag     540
   gtgtctccgc gcatgcaacc gaagcatgct gatcgacatg tgtgtgctgc tgtggcgcct     600
   tgcatgagtt ctagtcatgc agcctgacat ctgggaggag tggtggcgtc ctgcatatgt     660
   cctggacatg aagccagcca tgtggagcac gaggtgccgc cgcgcatgtg tccggagcca     720
   tgcgaagcga cacacaggct gccactaacc tgaagctgat tggttgctgt cttctataaa     780
   tagcccacga ccccagctca tttcatcaca tccatacctg tacaaaccac cttagaaacg     840
   tgagagaaaa gtagaaaaag aaagcaagag tttccgatct atttcgagaa ttttagagag     900
   attgcgaggt cagttctcta ctgatttcga gtcagcgcct agggacggtt ctgtccaact 960
   gaattcgtcc agaccactca gttcctttga tgatcaacta gatatgctgt ccggagttag 1020
   ttcagttcta cgggttcaga tcagtcgaag ttttgctcga tactccgccg ggaagtccga 1080
   agaactgtcc agaagctaga ggaggttctg tccgagtcca tatcagcctg tcgaggcctg 1140
   tcagtttctt catggtgaag ccgaggttgt gtccaagaca agatcagtcc agtccactcc 1200
   agtcatgtcg tcaattgggt tttggccaag tcttctccga tcaaccagct gcttatcagc 1260
   aaagaacact gtgagttatg atcaattgat tgctgacttg ttttcatgca ggttcccgtt 1320
   acttagaagt tggatcatgg caggaggtcg gctctaactg agtcacggtt tgactagtta 1380
   ataattgagg ttatgttgat tgagttgata gcatgctggt tattgcttga gaaccgtagt 1440
   agcatgctaa tggttaggtt gattggttag ttagcgaatg cggaatgctt agatgatatc 1500
   gctaagttgt ggatagttag atattctgga attagttttt atgctagatt ctggaatatg 1560
   attgattctg ttaatttgcg attaatacta ggaaccttgt gttattttac cgggtttagt 1620
   attagtcatg tattggccat atagcatttg tgtaaaccac aatgctatgc atgtttgagg 1680
   tggattagtg tttcctcgac ctcgtaccca gcgggtttaa ggttactctt ccaactccgt 1740
   tgtccttttt gcaggtcgct ttaggtaagg atgatcggat agcttggtgc tcgacgttag 1800
   gaccgccgga gtagatttca tgccttttgt aaacggtatt gcgttatgtg ttttgttggc 1860
   tcgatttggc attaggccgg gcccagtctt gaattatttc aatgtatgga tatttcttga 1920
   atcaataaag taaatgtttt atatgcgctt catgagtact ctgatatctg actagtccgg 1980
   tctaacacaa cgttaggtcg tggtacgggt tgaaaagcct taggcctcga tctaacggaa 2040
   aacgctaact ctaggtacgg gttgcaaagc cttgtgcctt gacgcagcag gacgagttag 2100
   tggaggaact ggtcgaggtc gtggagtaaa ttttgtgact ctggccggat cgtccctagc 2160
   ccgtcacgta gcgcttccgg accatggtgt tgggttggac ggtcagtcat gttcttgttt 2220
   gattgttggc tggccgattg gcctttcatc tccaaccctt ggtgtgggtc atccgtcggt 2280
   catgttcttg tttgattgtt ggccggtggg tcgacctata cctaggacgg ttcgggggtg 2340
   ttacactaat catgtaagct cattcagaag aaagtttata gtttttttat atagatttta 2400
   gttttagcag gcaatgttca tagattttct tgcaaaacct tgtccacaat acgttttata 2460
   cttcttatcc acaatttatt ttattttatt ttaaaatatt gatttttatc caatatttct 2520
   cagaagtgct tcggactcat cagatcactt tccgataaac agttccgaca aaatttgtta 2580
   atggaacttt tcacctaatt gtagaataca aaatcttgtc cacaaagtta aattaagggg 2640
   gtgtattcaa tttaacattt tatgtgattt gatttttaat gggattttag atgatttcaa 2700
   taagttgcag agatttatgt gagttttgtt aaactactct agaatatcat ctaaaaccat 2760
   gagatttgag ttttaatttt ttttaactaa gaaactctac ctaaacaccc taaaatcatc 2820
   tgaaagcttt aaaactccac aacttaaaat attttcaata acaatggatt taagagtact 2880
   ttacgaaata tcaaattcaa taacattgta ttttaaatga gtttttaaaa ttcatgtttg 2940
   aataacagtg aatttgttat tttaatacaa atcacctaaa actagcagtg gaatacaccc 3000
   cgcctaaata ttcttttgtt cccttaattg tgttctcgtc tataactcat tcttgtaaca 3060
   tttgtctgta cacaacttac atgtccacta tttttgtatc cataatgttc gcctgtccac 3120
   ataatgtttg tctatccacg taatatttat ccaactgagt aaccataaca tccttacatg 3180
   gacacgaaag catcaacaac cagcgaacat gtatttgtgg acatgataga atccacatcc 3240
   atgaaatatg gatgactgta acttgctaaa ctgttcattt taatgtaatt gttggattaa 3300
   cagttttttt acgatcttgt ggtccttatg gaagtccaac tatcaaaaaa cttaatctaa 3360
   taaatgtcta aaagctaact ggaaaaacaa cacaaacaat attccaactt tctgtttcgt 3420
   ttcagtaaga gcaaaatagt ccaaaaactc tctcaatttc cgtgaatgta tgtagtgctg 3480
   ggttcgcggg tcaacccgcc ccgacccgcc ccgccccgcc ccgggtcgaa tcattttttc 3540
   gattcaaaaa ctcgacccgc ataacccgca aacaaaaact tttatatccg cacccgcccc 3600
   gccaaaaccc gcgggtaacc cgccaaaccc gcgggtaata ttaattatat taaaaatagt 3660
   tattttaatt aaaaatgatt attttctaat tatataataa ttattttaat taaaaataat 3720
   tattttttat ttatataata gttattttta aaaaatacta ttaaaaaata tatttataat 3780
   taaaattata caaatattta ttgtttttta tatattttac gaaaaaatgt tttttttcaa 3840
   aatttttttt tttttaattt tgcgggttgg cgggtacccg cgattcaaat tcggctgacc 3900
   cgcacccgcc ccgctcaaaa taatcttgac tcgcacccgc acccgcgatt taaaattttc 3960
   aaatggttcg acccgcaccc gccccgcggc ggatcaaatg gggcgggacc cgcaggcaat 4020
   gattaaaatt tccagctcta aatgtatgct acaagtggaa ggtagttttg ggtgcaaaga 4080
   aaacagccta ttaagtaatc aactctttaa tatattggga cgaatgagat gtttgtaaaa 4140
   ttatttaggt ccagatactt ggcgcaattt aagaaggctt ttatatattt gggccgaaaa 4200
   ggttcgccca ttacttaaaa aagcgacaac tccgtgacat attgttgttg tgctgggacc 4260
   caaaaacggc gtgcattttg tcgactttca gtggaactgg ctttttcttt ctgtccaaat 4320
   caaaaaagtt ttaaagatcc ttttgattgc aaccagagaa aaagataaca aaacttccac 4380
   tttttgtaac gtaaatacat taataaaaaa aaggtttcac gagtacattt taaacttaaa 4440
   gcagaaacaa ataagtaaaa gagaaggagt gtttattcct aatagagcta ggaagaaaag 4500
   ttaattgatt ttagatttgt cagaagcata aacgtagaga tctggatctg tctcgtagaa 4560
   gacaatatca ccagtgtcac tgacgtaatg atctttttta atacttgcga ccaaactctc 4620
   caccaagtga atcggtattg ctcctgacgt cttcggctct ctgtagtatc ttcctaacac 4680
   atgtttagct gctctcgtct gtccacaatt cattaattaa attagtaatt aatcaccatt 4740
   taatcaaatg aaactagaga gagagaaagc tagatcactc acggcatcga ccaagtgata 4800
   gtgagggatt tgtgggaaaa gatgatggat cacgtgagtt ccaatgtcgt gatggatgtt 4860
   gttgaagatt ccgtaatctc tatcaatagt tgttaatcct ccacgtaaat aactccattc 4920
   ctattattgt atgcaaaaca tcaaaaatta agattaatca atactaacca ttattgcttt 4980
   ctgtacattt ctttttaaaa attgatttaa ttaccttgcc tctgtaccaa ggcaacttct 5040
   catcgtgacc atgatgatgc aagtacgtga cagcgtccaa ccacatcaca aagatctgaa 5100
   aattttccaa acccttatgt caaaaaacaa atttattatt aataatatat aaatttcttg 5160
   taataatatg tgaaacttac aatgtaagga acgccataga ctttgagaac tgtgactgga 5220
   tcaacgagga acgatagata aacaagagtg gccaacatta tggaccagca agtagttgaa 5280
   gttgcaataa gcttcctctc gcttggagca aataaactac tgtatgggtt aaaatgtgac 5340
   ccttcttttc caggacttct gtaccactgt agttaaaatc caatcaaaat taatttatat 5400
   attggcttaa aactcaaaat ataaaatcat ttgtaatttt aagaaaaaat agaaattgta 5460
   ttttttttac cagatagatc gggtaagcga gcatgggcag agggacagtg tatctgagca 5520
   tccgagtact atggggcaag ttcttgtaca acttttctgg caactgaaat acataattat 5580
   aattaatatg actattacta ttactattac taattactat tacggagtag tacttactag 5640
   tattaaatat tcattgaaaa tttgtcattc tggttatgta ttcgtattaa ttcatgtgtt 5700
   tataagtttt atactaatag ctttcaagat tgcagacaaa agtattacga aaacgccaaa 5760
   actgaaaagg aaaaaataac gaagaaagta aacggatttc gaggagaatc atgttatgct 5820
   aaggactcga atataagtgg tccatcgata aagttaggta ctataaaagt atagattttt 5880
   cattttctga gttactgcgt aacctctaaa aaaaactctc taaatagagt ttactctaaa 5940
   tttaaagttt caaagtggtt ttcttcgaaa acaaacttca aacataactt caaaattatt 6000
   tgtattttac acaatgatcc ttatttgtta taactaagag catgattaac ctgggattct 6060
   taggatgggg ttcttaccgg aagttaagaa actgtttctt aacgtttaac taaaactcca 6120
   ctctaagaac tccgggttaa tcatggtcta atataaatcc ataaaaaaaa ttataaataa 6180
   ctagcacata tataaaaata ttacagtaat attaattaat aaaaatttac attaaatata 6240
   taaaattata aatagaaata tataattaaa tattaaacta gaagcaaaat accatattat 6300
   ttcataaaat tattttcgta atgctccatc ttcggttaca caaaatttgt ttagacaata 6360
   attttagagg ttccagagca aatttaccag attattagta ttgttataat atttaaattt 6420
   tctaatagtt atgtcttcat gtatcttatt ttaaattttt tattattaca tttcttttgt 6480
   aatattttgt tgactaatta tagtcttaaa tattataaat cttatttaac atttttatta 6540
   cttttatgta taaaatttga atttataaaa acaaattgga aatatttata atatataaaa 6600
   aatttaagaa ttaaaacgat aaatgaaaaa atacttaaga attataaatg taacgtgtaa 6660
   ttaattataa tgatcaaaat gcaaaaaaaa aacttcaaat ttgaagtttc gaagttcatt 6720
   tttgaaaaac aaaaaaatct ttatatttga agttataaaa tttttttttg agatagatcc 6780
   gagaacatta attaccgctg aactattaca cttgcaaatt gttttttact acagctagaa 6840
   aacagatctg acaagtggcc ggtctgacct cagactgaaa acataaacta ataaaataaa 6900
   catatagaat cctaggagta tgattattgg ggtttttagg aagaggttct tagcggaata 6960
   taagaacccg tttcttaact tttaactaaa aaaattaaga acgtgttcat aaaactctta 7020
   tttaaaagct ggttcttagc ttttttagtt aaaagttaag agacaggttc tcatattccg 7080
   ttaagaaccc caccttaaga acttcaataa tcataagaac ttagacataa gaatgaattc 7140
   ccaaaaaaga acaaataaat aaaagacaag agaaacaatg agagtaggaa agattaccgg 7200
   aacccaagac tcgtcgtttt caacatggcc atggttctgg tggtgtgtcc gatggcttat 7260
   tctcctgcaa accccaatta caaaagttat gtatttattt atttttgtga aaatgaaatt 7320
   gtctctataa tgatttaaca atctcactca ttttatattt attttgtttt tttagttgat 7380
   atatttattg aacaactaac aatagagtgc tctaacaatc ccattctttt ttttgagcaa 7440
   aaaaacattt gatgcttttt actaataaac attgtgcaga aataagtaaa aaaaactata 7500
   aatcctcagg aaattgatgc atgtaagtct ttttcgaaga tgtttgaagc tgatgtaaac 7560
   aaataacaat aagtgaaaac ctaaaaaaaa atcaaaatct aattatactt aatgaactaa 7620
   gaaaactcag gaccatagat aggcgcatat catttaagaa aaggtttgga ttctttttca 7680
   ttggctgcta aagatttgat gcttttgaac agaaaaagca acctcatata gtcacatgca 7740
   ttgtttaata ggattcttat ttaattataa aattgctact ctagcaacaa aaaaaagttg 7800
   gtagcttcca gttaatactg attacagttt cctagcattg cacccaagaa taacaaacga 7860
   aaatgtaaga aatacgaaaa caagtactaa taatttacat ggaaatagtt aataaatgac 7920
   ttaccaacca tggtaaggaa cgaggatgaa tgaatgaaga atgtgaccaa ccacactgtt 7980
   cagcagagga atgtctgaga aactcccatg tccactgcca ttattcaatt ttattttcac 8040
   atcattattt aacataaaaa cgtatttatc atttagtgca caatttattt taacttttct 8100
   acatttgttt taactcaaac tctttaacaa ggtaacaaat ccggtatatg acgtgtcact 8160
   tgtctaaatc acaaaataga ttggaacaca aaaagaagaa aaaaacaata tatttttctt 8220
   gtcaaaaaac aatatatttg ttgccaaaaa aataaacagt atattttctt gatattatac 8280
   tatactataa ttataattaa aagttccggg gatctagaga aagagaaaca aaaattgaga 8340
   acatcaaaac gtagatccat aaaatgcgga aataatatta attatagaaa agaagatatt 8400
   ttgttacgag tctgacgact gatgagtgac gatgcttgaa cattgatgaa gaaaaaaatc 8460
   ttagatccta tattttcttt tattttttaa taattaaaca tgaaaaagta acctcaaaag 8520
   aattaaatct tgaagtcagt gacgatactc atcgaacgtg cattcaagaa attaataaat 8580
   tgaacaaaaa gagacaaaat aattaaaact gaaaatttaa tttaccagtc gtggccaaga 8640
   acgaagatgg cccagaaaag ggttccttgg gcaacccagt agagtggcca gaggaaccag 8700
   ctatcaaaat acacggcggc catggccaga gccgcgacgg cgaaaatgtc tctggcgacg 8760
   tagctcatag atctcaaagg actcttcacc cagcaatgct taggaatcgc cgcccttata 8820
   tctccgatct taaacggtgg ttgtgcgctt ggatcaaacc cttcttcctt ccgggcaccg 8880
   gaatctccgt taacattgct gcgctggtcc atagcaacaa ccatcgctgg agagagagat 8940
   ttggacgaag tttctctctc tagatgtgtg gcctttcagt gaaatgtggt gaataaaggt 9000
   ttgatggatt ttttgggtgt gtgaggttgg cttatataaa gggagaagat gtatttatgg 9060
   acattgagaa aatattccaa attgtttttt aatgattaat aatttatttt ttatttatca 9120
   aaagaataaa aatggtaatt tagctgtaac ttttgtacaa tgggttgggt gtataatgtt 9180
   ccaaaaaaaa gggttgggtg tattactctg ttacgtcgtt caacgcaatg aaaccaaatt 9240
   ggagtaaatg tgtttctttt ctatttttag attttccttg gacggaagga ttgtaccaaa 9300
   taaatttatt tgtgtttctt actctagaat caaataccat atgtagatgc agtgaaatgg 9360
   aagacaaaca taacgatcct ctagcatata tattttgttc cctaaaattt tgttgattat 9420
   ttattgacta ggataagatc accttgggcg ggatagacat cgtttatata aagtggttaa 9480
   gaaaatacat cgtgtatata aattattttt acatattacc atttatttta catgaaataa 9540
   taaaataata aatatatatt aaataaattg aaaagtctat aactattatg tatataatta 9600
   agttggtgta aacacataaa tcaaaacaaa cactcttttc tatttaaaat aatattgaga 9660
   taaaaaaatc taaaaaatca attatatcta tggtatataa ttaaatttaa atgatattaa 9720
   catatagaag tatattttaa aatatctatc cgttaaataa tgcttcatac tcatatagtt 9780
   ttatgacaat ttgtattttt taaactattg aaaataaaat tttcaatttg atacttttaa 9840
   tagttttagt aatttataac tgtttttaaa aattcaataa aaaatttgaa attaaaatat 9900
   taagttctca atatttcttc aatggaaatt tcaaattaaa ctattatgtt cttatatggt 9960
   atatagttta atttaaacga tagtaaaaac atatttttaa tatgaaaata tattaaataa 10020
   gacattttat tcatatgatt tttatgatca tttatatatt gtataacaaa aaaatttaag 10080
   ccactgatca caaaattttc aatgtaatat ttttaacagt tttagtaatt tatagttgtt 10140
   taaaaaaatt caaattataa catataagaa aaaatctaaa tttttattct atgattaata 10200
   tgattgttta atttattttt taaatataaa acaaaaaata atagaggaaa cacaaattgt 10260
   tatcaatttt ttattattca aaatcactaa ttgtcatata tatattgatc acattaaata 10320
   attttgtagc ttttattcaa ggaactaaat aaaaaaaatt ttggtacatt aataattagt 10380
   tttgtagtta ctttaatgag aactactgtg tatatttaga ttgaccaact tatttctgta 10440
   agtaatccga gaaccattct agtgattaga gatgacaatt atggatctgg accgcgggcc 10500
   tggcccgtaa aggactgtcg cgggacggta ttgggacgag gttttctagg cccgaaaatt 10560
   tgcgggcttc gcgggacagg tctttacggg actgggcctt ttgcgggatg ggccgaaacg 10620
   ggtcttgcgg gattacatgg acccgcattt ttt 10653
<210> 14
   <211> 23648
   <212> DNA
   <213> Brassica napus
<400> 14
   cccattcgaa aataaacatc aaacccaaga aaccctagaa acagaaagac accgtcgcca      60
   tcgccttcaa agaaatataa aaaattatat tttatattcc attaaaaatt attgaccaaa     120
   aaataaacta ttaaaatttt aaaccgctgg tacgtacatt gctaagacat agattaatag     180
   atgagcaatt ttccaagctt atctcgaagc cttggagaag taagttgaag agatgaactc     240
   agctacggca aacaacaata ccactacatg caatccaaga gtcacctcag aggttcaaat     300
   cctaaaatag cagagtacat ggtactccga agagaaaatt ctgccttgac cgtagctagg     360
   ggaactagac gaaggttctg aacccgcaat caacccatgc aaactctaga tcgcccgctg     420
   tgacatgaag cataactctc taaaatcatg tgggaactgg agagcatgcc atagatgctc     480
   aagccacgaa tactgatcgt gcaaacttac atttggacaa cgatggcaca gacacggaca     540
   catgtgcata gatgcatgaa actttttgaa tttttttttg aatttttttt ttgaaactat     600
   tttttagctc taggagacta tatttgaaat atttagatga aatatttagg taattttaat     660
   ccttgaagac tatatttgtg acaaaaggtt ttttagggtc agtctagaaa atttattttt     720
   ctttttaacc tttgaaattg tatgtttttc agtgtataaa tggattaata actaaagaag     780
   agtttcacga gtacattttt aacttaaaaa caccaataaa taagtgaaaa aagactggtt     840
   tattcataat agggctaaac aaaaaggggg aagaaagttt aattgatttt cgatttgaca     900
   gaagcataaa cgtagagatc tggatcagtc tcgtagaaga caatgtcacc ggtgtcactg     960
   acgtaatgat ctttcttaat acttgctacc aaactctcca ccaagtggat cggtattgct    1020
   cctgacgtct tcggttctct gtagtatctt cccaacgcat gtttagctgc ttttgtctgt    1080
   cacattccat ttgttaaaat cagtacatta gtaattaatc accctttaat gaaatggttt    1140
   agatgaaagt agcgagagag tgagatgact cacagcatcg accaagtgat agtgagggat    1200
   ttgtgggaaa agatgatgga tcacgtgagt tccaatgtcg tgatgaatgt tgttgaaaat    1260
   tccgtaatct ctatcaatag ttgttaatcc tccacgtaag taactccatt cctattatta    1320
   atcacaaaac atcaagaatt aagattaatc aaatactaat aatttttttt tgtgaaacat    1380
   cgtaatctct ataaaatatt atttgagaag tcggttttct atgtatcgct ctcacgttaa 1440
   ctctcacgat agttgattac actaatacac ttaatgaatt aaaaatatta catttaaaat 1500
   actattattt atttttttat ttagtttcct ttttaaaatt ttccaaaaaa acatatacat 1560
   ataataaaaa ggaatttttt tataaactta aaaaattata ttttacttgt ataatattaa 1620
   tttcaaatac aatctcactt ttgttcactg cttattttta agagttatta aaaaactaaa 1680
   attaaaatta aaaaataatc attgtttgat caaatagtta caaaataatc acagttttta 1740
   aatgttatgt ttttatgttt gtagaactta atggaaccat aagcaaaata ccaaagcaaa 1800
   tatgttttca tttttaagat tatttaaaat aaatttcagt ttccattcaa taaattaaat 1860
   acataaagtt atttagaatt tataaaatat tttaattact gtaaaatatt aaccaaatgt 1920
   tacaatttag ttcttttgta aaatttatat atatatatgc atgagacttc agaatattat 1980
   cgttatatta atttatgtaa tttagaatca gacactttat tttatttttt atttcatttt 2040
   aagcacaata tatatattaa gttatacata atctttataa aatatattta aagttctaag 2100
   acaacaacca cctaaatgaa aataagaaat taatcaaaat tttaatatag ttaaaataaa 2160
   aaatattaca gttgaattct gagatgcaat ccaatttacc caaatgataa ctaaatcgac 2220
   tgtaaaaaca acaaaaccga ttagatataa catatataaa atcatattta taattaaagt 2280
   aatataaatt ttattaatat aaatcatgca tacaaattat aaactaattt aaaattaaaa 2340
   ataaataaca attaattatt atagtatatt tactttagaa atatttatat ccgtacatga 2400
   gcacgggaaa atcacctagg agttaattta attaccttgc ctctgtacca aggcaacttc 2460
   tcatcatgac catggtgatg caagtaagtg acagcgtcca accacatcac aaagatctga 2520
   atttcaaaag tttatgacaa aaacaaatca tatagtatat atattgaata ataaatatat 2580
   accctttatg atatactaag aaacttacga tgtaaggaac accgtatact ttgagaactg 2640
   tgactggacc aacgaggaag gaaagacaga taagaatggc caacattatg gaccagcaag 2700
   tagttgaagt tgcaataagc tttctctcgc ttggagcaaa taaaccactg tatgggttaa 2760
   aatgtgaccc ttcttttcca ggacttctgt accactgcag taaaaccaaa gaaaaaataa 2820
   tttatattgt tttaaaacac aatctaaaat gaattgtggt aagttttagg aattaaaaaa 2880
   taccagatag atcgggtaag cgagcatggg cagagggaca gtgtatctga gcatccgagt 2940
   actgtggggt aaaatcttgt ataacttttc tggtaactga aaggaacaat taaaatgaat 3000
   tttagtaatc aagattaagt acttgcaaaa atagtactta gatatgtatt gatatatata 3060
   ttcattgcat gctatgtgtt tataaacttt tgtttttatt attttttgtt agttttcaaa 3120
   acacaggcaa actattacga aaacaccaaa ttagagaaag aaaataataa tagtataaaa 3180
   gtaaatgcat ttagaggagt aagaaactca aatataaaag catttgcatt agtgagtttt 3240
   tgacgagatt ttatcacaaa ttatattata ttaatttata attattattt ttgaaaattt 3300
   gaaaaattta taccaaaata ttttatttga aagactttca catgagtttc gcataaacat 3360
   gtctttcatt ttttttaaaa aaactcttta attaagtaat aataaacttc ttccgtttca 3420
   atttaattgt cgttgtaaat taaaattttg ttttaaaata agtatcgttt tataatttca 3480
   atgtaaaaat tatgaataat attttctagt ttatttttta ttggttaaaa tattgttagg 3540
   tgtataatta gtgatgtttt tattttaaaa atggacaaaa tattttattt tttgtaatct 3600
   atgtgtataa atctaaaact gtaactaaaa taaatcggag gaagtaatta gaagattcac 3660
   cgatacaaat aggcgtggtc cgttgtcaca tactattatg tatattttat tttacaaaaa 3720
   tgttacttct ataaatcgct aaaaagaatc aattaccggt taactgtgac actagcaaac 3780
   tgtttttact acagctagaa atcaaatctg acaagtggtc gttctggcct caaatttcga 3840
   aaaacaaatt attttgacaa agaaaaatag aaattattaa agagggaaat gttaccggaa 3900
   cccaagactc gtcgttttca acatggccat ggttctggtg gtgtgtccga tggcttattc 3960
   tcctgcagcc tcaaattatt aaatatgtgt ttacataaaa attaaattgt ccatggaggt 4020
   gattggttgg gttttatcta cctactttag ctttattttt ttctaaatca ttaaacttta 4080
   ccaatcatgc tttacgttta cttttcaaaa ttaaagtcta catcaaattt ctattaattt 4140
   ttaccaatca tgctttaact ttaaaaataa agctacagca aaaaaaaaac caaacatttt 4200
   tcttatgtat tttagttaaa caacttacat ctttcattta taagctgtag aaactgtaag 4260
   aacaaaaaat atctataata ttaaataaat aagataatca taataaaaaa acatctataa 4320
   atattttact ctaattttgg gtgcttttaa attattgaaa tattttaaat aatatagatt 4380
   atttacatat cacattttaa ataacagtaa actttgataa ttttaaaaaa tattaatata 4440
   aattatttta agtgataaaa ataataatta ttttatatat acatgcatca catattttac 4500
   atattttatt ttaaaatatc tgcagcctat agcttacagc tacaacaaat ttaactacag 4560
   caaaagtctc tgcaaaaata atcaacagta acaactttac aactacaacc aatttatcta 4620
   cagctaaaat tctacggcca cagtcgaacc aatcatcacc tatatagtgt tgctttcatg 4680
   gcagattcta acaatctcac tctatttttt ttctcttttt ttttgatcaa acaatctcac 4740
   tcttttaagt tttaagttac tagtaataaa ttgaccaaaa atagtttcca gtaataaatt 4800
   atttttattg ccaggaataa gttacaataa tgtcgcagca aaataatgca tgtaagtcta 4860
   ttttcaaaga tatttgaaga tgatgttaca tttaccaaac aaaaaattat gatgttacta 4920
   caggaaacca tttttattgg aggtttgaag tcagtttctc aatattaaaa atagagaaat 4980
   agaaaagaat aaaagataaa aggagtaatt tcccaataaa caaagtcatg aataattctt 5040
   caaatatcta acctttaata attggtttaa tattatttat aaaatgaata tttaattata 5100
   aataaattag ctgttgttca aaaaaaatta taaataaatt atattcaaaa tactaatgac 5160
   cagatatagg cccattgcat ttaataaaag ttttgattcc tttttccttc gttgctaaag 5220
   attcgatgct tttcgtcaag aataagaaaa gctacctcac atatatagtc atactttcac 5280
   atgcattatt taattataaa attggctcta gccaaaaaaa aaaagaacga gcaatgaata 5340
   gattcttgca ccaagtaatt catttaacat ttaaaccaaa aaaagtataa caaatgaaag 5400
   tttaataatt aataataata ataaaaaggg ttaataagtt gacttaccaa ccatggtaag 5460
   gaacgaggat gaaggaatga agaatatggc caaccacgct attcagcaga ggaatgtctg 5520
   agaaactccc atgtccactg ccattattca attatatttc acatcattat tcatcgtaaa 5580
   tatagtatat catttattgc actatttatt taaactttcc atgtttgttt taaaagcttc 5640
   aacaaggtaa tgacgtgaca catttctaaa tctcgaaata gattggaata caccaaaata 5700
   acaaagaaac aatattatct ttcttgtttt agaaaaacaa tagatattct tgattttata 5760
   ctttaattat aagttgagag atccataaaa tgcggaagca gtcgtaatta tagaaaataa 5820
   agatgtggtt ttgtaacgag tcgtacgacc gatgaaaggt ggtggaacaa tgatttaaaa 5880
   agaaaatcta aaaaaaaaaa tcttagatct tcaaaaaatg aacatcaaaa gaatcaattc 5940
   ataaagtact gacaatactc atagaacgtg cattcaataa atcgatgcaa tgcaaaatgg 6000
   aagaaacttt accagtcgtg gccgaggacg aagatggccc agaaaagggt tccttgggcg 6060
   acccaataga gaggccagag gaaccagcta tcaaaataca cggcggcaat ggccaaagca 6120
   gcgacggcac aaatgtctct ggctacgtag ctcatagatc tcaaaggact tttcacccaa 6180
   caatgcttag gaatcgcagc ccttatgtcc ccgatcttaa acggcggttg tgcgctcgga 6240
   tcaaaccctt cttccttccg ggcaccggca tcttcgttca cattggtgcg ttggtccata 6300
   gcaacaacca tcctgggaga gagagagaga gatttggagg aagattctct ctctataatt 6360
   caaaaaaaag aaagtgtggg aactggaatg tggtgaagaa agggttcgat gtattttgcg 6420
   gtctgtgaag tttgtttata taaaggggaa ggaagatgtg tagtctgtag acattgagat 6480
   gctcaaactg tttttattaa ataattatat atttaaagaa taaaaagggt aatttgctgt 6540
   aattttaaat gcaatgggtt tgttattttg ttacatcgtt ctattcggtg aaattaaatg 6600
   ggaaattgaa ggctataacc acaaaaaaaa cgtaattcac cgtctagcca tttaacctaa 6660
   cgatcttata cacgctgtta caaatataaa ataatactgt aatattccta aaacacaacc 6720
   ggctcaacct gctacaaaaa aataattaaa tattttaatt attcaccgtt gaaaagtaac 6780
   tcgtgtctta cccttgttca catcttcccc ttttaacttc tctggtaatt ttgctgcagt 6840
   cgaacggtct ccggcaccgc tttcttccat cgcctccact ctgcatgcaa tcgacatctt 6900
   ttccatctct tccgtcctct gttttcaatc ttcttcggcg ttacagttct tggttaaggt 6960
   ttcagcgacg gtagtaagaa accctttcta cccaacttca ccacttcgat tcttgtcaaa 7020
   tctctgaacc ttccgtaagt tctcattcta tttcgtagat tctcttcact gtgtcgtcct 7080
   ctgtgcttat tttctttcaa attggggctc gtctcataat cagagtgtta taatttctag 7140
   gtctatatca ccggcagatg tgaatatcga tcgtccccga tacggaatta gtctctccca 7200
   ctttccaatt tgatttaggt tttggtgttt cccgggttgt gtgaggttcc ttgaatttga 7260
   attttcttcg tagaggatat gcaacgtatt tagtttagta ctttatgtat ttcgtgtgga 7320
   atatcattgc ttacaaaggg tttgtcgata atacatagta tgttattttg attctccatt 7380
   ccatttggaa tgtaatagta cacttaccca attaagctat tccattgtgt aacgcatcaa 7440
   ttcattcctt tgtttgtaat gtacttatgg agttgctcat tctaatttat ggttcccctt 7500
   tatgttcttc ttctttctta atcgtgaatt gatgtttctg tatcagtgct ttttaaaaat 7560
   agtgtatgaa tatcgactac cgtgtggaat atcgtctctt gtacatagtc tagaaattat 7620
   gctttcttgt atggaatatc atctttatgt catagttgtc ttgtgtttta tttgcaacat 7680
   tacatttggt ttatctacat catgtcacgt tgaataaaca ctacaagaaa acacatgctt 7740
   aacgacgaaa attaacgagg aaaaacaatc ctcgtaaatt tgcgtcgaat ttacgacgaa 7800
   tttacgtgaa aaactaaagt catccttatt tcctcgtaac gtaacgacaa aactgtttcg 7860
   tcgtaaagtg gatgtaattt tacgagtatt ttacgaggaa aaactatttc ctcgtaaata 7920
   cgacgtaaat tttgcgtggt atttacgagg gaatagttta cgtgtattta gcgaggaaat 7980
   ttttgaatcc accaacttca taggtgttac acgttttttt tgcccaccta attaattttc 8040
   gtcgtaaatt catagcaaaa ttacaactac cagattcgaa ttttcctata aatatggatg 8100
   tttgaacatc attttaaaca caccaacaac aaaaaacgtg aaagaaaaaa aatggctggc 8160
   tccgggacta tttacgagtt gcggaagtgg atgtatatgc atagagatgc taacgggaga 8220
   gtgacgaaag aataccttgc gggtctggag acatttatgc atcaagcaga ttcaacaccg 8280
   ctcgcccaag aaagtggtaa gatgttctgt ccttgtcgga aatgcaacaa ttcgaaactg 8340
   gcaaaccgtg aaaatgtttg gaagcattta ataaatagag gtttcacggc aaattactat 8400
   atctggtttc aacatggaga aggttttaat tatgatcaga atgaagctag tagtagtaat 8460
   agcaattctc aggaaaaaga accggttgat catcatttgc ataatgaaca tagttaccat 8520
   caggaggaga tggtagatta tgatagggtt catgatatgg tagttgatgc attcgtagct 8580
   catgatgaag atgaagaacc taatataggt gcaaaaaagt tttacgaaat gttaaacgcg 8640
   gcgaatcaac cactttacag tggttgtaga gaaggtctct ctaaattgtc gttagctgct 8700
   agaatgatga atattaaaac tgatcacaat ctacctgaaa gttgcatgaa cgaatgggcg 8760
   gacttgttta aagagtattt gccggaagac aatgtgtctg ctgattctta ttatgagatt 8820
   cagaaactgg tttatagttt tgggttgcct tcggagatga tagatgtttg catcgacaac 8880
   tgcatgatct attggggaga tgatgagaag ctagaagaat gtcgattctg caagaagcca 8940
   cgattcaagc cgcaaggacg gggacgtaat agggtaccgt accaaaggat gtggtaccta 9000
   ccaattacag acagattgaa aagattgtat caatcagagc agactgctgg aaagatgaga 9060
   tggcatgccg aacatactca gacggatggt gagatggctc atccatcaga tgcaagagcc 9120
   tggaaacatt tcaacaaagt acatccagat ttcgctagca atatccggaa tgtgtatctc 9180
   ggattatgca cagatggatt tagtccgttc ggaatgtcag ggagacaata ttcattgtgg 9240
   ccagtctttc ttactccata caacctgcca ccggagatgt gcatgcaacg ggagttacta 9300
   ttcttgacca tattaatacc tggtccgaac catccaaaaa ggtccctgga tgttttccta 9360
   caaccactga taaaagagtt gaaggatttg tggtcaacag ggttgaggac gtatgactgt 9420
   tcaacgaaga cgaattttac gatgcgagcg atgcttttgt ggaccataag tgatttccct 9480
   gcctatggga tgttgtctgg atggactaca catgggagat tagcttgtcc atattgtaat 9540
   ggaacgacag atgcgtttca actgaagaat ggtaggaaga caagttggtt tgactgtcac 9600
   cgtcgatttc ttcccattgg ccatccttac cgaagaaaca agaatttgtt taggcacaaa 9660
   agggttgtga gagacactcc tcctccatat ctaactggag aacaaattga agcgcaaatc 9720
   gactactacg gagctaacga aacagttcgt tggggtggta attggcatgt ccctcgtaat 9780
   atgccagatt cttacggtgt tcatcacaac tggcacaaga agagtatatt ttgggagttg 9840
   ccatattgaa aggatcttct tctgcgccac aacctcgatg tgatacatat agagaagaat 9900
   ttctttgaga acatcatgaa tacaatattg aatgtcccag ggaagacaaa agacaacata 9960
   aaatcgaggt tggacttgcc agatatttgc tcaagaagcg agttacatat taaaagcaat 10020
   ggacaagttc ccgttccgat attcagatta tcttcagaaa aaaagtcggt gttgttcaac 10080
   tgggtggcat cagaagtgaa gttccccgat gggtatgttt cgaatctctc tagatgtgtt 10140
   gaaaagggtc aaaagttctc cgggatgaag agtcatgatt gtcatgtatt tatgcaacga 10200
   ctactgccct ttgcatttgc ggagctattt ccaacaaacg tacatgaagc acttgcaggt 10260
   acgtagtgta ttatatcaca ataatttaca aaataatata tgactaacaa tgtgtttatt 10320
   ttttttgaat ataaaaggca ttggagcatt tttcagggat ctgagcacac gcactcttaa 10380
   agaagaagtt gaggaacagc ttcaggagaa cattcccatc ttattgtgca acttggagaa 10440
   gatatttcct cccggatttt ttgacgtcat ggagcatcta gctgtccacc tcccatatga 10500
   ggcattgctt cgtggacctg tacattacgg atggatgtat cagtatgagc gagccatgaa 10560
   atatttgaag ggaaaagcaa agaacctcgc caaagttgaa ggttctataa ttgctggaag 10620
   tttgacggaa gaagtttctc acttcacatc gtactacttt gcgtcaaaag tacgtacacg 10680
   gagaagagct ccaagaagat atgatgatgg tggtgttgcg ccaacatatg cagttgctgg 10740
   tgttccagac atctttagcc agattgggcg actcggtggg aagtctaaag aggtttggtg 10800
   gtcgagtgaa caagacgctc atagtgcaca cacctatatt ctactcaatt gcgaagatcc 10860
   attgatgcgt tattttgaaa ggtaacatat attgacactt cgaaacacat ataagtataa 10920
   ttaattgtat aattgcgaga gattcattcc tataaaatgt gattttacag cctatttgtt 10980
   tctcaagtcg aagaaacatt tcctggtata tccacaagtg acgtagacaa aaggaaagat 11040
   caacatttca ttaagtggtt gcggaatcag gtattaacta aaactttttt ttcatacatt 11100
   atctgtattt cattaacatt ctctttattt ttgcaggttg attatgacga cgacgatgca 11160
   gattattcta agtggttaca cgaagtaatt caatctccac ttgtaaaggt caccacatca 11220
   cagatgtatt tcacacgagg ctatactttt catacatatg actatggtag acagcgggcg 11280
   accagtaact atggagtatg tgtgaaaggg aaaacagatt tctacgggat cttgacggag 11340
   attattgaag tcgaatttcc agggatactg aagctgaaat gcgtcctctt caaatgtgaa 11400
   tggttcgacc ccgttgtcaa cagaggtgtt cggtctaaca aattcggtgt agttgatgtc 11460
   aacggtggac gaaggtacaa caaattcgag cctttcatct tagcttcaca agcagaccaa 11520
   gttagcttcc ttccataccc tcggatgaga gattcaggta ttaattggtt agcagtaatc 11580
   aaagttacac ctcgaggacg aatcatcagt ggagaagaac caccattgca agaagaacag 11640
   ataaatgaag ttgaggaacc tgaacaagaa attgatgaca tccttctcat tgatccgcat 11700
   aatcacgagt acgaagatct taccgatgat gccacagacg aagctgttga agacgagttt 11760
   aatgaaaatg atgatgtttc tagtgatgac gagaatgtcg atgtatccga ttgatgtatt 11820
   tgttttatga ataagatgag agagtttgtt ttatgaataa gataatgtgg ggtttgtttt 11880
   atgaataagg taatgtggga gtttgtttta tgaataagca aatgtgggaa ttgtggtttg 11940
   gaatggaaat aaagatgggg tttggaatat atgaagtaga aaataaggaa tataaggttt 12000
   ggggtttcgg gttttggatt ctagggattt aaacataaca gtcgttaatt ccacgtaagc 12060
   ttaaatcgtc gtaaagtcct cgtattccaa ctagtaaata acgacgaagg actcgttaat 12120
   tccacgtaag actaaatcgt cgtaaatacc acgtaggatg aattcgtcgt aaaaaccacg 12180
   taggatgaat cgtcgtaaat ataacgtaac ataacgagga aataacgacg aaacctaaaa 12240
   ataaatatgg aatatgggat ttggggtttg gggtttcagg tttcgggttt cgggtttggg 12300
   gtttggggtt tcgggttttg gatttcgggt ttcgggtttc gggtttttgg tttcgggttt 12360
   ggggtttcgg ggtttggggt ttcgggtttt ggatttcgag tttcgggttt cgggtttcgg 12420
   gtttcgggtt tggggttcta gggatttaac cataacactc gttaaaaata acgacgaaac 12480
   ttaaaattaa atatggggtt tggaatatat gaagtagaaa attaaagatg ggggtttggg 12540
   tttcgggttt cgggtttcgg gtttgggggt tggggtttgg ggtttcgggt ttgggtttcg 12600
   ggtttcggat tctagggatt taaacataac actcgttaat tccacgtaag cacaaatcgt 12660
   cgtaactacc tcgtaggatg aaatcgtcgt aactaccacg taaaatgatt taaacaaaac 12720
   actcgttaat tccacgtaag cacaaatcgt cgtaaagtcc tcgtaggatg aaatcgtcgt 12780
   aactaccacg taaaatgatt taaacaaaac actcgttaat tccacgtaag cacaaatcgt 12840
   cgtaaagacc acgtaaacgg atttatacat aaacccgtta attccacgta agtacaaatc 12900
   gtcgtaaata tctcgtagtg tacaaacttg gaaaaaaaag gaaaaggaga aaaataccag 12960
   attaacatgt ggcaagactt ccaacaatta taatacgtaa gtctcgccca catgaattct 13020
   aatatcttct ccttttccta tttttttcaa atatttataa tttgaatagg atttttttga 13080
   ggattgtgat ttgagataag gtgtgatttg ggagtttgtg tgtggtttga gagtgagagt 13140
   tgtgggtata tttataggaa agcaagcctc gttaattcct cgtaaagtaa atcgtcgtta 13200
   atacctcgta taaaaaaaca cgggcctttg tgattactcg caatttcctc gtaaaaaaaa 13260
   agacgggcct ttgtaactgc tcgctatttc gtcgtaaact tacgaggaat ttgcggcgat 13320
   atgtaatctt atatatacac ccgagcgctc attctttctt tcctctctac ttcctctcta 13380
   cttcctctcc atttcgtagc aatagtaagc ctctctgatt cctctctaat ttggttagtt 13440
   taggatagat taggtggtta gtatagggaa tttagatagg tttgcggatt ttatgttatt 13500
   tagtgttgat taggtggata atgttgggaa atatattgtt gatgttaatt ttaaaaattt 13560
   catttttttc ccaggttcga aaaggaagac ttactgccca ttacagagag atcttcggtg 13620
   agccgggtag tcgtttagac caggcctctt cttccgctcc cagttcttcg ggccaggaga 13680
   ctgtccccga gactcagtac actcagagag tctctgggtc tacttcttct agtgcaccat 13740
   cggctcctca tgtgcctcct ccgatgcctc ctcctgtgcc tcctccgatg gcacctccga 13800
   tggtcgccga tattcatcct gatctgatgg tgcctccgag tgctccttac tcgcagtaca 13860
   ctgtagagga cattctccgt ctgccaggca gagaaggttt accagtcatc gacccagacc 13920
   gaccggacgg aacgttgtgg tatgttgcat taattttttt taattcgttt aaatttcttt 13980
   tataacatta aaaataattt atattttaaa tttgtatttt ccaggtgggg ggttgacgga 14040
   tgtcttgcat cggacgtaac cgacacaatc aagggttact tctccatggc acatccaaac 14100
   tggagtaaga cgcctcacta cgtcagaaag acgtggttca aaatttacgc tgtaagtttc 14160
   tattaattaa ttatatatat tttaattttt tcatgattta tatatatact ttctaaaaaa 14220
   ctaattgtta atttattttt tccaacagca aaaatataat tgggccttgg gaatcactga 14280
   gagggtgagg aagaagttta acgcgaaagc gaaagttcgc ttgttggaca cggtctccaa 14340
   ctggaagggt gactggatcg tgaaggggta tgagtgtggc aaacccgctg agctcaccac 14400
   ggatgtgtgg gatggcctca tccgttattg gcgccttcct gattccatta gaatcgccca 14460
   ggcttactct aactcccgta acacggtcga tgagcacggg aacgggccga tgcttcacac 14520
   tacgggccaa aaaccccacg ccggtgtccg tttggaaatg gtaattaaat attttattaa 14580
   ataatttttt taatatatat attaatttat tctaactttc ttaaatgttt tttaggccaa 14640
   agagacggga catctcccgt ctcttatgga actttacgag aggacctaca agaacaagac 14700
   gggcgtattt gtagatggca agtccgagca aatctacaac gatgtagttg ctcgggttga 14760
   agaccgccag actcagctga cccagcaatc taccgacgga ttacccgtca ccttatccac 14820
   acttgaagtg gataagattt acgaggaggt aaattttcaa aaaaattaat tttttattat 14880
   tcatttaatt taactttaaa tttttactta caatatttat ttttgttttt aaggttgtcc 14940
   ccaaaaaaag ggacggacgt tgggtattgg ttccgtcaac gttgttccga gagcgacatc 15000
   gtcttatggt cagcgacggg atgatgaagt cactgagctg cgtagagagt ccgctcagct 15060
   gcgtaacgag ttgaccgcga caaaatctcg tatgggtgga gtcgagggct tcttggacgt 15120
   tattgcggcc acaaatccgg aatgggagtc catgttgagg aacatgcgac aacaacatcc 15180
   cattcaaggc gagtcatctg acgtacataa cgaggcggat gttatgagga ggagtgatga 15240
   attctaccgg gcgatgaacg accctaagtt ttttttttgg ttgttgtatt atataaattc 15300
   aaaacttatt tatatataaa atattttcat attgatttat ttttattttg aattttaatt 15360
   tattattaaa ttaaataatt ttaattattt tttaattata tttttaaatt ctgtaaaata 15420
   ataaaaacga agtaaattcg tagccaatgt acgacctctt tacgtggaaa cctcacgagg 15480
   aaatgacgag aaacatttaa cgagtatttt acgaagaatc atttacgagt aaataagagg 15540
   aaaagtttac gaccatttta cgaggaaatc atttcgtggt tgttacgtgt attttgcgag 15600
   gaaactcttt caaggtattt gtgtgtaggt tacgaggaac tattttcgag gtatttacga 15660
   ggtattatgg cgacgtcctt acgtggaata ttgacgtggt ctttacgacg aatcgtccta 15720
   cttcgtcttt acgacgaaat atattcctcg ctaagttacg acgaattagc gaggaaatat 15780
   gtgttacgac agacgtgtaa cgagcaaacg cgtttcctcg ctaattcgtc gtaaagcctc 15840
   tcttacgacg aattagcgag gaaaaccgcc ctcgttaaga ttatgttttc ttttagtgaa 15900
   aatgaaaata taaggttgtt gtattctact ttacatggaa ttgtagcttt atatctcatg 15960
   aacatatcat tcctcttcgt tcttgttctg tcttaagtga taattcattg atatatttaa 16020
   tattttagct tccaccttcc tcactatttc caactcttac tttgaatctt caggtttggt 16080
   atgaacgagt taggccttcc aagcagattg cttgagaccg gctgtgaacc cattggcaag 16140
   aaaagggtta acaattattc aatctccggt ggattgaagt gataaagagt gcattagagg 16200
   atgaagacct agcgatgttg aatgcgtcac agtttgggtg agtcttgcag atggggaccc 16260
   ataccttctc ggttacgttt cttcacttta ttctatcccg ccagctggtc actgtgaagg 16320
   aattctagct gtggtggctc tttgtgggga aacctattcg ctatgttaca actgttctgc 16380
   agtataaatg gtaggtgggt ttaagttccc gaatagttgg attgccaatg gagtagggtt 16440
   tatatttctc tattttgggt ttagtttttt ctttcacatg ttatcttatc attcccatta 16500
   catttgtatt tcatattgct ccatccttgc tgaactatgg cgacaatagc cttgcaatta 16560
   tgaataagac aaatatgtac gtaacactat accacatatc tagtaatgga ttgtgtttta 16620
   tgttttcttg cggggttcag tgtttaattt caagtgttct cttatcttcc ccattacatt 16680
   aatattgtat gtaaaatact cctatatgga atatgaaaaa tagaaaataa catagtttat 16740
   attatatgaa atagaaaatt gtacgtgata ttgtccctac gtttcctatt gccaacgaat 16800
   ttggggttgc tttaccaatg gattgaagtt tatatttctc taacttgggt ttagtgttta 16860
   cttccaaatg atgtctactc attcctctta gctttgtatt gtatgttgcc cagttgtgga 16920
   tgtaatatac ctagcatagc actttttaaa aggattgtgt ctataggaaa ttaaatgtct 16980
   tcagtactca tctttgtatg aattttccgg tttgaaaacc catcgtttat gagggtcgat 17040
   atcccacgcc ccaacaacaa gtaatactct atcttcagta ctcatatgcg ataagaagta 17100
   aatgaagatt catttatata tcagtctcta ttccatgtaa aacttgtttt tagtacatat 17160
   tctcactgca aattagctgg ttgttacttc caggcagtaa ctccttaact ttcttcacct 17220
   ccttgctttg agtttcttca gatggactta cagtgataag tagatggaat acaacattta 17280
   ttgctacaac tactaattta caccacttgt ttaactccat acagtaaaaa tatctttact 17340
   aactccacct gtttacgtag cttcctccca ctctttaata tggagtagct gtaagccatt 17400
   actggacatt tactccatta attacgtcac cgtctgtccc caaccgtaga agtcattgtc 17460
   tttgtaagtg attatgttgg taaattacat tcccaagttt atattctatt tgttcatgtg 17520
   gcatggaacg tacactcata taaatttgat ggtttgtatc ataccgttgc attcaaatgg 17580
   ttttggttga ccagacctaa gcctgacgtc gacgttgtta tattagaaat accccacgct 17640
   atatgtaccc acctcctcat ccaactccat gtatcgtact ttctaactcc cacgcaactg 17700
   tagctatgat attgttttat accatatgga atagtttggc tgtacaataa atagtaactg 17760
   ttgattttgc cgatctgaag ctagcaacat gagtatttgg cttcatttgg gtaaagtttt 17820
   acgtactttc cttgcgtcat gcacatttca actgtaccaa aagtatttac caaaatattt 17880
   acatgctttt tcaattggaa ctacatttat agggatacta ttacttttat actatgtagt 17940
   atggatcttg tgtaactgct atagaataca cttgtttcat cgtcgacaat tcgccatttg 18000
   tttatggtac acttgcgatt cttatctact cctacatctg catctccttc cataatgtgt 18060
   tgaatacatc tcagttacgc gctccgtaag tttttgtgat tgtaaaccca ccacgtcgtt 18120
   tactttgtaa tatagaaccg gtaacctgtt gctttaaggg gcataaccgg gtgggaggaa 18180
   tcacaaaagc ctgacaatga attatgtcaa aatcaacgct gcttttttaa tttattccca 18240
   gaaaatggct atttcgccaa ttaaccctta attgaattag aaaaatatgt atgcgaaagt 18300
   aaatatataa gacatatttt atggaccggg gatgtcctag gtcgaggact aatcataatg 18360
   aactgtttag catgtttttc aggcgacaga taaatccgct gttctacgtg gaaattagat 18420
   atccacgccc tttaacaacg taaatgagta aatctgaatg ataggtttca aaacgataat 18480
   gcctaacact ttcccgccgt atcacacgac catatccata tggttgaaat atataaattc 18540
   ttctatttat attttgtctc agattatatt acttctagag gcggatgaaa aaaatatgaa 18600
   aatctgaacc tgagaattca aactattttg aatttgacat aagcatccaa atggttattg 18660
   ttctatggta tttcagattt tagtttttac ccagatcaaa ataatggaaa tcgaaaaaaa 18720
   aactcaaatt ttttaaaaac ctttcaaaat acaaaatgga tcaattttga ataattatcc 18780
   aaaatactta aagatccaat aattattcaa aatacttaat gaactataat atttaattta 18840
   taaaattagt aatttatcaa aatatcatat ttagatttat atatttttta aatatgttta 18900
   tatgtaaaat aaaaagaata gattttttgt gaattatata tataattaag ttttataaac 18960
   ttagcttcca tagtgtttat taaataattt gacatatata tatatatata atatgaatca 19020
   cataatgtta atgtttttaa atataatctg ataaataatt ataaatactt tgaagtgttg 19080
   aaaaagtttg aaatgaattt cattttaaat aaaaaccata cataacaata ttttgttatg 19140
   tttatataat ttttatacat catttattaa tttataattg taatgagaca atataattta 19200
   tgatttttta taaatgttaa tttactgaat attaatttgt tgaaaatatt aatttattga 19260
   atactaattt attaaatatt aaaatatgat tttattgaat attaaaaata ttaatttatc 19320
   gaatactaat ttataaagct tctggtagtc gtctgttaac tcatatattt ctctaactac 19380
   tactgaataa gcttgtgact tattatacac gtctatacgt gtttatctat aaattgttta 19440
   cgtcgaaaga atcatttcgt agatacccac gatgttaccc aagttcaaga attaagaaaa 19500
   ttaactatct atcattacgc tacttaccaa aaataatatg aagagtaggc ccgcgggaat 19560
   atgctgcctg ttataaattt gcaaatgaca ataaaaataa cttatatatt acactgaaat 19620
   atctctaatg tgtagggtta taaagaaaaa tataattagt taagctattc gttaacattt 19680
   ttgccccgtt aattaactat gtattttggg ttttgaacta atcataaaaa tcatttaaaa 19740
   gacacaattg ttgattcaga cgaacaaacc aatacaagtg ttgagggaat gtagttggta 19800
   tagaacacgt cgtaagaatg atgtattttc gtgtaccatg ggccggccct ggcataaagc 19860
   ccataaaaca agtactttag gcaccaaata taataaaaaa ttcatgggca ccaaattttt 19920
   ttaaagtcac cttagtctaa tgcattttac gttatcctct tgagcaacaa gacacgagtt 19980
   tgacgcgtac tttcttttct tccttttttg ataataatgt catttttgat aatactaaca 20040
   atttaatatg atttattcat ggatacatat agacacggtt agtttcttaa tctgccgaca 20100
   aaaaaattac ttaatctaac aactattttc tttaattata catcttatat taaattgtgt 20160
   gacactagat aaataaattt aattaaacaa aaaacttttt ttggcaaata acaaacctat 20220
   gtattagtaa caactaacaa tcatttagat tcttgacgtc ttttgtgtat ttcttgcgtt 20280
   gatctttgct ataatagctg aagaagttat aacaattaac tcaaatgatc aattcttcat 20340
   attccaagtt tgttttaatt gatatccatc ggaatagctg gcatatgtca tgtaagtttt 20400
   catttttttt ctacttgttg gtttctattg cttaaaaaaa taaaatttta ataaacaaaa 20460
   aattattgtg agattttaac attcgatgct gacaaataat ttttgaaaaa aaaaaaactt 20520
   cagaaaataa ggttttcatt ttttcttaaa gtaaataaat tttaatatga tttaatgcta 20580
   ttttattaaa taacaaaaat aaagcaaaaa ttaatatatt gaaagggcat atttgttaag 20640
   tacgctttag gcaccagtta agtccggagc gacactgcca catatacaac tcgctaaaac 20700
   aaagatttat tgtactgcat gaaccttcca accacatata gactcatcac cacgacaaaa 20760
   aaaaaaaaaa cctggactac actcaagttg cgcaagccag tcatggaccg tagagtagct 20820
   tggttcagac cgtgtagagg atcatatgta aaggaagttc gaattcatag caccagagac 20880
   catcgtttct agctaggtcc atacaaactg agttttttca ttgttttgga ggagattcgc 20940
   gcggaaccga agttccgaaa cccgatattg tcaatttgtc ataagtgaat tggcttctat 21000
   acttctcgta acaaaatcat taacatggat tagtggtcca ccaaaactga aagataacat 21060
   gttaaagaag tggactacta catagtccca gactcccaat caacataagt tacataacca 21120
   atagatgaca aatggtccat caattatcaa atttgcttgc ttttgttttg caggtgttat 21180
   ttagtgtatc catcccgaaa cgcattttct aaactcgtag tcttgtgcag ttttctagtc 21240
   caatcttcat attgttacca agaaaaatac ttgttatgtg aatatttttt ggattgcatg 21300
   ccactagccg aaatttcatg gatcaatgga tctttggcgt acatatattt attagaacac 21360
   tttcattaca gaggatcctc aggaaaatat ctcataataa aataaaaaca ataaaaagag 21420
   aataagatag gatttttaaa tgattatttt tgaaaaaact catgaaaact ccatgaaata 21480
   cttgtctttt ccatataggt tcaattttta ttttaaatta ttcttaatca attatttaaa 21540
   tttcatttaa atactgatat tttgtttgag aatcaatgat gctctaagtg gcaataattg 21600
   taaccagtac tagtttcttt attgaccaac tgatatgaaa cgagaatttt ctattttcta 21660
   tttttgtttt agtatttatg tttctgttgc catgacaaag aaagagtgct aaaagatgag 21720
   agatgttgct tgttaattgt tatatacgag tagagtataa ccatatcccg atttacatag 21780
   ataggattag gaagcgatac gttatacata tcaggataga aatattagtt gaaaatgagc 21840
   actacgcgag atgttaaaga aaaaaaggcg tacattaaag cccttaatat tcgacataag 21900
   agcaccagca tcagcattag aggttcgtgg acagtggcgg agccagacga aagttttacc 21960
   aggggcaatg taaaatttat cttcagttta tagggagcag tataagaaaa ttcaccatta 22020
   taatcatata attctcaaat aaacaatgga aaaaaatatt ataatatgct acagtaatga 22080
   tcctgtctcg ccacgctcct tccgtatgaa cccgagttgt cactgttcag cgggctccac 22140
   gccacgtggc ggtctgctat tggtcaattt atttatttat tttttaaaaa aaaaacaaaa 22200
   taaaaataat agtaataaaa taataaaaaa ttcaaattat gaaccccaac cgtgggttca 22260
   ttaatgctgg tgctctaata tgtgtgcctt aaataaaaac gtggctaatc tatcaataca 22320
   aagcacagtt aaaggtacaa ccattaagaa aaagaagagt taaagatcac gatcacttca 22380
   tgaatacacg tctcttcaac atcacaaacc attcatatgt atggtttaat atctaacaga 22440
   gtatatattt ttcaaagaga ctattatgga agagtccata ttaattttct aaggaggggt 22500
   gcatccacag attgattttc tttcatattt taaatgggtt gtaaacaata attcatatcc 22560
   ttatgattat ataggtttag tgccgtggag tttattcgaa cccggatctc tctgaagtct 22620
   acataccatt agaccaatct catgtggtta atcaagccaa tttttgaaga taactagatg 22680
   taaattaaac aattcaaaga gttgttcaga aaaaaagagt gatggctttg gacaagaaaa 22740
   agaaagatgg gatgttgtac gtgcacgtgt aaacgacgaa acacgttggg ttctattcct 22800
   aaagaagcat tggctctact ttctaacaaa tctctaatta tccaattaat tatttgatcc 22860
   taaacaatga catctcgttt gaggttttct ccttttttcg attcatcaat atttccctag 22920
   gaaaaagttt tgtttctgtc aacttgtaaa tgatcaccgt gaatatctta caacgacgca 22980
   tattcccatt acagaagaac aggtttcagt ttggatcaac ctaataagtt tcagacttta 23040
   ctaccttcac tacaagaaaa cacaaattta acgacggcca aaatcgtcgt tatttcctcg 23100
   gaaaagaagg cttacgagga aatggcgatg aaaggcgttt cgtcgttata tgattgtcgt 23160
   aagagaagat tcgtcgccat ttcctcgtta attagcgagg ttatattttc ctcgtaaaga 23220
   agaattaagt tttcgtcgta aagaccacgt ggggtttcca cgtaacgcgg tcgttgtgct 23280
   tcctcgtaag aaactcgtaa atgattcgtc gtaaaagacc cgcaaaaacc tctaaataaa 23340
   ttcgtcgtaa taaaaacgta agaaacacgg aaacaattcg tcgtaataga atcgtaacta 23400
   aatccacgta aaatcctcgt taattgttcc tcgatatttc gtcgttaatt ttcctcgtta 23460
   atacatcggg aattagcgac gcaattactt tgttttctat ttactgaatt tataaataaa 23520
   aattatattt atttaattta ttaataaaat tttaattgaa attaaatcga atagaaaata 23580
   tttttttggc cgaattaaaa tgaaattata taatatataa ataagttttg aattttaaaa 23640
   tacaataa 23648
<210> 15
   <211> 3247
   <212> DNA
   <213> Brassica napus
<400> 15
   catcagaccc tttcttcacc acatttcact cagagcccac acagttttag agagagagag        60
   aaacatccct caaagctctc tctctttctc cggcgatggt tgtcgctatg gaccagcgta       120
   gcaatgcgaa cggagacgaa aggtttgatc cgagcgcaca accaccgttc aagatcggag 180
   atataagggc ggccattcct aagcattgtt gggtaaagag tcctttgaga tccatgagct 240
   atgtcgccag agacattttc gccgtcgtgg ctcttgccgt cgccgccgtg tattttgata 300
   gctggttctt ttggcctctt tattgggccg cccaaggaac cctgttctgg gctatcttcg 360
   tactcggcca cgactggtaa tttaattttt ctttcaactt cttaattttg atatgtttat 420
   atgttttttt cgttttttgc attgtctttg atttcttgac cgtacgttcg atatgagatt 480
   ttcactgact tcaagatttg attctcttca ggtttacttt tttcaatttt aattattatg 540
   ttcatccaat ttggcctatt ttaaaagcaa aaggggatct aagattttta attcttttgt 600
   ttttttttgg ttctttttca tcagtcgtaa cactcctaac taaacatctt tttctttcct 660
   ataattattg ttgtttccgc gttttatgga tctacgtttg aaattttcaa taaaacacat 720
   tttattgttt tctgtaacaa tttaattact gtttattggt tcttttaatt attgtgtgtt 780
   gttccaatct attttcgaaa tatagtcatg tgacacgtca tattctattt ttgttacctt 840
   gttgaaacgt ttgaattgag gaaagttcag ttaacattgt gcaataaatg ataaatgtgt 900
   ttatgatgta aaatttcatt tgaataatac agtggacatg ggagcttctc agacattcct 960
   cttctgaata ctgcggttgg tcatattctt cattccttca ttctcgttcc ataccatggt 1020
   tggtaagtca tttattttaa cttctttttt catgcaaatt tattcttgtt ttcgtatttc 1080
   ttacattttc cttgtcattc ttggtgcatg ttagcaaaca gtaatctgat aactgaaaat 1140
   atattaattt ttcatagtaa aataatgcat gtgactaaaa gcatcaaaat ctttagcatc 1200
   gaagaaaaaa gaaccaaact tttatttaat gctatgggcc tatttatggt ccaattagct 1260
   attatcatat gacatgtcct tgaataaatt aatgtataag tttaatataa tatttatata 1320
   tatttgtttt aatggcttat tttattgtta aatggataca tcagcttgaa atatctacga 1380
   acatgcatca ttttcctaga tacatttgtt tgttgctcaa aaaatgaata acgtagttaa 1440
   acgagtgaga ttcttagcat ctgcctcgaa aacgatatgt tattgacaat tccaatttca 1500
   tttttatgaa aataaaataa tagtttattt tataattggg ggtggttgca ggagaataag 1560
   ccatcggaca caccaccaga accatggcca tgttgaaaac gacgagtctt gggttccggt 1620
   aatccccctc tcattatttt tttttctttt tttgaaactc tttcatttta attttcttag 1680
   aattctatgt atttatttta atcaatcctt tttccagtgt gaggcttgga cgaccacttg 1740
   tcagatttgt cgtttagctg tagtaaacaa ctgatttaaa ttgtttatgg tactgtagtt 1800
   aactttaaca acgggccact tatattcgag ccattggcat aaaatgattc ttctcgaaat 1860
   tcgtttactt ttcttagtat ttttcagttt tgtagtttac gtagaactaa taaaaagaaa 1920
   aaaacttata aacacaccac atgcaatgaa taaattcgaa tatataacca tactgttaaa 1980
   tattaattaa cattttaatc ttaattttgc attccagttg ccagaaaaat tatacaagaa 2040
   tttgtcccac agtacacgga tgctcagata cactgtccct ctccccatgc tcgcttaccc 2100
   tctctatctg gtaaatccta attcctcatt tttcttcctg attataatta caattttgaa 2160
   tttttagatt ttgagtatta actaaatata aattaaattt gtttggggat gactacagtg 2220
   gtacagaagt cctggtaaag aagggtcaca ttataaccca tacagtagtt tatttgcccc 2280
   aagcgagaga aagcttattg caacttcaac tacttgctgg tcgatcatgt tggccactct 2340
   tgtttatcta tcattcctcg ttggtccagt cacagttcta aaagtctatg gtgttcctta 2400
   cattgtaagt ttcatatatt tcattattat atcattgcta atataatttg tttttgacat 2460
   aaagttttgg aaaaatttca gatctttgta atgtggttgg acgctgtcac gtacttgcat 2520
   catcatggtc acgatgataa gttgccttgg tacagaggca aggtaagtag atcaacatta 2580
   atttataaga agcaacaatg attagtattt gattaatcta aattattgat gttttgtgta 2640
   caataatagg aatggagtta tttacgtgga ggattaacaa ctattgatag agattacggg 2700
   atcttcaaca acattcatca cgatattgga actcacgtga tccatcatct tttcccacaa 2760
   atccctcact atcacttggt tgatgccgtg agtgatctcg ctctctctct agtttcattt 2820
   gattaaaatt aaagggtgat taattactaa attagtgatc ttaattaatg atatgcgaca 2880
   gacgaaatca gctaaacatg tgttgggaag atactacaga gaaccaaaga cgtcaggagc 2940
   aataccgatc cacttggtgg aaagtttggt ggcaagtatt aagaaagatc attacgtcag 3000
   tgacactggt gatattgtct tctacgagac agatccagat ctctacgttt atgcttctga 3060
   caaatccaaa atcaactaac ctttcttcct agctctattt aggaataaaa cagtcctttg 3120
   gtttttactt atttctggtt gtttttaagt taaatgtact cgtgaaactt tttttaatta 3180
   aatgtattta cattacaaat caagtttttg ttcgttttct ttatgttttt agttacaata 3240
   aataaag 3247
<210> 16
   <211> 4014
   <212> DNA
   <213> Brassica napus
<400> 16
   catcgaaccc tttcttcacc acattccact tcccacactc tctttttttt tgaattatag      60
   agagagaatc ctcctccaaa tctctctctc tcccaggatg gttgttgcta tggaccaacg     120
   caccaatgtg aacggagatg ccggtgcccg gaaggaagaa gggtttgatc cgagcgcaca     180
   accgccgttt aagatcgggg acataagggc tgcgattcct aagcattgtt gggtgaaaag     240
   tcctttgaga tctatgagct acgtagccag agacatttgt gccgtcgcgg ctttggccat     300
   tgccgccgtg tattttgata gctggttcct ctgtcctctc tattgggtcg cccaaggaac     360
   ccttttctgg gccatcttcg tcctcggcca cgactggtaa agtttcttcc attttgcatt     420
   gcatcgattt attgaatgca cgttctacga gtattgtttg tcagttactt cgtaaaatga 480
   ttcttttgat gttcattttt tgaagatcta agattttttt tttagatttt ctttttaaat 540
   cattgttcca ccaccacctt tcatcggtcg tacgactcgt tacaacacca catctttatt 600
   ttctataatt actactgctt ccgcatttta tggatctctc aacttataat taaagtataa 660
   tatcaagaat atctattatt tttcttaaac aagaaagata atattgtttc tttgttattt 720
   tggtgtattt ccaatctatt tcgagattta gaaatgtgac acgtcattac cttgttgaag 780
   tgtttaaaac aaacatggaa agtttaaata aatagtgcaa taaatgatat atatgtatat 840
   gatgaataat gatgtgaaat ataattgaat aatggcagtg gacatgggag tttctcagac 900
   attcctctgc tgaatagtgt ggttggccat attcttcatt ccttcatcct cgttccttac 960
   catggttggt aagtcagctt atcaaccctt tttactatat tattaattat taaacttgca 1020
   tttgtatact tggtgcaagt tggtaaatgt aatctgataa ctgaaaatct attcattgct 1080
   cgttctattt tttttttggc tagagacaat tttataatta aataatgcat gtgagaatat 1140
   gactatttat gtgaggtagc ttttcttatt cctgtcgaaa agcatcaaat ctttagcaac 1200
   gaaggaaaaa ggaatcaaat tttttattaa atgcaatggg tctatgtctt ggtcattagt 1260
   tttttgcata taatttattt atattttttt cttaacagca gctaatttaa ttataattaa 1320
   atattcattt tataaataat attagaccaa ttattaaagg ttagatattt taagaattat 1380
   tcatgacttt gtttattgga actcctttta tcttttaatc ttttctattt ctccattttt 1440
   aataatgaga aactgacttc aaatctccaa taaagatggt cttatgtagt aacagtataa 1500
   ttttttgttt ggtaaatgta acatcatctt caaatatctt tgaaaataga cttacatgca 1560
   ttattttgct gcgacattat tgtcacttat tcctggcaat aaattagttt attactgaac 1620
   ttttttttgg tcaatttatt actagtaact ttaaacttaa aagagtgaga ttgtttgatc 1680
   aaaaaaaata aaaatagagt gagatagtta gaatctgcca tgaaagcaac actatataga 1740
   caatttaatt tttatgaaaa cacatttaat aatttgaggc tgcaggagaa taagccatcg 1800
   gacacaccac cagaaccatg gccatgttga aaacgacgag tcttgggttc cggtaacatt 1860
   tccctcttta ataatttcta tttttctgtc aaaataatta gtttttcgaa atttgaggcc 1920
   agaacgacca cttgtcaaat ttgattttta gctgtagtaa aaacagtttg ctagtgtcac 1980
   agttaaccgg taattgattc tttttaacga tttatagaag taacattttt gtaaaataaa 2040
   atatacatta tggtatgtga caacggacca cgcttatttg tattggtgaa tcttttaatt 2100
   actccctcca atttatttta gttgcagatt tagatttatg cacatagatt aataaaaata 2160
   ttttgcacat tttcaaaata aaaacaccat tacttataca actaaccata tttcaaccaa 2220
   taaaaataaa ttagaaaata ttatttataa attttgtatt gaaattataa aataatactt 2280
   attttaaaac gaaattaatt tacaacgaca attaaactga aacggaaaga aattattaat     2340
   acttaattaa agagttttta gaaaaattga aagacatgtt tatgcgaaac tcatgtgaaa     2400
   gtctttgaaa taatagattt tggtataaat atttcaaatt ttcttaaaat aataattata     2460
   tattaatata atttgtgata aaatctcgtc aaaaactcac taatgcaaat gcttttattt     2520
   tgaatttctt actcctctaa atgcatttac ttttatacta atattatttt ctttctctaa     2580
   tttggcgttt cgtaatagtt tgtctgtatt ttgaaaacta acaaaaaata ataaaaacaa     2640
   aagcttataa acacatagca tgcaatgaat atgtacgaat atatatacca atacatatct     2700
   aagtactatt tttccaagta cttaatcttg attactaaaa ttcattttaa ttgttccttt     2760
   cagttaccag aaaggttata caagaattta ccccacagta ctcggatgct cagatacact     2820
   gtccctctgc ccatgctcgc ttacccgatc tatctggtat tttttaattc ctaaaattta     2880
   ctacaagtca ttttagactg tgttttaaaa caatataatt atttttgttt ggttttactg     2940
   cagtggtaca gaagtcctgg aaaagaaggg tcacatttta acccatacag tggtttattt     3000
   gctccaagcg agagaaagct tattgcaact tcgactactt gctggtccat aatgttggca     3060
   attcttatct gtctttcctt cctcgttggt ccagtcacag ttctcaaagt atacggtgtt     3120
   ccttacattg taagtttctt agtatatcat aaagggtata tatttattat tcaatatata     3180
   tactatatga tttgtttttg tcatatattt ttgaaatatt cagatctttg tgatgtggtt     3240
   ggacgctgtc acttacttgc atcaccatgg tcatgatgag aagttgcctt ggtacagagg     3300
   caaggtaatt aaattaacta ttacaagtat tttacaaaaa actaatgatt agtatatttg     3360
   attaatctta attcttgatg ttttgtgatt aataatagga atggagttac ttacgtggag     3420
   gattaacaac tattgataga gattacggaa ttttcaacaa cattcatcac gacattggaa     3480
   ctcacgtgat ccatcatctt ttcccacaaa tccctcacta tcacttggtc gatgctgtga     3540
   gtcatctcac tctctggcta ctttcatcaa aaccatttga ttaaagggtg attaattact     3600
   aatgtagtga ttttaacaaa tggaatgtga cagacaaaag cagctaaaca tgtgttggga     3660
   agatactaca gagaaccaaa gacgtcagga gcaataccga tccacttggt ggagagtttg     3720
   gtagcaagta ttaagaaaga tcattacgtc agtgacactg gtgacattgt cttctacgag     3780
   actgatccag atctctacgt ttatgcttct gtcaaatcga aaatcaatta aactttcttc     3840
   cccctttttg tttagcacta ttatgaataa accagttttt tttacttata tattgttgtt     3900
   tttaagttaa aaatgtactc gtgaaactct tcttaattta gatattattc catttacact     3960
   gaaaaacata caatttcaaa ggttgaaaag aaagacaaaa ttttctagaa tgac           4014
<210> 17
   <211> 4761
   <212> DNA
   <213> Brassica napus
<400> 17
   catcaaacct ttcttcacca catttcactg aaaggccaca catctagaga gagaaacttc      60
   gtccaaatct ctctctccag caatggttgt tgctatggac cagcgcagca atgttaacgg     120
   agattccggt gcccggaagg aagaagggtt tgatccaagc gaacaaccac cgtttaagat     180
   cggagatatc agggcggcga ttcctaagca ttgttgggtg aagagtcctt tgagatctat     240
   gagctacgtc gccagagaca ttttcgccgt cgcggctctg gccatggccg ccgtgtattt     300
   tgatagctgg ttcctctggc cactctactg ggttgcccaa ggaacccttt tctgggccat     360
   cttcgttctt ggccacgact ggtaaattaa attttctgtt ttaattattt tgactctttt     420
   tgttcaattt attaatttct tgaatgcacg ttcgatgagt atcgtcgtca ctgacttcaa     480
   gatttaattc ttttgaggtt accttttcat gttcaattat taaaaaataa aataaaatat     540
   aggatctaag atttttttct tcatcagttc aagcatcatc actcatcagt cgtaagactc     600
   gtaacaaaat atcttctttt ctataattaa tattatttcc gcatttaatg gatctacgtt     660
   ttgatgttct caaattttgt ttctctttct ctagatcccc ggaactttta attataatta     720
   tagtatagta taatatcaag aaaatatact gtttattttt tttggcaaca aatatattac     780
   tcttgtttct ttgacaagaa aaaaatatat tgtttttttc ttctttttgt gttccaatct     840
   attttcgaga tttagacaag tgacacgtca tataccggat ttgttacctt gttaaagagt     900
   ttgggttaaa acaaatgtag aaaagttaaa ataaattgtg caataaatga taaatacgtt     960
   tttatgttaa acaatgatgt gaaaataaaa ttgaataatg gcagtggaca tgggagtttt    1020
   tcagacattc ctctgctgaa cagtgtggtt ggtcacattc ttcattcatt catcctcgtt    1080
   ccttaccatg gttggtaagt catttattaa ctatttccat gtaaactatt agtacttgtt    1140
   ttcgtatttc ttacattttc gtttgtcatt cttcttgggt gcatgctagc aaactgtaat    1200
   cagtattaac tgggaactac caactgtttt ttttttgcta gagtagcaat tttataatta    1260
   aataagaatc ctattaaaca atgcatgtga caatatgagg ttgcttttct gttcaaaaca    1320
   aatctttaga agccaatgaa aaagaatcca aaactttttt ttaaatgata tgcgcctatc    1380
   tattggtcct gactcctgag ttttcttact ttcttaagta taattagatt ttgatttttt    1440
   tttataggtt ttcactattg ttatttgttt acatcagctt cagatatctt cgaaaaagat    1500
   ttacatgcat caatttcatg aggatttata gtttttcttt tacttatttc cgacacaatg    1560
   tttagtagta aaaagcatta aatgtttttt tgctcaaaaa aaaaagaatg ggattgttag    1620
   agcactctat tgttagttgt tcaataaata taccaactaa aaaaacaaaa taaatataaa    1680
   atgagtgaga ttgttaaatc attatagaga caatttcatt ttcacaaaaa taaataaata    1740
   cataactttt tataattggg gtttgcagga gaataagcca tcggacacac caccagaacc    1800
   atggccatgt tgaaaacgac gagtcttggg ttccggtaat ctttcctact ctcgtagttt    1860
   ctcttgtctt ttatttattt gtttgttttt cggaatttat tcttatgtct atgttcttag 1920
   gattctatat gtttatttta ttagtttatg ttttcagtct gaggtcagac cgaccacttg 1980
   tcagatctgt tttctagctg tagtaaaaaa caatttgcaa gtgtaatagt tcagcataat 2040
   tgatcttgtt agagcatttc caaaacaaac tttataattt taaatataca gttttttgtt 2100
   ctctaaaaaa gaatttaaaa attttaaagt ttgagggacg aaacttcaaa tttgaacttt 2160
   cactactcaa cttcaaattt gaaatttcat cttttttatt tacattttga tcattataat 2220
   taattataca ttacatttat gattcttaag tattttctca tttattgttt taattcttaa 2280
   attttttata catcataaat atttccaatt tgtttttata aattcaaatt ttacacaaaa 2340
   aagtaataaa aattttaaat aagatttata atattttaaa actataatta ggcaaaaaaa 2400
   atattacaaa aaaatgtaat aaaaacttta aaataagata tatcaagaca taattattag 2460
   aaattttaaa tattataaca atattaataa tctggtaaat ttgctccaaa acctcaaaaa 2520
   tttctaaatt attgtccaaa caaatttgtt taaccgaata tggagcatta caaaaataat 2580
   tttatggaat agtgtggtat tttgcttgta gttaatattt aattatgtat ttctatttat 2640
   aattttatat atttaatgta agattttttt aattaatatt actgtaatat ttttatatat 2700
   gtactagtta tttataaaag ttttatagat ttgtattagt tataacaaaa ataaggatca 2760
   ttgtgtaaaa tacaaataat tttgaaatta cgtttaaagt tttggttatg aaaaaaatac 2820
   tttgaaactt taaatttaga gttttgcaaa ctttaaaatg ttagatagat agtttttttg 2880
   gagatgcatt tagtggttat ggtagtaact cagaaaatga aaaatctata cttttatact 2940
   ccctccgttt tttaatataa gtcgttttac agttatacac gtagattaag aaaaccatta 3000
   atttcttata ttttctagac aaaaacatca ttaattattt acctaaccac aattcaacca 3060
   atataaaaat agaagatata ttaccattgg tcatacaaca ttaattatta ataaatttta 3120
   catagaaaac cgaaaacgac atataatttg gaacaaaaaa atttctctaa aacgacttat 3180
   attaaaaaac ggagggagta gtacctaact ttaacgatgg accacttata ttcgagtcct 3240
   tagcataaaa tgattctcct cgaaatccgt ttactttctt cattattttt tccttttcag 3300
   ttttggcgtt ttcgtaatac ttttgtcttc aatcttgaaa gctattagta taaaaactta 3360
   taaacacatc acatgcaatg aattaatacg aatacataac cagaatgaca aattttcaat 3420
   gaatatttaa taccagtaag tactactccg taatagtaat agtaatagtc atattaattt 3480
   ttttttgtca tcaaacaaac agtaatagta atattaatta taattatgta tttcagttgc 3540
   cagaaaagtt gtacaagaac ttgccccata gtactcggat gctcagatac actgttcctc 3600
   tgcccatgct cgcttacccg atctatctgg taaaaaaaaa tacaatttca atttttttct 3660
   taaaattaca aatggtttta tattttgagt tttaagccaa tatataaatt aattttgatt 3720
   ggattttaac tacagtggta cagaagtcct ggaaaagaag ggtcacattt taacccatac 3780
   agtagtttat ttgctccaag cgagaggaag cttattgcaa cttcaacaac ttgctggtcc 3840
   ataatgttgg ccactcttgt ttatctatcg ttcctcgttg gtccagtcac agttctcaaa 3900
   gtctatggtg ttccttacat tgtaagtttc acatattatt acaagagatt tatatattat 3960
   taataataaa tttgtttttt gacataaagt tttggaaaat tttcagatct ttgtaatgtg 4020
   gttggacgct gtcacgtact tgcatcatca tggtcacgat gagaagttgc cttggtacag 4080
   aggcaaggta aataaatcaa tttttaaaaa gaaatgtaca gaaagcaata atggttagta 4140
   ttgattaatc ttaatttttg atgttttgca tacaataata ggaatggagt tatttacgtg 4200
   gaggattaac aactattgat agagattacg gaatcttcaa caacatccat cacgacattg 4260
   gaactcacgt gatccatcat cttttcccac aaatccctca ctatcacttg gtcgatgcgg 4320
   tgagtgatct agctttctct ctctctagtt tcatttgatt aaatggtgat taattactaa 4380
   tttaattaat gaattgtgga cagacgagag cagctaaaca tgtgttagga agatactaca 4440
   gagagccgaa gacgtcagga gcaataccga ttcacttggt ggagagtttg gtcgcaagta 4500
   ttaaaaaaga tcattacgtc agtgacactg gtgatattgt cttctacgag acagatccag 4560
   atctctacgt ttatgcttcg gacaaatcta aaatcaatta acttttcttc ctagctctat 4620
   taggaataaa cactccttct cttttactta tttgtttctg ctttaagttt aaaatgtact 4680
   cgtgaaacct tttttattaa tgtatttacg ttacaaaaag tggaagtttt gttatctttt 4740
   tctctagttg caatcaaaag g 4761
<210> 18
   <211> 3827
   <212> DNA
   <213> Brassica napus
<400> 18
   catcaaactc tctccaccac atttcactca gagcccacac agttttagag agagagaaac        60
   atccctcaaa gctctctctt tctccggcga tggttgtcgc tatggaccag cgtagcaatg       120
   tgaacggaga ttccaaggac gaaaggtttg atccgagcgc acaaccaccg tttaagatcg       180
   gagatataag ggctgcgatt cctaagcatt gttgggtcaa gagtcctttg agatccatga       240
   gctacgtcgc gagagacatt ttctccgtcg tggctctggc cgtcgccgcc gtgtattttg       300
   atagctggtt cttctggcct ctttattggg ccgcccaagg aacccttttc tgggccatct       360
   tcgtactcgg ccacgactgg taatttaatt ttcaatttat tttttcttca acttcttaat       420
   tttgatatgt ttatatgttt tttcgttttt tgcatcgtct ttgatttctt gaacgcacgt       480
   tcgatatgag attttcactg acttcaagat ttgattctct tcaggtttac ttttaaaaaa       540
   aaaaattatt atgttcaccc aaattggcct attttaaaag caaaagggga tctaagattt       600
   ttaattcttc tctttttcag tcgtaacact gctaactttt ttttttgatc aaatcgtaac      660
   actcataagt cctaactaaa catctttttc tttcctataa ttattgttgg ttccgcattt      720
   tatggatcta cgtttgaaag tttcaataaa acacatttta ttgtttgaaa gtaacaatat      780
   aattactgta tattgattct tttaattatt gtgtgttgtt ccaatctact ttcgaaatat      840
   agtcatgtga cacgtcatat tctatttttg ttaccttgtt ggaacgtttg aattgagtaa      900
   agtttaatta acattgtgca ataaatgata aacatgttta tgatgtaaaa ttcaatttga      960
   ataatacagt ggacatggga gcttctcaga cattcctctt ctgaatactg cggttggtca     1020
   tattcttcat tccttcattc tcgttccata ccatggttgg taagtcattt atttaaacat     1080
   ctttttcatg caaatttatt cttgttttcg tatttcttac attttccttg tcattcttgg     1140
   tgcatgttag caaactgtaa tctgataact gaaaatatat taattttcca tagtaaaata     1200
   atgcatgtga ctaaaagcat caaaatcttt agcatcgaag aaaaaagaac caaactttta     1260
   tttaatgcta tgggcctatt tatggtccaa ttagctatta tcatatgaca tgtccttgaa     1320
   taaattaatg tagcttcata tgtgagttta ataatattta tatatttttg ttttaatggc     1380
   ttattttatt gttaaatgga tacatcagct tgaaatgtct acgaacatgc atcattttcc     1440
   tagatacact tgtttgttgc tcaaaaatga ataacttagt taaacgagtg agcatgttct     1500
   atggggtttc ttagagcatg attattgaga agttcctaga gtgaggttct taccggaata     1560
   taagaatcta tctcttaact tttaactaaa aaaattaaga accggctttt aaaactcgta     1620
   tttaagaacc gttttttagt ttttttagtt aaaaatcaag agacgagttc ttatattccg     1680
   ctaagaactc caccctgaga acttctcaat aatcatgctc ttagtgctct aagaagggtc     1740
   cttaacaaaa tattaataat aagatatagt gtgggcccaa aaaaaacaaa aaaccggtta     1800
   caaaagtcgc gaaagaagga tcgattttgg tcttttactt gtactgtttg tggatcccac     1860
   tggtggtggt ccgcgattgg tttctttttt aatttaattt atttttttaa tcggagaaaa     1920
   aaattaagaa accaaaaaac agttttaatc atggcctcat gttggggttg agttttatat     1980
   tctgataaga atcccatctt aaaaaccccg ttaaacatgc tcttaccatc tgcttcgaaa     2040
   atgatatgtt attgacaatt ccaatttcat ttttatgaaa ataaaataat agtttatttt     2100
   ataactgagg gtggttgcag gagaataagc catcggacac accaccagaa ccatggccat     2160
   gttgaaaacg acgagtcttg ggttccggta atctttccct ctctcatatt ttttttcttt     2220
   tttttgaaat tctttcattt taattttctt aggattctat gtatttattt taatcaatcc     2280
   tttttccagt ttgaggctag gacgaccact tgtcagattt gtcgtttagc tgtagtaaac     2340
   aactgattta aattgtttat agtactgtag ttaactttaa caacggacca cttatattcg     2400
   agccattggc ataaaatgat tcttctcgaa attcgtttac ttttcttagt atttttcaat     2460
   tttggagttt acgtagaact aataaaaaga aaaacttata aacacaccac atgcaatgaa     2520
   taaattcgaa tatataacca tactgttaaa tattaattta cattttaatc ttaattttgc 2580
   attccagttg ccagaaaaat tatacaagaa tttgtcccac agtacacgga tgctcagata 2640
   cactgtccct ctccccatgc tcgcttaccc tctctatctg gtaaatccta attcctaatt 2700
   tttcttcctg attataatta caattttgaa tttttagatt ttgagtatta actaaatata 2760
   aattaaattt gtttggggat gactacagtg gtacagaagt cctggtaaag aagggtcaca 2820
   ttataaccca tacagtagtt tatttgcccc aagcgagaga aagcttattg caacttcaac 2880
   tacttgctgg tcgatcgtgt tggccactct tgtttatcta tcattcctcg ttggtccagt 2940
   cacagttcta aaagtctatg gtgttcctta cattgtaagt ttcatatatt tctttattat 3000
   atcattgcta atataatttg tttttgacat aaaagttttg gaaaaatttc agatctttgt 3060
   aatgtggttg gacgctgtca cgtacttgca tcatcatggt cacgatgata agctgccttg 3120
   gtacagaggc aaggtaagta gatcaacatt atttataaga agcaataatg attagtagtt 3180
   gaataatctg aatttttgat gtttttgtac aataatagga atggagttat ttacgtggag 3240
   gattaacaac tgttgataga gattacggga tcttcaacaa cattcatcac gatattggaa 3300
   ctcacgtgat ccatcatctt ttcccacaaa tccctcacta tcacttggtc gatgccgtga 3360
   gtgatctcgc tctctctcta gtttcatttg attatattaa agggtgatta attactaaat 3420
   tagtgatctt aattaatgac atgcgacaga cgaaagcagc taaacatgtg ttgggaagat 3480
   actacagaga accaaagacg tcaggagcaa taccgatcca cttagtggaa agtttggtgg 3540
   caagtattaa gaaagatcat tacgtcagtg acactggtga tattgtcttc tacgagacag 3600
   atccagatct ctacgtttat gcttctgaca aatccaaaat caattaatct ttcttcctag 3660
   ctctatttag gaataaaaca ctcctttggt tttacttatt tctggttgtt tttaagttaa 3720
   aaatgtactc gtgaaacttt tttttattaa atgtatttac attacaaatc gtaaaagttt 3780
   ttgttcgttt tctctatgtt tttagttaca aacttacaat caaaaag 3827
<210> 19
   <211> 4668
   <212> DNA
   <213> Brassica napus
<400> 19
   catcaaacct ttattcacca catttcactg aaaggccaca catctagaga gagaaacttc     60
   gtccaaatct ctctctccag cgatggttgt tgctatggac cagcgcagca atgttaacgg    120
   agattccggt gcccggaagg aagaagggtt tgatccaagc gcacaaccac cgtttaagat    180
   cggagatata agggcggcga ttcctaagca ttgctgggtg aagagtcctt tgagatctat    240
   gagctacgtc gccagagaca ttttcgccgt cgcggctctg gccatggccg ccgtgtattt    300
   tgatagctgg ttcctctggc cactctactg ggttgcccaa ggaacccttt tctgggccat    360
   cttcgttctt ggccacgact ggtaaattaa attttcagtt ttaattattt tgtctctttt 420
   tgttcaattt attaatttct tgaatgcacg ttcgatgagt atcgtcactg acttcaagat 480
   ttaattcttt tgaggttact ttttcatgtt taattattaa aaaataaaag aaaatatagg 540
   atctaagatt tttttcttca tcaatgttca agcatcgtca ctcatcagtc gtcagactcg 600
   taacaaaata tcttcttttc tataattaat attatttccg cattttatgg atctacgttt 660
   tgatgttctc aatttttgtt tctctttctc tagatccccg gaacttttaa ttataattat 720
   agtatagtat aatatcaaga aaatatactg tttatttttt tggcaacaaa tatattgttt 780
   tttgacaaga aaaatatatt gtttttttct tctttttgtg ttccaatcta ttttgtgatt 840
   tagacaagtg acacgtcata taccggattt gttaccttgt taaagagttt gagttaaaac 900
   aaatgtagaa aagttaaaat aaattgtgca ctaaatgata aatacgtttt tatgttaaat 960
   aatgatgtga aaataaaatt gaataatggc agtggacatg ggagtttctc agacattcct 1020
   ctgctgaaca gtgtggttgg tcacattctt cattcattca tcctcgttcc ttaccatggt 1080
   tggtaagtca tttattaact atttccatgt aaattattag tacttgtttt cgtatttctt 1140
   acattttcgt ttgttattct tgggtgcaat gctaggaaac tgtaatcagt attaactgga 1200
   agctaccaac ttttttttgt tgctagagta gcaattttat aattaaataa gaatcctatt 1260
   aaacaatgca tgtgactata tgaggttgct ttttctgttc aaaagcatca aatctttagc 1320
   agccaatgaa aaagaatcca aaccttttct taaatgatat gcgcctatct atggtcctga 1380
   gttttcttag ttcattaagt ataattagat tttgattttt ttttaggttt tcacttattg 1440
   ttatttgttt acatcagctt caaacatctt cgaaaaagac ttacatgcat caatttcctg 1500
   aggatttata gtttttttta cttatttctg cacaatgttt attagtaaaa agcatcaaat 1560
   gtttttttgc tcaaaaaaaa gaatgggatt gttagagcac tctattgtta gttgttcaat 1620
   aaatatatca actaaaaaaa caaaataaat ataaaatgag tgagattgtt aaatcattat 1680
   agagacaatt tcattttcac aaaaataaat aaatacataa cttttgtaat tggggtttgc 1740
   aggagaataa gccatcggac acaccaccag aaccatggcc atgttgaaaa cgacgagtct 1800
   tgggttccgg taatctttcc tactctcatt gtttctcttg tcttttattt atttgttctt 1860
   ttttgggaat tcattcttat gtctaagttc ttatgattat tgaagttctt aaggtggggt 1920
   tcttaacgga atatgagaac ctgtctctta acttttaact aaaaaagcta agaaccagct 1980
   tttaaataag agttttatga acacgttctt aattttttta gttaaaagtt aagaaacggg 2040
   ttcttatatt ccgctaagaa cctcttccta aaaaccccaa taatcatact cctaggattc 2100
   tatatgttta ttttattagt ttatgttttc agtctgaggt cagaccggcc acttgtcaga 2160
   tctgttttct agctgtagta aaaaacaatt tgcaagtgta atagttcagc ggtaattaat 2220
   gttctcggat ctatctcaaa aaaaaatttt ataacttcaa atataaagat ttttttgttt 2280
   ttcaaaaatg aacttcgaaa cttcaaattt gaagtttttt ttttgcattt tgatcattat 2340
   aattaattac acgttacatt tataattctt aagtattttt tcatttatcg ttttaattct 2400
   taaatttttt atatattata aatatttcca atttgttttt ataaattcaa attttataca 2460
   taaaagtaat aaaaatgtta aataagattt ataatattta agactataat tagtcaacaa 2520
   aatattacaa aagaaatgta ataataaaaa atttaaaata agatacatga agacataact 2580
   attagaaaat ttaaatatta taacaatact aataatctgg taaatttgct ctggaacctc 2640
   taaaattatt gtctaaacaa attttgtgta accgaagatg gagcattacg aaaataattt 2700
   tatgaaataa tatggtattt tgcttctagt ttaatattta attatatatt tctatttata 2760
   attttatata tttaatgtaa atttttatta attaatatta ctgtaatatt tttatatatg 2820
   tgctagttat ttataatttt ttttatggat ttatattaga ccatgattaa cccggagttc 2880
   ttagagtgga gttttagtta aacgttaaga aacagtttct taacttccgg taagaacccc 2940
   atcctaagaa tcccaggtta atcatgctct tagttataac aaataaggat cattgtgtaa 3000
   aatacaaata attttgaagt tatgtttgaa gtttgttttc gaagaaaacc actttgaaac 3060
   tttaaattta gagtaaactc tatttagaga gtttttttta gaggttacgc agtaactcag 3120
   aaaatgaaaa atctatactt ttatagtacc taactttatc gatggaccac ttatattcga 3180
   gtccttagca taacatgatt ctcctcgaaa tccgtttact ttcttcgtta ttttttcctt 3240
   ttcagttttg gcgttttcgt aatacttttg tctgcaatct tgaaagctat tagtataaaa 3300
   cttataaaca catgaattaa tacgaataca taaccagaat gacaaatttt caatgaatat 3360
   ttaatactag taagtactac tccgtaatag taattagtaa tagtaatagt aatagtcata 3420
   ttaattataa ttatgtattt cagttgccag aaaagttgta caagaacttg ccccatagta 3480
   ctcggatgct cagatacact gtccctctgc ccatgctcgc ttacccgatc tatctggtaa 3540
   aaaaaataca atttctattt tttcttaaaa ttacaaatga ttttatattt tgagttttaa 3600
   gccaatatat aaattaattt tgattggatt ttaactacag tggtacagaa gtcctggaaa 3660
   agaagggtca cattttaacc catacagtag tttatttgct ccaagcgaga ggaagcttat 3720
   tgcaacttca actacttgct ggtccataat gttggccact cttgtttatc tatcgttcct 3780
   cgttgatcca gtcacagttc tcaaagtcta tggcgttcct tacattgtaa gtttcacata 3840
   ttattacaag aaatttatat attattaata ataaatttgt tttttgacat aagggtttgg 3900
   aaaattttca gatctttgtg atgtggttgg acgctgtcac gtacttgcat catcatggtc 3960
   acgatgagaa gttgccttgg tacagaggca aggtaattaa atcaattttt aaaaagaaat 4020
   gtacagaaag caataatggt tagtattgat taatcttaat ttttgatgtt ttgcatacaa 4080
   taataggaat ggagttattt acgtggagga ttaacaacta ttgatagaga ttacggaatc 4140
   ttcaacaaca tccatcacga cattggaact cacgtgatcc atcatctttt cccacaaatc     4200
   cctcactatc acttggtcga tgccgtgagt gatctagctt tctctctctc tagtttcatt     4260
   tgattaaatg gtgattaatt actaatttaa ttaatgaatt gtggacagac gagagcagct     4320
   aaacatgtgt taggaagata ctacagagag ccgaagacgt caggagcaat accgattcac     4380
   ttggtggaga gtttggtcgc aagtattaaa aaagatcatt acgtcagtga cactggtgat     4440
   attgtcttct acgagacaga tccagatctc tacgtttatg cttctgacaa atctaaaatc     4500
   aattaacttt tcttcctagc tctattagga ataaacactc cttctctttt acttatttgt     4560
   ttctgcttta agtttaaaat gtactcgtga aacctttttt ttattaatgt atttacgtta     4620
   caaaaagtgg aagttttgtt atctttttct ctggttgcaa tcaaaagg                  4668
<210> 20
   <211> 5714
   <212> DNA
   <213> Brassica napus
<400> 20
   catcgaaccc tttcttcacc acattccagt tcccacactt tctttttttt gaattataga      60
   gagagaatct tcctccaaat ctctctctct ctctcccagg atggttgttg ctatggacca     120
   acgcaccaat gtgaacgaag atgccggtgc ccggaaggaa gaagggtttg atccgagcgc     180
   acaaccgccg tttaagatcg gggacataag ggctgcgatt cctaagcatt gttgggtgaa     240
   aagtcctttg agatctatga gctacgtagc cagagacatt tgtgccgtcg ctgctttggc     300
   cattgccgcc gtgtattttg atagctggtt cctctggcct ctctattggg tcgcccaagg     360
   aacccttttc tgggccatct tcgtcctcgg ccacgactgg taaagtttct tccattttgc      420
   attgcatcga tttattgaat gcacgttcta tgagtattgt cagtacttta tgaattgatt     480
   cttttgatgt tcattttttg aagatctaag attttttttt ttagattttc tttttaaatc      540
   attgttccac cacctttcat cggtcgtacg actcgttaca aaaccacatc tttattttct     600
   ataattacga ctgcttccgc attttatgga tctctcaact tataattaaa gtataaaatc     660
   aagaatatct attgtttttc taaaacaaga aagataatat tgtttctttg ttattttggt     720
   gtattccaat ctatttcgag atttagaaat gtgtcacgtc attaccttgt tgaagctttt     780
   aaaacaaaca tggaaagttt aaataaatag tgcaataaat gatatactat atttacgatg     840
   aataatgatg tgaaatataa ttgaataatg gcagtggaca tgtgagtttc tcagacattc     900
   ctctgctgaa tagcgtggtt ggccatattc ttcattcctt catcctcgtt ccttaccatg     960
   gttggtaagt caacttatta acccttttta ttattattat taattattaa actttcattt    1020
   gttatacttt ttttggttta aatgttaaat gaattacttg gtgcaagaat ctattcattg    1080
   ctcgttcttt ttttttttgg ctagagccaa ttttataatt aaataatgca tgtgaaagta    1140
   tgactatata tgtgaggtag cttttcttat tcttgacgaa aagcatcgaa tctttagcaa 1200
   cgaaggaaaa aggaatcaaa acttttatta aatgcaatgg gcctatatct ggtcattagt 1260
   attttgaata taatttattt ataatttttt ttgaacaaca gctaatttat ttataattaa 1320
   atattcattt tataaataat attaaaccaa ttattaaagg ttagatattt gaagaattat 1380
   tcatgacttt gtttattggg aaattactcc ttttatcttt tattcttttc tatttctcta 1440
   tttttaatat tgagaaactg acttcaaacc tccaataaaa atggtttcct gtagtaacat 1500
   cataattttt tgtttggtaa atgtaacatc atcttcaaat atctttgaaa atagacttac 1560
   atgcattatt ttgctgcgac attattgtaa cttattcctg gcaataaaaa taatttatta 1620
   ctggaaacta tttttggtca atttattact agtaacttaa aacttaaaag agtgagattg 1680
   tttgatcaaa aaaaaagaga aaaaaaatag agtgagattg ttagaatctg ccatgaaagc 1740
   aacactatat aggtgatgat tggttcgact gtggccgtag aattttagct gtagataaat 1800
   tggttgtagt tgtaaagttg ttactgttga ttatttttgc agagactttt gctgtagtta 1860
   aatttgttgt agctgtaagc tataggctgc agatatttta aaataaaata tgtaaaatat 1920
   gtgatgcatg tatatataaa ataattatta tttttatcac ttaaaataat ttatattaat 1980
   attttttaaa attatcaaag tttactgtta tttaaaatgt gatatgtaaa taatctatat 2040
   tatttaaaat atttcaataa tttaaaagca cccaaaatta gagtaaaata tttatagatg 2100
   tttttttatt atgattatct tatttattta atattataga tattttttgt tcttacagtt 2160
   tctacagctt ataaatgaaa gatgtaagtt gtttaactaa aatacataag aaaaatgttt 2220
   ggtttttttt ttgctgtagc tttattttta aagttaaagc atgattggta aaaattaata 2280
   gaaatttgat gtagacttta attttgaaaa gtaaacgtaa agcatgattg gtaaagttta 2340
   atgatttaga aaaaaataaa gctaaagtag gtagataaaa cccaaccaat cacctccatg 2400
   gacaatttaa tttttatgta aacacatatt taataatttg aggctgcagg agaataagcc 2460
   atcggacaca ccaccagaac catggccatg ttgaaaacga cgagtcttgg gttccggtaa 2520
   catttccctc tttaataatt tctatttttc tttgtcaaaa taatttgttt ttcgaaattt 2580
   gaggccagaa cgaccacttg tcagatttga tttctagctg tagtaaaaac agtttgctag 2640
   tgtcacagtt aaccggtaat tgattctttt tagcgattta tagaagtaac atttttgtaa 2700
   aataaaatat acataatagt atgtgacaac ggaccacgcc tatttgtatc ggtgaatctt 2760
   ctaattactt cctccgattt attttagtta cagttttaga tttatacaca tagattacaa 2820
   aaaataaaat attttgtcca tttttaaaat aaaaacatca ctaattatac acctaacaat 2880
   attttaacca ataaaaaata aactagaaaa tattattcat aatttttaca ttgaaattat 2940
   aaaacgatac ttattttaaa acaaaatttt aatttacaac gacaattaaa ttgaaacgga 3000
   agaagtttat tattacttaa ttaaagagtt tttttaaaaa aaatgaaaga catgtttatg 3060
   cgaaactcat gtgaaagtct ttcaaataaa atattttggt ataaattttt caaattttca 3120
   aaaataataa ttataaatta atataatata atttgtgata aaatctcgtc aaaaactcac 3180
   taatgcaaat gcttttatat ttgagtttct tactcctcta aatgcattta cttttatact 3240
   attattattt tctttctcta atttggtgtt ttcgtaatag tttgcctgtg ttttgaaaac 3300
   taacaaaaaa taataaaaac aaaagtttat aaacacatag catgcaatga atatatatat 3360
   caatacatat ctaagtacta tttttgcaag tacttaatct tgattactaa aattcatttt 3420
   aattgttcct ttcagttacc agaaaagtta tacaagattt taccccacag tactcggatg 3480
   ctcagataca ctgtccctct gcccatgctc gcttacccga tctatctggt attttttaat 3540
   tcctaaaact taccacaatt cattttagat tgtgttttaa aacaatataa attatttttt 3600
   ctttggtttt actgcagtgg tacagaagtc ctggaaaaga agggtcacat tttaacccat 3660
   acagtggttt atttgctcca agcgagagaa agcttattgc aacttcaact acttgctggt 3720
   ccataatgtt ggccattctt atctgtcttt ccttcctcgt tggtccagtc acagttctca 3780
   aagtatacgg tgttccttac atcgtaagtt tcttagtata tcataaaggg tatatattta 3840
   ttattcaata tatatactat atgatttgtt tttgtcataa acttttgaaa ttcagatctt 3900
   tgtgatgtgg ttggacgctg tcacttactt gcatcaccat ggtcatgatg agaagttgcc 3960
   ttggtacaga ggcaaggtaa ttaaattaac tcctaggtga ttttcccgtg ctcatgtacg 4020
   gatataaata tttctaaagt aaatatacta taataattaa ttgttattta tttttaattt 4080
   taaattagtt tataatttgt atgcatgatt tatattaata aaatttatat tactttaatt 4140
   ataaatatga ttttatatat gttatatcta atcggttttg ttgtttttac agtcgattta 4200
   gttatcattt gggtaaattg gattgcatct cagaattcaa ctgtaatatt ttttatttta 4260
   actatattaa aattttgatt aatttcttat tttcatttag gtggttgttg tcttagaact 4320
   ttaaatatat tttataaaga ttatgtataa cttaatatat atattgtgct taaaatgaaa 4380
   taaaaaataa aataaagtgt ctgattctaa attacataaa ttaatataac gataatattc 4440
   tgaagtctca tgcatatata tatataaatt ttacaaaaga actaaattgt aacatttggt 4500
   taatatttta cagtaattaa aatattttat aaattctaaa taactttatg tatttaattt 4560
   attgaatgga aactgaaatt tattttaaat aatcttaaaa atgaaaacat atttgctttg 4620
   gtattttgct tatggttcca ttaagttcta caaacataaa aacataacat ttaaaaactg 4680
   tgattatttt gtaactattt gatcaaacaa tgattatttt ttaattttaa ttttagtttt 4740
   ttaataactc ttaaaaataa gcagtgaaca aaagtgagat tgtatttgaa attaatatta 4800
   tacaagtaaa atataatttt ttaagtttat aaaaaaattc ctttttatta tatgtatatg 4860
   tttttttgga aaattttaaa aaggaaacta aataaaaaaa taaataatag tattttaaat 4920
   gtaatatttt taattcatta agtgtattag tgtaatcaac tatcgtgaga gttaacgtga 4980
   gagcgataca tagaaaaccg acttctcaaa taatatttta tagagattac gatgtttcac 5040
   aaaaaaaaat tattagtatt tgattaatct taattcttga tgttttgtga ttaataatag 5100
   gaatggagtt acttacgtgg aggattaaca actattgata gagattacgg aattttcaac 5160
   aacattcatc acgacattgg aactcacgtg atccatcatc ttttcccaca aatccctcac 5220
   tatcacttgg tcgatgctgt gagtcatctc actctctcgc tactttcatc taaaccattt 5280
   cattaaaggg tgattaatta ctaatgtact gattttaaca aatggaatgt gacagacaaa 5340
   agcagctaaa catgcgttgg gaagatacta cagagaaccg aagacgtcag gagcaatacc 5400
   gatccacttg gtggagagtt tggtagcaag tattaagaaa gatcattacg tcagtgacac 5460
   cggtgacatt gtcttctacg agactgatcc agatctctac gtttatgctt ctgtcaaatc 5520
   gaaaatcaat taaactttct tccccctttt tgtttagccc tattatgaat aaaccagtct 5580
   tttttcactt atttattggt gtttttaagt taaaaatgta ctcgtgaaac tcttctttta 5640
   ttattaatcc atttatacac tgaaaaacat acaatttcaa aggttaaaaa gaaaaataaa 5700
   ttttctagac tgac 5714
<210> 21
   <211> 20
   <212> DNA
   <213> Artificial Sequence
<220>
   <223> D_uni_F3_F1 Primer Sequence
<400> 21
   gaataagcca tcggacacac 20
<210> 22
   <211> 20
   <212> DNA
   <213> Artificial Sequence
<220>
   <223> D_spec_F3_F2 Primer Sequence
<400> 22
   atgcgaacgg agacgaaagg 20
<210> 23
   <211> 20
   <212> DNA
   <213> Artificial Sequence
<220>
   <223> D_spec_F3_F3 Primer Sequence
<400> 23
   tgttaacgga gattccggtg 20
<210> 24
   <211> 20
   <212> DNA
   <213> Artificial Sequence
<220>
   <223> D_spec_F3_F4 Primer Sequence
<400> 24
   gtagcaatgt gaacggagat 20
<210> 25
   <211> 19
   <212> DNA
   <213> Artificial Sequence
<220>
   <223> D_uni_F3_R1 Primer Sequence
<400> 25
   cagtgtatct gagcatccg 19
<210> 26
   <211> 20
   <212> DNA
   <213> Artificial Sequence
<220>
   <223> D_spec_F3_R2 Primer Sequence
<400> 26
   gtggccgagt acgaagatag 20
<210> 27
   <211> 19
   <212> DNA
   <213> Artificial Sequence
<220>
   <223> D_spec_F3_R3 Primer Sequence
<400> 27
   cagtagagtg gccagagga 19
<210> 28
   <211> 21
   <212> DNA
   <213> Artificial Sequence
<220>
   <223> D_UnivF2_F1 Primer Sequence
<400> 28
   atgggtgcag gtggaagaat g 21
<210> 29
   <211> 20
   <212> DNA
   <213> Artificial Sequence
<220>
   <223> D_UnivF2_F2 Primer Sequence
<400> 29
   agcgtctcca gatatacatc 20
<210> 30
   <211> 20
   <212> DNA
   <213> Artificial Sequence
<220>
   <223> D_UnivF2_R1 Primer Sequence
<400> 30
   atgtatatct ggagacgctc 20
<210> 31
   <211> 20
   <212> DNA
   <213> Artificial Sequence
<220>
   <223> D_UnivF2_R2 Primer Sequence
<400> 31
   tagatacact ccttcgcctc 20
<210> 32
   <211> 20
   <212> DNA
   <213> Artificial Sequence
<220>
   <223> D_SpecificF2_F3 Primer Sequence
<400> 32
   tctttctcct acctcatctg 20
<210> 33
   <211> 20
   <212> DNA
   <213> Artificial Sequence
<220>
   <223> D_SpecificF2_R3 Primer Sequence
<400> 33
   ttcgtagctt ccatcgcgtg 20
<210> 34
   <211> 19
   <212> DNA
   <213> Artificial Sequence
<220>
   <223> D_UnivF2_F4 Primer Sequence
<400> 34
   gacgccacca ttccaacac 19
<210> 35
   <211> 20
   <212> DNA
   <213> Artificial Sequence
<220>
   <223> D_UnivF2_R4 Artificial Sequence
<400> 35
   acttgccgta ccacttgatg 20
<210> 36
   <211> 28
   <212> DNA
   <213> Artificial Sequence
<220>
   <223> 27961 ZFP Target Site
<400> 36
   cgccggagaa agagagagag ctttgagg 28
<210> 37
   <211> 28
   <212> DNA
   <213> Artificial Sequence
<220>
   <223> 27962 ZFP Target Site
<400> 37
   tggttgtcgc tatggaccag cgtagcaa 28
<210> 38
   <211> 28
   <212> DNA
   <213> Artificial Sequence
<220>
   <223> 27969 ZFP Target Site
<400> 38
   tctccgttcg cattgctacg ctggtcca 28
<210> 39
   <211> 28
   <212> DNA
   <213> Artificial Sequence
<220>
   <223> ZFP Target Site
<400> 39
   gaaaggtttg atccgagcgc acaaccac 28
<210> 40
   <211> 28
   <212> DNA
   <213> Artificial Sequence
<220>
   <223> 27973 ZFP Target Site
<400> 40
   tctccgttcg cattgctacg ctggtcca 28
<210> 41
   <211> 28
   <212> DNA
   <213> Artificial Sequence
<220>
   <223> 27974 ZFP Target Site
<400> 41
   tcggagatat aagggcggcc attcctaa 28
<210> 42
   <211> 28
   <212> DNA
   <213> Artificial Sequence
<220>
   <223> 27987 Target Site
<400> 42
   tagcccagaa cagggttcct tgggcggc 28
<210> 43
   <211> 28
   <212> DNA
   <213> Artificial Sequence
<220>
   <223> 27988 ZFP Target Site
<400> 43
   cttcgtactc ggccacgact ggtaattt 28
<210> 44
   <211> 28
   <212> DNA
   <213> Artificial Sequence
<220>
   <223> 27989 ZFP Target Site
<400> 44
   ttgaagttgc aataagcttt ctctcgct 28
<210> 45
   <211> 28
   <212> DNA
   <213> Artificial Sequence
<220>
   <223> 27990 ZFP Target Site
<400> 45
   acttgctggt cgatcatgtt ggccactc 28
<210> 46
   <211> 28
   <212> DNA
   <213> Artificial Sequence
<220>
   <223> 27991 ZFP Target Site
<400> 46
   aagtagttga agttgcaata agctttct 28
<210> 47
   <211> 28
   <212> DNA
   <213> Artificial Sequence
<220>
   <223> 27992 ZFP Target Site
<400> 47
   tggtcgatca tgttggccac tcttgttt 28
<210> 48
   <211> 28
   <212> DNA
   <213> Artificial Sequence
<220>
   <223> 28004 ZFP Target Site
<400> 48
   aacgagaatg aaggaatgaa gaatatga 28
<210> 49
   <211> 28
   <212> DNA
   <213> Artificial Sequence
<220>
   <223> 28005 ZFP Target Site
<400> 49
   ataccatggt tggtaagtca tttatttt 28
<210> 50
   <211> 28
   <212> DNA
   <213> Artificial Sequence
<220>
   <223> 28021 ZFP Target Site
<400> 50
   ccaacgagga atgatagata aacaagag 28
<210> 51
   <211> 28
   <212> DNA
   <213> Artificial Sequence
<220>
   <223> 28022 ZFP Target Site
<400> 51
   cagtcacagt tctaaaagtc tatggtgt 28
<210> 52
   <211> 28
   <212> DNA
   <213> Artificial Sequence
<220>
   <223> 28023 ZFP Target Site
<400> 52
   tgtgactgga ccaacgagga atgataga 28
<210> 53
   <211> 28
   <212> DNA
   <213> Artificial Sequence
<220>
   <223> 28024 ZFP Target Site
<400> 53
   tctaaaagtc tatggtgttc cttacatt 28
<210> 54
   <211> 28
   <212> DNA
   <213> Artificial Sequence
<220>
   <223> 28025 ZFP Target Site
<400> 54
   cgccggagaa agagagagct ttgaggga 28
<210> 55
   <211> 28
   <212> DNA
   <213> Artificial Sequence
<220>
   <223> 28026 ZFP Target Site
<400> 55
   tggttgtcgc tatggaccag cgtagcaa 28
<210> 56
   <211> 28
   <212> DNA
   <213> Artificial Sequence
<220>
   <223> 28035 ZFP Target Site
<400> 56
   cttaaacggt ggttgtgcgc tcggatca 28
<210> 57
   <211> 28
   <212> DNA
   <213> Artificial Sequence
<220>
   <223> 28036 ZFP Target Site
<400> 57
   tcggagatat aagggctgcg attcctaa 28
<210> 58
   <211> 28
   <212> DNA
   <213> Artificial Sequence
<220>
   <223> 28039 ZFP Target Site
<400> 58
   tctccgatct taaacggtgg ttgtgcgc 28
<210> 59
   <211> 28
   <212> DNA
   <213> Artificial Sequence
<220>
   <223> 28040 ZFP Target Site
<400> 59
   ataagggctg cgattcctaa gcattgtt 28
<210> 60
   <211> 28
   <212> DNA
   <213> Artificial Sequence
<220>
   <223> 28051 ZFP Target Site
<400> 60
   agatggccca gaaaagggtt ccttgggc 28
<210> 61
   <211> 28
   <212> DNA
   <213> Artificial Sequence
<220>
   <223> 28052 ZFP Target Site
<400> 61
   cgtactcggc cacgactggt aatttaat 28
<210> 62
   <211> 28
   <212> DNA
   <213> Artificial Sequence
<220>
   <223> 28053 ZFP Target Site
<400> 62
   ttgaagttgc aataagcttt ctctcgct 28
<210> 63
   <211> 28
   <212> DNA
   <213> Artificial Sequence
<220>
   <223> 28054 ZFP Target Site
<400> 63
   acttgctggt cgatcgtgtt ggccactc 28
<210> 64
   <211> 28
   <212> DNA
   <213> Artificial Sequence
<220>
   <223> 28055 ZFP Target Site
<400> 64
   aagtagttga agttgcaata agctttct 28
<210> 65
   <211> 28
   <212> DNA
   <213> Artificial Sequence
<220>
   <223> 28056 ZFP Target Site
<400> 65
   tggtcgatcg tgttggccac tcttgttt 28
<210> 66
   <211> 28
   <212> DNA
   <213> Artificial Sequence
<220>
   <223> 24800 ZFP Target Site
<400> 66
   cccaaagggt tgttgaggta cttgccgt 28
<210> 67
   <211> 28
   <212> DNA
   <213> Artificial Sequence
<220>
   <223> 24801 ZFP Target Site
<400> 67
   cgcaccgtga tgttaacggt tcagttca 28
<210> 68
   <211> 28
   <212> DNA
   <213> Artificial Sequence
<220>
   <223> 24794 ZFP Target Site
<400> 68
   taagggacga ggaggaagga gtggaaga 28
<210> 69
   <211> 28
   <212> DNA
   <213> Artificial Sequence
<220>
   <223> 24795 ZFP Target Site
<400> 69
   ttctcctgga agtacagtca tcgacgcc 28
<210> 70
   <211> 28
   <212> DNA
   <213> Artificial Sequence
<220>
   <223> 24810 ZFP Target Site
<400> 70
   gtcgctgaag gcgtggtggc cgcactcg 28
<210> 71
   <211> 28
   <212> DNA
   <213> Artificial Sequence
<220>
   <223> 24811 ZFP Target Site
<400> 71
   cagtggctgg acgacaccgt cggcctca 28
<210> 72
   <211> 28
   <212> DNA
   <213> Artificial Sequence
<220>
   <223> 24814 ZFP Target Site
<400> 72
   gagaagtaag ggacgaggag gaaggagt 28
<210> 73
   <211> 28
   <212> DNA
   <213> Artificial Sequence
<220>
   <223> 24815 ZFP Target Site
<400> 73
   gaagtacagt catcgacgcc accattcc 28
<210> 74
   <211> 28
   <212> DNA
   <213> Artificial Sequence
<220>
   <223> 24818 ZFP Target Site
<400> 74
   tcccaaaggg ttgttgaggt acttgccg 28
<210> 75
   <211> 28
   <212> DNA
   <213> Artificial Sequence
<220>
   <223> 24819 ZFP Target Site
<400> 75
   accgtgatgt taacggttca gttcactc 28
<210> 76
   <211> 28
   <212> DNA
   <213> Artificial Sequence
<220>
   <223> 24796 ZFP Target Site
<400> 76
   gagaagtaag ggacgaggag gaaggagt 28
<210> 77
   <211> 28
   <212> DNA
   <213> Artificial Sequence
<220>
   <223> 24797 ZFP Target Site
<400> 77
   tggaagtaca gtcatcgacg ccaccatt 28
<210> 78
   <211> 28
   <212> DNA
   <213> Artificial Sequence
<220>
   <223> 24836 ZFP Target Site
<400> 78
   gtagagaccg tagcagacgg cgaggatg 28
<210> 79
   <211> 28
   <212> DNA
   <213> Artificial Sequence
<220>
   <223> 24837 ZFP Target Site
<400> 79
   gctacgctgc tgtccaagga gttgcctc 28
<210> 80
   <211> 28
   <212> DNA
   <213> Artificial Sequence
<220>
   <223> 24844 ZFP Target Site
<400> 80
   gaggccaggc gaagtaggag agagggtg 28
<210> 81
   <211> 28
   <212> DNA
   <213> Artificial Sequence
<220>
   <223> 24845 ZFP Target Site
<400> 81
   actgggcctg ccagggctgc gtcctaac 28
<210> 82
   <211> 28
   <212> DNA
   <213> Artificial Sequence
<220>
   <223> 24820 ZFP Target Site
<400> 82
   gagaggccag gcgaagtagg agagaggg 28
<210> 83
   <211> 28
   <212> DNA
   <213> Artificial Sequence
<220>
   <223> 24821 ZFP Target Site
<400> 83
   actgggcctg ccagggctgc gtcctaac 28
<210> 84
   <211> 28
   <212> DNA
   <213> Artificial Sequence
<220>
   <223> 24828 ZFP Target Site
<400> 84
   aggcccagta gagaggccag gcgaagta 28
<210> 85
   <211> 28
   <212> DNA
   <213> Artificial Sequence
<220>
   <223> 24829 ZFP Target Site
<400> 85
   ccagggctgc gtcctaaccg gcgtctgg 28
<210> 86
   <211> 28
   <212> DNA
   <213> Artificial Sequence
<220>
   <223> 24832 ZFP Target Site
<400> 86
   tagtcgctga aggcgtggtg gccgcact 28
<210> 87
   <211> 28
   <212> DNA
   <213> Artificial Sequence
<220>
   <223> 24833 ZFP Target Site
<400> 87
   agtggctgga cgacaccgtc ggcctcat 28
<210> 88
   <211> 56
   <212> DNA
   <213> Artificial Sequence
<220>
   <223> FAD2_ZFN_Locus1_F PCR Primer
<400> 88
   acactctttc cctacacgac gctcttccga tctcgggccc tctcycytac ytcgcc 56
<210> 89
   <211> 57
   <212> DNA
   <213> Artificial Sequence
<220>
   <223> FAD2_ZFN_Locus1_F2A PCR Primer
<400> 89
   acactctttc cctacacgac gctcttccga tctacgtacc ctctcycyta cytcgcc 57
<210> 90
   <211> 57
   <212> DNA
   <213> Artificial Sequence
<220>
   <223> FAD2_ZFN_Locus1_F2B PCR Primer
<400> 90
   acactctttc cctacacgac gctcttccga tctcgtaccc ctctcycyta cytcgcc 57
<210> 91
   <211> 57
   <212> DNA
   <213> Artificial Sequence
<220>
   <223> FAD2_ZFN_Locus1_F2C PCR Primer
<400> 91
   acactctttc cctacacgac gctcttccga tctgtacgcc ctctcycyta cytcgcc 57
<210> 92
   <211> 59
   <212> DNA
   <213> Artificial Sequence
<220>
   <223> FAD2_ZFN_Locus2_F1D PCR Primer
<400> 92
   acactctttc cctacacgac gctcttccga tcttacgtgt catagcccac gagtgcggc 59
<210> 93
   <211> 59
   <212> DNA
   <213> Artificial Sequence
<220>
   <223> FAD2_ZFN_Locus2_F1E PCR Primer
<400> 93
   acactctttc cctacacgac gctcttccga tctctgacgt catagcccac gagtgcggc 59
<210> 94
   <211> 59
   <212> DNA
   <213> Artificial Sequence
<220>
   <223> FAD2_ZFN_Locus3_F2F PCR Primer
<400> 94
   acactctttc cctacacgac gctcttccga tcttgactgt cggcctcatc ttccactcc 59
<210> 95
   <211> 59
   <212> DNA
   <213> Artificial Sequence
<220>
   <223> FAD2_ZFN_Locus3_F2G PCR Primer
<400> 95
   acactctttc cctacacgac gctcttccga tctgactggt cggcctcatc ttccactcc 59
<210> 96
   <211> 59
   <212> DNA
   <213> Artificial Sequence
<220>
   <223> FAD2_ZFN_Locus3_F2H PCR Primer
<400> 96
   acactctttc cctacacgac gctcttccga tctactgagt cggcctcatc ttccactcc 59
<210> 97
   <211> 58
   <212> DNA
   <213> Artificial Sequence
<220>
   <223> FAD2_ZFN_Locus4_F1J PCR Primer
<400> 97
   acactctttc cctacacgac gctcttccga tctgctagca gacatcaagt ggtacggc 58
<210> 98
   <211> 58
   <212> DNA
   <213> Artificial Sequence
<220>
   <223> FAD2_ZFN_Locus4_F1K PCR Primer
<400> 98
   acactctttc cctacacgac gctcttccga tctctagcca gacatcaagt ggtacggc 58
<210> 99
   <211> 59
   <212> DNA
   <213> Artificial Sequence
<220>
   <223> FAD2_ZFN_Locus5_F1L PCR Primer
<400> 99
   acactctttc cctacacgac gctcttccga tcttagctat ctccgacgct ggcatcctc 59
<210> 100
   <211> 62
   <212> DNA
   <213> Artificial Sequence
<220>
   <223> FAD2_ZFN_Locus1_R1A PCR Primer
<400> 100
   cggtctcggc attcctgctg aaccgctctt ccgatctacg tactggtagt cgctgaaggc 60
   gt 62
<210> 101
   <211> 62
   <212> DNA
   <213> Artificial Sequence
<220>
   <223> FAD2_ZFN_Locus1_R1B PCR Primer
<400> 101
   cggtctcggc attcctgctg aaccgctctt ccgatctcgt acctggtagt cgctgaaggc 60
   gt 62
<210> 102
   <211> 62
   <212> DNA
   <213> Artificial Sequence
<220>
   <223> FAD2_ZFN_Locus1_R1C PCR Primer
<400> 102
   cggtctcggc attcctgctg aaccgctctt ccgatctgta cgctggtagt cgctgaaggc 60
   gt 62
<210> 103
   <211> 63
   <212> DNA
   <213> Artificial Sequence
<220>
   <223> FAD2_ZFN_Locus2_R1D PCR Primer
<400> 103
   cggtctcggc attcctgctg aaccgctctt ccgatcttac gtggacgagg aggaaggagt 60
   gga 63
<210> 104
   <211> 63
   <212> DNA
   <213> Artificial Sequence
<220>
   <223> FAD2_ZFN_Locus2_R1E PCR Primer
<400> 104
   cggtctcggc attcctgctg aaccgctctt ccgatctctg acggacgagg aggaaggagt 60
   gga 63
<210> 105
   <211> 63
   <212> DNA
   <213> Artificial Sequence
<220>
   <223> FAD2_ZFN_Locus3_R1F PCR Primer
<400> 105
   cggtctcggc attcctgctg aaccgctctt ccgatcttga ctagtgttgg aatggtggcg 60
   tcg 63
<210> 106
   <211> 63
   <212> DNA
   <213> Artificial Sequence
<220>
   <223> FAD2_ZFN_Locus3_R1G PCR Primer
<400> 106
   cggtctcggc attcctgctg aaccgctctt ccgatctgac tgagtgttgg aatggtggcg 60
   tcg 63
<210> 107
   <211> 63
   <212> DNA
   <213> Artificial Sequence
<220>
   <223> FAD2_ZFN_Locus3_R1H PCR Primer
<400> 107
   cggtctcggc attcctgctg aaccgctctt ccgatctact gaagtgttgg aatggtggcg 60
   tcg 63
<210> 108
   <211> 63
   <212> DNA
   <213> Artificial Sequence
<220>
   <223> FAD2_ZFN_Locus4_R1J PCR Primer
<400> 108
   cggtctcggc attcctgctg aaccgctctt ccgatctgct agcccgagac gttgaaggct 60
   aag 63
<210> 109
   <211> 63
   <212> DNA
   <213> Artificial Sequence
<220>
   <223> FAD2_ZFN_Locus4_R1K PCR Primer
<400> 109
   cggtctcggc attcctgctg aaccgctctt ccgatctcta gccccgagac gttgaaggct 60
   aag 63
<210> 110
   <211> 63
   <212> DNA
   <213> Artificial Sequence
<220>
   <223> FAD2_ZFN_Locus5_R1L PCR Primer
<400> 110
   cggtctcggc attcctgctg aaccgctctt ccgatcttag ctgaaggatg cgtgtgctgc 60
   aag 63
<210> 111
   <211> 58
   <212> DNA
   <213> Artificial Sequence
<220>
   <223> FAD3_ZFN_Locus1A_F3 PCR Primer
<400> 111
   acactctttc cctacacgac gctcttccga tctacgtacc tttcttcacc acattyca 58
<210> 112
   <211> 58
   <212> DNA
   <213> Artificial Sequence
<220>
   <223> FAD3_ZFN_Locus1B_F3 PCR Primer
<400> 112
   acactctttc cctacacgac gctcttccga tctcgtaccc tttcttcacc acattyca 58
<210> 113
   <211> 58
   <212> DNA
   <213> Artificial Sequence
<220>
   <223> FAD3_ZFN_Locus2C_F1 PCR Primer
<400> 113
   acactctttc cctacacgac gctcttccga tctctgacga tggttgtcgc tatggacc 58
<210> 114
   <211> 58
   <212> DNA
   <213> Artificial Sequence
<220>
   <223> FAD3_ZFN_Locus3D_F1 PCR Primer
<400> 114
   acactctttc cctacacgac gctcttccga tcttgactcg aaaggtttga tccragcg 58
<210> 115
   <211> 58
   <212> DNA
   <213> Artificial Sequence
<220>
   <223> FAD3_ZFN_Locus3E_F1 PCR Primer
<400> 115
   acactctttc cctacacgac gctcttccga tctgactgcg aaaggtttga tccragcg 58
<210> 116
   <211> 58
   <212> DNA
   <213> Artificial Sequence
<220>
   <223> FAD3_ZFN_Locus3F_F1 PCR Primer
<400> 116
   acactctttc cctacacgac gctcttccga tctactgacg aaaggtttga tccragcg 58
<210> 117
   <211> 61
   <212> DNA
   <213> Artificial Sequence
<220>
   <223> FAD3_ZFN_Locus4G_F1 PCR Primer
<400> 117
   acactctttc cctacacgac gctcttccga tctgctagcc gtgtattttg atagctggtt 60
   c 61
<210> 118
   <211> 61
   <212> DNA
   <213> Artificial Sequence
<220>
   <223> FAD3_ZFN_Locus4H_F1 PCR Primer
<400> 118
   acactctttc cctacacgac gctcttccga tctctagccc gtgtattttg atagctggtt 60
   c 61
<210> 119
   <211> 60
   <212> DNA
   <213> Artificial Sequence
<220>
   <223> FAD3_ZFN_Locus5J_F1 PCR Primer
<400> 119
   acactctttc cctacacgac gctcttccga tcttagctgg agcttctcag acattcctct 60
<210> 120
   <211> 60
   <212> DNA
   <213> Artificial Sequence
<220>
   <223> FAD3_ZFN_Locus6K_F1 PCR Primer
<400> 120
   acactctttc cctacacgac gctcttccga tcttcagtgt ttatttgccc caagcgagag 60
<210> 121
   <211> 60
   <212> DNA
   <213> Artificial Sequence
<220>
   <223> FAD3_ZFN_Locus6L_F1 PCR Primer
<400> 121
   acactctttc cctacacgac gctcttccga tctcagtcgt ttatttgccc caagcgagag 60
<210> 122
   <211> 60
   <212> DNA
   <213> Artificial Sequence
<220>
   <223> FAD3_ZFN_Locus6M_F1 PCR Primer
<400> 122
   acactctttc cctacacgac gctcttccga tctagtcagt ttatttgccc caagcgagag 60
<210> 123
   <211> 60
   <212> DNA
   <213> Artificial Sequence
<220>
   <223> FAD3_ZFN_Locus6N_F1 PCR Primer
<400> 123
   acactctttc cctacacgac gctcttccga tctgtcaggt ttatttgccc caagcgagag 60
<210> 124
   <211> 61
   <212> DNA
   <213> Artificial Sequence
<220>
   <223> FAD3_ZFN_Locus7P_F3 PCR Primer
<400> 124
   acactctttc cctacacgac gctcttccga tctgtacgac ttcaactact tgctggtcsa 60
   t 61
<210> 125
   <211> 61
   <212> DNA
   <213> Artificial Sequence
<220>
   <223> FAD3_ZFN_Locus7Q_F3 PCR Primer
<400> 125
   acactctttc cctacacgac gctcttccga tcttacgtac ttcaactact tgctggtcsa 60
   t 61
<210> 126
   <211> 62
   <212> DNA
   <213> Artificial Sequence
<220>
   <223> FAD3_ZFN_Locus1A_R1 PCR Primer
<400> 126
   cggtctcggc attcctgctg aaccgctctt ccgatctacg tacgttcaca ttgstrcgyt 60
   gg 62
<210> 127
   <211> 62
   <212> DNA
   <213> Artificial Sequence
<220>
   <223> FAD3_ZFN_Locus1B_R1 PCR Primer
<400> 127
   cggtctcggc attcctgctg aaccgctctt ccgatctcgt accgttcaca ttgstrcgyt 60
   gg 62
<210> 128
   <211> 63
   <212> DNA
   <213> Artificial Sequence
<220>
   <223> FAD3_ZFN_Locus2C_R1 PCR Primer
<400> 128
   cggtctcggc attcctgctg aaccgctctt ccgatctctg acccgatctt aaacggyggt 60
   tgt 63
<210> 129
   <211> 65
   <212> DNA
   <213> Artificial Sequence
<220>
   <223> FAD3_ZFN_Locus3D_R1 PCR Primer
<400> 129
   cggtctcggc attcctgctg aaccgctctt ccgatcttga cttagctcat ggatctcaaa 60
   ggact 65
<210> 130
   <211> 65
   <212> DNA
   <213> Artificial Sequence
<220>
   <223> FAD3_ZFN_Locus3E_R1 PCR Primer
<400> 130
   cggtctcggc attcctgctg aaccgctctt ccgatctgac tgtagctcat ggatctcaaa 60
   ggact 65
<210> 131
   <211> 65
   <212> DNA
   <213> Artificial Sequence
<220>
   <223> FAD3_ZFN_Locus3F_R1 PCR Primer
<400> 131
   cggtctcggc attcctgctg aaccgctctt ccgatctact gatagctcat ggatctcaaa 60
   ggact 65
<210> 132
   <211> 62
   <212> DNA
   <213> Artificial Sequence
<220>
   <223> FAD3_ZFN_Locus4G_R_uni PCR Primer
<400> 132
   cggtctcggc attcctgctg aaccgctctt ccgatctgct agttaaatta ccagtcgtgg 60
   cc 62
<210> 133
   <211> 62
   <212> DNA
   <213> Artificial Sequence
<220>
   <223> FAD3_ZFN_Locus4H_R_uni PCR Primer
<400> 133
   cggtctcggc attcctgctg aaccgctctt ccgatctcta gcttaaatta ccagtcgtgg 60
   cc 62
<210> 134
   <211> 66
   <212> DNA
   <213> Artificial Sequence
<220>
   <223> FAD3_ZFN_Locus5J_R2 PCR Primer
<400> 134
   cggtctcggc attcctgctg aaccgctctt ccgatcttag ctcttttttc ttcgatkcta 60
   aagatt 66
<210> 135
   <211> 62
   <212> DNA
   <213> Artificial Sequence
<220>
   <223> FAD3_ZFN_Locus6K_R1 PCR Primer
<400> 135
   cggtctcggc attcctgctg aaccgctctt ccgatcttca gtctgtgact ggaccaacga 60
   gg 62
<210> 136
   <211> 62
   <212> DNA
   <213> Artificial Sequence
<220>
   <223> FAD3_ZFN_Locus6L_Rl PCR Primer
<400> 136
   cggtctcggc attcctgctg aaccgctctt ccgatctcag tcctgtgact ggaccaacga 60
   gg 62
<210> 137
   <211> 62
   <212> DNA
   <213> Artificial Sequence
<220>
   <223> FAD3_ZFN_Locus6M_R1 PCR Primer
<400> 137
   cggtctcggc attcctgctg aaccgctctt ccgatctagt cactgtgact ggaccaacga 60
   gg 62
<210> 138
   <211> 62
   <212> DNA
   <213> Artificial Sequence
<220>
   <223> FAD3_ZFN_Locus6N_R1 PCR Primer
<400> 138
   cggtctcggc attcctgctg aaccgctctt ccgatctgtc agctgtgact ggaccaacga 60
   gg 62
<210> 139
   <211> 66
   <212> DNA
   <213> Artificial Sequence
<220>
   <223> FAD3_ZFN_Locus7P_R1 PCR Primer
<400> 139
   cggtctcggc attcctgctg aaccgctctt ccgatctgta cgacttacaa tgtaaggaac 60
   rccrta 66
<210> 140
   <211> 66
   <212> DNA
   <213> Artificial Sequence
<220>
   <223> FAD3_ZFN_Locus7Q_R1 PCR Primer
<400> 140
   cggtctcggc attcctgctg aaccgctctt ccgatcttac gtacttacaa tgtaaggaac        60
   rccrta                                                                   66
<210> 141
   <211> 13472
   <212> DNA
   <213> Artificial Sequence
<220>
   <223> T-stand Insert Region of pDAS000130
<400> 141
   gaaatatcct tcctattcaa agttatatat atttgtttac ttttgtttta gatctggacc        60
   tgagacatgt aagtacatat ttgttgaatc tttgggtaaa aacttatgtc tctgggtaaa       120
   atttgctgag agatttgacc gattcctatt ggctctggat tctgtagtta cctaatacat       180
   gaaaaagttt catttggcct atgctcactt catgcttata aactttttct tgcaaattaa       240
   ttggattaga tgctccttca tagattcaga tgcaatagat ttgcatgaag aaaataatag       300
   gattcatgat agtaaaaaga ttgtattttt gtttgtttgt ttatgtttaa aagtctatat       360
   gttgacaata gagttgctat caactgtttc atttaggttt atgtttttgt caagttgctt       420
   attctaagag acattgtgat tatgacttgt cttctctaac gtagtttagt aataaaagac       480
   gaaagaaatt gatatccaca agaaagagat gtaagctgta acgtatcaaa tctcattaat       540
   aactagtagt attctcaacg ctatcgttta tttctttctt tggtttgcca ctatatgccg       600
   cttctctgct ctttatccca cgtactatcc attttttttg tggtagtcca tttttttgaa       660
   actttaataa cgtaacactg aatattaatt tgttggttta attaactttg agtctttgct       720
   tttggtttat gcagaaacat gggtgcaggt ggaagaatgc aagtgtctcc tccctccaaa       780
   aagtctgaaa ccgacaacat caagcgcgta ccctgcgaga caccgccctt cactgtcgga       840
   gaactcaaga aagcaatccc accgcactgt ttcaaacgct cgatccctcg ctctttctcc       900
   tacctcatct gggacatcat catagcctcc tgcttctact acgtcgccac cacttacttc       960
   cctctcctcc ctcaccctct ctcctacttc gcctggcctc tctactgggc cggtaccgcc      1020
   ttttgcagtt tatctctatg cccgggacaa gtggagtcca tgctcaacac cgtgcaggat      1080
   gaggatgacc accgcggtag cgacttcgtg ggcgaggaaa gcctttcgtc caaggtggtc 1140
   cctcctcgca atcttgttgg atggtgaata ttataaaagc ctgcccttct cgcgggtgtt 1200
   taaacgtcga cctgcaggtc aacggatcag gatattcttg tttaagatgt tgaactctat 1260
   ggaggtttgt atgaactgat gatctaggac cggataagtt cccttcttca tagcgaactt 1320
   attcaaagaa tgttttgtgt atcattcttg ttacattgtt attaatgaaa aaatattatt 1380
   ggtcattgga ctgaacacga gtgttaaata tggaccaggc cccaaataag atccattgat 1440
   atatgaatta aataacaaga ataaatcgag tcaccaaacc acttgccttt tttaacgaga 1500
   cttgttcacc aacttgatac aaaagtcatt atcctatgca aatcaataat catacaaaaa 1560
   tatccaataa cactaaaaaa ttaaaagaaa tggataattt cacaatatgt tatacgataa 1620
   agaagttact tttccaagaa attcactgat tttataagcc cacttgcatt agataaatgg 1680
   caaaaaaaaa caaaaaggaa aagaaataaa gcacgaagaa ttctagaaaa tacgaaatac 1740
   gcttcaatgc agtgggaccc acggttcaat tattgccaat tttcagctcc accgtatatt 1800
   taaaaaataa aacgataatg ctaaaaaaat ataaatcgta acgatcgtta aatctcaacg 1860
   gctggatctt atgacgaccg ttagaaattg tggttgtcga cgagtcagta ataaacggcg 1920
   tcaaagtggt tgcagccggc acacacgagt cgtgtttatc aactcaaagc acaaatactt 1980
   ttcctcaacc taaaaataag gcaattagcc aaaaacaact ttgcgtgtaa acaacgctca 2040
   atacacgtgt cattttatta ttagctattg cttcaccgcc ttagctttct cgtgacctag 2100
   tcgtcctcgt cttttcttct tcttcttcta taaaacaata cccaaagagc tcttcttctt 2160
   cacaattcag atttcaattt ctcaaaatct taaaaacttt ctctcaattc tctctaccgt 2220
   gatcaaggta aatttctgtg ttccttattc tctcaaaatc ttcgattttg ttttcgttcg 2280
   atcccaattt cgtatatgtt ctttggttta gattctgtta atcttagatc gaagacgatt 2340
   ttctgggttt gatcgttaga tatcatctta attctcgatt agggtttcat agatatcatc 2400
   cgatttgttc aaataatttg agttttgtcg aataattact cttcgatttg tgatttctat 2460
   ctagatctgg tgttagtttc tagtttgtgc gatcgaattt gtcgattaat ctgagttttt 2520
   ctgattaaca gatggcttca tctgagaacg ttatcactga gttcatgagg ttcaaggtga 2580
   ggatggaagg tactgttaac ggacatgagt tcgagatcga gggtgagggt gaaggtagac 2640
   cttacgaggg acataacacc gttaagctta aggttacaaa gggtggacct cttcctttcg 2700
   cttgggatat cctttctcct caattccaat acggaagcaa ggtaagtttg tggattcttc 2760
   gtccatgtga tctttgagtt tctttagagc ttgtgaggga ttagtaagta acaatgcttg 2820
   agttttttgc tgctgggctt cgaaaagttt gtcacttgtt ggtttgatcc acaaggtctt 2880
   cttctccata gctactagac atgttttagc ttaagattca agtttatata tgccttgtgg 2940
   attaatcatt gcctgattct tccgtgtcat ctctgagttt atttagagct tggaagtggt 3000
   gtagtaataa ctaacaatac tcttgataag ttgtagcaat gctcttgatt agtggatgta 3060
   atatgatgtt gataagatat atgaggcaca gaaccaaaag tggtgcttcc actagacccg 3120
   tttttagcct aaggttcaag tttatacctt gtagatgttt ctgtattgtc tgattcttcc 3180
   ctgtgatatt tgaatttctt agagctttgg aagtgatata ggaacaatgc tcttgtgtgt 3240
   ttgtctctat gaagattatc gctgtcgtgt ttcatccgag tgtgcgggat tttttgctgc 3300
   tgggtttagc ctttcttcaa aaagttatta cttgttagtt ttattgtttt ggtcttgata 3360
   agagatgtta ggacagacat ggtgcttctt gtctatagcc actagaccta ttttagcata 3420
   aggttaacga aattctctct acataccttg tggatttgtt tacattgcct gatctttcct 3480
   gtgatcgctg tcatgtttct ttggaatgat tgatgtttat aaatggaaaa atctttgtgc 3540
   agaagactcc cgcccatctc tctatgcccg ggacaagtgc caccccacag tggggcagga 3600
   tgaggatgac caccatgggg tcgcagcgtg tgcgtgtccg tcgtacgttc tggccggccg 3660
   ggccttgggc gcgcgatcag aagcgttgcg ttggcgtgtg tgtgcttctg gtttgcttta 3720
   attttaccaa gtttgtttca aggtggatcg cgtggtcaag gcccgtgtgc tttaaagacc 3780
   caccggcact ggcagtgagt gttgctgctt gtgtaggctt tggtacgtat gggctttatt 3840
   tgcttctgga tgttgtgtac tacttgggtt tgttgaatta ttatgagcag ttgcgtattg 3900
   taattcagct gggctacctg gacattgtta tgtattaata aatgctttgc tttcttctaa 3960
   agatctttaa gtgctgttta aacaaccgac aaccactttg cggacttcct ttcaagagaa 4020
   ttcaataagg ttaattccta attgaaatcc gaagataaga ttcccacaca cttgtggctg 4080
   atatcaaaag gctactgcct atttaaacac atctctggag actgagaaaa tcagacctcc 4140
   aagcatgaag aagcctgagc ttactgctac ttctgttgag aagttcctca tcgagaagtt 4200
   cgattctgtg tctgatctta tgcagctctc tgagggtgag gaatcaagag ctttctcttt 4260
   cgatgttggt ggaagaggat acgttctcag agttaactct tgcgctgacg gattctacaa 4320
   ggatagatac gtgtacagac acttcgcttc agctgctctc cctatccctg aagttcttga 4380
   tatcggagag ttctctgagt ctcttaccta ctgtatctca agaagggctc agggtgttac 4440
   tcttcaagat cttcctgaga ctgagcttcc tgctgttctt caacctgttg ctgaggctat 4500
   ggatgctatc gctgctgctg atctttctca aacttctgga ttcggacctt tcggtcctca 4560
   gggaatcgga cagtacacta cttggagaga tttcatctgc gctatcgctg atcctcatgt 4620
   ttaccattgg cagaccgtta tggatgatac cgtttctgct tctgttgctc aagctcttga 4680
   tgagcttatg ctttgggctg aggattgtcc tgaggttaga catcttgttc acgctgattt 4740
   cggatctaac aacgttctca ccgataacgg aagaatcacc gctgttatcg attggtctga 4800
   ggctatgttc ggagattctc aatacgaggt ggccaacata ttcttttgga ggccttggct 4860
   tgcttgtatg gaacaacaga ctagatactt cgagagaagg catcctgagc ttgctggatc 4920
   tcctagactt agagcttaca tgcttaggat cggacttgat cagctttacc agtctctcgt 4980
   tgatggaaac ttcgatgatg ctgcttgggc tcagggaaga tgtgatgcta tcgttagatc 5040
   tggtgctgga actgttggaa gaactcaaat cgctagaaga tctgctgctg tttggactga 5100
   tggatgtgtt gaagttctcg ctgattctgg aaacagaagg ccttctacta gacctagagc 5160
   caagaagtga agatcggcgg caatagcttc ttagcgccat cccgggttga tcctatctgt 5220
   gttgaaatag ttgcggtggg caaggctctc tttcagaaag acaggcggcc aaaggaaccc 5280
   aaggtgaggt gggctatggc tctcagttcc ttgtggaagc gcttggtcta aggtgcagag 5340
   gtgttagcgg gatgaagcaa aagtgtccga ttgtaacaag atatgttgat cctacgtaag 5400
   gatattaaag tatgtattca tcactaatat aatcagtgta ttccaatatg tactacgatt 5460
   tccaatgtct ttattgtcgc cgtatgtaat cggcgtcaca aaataatccc cggtgacttt 5520
   cttttaatcc aggatgaaat aatatgttat tataattttt gcgatttggt ccgttatagg 5580
   aattgaagtg tgcttgcggt cgccaccact cccatttcat aattttacat gtatttgaaa 5640
   aataaaaatt tatggtattc aatttaaaca cgtatacttg taaagaatga tatcttgaaa 5700
   gaaatatagt ttaaatattt attgataaaa taacaagtca ggtattatag tccaagcaaa 5760
   aacataaatt tattgatgca agtttaaatt cagaaatatt tcaataactg attatatcag 5820
   ctggtacatt gccgtagatg aaagactgag tgcgatatta tggtgtaata catagtttaa 5880
   acgggcccaa gactcccgcc catctctcta tgcccgggac aagtgccacc ccacagtggg 5940
   gcaggatgag gatgaccagt cagttttact tcccttaatt ttctatgtac tttcataatt 6000
   acttatgtta ttttcttcat gagttttaat gcaaattact atatggactc tagtgaaaac 6060
   gttcagaatc ctataaacat gactactgag acgaacttga gagtagtttt gatcatacac 6120
   acgtttcatg tggtacttga gagttactaa tttttgtcat cttcgtataa gtagtaaaag 6180
   atactacaag aatagtttag tagaaaatac tagcggtagg tgaagatttg tcgctatgta 6240
   ctattattgt ctagtaactt gagtaacaat ttcgtggtct aaatatcaaa taaaaatgga 6300
   tgagtggttc accaaatcta ggcatcaaaa ctattaatgt cattgtctag atcttaggtg 6360
   acaccacatt tcgaatattt attggtaatt gagatgttaa agtaccaata tttgacttaa 6420
   taaactaaaa gattttggct ttatcaaatg tagacattga tgacatatcg ttgtcattat 6480
   cttgagtata tacaagtcga tcaattaggt gaaagtttag tgtctcgtgg ttggtaaacg 6540
   attaatacag tagtatattt tatccaaaga caaaatccaa atcatttcac cagtatgaat 6600
   agtattattt tatcttaaaa gctaaaatct taaaaaccaa ggtagcaccc acgttgagct 6660
   agacgatcaa atcgatttct gctttgtcca atttaccaag ctatttaaag ccaaataatt 6720
   gaaatatagg taggtcgtta tattaggcta agatttatct caaatgctta actaaaggaa 6780
   taacaaggga ttctagttgt gtggttttat aagattggtc caatttcact taagtttgtt 6840
   tattgtagaa ttttatatgt gaataatttg aattccaatt gaaaagatat tatagtaaaa 6900
   gaaaaaatag tgcgaacaaa aaactttaat cccataaaaa gaaaaagaaa aatgaaaagt 6960
   tcttctaaca tccatatttt gcatcatatc ataaagataa gaaagataca tatcatagac 7020
   gtacagataa acaaacatat catcatttgt gaaatacata gtacaataat ttgcttttaa 7080
   atagagttta agtcacacac actgacacac acgataaaac gataatgtct gcaaaaacac 7140
   tttaatccca ttgcctagag gacagcttct ccactttgtc tttaaggttg gttttgccgt 7200
   gttgttttta tctttatata atgatctatt ttttggatta tgaaatgaat tcacacattt 7260
   taattattta agaagatcca tatacaggtt tataacagta ctaagtgatg attatttttt 7320
   gtttttgcat agtttagttt attgggtaaa cattcattac gtgtctcttt atacgaatca 7380
   cccatccaaa atttcaagta gtcttttagt tcatttatta tttcataact atttgactta 7440
   ttgatttgac aagaaacaac aaaagtgttg acttattgat agattgtggg atcataaaag 7500
   taattaagcg tcaaccacga cccacaacaa caaagcacat gttatacatt aatatctcgt 7560
   ttacttaatt acagttttca gaatgccgtt tcatgtcttg tcactggcga tgttattatc 7620
   atgttggaca atattcgact gttgtcgttt ttacattttc gtattgacta aaactaaaaa 7680
   aacaaaactc tgtttcaggt tgggcctagg atccacattg tacacacatt tgcttaagtc 7740
   tatggaggcg caaggtttta agtctgtggt tgctgttata ggccttccaa acgatccatc 7800
   tgttaggttg catgaggctt tgggatacac agcccggggt acattgcgcg cagctggata 7860
   caagcatggt ggatggcatg atgttggttt ttggcaaagg gattttgagt tgccagctcc 7920
   tccaaggcca gttaggccag ttacccagat ctaatatcaa aatctattta gaaatacaca 7980
   atattttgtt gcaggcttgc tggagaatcg atctgctatc ataaaaatta caaaaaaatt 8040
   ttatttgcct caattatttt aggattggta ttaaggacgc ttaaattatt tgtcgggtca 8100
   ctacgcatca ttgtgattga gaagatcagc gatacgaaat attcgtagta ctatcgataa 8160
   tttatttgaa aattcataag aaaagcaaac gttacatgaa ttgatgaaac aatacaaaga 8220
   cagataaagc cacgcacatt taggatattg gccgagatta ctgaatattg agtaagatca 8280
   cggaatttct gacaggagca tgtcttcaat tcagcccaaa tggcagttga aatactcaaa 8340
   ccgccccata tgcaggagcg gatcattcat tgtttgtttg gttgcctttg ccaacatggg 8400
   agtccaaggt tgtttaaaca tttaaatacc ctgccaagct tgaggtagcc tccaatttga 8460
   cggtgccgcc agcgacgccg tctggaactg tcctttttga ggaccactcc gtttgtggag 8520
   atcatgagag tccatgctca acaccgtgca ctagggacag gattgaagac tcccgcccat 8580
   ctcactaggg acaggattgc caccccacag tggggcctag aaagactgga gttgcagagt 8640
   ttgtgtcttc tagattaatc ctccaaactt ttgattaacc aaaaaaatta tcaaactaac 8700
   atgttctcct tttttcttta gaaattctaa cgaatttatc tttatactga tttgaatata 8760
   cttaatttgg tcatttggat gccctttaca acctccttac caaactattg atcacagttt 8820
   ctattgctaa aatcaccaac aaaacgcatg tcgccattca taattatggt ttcacaccta 8880
   caactaggct aataagtaaa taagtagaca actagactca ggtttgaaaa aaccataaaa 8940
   gccatatagc gttttctcat tgaaactgcg aacacgatcg tgtgaatgtt gcagtttcta 9000
   gttttgatac aaacaaacaa aaacacaatt taatcttaga ttaaaaagaa aaaagagaac 9060
   ggagcccact agccactcct tcaaacgtgt cttaccaact ctcttctaga aacaaattag 9120
   gcttcacctt cctcttccaa cctctctctc tctctctctc tctttttctc aaaccatctc 9180
   tccataaagc cctaatttct tcatcacaag aatcagaaga agaaagatgg acctgcatct 9240
   aattttcggt ccaacttgca caggaaagac gacgaccgcg atagctcttg cccagcagac 9300
   agggcttcca gtcctttcgc ttgatcgggt ccaatgctgt cctcaactat caaccggaag 9360
   cggacgacca acagtggaag aactgaaagg aacgacgcgt ctctaccttg atgatcggcc 9420
   tctggtggag ggtatcatcg cagccaagca agctcatcat aggctgatcg aggaggtgta 9480
   taatcatgag gccaacggcg ggcttattct tgagggagga tccacctcgt tgctcaactg 9540
   catggcgcga aacagctatt ggagtgcaga ttttcgttgg catattattc gccacaagtt 9600
   acccgaccaa gagaccttca tgaaagcggc caaggccaga gttaagcaga tgttgcaccc 9660
   cgctgcaggc cattctatta ttcaagagtt ggtttatctt tggaatgaac ctcggctgag 9720
   gcccattctg aaagagatcg atggatatcg atatgccatg ttgtttgcta gccagaacca 9780
   gatcacggca gatatgctat tgcagcttga cgcaaatatg gaaggtaagt tgattaatgg 9840
   gatcgctcag gagtatttca tccatgcgcg ccaacaggaa cagaaattcc cccaagttaa 9900
   cgcagccgct ttcgacggat tcgaaggtca tccgttcgga atgtattaga aatcaccagt 9960
   ctctctctac aaatctatct ctctctattt ttctccagaa taatgtgtga gtagttccca 10020
   gataagggaa ttagggttct tatagggttt cgctcatgtg ttgagcatat aagaaaccct 10080
   tagtatgtat ttgtatttgt aaaatacttc tatcaataaa atttctaatt cctaaaacca 10140
   aaatccagtg tttaaacgag tccatgctca acaccgtgca ctagggacag gattgaagac 10200
   tcccgcccat ctcactaggg acaggattgc caccccacag tggggcctag aaagactgga 10260
   gttgcagaca ttaaggatga ccagttcgta aaggtcctgc ggtgtctatt gcttttcata 10320
   ggttaataag tgtttgctag actgtggtga aaggcctatc cgaagtaagg ccggccggat 10380
   ccttcatctt tggacaaggg aataaagact ccccacttgc tactaagaac aatacctaag 10440
   ttgcccagac atgactgtac ccattcagag acctaccacc cattagggct atgacactaa 10500
   cactagcccc tggaggttga ccatgctagg cagtgggggt ctcacctatg acccactcag 10560
   ataggggttt aaaccagtgg gtgggatctc agcctcatat aggtgtttgt ggtgagcttt 10620
   ctcctagaca agagaaccct gaagaacagc aagaaccagc taatatgata tgtagacata 10680
   gtgggttgct caaattttgt gtttagtcat attagaattg acctcagtga ccactcagaa 10740
   agtgcccaag cccatctata ggggccaaag tgctattgac tggtgtgtct gtgaattgtt 10800
   cctccctaca gagttggtgc tgatatatcc tagcattctt tggaaaacct agctagggac 10860
   tgtcaagtgt aagatacctc ctgaattgga gggaacacta gctgccctgt accttctggc 10920
   tagtacctta caccctgaat gggttagggg gtctattatt tgctggaaat ataccagttt 10980
   cagtagggct gctgccttag gtcccacaag gtgtaacatg tgctcaatag ttgcactacc 11040
   acatgcacgt gaacttaatg atgttatagc cacaacacca accttggttt gcagtttgac 11100
   atccctctgg aatgggtgta gtcatcttgc tctggatctg cctgaatcat tggggctgta 11160
   tgcagcctgg gcttaaagtg aagaatggga tgtcccagaa atattttggg tgagaagaat 11220
   cctggagtag atggtgacct gactatccct gtcctatggg cacaatctat catcagatat 11280
   tgcattcaaa gggctatcat gggatcaagt cctaagtcaa ctgttgttta cctggcagac 11340
   attcatctag gagttctctt ttatgccacc ccacagtgat ccgccttttg cagtttatcc 11400
   actagggaca ggattgccac cccacagtgg ggcctctatg cccgggacaa gtgtaaaata 11460
   tagagtatag gggttatcat cacagagaag ctattgctgg agggcctctg ttatttcctc 11520
   tccatgccac tcccattttt aacctaccaa ctgaaatccc aagggagact ccaccctgta 11580
   actagagtcc tcagaggtga gccatcccat attaacaaat gggcattagg gctaggatgc 11640
   caagggatac ctgaaatggg aagttgtggg gctgagtcct cctgggaatc agagataata 11700
   tgtaaacagt ttgttgagag attgatgaga gctgactttg agaggtggcc atgctccctg 11760
   gtcctcaata gggaaggcac tacacaagaa acctgggttt gatcaactgc actgtgtcct 11820
   actcacacat tgtgtgcctg gaaaaatgtt acttagtatt tggagggcct ccagaacccc 11880
   cctgggtgca agactgggtg ctagtgactg ggtgaatgag tcttggacac agtggccttg 11940
   tctaggttgt gtgaggtggc taggcatcat ggcaatacct cataattgat gagtgaggaa 12000
   acaagactaa gtccttgact cctcttatta catgacctgg tggatattat gtttaaactc 12060
   tgcaagctgg aatgagtact gggtgcagat cccctgggat tctggctaca aaggtgaatg 12120
   atagctagtc tgtttattag tagccaaaaa agtcagtgag gggtgagtgc cctgggatgt 12180
   tgttaagttc acattgcaca cttggagacc ctctccatcc agtaacatac cagagaaaac 12240
   tgaccaagcc ctcatgggtg tatgggaaca acaaacctcc tggctacttc aagggcacat 12300
   aacaccagca aggagcctgt cataaccacc atctcaaaca atagaacttc ctaagtgaag 12360
   caatgacttc aaatctactt gaaggcatgg agtataagcc atgttccttt cagaggggac 12420
   tgtacttctg tagattactt tccctcatta accagatctg gccggccgca tgccagggct     12480
   gcgtcctaac cggcgtctgg gtcatagccc acgagtgcgg ccaccacgcc ttcagcgact     12540
   accagtggct ggacgacacc gtcggcctca tcttccactc cttcctcctc gtcccttact     12600
   tctcctggaa gtacagtcat cgacgccacc attccaacac tggctccctc gagagagacg     12660
   aagtgtttgt ccccaagaag aagtcagaca tcaagtggta cggcaagtac ctcaacaacc     12720
   ctttgggacg caccgtgatg ttaacggttc agttcactct cggctggcct ttgtacttag     12780
   ccttcaacgt ctcggggaga ccttacgacg gcggcttcgc ttgccatttc caccccaacg     12840
   ctcccatcta caacgaccgt gagcgtctcc agatatacat ctccgacgct ggcatcctcg     12900
   ccgtctgcta cggtctctac cgctacgctg ctgtccaagg agttgcctcg atggtctgct     12960
   tctacggagt tcctcttctg attgtcaacg ggttcttagt tttgatcact tacttgcagc     13020
   acacgcatcc ttccctgcct cactatgact cgtctgagtg ggattggttg aggggagctt     13080
   tggccaccgt tgacagagac tacggaatct tgaacaaggt cttccacaat atcacggaca     13140
   cgcacgtggc gcatcacctg ttctcgacca tgccgcatta tcacgcgatg gaagctacga     13200
   aggcgataaa gccgatactg ggagagtatt atcagttcga tgggacgccg gtggttaagg     13260
   cgatgtggag ggaggcgaag gagtgtatct atgtggaacc ggacaggcaa ggtgagaaga     13320
   aaggtgtgtt ctggtacaac aataagttat gaagcaaaga agaaactgaa cctttctcat     13380
   ctatgattgt ctttgtttta agaagctatg tttctgtttc aataatcttt aattatccat     13440
   tttgttgtgt tttctgacat tttggctaaa at                                   13472
<210> 142
   <211> 13462
   <212> DNA
   <213> Artificial Sequence
<220>
   <223> T-strand Insert Region of pDAS000271
<400> 142
   taaataaaaa ctgatggaag tctgtttctt aagtcaaata catcacagtg atgtggcaac       60
   tattttccct caaattaata cgttttaaaa aaaatctata taaatgttgg catgtctaca      120
   atctacatga tatccatatg gatcgttttt tatgatttat acatagtcag gaaattttag      180
   cagaaacaaa atagagtacg aagactaaca taatattttc gactacatgt attttttttg       240
   cgaaattgta aatatcaatc agtgaaaatg aaaaaccata caagttgact accatttcgg      300
   tgcacaatcc ttacttctaa ggaaaaacta aagagaaaca aaagaagaaa atcttggtaa      360
   attttgatac cattaccatg gttacttata ctcgataatg caattttaaa atcttctgta      420
   aattttatag cattgttttt tttgtaacac atttctctaa cttagttttc atcgaaatga      480
   acgacgtaac aaagatacat tgcgcacagg ttaccgcaaa aatacaattt ttattcttca      540
   aagaataaaa aagtttccta aattaagaaa aaaagaaaac agtttggtgt ctctacacat 600
   cttctccctt tatataaaca aaccacacat accccaaagt ccatcaaact ctctccacca 660
   catttcactc agagcccaca cagttttaga gagagagaaa catccctcaa agctctctct 720
   ttctccggcg atggttgtcg ctatggacca gcgtagcaat gtgaacggag attccaagga 780
   cgaaaggttt gatccgagcg cacaaccacc gtttaagatc ggagatataa gggctgcgat 840
   tcctaagcat tgttgggtca agagtccttt gagatccatg agctacgtcg cgagagacat 900
   tttctccgtc gtggctctgg ccgtcgccgc cgtgtatttt gatagctggt tcttctggcc 960
   tctttattgg gccgcccaag gaaccctttt ctgggccatc ggtaccgcct tttgcagttt 1020
   atctctatgc ccgggacaag tggagtccat gctcaacacc gtgcaggatg aggatgacca 1080
   ccgcggtagc gacttcgtgg gcgaggaaag cctttcgtcc aaggtggtcc ctcctcgcaa 1140
   tcttgttgga tggtgaatat tataaaagcc tgcccttctc gcgggtgttt aaacgtcgac 1200
   ctgcaggtca acggatcagg atattcttgt ttaagatgtt gaactctatg gaggtttgta 1260
   tgaactgatg atctaggacc ggataagttc ccttcttcat agcgaactta ttcaaagaat 1320
   gttttgtgta tcattcttgt tacattgtta ttaatgaaaa aatattattg gtcattggac 1380
   tgaacacgag tgttaaatat ggaccaggcc ccaaataaga tccattgata tatgaattaa 1440
   ataacaagaa taaatcgagt caccaaacca cttgcctttt ttaacgagac ttgttcacca 1500
   acttgataca aaagtcatta tcctatgcaa atcaataatc atacaaaaat atccaataac 1560
   actaaaaaat taaaagaaat ggataatttc acaatatgtt atacgataaa gaagttactt 1620
   ttccaagaaa ttcactgatt ttataagccc acttgcatta gataaatggc aaaaaaaaac 1680
   aaaaaggaaa agaaataaag cacgaagaat tctagaaaat acgaaatacg cttcaatgca 1740
   gtgggaccca cggttcaatt attgccaatt ttcagctcca ccgtatattt aaaaaataaa 1800
   acgataatgc taaaaaaata taaatcgtaa cgatcgttaa atctcaacgg ctggatctta 1860
   tgacgaccgt tagaaattgt ggttgtcgac gagtcagtaa taaacggcgt caaagtggtt 1920
   gcagccggca cacacgagtc gtgtttatca actcaaagca caaatacttt tcctcaacct 1980
   aaaaataagg caattagcca aaaacaactt tgcgtgtaaa caacgctcaa tacacgtgtc 2040
   attttattat tagctattgc ttcaccgcct tagctttctc gtgacctagt cgtcctcgtc 2100
   ttttcttctt cttcttctat aaaacaatac ccaaagagct cttcttcttc acaattcaga 2160
   tttcaatttc tcaaaatctt aaaaactttc tctcaattct ctctaccgtg atcaaggtaa 2220
   atttctgtgt tccttattct ctcaaaatct tcgattttgt tttcgttcga tcccaatttc 2280
   gtatatgttc tttggtttag attctgttaa tcttagatcg aagacgattt tctgggtttg 2340
   atcgttagat atcatcttaa ttctcgatta gggtttcata gatatcatcc gatttgttca 2400
   aataatttga gttttgtcga ataattactc ttcgatttgt gatttctatc tagatctggt 2460
   gttagtttct agtttgtgcg atcgaatttg tcgattaatc tgagtttttc tgattaacag 2520
   atggcttcat ctgagaacgt tatcactgag ttcatgaggt tcaaggtgag gatggaaggt 2580
   actgttaacg gacatgagtt cgagatcgag ggtgagggtg aaggtagacc ttacgaggga 2640
   cataacaccg ttaagcttaa ggttacaaag ggtggacctc ttcctttcgc ttgggatatc 2700
   ctttctcctc aattccaata cggaagcaag gtaagtttgt ggattcttcg tccatgtgat 2760
   ctttgagttt ctttagagct tgtgagggat tagtaagtaa caatgcttga gttttttgct 2820
   gctgggcttc gaaaagtttg tcacttgttg gtttgatcca caaggtcttc ttctccatag 2880
   ctactagaca tgttttagct taagattcaa gtttatatat gccttgtgga ttaatcattg 2940
   cctgattctt ccgtgtcatc tctgagttta tttagagctt ggaagtggtg tagtaataac 3000
   taacaatact cttgataagt tgtagcaatg ctcttgatta gtggatgtaa tatgatgttg 3060
   ataagatata tgaggcacag aaccaaaagt ggtgcttcca ctagacccgt ttttagccta 3120
   aggttcaagt ttataccttg tagatgtttc tgtattgtct gattcttccc tgtgatattt 3180
   gaatttctta gagctttgga agtgatatag gaacaatgct cttgtgtgtt tgtctctatg 3240
   aagattatcg ctgtcgtgtt tcatccgagt gtgcgggatt ttttgctgct gggtttagcc 3300
   tttcttcaaa aagttattac ttgttagttt tattgttttg gtcttgataa gagatgttag 3360
   gacagacatg gtgcttcttg tctatagcca ctagacctat tttagcataa ggttaacgaa 3420
   attctctcta cataccttgt ggatttgttt acattgcctg atctttcctg tgatcgctgt 3480
   catgtttctt tggaatgatt gatgtttata aatggaaaaa tctttgtgca gaagactccc 3540
   gcccatctct ctatgcccgg gacaagtgcc accccacagt ggggcaggat gaggatgacc 3600
   accatggggt cgcagcgtgt gcgtgtccgt cgtacgttct ggccggccgg gccttgggcg 3660
   cgcgatcaga agcgttgcgt tggcgtgtgt gtgcttctgg tttgctttaa ttttaccaag 3720
   tttgtttcaa ggtggatcgc gtggtcaagg cccgtgtgct ttaaagaccc accggcactg 3780
   gcagtgagtg ttgctgcttg tgtaggcttt ggtacgtatg ggctttattt gcttctggat 3840
   gttgtgtact acttgggttt gttgaattat tatgagcagt tgcgtattgt aattcagctg 3900
   ggctacctgg acattgttat gtattaataa atgctttgct ttcttctaaa gatctttaag 3960
   tgctgtttaa acaaccgaca accactttgc ggacttcctt tcaagagaat tcaataaggt 4020
   taattcctaa ttgaaatccg aagataagat tcccacacac ttgtggctga tatcaaaagg 4080
   ctactgccta tttaaacaca tctctggaga ctgagaaaat cagacctcca agcatgaaga 4140
   agcctgagct tactgctact tctgttgaga agttcctcat cgagaagttc gattctgtgt 4200
   ctgatcttat gcagctctct gagggtgagg aatcaagagc tttctctttc gatgttggtg 4260
   gaagaggata cgttctcaga gttaactctt gcgctgacgg attctacaag gatagatacg 4320
   tgtacagaca cttcgcttca gctgctctcc ctatccctga agttcttgat atcggagagt 4380
   tctctgagtc tcttacctac tgtatctcaa gaagggctca gggtgttact cttcaagatc 4440
   ttcctgagac tgagcttcct gctgttcttc aacctgttgc tgaggctatg gatgctatcg 4500
   ctgctgctga tctttctcaa acttctggat tcggaccttt cggtcctcag ggaatcggac 4560
   agtacactac ttggagagat ttcatctgcg ctatcgctga tcctcatgtt taccattggc 4620
   agaccgttat ggatgatacc gtttctgctt ctgttgctca agctcttgat gagcttatgc 4680
   tttgggctga ggattgtcct gaggttagac atcttgttca cgctgatttc ggatctaaca 4740
   acgttctcac cgataacgga agaatcaccg ctgttatcga ttggtctgag gctatgttcg 4800
   gagattctca atacgaggtg gccaacatat tcttttggag gccttggctt gcttgtatgg 4860
   aacaacagac tagatacttc gagagaaggc atcctgagct tgctggatct cctagactta 4920
   gagcttacat gcttaggatc ggacttgatc agctttacca gtctctcgtt gatggaaact 4980
   tcgatgatgc tgcttgggct cagggaagat gtgatgctat cgttagatct ggtgctggaa 5040
   ctgttggaag aactcaaatc gctagaagat ctgctgctgt ttggactgat ggatgtgttg 5100
   aagttctcgc tgattctgga aacagaaggc cttctactag acctagagcc aagaagtgaa 5160
   gatcggcggc aatagcttct tagcgccatc ccgggttgat cctatctgtg ttgaaatagt 5220
   tgcggtgggc aaggctctct ttcagaaaga caggcggcca aaggaaccca aggtgaggtg 5280
   ggctatggct ctcagttcct tgtggaagcg cttggtctaa ggtgcagagg tgttagcggg 5340
   atgaagcaaa agtgtccgat tgtaacaaga tatgttgatc ctacgtaagg atattaaagt 5400
   atgtattcat cactaatata atcagtgtat tccaatatgt actacgattt ccaatgtctt 5460
   tattgtcgcc gtatgtaatc ggcgtcacaa aataatcccc ggtgactttc ttttaatcca 5520
   ggatgaaata atatgttatt ataatttttg cgatttggtc cgttatagga attgaagtgt 5580
   gcttgcggtc gccaccactc ccatttcata attttacatg tatttgaaaa ataaaaattt 5640
   atggtattca atttaaacac gtatacttgt aaagaatgat atcttgaaag aaatatagtt 5700
   taaatattta ttgataaaat aacaagtcag gtattatagt ccaagcaaaa acataaattt 5760
   attgatgcaa gtttaaattc agaaatattt caataactga ttatatcagc tggtacattg 5820
   ccgtagatga aagactgagt gcgatattat ggtgtaatac atagtttaaa cgggcccaag 5880
   actcccgccc atctctctat gcccgggaca agtgccaccc cacagtgggg caggatgagg 5940
   atgaccagtc agttttactt cccttaattt tctatgtact ttcataatta cttatgttat 6000
   tttcttcatg agttttaatg caaattacta tatggactct agtgaaaacg ttcagaatcc 6060
   tataaacatg actactgaga cgaacttgag agtagttttg atcatacaca cgtttcatgt 6120
   ggtacttgag agttactaat ttttgtcatc ttcgtataag tagtaaaaga tactacaaga 6180
   atagtttagt agaaaatact agcggtaggt gaagatttgt cgctatgtac tattattgtc 6240
   tagtaacttg agtaacaatt tcgtggtcta aatatcaaat aaaaatggat gagtggttca 6300
   ccaaatctag gcatcaaaac tattaatgtc attgtctaga tcttaggtga caccacattt 6360
   cgaatattta ttggtaattg agatgttaaa gtaccaatat ttgacttaat aaactaaaag 6420
   attttggctt tatcaaatgt agacattgat gacatatcgt tgtcattatc ttgagtatat 6480
   acaagtcgat caattaggtg aaagtttagt gtctcgtggt tggtaaacga ttaatacagt 6540
   agtatatttt atccaaagac aaaatccaaa tcatttcacc agtatgaata gtattatttt 6600
   atcttaaaag ctaaaatctt aaaaaccaag gtagcaccca cgttgagcta gacgatcaaa 6660
   tcgatttctg ctttgtccaa tttaccaagc tatttaaagc caaataattg aaatataggt 6720
   aggtcgttat attaggctaa gatttatctc aaatgcttaa ctaaaggaat aacaagggat 6780
   tctagttgtg tggttttata agattggtcc aatttcactt aagtttgttt attgtagaat 6840
   tttatatgtg aataatttga attccaattg aaaagatatt atagtaaaag aaaaaatagt 6900
   gcgaacaaaa aactttaatc ccataaaaag aaaaagaaaa atgaaaagtt cttctaacat 6960
   ccatattttg catcatatca taaagataag aaagatacat atcatagacg tacagataaa 7020
   caaacatatc atcatttgtg aaatacatag tacaataatt tgcttttaaa tagagtttaa 7080
   gtcacacaca ctgacacaca cgataaaacg ataatgtctg caaaaacact ttaatcccat 7140
   tgcctagagg acagcttctc cactttgtct ttaaggttgg ttttgccgtg ttgtttttat 7200
   ctttatataa tgatctattt tttggattat gaaatgaatt cacacatttt aattatttaa 7260
   gaagatccat atacaggttt ataacagtac taagtgatga ttattttttg tttttgcata 7320
   gtttagttta ttgggtaaac attcattacg tgtctcttta tacgaatcac ccatccaaaa 7380
   tttcaagtag tcttttagtt catttattat ttcataacta tttgacttat tgatttgaca 7440
   agaaacaaca aaagtgttga cttattgata gattgtggga tcataaaagt aattaagcgt 7500
   caaccacgac ccacaacaac aaagcacatg ttatacatta atatctcgtt tacttaatta 7560
   cagttttcag aatgccgttt catgtcttgt cactggcgat gttattatca tgttggacaa 7620
   tattcgactg ttgtcgtttt tacattttcg tattgactaa aactaaaaaa acaaaactct 7680
   gtttcaggtt gggcctagga tccacattgt acacacattt gcttaagtct atggaggcgc 7740
   aaggttttaa gtctgtggtt gctgttatag gccttccaaa cgatccatct gttaggttgc 7800
   atgaggcttt gggatacaca gcccggggta cattgcgcgc agctggatac aagcatggtg 7860
   gatggcatga tgttggtttt tggcaaaggg attttgagtt gccagctcct ccaaggccag 7920
   ttaggccagt tacccagatc taatatcaaa atctatttag aaatacacaa tattttgttg 7980
   caggcttgct ggagaatcga tctgctatca taaaaattac aaaaaaattt tatttgcctc 8040
   aattatttta ggattggtat taaggacgct taaattattt gtcgggtcac tacgcatcat 8100
   tgtgattgag aagatcagcg atacgaaata ttcgtagtac tatcgataat ttatttgaaa 8160
   attcataaga aaagcaaacg ttacatgaat tgatgaaaca atacaaagac agataaagcc 8220
   acgcacattt aggatattgg ccgagattac tgaatattga gtaagatcac ggaatttctg 8280
   acaggagcat gtcttcaatt cagcccaaat ggcagttgaa atactcaaac cgccccatat 8340
   gcaggagcgg atcattcatt gtttgtttgg ttgcctttgc caacatggga gtccaaggtt 8400
   gtttaaacat ttaaataccc tgccaagctt gaggtagcct ccaatttgac ggtgccgcca 8460
   gcgacgccgt ctggaactgt cctttttgag gaccactccg tttgtggaga tcatgagagt 8520
   ccatgctcaa caccgtgcac tagggacagg attgaagact cccgcccatc tcactaggga 8580
   caggattgcc accccacagt ggggcctaga aagactggag ttgcagagtt tgtgtcttct 8640
   agattaatcc tccaaacttt tgattaacca aaaaaattat caaactaaca tgttctcctt 8700
   ttttctttag aaattctaac gaatttatct ttatactgat ttgaatatac ttaatttggt 8760
   catttggatg ccctttacaa cctccttacc aaactattga tcacagtttc tattgctaaa 8820
   atcaccaaca aaacgcatgt cgccattcat aattatggtt tcacacctac aactaggcta 8880
   ataagtaaat aagtagacaa ctagactcag gtttgaaaaa accataaaag ccatatagcg 8940
   ttttctcatt gaaactgcga acacgatcgt gtgaatgttg cagtttctag ttttgataca 9000
   aacaaacaaa aacacaattt aatcttagat taaaaagaaa aaagagaacg gagcccacta 9060
   gccactcctt caaacgtgtc ttaccaactc tcttctagaa acaaattagg cttcaccttc 9120
   ctcttccaac ctctctctct ctctctctct ctttttctca aaccatctct ccataaagcc 9180
   ctaatttctt catcacaaga atcagaagaa gaaagatgga cctgcatcta attttcggtc 9240
   caacttgcac aggaaagacg acgaccgcga tagctcttgc ccagcagaca gggcttccag 9300
   tcctttcgct tgatcgggtc caatgctgtc ctcaactatc aaccggaagc ggacgaccaa 9360
   cagtggaaga actgaaagga acgacgcgtc tctaccttga tgatcggcct ctggtggagg 9420
   gtatcatcgc agccaagcaa gctcatcata ggctgatcga ggaggtgtat aatcatgagg 9480
   ccaacggcgg gcttattctt gagggaggat ccacctcgtt gctcaactgc atggcgcgaa 9540
   acagctattg gagtgcagat tttcgttggc atattattcg ccacaagtta cccgaccaag 9600
   agaccttcat gaaagcggcc aaggccagag ttaagcagat gttgcacccc gctgcaggcc 9660
   attctattat tcaagagttg gtttatcttt ggaatgaacc tcggctgagg cccattctga 9720
   aagagatcga tggatatcga tatgccatgt tgtttgctag ccagaaccag atcacggcag 9780
   atatgctatt gcagcttgac gcaaatatgg aaggtaagtt gattaatggg atcgctcagg 9840
   agtatttcat ccatgcgcgc caacaggaac agaaattccc ccaagttaac gcagccgctt 9900
   tcgacggatt cgaaggtcat ccgttcggaa tgtattagaa atcaccagtc tctctctaca 9960
   aatctatctc tctctatttt tctccagaat aatgtgtgag tagttcccag ataagggaat 10020
   tagggttctt atagggtttc gctcatgtgt tgagcatata agaaaccctt agtatgtatt 10080
   tgtatttgta aaatacttct atcaataaaa tttctaattc ctaaaaccaa aatccagtgt 10140
   ttaaacgagt ccatgctcaa caccgtgcac tagggacagg attgaagact cccgcccatc 10200
   tcactaggga caggattgcc accccacagt ggggcctaga aagactggag ttgcagacat 10260
   taaggatgac cagttcgtaa aggtcctgcg gtgtctattg cttttcatag gttaataagt 10320
   gtttgctaga ctgtggtgaa aggcctatcc gaagtaaggc cggccggatc cttcatcttt 10380
   ggacaaggga ataaagactc cccacttgct actaagaaca atacctaagt tgcccagaca 10440
   tgactgtacc cattcagaga cctaccaccc attagggcta tgacactaac actagcccct 10500
   ggaggttgac catgctaggc agtgggggtc tcacctatga cccactcaga taggggttta 10560
   aaccagtggg tgggatctca gcctcatata ggtgtttgtg gtgagctttc tcctagacaa 10620
   gagaaccctg aagaacagca agaaccagct aatatgatat gtagacatag tgggttgctc 10680
   aaattttgtg tttagtcata ttagaattga cctcagtgac cactcagaaa gtgcccaagc 10740
   ccatctatag gggccaaagt gctattgact ggtgtgtctg tgaattgttc ctccctacag 10800
   agttggtgct gatatatcct agcattcttt ggaaaaccta gctagggact gtcaagtgta 10860
   agatacctcc tgaattggag ggaacactag ctgccctgta ccttctggct agtaccttac 10920
   accctgaatg ggttaggggg tctattattt gctggaaata taccagtttc agtagggctg 10980
   ctgccttagg tcccacaagg tgtaacatgt gctcaatagt tgcactacca catgcacgtg 11040
   aacttaatga tgttatagcc acaacaccaa ccttggtttg cagtttgaca tccctctgga 11100
   atgggtgtag tcatcttgct ctggatctgc ctgaatcatt ggggctgtat gcagcctggg 11160
   cttaaagtga agaatgggat gtcccagaaa tattttgggt gagaagaatc ctggagtaga 11220
   tggtgacctg actatccctg tcctatgggc acaatctatc atcagatatt gcattcaaag 11280
   ggctatcatg ggatcaagtc ctaagtcaac tgttgtttac ctggcagaca ttcatctagg 11340
   agttctcttt tatgccaccc cacagtgatc cgccttttgc agtttatcca ctagggacag 11400
   gattgccacc ccacagtggg gcctctatgc ccgggacaag tgtaaaatat agagtatagg 11460
   ggttatcatc acagagaagc tattgctgga gggcctctgt tatttcctct ccatgccact 11520
   cccattttta acctaccaac tgaaatccca agggagactc caccctgtaa ctagagtcct 11580
   cagaggtgag ccatcccata ttaacaaatg ggcattaggg ctaggatgcc aagggatacc 11640
   tgaaatggga agttgtgggg ctgagtcctc ctgggaatca gagataatat gtaaacagtt 11700
   tgttgagaga ttgatgagag ctgactttga gaggtggcca tgctccctgg tcctcaatag 11760
   ggaaggcact acacaagaaa cctgggtttg atcaactgca ctgtgtccta ctcacacatt 11820
   gtgtgcctgg aaaaatgtta cttagtattt ggagggcctc cagaaccccc ctgggtgcaa 11880
   gactgggtgc tagtgactgg gtgaatgagt cttggacaca gtggccttgt ctaggttgtg 11940
   tgaggtggct aggcatcatg gcaatacctc ataattgatg agtgaggaaa caagactaag 12000
   tccttgactc ctcttattac atgacctggt ggatattatg tttaaactct gcaagctgga 12060
   atgagtactg ggtgcagatc ccctgggatt ctggctacaa aggtgaatga tagctagtct 12120
   gtttattagt agccaaaaaa gtcagtgagg ggtgagtgcc ctgggatgtt gttaagttca 12180
   cattgcacac ttggagaccc tctccatcca gtaacatacc agagaaaact gaccaagccc 12240
   tcatgggtgt atgggaacaa caaacctcct ggctacttca agggcacata acaccagcaa 12300
   ggagcctgtc ataaccacca tctcaaacaa tagaacttcc taagtgaagc aatgacttca 12360
   aatctacttg aaggcatgga gtataagcca tgttcctttc agaggggact gtacttctgt 12420
   agattacttt ccctcattaa ccagatctgg ccggccgcat gcgtactcgg ccacgactgg 12480
   taatttaatt ttcaatttat tttttcttca acttcttaat tttgatatgt ttatatgttt 12540
   tttcgttttt tgcatcgtct ttgatttctt gaacgcacgt tcgatatgag attttcactg 12600
   acttcaagat ttgattctct tcaggtttac ttttaaaaaa aaaaattatt atgttcaccc 12660
   aaattggcct attttaaaag caaaagggga tctaagattt ttaattcttc tctttttcag 12720
   tcgtaacact gctaactttt ttttttgatc aaatcgtaac actcataagt cctaactaaa 12780
   catctttttc tttcctataa ttattgttgg ttccgcattt tatggatcta cgtttgaaag 12840
   tttcaataaa acacatttta ttgtttgaaa gtaacaatat aattactgta tattgattct 12900
   tttaattatt gtgtgttgtt ccaatctact ttcgaaatat agtcatgtga cacgtcatat 12960
   tctatttttg ttaccttgtt ggaacgtttg aattgagtaa agtttaatta acattgtgca 13020
   ataaatgata aacatgttta tgatgtaaaa ttcaatttga ataatacagt ggacatggga 13080
   gcttctcaga cattcctctt ctgaatactg cggttggtca tattcttcat tccttcattc 13140
   tcgttccata ccatggttgg taagtcattt atttaaacat ctttttcatg caaatttatt 13200
   cttgttttcg tatttcttac attttccttg tcattcttgg tgcatgttag caaactgtaa 13260
   tctgataact gaaaatatat taattttcca tagtaaaata atgcatgtga ctaaaagcat 13320
   caaaatcttt agcatcgaag aaaaaagaac caaactttta tttaatgcta tgggcctatt 13380
   tatggtccaa ttagctatta tcatatgaca tgtccttgaa taaattaatg tagcttcata 13440
   tgtgagttta ataatattta ta 13462
<210> 143
   <211> 13462
   <212> DNA
   <213> Artificial Sequence
<220>
   <223> T-strand Insert Region of pDAS000272
<400> 143
   ggtttctttt ttaatttaat ttattttttt aatcggagaa aaaaattaag aaaccaaaaa      60
   acagttttaa tcatggcctc atgttggggt tgagttttat attctgataa gaatcccatc     120
   ttaaaaaccc cgttaaacat gctcttacca tctgcttcga aaatgatatg ttattgacaa     180
   ttccaatttc atttttatga aaataaaata atagtttatt ttataactga gggtggttgc     240
   aggagaataa gccatcggac acaccaccag aaccatggcc atgttgaaaa cgacgagtct     300
   tgggttccgg taatctttcc ctctctcata ttttttttct tttttttgaa attctttcat     360
   tttaattttc ttaggattct atgtatttat tttaatcaat cctttttcca gtttgaggct     420
   aggacgacca cttgtcagat ttgtcgttta gctgtagtaa acaactgatt taaattgttt     480
   atagtactgt agttaacttt aacaacggac cacttatatt cgagccattg gcataaaatg     540
   attcttctcg aaattcgttt acttttctta gtatttttca attttggagt ttacgtagaa     600
   ctaataaaaa gaaaaactta taaacacacc acatgcaatg aataaattcg aatatataac     660
   catactgtta aatattaatt tacattttaa tcttaatttt gcattccagt tgccagaaaa     720
   attatacaag aatttgtccc acagtacacg gatgctcaga tacactgtcc ctctccccat     780
   gctcgcttac cctctctatc tggtaaatcc taattcctaa tttttcttcc tgattataat     840
   tacaattttg aatttttaga ttttgagtat taactaaata taaattaaat ttgtttgggg     900
   atgactacag tggtacagaa gtcctggtaa agaagggtca cattataacc catacagtag     960
   tttatttgcc ccaagcgaga gaaagcttat tgcaacttca ggtaccgcct tttgcagttt    1020
   atctctatgc ccgggacaag tggagtccat gctcaacacc gtgcaggatg aggatgacca    1080
   ccgcggtagc gacttcgtgg gcgaggaaag cctttcgtcc aaggtggtcc ctcctcgcaa    1140
   tcttgttgga tggtgaatat tataaaagcc tgcccttctc gcgggtgttt aaacgtcgac    1200
   ctgcaggtca acggatcagg atattcttgt ttaagatgtt gaactctatg gaggtttgta    1260
   tgaactgatg atctaggacc ggataagttc ccttcttcat agcgaactta ttcaaagaat    1320
   gttttgtgta tcattcttgt tacattgtta ttaatgaaaa aatattattg gtcattggac    1380
   tgaacacgag tgttaaatat ggaccaggcc ccaaataaga tccattgata tatgaattaa    1440
   ataacaagaa taaatcgagt caccaaacca cttgcctttt ttaacgagac ttgttcacca    1500
   acttgataca aaagtcatta tcctatgcaa atcaataatc atacaaaaat atccaataac    1560
   actaaaaaat taaaagaaat ggataatttc acaatatgtt atacgataaa gaagttactt    1620
   ttccaagaaa ttcactgatt ttataagccc acttgcatta gataaatggc aaaaaaaaac    1680
   aaaaaggaaa agaaataaag cacgaagaat tctagaaaat acgaaatacg cttcaatgca    1740
   gtgggaccca cggttcaatt attgccaatt ttcagctcca ccgtatattt aaaaaataaa    1800
   acgataatgc taaaaaaata taaatcgtaa cgatcgttaa atctcaacgg ctggatctta    1860
   tgacgaccgt tagaaattgt ggttgtcgac gagtcagtaa taaacggcgt caaagtggtt 1920
   gcagccggca cacacgagtc gtgtttatca actcaaagca caaatacttt tcctcaacct 1980
   aaaaataagg caattagcca aaaacaactt tgcgtgtaaa caacgctcaa tacacgtgtc 2040
   attttattat tagctattgc ttcaccgcct tagctttctc gtgacctagt cgtcctcgtc 2100
   ttttcttctt cttcttctat aaaacaatac ccaaagagct cttcttcttc acaattcaga 2160
   tttcaatttc tcaaaatctt aaaaactttc tctcaattct ctctaccgtg atcaaggtaa 2220
   atttctgtgt tccttattct ctcaaaatct tcgattttgt tttcgttcga tcccaatttc 2280
   gtatatgttc tttggtttag attctgttaa tcttagatcg aagacgattt tctgggtttg 2340
   atcgttagat atcatcttaa ttctcgatta gggtttcata gatatcatcc gatttgttca 2400
   aataatttga gttttgtcga ataattactc ttcgatttgt gatttctatc tagatctggt 2460
   gttagtttct agtttgtgcg atcgaatttg tcgattaatc tgagtttttc tgattaacag 2520
   atggcttcat ctgagaacgt tatcactgag ttcatgaggt tcaaggtgag gatggaaggt 2580
   actgttaacg gacatgagtt cgagatcgag ggtgagggtg aaggtagacc ttacgaggga 2640
   cataacaccg ttaagcttaa ggttacaaag ggtggacctc ttcctttcgc ttgggatatc 2700
   ctttctcctc aattccaata cggaagcaag gtaagtttgt ggattcttcg tccatgtgat 2760
   ctttgagttt ctttagagct tgtgagggat tagtaagtaa caatgcttga gttttttgct 2820
   gctgggcttc gaaaagtttg tcacttgttg gtttgatcca caaggtcttc ttctccatag 2880
   ctactagaca tgttttagct taagattcaa gtttatatat gccttgtgga ttaatcattg 2940
   cctgattctt ccgtgtcatc tctgagttta tttagagctt ggaagtggtg tagtaataac 3000
   taacaatact cttgataagt tgtagcaatg ctcttgatta gtggatgtaa tatgatgttg 3060
   ataagatata tgaggcacag aaccaaaagt ggtgcttcca ctagacccgt ttttagccta 3120
   aggttcaagt ttataccttg tagatgtttc tgtattgtct gattcttccc tgtgatattt 3180
   gaatttctta gagctttgga agtgatatag gaacaatgct cttgtgtgtt tgtctctatg 3240
   aagattatcg ctgtcgtgtt tcatccgagt gtgcgggatt ttttgctgct gggtttagcc 3300
   tttcttcaaa aagttattac ttgttagttt tattgttttg gtcttgataa gagatgttag 3360
   gacagacatg gtgcttcttg tctatagcca ctagacctat tttagcataa ggttaacgaa 3420
   attctctcta cataccttgt ggatttgttt acattgcctg atctttcctg tgatcgctgt 3480
   catgtttctt tggaatgatt gatgtttata aatggaaaaa tctttgtgca gaagactccc 3540
   gcccatctct ctatgcccgg gacaagtgcc accccacagt ggggcaggat gaggatgacc 3600
   accatggggt cgcagcgtgt gcgtgtccgt cgtacgttct ggccggccgg gccttgggcg 3660
   cgcgatcaga agcgttgcgt tggcgtgtgt gtgcttctgg tttgctttaa ttttaccaag 3720
   tttgtttcaa ggtggatcgc gtggtcaagg cccgtgtgct ttaaagaccc accggcactg 3780
   gcagtgagtg ttgctgcttg tgtaggcttt ggtacgtatg ggctttattt gcttctggat 3840
   gttgtgtact acttgggttt gttgaattat tatgagcagt tgcgtattgt aattcagctg 3900
   ggctacctgg acattgttat gtattaataa atgctttgct ttcttctaaa gatctttaag 3960
   tgctgtttaa acaaccgaca accactttgc ggacttcctt tcaagagaat tcaataaggt 4020
   taattcctaa ttgaaatccg aagataagat tcccacacac ttgtggctga tatcaaaagg 4080
   ctactgccta tttaaacaca tctctggaga ctgagaaaat cagacctcca agcatgaaga 4140
   agcctgagct tactgctact tctgttgaga agttcctcat cgagaagttc gattctgtgt 4200
   ctgatcttat gcagctctct gagggtgagg aatcaagagc tttctctttc gatgttggtg 4260
   gaagaggata cgttctcaga gttaactctt gcgctgacgg attctacaag gatagatacg 4320
   tgtacagaca cttcgcttca gctgctctcc ctatccctga agttcttgat atcggagagt 4380
   tctctgagtc tcttacctac tgtatctcaa gaagggctca gggtgttact cttcaagatc 4440
   ttcctgagac tgagcttcct gctgttcttc aacctgttgc tgaggctatg gatgctatcg 4500
   ctgctgctga tctttctcaa acttctggat tcggaccttt cggtcctcag ggaatcggac 4560
   agtacactac ttggagagat ttcatctgcg ctatcgctga tcctcatgtt taccattggc 4620
   agaccgttat ggatgatacc gtttctgctt ctgttgctca agctcttgat gagcttatgc 4680
   tttgggctga ggattgtcct gaggttagac atcttgttca cgctgatttc ggatctaaca 4740
   acgttctcac cgataacgga agaatcaccg ctgttatcga ttggtctgag gctatgttcg 4800
   gagattctca atacgaggtg gccaacatat tcttttggag gccttggctt gcttgtatgg 4860
   aacaacagac tagatacttc gagagaaggc atcctgagct tgctggatct cctagactta 4920
   gagcttacat gcttaggatc ggacttgatc agctttacca gtctctcgtt gatggaaact 4980
   tcgatgatgc tgcttgggct cagggaagat gtgatgctat cgttagatct ggtgctggaa 5040
   ctgttggaag aactcaaatc gctagaagat ctgctgctgt ttggactgat ggatgtgttg 5100
   aagttctcgc tgattctgga aacagaaggc cttctactag acctagagcc aagaagtgaa 5160
   gatcggcggc aatagcttct tagcgccatc ccgggttgat cctatctgtg ttgaaatagt 5220
   tgcggtgggc aaggctctct ttcagaaaga caggcggcca aaggaaccca aggtgaggtg 5280
   ggctatggct ctcagttcct tgtggaagcg cttggtctaa ggtgcagagg tgttagcggg 5340
   atgaagcaaa agtgtccgat tgtaacaaga tatgttgatc ctacgtaagg atattaaagt 5400
   atgtattcat cactaatata atcagtgtat tccaatatgt actacgattt ccaatgtctt 5460
   tattgtcgcc gtatgtaatc ggcgtcacaa aataatcccc ggtgactttc ttttaatcca 5520
   ggatgaaata atatgttatt ataatttttg cgatttggtc cgttatagga attgaagtgt 5580
   gcttgcggtc gccaccactc ccatttcata attttacatg tatttgaaaa ataaaaattt 5640
   atggtattca atttaaacac gtatacttgt aaagaatgat atcttgaaag aaatatagtt 5700
   taaatattta ttgataaaat aacaagtcag gtattatagt ccaagcaaaa acataaattt 5760
   attgatgcaa gtttaaattc agaaatattt caataactga ttatatcagc tggtacattg 5820
   ccgtagatga aagactgagt gcgatattat ggtgtaatac atagtttaaa cgggcccaag 5880
   actcccgccc atctctctat gcccgggaca agtgccaccc cacagtgggg caggatgagg 5940
   atgaccagtc agttttactt cccttaattt tctatgtact ttcataatta cttatgttat 6000
   tttcttcatg agttttaatg caaattacta tatggactct agtgaaaacg ttcagaatcc 6060
   tataaacatg actactgaga cgaacttgag agtagttttg atcatacaca cgtttcatgt 6120
   ggtacttgag agttactaat ttttgtcatc ttcgtataag tagtaaaaga tactacaaga 6180
   atagtttagt agaaaatact agcggtaggt gaagatttgt cgctatgtac tattattgtc 6240
   tagtaacttg agtaacaatt tcgtggtcta aatatcaaat aaaaatggat gagtggttca 6300
   ccaaatctag gcatcaaaac tattaatgtc attgtctaga tcttaggtga caccacattt 6360
   cgaatattta ttggtaattg agatgttaaa gtaccaatat ttgacttaat aaactaaaag 6420
   attttggctt tatcaaatgt agacattgat gacatatcgt tgtcattatc ttgagtatat 6480
   acaagtcgat caattaggtg aaagtttagt gtctcgtggt tggtaaacga ttaatacagt 6540
   agtatatttt atccaaagac aaaatccaaa tcatttcacc agtatgaata gtattatttt 6600
   atcttaaaag ctaaaatctt aaaaaccaag gtagcaccca cgttgagcta gacgatcaaa 6660
   tcgatttctg ctttgtccaa tttaccaagc tatttaaagc caaataattg aaatataggt 6720
   aggtcgttat attaggctaa gatttatctc aaatgcttaa ctaaaggaat aacaagggat 6780
   tctagttgtg tggttttata agattggtcc aatttcactt aagtttgttt attgtagaat 6840
   tttatatgtg aataatttga attccaattg aaaagatatt atagtaaaag aaaaaatagt 6900
   gcgaacaaaa aactttaatc ccataaaaag aaaaagaaaa atgaaaagtt cttctaacat 6960
   ccatattttg catcatatca taaagataag aaagatacat atcatagacg tacagataaa 7020
   caaacatatc atcatttgtg aaatacatag tacaataatt tgcttttaaa tagagtttaa 7080
   gtcacacaca ctgacacaca cgataaaacg ataatgtctg caaaaacact ttaatcccat 7140
   tgcctagagg acagcttctc cactttgtct ttaaggttgg ttttgccgtg ttgtttttat 7200
   ctttatataa tgatctattt tttggattat gaaatgaatt cacacatttt aattatttaa 7260
   gaagatccat atacaggttt ataacagtac taagtgatga ttattttttg tttttgcata 7320
   gtttagttta ttgggtaaac attcattacg tgtctcttta tacgaatcac ccatccaaaa 7380
   tttcaagtag tcttttagtt catttattat ttcataacta tttgacttat tgatttgaca 7440
   agaaacaaca aaagtgttga cttattgata gattgtggga tcataaaagt aattaagcgt 7500
   caaccacgac ccacaacaac aaagcacatg ttatacatta atatctcgtt tacttaatta 7560
   cagttttcag aatgccgttt catgtcttgt cactggcgat gttattatca tgttggacaa 7620
   tattcgactg ttgtcgtttt tacattttcg tattgactaa aactaaaaaa acaaaactct 7680
   gtttcaggtt gggcctagga tccacattgt acacacattt gcttaagtct atggaggcgc 7740
   aaggttttaa gtctgtggtt gctgttatag gccttccaaa cgatccatct gttaggttgc 7800
   atgaggcttt gggatacaca gcccggggta cattgcgcgc agctggatac aagcatggtg 7860
   gatggcatga tgttggtttt tggcaaaggg attttgagtt gccagctcct ccaaggccag 7920
   ttaggccagt tacccagatc taatatcaaa atctatttag aaatacacaa tattttgttg 7980
   caggcttgct ggagaatcga tctgctatca taaaaattac aaaaaaattt tatttgcctc 8040
   aattatttta ggattggtat taaggacgct taaattattt gtcgggtcac tacgcatcat 8100
   tgtgattgag aagatcagcg atacgaaata ttcgtagtac tatcgataat ttatttgaaa 8160
   attcataaga aaagcaaacg ttacatgaat tgatgaaaca atacaaagac agataaagcc 8220
   acgcacattt aggatattgg ccgagattac tgaatattga gtaagatcac ggaatttctg 8280
   acaggagcat gtcttcaatt cagcccaaat ggcagttgaa atactcaaac cgccccatat 8340
   gcaggagcgg atcattcatt gtttgtttgg ttgcctttgc caacatggga gtccaaggtt 8400
   gtttaaacat ttaaataccc tgccaagctt gaggtagcct ccaatttgac ggtgccgcca 8460
   gcgacgccgt ctggaactgt cctttttgag gaccactccg tttgtggaga tcatgagagt 8520
   ccatgctcaa caccgtgcac tagggacagg attgaagact cccgcccatc tcactaggga 8580
   caggattgcc accccacagt ggggcctaga aagactggag ttgcagagtt tgtgtcttct 8640
   agattaatcc tccaaacttt tgattaacca aaaaaattat caaactaaca tgttctcctt 8700
   ttttctttag aaattctaac gaatttatct ttatactgat ttgaatatac ttaatttggt 8760
   catttggatg ccctttacaa cctccttacc aaactattga tcacagtttc tattgctaaa 8820
   atcaccaaca aaacgcatgt cgccattcat aattatggtt tcacacctac aactaggcta 8880
   ataagtaaat aagtagacaa ctagactcag gtttgaaaaa accataaaag ccatatagcg 8940
   ttttctcatt gaaactgcga acacgatcgt gtgaatgttg cagtttctag ttttgataca 9000
   aacaaacaaa aacacaattt aatcttagat taaaaagaaa aaagagaacg gagcccacta 9060
   gccactcctt caaacgtgtc ttaccaactc tcttctagaa acaaattagg cttcaccttc 9120
   ctcttccaac ctctctctct ctctctctct ctttttctca aaccatctct ccataaagcc 9180
   ctaatttctt catcacaaga atcagaagaa gaaagatgga cctgcatcta attttcggtc 9240
   caacttgcac aggaaagacg acgaccgcga tagctcttgc ccagcagaca gggcttccag 9300
   tcctttcgct tgatcgggtc caatgctgtc ctcaactatc aaccggaagc ggacgaccaa 9360
   cagtggaaga actgaaagga acgacgcgtc tctaccttga tgatcggcct ctggtggagg 9420
   gtatcatcgc agccaagcaa gctcatcata ggctgatcga ggaggtgtat aatcatgagg 9480
   ccaacggcgg gcttattctt gagggaggat ccacctcgtt gctcaactgc atggcgcgaa 9540
   acagctattg gagtgcagat tttcgttggc atattattcg ccacaagtta cccgaccaag 9600
   agaccttcat gaaagcggcc aaggccagag ttaagcagat gttgcacccc gctgcaggcc 9660
   attctattat tcaagagttg gtttatcttt ggaatgaacc tcggctgagg cccattctga 9720
   aagagatcga tggatatcga tatgccatgt tgtttgctag ccagaaccag atcacggcag 9780
   atatgctatt gcagcttgac gcaaatatgg aaggtaagtt gattaatggg atcgctcagg 9840
   agtatttcat ccatgcgcgc caacaggaac agaaattccc ccaagttaac gcagccgctt 9900
   tcgacggatt cgaaggtcat ccgttcggaa tgtattagaa atcaccagtc tctctctaca 9960
   aatctatctc tctctatttt tctccagaat aatgtgtgag tagttcccag ataagggaat 10020
   tagggttctt atagggtttc gctcatgtgt tgagcatata agaaaccctt agtatgtatt 10080
   tgtatttgta aaatacttct atcaataaaa tttctaattc ctaaaaccaa aatccagtgt 10140
   ttaaacgagt ccatgctcaa caccgtgcac tagggacagg attgaagact cccgcccatc 10200
   tcactaggga caggattgcc accccacagt ggggcctaga aagactggag ttgcagacat 10260
   taaggatgac cagttcgtaa aggtcctgcg gtgtctattg cttttcatag gttaataagt 10320
   gtttgctaga ctgtggtgaa aggcctatcc gaagtaaggc cggccggatc cttcatcttt 10380
   ggacaaggga ataaagactc cccacttgct actaagaaca atacctaagt tgcccagaca 10440
   tgactgtacc cattcagaga cctaccaccc attagggcta tgacactaac actagcccct 10500
   ggaggttgac catgctaggc agtgggggtc tcacctatga cccactcaga taggggttta 10560
   aaccagtggg tgggatctca gcctcatata ggtgtttgtg gtgagctttc tcctagacaa 10620
   gagaaccctg aagaacagca agaaccagct aatatgatat gtagacatag tgggttgctc 10680
   aaattttgtg tttagtcata ttagaattga cctcagtgac cactcagaaa gtgcccaagc 10740
   ccatctatag gggccaaagt gctattgact ggtgtgtctg tgaattgttc ctccctacag 10800
   agttggtgct gatatatcct agcattcttt ggaaaaccta gctagggact gtcaagtgta 10860
   agatacctcc tgaattggag ggaacactag ctgccctgta ccttctggct agtaccttac 10920
   accctgaatg ggttaggggg tctattattt gctggaaata taccagtttc agtagggctg 10980
   ctgccttagg tcccacaagg tgtaacatgt gctcaatagt tgcactacca catgcacgtg 11040
   aacttaatga tgttatagcc acaacaccaa ccttggtttg cagtttgaca tccctctgga 11100
   atgggtgtag tcatcttgct ctggatctgc ctgaatcatt ggggctgtat gcagcctggg 11160
   cttaaagtga agaatgggat gtcccagaaa tattttgggt gagaagaatc ctggagtaga 11220
   tggtgacctg actatccctg tcctatgggc acaatctatc atcagatatt gcattcaaag 11280
   ggctatcatg ggatcaagtc ctaagtcaac tgttgtttac ctggcagaca ttcatctagg 11340
   agttctcttt tatgccaccc cacagtgatc cgccttttgc agtttatcca ctagggacag 11400
   gattgccacc ccacagtggg gcctctatgc ccgggacaag tgtaaaatat agagtatagg 11460
   ggttatcatc acagagaagc tattgctgga gggcctctgt tatttcctct ccatgccact 11520
   cccattttta acctaccaac tgaaatccca agggagactc caccctgtaa ctagagtcct 11580
   cagaggtgag ccatcccata ttaacaaatg ggcattaggg ctaggatgcc aagggatacc 11640
   tgaaatggga agttgtgggg ctgagtcctc ctgggaatca gagataatat gtaaacagtt 11700
   tgttgagaga ttgatgagag ctgactttga gaggtggcca tgctccctgg tcctcaatag 11760
   ggaaggcact acacaagaaa cctgggtttg atcaactgca ctgtgtccta ctcacacatt 11820
   gtgtgcctgg aaaaatgtta cttagtattt ggagggcctc cagaaccccc ctgggtgcaa 11880
   gactgggtgc tagtgactgg gtgaatgagt cttggacaca gtggccttgt ctaggttgtg 11940
   tgaggtggct aggcatcatg gcaatacctc ataattgatg agtgaggaaa caagactaag 12000
   tccttgactc ctcttattac atgacctggt ggatattatg tttaaactct gcaagctgga 12060
   atgagtactg ggtgcagatc ccctgggatt ctggctacaa aggtgaatga tagctagtct 12120
   gtttattagt agccaaaaaa gtcagtgagg ggtgagtgcc ctgggatgtt gttaagttca 12180
   cattgcacac ttggagaccc tctccatcca gtaacatacc agagaaaact gaccaagccc 12240
   tcatgggtgt atgggaacaa caaacctcct ggctacttca agggcacata acaccagcaa 12300
   ggagcctgtc ataaccacca tctcaaacaa tagaacttcc taagtgaagc aatgacttca 12360
   aatctacttg aaggcatgga gtataagcca tgttcctttc agaggggact gtacttctgt 12420
   agattacttt ccctcattaa ccagatctgg ccggccgcat gccttgctgg tcgatcgtgt 12480
   tggccactct tgtttatcta tcattcctcg ttggtccagt cacagttcta aaagtctatg 12540
   gtgttcctta cattgtaagt ttcatatatt tctttattat atcattgcta atataatttg 12600
   tttttgacat aaaagttttg gaaaaatttc agatctttgt aatgtggttg gacgctgtca 12660
   cgtacttgca tcatcatggt cacgatgata agctgccttg gtacagaggc aaggtaagta 12720
   gatcaacatt atttataaga agcaataatg attagtagtt gaataatctg aatttttgat 12780
   gtttttgtac aataatagga atggagttat ttacgtggag gattaacaac tgttgataga 12840
   gattacggga tcttcaacaa cattcatcac gatattggaa ctcacgtgat ccatcatctt 12900
   ttcccacaaa tccctcacta tcacttggtc gatgccgtga gtgatctcgc tctctctcta 12960
   gtttcatttg attatattaa agggtgatta attactaaat tagtgatctt aattaatgac 13020
   atgcgacaga cgaaagcagc taaacatgtg ttgggaagat actacagaga accaaagacg 13080
   tcaggagcaa taccgatcca cttagtggaa agtttggtgg caagtattaa gaaagatcat 13140
   tacgtcagtg acactggtga tattgtcttc tacgagacag atccagatct ctacgtttat 13200
   gcttctgaca aatccaaaat caattaatct ttcttcctag ctctatttag gaataaaaca 13260
   ctcctttggt tttacttatt tctggttgtt tttaagttaa aaatgtactc gtgaaacttt 13320
   tttttattaa atgtatttac attacaaatc gtaaaagttt ttgttcgttt tctctatgtt 13380
   tttagttaca aacttacaat caaaaaggtc ttaaaaactt tttgatggtg ggacggacaa 13440
   aagaaaaagt tcgactgaga gt 13462
<210> 144
   <211> 13462
   <212> DNA
   <213> Artificial Sequence
<220>
   <223> T-strand Insert Region of pDAS000273
<400> 144
   aatatttata tatatttgtt ttaatggctt attttattgt taaatggata catcagcttg      60
   aaatatctac gaacatgcat cattttccta gatacatttg tttgttgctc aaaaaatgaa     120
   taacgtagtt aaacgagtga gattcttagc atctgcctcg aaaacgatat gttattgaca     180
   attccaattt catttttatg aaaataaaat aatagtttat tttataattg ggggtggttg     240
   caggagaata agccatcgga cacaccacca gaaccatggc catgttgaaa acgacgagtc     300
   ttgggttccg gtaatccccc tctcattatt ttttttcttt tttttgaaac tctttcattt     360
   taattttctt agaattctat gtatttattt taatcaatcc tttttccagt gtgaggcttg     420
   gacgaccact tgtcagattt gtcgtttagc tgtagtaaac aactgattta aattgtttat     480
   ggtactgtag ttaactttaa caacgggcca cttatattcg agccattggc ataaaatgat     540
   tcttctcgaa attcgtttac ttttcttagt atttttcagt tttgtagttt acgtagaact     600
   aataaaaaga aaaaaactta taaacacacc acatgcaatg aataaattcg aatatataac     660
   catactgtta aatattaatt aacattttaa tcttaatttt gcattccagt tgccagaaaa     720
   attatacaag aatttgtccc acagtacacg gatgctcaga tacactgtcc ctctccccat     780
   gctcgcttac cctctctatc tggtaaatcc taattcctca tttttcttcc tgattataat     840
   tacaattttg aatttttaga ttttgagtat taactaaata taaattaaat ttgtttgggg     900
   atgactacag tggtacagaa gtcctggtaa agaagggtca cattataacc catacagtag     960
   tttatttgcc ccaagcgaga gaaagcttat tgcaacttca ggtaccgcct tttgcagttt    1020
   atctctatgc ccgggacaag tggagtccat gctcaacacc gtgcaggatg aggatgacca    1080
   ccgcggtagc gacttcgtgg gcgaggaaag cctttcgtcc aaggtggtcc ctcctcgcaa    1140
   tcttgttgga tggtgaatat tataaaagcc tgcccttctc gcgggtgttt aaacgtcgac    1200
   ctgcaggtca acggatcagg atattcttgt ttaagatgtt gaactctatg gaggtttgta    1260
   tgaactgatg atctaggacc ggataagttc ccttcttcat agcgaactta ttcaaagaat 1320
   gttttgtgta tcattcttgt tacattgtta ttaatgaaaa aatattattg gtcattggac 1380
   tgaacacgag tgttaaatat ggaccaggcc ccaaataaga tccattgata tatgaattaa 1440
   ataacaagaa taaatcgagt caccaaacca cttgcctttt ttaacgagac ttgttcacca 1500
   acttgataca aaagtcatta tcctatgcaa atcaataatc atacaaaaat atccaataac 1560
   actaaaaaat taaaagaaat ggataatttc acaatatgtt atacgataaa gaagttactt 1620
   ttccaagaaa ttcactgatt ttataagccc acttgcatta gataaatggc aaaaaaaaac 1680
   aaaaaggaaa agaaataaag cacgaagaat tctagaaaat acgaaatacg cttcaatgca 1740
   gtgggaccca cggttcaatt attgccaatt ttcagctcca ccgtatattt aaaaaataaa 1800
   acgataatgc taaaaaaata taaatcgtaa cgatcgttaa atctcaacgg ctggatctta 1860
   tgacgaccgt tagaaattgt ggttgtcgac gagtcagtaa taaacggcgt caaagtggtt 1920
   gcagccggca cacacgagtc gtgtttatca actcaaagca caaatacttt tcctcaacct 1980
   aaaaataagg caattagcca aaaacaactt tgcgtgtaaa caacgctcaa tacacgtgtc 2040
   attttattat tagctattgc ttcaccgcct tagctttctc gtgacctagt cgtcctcgtc 2100
   ttttcttctt cttcttctat aaaacaatac ccaaagagct cttcttcttc acaattcaga 2160
   tttcaatttc tcaaaatctt aaaaactttc tctcaattct ctctaccgtg atcaaggtaa 2220
   atttctgtgt tccttattct ctcaaaatct tcgattttgt tttcgttcga tcccaatttc 2280
   gtatatgttc tttggtttag attctgttaa tcttagatcg aagacgattt tctgggtttg 2340
   atcgttagat atcatcttaa ttctcgatta gggtttcata gatatcatcc gatttgttca 2400
   aataatttga gttttgtcga ataattactc ttcgatttgt gatttctatc tagatctggt 2460
   gttagtttct agtttgtgcg atcgaatttg tcgattaatc tgagtttttc tgattaacag 2520
   atggcttcat ctgagaacgt tatcactgag ttcatgaggt tcaaggtgag gatggaaggt 2580
   actgttaacg gacatgagtt cgagatcgag ggtgagggtg aaggtagacc ttacgaggga 2640
   cataacaccg ttaagcttaa ggttacaaag ggtggacctc ttcctttcgc ttgggatatc 2700
   ctttctcctc aattccaata cggaagcaag gtaagtttgt ggattcttcg tccatgtgat 2760
   ctttgagttt ctttagagct tgtgagggat tagtaagtaa caatgcttga gttttttgct 2820
   gctgggcttc gaaaagtttg tcacttgttg gtttgatcca caaggtcttc ttctccatag 2880
   ctactagaca tgttttagct taagattcaa gtttatatat gccttgtgga ttaatcattg 2940
   cctgattctt ccgtgtcatc tctgagttta tttagagctt ggaagtggtg tagtaataac 3000
   taacaatact cttgataagt tgtagcaatg ctcttgatta gtggatgtaa tatgatgttg 3060
   ataagatata tgaggcacag aaccaaaagt ggtgcttcca ctagacccgt ttttagccta 3120
   aggttcaagt ttataccttg tagatgtttc tgtattgtct gattcttccc tgtgatattt 3180
   gaatttctta gagctttgga agtgatatag gaacaatgct cttgtgtgtt tgtctctatg 3240
   aagattatcg ctgtcgtgtt tcatccgagt gtgcgggatt ttttgctgct gggtttagcc 3300
   tttcttcaaa aagttattac ttgttagttt tattgttttg gtcttgataa gagatgttag 3360
   gacagacatg gtgcttcttg tctatagcca ctagacctat tttagcataa ggttaacgaa 3420
   attctctcta cataccttgt ggatttgttt acattgcctg atctttcctg tgatcgctgt 3480
   catgtttctt tggaatgatt gatgtttata aatggaaaaa tctttgtgca gaagactccc 3540
   gcccatctct ctatgcccgg gacaagtgcc accccacagt ggggcaggat gaggatgacc 3600
   accatggggt cgcagcgtgt gcgtgtccgt cgtacgttct ggccggccgg gccttgggcg 3660
   cgcgatcaga agcgttgcgt tggcgtgtgt gtgcttctgg tttgctttaa ttttaccaag 3720
   tttgtttcaa ggtggatcgc gtggtcaagg cccgtgtgct ttaaagaccc accggcactg 3780
   gcagtgagtg ttgctgcttg tgtaggcttt ggtacgtatg ggctttattt gcttctggat 3840
   gttgtgtact acttgggttt gttgaattat tatgagcagt tgcgtattgt aattcagctg 3900
   ggctacctgg acattgttat gtattaataa atgctttgct ttcttctaaa gatctttaag 3960
   tgctgtttaa acaaccgaca accactttgc ggacttcctt tcaagagaat tcaataaggt 4020
   taattcctaa ttgaaatccg aagataagat tcccacacac ttgtggctga tatcaaaagg 4080
   ctactgccta tttaaacaca tctctggaga ctgagaaaat cagacctcca agcatgaaga 4140
   agcctgagct tactgctact tctgttgaga agttcctcat cgagaagttc gattctgtgt 4200
   ctgatcttat gcagctctct gagggtgagg aatcaagagc tttctctttc gatgttggtg 4260
   gaagaggata cgttctcaga gttaactctt gcgctgacgg attctacaag gatagatacg 4320
   tgtacagaca cttcgcttca gctgctctcc ctatccctga agttcttgat atcggagagt 4380
   tctctgagtc tcttacctac tgtatctcaa gaagggctca gggtgttact cttcaagatc 4440
   ttcctgagac tgagcttcct gctgttcttc aacctgttgc tgaggctatg gatgctatcg 4500
   ctgctgctga tctttctcaa acttctggat tcggaccttt cggtcctcag ggaatcggac 4560
   agtacactac ttggagagat ttcatctgcg ctatcgctga tcctcatgtt taccattggc 4620
   agaccgttat ggatgatacc gtttctgctt ctgttgctca agctcttgat gagcttatgc 4680
   tttgggctga ggattgtcct gaggttagac atcttgttca cgctgatttc ggatctaaca 4740
   acgttctcac cgataacgga agaatcaccg ctgttatcga ttggtctgag gctatgttcg 4800
   gagattctca atacgaggtg gccaacatat tcttttggag gccttggctt gcttgtatgg 4860
   aacaacagac tagatacttc gagagaaggc atcctgagct tgctggatct cctagactta 4920
   gagcttacat gcttaggatc ggacttgatc agctttacca gtctctcgtt gatggaaact 4980
   tcgatgatgc tgcttgggct cagggaagat gtgatgctat cgttagatct ggtgctggaa 5040
   ctgttggaag aactcaaatc gctagaagat ctgctgctgt ttggactgat ggatgtgttg 5100
   aagttctcgc tgattctgga aacagaaggc cttctactag acctagagcc aagaagtgaa 5160
   gatcggcggc aatagcttct tagcgccatc ccgggttgat cctatctgtg ttgaaatagt 5220
   tgcggtgggc aaggctctct ttcagaaaga caggcggcca aaggaaccca aggtgaggtg 5280
   ggctatggct ctcagttcct tgtggaagcg cttggtctaa ggtgcagagg tgttagcggg 5340
   atgaagcaaa agtgtccgat tgtaacaaga tatgttgatc ctacgtaagg atattaaagt 5400
   atgtattcat cactaatata atcagtgtat tccaatatgt actacgattt ccaatgtctt 5460
   tattgtcgcc gtatgtaatc ggcgtcacaa aataatcccc ggtgactttc ttttaatcca 5520
   ggatgaaata atatgttatt ataatttttg cgatttggtc cgttatagga attgaagtgt 5580
   gcttgcggtc gccaccactc ccatttcata attttacatg tatttgaaaa ataaaaattt 5640
   atggtattca atttaaacac gtatacttgt aaagaatgat atcttgaaag aaatatagtt 5700
   taaatattta ttgataaaat aacaagtcag gtattatagt ccaagcaaaa acataaattt 5760
   attgatgcaa gtttaaattc agaaatattt caataactga ttatatcagc tggtacattg 5820
   ccgtagatga aagactgagt gcgatattat ggtgtaatac atagtttaaa cgggcccaag 5880
   actcccgccc atctctctat gcccgggaca agtgccaccc cacagtgggg caggatgagg 5940
   atgaccagtc agttttactt cccttaattt tctatgtact ttcataatta cttatgttat 6000
   tttcttcatg agttttaatg caaattacta tatggactct agtgaaaacg ttcagaatcc 6060
   tataaacatg actactgaga cgaacttgag agtagttttg atcatacaca cgtttcatgt 6120
   ggtacttgag agttactaat ttttgtcatc ttcgtataag tagtaaaaga tactacaaga 6180
   atagtttagt agaaaatact agcggtaggt gaagatttgt cgctatgtac tattattgtc 6240
   tagtaacttg agtaacaatt tcgtggtcta aatatcaaat aaaaatggat gagtggttca 6300
   ccaaatctag gcatcaaaac tattaatgtc attgtctaga tcttaggtga caccacattt 6360
   cgaatattta ttggtaattg agatgttaaa gtaccaatat ttgacttaat aaactaaaag 6420
   attttggctt tatcaaatgt agacattgat gacatatcgt tgtcattatc ttgagtatat 6480
   acaagtcgat caattaggtg aaagtttagt gtctcgtggt tggtaaacga ttaatacagt 6540
   agtatatttt atccaaagac aaaatccaaa tcatttcacc agtatgaata gtattatttt 6600
   atcttaaaag ctaaaatctt aaaaaccaag gtagcaccca cgttgagcta gacgatcaaa 6660
   tcgatttctg ctttgtccaa tttaccaagc tatttaaagc caaataattg aaatataggt 6720
   aggtcgttat attaggctaa gatttatctc aaatgcttaa ctaaaggaat aacaagggat 6780
   tctagttgtg tggttttata agattggtcc aatttcactt aagtttgttt attgtagaat 6840
   tttatatgtg aataatttga attccaattg aaaagatatt atagtaaaag aaaaaatagt 6900
   gcgaacaaaa aactttaatc ccataaaaag aaaaagaaaa atgaaaagtt cttctaacat 6960
   ccatattttg catcatatca taaagataag aaagatacat atcatagacg tacagataaa 7020
   caaacatatc atcatttgtg aaatacatag tacaataatt tgcttttaaa tagagtttaa 7080
   gtcacacaca ctgacacaca cgataaaacg ataatgtctg caaaaacact ttaatcccat 7140
   tgcctagagg acagcttctc cactttgtct ttaaggttgg ttttgccgtg ttgtttttat 7200
   ctttatataa tgatctattt tttggattat gaaatgaatt cacacatttt aattatttaa 7260
   gaagatccat atacaggttt ataacagtac taagtgatga ttattttttg tttttgcata 7320
   gtttagttta ttgggtaaac attcattacg tgtctcttta tacgaatcac ccatccaaaa 7380
   tttcaagtag tcttttagtt catttattat ttcataacta tttgacttat tgatttgaca 7440
   agaaacaaca aaagtgttga cttattgata gattgtggga tcataaaagt aattaagcgt 7500
   caaccacgac ccacaacaac aaagcacatg ttatacatta atatctcgtt tacttaatta 7560
   cagttttcag aatgccgttt catgtcttgt cactggcgat gttattatca tgttggacaa 7620
   tattcgactg ttgtcgtttt tacattttcg tattgactaa aactaaaaaa acaaaactct 7680
   gtttcaggtt gggcctagga tccacattgt acacacattt gcttaagtct atggaggcgc 7740
   aaggttttaa gtctgtggtt gctgttatag gccttccaaa cgatccatct gttaggttgc 7800
   atgaggcttt gggatacaca gcccggggta cattgcgcgc agctggatac aagcatggtg 7860
   gatggcatga tgttggtttt tggcaaaggg attttgagtt gccagctcct ccaaggccag 7920
   ttaggccagt tacccagatc taatatcaaa atctatttag aaatacacaa tattttgttg 7980
   caggcttgct ggagaatcga tctgctatca taaaaattac aaaaaaattt tatttgcctc 8040
   aattatttta ggattggtat taaggacgct taaattattt gtcgggtcac tacgcatcat 8100
   tgtgattgag aagatcagcg atacgaaata ttcgtagtac tatcgataat ttatttgaaa 8160
   attcataaga aaagcaaacg ttacatgaat tgatgaaaca atacaaagac agataaagcc 8220
   acgcacattt aggatattgg ccgagattac tgaatattga gtaagatcac ggaatttctg 8280
   acaggagcat gtcttcaatt cagcccaaat ggcagttgaa atactcaaac cgccccatat 8340
   gcaggagcgg atcattcatt gtttgtttgg ttgcctttgc caacatggga gtccaaggtt 8400
   gtttaaacat ttaaataccc tgccaagctt gaggtagcct ccaatttgac ggtgccgcca 8460
   gcgacgccgt ctggaactgt cctttttgag gaccactccg tttgtggaga tcatgagagt 8520
   ccatgctcaa caccgtgcac tagggacagg attgaagact cccgcccatc tcactaggga 8580
   caggattgcc accccacagt ggggcctaga aagactggag ttgcagagtt tgtgtcttct 8640
   agattaatcc tccaaacttt tgattaacca aaaaaattat caaactaaca tgttctcctt 8700
   ttttctttag aaattctaac gaatttatct ttatactgat ttgaatatac ttaatttggt 8760
   catttggatg ccctttacaa cctccttacc aaactattga tcacagtttc tattgctaaa 8820
   atcaccaaca aaacgcatgt cgccattcat aattatggtt tcacacctac aactaggcta 8880
   ataagtaaat aagtagacaa ctagactcag gtttgaaaaa accataaaag ccatatagcg 8940
   ttttctcatt gaaactgcga acacgatcgt gtgaatgttg cagtttctag ttttgataca 9000
   aacaaacaaa aacacaattt aatcttagat taaaaagaaa aaagagaacg gagcccacta 9060
   gccactcctt caaacgtgtc ttaccaactc tcttctagaa acaaattagg cttcaccttc 9120
   ctcttccaac ctctctctct ctctctctct ctttttctca aaccatctct ccataaagcc 9180
   ctaatttctt catcacaaga atcagaagaa gaaagatgga cctgcatcta attttcggtc 9240
   caacttgcac aggaaagacg acgaccgcga tagctcttgc ccagcagaca gggcttccag 9300
   tcctttcgct tgatcgggtc caatgctgtc ctcaactatc aaccggaagc ggacgaccaa 9360
   cagtggaaga actgaaagga acgacgcgtc tctaccttga tgatcggcct ctggtggagg 9420
   gtatcatcgc agccaagcaa gctcatcata ggctgatcga ggaggtgtat aatcatgagg 9480
   ccaacggcgg gcttattctt gagggaggat ccacctcgtt gctcaactgc atggcgcgaa 9540
   acagctattg gagtgcagat tttcgttggc atattattcg ccacaagtta cccgaccaag 9600
   agaccttcat gaaagcggcc aaggccagag ttaagcagat gttgcacccc gctgcaggcc 9660
   attctattat tcaagagttg gtttatcttt ggaatgaacc tcggctgagg cccattctga 9720
   aagagatcga tggatatcga tatgccatgt tgtttgctag ccagaaccag atcacggcag 9780
   atatgctatt gcagcttgac gcaaatatgg aaggtaagtt gattaatggg atcgctcagg 9840
   agtatttcat ccatgcgcgc caacaggaac agaaattccc ccaagttaac gcagccgctt 9900
   tcgacggatt cgaaggtcat ccgttcggaa tgtattagaa atcaccagtc tctctctaca 9960
   aatctatctc tctctatttt tctccagaat aatgtgtgag tagttcccag ataagggaat 10020
   tagggttctt atagggtttc gctcatgtgt tgagcatata agaaaccctt agtatgtatt 10080
   tgtatttgta aaatacttct atcaataaaa tttctaattc ctaaaaccaa aatccagtgt 10140
   ttaaacgagt ccatgctcaa caccgtgcac tagggacagg attgaagact cccgcccatc 10200
   tcactaggga caggattgcc accccacagt ggggcctaga aagactggag ttgcagacat 10260
   taaggatgac cagttcgtaa aggtcctgcg gtgtctattg cttttcatag gttaataagt 10320
   gtttgctaga ctgtggtgaa aggcctatcc gaagtaaggc cggccggatc cttcatcttt 10380
   ggacaaggga ataaagactc cccacttgct actaagaaca atacctaagt tgcccagaca 10440
   tgactgtacc cattcagaga cctaccaccc attagggcta tgacactaac actagcccct 10500
   ggaggttgac catgctaggc agtgggggtc tcacctatga cccactcaga taggggttta 10560
   aaccagtggg tgggatctca gcctcatata ggtgtttgtg gtgagctttc tcctagacaa 10620
   gagaaccctg aagaacagca agaaccagct aatatgatat gtagacatag tgggttgctc 10680
   aaattttgtg tttagtcata ttagaattga cctcagtgac cactcagaaa gtgcccaagc 10740
   ccatctatag gggccaaagt gctattgact ggtgtgtctg tgaattgttc ctccctacag 10800
   agttggtgct gatatatcct agcattcttt ggaaaaccta gctagggact gtcaagtgta 10860
   agatacctcc tgaattggag ggaacactag ctgccctgta ccttctggct agtaccttac 10920
   accctgaatg ggttaggggg tctattattt gctggaaata taccagtttc agtagggctg 10980
   ctgccttagg tcccacaagg tgtaacatgt gctcaatagt tgcactacca catgcacgtg 11040
   aacttaatga tgttatagcc acaacaccaa ccttggtttg cagtttgaca tccctctgga 11100
   atgggtgtag tcatcttgct ctggatctgc ctgaatcatt ggggctgtat gcagcctggg 11160
   cttaaagtga agaatgggat gtcccagaaa tattttgggt gagaagaatc ctggagtaga 11220
   tggtgacctg actatccctg tcctatgggc acaatctatc atcagatatt gcattcaaag 11280
   ggctatcatg ggatcaagtc ctaagtcaac tgttgtttac ctggcagaca ttcatctagg 11340
   agttctcttt tatgccaccc cacagtgatc cgccttttgc agtttatcca ctagggacag 11400
   gattgccacc ccacagtggg gcctctatgc ccgggacaag tgtaaaatat agagtatagg 11460
   ggttatcatc acagagaagc tattgctgga gggcctctgt tatttcctct ccatgccact 11520
   cccattttta acctaccaac tgaaatccca agggagactc caccctgtaa ctagagtcct 11580
   cagaggtgag ccatcccata ttaacaaatg ggcattaggg ctaggatgcc aagggatacc 11640
   tgaaatggga agttgtgggg ctgagtcctc ctgggaatca gagataatat gtaaacagtt 11700
   tgttgagaga ttgatgagag ctgactttga gaggtggcca tgctccctgg tcctcaatag 11760
   ggaaggcact acacaagaaa cctgggtttg atcaactgca ctgtgtccta ctcacacatt 11820
   gtgtgcctgg aaaaatgtta cttagtattt ggagggcctc cagaaccccc ctgggtgcaa 11880
   gactgggtgc tagtgactgg gtgaatgagt cttggacaca gtggccttgt ctaggttgtg 11940
   tgaggtggct aggcatcatg gcaatacctc ataattgatg agtgaggaaa caagactaag 12000
   tccttgactc ctcttattac atgacctggt ggatattatg tttaaactct gcaagctgga 12060
   atgagtactg ggtgcagatc ccctgggatt ctggctacaa aggtgaatga tagctagtct 12120
   gtttattagt agccaaaaaa gtcagtgagg ggtgagtgcc ctgggatgtt gttaagttca 12180
   cattgcacac ttggagaccc tctccatcca gtaacatacc agagaaaact gaccaagccc 12240
   tcatgggtgt atgggaacaa caaacctcct ggctacttca agggcacata acaccagcaa 12300
   ggagcctgtc ataaccacca tctcaaacaa tagaacttcc taagtgaagc aatgacttca 12360
   aatctacttg aaggcatgga gtataagcca tgttcctttc agaggggact gtacttctgt 12420
   agattacttt ccctcattaa ccagatctgg ccggccgcat gccttgctgg tcgatcatgt 12480
   tggccactct tgtttatcta tcattcctcg ttggtccagt cacagttcta aaagtctatg 12540
   gtgttcctta cattgtaagt ttcatatatt tcattattat atcattgcta atataatttg 12600
   tttttgacat aaagttttgg aaaaatttca gatctttgta atgtggttgg acgctgtcac 12660
   gtacttgcat catcatggtc acgatgataa gttgccttgg tacagaggca aggtaagtag 12720
   atcaacatta atttataaga agcaacaatg attagtattt gattaatcta aattattgat 12780
   gttttgtgta caataatagg aatggagtta tttacgtgga ggattaacaa ctattgatag 12840
   agattacggg atcttcaaca acattcatca cgatattgga actcacgtga tccatcatct 12900
   tttcccacaa atccctcact atcacttggt tgatgccgtg agtgatctcg ctctctctct 12960
   agtttcattt gattaaaatt aaagggtgat taattactaa attagtgatc ttaattaatg 13020
   atatgcgaca gacgaaatca gctaaacatg tgttgggaag atactacaga gaaccaaaga 13080
   cgtcaggagc aataccgatc cacttggtgg aaagtttggt ggcaagtatt aagaaagatc 13140
   attacgtcag tgacactggt gatattgtct tctacgagac agatccagat ctctacgttt 13200
   atgcttctga caaatccaaa atcaactaac ctttcttcct agctctattt aggaataaaa 13260
   cagtcctttg gtttttactt atttctggtt gtttttaagt taaatgtact cgtgaaactt 13320
   tttttaatta aatgtattta cattacaaat caagtttttg ttcgttttct ttatgttttt 13380
   agttacaata aataaaggtc ttaaaaactt tttgttggtg gggacaaaag aaaaagttcg 13440
   actgagagag tcgacaaaat gc 13462
<210> 145
   <211> 13462
   <212> DNA
   <213> Artificial Sequence
<220>
   <223> T-strand Insert Region of pDAS000274
<400> 145
   ttttttaatt taatttattt ttttaatcgg agaaaaaaat taagaaacca aaaaacagtt 60
   ttaatcatgg cctcatgttg gggttgagtt ttatattctg ataagaatcc catcttaaaa 120
   accccgttaa acatgctctt accatctgct tcgaaaatga tatgttattg acaattccaa 180
   tttcattttt atgaaaataa aataatagtt tattttataa ctgagggtgg ttgcaggaga 240
   ataagccatc ggacacacca ccagaaccat ggccatgttg aaaacgacga gtcttgggtt 300
   ccggtaatct ttccctctct catatttttt ttcttttttt tgaaattctt tcattttaat 360
   tttcttagga ttctatgtat ttattttaat caatcctttt tccagtttga ggctaggacg 420
   accacttgtc agatttgtcg tttagctgta gtaaacaact gatttaaatt gtttatagta 480
   ctgtagttaa ctttaacaac ggaccactta tattcgagcc attggcataa aatgattctt 540
   ctcgaaattc gtttactttt cttagtattt ttcaattttg gagtttacgt agaactaata 600
   aaaagaaaaa cttataaaca caccacatgc aatgaataaa ttcgaatata taaccatact 660
   gttaaatatt aatttacatt ttaatcttaa ttttgcattc cagttgccag aaaaattata 720
   caagaatttg tcccacagta cacggatgct cagatacact gtccctctcc ccatgctcgc 780
   ttaccctctc tatctggtaa atcctaattc ctaatttttc ttcctgatta taattacaat 840
   tttgaatttt tagattttga gtattaacta aatataaatt aaatttgttt ggggatgact 900
   acagtggtac agaagtcctg gtaaagaagg gtcacattat aacccataca gtagtttatt 960
   tgccccaagc gagagaaagc ttattgcaac ttcaactact ggtaccgcct tttgcagttt 1020
   atctctatgc ccgggacaag tggagtccat gctcaacacc gtgcaggatg aggatgacca 1080
   ccgcggtagc gacttcgtgg gcgaggaaag cctttcgtcc aaggtggtcc ctcctcgcaa 1140
   tcttgttgga tggtgaatat tataaaagcc tgcccttctc gcgggtgttt aaacgtcgac 1200
   ctgcaggtca acggatcagg atattcttgt ttaagatgtt gaactctatg gaggtttgta 1260
   tgaactgatg atctaggacc ggataagttc ccttcttcat agcgaactta ttcaaagaat 1320
   gttttgtgta tcattcttgt tacattgtta ttaatgaaaa aatattattg gtcattggac 1380
   tgaacacgag tgttaaatat ggaccaggcc ccaaataaga tccattgata tatgaattaa 1440
   ataacaagaa taaatcgagt caccaaacca cttgcctttt ttaacgagac ttgttcacca 1500
   acttgataca aaagtcatta tcctatgcaa atcaataatc atacaaaaat atccaataac 1560
   actaaaaaat taaaagaaat ggataatttc acaatatgtt atacgataaa gaagttactt 1620
   ttccaagaaa ttcactgatt ttataagccc acttgcatta gataaatggc aaaaaaaaac 1680
   aaaaaggaaa agaaataaag cacgaagaat tctagaaaat acgaaatacg cttcaatgca 1740
   gtgggaccca cggttcaatt attgccaatt ttcagctcca ccgtatattt aaaaaataaa 1800
   acgataatgc taaaaaaata taaatcgtaa cgatcgttaa atctcaacgg ctggatctta 1860
   tgacgaccgt tagaaattgt ggttgtcgac gagtcagtaa taaacggcgt caaagtggtt 1920
   gcagccggca cacacgagtc gtgtttatca actcaaagca caaatacttt tcctcaacct 1980
   aaaaataagg caattagcca aaaacaactt tgcgtgtaaa caacgctcaa tacacgtgtc 2040
   attttattat tagctattgc ttcaccgcct tagctttctc gtgacctagt cgtcctcgtc 2100
   ttttcttctt cttcttctat aaaacaatac ccaaagagct cttcttcttc acaattcaga 2160
   tttcaatttc tcaaaatctt aaaaactttc tctcaattct ctctaccgtg atcaaggtaa 2220
   atttctgtgt tccttattct ctcaaaatct tcgattttgt tttcgttcga tcccaatttc 2280
   gtatatgttc tttggtttag attctgttaa tcttagatcg aagacgattt tctgggtttg 2340
   atcgttagat atcatcttaa ttctcgatta gggtttcata gatatcatcc gatttgttca 2400
   aataatttga gttttgtcga ataattactc ttcgatttgt gatttctatc tagatctggt 2460
   gttagtttct agtttgtgcg atcgaatttg tcgattaatc tgagtttttc tgattaacag 2520
   atggcttcat ctgagaacgt tatcactgag ttcatgaggt tcaaggtgag gatggaaggt 2580
   actgttaacg gacatgagtt cgagatcgag ggtgagggtg aaggtagacc ttacgaggga 2640
   cataacaccg ttaagcttaa ggttacaaag ggtggacctc ttcctttcgc ttgggatatc 2700
   ctttctcctc aattccaata cggaagcaag gtaagtttgt ggattcttcg tccatgtgat 2760
   ctttgagttt ctttagagct tgtgagggat tagtaagtaa caatgcttga gttttttgct 2820
   gctgggcttc gaaaagtttg tcacttgttg gtttgatcca caaggtcttc ttctccatag 2880
   ctactagaca tgttttagct taagattcaa gtttatatat gccttgtgga ttaatcattg 2940
   cctgattctt ccgtgtcatc tctgagttta tttagagctt ggaagtggtg tagtaataac 3000
   taacaatact cttgataagt tgtagcaatg ctcttgatta gtggatgtaa tatgatgttg 3060
   ataagatata tgaggcacag aaccaaaagt ggtgcttcca ctagacccgt ttttagccta 3120
   aggttcaagt ttataccttg tagatgtttc tgtattgtct gattcttccc tgtgatattt 3180
   gaatttctta gagctttgga agtgatatag gaacaatgct cttgtgtgtt tgtctctatg 3240
   aagattatcg ctgtcgtgtt tcatccgagt gtgcgggatt ttttgctgct gggtttagcc 3300
   tttcttcaaa aagttattac ttgttagttt tattgttttg gtcttgataa gagatgttag 3360
   gacagacatg gtgcttcttg tctatagcca ctagacctat tttagcataa ggttaacgaa 3420
   attctctcta cataccttgt ggatttgttt acattgcctg atctttcctg tgatcgctgt 3480
   catgtttctt tggaatgatt gatgtttata aatggaaaaa tctttgtgca gaagactccc 3540
   gcccatctct ctatgcccgg gacaagtgcc accccacagt ggggcaggat gaggatgacc 3600
   accatggggt cgcagcgtgt gcgtgtccgt cgtacgttct ggccggccgg gccttgggcg 3660
   cgcgatcaga agcgttgcgt tggcgtgtgt gtgcttctgg tttgctttaa ttttaccaag 3720
   tttgtttcaa ggtggatcgc gtggtcaagg cccgtgtgct ttaaagaccc accggcactg 3780
   gcagtgagtg ttgctgcttg tgtaggcttt ggtacgtatg ggctttattt gcttctggat 3840
   gttgtgtact acttgggttt gttgaattat tatgagcagt tgcgtattgt aattcagctg 3900
   ggctacctgg acattgttat gtattaataa atgctttgct ttcttctaaa gatctttaag 3960
   tgctgtttaa acaaccgaca accactttgc ggacttcctt tcaagagaat tcaataaggt 4020
   taattcctaa ttgaaatccg aagataagat tcccacacac ttgtggctga tatcaaaagg 4080
   ctactgccta tttaaacaca tctctggaga ctgagaaaat cagacctcca agcatgaaga 4140
   agcctgagct tactgctact tctgttgaga agttcctcat cgagaagttc gattctgtgt 4200
   ctgatcttat gcagctctct gagggtgagg aatcaagagc tttctctttc gatgttggtg 4260
   gaagaggata cgttctcaga gttaactctt gcgctgacgg attctacaag gatagatacg 4320
   tgtacagaca cttcgcttca gctgctctcc ctatccctga agttcttgat atcggagagt 4380
   tctctgagtc tcttacctac tgtatctcaa gaagggctca gggtgttact cttcaagatc 4440
   ttcctgagac tgagcttcct gctgttcttc aacctgttgc tgaggctatg gatgctatcg 4500
   ctgctgctga tctttctcaa acttctggat tcggaccttt cggtcctcag ggaatcggac 4560
   agtacactac ttggagagat ttcatctgcg ctatcgctga tcctcatgtt taccattggc 4620
   agaccgttat ggatgatacc gtttctgctt ctgttgctca agctcttgat gagcttatgc 4680
   tttgggctga ggattgtcct gaggttagac atcttgttca cgctgatttc ggatctaaca 4740
   acgttctcac cgataacgga agaatcaccg ctgttatcga ttggtctgag gctatgttcg 4800
   gagattctca atacgaggtg gccaacatat tcttttggag gccttggctt gcttgtatgg 4860
   aacaacagac tagatacttc gagagaaggc atcctgagct tgctggatct cctagactta 4920
   gagcttacat gcttaggatc ggacttgatc agctttacca gtctctcgtt gatggaaact 4980
   tcgatgatgc tgcttgggct cagggaagat gtgatgctat cgttagatct ggtgctggaa 5040
   ctgttggaag aactcaaatc gctagaagat ctgctgctgt ttggactgat ggatgtgttg 5100
   aagttctcgc tgattctgga aacagaaggc cttctactag acctagagcc aagaagtgaa 5160
   gatcggcggc aatagcttct tagcgccatc ccgggttgat cctatctgtg ttgaaatagt 5220
   tgcggtgggc aaggctctct ttcagaaaga caggcggcca aaggaaccca aggtgaggtg 5280
   ggctatggct ctcagttcct tgtggaagcg cttggtctaa ggtgcagagg tgttagcggg 5340
   atgaagcaaa agtgtccgat tgtaacaaga tatgttgatc ctacgtaagg atattaaagt 5400
   atgtattcat cactaatata atcagtgtat tccaatatgt actacgattt ccaatgtctt 5460
   tattgtcgcc gtatgtaatc ggcgtcacaa aataatcccc ggtgactttc ttttaatcca 5520
   ggatgaaata atatgttatt ataatttttg cgatttggtc cgttatagga attgaagtgt 5580
   gcttgcggtc gccaccactc ccatttcata attttacatg tatttgaaaa ataaaaattt 5640
   atggtattca atttaaacac gtatacttgt aaagaatgat atcttgaaag aaatatagtt 5700
   taaatattta ttgataaaat aacaagtcag gtattatagt ccaagcaaaa acataaattt 5760
   attgatgcaa gtttaaattc agaaatattt caataactga ttatatcagc tggtacattg 5820
   ccgtagatga aagactgagt gcgatattat ggtgtaatac atagtttaaa cgggcccaag 5880
   actcccgccc atctctctat gcccgggaca agtgccaccc cacagtgggg caggatgagg 5940
   atgaccagtc agttttactt cccttaattt tctatgtact ttcataatta cttatgttat 6000
   tttcttcatg agttttaatg caaattacta tatggactct agtgaaaacg ttcagaatcc 6060
   tataaacatg actactgaga cgaacttgag agtagttttg atcatacaca cgtttcatgt 6120
   ggtacttgag agttactaat ttttgtcatc ttcgtataag tagtaaaaga tactacaaga 6180
   atagtttagt agaaaatact agcggtaggt gaagatttgt cgctatgtac tattattgtc 6240
   tagtaacttg agtaacaatt tcgtggtcta aatatcaaat aaaaatggat gagtggttca 6300
   ccaaatctag gcatcaaaac tattaatgtc attgtctaga tcttaggtga caccacattt 6360
   cgaatattta ttggtaattg agatgttaaa gtaccaatat ttgacttaat aaactaaaag 6420
   attttggctt tatcaaatgt agacattgat gacatatcgt tgtcattatc ttgagtatat 6480
   acaagtcgat caattaggtg aaagtttagt gtctcgtggt tggtaaacga ttaatacagt 6540
   agtatatttt atccaaagac aaaatccaaa tcatttcacc agtatgaata gtattatttt 6600
   atcttaaaag ctaaaatctt aaaaaccaag gtagcaccca cgttgagcta gacgatcaaa 6660
   tcgatttctg ctttgtccaa tttaccaagc tatttaaagc caaataattg aaatataggt 6720
   aggtcgttat attaggctaa gatttatctc aaatgcttaa ctaaaggaat aacaagggat 6780
   tctagttgtg tggttttata agattggtcc aatttcactt aagtttgttt attgtagaat 6840
   tttatatgtg aataatttga attccaattg aaaagatatt atagtaaaag aaaaaatagt 6900
   gcgaacaaaa aactttaatc ccataaaaag aaaaagaaaa atgaaaagtt cttctaacat 6960
   ccatattttg catcatatca taaagataag aaagatacat atcatagacg tacagataaa 7020
   caaacatatc atcatttgtg aaatacatag tacaataatt tgcttttaaa tagagtttaa 7080
   gtcacacaca ctgacacaca cgataaaacg ataatgtctg caaaaacact ttaatcccat 7140
   tgcctagagg acagcttctc cactttgtct ttaaggttgg ttttgccgtg ttgtttttat 7200
   ctttatataa tgatctattt tttggattat gaaatgaatt cacacatttt aattatttaa 7260
   gaagatccat atacaggttt ataacagtac taagtgatga ttattttttg tttttgcata 7320
   gtttagttta ttgggtaaac attcattacg tgtctcttta tacgaatcac ccatccaaaa 7380
   tttcaagtag tcttttagtt catttattat ttcataacta tttgacttat tgatttgaca 7440
   agaaacaaca aaagtgttga cttattgata gattgtggga tcataaaagt aattaagcgt 7500
   caaccacgac ccacaacaac aaagcacatg ttatacatta atatctcgtt tacttaatta 7560
   cagttttcag aatgccgttt catgtcttgt cactggcgat gttattatca tgttggacaa 7620
   tattcgactg ttgtcgtttt tacattttcg tattgactaa aactaaaaaa acaaaactct 7680
   gtttcaggtt gggcctagga tccacattgt acacacattt gcttaagtct atggaggcgc 7740
   aaggttttaa gtctgtggtt gctgttatag gccttccaaa cgatccatct gttaggttgc 7800
   atgaggcttt gggatacaca gcccggggta cattgcgcgc agctggatac aagcatggtg 7860
   gatggcatga tgttggtttt tggcaaaggg attttgagtt gccagctcct ccaaggccag 7920
   ttaggccagt tacccagatc taatatcaaa atctatttag aaatacacaa tattttgttg 7980
   caggcttgct ggagaatcga tctgctatca taaaaattac aaaaaaattt tatttgcctc 8040
   aattatttta ggattggtat taaggacgct taaattattt gtcgggtcac tacgcatcat 8100
   tgtgattgag aagatcagcg atacgaaata ttcgtagtac tatcgataat ttatttgaaa 8160
   attcataaga aaagcaaacg ttacatgaat tgatgaaaca atacaaagac agataaagcc 8220
   acgcacattt aggatattgg ccgagattac tgaatattga gtaagatcac ggaatttctg 8280
   acaggagcat gtcttcaatt cagcccaaat ggcagttgaa atactcaaac cgccccatat 8340
   gcaggagcgg atcattcatt gtttgtttgg ttgcctttgc caacatggga gtccaaggtt 8400
   gtttaaacat ttaaataccc tgccaagctt gaggtagcct ccaatttgac ggtgccgcca 8460
   gcgacgccgt ctggaactgt cctttttgag gaccactccg tttgtggaga tcatgagagt 8520
   ccatgctcaa caccgtgcac tagggacagg attgaagact cccgcccatc tcactaggga 8580
   caggattgcc accccacagt ggggcctaga aagactggag ttgcagagtt tgtgtcttct 8640
   agattaatcc tccaaacttt tgattaacca aaaaaattat caaactaaca tgttctcctt 8700
   ttttctttag aaattctaac gaatttatct ttatactgat ttgaatatac ttaatttggt 8760
   catttggatg ccctttacaa cctccttacc aaactattga tcacagtttc tattgctaaa 8820
   atcaccaaca aaacgcatgt cgccattcat aattatggtt tcacacctac aactaggcta 8880
   ataagtaaat aagtagacaa ctagactcag gtttgaaaaa accataaaag ccatatagcg 8940
   ttttctcatt gaaactgcga acacgatcgt gtgaatgttg cagtttctag ttttgataca 9000
   aacaaacaaa aacacaattt aatcttagat taaaaagaaa aaagagaacg gagcccacta 9060
   gccactcctt caaacgtgtc ttaccaactc tcttctagaa acaaattagg cttcaccttc 9120
   ctcttccaac ctctctctct ctctctctct ctttttctca aaccatctct ccataaagcc 9180
   ctaatttctt catcacaaga atcagaagaa gaaagatgga cctgcatcta attttcggtc 9240
   caacttgcac aggaaagacg acgaccgcga tagctcttgc ccagcagaca gggcttccag 9300
   tcctttcgct tgatcgggtc caatgctgtc ctcaactatc aaccggaagc ggacgaccaa 9360
   cagtggaaga actgaaagga acgacgcgtc tctaccttga tgatcggcct ctggtggagg 9420
   gtatcatcgc agccaagcaa gctcatcata ggctgatcga ggaggtgtat aatcatgagg 9480
   ccaacggcgg gcttattctt gagggaggat ccacctcgtt gctcaactgc atggcgcgaa 9540
   acagctattg gagtgcagat tttcgttggc atattattcg ccacaagtta cccgaccaag 9600
   agaccttcat gaaagcggcc aaggccagag ttaagcagat gttgcacccc gctgcaggcc 9660
   attctattat tcaagagttg gtttatcttt ggaatgaacc tcggctgagg cccattctga 9720
   aagagatcga tggatatcga tatgccatgt tgtttgctag ccagaaccag atcacggcag 9780
   atatgctatt gcagcttgac gcaaatatgg aaggtaagtt gattaatggg atcgctcagg 9840
   agtatttcat ccatgcgcgc caacaggaac agaaattccc ccaagttaac gcagccgctt 9900
   tcgacggatt cgaaggtcat ccgttcggaa tgtattagaa atcaccagtc tctctctaca 9960
   aatctatctc tctctatttt tctccagaat aatgtgtgag tagttcccag ataagggaat 10020
   tagggttctt atagggtttc gctcatgtgt tgagcatata agaaaccctt agtatgtatt 10080
   tgtatttgta aaatacttct atcaataaaa tttctaattc ctaaaaccaa aatccagtgt 10140
   ttaaacgagt ccatgctcaa caccgtgcac tagggacagg attgaagact cccgcccatc 10200
   tcactaggga caggattgcc accccacagt ggggcctaga aagactggag ttgcagacat 10260
   taaggatgac cagttcgtaa aggtcctgcg gtgtctattg cttttcatag gttaataagt 10320
   gtttgctaga ctgtggtgaa aggcctatcc gaagtaaggc cggccggatc cttcatcttt 10380
   ggacaaggga ataaagactc cccacttgct actaagaaca atacctaagt tgcccagaca 10440
   tgactgtacc cattcagaga cctaccaccc attagggcta tgacactaac actagcccct 10500
   ggaggttgac catgctaggc agtgggggtc tcacctatga cccactcaga taggggttta 10560
   aaccagtggg tgggatctca gcctcatata ggtgtttgtg gtgagctttc tcctagacaa 10620
   gagaaccctg aagaacagca agaaccagct aatatgatat gtagacatag tgggttgctc 10680
   aaattttgtg tttagtcata ttagaattga cctcagtgac cactcagaaa gtgcccaagc 10740
   ccatctatag gggccaaagt gctattgact ggtgtgtctg tgaattgttc ctccctacag 10800
   agttggtgct gatatatcct agcattcttt ggaaaaccta gctagggact gtcaagtgta 10860
   agatacctcc tgaattggag ggaacactag ctgccctgta ccttctggct agtaccttac 10920
   accctgaatg ggttaggggg tctattattt gctggaaata taccagtttc agtagggctg 10980
   ctgccttagg tcccacaagg tgtaacatgt gctcaatagt tgcactacca catgcacgtg 11040
   aacttaatga tgttatagcc acaacaccaa ccttggtttg cagtttgaca tccctctgga 11100
   atgggtgtag tcatcttgct ctggatctgc ctgaatcatt ggggctgtat gcagcctggg 11160
   cttaaagtga agaatgggat gtcccagaaa tattttgggt gagaagaatc ctggagtaga 11220
   tggtgacctg actatccctg tcctatgggc acaatctatc atcagatatt gcattcaaag 11280
   ggctatcatg ggatcaagtc ctaagtcaac tgttgtttac ctggcagaca ttcatctagg 11340
   agttctcttt tatgccaccc cacagtgatc cgccttttgc agtttatcca ctagggacag 11400
   gattgccacc ccacagtggg gcctctatgc ccgggacaag tgtaaaatat agagtatagg 11460
   ggttatcatc acagagaagc tattgctgga gggcctctgt tatttcctct ccatgccact 11520
   cccattttta acctaccaac tgaaatccca agggagactc caccctgtaa ctagagtcct 11580
   cagaggtgag ccatcccata ttaacaaatg ggcattaggg ctaggatgcc aagggatacc 11640
   tgaaatggga agttgtgggg ctgagtcctc ctgggaatca gagataatat gtaaacagtt 11700
   tgttgagaga ttgatgagag ctgactttga gaggtggcca tgctccctgg tcctcaatag 11760
   ggaaggcact acacaagaaa cctgggtttg atcaactgca ctgtgtccta ctcacacatt 11820
   gtgtgcctgg aaaaatgtta cttagtattt ggagggcctc cagaaccccc ctgggtgcaa 11880
   gactgggtgc tagtgactgg gtgaatgagt cttggacaca gtggccttgt ctaggttgtg 11940
   tgaggtggct aggcatcatg gcaatacctc ataattgatg agtgaggaaa caagactaag 12000
   tccttgactc ctcttattac atgacctggt ggatattatg tttaaactct gcaagctgga 12060
   atgagtactg ggtgcagatc ccctgggatt ctggctacaa aggtgaatga tagctagtct 12120
   gtttattagt agccaaaaaa gtcagtgagg ggtgagtgcc ctgggatgtt gttaagttca 12180
   cattgcacac ttggagaccc tctccatcca gtaacatacc agagaaaact gaccaagccc 12240
   tcatgggtgt atgggaacaa caaacctcct ggctacttca agggcacata acaccagcaa 12300
   ggagcctgtc ataaccacca tctcaaacaa tagaacttcc taagtgaagc aatgacttca 12360
   aatctacttg aaggcatgga gtataagcca tgttcctttc agaggggact gtacttctgt 12420
   agattacttt ccctcattaa ccagatctgg ccggccgcat gcggtcgatc gtgttggcca 12480
   ctcttgttta tctatcattc ctcgttggtc cagtcacagt tctaaaagtc tatggtgttc 12540
   cttacattgt aagtttcata tatttcttta ttatatcatt gctaatataa tttgtttttg 12600
   acataaaagt tttggaaaaa tttcagatct ttgtaatgtg gttggacgct gtcacgtact 12660
   tgcatcatca tggtcacgat gataagctgc cttggtacag aggcaaggta agtagatcaa 12720
   cattatttat aagaagcaat aatgattagt agttgaataa tctgaatttt tgatgttttt 12780
   gtacaataat aggaatggag ttatttacgt ggaggattaa caactgttga tagagattac 12840
   gggatcttca acaacattca tcacgatatt ggaactcacg tgatccatca tcttttccca 12900
   caaatccctc actatcactt ggtcgatgcc gtgagtgatc tcgctctctc tctagtttca 12960
   tttgattata ttaaagggtg attaattact aaattagtga tcttaattaa tgacatgcga 13020
   cagacgaaag cagctaaaca tgtgttggga agatactaca gagaaccaaa gacgtcagga 13080
   gcaataccga tccacttagt ggaaagtttg gtggcaagta ttaagaaaga tcattacgtc 13140
   agtgacactg gtgatattgt cttctacgag acagatccag atctctacgt ttatgcttct 13200
   gacaaatcca aaatcaatta atctttcttc ctagctctat ttaggaataa aacactcctt 13260
   tggttttact tatttctggt tgtttttaag ttaaaaatgt actcgtgaaa ctttttttta 13320
   ttaaatgtat ttacattaca aatcgtaaaa gtttttgttc gttttctcta tgtttttagt 13380
   tacaaactta caatcaaaaa ggtcttaaaa actttttgat ggtgggacgg acaaaagaaa 13440
   aagttcgact gagagtcgac aa 13462
<210> 146
   <211> 13462
   <212> DNA
   <213> Artificial Sequence
<220>
   <223> T-strand Insert Region of pDAS000275
<400> 146
   tatatatatt tgttttaatg gcttatttta ttgttaaatg gatacatcag cttgaaatat       60
   ctacgaacat gcatcatttt cctagataca tttgtttgtt gctcaaaaaa tgaataacgt      120
   agttaaacga gtgagattct tagcatctgc ctcgaaaacg atatgttatt gacaattcca 180
   atttcatttt tatgaaaata aaataatagt ttattttata attgggggtg gttgcaggag 240
   aataagccat cggacacacc accagaacca tggccatgtt gaaaacgacg agtcttgggt 300
   tccggtaatc cccctctcat tatttttttt tctttttttg aaactctttc attttaattt 360
   tcttagaatt ctatgtattt attttaatca atcctttttc cagtgtgagg cttggacgac 420
   cacttgtcag atttgtcgtt tagctgtagt aaacaactga tttaaattgt ttatggtact 480
   gtagttaact ttaacaacgg gccacttata ttcgagccat tggcataaaa tgattcttct 540
   cgaaattcgt ttacttttct tagtattttt cagttttgta gtttacgtag aactaataaa 600
   aagaaaaaaa cttataaaca caccacatgc aatgaataaa ttcgaatata taaccatact 660
   gttaaatatt aattaacatt ttaatcttaa ttttgcattc cagttgccag aaaaattata 720
   caagaatttg tcccacagta cacggatgct cagatacact gtccctctcc ccatgctcgc 780
   ttaccctctc tatctggtaa atcctaattc ctcatttttc ttcctgatta taattacaat 840
   tttgaatttt tagattttga gtattaacta aatataaatt aaatttgttt ggggatgact 900
   acagtggtac agaagtcctg gtaaagaagg gtcacattat aacccataca gtagtttatt 960
   tgccccaagc gagagaaagc ttattgcaac ttcaactact ggtaccgcct tttgcagttt 1020
   atctctatgc ccgggacaag tggagtccat gctcaacacc gtgcaggatg aggatgacca 1080
   ccgcggtagc gacttcgtgg gcgaggaaag cctttcgtcc aaggtggtcc ctcctcgcaa 1140
   tcttgttgga tggtgaatat tataaaagcc tgcccttctc gcgggtgttt aaacgtcgac 1200
   ctgcaggtca acggatcagg atattcttgt ttaagatgtt gaactctatg gaggtttgta 1260
   tgaactgatg atctaggacc ggataagttc ccttcttcat agcgaactta ttcaaagaat 1320
   gttttgtgta tcattcttgt tacattgtta ttaatgaaaa aatattattg gtcattggac 1380
   tgaacacgag tgttaaatat ggaccaggcc ccaaataaga tccattgata tatgaattaa 1440
   ataacaagaa taaatcgagt caccaaacca cttgcctttt ttaacgagac ttgttcacca 1500
   acttgataca aaagtcatta tcctatgcaa atcaataatc atacaaaaat atccaataac 1560
   actaaaaaat taaaagaaat ggataatttc acaatatgtt atacgataaa gaagttactt 1620
   ttccaagaaa ttcactgatt ttataagccc acttgcatta gataaatggc aaaaaaaaac 1680
   aaaaaggaaa agaaataaag cacgaagaat tctagaaaat acgaaatacg cttcaatgca 1740
   gtgggaccca cggttcaatt attgccaatt ttcagctcca ccgtatattt aaaaaataaa 1800
   acgataatgc taaaaaaata taaatcgtaa cgatcgttaa atctcaacgg ctggatctta 1860
   tgacgaccgt tagaaattgt ggttgtcgac gagtcagtaa taaacggcgt caaagtggtt 1920
   gcagccggca cacacgagtc gtgtttatca actcaaagca caaatacttt tcctcaacct 1980
   aaaaataagg caattagcca aaaacaactt tgcgtgtaaa caacgctcaa tacacgtgtc 2040
   attttattat tagctattgc ttcaccgcct tagctttctc gtgacctagt cgtcctcgtc 2100
   ttttcttctt cttcttctat aaaacaatac ccaaagagct cttcttcttc acaattcaga 2160
   tttcaatttc tcaaaatctt aaaaactttc tctcaattct ctctaccgtg atcaaggtaa 2220
   atttctgtgt tccttattct ctcaaaatct tcgattttgt tttcgttcga tcccaatttc 2280
   gtatatgttc tttggtttag attctgttaa tcttagatcg aagacgattt tctgggtttg 2340
   atcgttagat atcatcttaa ttctcgatta gggtttcata gatatcatcc gatttgttca 2400
   aataatttga gttttgtcga ataattactc ttcgatttgt gatttctatc tagatctggt 2460
   gttagtttct agtttgtgcg atcgaatttg tcgattaatc tgagtttttc tgattaacag 2520
   atggcttcat ctgagaacgt tatcactgag ttcatgaggt tcaaggtgag gatggaaggt 2580
   actgttaacg gacatgagtt cgagatcgag ggtgagggtg aaggtagacc ttacgaggga 2640
   cataacaccg ttaagcttaa ggttacaaag ggtggacctc ttcctttcgc ttgggatatc 2700
   ctttctcctc aattccaata cggaagcaag gtaagtttgt ggattcttcg tccatgtgat 2760
   ctttgagttt ctttagagct tgtgagggat tagtaagtaa caatgcttga gttttttgct 2820
   gctgggcttc gaaaagtttg tcacttgttg gtttgatcca caaggtcttc ttctccatag 2880
   ctactagaca tgttttagct taagattcaa gtttatatat gccttgtgga ttaatcattg 2940
   cctgattctt ccgtgtcatc tctgagttta tttagagctt ggaagtggtg tagtaataac 3000
   taacaatact cttgataagt tgtagcaatg ctcttgatta gtggatgtaa tatgatgttg 3060
   ataagatata tgaggcacag aaccaaaagt ggtgcttcca ctagacccgt ttttagccta 3120
   aggttcaagt ttataccttg tagatgtttc tgtattgtct gattcttccc tgtgatattt 3180
   gaatttctta gagctttgga agtgatatag gaacaatgct cttgtgtgtt tgtctctatg 3240
   aagattatcg ctgtcgtgtt tcatccgagt gtgcgggatt ttttgctgct gggtttagcc 3300
   tttcttcaaa aagttattac ttgttagttt tattgttttg gtcttgataa gagatgttag 3360
   gacagacatg gtgcttcttg tctatagcca ctagacctat tttagcataa ggttaacgaa 3420
   attctctcta cataccttgt ggatttgttt acattgcctg atctttcctg tgatcgctgt 3480
   catgtttctt tggaatgatt gatgtttata aatggaaaaa tctttgtgca gaagactccc 3540
   gcccatctct ctatgcccgg gacaagtgcc accccacagt ggggcaggat gaggatgacc 3600
   accatggggt cgcagcgtgt gcgtgtccgt cgtacgttct ggccggccgg gccttgggcg 3660
   cgcgatcaga agcgttgcgt tggcgtgtgt gtgcttctgg tttgctttaa ttttaccaag 3720
   tttgtttcaa ggtggatcgc gtggtcaagg cccgtgtgct ttaaagaccc accggcactg 3780
   gcagtgagtg ttgctgcttg tgtaggcttt ggtacgtatg ggctttattt gcttctggat 3840
   gttgtgtact acttgggttt gttgaattat tatgagcagt tgcgtattgt aattcagctg 3900
   ggctacctgg acattgttat gtattaataa atgctttgct ttcttctaaa gatctttaag 3960
   tgctgtttaa acaaccgaca accactttgc ggacttcctt tcaagagaat tcaataaggt 4020
   taattcctaa ttgaaatccg aagataagat tcccacacac ttgtggctga tatcaaaagg 4080
   ctactgccta tttaaacaca tctctggaga ctgagaaaat cagacctcca agcatgaaga 4140
   agcctgagct tactgctact tctgttgaga agttcctcat cgagaagttc gattctgtgt 4200
   ctgatcttat gcagctctct gagggtgagg aatcaagagc tttctctttc gatgttggtg 4260
   gaagaggata cgttctcaga gttaactctt gcgctgacgg attctacaag gatagatacg 4320
   tgtacagaca cttcgcttca gctgctctcc ctatccctga agttcttgat atcggagagt 4380
   tctctgagtc tcttacctac tgtatctcaa gaagggctca gggtgttact cttcaagatc 4440
   ttcctgagac tgagcttcct gctgttcttc aacctgttgc tgaggctatg gatgctatcg 4500
   ctgctgctga tctttctcaa acttctggat tcggaccttt cggtcctcag ggaatcggac 4560
   agtacactac ttggagagat ttcatctgcg ctatcgctga tcctcatgtt taccattggc 4620
   agaccgttat ggatgatacc gtttctgctt ctgttgctca agctcttgat gagcttatgc 4680
   tttgggctga ggattgtcct gaggttagac atcttgttca cgctgatttc ggatctaaca 4740
   acgttctcac cgataacgga agaatcaccg ctgttatcga ttggtctgag gctatgttcg 4800
   gagattctca atacgaggtg gccaacatat tcttttggag gccttggctt gcttgtatgg 4860
   aacaacagac tagatacttc gagagaaggc atcctgagct tgctggatct cctagactta 4920
   gagcttacat gcttaggatc ggacttgatc agctttacca gtctctcgtt gatggaaact 4980
   tcgatgatgc tgcttgggct cagggaagat gtgatgctat cgttagatct ggtgctggaa 5040
   ctgttggaag aactcaaatc gctagaagat ctgctgctgt ttggactgat ggatgtgttg 5100
   aagttctcgc tgattctgga aacagaaggc cttctactag acctagagcc aagaagtgaa 5160
   gatcggcggc aatagcttct tagcgccatc ccgggttgat cctatctgtg ttgaaatagt 5220
   tgcggtgggc aaggctctct ttcagaaaga caggcggcca aaggaaccca aggtgaggtg 5280
   ggctatggct ctcagttcct tgtggaagcg cttggtctaa ggtgcagagg tgttagcggg 5340
   atgaagcaaa agtgtccgat tgtaacaaga tatgttgatc ctacgtaagg atattaaagt 5400
   atgtattcat cactaatata atcagtgtat tccaatatgt actacgattt ccaatgtctt 5460
   tattgtcgcc gtatgtaatc ggcgtcacaa aataatcccc ggtgactttc ttttaatcca 5520
   ggatgaaata atatgttatt ataatttttg cgatttggtc cgttatagga attgaagtgt 5580
   gcttgcggtc gccaccactc ccatttcata attttacatg tatttgaaaa ataaaaattt 5640
   atggtattca atttaaacac gtatacttgt aaagaatgat atcttgaaag aaatatagtt 5700
   taaatattta ttgataaaat aacaagtcag gtattatagt ccaagcaaaa acataaattt 5760
   attgatgcaa gtttaaattc agaaatattt caataactga ttatatcagc tggtacattg 5820
   ccgtagatga aagactgagt gcgatattat ggtgtaatac atagtttaaa cgggcccaag 5880
   actcccgccc atctctctat gcccgggaca agtgccaccc cacagtgggg caggatgagg 5940
   atgaccagtc agttttactt cccttaattt tctatgtact ttcataatta cttatgttat 6000
   tttcttcatg agttttaatg caaattacta tatggactct agtgaaaacg ttcagaatcc 6060
   tataaacatg actactgaga cgaacttgag agtagttttg atcatacaca cgtttcatgt 6120
   ggtacttgag agttactaat ttttgtcatc ttcgtataag tagtaaaaga tactacaaga 6180
   atagtttagt agaaaatact agcggtaggt gaagatttgt cgctatgtac tattattgtc 6240
   tagtaacttg agtaacaatt tcgtggtcta aatatcaaat aaaaatggat gagtggttca 6300
   ccaaatctag gcatcaaaac tattaatgtc attgtctaga tcttaggtga caccacattt 6360
   cgaatattta ttggtaattg agatgttaaa gtaccaatat ttgacttaat aaactaaaag 6420
   attttggctt tatcaaatgt agacattgat gacatatcgt tgtcattatc ttgagtatat 6480
   acaagtcgat caattaggtg aaagtttagt gtctcgtggt tggtaaacga ttaatacagt 6540
   agtatatttt atccaaagac aaaatccaaa tcatttcacc agtatgaata gtattatttt 6600
   atcttaaaag ctaaaatctt aaaaaccaag gtagcaccca cgttgagcta gacgatcaaa 6660
   tcgatttctg ctttgtccaa tttaccaagc tatttaaagc caaataattg aaatataggt 6720
   aggtcgttat attaggctaa gatttatctc aaatgcttaa ctaaaggaat aacaagggat 6780
   tctagttgtg tggttttata agattggtcc aatttcactt aagtttgttt attgtagaat 6840
   tttatatgtg aataatttga attccaattg aaaagatatt atagtaaaag aaaaaatagt 6900
   gcgaacaaaa aactttaatc ccataaaaag aaaaagaaaa atgaaaagtt cttctaacat 6960
   ccatattttg catcatatca taaagataag aaagatacat atcatagacg tacagataaa 7020
   caaacatatc atcatttgtg aaatacatag tacaataatt tgcttttaaa tagagtttaa 7080
   gtcacacaca ctgacacaca cgataaaacg ataatgtctg caaaaacact ttaatcccat 7140
   tgcctagagg acagcttctc cactttgtct ttaaggttgg ttttgccgtg ttgtttttat 7200
   ctttatataa tgatctattt tttggattat gaaatgaatt cacacatttt aattatttaa 7260
   gaagatccat atacaggttt ataacagtac taagtgatga ttattttttg tttttgcata 7320
   gtttagttta ttgggtaaac attcattacg tgtctcttta tacgaatcac ccatccaaaa 7380
   tttcaagtag tcttttagtt catttattat ttcataacta tttgacttat tgatttgaca 7440
   agaaacaaca aaagtgttga cttattgata gattgtggga tcataaaagt aattaagcgt 7500
   caaccacgac ccacaacaac aaagcacatg ttatacatta atatctcgtt tacttaatta 7560
   cagttttcag aatgccgttt catgtcttgt cactggcgat gttattatca tgttggacaa 7620
   tattcgactg ttgtcgtttt tacattttcg tattgactaa aactaaaaaa acaaaactct 7680
   gtttcaggtt gggcctagga tccacattgt acacacattt gcttaagtct atggaggcgc 7740
   aaggttttaa gtctgtggtt gctgttatag gccttccaaa cgatccatct gttaggttgc 7800
   atgaggcttt gggatacaca gcccggggta cattgcgcgc agctggatac aagcatggtg 7860
   gatggcatga tgttggtttt tggcaaaggg attttgagtt gccagctcct ccaaggccag 7920
   ttaggccagt tacccagatc taatatcaaa atctatttag aaatacacaa tattttgttg 7980
   caggcttgct ggagaatcga tctgctatca taaaaattac aaaaaaattt tatttgcctc 8040
   aattatttta ggattggtat taaggacgct taaattattt gtcgggtcac tacgcatcat 8100
   tgtgattgag aagatcagcg atacgaaata ttcgtagtac tatcgataat ttatttgaaa 8160
   attcataaga aaagcaaacg ttacatgaat tgatgaaaca atacaaagac agataaagcc 8220
   acgcacattt aggatattgg ccgagattac tgaatattga gtaagatcac ggaatttctg 8280
   acaggagcat gtcttcaatt cagcccaaat ggcagttgaa atactcaaac cgccccatat 8340
   gcaggagcgg atcattcatt gtttgtttgg ttgcctttgc caacatggga gtccaaggtt 8400
   gtttaaacat ttaaataccc tgccaagctt gaggtagcct ccaatttgac ggtgccgcca 8460
   gcgacgccgt ctggaactgt cctttttgag gaccactccg tttgtggaga tcatgagagt 8520
   ccatgctcaa caccgtgcac tagggacagg attgaagact cccgcccatc tcactaggga 8580
   caggattgcc accccacagt ggggcctaga aagactggag ttgcagagtt tgtgtcttct 8640
   agattaatcc tccaaacttt tgattaacca aaaaaattat caaactaaca tgttctcctt 8700
   ttttctttag aaattctaac gaatttatct ttatactgat ttgaatatac ttaatttggt 8760
   catttggatg ccctttacaa cctccttacc aaactattga tcacagtttc tattgctaaa 8820
   atcaccaaca aaacgcatgt cgccattcat aattatggtt tcacacctac aactaggcta 8880
   ataagtaaat aagtagacaa ctagactcag gtttgaaaaa accataaaag ccatatagcg 8940
   ttttctcatt gaaactgcga acacgatcgt gtgaatgttg cagtttctag ttttgataca 9000
   aacaaacaaa aacacaattt aatcttagat taaaaagaaa aaagagaacg gagcccacta 9060
   gccactcctt caaacgtgtc ttaccaactc tcttctagaa acaaattagg cttcaccttc 9120
   ctcttccaac ctctctctct ctctctctct ctttttctca aaccatctct ccataaagcc 9180
   ctaatttctt catcacaaga atcagaagaa gaaagatgga cctgcatcta attttcggtc 9240
   caacttgcac aggaaagacg acgaccgcga tagctcttgc ccagcagaca gggcttccag 9300
   tcctttcgct tgatcgggtc caatgctgtc ctcaactatc aaccggaagc ggacgaccaa 9360
   cagtggaaga actgaaagga acgacgcgtc tctaccttga tgatcggcct ctggtggagg 9420
   gtatcatcgc agccaagcaa gctcatcata ggctgatcga ggaggtgtat aatcatgagg 9480
   ccaacggcgg gcttattctt gagggaggat ccacctcgtt gctcaactgc atggcgcgaa 9540
   acagctattg gagtgcagat tttcgttggc atattattcg ccacaagtta cccgaccaag 9600
   agaccttcat gaaagcggcc aaggccagag ttaagcagat gttgcacccc gctgcaggcc 9660
   attctattat tcaagagttg gtttatcttt ggaatgaacc tcggctgagg cccattctga 9720
   aagagatcga tggatatcga tatgccatgt tgtttgctag ccagaaccag atcacggcag 9780
   atatgctatt gcagcttgac gcaaatatgg aaggtaagtt gattaatggg atcgctcagg 9840
   agtatttcat ccatgcgcgc caacaggaac agaaattccc ccaagttaac gcagccgctt 9900
   tcgacggatt cgaaggtcat ccgttcggaa tgtattagaa atcaccagtc tctctctaca 9960
   aatctatctc tctctatttt tctccagaat aatgtgtgag tagttcccag ataagggaat 10020
   tagggttctt atagggtttc gctcatgtgt tgagcatata agaaaccctt agtatgtatt 10080
   tgtatttgta aaatacttct atcaataaaa tttctaattc ctaaaaccaa aatccagtgt 10140
   ttaaacgagt ccatgctcaa caccgtgcac tagggacagg attgaagact cccgcccatc 10200
   tcactaggga caggattgcc accccacagt ggggcctaga aagactggag ttgcagacat 10260
   taaggatgac cagttcgtaa aggtcctgcg gtgtctattg cttttcatag gttaataagt 10320
   gtttgctaga ctgtggtgaa aggcctatcc gaagtaaggc cggccggatc cttcatcttt 10380
   ggacaaggga ataaagactc cccacttgct actaagaaca atacctaagt tgcccagaca 10440
   tgactgtacc cattcagaga cctaccaccc attagggcta tgacactaac actagcccct 10500
   ggaggttgac catgctaggc agtgggggtc tcacctatga cccactcaga taggggttta 10560
   aaccagtggg tgggatctca gcctcatata ggtgtttgtg gtgagctttc tcctagacaa 10620
   gagaaccctg aagaacagca agaaccagct aatatgatat gtagacatag tgggttgctc 10680
   aaattttgtg tttagtcata ttagaattga cctcagtgac cactcagaaa gtgcccaagc 10740
   ccatctatag gggccaaagt gctattgact ggtgtgtctg tgaattgttc ctccctacag 10800
   agttggtgct gatatatcct agcattcttt ggaaaaccta gctagggact gtcaagtgta 10860
   agatacctcc tgaattggag ggaacactag ctgccctgta ccttctggct agtaccttac 10920
   accctgaatg ggttaggggg tctattattt gctggaaata taccagtttc agtagggctg 10980
   ctgccttagg tcccacaagg tgtaacatgt gctcaatagt tgcactacca catgcacgtg 11040
   aacttaatga tgttatagcc acaacaccaa ccttggtttg cagtttgaca tccctctgga 11100
   atgggtgtag tcatcttgct ctggatctgc ctgaatcatt ggggctgtat gcagcctggg 11160
   cttaaagtga agaatgggat gtcccagaaa tattttgggt gagaagaatc ctggagtaga 11220
   tggtgacctg actatccctg tcctatgggc acaatctatc atcagatatt gcattcaaag 11280
   ggctatcatg ggatcaagtc ctaagtcaac tgttgtttac ctggcagaca ttcatctagg 11340
   agttctcttt tatgccaccc cacagtgatc cgccttttgc agtttatcca ctagggacag 11400
   gattgccacc ccacagtggg gcctctatgc ccgggacaag tgtaaaatat agagtatagg 11460
   ggttatcatc acagagaagc tattgctgga gggcctctgt tatttcctct ccatgccact 11520
   cccattttta acctaccaac tgaaatccca agggagactc caccctgtaa ctagagtcct 11580
   cagaggtgag ccatcccata ttaacaaatg ggcattaggg ctaggatgcc aagggatacc 11640
   tgaaatggga agttgtgggg ctgagtcctc ctgggaatca gagataatat gtaaacagtt 11700
   tgttgagaga ttgatgagag ctgactttga gaggtggcca tgctccctgg tcctcaatag 11760
   ggaaggcact acacaagaaa cctgggtttg atcaactgca ctgtgtccta ctcacacatt 11820
   gtgtgcctgg aaaaatgtta cttagtattt ggagggcctc cagaaccccc ctgggtgcaa 11880
   gactgggtgc tagtgactgg gtgaatgagt cttggacaca gtggccttgt ctaggttgtg 11940
   tgaggtggct aggcatcatg gcaatacctc ataattgatg agtgaggaaa caagactaag 12000
   tccttgactc ctcttattac atgacctggt ggatattatg tttaaactct gcaagctgga 12060
   atgagtactg ggtgcagatc ccctgggatt ctggctacaa aggtgaatga tagctagtct 12120
   gtttattagt agccaaaaaa gtcagtgagg ggtgagtgcc ctgggatgtt gttaagttca 12180
   cattgcacac ttggagaccc tctccatcca gtaacatacc agagaaaact gaccaagccc 12240
   tcatgggtgt atgggaacaa caaacctcct ggctacttca agggcacata acaccagcaa 12300
   ggagcctgtc ataaccacca tctcaaacaa tagaacttcc taagtgaagc aatgacttca 12360
   aatctacttg aaggcatgga gtataagcca tgttcctttc agaggggact gtacttctgt 12420
   agattacttt ccctcattaa ccagatctgg ccggccgcat gcggtcgatc atgttggcca 12480
   ctcttgttta tctatcattc ctcgttggtc cagtcacagt tctaaaagtc tatggtgttc 12540
   cttacattgt aagtttcata tatttcatta ttatatcatt gctaatataa tttgtttttg 12600
   acataaagtt ttggaaaaat ttcagatctt tgtaatgtgg ttggacgctg tcacgtactt 12660
   gcatcatcat ggtcacgatg ataagttgcc ttggtacaga ggcaaggtaa gtagatcaac 12720
   attaatttat aagaagcaac aatgattagt atttgattaa tctaaattat tgatgttttg 12780
   tgtacaataa taggaatgga gttatttacg tggaggatta acaactattg atagagatta 12840
   cgggatcttc aacaacattc atcacgatat tggaactcac gtgatccatc atcttttccc 12900
   acaaatccct cactatcact tggttgatgc cgtgagtgat ctcgctctct ctctagtttc 12960
   atttgattaa aattaaaggg tgattaatta ctaaattagt gatcttaatt aatgatatgc 13020
   gacagacgaa atcagctaaa catgtgttgg gaagatacta cagagaacca aagacgtcag 13080
   gagcaatacc gatccacttg gtggaaagtt tggtggcaag tattaagaaa gatcattacg 13140
   tcagtgacac tggtgatatt gtcttctacg agacagatcc agatctctac gtttatgctt 13200
   ctgacaaatc caaaatcaac taacctttct tcctagctct atttaggaat aaaacagtcc 13260
   tttggttttt acttatttct ggttgttttt aagttaaatg tactcgtgaa acttttttta 13320
   attaaatgta tttacattac aaatcaagtt tttgttcgtt ttctttatgt ttttagttac 13380
   aataaataaa ggtcttaaaa actttttgtt ggtggggaca aaagaaaaag ttcgactgag 13440
   agagtcgaca aaatgcacgc cg 13462
<210> 147
   <211> 5521
   <212> DNA
   <213> Artificial Sequence
<220>
   <223> T-strand Insert Region of pDAS000031
<400> 147
   tggcaggata tattgtggtg taaacaaatt gacgcttaga caacttaata acacattgcg      60
   gacgttttta atgtactgaa ttaacgccga attgaattcg agctcggtac cggggacaac     120
   tttgtataga aaagttgggt ggtttaaact atgtattaca ccataatatc gcactcagtc     180
   tttcatctac ggcaatgtac cagctgatat aatcagttat tgaaatattt ctgaatttaa     240
   acttgcatca ataaatttat gtttttgctt ggactataat acctgacttg ttattttatc     300
   aataaatatt taaactatat ttctttcaag atatcattct ttacaagtat acgtgtttaa     360
   attgaatacc ataaattttt atttttcaaa tacatgtaaa attatgaaat gggagtggtg     420
   gcgaccgcaa gcacacttca attcctataa cggaccaaat cgcaaaaatt ataataacat     480
   attatttcat cctggattaa aagaaagtca ccggggatta ttttgtgacg ccgattacat     540
   acggcgacaa taaagacatt ggaaatcgta gtacatattg gaatacactg attatattag     600
   tgatgaatac atactttaat atccttacgt aggatcaaca tatcttgtta caatcggaca     660
   cttttgcttc atcccgctaa cacctctgca ccttagacca agcgcttcca caaggaactg     720
   agagccatag cccacctcac cttgggttcc tttggccgcc tgtctttctg aaagagagcc     780
   ttgcccaccg caactatttc aacacagata ggatcaaccc gggatggcgc taagaagcta     840
   ttgccgccga tcttcacttc ttggctctag gtctagtaga aggccttctg tttccagaat     900
   cagcgagaac ttcaacacat ccatcagtcc aaacagcagc agatcttcta gcgatttgag     960
   ttcttccaac agttccagca ccagatctaa cgatagcatc acatcttccc tgagcccaag    1020
   cagcatcatc gaagtttcca tcaacgagag actggtaaag ctgatcaagt ccgatcctaa    1080
   gcatgtaagc tctaagtcta ggagatccag caagctcagg atgccttctc tcgaagtatc    1140
   tagtctgttg ttccatacaa gcaagccaag gcctccaaaa gaatatgttg gccacctcgt    1200
   attgagaatc tccgaacata gcctcagacc aatcgataac agcggtgatt cttccgttat    1260
   cggtgagaac gttgttagat ccgaaatcag cgtgaacaag atgtctaacc tcaggacaat    1320
   cctcagccca aagcataagc tcatcaagag cttgagcaac agaagcagaa acggtatcat    1380
   ccataacggt ctgccaatgg taaacatgag gatcagcgat agcgcagatg aaatctctcc    1440
   aagtagtgta ctgtccgatt ccctgaggac cgaaaggtcc gaatccagaa gtttgagaaa 1500
   gatcagcagc agcgatagca tccatagcct cagcaacagg ttgaagaaca gcaggaagct 1560
   cagtctcagg aagatcttga agagtaacac cctgagccct tcttgagata cagtaggtaa 1620
   gagactcaga gaactctccg atatcaagaa cttcagggat agggagagca gctgaagcga 1680
   agtgtctgta cacgtatcta tccttgtaga atccgtcagc gcaagagtta actctgagaa 1740
   cgtatcctct tccaccaaca tcgaaagaga aagctcttga ttcctcaccc tcagagagct 1800
   gcataagatc agacacagaa tcgaacttct cgatgaggaa cttctcaaca gaagtagcag 1860
   taagctcagg cttcttcatg cttggaggtc tgattttctc agtctccaga gatgtgttta 1920
   aataggcagt agccttttga tatcagccac aagtgtgtgg gaatcttatc ttcggatttc 1980
   aattaggaat taaccttatt gaattctctt gaaaggaagt ccgcaaagtg gttgtcggtt 2040
   gtttaaacca acttttgtat acaaagttgt cccctctaga gtcgacctgc aggcatgcaa 2100
   gcttagcttg agcttggatc agattgtcgt ttcccgcctt cagtttatca caagtttgta 2160
   caaaaaagca ggctgtcgac ctgcaggtca acggatcagg atattcttgt ttaagatgtt 2220
   gaactctatg gaggtttgta tgaactgatg atctaggacc ggataagttc ccttcttcat 2280
   agcgaactta ttcaaagaat gttttgtgta tcattcttgt tacattgtta ttaatgaaaa 2340
   aatattattg gtcattggac tgaacacgag tgttaaatat ggaccaggcc ccaaataaga 2400
   tccattgata tatgaattaa ataacaagaa taaatcgagt caccaaacca cttgcctttt 2460
   ttaacgagac ttgttcacca acttgataca aaagtcatta tcctatgcaa atcaataatc 2520
   atacaaaaat atccaataac actaaaaaat taaaagaaat ggataatttc acaatatgtt 2580
   atacgataaa gaagttactt ttccaagaaa ttcactgatt ttataagccc acttgcatta 2640
   gataaatggc aaaaaaaaac aaaaaggaaa agaaataaag cacgaagaat tctagaaaat 2700
   acgaaatacg cttcaatgca gtgggaccca cggttcaatt attgccaatt ttcagctcca 2760
   ccgtatattt aaaaaataaa acgataatgc taaaaaaata taaatcgtaa cgatcgttaa 2820
   atctcaacgg ctggatctta tgacgaccgt tagaaattgt ggttgtcgac gagtcagtaa 2880
   taaacggcgt caaagtggtt gcagccggca cacacgagtc gtgtttatca actcaaagca 2940
   caaatacttt tcctcaacct aaaaataagg caattagcca aaaacaactt tgcgtgtaaa 3000
   caacgctcaa tacacgtgtc attttattat tagctattgc ttcaccgcct tagctttctc 3060
   gtgacctagt cgtcctcgtc ttttcttctt cttcttctat aaaacaatac ccaaagagct 3120
   cttcttcttc acaattcaga tttcaatttc tcaaaatctt aaaaactttc tctcaattct 3180
   ctctaccgtg atcaaggtaa atttctgtgt tccttattct ctcaaaatct tcgattttgt 3240
   tttcgttcga tcccaatttc gtatatgttc tttggtttag attctgttaa tcttagatcg 3300
   aagacgattt tctgggtttg atcgttagat atcatcttaa ttctcgatta gggtttcata 3360
   gatatcatcc gatttgttca aataatttga gttttgtcga ataattactc ttcgatttgt 3420
   gatttctatc tagatctggt gttagtttct agtttgtgcg atcgaatttg tcgattaatc 3480
   tgagtttttc tgattaacag atggcttcat ctgagaacgt tatcactgag ttcatgaggt 3540
   tcaaggtgag gatggaaggt actgttaacg gacatgagtt cgagatcgag ggtgagggtg 3600
   aaggtagacc ttacgaggga cataacaccg ttaagcttaa ggttacaaag ggtggacctc 3660
   ttcctttcgc ttgggatatc ctttctcctc aattccaata cggaagcaag gtaagtttgt 3720
   ggattcttcg tccatgtgat ctttgagttt ctttagagct tgtgagggat tagtaagtaa 3780
   caatgcttga gttttttgct gctgggcttc gaaaagtttg tcacttgttg gtttgatcca 3840
   caaggtcttc ttctccatag ctactagaca tgttttagct taagattcaa gtttatatat 3900
   gccttgtgga ttaatcattg cctgattctt ccgtgtcatc tctgagttta tttagagctt 3960
   ggaagtggtg tagtaataac taacaatact cttgataagt tgtagcaatg ctcttgatta 4020
   gtggatgtaa tatgatgttg ataagatata tgaggcacag aaccaaaagt ggtgcttcca 4080
   ctagacccgt ttttagccta aggttcaagt ttataccttg tagatgtttc tgtattgtct 4140
   gattcttccc tgtgatattt gaatttctta gagctttgga agtgatatag gaacaatgct 4200
   cttgtgtgtt tgtctctatg aagattatcg ctgtcgtgtt tcatccgagt gtgcgggatt 4260
   ttttgctgct gggtttagcc tttcttcaaa aagttattac ttgttagttt tattgttttg 4320
   gtcttgataa gagatgttag gacagacatg gtgcttcttg tctatagcca ctagacctat 4380
   tttagcataa ggttaacgaa attctctcta cataccttgt ggatttgttt acattgcctg 4440
   atctttcctg tgatcgctgt catgtttctt tggaatgatt gatgtttata aatggaaaaa 4500
   tctttgtgca ggtttacgtt aagcaccctg ctgatatccc tgattacaag aagctttcat 4560
   tccctgaggg attcaagtgg gagagagtta tgaacttcga ggatggtggt gttgctactg 4620
   ttactcagga ttcttcactt caggacggat gcttcatcta caaggttaag ttcatcggag 4680
   tgaacttccc ttctgatgga cctgttatgc agaaaaagac tatgggatgg gaggcttcta 4740
   ccgagagact ttaccctaga gatggtgttc ttaagggtga gactcacaag gctcttaagc 4800
   ttaaagatgg tggacactac ctcgtcgagt tcaagtctat ctacatggct aagaagcctg 4860
   ttcagcttcc tggttactac tacgttgacg ctaagcttga tatcacctct cacaacgagg 4920
   actacactat cgttgagcaa tacgagagaa ctgagggtag acatcacttg ttcctctgat 4980
   atcaaaatct atttagaaat acacaatatt ttgttgcagg cttgctggag aatcgatctg 5040
   ctatcataaa aattacaaaa aaattttatt tgcctcaatt attttaggat tggtattaag 5100
   gacgcttaaa ttatttgtcg ggtcactacg catcattgtg attgagaaga tcagcgatac 5160
   gaaatattcg tagtactatc gataatttat ttgaaaattc ataagaaaag caaacgttac 5220
   atgaattgat gaaacaatac aaagacagat aaagccacgc acatttagga tattggccga     5280
   gattactgaa tattgagtaa gatcacggaa tttctgacag gagcatgtct tcaattcagc     5340
   ccaaatggca gttgaaatac tcaaaccgcc ccatatgcag gagcggatca ttcattgttt     5400
   gtttggttgc ctttgccaac atgggagtcc aaggtttacc cagctttctt gtacaaagtg     5460
   gtgataaact atcagtgttt gacaggatat attggcgggt aaacctaaga gaaaagagcg     5520
   t                                                                     5521
<210> 148
   <211> 11708
   <212> DNA
   <213> Artificial Sequence
<220>
   <223> T-strand Insert Region of pDAS000036
<400> 148
<210> 149
   <211> 11707
   <212> DNA
   <213> Artificial Sequence
<220>
   <223> T-strand Insert Region of pDAS000037
<400> 149
   tggcaggata tattgtggtg taaacaaatt gacgcttaga caacttaata acacattgcg      60
   gacgttttta atgtactgaa ttaacgccga attgaattcg agctcggtac ccggggatcc     120
   tctagagtcg acctgcaggc atgcaagctt agcttgagct tggatcagat tgtcgtttcc     180
   cgccttcagt ttatcacaag tttgtacaaa aaagcaggct aagactcccg cccatctcac     240
   tagggacagg attggagtcc atgctcaaca ccgtgcagga tgaggatgac catagcgact     300
   tcgtgggcga ggaaagcctt tcgtccaagg tggtccctcc tcgcaatctt gttggatggt     360
   gaatattata aaagcctgcc cttctcgcgg gtgtttaaac gtcgacctgc aggtcaacgg     420
   atcaggatat tcttgtttaa gatgttgaac tctatggagg tttgtatgaa ctgatgatct     480
   aggaccggat aagttccctt cttcatagcg aacttattca aagaatgttt tgtgtatcat     540
   tcttgttaca ttgttattaa tgaaaaaata ttattggtca ttggactgaa cacgagtgtt 600
   aaatatggac caggccccaa ataagatcca ttgatatatg aattaaataa caagaataaa 660
   tcgagtcacc aaaccacttg ccttttttaa cgagacttgt tcaccaactt gatacaaaag 720
   tcattatcct atgcaaatca ataatcatac aaaaatatcc aataacacta aaaaattaaa 780
   agaaatggat aatttcacaa tatgttatac gataaagaag ttacttttcc aagaaattca 840
   ctgattttat aagcccactt gcattagata aatggcaaaa aaaaacaaaa aggaaaagaa 900
   ataaagcacg aagaattcta gaaaatacga aatacgcttc aatgcagtgg gacccacggt 960
   tcaattattg ccaattttca gctccaccgt atatttaaaa aataaaacga taatgctaaa 1020
   aaaatataaa tcgtaacgat cgttaaatct caacggctgg atcttatgac gaccgttaga 1080
   aattgtggtt gtcgacgagt cagtaataaa cggcgtcaaa gtggttgcag ccggcacaca 1140
   cgagtcgtgt ttatcaactc aaagcacaaa tacttttcct caacctaaaa ataaggcaat 1200
   tagccaaaaa caactttgcg tgtaaacaac gctcaataca cgtgtcattt tattattagc 1260
   tattgcttca ccgccttagc tttctcgtga cctagtcgtc ctcgtctttt cttcttcttc 1320
   ttctataaaa caatacccaa agagctcttc ttcttcacaa ttcagatttc aatttctcaa 1380
   aatcttaaaa actttctctc aattctctct accgtgatca aggtaaattt ctgtgttcct 1440
   tattctctca aaatcttcga ttttgttttc gttcgatccc aatttcgtat atgttctttg 1500
   gtttagattc tgttaatctt agatcgaaga cgattttctg ggtttgatcg ttagatatca 1560
   tcttaattct cgattagggt ttcatagata tcatccgatt tgttcaaata atttgagttt 1620
   tgtcgaataa ttactcttcg atttgtgatt tctatctaga tctggtgtta gtttctagtt 1680
   tgtgcgatcg aatttgtcga ttaatctgag tttttctgat taacagatgg cttcatctga 1740
   gaacgttatc actgagttca tgaggttcaa ggtgaggatg gaaggtactg ttaacggaca 1800
   tgagttcgag atcgagggtg agggtgaagg tagaccttac gagggacata acaccgttaa 1860
   gcttaaggtt acaaagggtg gacctcttcc tttcgcttgg gatatccttt ctcctcaatt 1920
   ccaatacgga agcaaggtaa gtttgtggat tcttcgtcca tgtgatcttt gagtttcttt 1980
   agagcttgtg agggattagt aagtaacaat gcttgagttt tttgctgctg ggcttcgaaa 2040
   agtttgtcac ttgttggttt gatccacaag gtcttcttct ccatagctac tagacatgtt 2100
   ttagcttaag attcaagttt atatatgcct tgtggattaa tcattgcctg attcttccgt 2160
   gtcatctctg agtttattta gagcttggaa gtggtgtagt aataactaac aatactcttg 2220
   ataagttgta gcaatgctct tgattagtgg atgtaatatg atgttgataa gatatatgag 2280
   gcacagaacc aaaagtggtg cttccactag acccgttttt agcctaaggt tcaagtttat 2340
   accttgtaga tgtttctgta ttgtctgatt cttccctgtg atatttgaat ttcttagagc 2400
   tttggaagtg atataggaac aatgctcttg tgtgtttgtc tctatgaaga ttatcgctgt 2460
   cgtgtttcat ccgagtgtgc gggatttttt gctgctgggt ttagcctttc ttcaaaaagt 2520
   tattacttgt tagttttatt gttttggtct tgataagaga tgttaggaca gacatggtgc 2580
   ttcttgtcta tagccactag acctatttta gcataaggtt aacgaaattc tctctacata 2640
   ccttgtggat ttgtttacat tgcctgatct ttcctgtgat cgctgtcatg tttctttgga 2700
   atgattgatg tttataaatg gaaaaatctt tgtgcagaag actcccgccc atccaggatg 2760
   aggatgacca ccaccccaca gtggggcagg atgaggatga ccaggtcgca gcgtgtgcgt 2820
   gtccgtcgta cgttctggcc ggccgggcct tgggcgcgcg atcagaagcg ttgcgttggc 2880
   gtgtgtgtgc ttctggtttg ctttaatttt accaagtttg tttcaaggtg gatcgcgtgg 2940
   tcaaggcccg tgtgctttaa agacccaccg gcactggcag tgagtgttgc tgcttgtgta 3000
   ggctttggta cgtatgggct ttatttgctt ctggatgttg tgtactactt gggtttgttg 3060
   aattattatg agcagttgcg tattgtaatt cagctgggct acctggacat tgttatgtat 3120
   taataaatgc tttgctttct tctaaagatc tttaagtgct acaactttgt atacaaaagt 3180
   tggtttaaac aaccgacaac cactttgcgg acttcctttc aagagaattc aataaggtta 3240
   attcctaatt gaaatccgaa gataagattc ccacacactt gtggctgata tcaaaaggct 3300
   actgcctatt taaacacatc tctggagact gagaaaatca gacctccaag catgaagaag 3360
   cctgagctta ctgctacttc tgttgagaag ttcctcatcg agaagttcga ttctgtgtct 3420
   gatcttatgc agctctctga gggtgaggaa tcaagagctt tctctttcga tgttggtgga 3480
   agaggatacg ttctcagagt taactcttgc gctgacggat tctacaagga tagatacgtg 3540
   tacagacact tcgcttcagc tgctctccct atccctgaag ttcttgatat cggagagttc 3600
   tctgagtctc ttacctactg tatctcaaga agggctcagg gtgttactct tcaagatctt 3660
   cctgagactg agcttcctgc tgttcttcaa cctgttgctg aggctatgga tgctatcgct 3720
   gctgctgatc tttctcaaac ttctggattc ggacctttcg gtcctcaggg aatcggacag 3780
   tacactactt ggagagattt catctgcgct atcgctgatc ctcatgttta ccattggcag 3840
   accgttatgg atgataccgt ttctgcttct gttgctcaag ctcttgatga gcttatgctt 3900
   tgggctgagg attgtcctga ggttagacat cttgttcacg ctgatttcgg atctaacaac 3960
   gttctcaccg ataacggaag aatcaccgct gttatcgatt ggtctgaggc tatgttcgga 4020
   gattctcaat acgaggtggc caacatattc ttttggaggc cttggcttgc ttgtatggaa 4080
   caacagacta gatacttcga gagaaggcat cctgagcttg ctggatctcc tagacttaga 4140
   gcttacatgc ttaggatcgg acttgatcag ctttaccagt ctctcgttga tggaaacttc 4200
   gatgatgctg cttgggctca gggaagatgt gatgctatcg ttagatctgg tgctggaact 4260
   gttggaagaa ctcaaatcgc tagaagatct gctgctgttt ggactgatgg atgtgttgaa 4320
   gttctcgctg attctggaaa cagaaggcct tctactagac ctagagccaa gaagtgaaga 4380
   tcggcggcaa tagcttctta gcgccatccc gggttgatcc tatctgtgtt gaaatagttg 4440
   cggtgggcaa ggctctcttt cagaaagaca ggcggccaaa ggaacccaag gtgaggtggg 4500
   ctatggctct cagttccttg tggaagcgct tggtctaagg tgcagaggtg ttagcgggat 4560
   gaagcaaaag tgtccgattg taacaagata tgttgatcct acgtaaggat attaaagtat 4620
   gtattcatca ctaatataat cagtgtattc caatatgtac tacgatttcc aatgtcttta 4680
   ttgtcgccgt atgtaatcgg cgtcacaaaa taatccccgg tgactttctt ttaatccagg 4740
   atgaaataat atgttattat aatttttgcg atttggtccg ttataggaat tgaagtgtgc 4800
   ttgcggtcgc caccactccc atttcataat tttacatgta tttgaaaaat aaaaatttat 4860
   ggtattcaat ttaaacacgt atacttgtaa agaatgatat cttgaaagaa atatagttta 4920
   aatatttatt gataaaataa caagtcaggt attatagtcc aagcaaaaac ataaatttat 4980
   tgatgcaagt ttaaattcag aaatatttca ataactgatt atatcagctg gtacattgcc 5040
   gtagatgaaa gactgagtgc gatattatgg tgtaatacat agtttaaacc acccaacttt 5100
   tctatacaaa gttgaagact cccgcccatc caggatgagg atgaccacca ccccacagtg 5160
   gggcaggatg aggatgacca gtcagtttta cttcccttaa ttttctatgt actttcataa 5220
   ttacttatgt tattttcttc atgagtttta atgcaaatta ctatatggac tctagtgaaa 5280
   acgttcagaa tcctataaac atgactactg agacgaactt gagagtagtt ttgatcatac 5340
   acacgtttca tgtggtactt gagagttact aatttttgtc atcttcgtat aagtagtaaa 5400
   agatactaca agaatagttt agtagaaaat actagcggta ggtgaagatt tgtcgctatg 5460
   tactattatt gtctagtaac ttgagtaaca atttcgtggt ctaaatatca aataaaaatg 5520
   gatgagtggt tcaccaaatc taggcatcaa aactattaat gtcattgtct agatcttagg 5580
   tgacaccaca tttcgaatat ttattggtaa ttgagatgtt aaagtaccaa tatttgactt 5640
   aataaactaa aagattttgg ctttatcaaa tgtagacatt gatgacatat cgttgtcatt 5700
   atcttgagta tatacaagtc gatcaattag gtgaaagttt agtgtctcgt ggttggtaaa 5760
   cgattaatac agtagtatat tttatccaaa gacaaaatcc aaatcatttc accagtatga 5820
   atagtattat tttatcttaa aagctaaaat cttaaaaacc aaggtagcac ccacgttgag 5880
   ctagacgatc aaatcgattt ctgctttgtc caatttacca agctatttaa agccaaataa 5940
   ttgaaatata ggtaggtcgt tatattaggc taagatttat ctcaaatgct taactaaagg 6000
   aataacaagg gattctagtt gtgtggtttt ataagattgg tccaatttca cttaagtttg 6060
   tttattgtag aattttatat gtgaataatt tgaattccaa ttgaaaagat attatagtaa 6120
   aagaaaaaat agtgcgaaca aaaaacttta atcccataaa aagaaaaaga aaaatgaaaa 6180
   gttcttctaa catccatatt ttgcatcata tcataaagat aagaaagata catatcatag 6240
   acgtacagat aaacaaacat atcatcattt gtgaaataca tagtacaata atttgctttt 6300
   aaatagagtt taagtcacac acactgacac acacgataaa acgataatgt ctgcaaaaac 6360
   actttaatcc cattgcctag aggacagctt ctccactttg tctttaaggt tggttttgcc 6420
   gtgttgtttt tatctttata taatgatcta ttttttggat tatgaaatga attcacacat 6480
   tttaattatt taagaagatc catatacagg tttataacag tactaagtga tgattatttt 6540
   ttgtttttgc atagtttagt ttattgggta aacattcatt acgtgtctct ttatacgaat 6600
   cacccatcca aaatttcaag tagtctttta gttcatttat tatttcataa ctatttgact 6660
   tattgatttg acaagaaaca acaaaagtgt tgacttattg atagattgtg ggatcataaa 6720
   agtaattaag cgtcaaccac gacccacaac aacaaagcac atgttataca ttaatatctc 6780
   gtttacttaa ttacagtttt cagaatgccg tttcatgtct tgtcactggc gatgttatta 6840
   tcatgttgga caatattcga ctgttgtcgt ttttacattt tcgtattgac taaaactaaa 6900
   aaaacaaaac tctgtttcag gttgggccta ggatccacat tgtacacaca tttgcttaag 6960
   tctatggagg cgcaaggttt taagtctgtg gttgctgtta taggccttcc aaacgatcca 7020
   tctgttaggt tgcatgaggc tttgggatac acagcccggg gtacattgcg cgcagctgga 7080
   tacaagcatg gtggatggca tgatgttggt ttttggcaaa gggattttga gttgccagct 7140
   cctccaaggc cagttaggcc agttacccag atctaatatc aaaatctatt tagaaataca 7200
   caatattttg ttgcaggctt gctggagaat cgatctgcta tcataaaaat tacaaaaaaa 7260
   ttttatttgc ctcaattatt ttaggattgg tattaaggac gcttaaatta tttgtcgggt 7320
   cactacgcat cattgtgatt gagaagatca gcgatacgaa atattcgtag tactatcgat 7380
   aatttatttg aaaattcata agaaaagcaa acgttacatg aattgatgaa acaatacaaa 7440
   gacagataaa gccacgcaca tttaggatat tggccgagat tactgaatat tgagtaagat 7500
   cacggaattt ctgacaggag catgtcttca attcagccca aatggcagtt gaaatactca 7560
   aaccgcccca tatgcaggag cggatcattc attgtttgtt tggttgcctt tgccaacatg 7620
   ggagtccaag gttatttaaa taccctgcca agcttgaggt agcctccaat ttgacggtgc 7680
   cgccagcgac gccgtctgga actgtccttt ttgaggacca ctccgtttgt ggagatcatg 7740
   aacaactttg tataataaag ttgaagactc ccgcccatct ctctatgccc gggacaagtg 7800
   gagtccatgc tcaacaccgt gcactaggga caggattggt ttaaacgttt gtgtcttcta 7860
   gattaatcct ccaaactttt gattaaccaa aaaaattatc aaactaacat gttctccttt 7920
   tttctttaga aattctaacg aatttatctt tatactgatt tgaatatact taatttggtc 7980
   atttggatgc cctttacaac ctccttacca aactattgat cacagtttct attgctaaaa 8040
   tcaccaacaa aacgcatgtc gccattcata attatggttt cacacctaca actaggctaa 8100
   taagtaaata agtagacaac tagactcagg tttgaaaaaa ccataaaagc catatagcgt 8160
   tttctcattg aaactgcgaa cacgatcgtg tgaatgttgc agtttctagt tttgatacaa 8220
   acaaacaaaa acacaattta atcttagatt aaaaagaaaa aagagaacgg agcccactag 8280
   ccactccttc aaacgtgtct taccaactct cttctagaaa caaattaggc ttcaccttcc 8340
   tcttccaacc tctctctctc tctctctctc tttttctcaa accatctctc cataaagccc 8400
   taatttcttc atcacaagaa tcagaagaag aaagatggac ctgcatctaa ttttcggtcc 8460
   aacttgcaca ggaaagacga cgaccgcgat agctcttgcc cagcagacag ggcttccagt 8520
   cctttcgctt gatcgggtcc aatgctgtcc tcaactatca accggaagcg gacgaccaac 8580
   agtggaagaa ctgaaaggaa cgacgcgtct ctaccttgat gatcggcctc tggtggaggg 8640
   tatcatcgca gccaagcaag ctcatcatag gctgatcgag gaggtgtata atcatgaggc 8700
   caacggcggg cttattcttg agggaggatc cacctcgttg ctcaactgca tggcgcgaaa 8760
   cagctattgg agtgcagatt ttcgttggca tattattcgc cacaagttac ccgaccaaga 8820
   gaccttcatg aaagcggcca aggccagagt taagcagatg ttgcaccccg ctgcaggcca 8880
   ttctattatt caagagttgg tttatctttg gaatgaacct cggctgaggc ccattctgaa 8940
   agagatcgat ggatatcgat atgccatgtt gtttgctagc cagaaccaga tcacggcaga 9000
   tatgctattg cagcttgacg caaatatgga aggtaagttg attaatggga tcgctcagga 9060
   gtatttcatc catgcgcgcc aacaggaaca gaaattcccc caagttaacg cagccgcttt 9120
   cgacggattc gaaggtcatc cgttcggaat gtattagaaa tcaccagtct ctctctacaa 9180
   atctatctct ctctattttt ctccagaata atgtgtgagt agttcccaga taagggaatt 9240
   agggttctta tagggtttcg ctcatgtgtt gagcatataa gaaaccctta gtatgtattt 9300
   gtatttgtaa aatacttcta tcaataaaat ttctaattcc taaaaccaaa atccagtgtt 9360
   taaacaagac tcccgcccat ctctctatgc ccgggacaag tggagtccat gctcaacacc 9420
   gtgcactagg gacaggattg cattaaggat gaccagttcg taaaggtcct gcggtgtcta 9480
   ttgcttttca taggttaata agtgtttgct agactgtggt gaaaggccta tccgaagtaa 9540
   ggccggccgg atccttcatc tttggacaag ggaataaaga ctccccactt gctactaaga 9600
   acaataccta agttgcccag acatgactgt acccattcag agacctacca cccattaggg 9660
   ctatgacact aacactagcc cctggaggtt gaccatgcta ggcagtgggg gtctcaccta 9720
   tgacccactc agataggggt ttaaaccagt gggtgggatc tcagcctcat ataggtgttt 9780
   gtggtgagct ttctcctaga caagagaacc ctgaagaaca gcaagaacca gctaatatga 9840
   tatgtagaca tagtgggttg ctcaaatttt gtgtttagtc atattagaat tgacctcagt 9900
   gaccactcag aaagtgccca agcccatcta taggggccaa agtgctattg actggtgtgt 9960
   ctgtgaattg ttcctcccta cagagttggt gctgatatat cctagcattc tttggaaaac 10020
   ctagctaggg actgtcaagt gtaagatacc tcctgaattg gagggaacac tagctgccct 10080
   gtaccttctg gctagtacct tacaccctga atgggttagg gggtctatta tttgctggaa 10140
   atataccagt ttcagtaggg ctgctgcctt aggtcccaca aggtgtaaca tgtgctcaat 10200
   agttgcacta ccacatgcac gtgaacttaa tgatgttata gccacaacac caaccttggt 10260
   ttgcagtttg acatccctct ggaatgggtg tagtcatctt gctctggatc tgcctgaatc 10320
   attggggctg tatgcagcct gggcttaaag tgaagaatgg gatgtcccag aaatattttg 10380
   ggtgagaaga atcctggagt agatggtgac ctgactatcc ctgtcctatg ggcacaatct 10440
   atcatcagat attgcattca aagggctatc atgggatcaa gtcctaagtc aactgttgtt 10500
   tacctggcag acattcatct aggagttctc ttttatgcca ccccacagtg atccgccttt 10560
   tgcagtttat ccactaggga caggattgcc accccacagt ggggcctcta tgcccgggac 10620
   aagtgtaaaa tatagagtat aggggttatc atcacagaga agctattgct ggagggcctc 10680
   tgttatttcc tctccatgcc actcccattt ttaacctacc aactgaaatc ccaagggaga 10740
   ctccaccctg taactagagt cctcagaggt gagccatccc atattaacaa atgggcatta 10800
   gggctaggat gccaagggat acctgaaatg ggaagttgtg gggctgagtc ctcctgggaa 10860
   tcagagataa tatgtaaaca gtttgttgag agattgatga gagctgactt tgagaggtgg 10920
   ccatgctccc tggtcctcaa tagggaaggc actacacaag aaacctgggt ttgatcaact 10980
   gcactgtgtc ctactcacac attgtgtgcc tggaaaaatg ttacttagta tttggagggc 11040
   ctccagaacc cccctgggtg caagactggg tgctagtgac tgggtgaatg agtcttggac 11100
   acagtggcct tgtctaggtt gtgtgaggtg gctaggcatc atggcaatac ctcataattg 11160
   atgagtgagg aaacaagact aagtccttga ctcctcttat tacatgacct ggtggatatt 11220
   atgtttaaac tctgcaagct ggaatgagta ctgggtgcag atcccctggg attctggcta 11280
   caaaggtgaa tgatagctag tctgtttatt agtagccaaa aaagtcagtg aggggtgagt 11340
   gccctgggat gttgttaagt tcacattgca cacttggaga ccctctccat ccagtaacat 11400
   accagagaaa actgaccaag ccctcatggg tgtatgggaa caacaaacct cctggctact 11460
   tcaagggcac ataacaccag caaggagcct gtcataacca ccatctcaaa caatagaact 11520
   tcctaagtga agcaatgact tcaaatctac ttgaaggcat ggagtataag ccatgttcct 11580
   ttcagagggg actgtacttc tgtagattac tttccctcat taaccagatc tggccggcct 11640
   acccagcttt cttgtacaaa gtggtgataa actatcagtg tttgacagga tatattggcg 11700
   ggtaaac 11707
<210> 150
   <211> 18
   <212> DNA
   <213> Artificial Sequence
<220>
   <223> pTiC58 Forward PCR Primer
<400> 150
   cgagaacttg gcaattcc 18
<210> 151
   <211> 18
   <212> DNA
   <213> Artificial Sequence
<220>
   <223> pTiC58 Reverse PCR Primer
<400> 151
   tggcgattct gagattcc 18
<210> 152
   <211> 22
   <212> DNA
   <213> Artificial Sequence
<220>
   <223> Actin Forward PCR Primer
<400> 152
   gactcatcgt actctccctt cg 22
<210> 153
   <211> 22
   <212> DNA
   <213> Artificial Sequence
<220>
   <223> Actin Reverse PCR Primer
<400> 153
   gactcatcgt actctccctt cg 22
<210> 154
   <211> 20
   <212> DNA
   <213> Artificial Sequence
<220>
   <223> HPH Forward PCR Primer
<400> 154
   tgttggtgga agaggatacg 20
<210> 155
   <211> 18
   <212> DNA
   <213> Artificial Sequence
<220>
   <223> HPH Reverse PCR Primer
<400> 155
   atcagcagca gcgatagc 18
<210> 156
   <211> 20
   <212> DNA
   <213> Artificial Sequence
<220>
   <223> 1F PCR Primer
<400> 156
   atgtccactg ggttcgtgcc 20
<210> 157
   <211> 20
   <212> DNA
   <213> Artificial Sequence
<220>
   <223> 1R PCR Primer
<400> 157
   gaagggaact tatccggtcc 20
<210> 158
   <211> 20
   <212> DNA
   <213> Artificial Sequence
<220>
   <223> 2F PCR Primer
<400> 158
   tgcgctgcca ttctccaaat 20
<210> 159
   <211> 20
   <212> DNA
   <213> Artificial Sequence
<220>
   <223> 2R PCR Primer
<400> 159
   accgagctcg aattcaattc 20
<210> 160
   <211> 20
   <212> DNA
   <213> Artificial Sequence
<220>
   <223> 3F PCR Primer
<400> 160
   cctgcattcg gttaaacacc 20
<210> 161
   <211> 20
   <212> DNA
   <213> Artificial Sequence
<220>
   <223> 3R PCR Primer
<400> 161
   ccatctggct tctgccttgc 20
<210> 162
   <211> 20
   <212> DNA
   <213> Artificial Sequence
<220>
   <223> 4F PCR Primer
<400> 162
   attccgatcc ccagggcagt 20
<210> 163
   <211> 20
   <212> DNA
   <213> Artificial Sequence
<220>
   <223> 4R PCR Primer
<400> 163
   gccaacgttg cagccttgct 20
<210> 164
   <211> 20
   <212> DNA
   <213> Artificial Sequence
<220>
   <223> 5F PCR Primer
<400> 164
   gccctgggat gttgttaagt 20
<210> 165
   <211> 20
   <212> DNA
   <213> Artificial Sequence
<220>
   <223> 5R PCR Primer
<400> 165
   gtaacttagg acttgtgcga 20
<210> 166
   <211> 19
   <212> DNA
   <213> Artificial Sequence
<220>
   <223> IPT-F PCR Primer
<400> 166
   tctctacctt gatgatcgg 19
<210> 167
   <211> 20
   <212> DNA
   <213> Artificial Sequence
<220>
   <223> IPT-R PCR Primer
<400> 167
   aacatctgct taactctggc 20
<210> 168
   <211> 19
   <212> DNA
   <213> Artificial Sequence
<220>
   <223> dsRED-F PCR Primer
<400> 168
   atggcttcat ctgagaacg 19
<210> 169
   <211> 19
   <212> DNA
   <213> Artificial Sequence
<220>
   <223> dsRED-R PCR Primer
<400> 169
   ttccgtattg gaattgagg 19
<210> 170
   <211> 21
   <212> DNA
   <213> Artificial Sequence
<220>
   <223> PAT-F PCR Primer
<400> 170
   ttgcttaagt ctatggaggc g 21
<210> 171
   <211> 20
   <212> DNA
   <213> Artificial Sequence
<220>
   <223> PAT-R PCR Primer
<400> 171
   tgggtaactg gcctaactgg 20
<210> 172
   <211> 21
   <212> DNA
   <213> Artificial Sequence
<220>
   <223> ELP-F PCR Primer
<400> 172
   atgatatgta gacatagtgg g 21
<210> 173
   <211> 19
   <212> DNA
   <213> Artificial Sequence
<220>
   <223> ELP-R PCR Primer
<400> 173
   agggtgtaag gtactagcc 19
<210> 174
   <211> 20
   <212> DNA
   <213> Artificial Sequence
<220>
   <223> Hph-F PCR Primer
<400> 174
   tgttggtgga agaggatacg 20
<210> 175
   <211> 18
   <212> DNA
   <213> Artificial Sequence
<220>
   <223> Hph-R PCR Primer
<400> 175
   atcagcagca gcgatagc 18
<210> 176
   <211> 24
   <212> DNA
   <213> Artificial Sequence
<220>
   <223> actin-F PCR Primer
<400> 176
   gtggagaaga actacgagct accc 24
<210> 177
   <211> 22
   <212> DNA
   <213> Artificial Sequence
<220>
   <223> actin-R PCR Primer
<400> 177
   gactcatcgt actctccctt cg 22
<210> 178
   <211> 7
   <212> PRT
   <213> Artificial Sequence
<220>
   <223> 27961 Finger 1
<400> 178
   Arg Ser Asp Asn Leu Ala Arg
   1 5
<210> 179
   <211> 7
   <212> PRT
   <213> Artificial Sequence
<220>
   <223> 27961 Finger 2
<400> 179
   Gln Lys Lys Asp Arg Ser Tyr
   1 5
<210> 180
   <211> 7
   <212> PRT
   <213> Artificial Sequence
<220>
   <223> 27961 Finger 3
<400> 180
   Arg Ser Asp Asn Leu Ala Arg
   1 5
<210> 181
   <211> 7
   <212> PRT
   <213> Artificial Sequence
<220>
   <223> 27961 Finger 4
<400> 181
   Gln Arg Gly Asn Arg Asn Thr
   1 5
<210> 182
   <211> 7
   <212> PRT
   <213> Artificial Sequence
<220>
   <223> 27961 Finger 5
<400> 182
   Arg Ser Asp His Leu Ser Arg
   1 5
<210> 183
   <211> 7
   <212> PRT
   <213> Artificial Sequence
<220>
   <223> 27961 Finger 6
<400> 183
   Arg Asn Gln Asp Arg Thr Asn
   1 5
<210> 184
   <211> 7
   <212> PRT
   <213> Artificial Sequence
<220>
   <223> 27962 Finger 1
<400> 184
   Asp Arg Ser Asn Leu Ser Arg
   1 5
<210> 185
   <211> 7
   <212> PRT
   <213> Artificial Sequence
<220>
   <223> 27962 Finger 2
<400> 185
   Arg Gln Asp Ser Arg Ser Gln
   1 5
<210> 186
   <211> 7
   <212> PRT
   <213> Artificial Sequence
<220>
   <223> 27962 Finger 3
<400> 186
   Gln Ser Ser Asp Leu Ser Arg
   1 5
<210> 187
   <211> 7
   <212> PRT
   <213> Artificial Sequence
<220>
   <223> 27962 Finger 4
<400> 187
   Asp Arg Ser Ala Leu Ala Arg
   1 5
<210> 188
   <211> 7
   <212> PRT
   <213> Artificial Sequence
<220>
   <223> 27962 Finger 5
<400> 188
   Thr Ser Gly Ser Leu Thr Arg
   1 5
<210> 189
   <211> 7
   <212> PRT
   <213> Artificial Sequence
<220>
   <223> 27973 Finger 1
<400> 189
   Gln Ser Ser Asp Leu Ser Arg
   1 5
<210> 190
   <211> 7
   <212> PRT
   <213> Artificial Sequence
<220>
   <223> 27973 Finger 2
<400> 190
   Ala Ala Ser Asn Arg Ser Lys
   1 5
<210> 191
   <211> 7
   <212> PRT
   <213> Artificial Sequence
<220>
   <223> 27973 Finger 3
<400> 191
   Thr Ser Gly Ser Leu Ser Arg
   1 5
<210> 192
   <211> 7
   <212> PRT
   <213> Artificial Sequence
<220>
   <223> 27973 Finger 4
<400> 192
   Arg Ser Asp Ala Leu Ala Arg
   1 5
<210> 193
   <211> 7
   <212> PRT
   <213> Artificial Sequence
<220>
   <223> 27973 Finger 5
<400> 193
   Arg Ser Asp Val Leu Ser Thr
   1 5
<210> 194
   <211> 7
   <212> PRT
   <213> Artificial Sequence
<220>
   <223> 27973 Finger 6
<400> 194
   Trp Gly Arg Leu Arg Lys Leu
   1 5
<210> 195
   <211> 7
   <212> PRT
   <213> Artificial Sequence
<220>
   <223> 27974 Finger 1
<400> 195
   Glu Arg Gly Thr Leu Ala Arg
   1 5
<210> 196
   <211> 7
   <212> PRT
   <213> Artificial Sequence
<220>
   <223> 27974 Finger 2
<400> 196
   Arg Ser Asp Asp Leu Thr Arg
   1 5
<210> 197
   <211> 7
   <212> PRT
   <213> Artificial Sequence
<220>
   <223> 27974 Finger 3
<400> 197
   Arg Ser Asp His Leu Ser Ala
   1 5
<210> 198
   <211> 7
   <212> PRT
   <213> Artificial Sequence
<220>
   <223> 27974 Finger 4
<400> 198
   Gln His Gly Ala Leu Gln Thr
   1 5
<210> 199
   <211> 7
   <212> PRT
   <213> Artificial Sequence
<220>
   <223> 27974 Finger 5
<400> 199
   Thr Ser Gly Asn Leu Thr Arg
   1 5
<210> 200
   <211> 7
   <212> PRT
   <213> Artificial Sequence
<220>
   <223> 27974 Finger 6
<400> 200
   Gln Ser Gly His Leu Ser Arg
   1 5
<210> 201
   <211> 7
   <212> PRT
   <213> Artificial Sequence
<220>
   <223> 27987 Finger 1
<400> 201
   Thr Ser Gly Ser Leu Thr Arg
   1 5
<210> 202
   <211> 7
   <212> PRT
   <213> Artificial Sequence
<220>
   <223> 27987 Finger 2
<400> 202
   Arg Ser Asp His Leu Ser Gln
   1 5
<210> 203
   <211> 7
   <212> PRT
   <213> Artificial Sequence
<220>
   <223> 27987 Finger 3
<400> 203
   Cys Thr Arg Asn Arg Trp Arg
   1 5
<210> 204
   <211> 7
   <212> PRT
   <213> Artificial Sequence
<220>
   <223> 27987 Finger 4
<400> 204
   Arg Ser Asp Asn Leu Ser Glu
   1 5
<210> 205
   <211> 7
   <212> PRT
   <213> Artificial Sequence
<220>
   <223> 27987 Finger 5
<400> 205
   Ala Ser Lys Thr Arg Lys Asn
   1 5
<210> 206
   <211> 7
   <212> PRT
   <213> Artificial Sequence
<220>
   <223> 27990 Finger 1
<400> 206
   Thr Ser Gly Ser Leu Ser Arg
   1 5
<210> 207
   <211> 7
   <212> PRT
   <213> Artificial Sequence
<220>
   <223> 27990 Finger 2
<400> 207
   Thr Ser Ser Asn Arg Ala Val
   1 5
<210> 208
   <211> 7
   <212> PRT
   <213> Artificial Sequence
<220>
   <223> 27990 Finger 3
<400> 208
   Thr Ser Gly Asn Leu Thr Arg
   1 5
<210> 209
   <211> 7
   <212> PRT
   <213> Artificial Sequence
<220>
   <223> 27990 Finger 4
<400> 209
   Asp Arg Ser Ala Leu Ala Arg
   1 5
<210> 210
   <211> 7
   <212> PRT
   <213> Artificial Sequence
<220>
   <223> 27990 Finger 5
<400> 210
   Arg Ser Asp Val Leu Ser Glu
   1 5
<210> 211
   <211> 7
   <212> PRT
   <213> Artificial Sequence
<220>
   <223> 27990 Finger 6
<400> 211
   Arg Asn Phe Ser Leu Thr Met
   1 5
<210> 212
   <211> 7
   <212> PRT
   <213> Artificial Sequence
<220>
   <223> 27991 Finger 1
<400> 212
   Gln Ser Gly Asp Leu Thr Arg
   1 5
<210> 213
   <211> 7
   <212> PRT
   <213> Artificial Sequence
<220>
   <223> 27991 Finger 2
<400> 213
   Thr Ser Gly Ser Leu Ser Arg
   1 5
<210> 214
   <211> 7
   <212> PRT
   <213> Artificial Sequence
<220>
   <223> 27991 Finger 3
<400> 214
   Gln Ser Gly Asn Leu Ala Arg
   1 5
<210> 215
   <211> 7
   <212> PRT
   <213> Artificial Sequence
<220>
   <223> 27991 Finger 4
<400> 215
   Thr Ser Gly Ser Leu Ser Arg
   1 5
<210> 216
   <211> 7
   <212> PRT
   <213> Artificial Sequence
<220>
   <223> 27991 Finger 5
<400> 216
   Gln Ser Gly Ser Leu Thr Arg
   1 5
<210> 217
   <211> 7
   <212> PRT
   <213> Artificial Sequence
<220>
   <223> 27992 Finger 1
<400> 217
   Asp Arg Ser His Leu Ala Arg
   1               5
<210> 218
   <211> 7
   <212> PRT
   <213> Artificial Sequence
<220>
   <223> 27992 Finger 2
<400> 218
   Thr Ser Gly Ser Leu Ser Arg
   1               5
<210> 219
   <211> 7
   <212> PRT
   <213> Artificial Sequence
<220>
   <223> 27992 Finger 3
<400> 219
   Thr Ser Ser Asn Arg Ala Val
   1               5
<210> 220
   <211> 7
   <212> PRT
   <213> Artificial Sequence
<220>
   <223> 27992 Finger 4
<400> 220
   Thr Ser Gly Asn Leu Thr Arg
   1               5
<210> 221
   <211> 7
   <212> PRT
   <213> Artificial Sequence
<220>
   <223> 27992 Finger 5
<400> 221
   Asp Arg Ser Ala Leu Ala Arg
   1 5
<210> 222
   <211> 7
   <212> PRT
   <213> Artificial Sequence
<220>
   <223> 28004 Finger 1
<400> 222
   Gln Ser Gly Asn Leu Ala Arg
   1 5
<210> 223
   <211> 7
   <212> PRT
   <213> Artificial Sequence
<220>
   <223> 28004 Finger 2
<400> 223
   His Leu Gly Asn Leu Lys Thr
   1 5
<210> 224
   <211> 7
   <212> PRT
   <213> Artificial Sequence
<220>
   <223> 28004 Finger 3
<400> 224
   Arg Ser Asp His Leu Ser Gln
   1 5
<210> 225
   <211> 7
   <212> PRT
   <213> Artificial Sequence
<220>
   <223> 28004 Finger 4
<400> 225
   Thr Ala Arg Leu Leu Lys Leu
   1 5
<210> 226
   <211> 7
   <212> PRT
   <213> Artificial Sequence
<220>
   <223> 28004 Finger5
<400> 226
   Gln Ser Gly Asn Leu Ala Arg
   1 5
<210> 227
   <211> 7
   <212> PRT
   <213> Artificial Sequence
<220>
   <223> 28004 Finger 6
<400> 227
   Gln Thr Ser His Leu Pro Gln
   1 5
<210> 228
   <211> 7
   <212> PRT
   <213> Artificial Sequence
<220>
   <223> 28005 Finger 1
<400> 228
   Arg Ser Asp Asn Leu Ser Val
   1 5
<210> 229
   <211> 7
   <212> PRT
   <213> Artificial Sequence
<220>
   <223> 28005 Finger 2
<400> 229
   Thr Ser Gly His Leu Ser Arg
   1 5
<210> 230
   <211> 7
   <212> PRT
   <213> Artificial Sequence
<220>
   <223> 28005 Finger 3
<400> 230
   Thr Ser Gly Ser Leu Thr Arg
   1 5
<210> 231
   <211> 7
   <212> PRT
   <213> Artificial Sequence
<220>
   <223> 28005 Finger 4
<400> 231
   Arg Ser Asp Ala Leu Ser Thr
   1 5
<210> 232
   <211> 7
   <212> PRT
   <213> Artificial Sequence
<220>
   <223> 28005 Finger 5
<400> 232
   Asp Arg Ser Thr Arg Thr Lys
   1 5
<210> 233
   <211> 7
   <212> PRT
   <213> Artificial Sequence
<220>
   <223> 28021 Finger 1
<400> 233
   Gln Asn Ala His Arg Lys Thr
   1 5
<210> 234
   <211> 7
   <212> PRT
   <213> Artificial Sequence
<220>
   <223> 28021 Finger 2
<400> 234
   Thr Ser Gly Asn Leu Thr Arg
   1 5
<210> 235
   <211> 7
   <212> PRT
   <213> Artificial Sequence
<220>
   <223> 28021 Finger 3
<400> 235
   Leu Lys Gln Met Leu Ala Val
   1 5
<210> 236
   <211> 7
   <212> PRT
   <213> Artificial Sequence
<220>
   <223> 28021 Finger 4
<400> 236
   Arg Ser Asp Asn Leu Ser Arg
   1 5
<210> 237
   <211> 7
   <212> PRT
   <213> Artificial Sequence
<220>
   <223> 28021 Finger 5
<400> 237
   Asp Asn Ser Asn Arg Lys Thr
   1 5
<210> 238
   <211> 7
   <212> PRT
   <213> Artificial Sequence
<220>
   <223> 28022 Finger 1
<400> 238
   Arg Ser Asp Asn Leu Ser Val
   1 5
<210> 239
   <211> 7
   <212> PRT
   <213> Artificial Sequence
<220>
   <223> 28022 Finger 2
<400> 239
   Gln Asn Ala Asn Arg Ile Thr
   1 5
<210> 240
   <211> 7
   <212> PRT
   <213> Artificial Sequence
<220>
   <223> 28022 Finger 3
<400> 240
   Thr Ser Gly Ser Leu Ser Arg
   1 5
<210> 241
   <211> 7
   <212> PRT
   <213> Artificial Sequence
<220>
   <223> 28022 Finger 4
<400> 241
   Gln Ser Ser Val Arg Asn Ser
   1 5
<210> 242
   <211> 7
   <212> PRT
   <213> Artificial Sequence
<220>
   <223> 28022 Finger 5
<400> 242
   Asp Arg Ser Ala Leu Ala Arg
   1 5
<210> 243
   <211> 7
   <212> PRT
   <213> Artificial Sequence
<220>
   <223> 28023 Finger 1
<400> 243
   Arg Ser Asp Asn Leu Ser Arg
   1 5
<210> 244
   <211> 7
   <212> PRT
   <213> Artificial Sequence
<220>
   <223> 28023 Finger 2
<400> 244
   Asp Asn Ser Asn Arg Lys Thr
   1               5
<210> 245
   <211> 7
   <212> PRT
   <213> Artificial Sequence
<220>
   <223> 28023 Finger 3
<400> 245
   Asp Arg Ser Asn Leu Thr Arg
   1               5
<210> 246
   <211> 7
   <212> PRT
   <213> Artificial Sequence
<220>
   <223> 28023 Finger 4
<400> 246
   Arg Ser Asp Val Leu Ser Glu
   1               5
<210> 247
   <211> 7
   <212> PRT
   <213> Artificial Sequence
<220>
   <223> 28023 Finger 5
<400> 247
   Thr Arg Asn Gly Leu Lys Tyr
   1               5
<210> 248
   <211> 7
   <212> PRT
   <213> Artificial Sequence
<220>
   <223> 28024 Finger 1
<400> 248
   Arg Ser Asp Ala Leu Ala Arg
   1 5
<210> 249
   <211> 7
   <212> PRT
   <213> Artificial Sequence
<220>
   <223> 28024 Finger 2
<400> 249
   Arg Ser Asp Val Leu Ser Glu
   1 5
<210> 250
   <211> 7
   <212> PRT
   <213> Artificial Sequence
<220>
   <223> 28024 finger 3
<400> 250
   Arg Ser Ser Asp Arg Thr Lys
   1 5
<210> 251
   <211> 7
   <212> PRT
   <213> Artificial Sequence
<220>
   <223> 28024 Finger 4
<400> 251
   Arg Ser Asp Asn Leu Ser Val
   1 5
<210> 252
   <211> 7
   <212> PRT
   <213> Artificial Sequence
<220>
   <223> 28024 Finger 5
<400> 252
   Gln Asn Ala Asn Arg Ile Thr
   1 5
<210> 253
   <211> 7
   <212> PRT
   <213> Artificial Sequence
<220>
   <223> 28025 Finger 1
<400> 253
   Gln Ser Ser Asp Leu Ser Arg
   1 5
<210> 254
   <211> 7
   <212> PRT
   <213> Artificial Sequence
<220>
   <223> 28025 Finger 2
<400> 254
   Gln Ser Thr His Arg Asn Ala
   1 5
<210> 255
   <211> 7
   <212> PRT
   <213> Artificial Sequence
<220>
   <223> 28025 Finger 3
<400> 255
   Arg Ser Asp Asn Leu Ala Arg
   1 5
<210> 256
   <211> 7
   <212> PRT
   <213> Artificial Sequence
<220>
   <223> 28025 Finger 4
<400> 256
   Gln Arg Gly Asn Arg Asn Thr
   1 5
<210> 257
   <211> 7
   <212> PRT
   <213> Artificial Sequence
<220>
   <223> 28025 Finger 5
<400> 257
   Arg Ser Asp His Leu Ser Arg
   1 5
<210> 258
   <211> 7
   <212> PRT
   <213> Artificial Sequence
<220>
   <223> 28025 Finger 6
<400> 258
   Arg Asn Gln Asp Arg Thr Asn
   1 5
<210> 259
   <211> 7
   <212> PRT
   <213> Artificial Sequence
<220>
   <223> 28026 Finger 1
<400> 259
   Asp Arg Ser Asn Leu Ser Arg
   1 5
<210> 260
   <211> 7
   <212> PRT
   <213> Artificial Sequence
<220>
   <223> 28026 Finger 2
<400> 260
   Arg Gln Asp Ser Arg Ser Gln
   1 5
<210> 261
   <211> 7
   <212> PRT
   <213> Artificial Sequence
<220>
   <223> 28026 Finger 3
<400> 261
   Gln Ser Ser Asp Leu Ser Arg
   1 5
<210> 262
   <211> 7
   <212> PRT
   <213> Artificial Sequence
<220>
   <223> 28026 Finger 4
<400> 262
   Asp Arg Ser Ala Leu Ala Arg
   1 5
<210> 263
   <211> 7
   <212> PRT
   <213> Artificial Sequence
<220>
   <223> 28026 Finger 5
<400> 263
   Thr Ser Gly Ser Leu Thr Arg
   1 5
<210> 264
   <211> 7
   <212> PRT
   <213> Artificial Sequence
<220>
   <223> 28035 Finger 1
<400> 264
   Gln Ser Ser Asp Leu Ser Arg
   1 5
<210> 265
   <211> 7
   <212> PRT
   <213> Artificial Sequence
<220>
   <223> 28035 Finger 2
<400> 265
   Ala Ala Ser Asn Arg Ser Lys
   1 5
<210> 266
   <211> 7
   <212> PRT
   <213> Artificial Sequence
<220>
   <223> 28035 Finger 3
<400> 266
   Thr Ser Gly Ser Leu Ser Arg
   1 5
<210> 267
   <211> 7
   <212> PRT
   <213> Artificial Sequence
<220>
   <223> 28035 Finger 4
<400> 267
   Arg Ser Asp Ala Leu Ala Arg
   1 5
<210> 268
   <211> 7
   <212> PRT
   <213> Artificial Sequence
<220>
   <223> 28035 Finger 5
<400> 268
   Arg Ser Asp Thr Leu Ser Gln
   1 5
<210> 269
   <211> 7
   <212> PRT
   <213> Artificial Sequence
<220>
   <223> 28035 Finger 6
<400> 269
   Gln Arg Asp His Arg Ile Lys
   1 5
<210> 270
   <211> 7
   <212> PRT
   <213> Artificial Sequence
<220>
   <223> 28036 Finger 1
<400> 270
   Arg Ser Asp Asp Leu Thr Arg
   1 5
<210> 271
   <211> 7
   <212> PRT
   <213> Artificial Sequence
<220>
   <223> 28036 Finger 2
<400> 271
   Gln Ser Ser Asp Leu Arg Arg
   1 5
<210> 272
   <211> 7
   <212> PRT
   <213> Artificial Sequence
<220>
   <223> 28036 Finger 3
<400> 272
   Arg Ser Asp His Leu Ser Ala
   1 5
<210> 273
   <211> 7
   <212> PRT
   <213> Artificial Sequence
<220>
   <223> 28036 Finger 4
<400> 273
   Gln His Gly Ala Leu Gln Thr
   1 5
<210> 274
   <211> 7
   <212> PRT
   <213> Artificial Sequence
<220>
   <223> 28036 Finger 5
<400> 274
   Thr Ser Gly Asn Leu Thr Arg
   1 5
<210> 275
   <211> 7
   <212> PRT
   <213> Artificial Sequence
<220>
   <223> 28036 Finger 6
<400> 275
   Gln Ser Gly His Leu Ser Arg
   1 5
<210> 276
   <211> 7
   <212> PRT
   <213> Artificial Sequence
<220>
   <223> 28039 Finger 1
<400> 276
   Thr Ser Gly Ser Leu Ser Arg
   1 5
<210> 277
   <211> 7
   <212> PRT
   <213> Artificial Sequence
<220>
   <223> 28039 Finger 2
<400> 277
   Arg Ser Asp Ala Leu Ala Arg
   1 5
<210> 278
   <211> 7
   <212> PRT
   <213> Artificial Sequence
<220>
   <223> 28039 Finger 3
<400> 278
   Arg Ser Asp Thr Leu Ser Gln
   1 5
<210> 279
   <211> 7
   <212> PRT
   <213> Artificial Sequence
<220>
   <223> 28039 Finger 4
<400> 279
   Gln Arg Asp His Arg Ile Lys
   1 5
<210> 280
   <211> 7
   <212> PRT
   <213> Artificial Sequence
<220>
   <223> 28039 Finger 5
<400> 280
   Thr Ser Gly Asn Leu Thr Arg
   1 5
<210> 281
   <211> 7
   <212> PRT
   <213> Artificial Sequence
<220>
   <223> 28039 FInger 6
<400> 281
   Asp Arg Gly Asp Leu Arg Lys
   1 5
<210> 282
   <211> 7
   <212> PRT
   <213> Artificial Sequence
<220>
   <223> 28040 Finger 1
<400> 282
   Asp Ser Ser Asp Arg Lys Lys
   1 5
<210> 283
   <211> 7
   <212> PRT
   <213> Artificial Sequence
<220>
   <223> 28040 Finger 2
<400> 283
   Thr Ser Gly Asn Leu Thr Arg
   1 5
<210> 284
   <211> 7
   <212> PRT
   <213> Artificial Sequence
<220>
   <223> 28040 Finger 3
<400> 284
   Asp Asn Tyr Asn Arg Ala Lys
   1               5
<210> 285
   <211> 7
   <212> PRT
   <213> Artificial Sequence
<220>
   <223> 28040 Finger 4
<400> 285
   Asp Arg Ser His Leu Thr Arg
   1               5
<210> 286
   <211> 7
   <212> PRT
   <213> Artificial Sequence
<220>
   <223> 28040 Finger 5
<400> 286
   Arg Ser Asp Asn Leu Thr Thr
   1               5
<210> 287
   <211> 7
   <212> PRT
   <213> Artificial Sequence
<220>
   <223> 28051 Finger 1
<400> 287
   Arg Ser Asp Asn Leu Ser Asn
   1               5
<210> 288
   <211> 7
   <212> PRT
   <213> Artificial Sequence
<220>
   <223> 28051 Finger 2
<400> 288
   Thr Ser Ser Ser Arg Ile Asn
   1               5
<210> 289
   <211> 7
   <212> PRT
   <213> Artificial Sequence
<220>
   <223> 28051 Finger 3
<400> 289
   Arg Ser Asp Asn Leu Ser Glu
   1               5
<210> 290
   <211> 7
   <212> PRT
   <213> Artificial Sequence
<220>
   <223> 28051 Finger 4
<400> 290
   Ala Ser Lys Thr Arg Lys Asn
   1               5
<210> 291
   <211> 7
   <212> PRT
   <213> Artificial Sequence
<220>
   <223> 28051 Finger 5
<400> 291
   Arg Ser Asp Ala Leu Thr Gln
   1               5
<210> 292
   <211> 7
   <212> PRT
   <213> Artificial Sequence
<220>
   <223> 28052 Finger 1
<400> 292
   Arg Ser Asp Thr Leu Ser Thr
   1               5
<210> 293
   <211> 7
   <212> PRT
   <213> Artificial Sequence
<220>
   <223> 28052 Finger 2
<400> 293
   Asp Arg Ser Ser Arg Ile Lys
   1 5
<210> 294
   <211> 7
   <212> PRT
   <213> Artificial Sequence
<220>
   <223> 28052 Finger 3
<400> 294
   Arg Ser Asp Asp Leu Ser Lys
   1 5
<210> 295
   <211> 7
   <212> PRT
   <213> Artificial Sequence
<220>
   <223> 28052 Finger 4
<400> 295
   Asp Asn Ser Asn Arg Ile Lys
   1 5
<210> 296
   <211> 7
   <212> PRT
   <213> Artificial Sequence
<220>
   <223> 28053 Finger 1
<400> 296
   Gln Ser Ser Asp Leu Ser Arg
   1 5
<210> 297
   <211> 7
   <212> PRT
   <213> Artificial Sequence
<220>
   <223> 28053 Finger 2
<400> 297
   Gln Ala Gly Asn Leu Ser Lys
   1 5
<210> 298
   <211> 7
   <212> PRT
   <213> Artificial Sequence
<220>
   <223> 28053 Finger 3
<400> 298
   Gln Ser Gly Asp Leu Thr Arg
   1 5
<210> 299
   <211> 7
   <212> PRT
   <213> Artificial Sequence
<220>
   <223> 28053 Finger 4
<400> 299
   Thr Ser Gly Ser Leu Ser Arg
   1 5
<210> 300
   <211> 7
   <212> PRT
   <213> Artificial Sequence
<220>
   <223> 28053 Finger 5
<400> 300
   Gln Ser Gly Asn Leu Ala Arg
   1 5
<210> 301
   <211> 7
   <212> PRT
   <213> Artificial Sequence
<220>
   <223> 28054 Finger 1
<400> 301
   Thr Ser Gly Ser Leu Ser Arg
   1 5
<210> 302
   <211> 7
   <212> PRT
   <213> Artificial Sequence
<220>
   <223> 28054 Finger 2
<400> 302
   Leu Arg Gln Thr Leu Arg Asp
   1 5
<210> 303
   <211> 7
   <212> PRT
   <213> Artificial Sequence
<220>
   <223> 28054 Finger 3
<400> 303
   Thr Ser Gly Asn Leu Thr Arg
   1 5
<210> 304
   <211> 7
   <212> PRT
   <213> Artificial Sequence
<220>
   <223> 28054 Finger 4
<400> 304
   Asp Arg Ser Ala Leu Ala Arg
   1 5
<210> 305
   <211> 7
   <212> PRT
   <213> Artificial Sequence
<220>
   <223> 28054 Finger 5
<400> 305
   Arg Ser Asp Val Leu Ser Glu
   1 5
<210> 306
   <211> 7
   <212> PRT
   <213> Artificial Sequence
<220>
   <223> 28054 Finger 6
<400> 306
   Arg Asn Phe Ser Leu Thr Met
   1 5
<210> 307
   <211> 7
   <212> PRT
   <213> Artificial Sequence
<220>
   <223> 28055 Finger 1
<400> 307
   Gln Ser Gly Asp Leu Thr Arg
   1 5
<210> 308
   <211> 7
   <212> PRT
   <213> Artificial Sequence
<220>
   <223> 28055 Finger 2
<400> 308
   Thr Ser Gly Ser Leu Ser Arg
   1 5
<210> 309
   <211> 7
   <212> PRT
   <213> Artificial Sequence
<220>
   <223> 28055 Finger 3
<400> 309
   Gln Ser Gly Asn Leu Ala Arg
   1 5
<210> 310
   <211> 7
   <212> PRT
   <213> Artificial Sequence
<220>
   <223> 28055 Finger 4
<400> 310
   Thr Ser Gly Ser Leu Ser Arg
   1 5
<210> 311
   <211> 7
   <212> PRT
   <213> Artificial Sequence
<220>
   <223> 28055 Finger 5
<400> 311
   Gln Ser Gly Ser Leu Thr Arg
   1 5
<210> 312
   <211> 7
   <212> PRT
   <213> Artificial Sequence
<220>
   <223> 28056 Finger 1
<400> 312
   Asp Arg Ser His Leu Ala Arg
   1 5
<210> 313
   <211> 7
   <212> PRT
   <213> Artificial Sequence
<220>
   <223> 28056 Finger 2
<400> 313
   Thr Ser Gly Ser Leu Ser Arg
   1 5
<210> 314
   <211> 7
   <212> PRT
   <213> Artificial Sequence
<220>
   <223> 28056 Finger 3
<400> 314
   Leu Arg Gln Thr Leu Arg Asp
   1 5
<210> 315
   <211> 7
   <212> PRT
   <213> Artificial Sequence
<220>
   <223> 28056 Finger 4
<400> 315
   Thr Ser Gly Asn Leu Thr Arg
   1 5
<210> 316
   <211> 7
   <212> PRT
   <213> Artificial Sequence
<220>
   <223> 28056 Finger 5
<400> 316
   Asp Arg Ser Ala Leu Ala Arg
   1 5
<210> 317
   <211> 7
   <212> PRT
   <213> Artificial Sequence
<220>
   <223> 24800 Finger 1
<400> 317
   Arg Ser Asp Asn Leu Ser Thr
   1 5
<210> 318
   <211> 7
   <212> PRT
   <213> Artificial Sequence
<220>
   <223> 24800 Finger 2
<400> 318
   His Ser His Ala Arg Ile Lys
   1 5
<210> 319
   <211> 7
   <212> PRT
   <213> Artificial Sequence
<220>
   <223> 24800 Finger 3
<400> 319
   His Arg Ser Ser Leu Arg Arg
   1 5
<210> 320
   <211> 7
   <212> PRT
   <213> Artificial Sequence
<220>
   <223> 24800 Finger 4
<400> 320
   Arg Ser Asp His Leu Ser Glu
   1 5
<210> 321
   <211> 7
   <212> PRT
   <213> Artificial Sequence
<220>
   <223> 24800 Finger 5
<400> 321
   Gln Asn Ala Asn Arg Ile Thr
   1 5
<210> 322
   <211> 7
   <212> PRT
   <213> Artificial Sequence
<220>
   <223> 24801 Finger 1
<400> 322
   Asp Arg Ser Asn Leu Ser Arg
   1 5
<210> 323
   <211> 7
   <212> PRT
   <213> Artificial Sequence
<220>
   <223> 24801 Finger 2
<400> 323
   His Arg Ser Ser Leu Arg Arg
   1 5
<210> 324
   <211> 7
   <212> PRT
   <213> Artificial Sequence
<220>
   <223> 24801 Finger 3
<400> 324
   Thr Ser Gly Asn Leu Thr Arg
   1 5
<210> 325
   <211> 7
   <212> PRT
   <213> Artificial Sequence
<220>
   <223> 24801 Finger 4
<400> 325
   Met Ser His His Leu Arg Asp
   1 5
<210> 326
   <211> 7
   <212> PRT
   <213> Artificial Sequence
<220>
   <223> 24801 Finger 5
<400> 326
   Asp Gln Ser Asn Leu Arg Ala
   1 5
<210> 327
   <211> 7
   <212> PRT
   <213> Artificial Sequence
<220>
   <223> 24794 finger 1
<400> 327
   Gln Ser Gly Asn Leu Ala Arg
   1 5
<210> 328
   <211> 7
   <212> PRT
   <213> Artificial Sequence
<220>
   <223> 24794 Finger 2
<400> 328
   Arg Ser Asp Asn Leu Ser Arg
   1 5
<210> 329
   <211> 7
   <212> PRT
   <213> Artificial Sequence
<220>
   <223> 24794 Finger 3
<400> 329
   Asp Asn Asn Ala Arg Ile Asn
   1 5
<210> 330
   <211> 7
   <212> PRT
   <213> Artificial Sequence
<220>
   <223> 24794 Finger 4
<400> 330
   Asp Arg Ser Asn Leu Ser Arg
   1 5
<210> 331
   <211> 7
   <212> PRT
   <213> Artificial Sequence
<220>
   <223> 24794 Finger 5
<400> 331
   Arg Ser Asp His Leu Thr Gln
   1 5
<210> 332
   <211> 7
   <212> PRT
   <213> Artificial Sequence
<220>
   <223> 24795 Finger 1
<400> 332
   Arg Ser Asp Asn Leu Arg Glu
   1 5
<210> 333
   <211> 7
   <212> PRT
   <213> Artificial Sequence
<220>
   <223> 24795 Finger 2
<400> 333
   Gln Ser Gly Ala Leu Ala Arg
   1 5
<210> 334
   <211> 7
   <212> PRT
   <213> Artificial Sequence
<220>
   <223> 24795 Finger 3
<400> 334
   Gln Ser Gly Asn Leu Ala Arg
   1 5
<210> 335
   <211> 7
   <212> PRT
   <213> Artificial Sequence
<220>
   <223> 24795 Finger 4
<400> 335
   Arg Ser Asp Val Leu Ser Glu
   1 5
<210> 336
   <211> 7
   <212> PRT
   <213> Artificial Sequence
<220>
   <223> 24795 Finger 5
<400> 336
   Ser Pro Ser Ser Arg Arg Thr
   1 5
<210> 337
   <211> 7
   <212> PRT
   <213> Artificial Sequence
<220>
   <223> 24810 Finger 1
<400> 337
   Arg Ser Asp Ser Leu Ser Arg
   1 5
<210> 338
   <211> 7
   <212> PRT
   <213> Artificial Sequence
<220>
   <223> 24810 Finger 2
<400> 338
   Arg Lys Asp Ala Arg Ile Thr
   1 5
<210> 339
   <211> 7
   <212> PRT
   <213> Artificial Sequence
<220>
   <223> 24810 Finger 3
<400> 339
   Arg Ser Asp His Leu Ser Ala
   1 5
<210> 340
   <211> 7
   <212> PRT
   <213> Artificial Sequence
<220>
   <223> 24810 Finger 4
<400> 340
   Trp Ser Ser Ser Leu Tyr Tyr
   1 5
<210> 341
   <211> 7
   <212> PRT
   <213> Artificial Sequence
<220>
   <223> 24810 Finger 5
<400> 341
   Asn Ser Arg Asn Leu Arg Asn
   1 5
<210> 342
   <211> 7
   <212> PRT
   <213> Artificial Sequence
<220>
   <223> 24811 Finger 1
<400> 342
   Asp Gln Ser Thr Leu Arg Asn
   1 5
<210> 343
   <211> 7
   <212> PRT
   <213> Artificial Sequence
<220>
   <223> 24811 Finger 2
<400> 343
   Asp Arg Ser Asn Leu Ser Arg
   1               5
<210> 344
   <211> 7
   <212> PRT
   <213> Artificial Sequence
<220>
   <223> 24811 Finger 3
<400> 344
   Asp Arg Ser Asn Leu Trp Arg
   1               5
<210> 345
   <211> 7
   <212> PRT
   <213> Artificial Sequence
<220>
   <223> 24811 Finger 4
<400> 345
   Asp Arg Ser Ala Leu Ser Arg
   1               5
<210> 346
   <211> 7
   <212> PRT
   <213> Artificial Sequence
<220>
   <223> 24811 Finger 5
<400> 346
   Arg Ser Asp Ala Leu Ala Arg
   1               5
<210> 347
   <211> 7
   <212> PRT
   <213> Artificial Sequence
<220>
   <223> 24814 Finger 1
<400> 347
   Arg Ser Asp Ala Leu Ser Arg
   1 5
<210> 348
   <211> 7
   <212> PRT
   <213> Artificial Sequence
<220>
   <223> 24814 Finger 2
<400> 348
   Asp Arg Ser Asp Leu Ser Arg
   1 5
<210> 349
   <211> 7
   <212> PRT
   <213> Artificial Sequence
<220>
   <223> 24814 Finger 3
<400> 349
   Arg Ser Asp His Leu Thr Gln
   1 5
<210> 350
   <211> 7
   <212> PRT
   <213> Artificial Sequence
<220>
   <223> 24814 Finger 4
<400> 350
   Gln Ser Gly Ala Leu Ala Arg
   1 5
<210> 351
   <211> 7
   <212> PRT
   <213> Artificial Sequence
<220>
   <223> 24814 Finger 5
<400> 351
   Gln Ser Gly Asn Leu Ala Arg
   1 5
<210> 352
   <211> 7
   <212> PRT
   <213> Artificial Sequence
<220>
   <223> 24815 Finger 1
<400> 352
   Asp Arg Ser Asn Leu Ser Arg
   1 5
<210> 353
   <211> 7
   <212> PRT
   <213> Artificial Sequence
<220>
   <223> 24815 Finger 2
<400> 353
   Asp Ser Ser Ala Arg Asn Thr
   1 5
<210> 354
   <211> 7
   <212> PRT
   <213> Artificial Sequence
<220>
   <223> 24815 Finger 3
<400> 354
   Asp Arg Ser Ser Arg Lys Arg
   1 5
<210> 355
   <211> 7
   <212> PRT
   <213> Artificial Sequence
<220>
   <223> 24815 Finger 4
<400> 355
   Gln Ser Gly Asp Leu Thr Arg
   1 5
<210> 356
   <211> 7
   <212> PRT
   <213> Artificial Sequence
<220>
   <223> 24815 Finger 5
<400> 356
   Leu Ala His His Leu Val Gln
   1 5
<210> 357
   <211> 7
   <212> PRT
   <213> Artificial Sequence
<220>
   <223> 24818 Finger 1
<400> 357
   Arg Ser Asp Asn Leu Ser Thr
   1 5
<210> 358
   <211> 7
   <212> PRT
   <213> Artificial Sequence
<220>
   <223> 24818 Finger 2
<400> 358
   His Ser His Ala Arg Ile Lys
   1 5
<210> 359
   <211> 7
   <212> PRT
   <213> Artificial Sequence
<220>
   <223> 24818 Finger 3
<400> 359
   Thr Ser Gly His Leu Ser Arg
   1 5
<210> 360
   <211> 7
   <212> PRT
   <213> Artificial Sequence
<220>
   <223> 24818 Finger 4
<400> 360
   Arg Ser Asp Asn Leu Ser Val
   1 5
<210> 361
   <211> 7
   <212> PRT
   <213> Artificial Sequence
<220>
   <223> 24818 Finger 5
<400> 361
   Ile Arg Ser Thr Leu Arg Asp
   1 5
<210> 362
   <211> 7
   <212> PRT
   <213> Artificial Sequence
<220>
   <223> 24819 Finger 1
<400> 362
   Thr Ser Gly His Leu Ser Arg
   1 5
<210> 363
   <211> 7
   <212> PRT
   <213> Artificial Sequence
<220>
   <223> 24819 Finger 2
<400> 363
   Asp Arg Ser Asn Leu Ser Arg
   1 5
<210> 364
   <211> 7
   <212> PRT
   <213> Artificial Sequence
<220>
   <223> 24819 Finger 3
<400> 364
   His Arg Ser Ser Leu Arg Arg
   1 5
<210> 365
   <211> 7
   <212> PRT
   <213> Artificial Sequence
<220>
   <223> 24819 Finger 4
<400> 365
   Thr Ser Gly Asn Leu Thr Arg
   1 5
<210> 366
   <211> 7
   <212> PRT
   <213> Artificial Sequence
<220>
   <223> 24819 Finger 5
<400> 366
   Met Ser His His Leu Arg Asp
   1 5
<210> 367
   <211> 7
   <212> PRT
   <213> Artificial Sequence
<220>
   <223> 24796 Finger 1
<400> 367
   Arg Ser Asp Ala Leu Ser Arg
   1 5
<210> 368
   <211> 7
   <212> PRT
   <213> Artificial Sequence
<220>
   <223> 24796 finger 2
<400> 368
   Asp Arg Ser Asp Leu Ser Arg
   1 5
<210> 369
   <211> 7
   <212> PRT
   <213> Artificial Sequence
<220>
   <223> 24796 Finger 3
<400> 369
   Arg Ser Asp His Leu Thr Gln
   1 5
<210> 370
   <211> 7
   <212> PRT
   <213> Artificial Sequence
<220>
   <223> 24796 Finger 4
<400> 370
   Gln Ser Gly Ala Leu Ala Arg
   1 5
<210> 371
   <211> 7
   <212> PRT
   <213> Artificial Sequence
<220>
   <223> 24796 Finger 5
<400> 371
   Gln Ser Gly Asn Leu Ala Arg
   1 5
<210> 372
   <211> 7
   <212> PRT
   <213> Artificial Sequence
<220>
   <223> 24797 Finger 1
<400> 372
   Arg Ser Ala Val Leu Ser Glu
   1 5
<210> 373
   <211> 7
   <212> PRT
   <213> Artificial Sequence
<220>
   <223> 24797 Finger 2
<400> 373
   Thr Asn Ser Asn Arg Ile Thr
   1 5
<210> 374
   <211> 7
   <212> PRT
   <213> Artificial Sequence
<220>
   <223> 24797 Finger 3
<400> 374
   Leu Lys Gln His Leu Asn Glu
   1 5
<210> 375
   <211> 7
   <212> PRT
   <213> Artificial Sequence
<220>
   <223> 24797 Finger 4
<400> 375
   Gln Ser Gly Ala Leu Ala Arg
   1 5
<210> 376
   <211> 7
   <212> PRT
   <213> Artificial Sequence
<220>
   <223> 24797 Finger 5
<400> 376
   Gln Ser Gly Asn Leu Ala Arg
   1 5
<210> 377
   <211> 7
   <212> PRT
   <213> Artificial Sequence
<220>
   <223> 24836 Finger 1
<400> 377
   Asp Arg Ser Asn Leu Ser Arg
   1 5
<210> 378
   <211> 7
   <212> PRT
   <213> Artificial Sequence
<220>
   <223> 24836 Finger 2
<400> 378
   Gln Ser Gly Asp Leu Thr Arg
   1 5
<210> 379
   <211> 7
   <212> PRT
   <213> Artificial Sequence
<220>
   <223> 24836 Finger 3
<400> 379
   Gln Ser Gly Ala Leu Ala Arg
   1               5
<210> 380
   <211> 7
   <212> PRT
   <213> Artificial Sequence
<220>
   <223> 24836 Finger 4
<400> 380
   Asp Arg Ser Asn Leu Ser Arg
   1               5
<210> 381
   <211> 7
   <212> PRT
   <213> Artificial Sequence
<220>
   <223> 24836 Finger 5
<400> 381
   Gln Arg Thr His Leu Thr Gln
   1               5
<210> 382
   <211> 7
   <212> PRT
   <213> Artificial Sequence
<220>
   <223> 24837 Finger 1
<400> 382
   Arg Ser Asp Asn Leu Ser Asn
   1               5
<210> 383
   <211> 7
   <212> PRT
   <213> Artificial Sequence
<220>
   <223> 24837 Finger 2
<400> 383
   Thr Asn Ser Asn Arg Ile Lys
   1 5
<210> 384
   <211> 7
   <212> PRT
   <213> Artificial Sequence
<220>
   <223> 24837 Finger 3
<400> 384
   Gln Ser Ser Asp Leu Ser Arg
   1 5
<210> 385
   <211> 7
   <212> PRT
   <213> Artificial Sequence
<220>
   <223> 24837 Finger 4
<400> 385
   Gln Ser Ser Asp Leu Arg Arg
   1 5
<210> 386
   <211> 7
   <212> PRT
   <213> Artificial Sequence
<220>
   <223> 24837 Finger 5
<400> 386
   Asp Arg Ser Asn Arg Ile Lys
   1 5
<210> 387
   <211> 7
   <212> PRT
   <213> Artificial Sequence
<220>
   <223> 24844 Finger 1
<400> 387
   Arg Ser Ala Asn Leu Ala Arg
   1 5
<210> 388
   <211> 7
   <212> PRT
   <213> Artificial Sequence
<220>
   <223> 24844 Finger 2
<400> 388
   Arg Ser Asp Asn Leu Thr Thr
   1 5
<210> 389
   <211> 7
   <212> PRT
   <213> Artificial Sequence
<220>
   <223> 24844 Finger 3
<400> 389
   Gln Ser Gly Glu Leu Ile Asn
   1 5
<210> 390
   <211> 7
   <212> PRT
   <213> Artificial Sequence
<220>
   <223> 24844 Finger 4
<400> 390
   Arg Ser Ala Asp Leu Ser Arg
   1 5
<210> 391
   <211> 7
   <212> PRT
   <213> Artificial Sequence
<220>
   <223> 24844 Finger 5
<400> 391
   Arg Ser Asp Asn Leu Ser Glu
   1 5
<210> 392
   <211> 7
   <212> PRT
   <213> Artificial Sequence
<220>
   <223> 24844 Finger 6
<400> 392
   Asp Arg Ser His Leu Ala Arg
   1 5
<210> 393
   <211> 7
   <212> PRT
   <213> Artificial Sequence
<220>
   <223> 24845 Finger 1
<400> 393
   Asp Arg Ser His Leu Ala Arg
   1 5
<210> 394
   <211> 7
   <212> PRT
   <213> Artificial Sequence
<220>
   <223> 24845 Finger 2
<400> 394
   Arg Ser Asp Asn Leu Ser Glu
   1 5
<210> 395
   <211> 7
   <212> PRT
   <213> Artificial Sequence
<220>
   <223> 24845 Finger 3
<400> 395
   Ser Lys Gln Tyr Leu Ile Lys
   1 5
<210> 396
   <211> 7
   <212> PRT
   <213> Artificial Sequence
<220>
   <223> 24845 Finger 4
<400> 396
   Glu Arg Gly Thr Leu Ala Arg
   1 5
<210> 397
   <211> 7
   <212> PRT
   <213> Artificial Sequence
<220>
   <223> 24845 Finger 5
<400> 397
   Arg Ser Asp His Leu Thr Thr
   1               5
<210> 398
   <211> 7
   <212> PRT
   <213> Artificial Sequence
<220>
   <223> 24820 Finger 1
<400> 398
   Gln Ser Gly Ala Leu Ala Arg
   1               5
<210> 399
   <211> 7
   <212> PRT
   <213> Artificial Sequence
<220>
   <223> 24820 Finger 2
<400> 399
   Gln Ser Gly Asn Leu Ala Arg
   1               5
<210> 400
   <211> 7
   <212> PRT
   <213> Artificial Sequence
<220>
   <223> 24820 Finger 3
<400> 400
   Asp Arg Ser His Leu Ala Arg
   1               5
<210> 401
   <211> 7
   <212> PRT
   <213> Artificial Sequence
<220>
   <223> 24820 Finger 4
<400> 401
   Asp Arg Ser Asp Leu Ser Arg
   1 5
<210> 402
   <211> 7
   <212> PRT
   <213> Artificial Sequence
<220>
   <223> 24820 Finger 5
<400> 402
   Arg Ser Asp Asn Leu Thr Arg
   1 5
<210> 403
   <211> 7
   <212> PRT
   <213> Artificial Sequence
<220>
   <223> 24821 Finger 1
<400> 403
   Asp Arg Ser His Leu Ala Arg
   1 5
<210> 404
   <211> 7
   <212> PRT
   <213> Artificial Sequence
<220>
   <223> 24821 Finger 2
<400> 404
   Arg Ser Asp Asn Leu Ser Glu
   1 5
<210> 405
   <211> 7
   <212> PRT
   <213> Artificial Sequence
<220>
   <223> 24821 Finger 3
<400> 405
   Ser Lys Gln Tyr Leu Ile Lys
   1 5
<210> 406
   <211> 7
   <212> PRT
   <213> Artificial Sequence
<220>
   <223> 24821 Finger 4
<400> 406
   Glu Arg Gly Thr Leu Ala Arg
   1 5
<210> 407
   <211> 7
   <212> PRT
   <213> Artificial Sequence
<220>
   <223> 24821 Finger 5
<400> 407
   Arg Ser Asp His Leu Thr Thr
   1 5
<210> 408
   <211> 7
   <212> PRT
   <213> Artificial Sequence
<220>
   <223> 24828 Finger 1
<400> 408
   Asp Arg Ser Asp Leu Ser Arg
   1 5
<210> 409
   <211> 7
   <212> PRT
   <213> Artificial Sequence
<220>
   <223> 24828 Finger 2
<400> 409
   Arg Ser Asp Asn Leu Thr Arg
   1 5
<210> 410
   <211> 7
   <212> PRT
   <213> Artificial Sequence
<220>
   <223> 24828 Finger 3
<400> 410
   Gln Arg Thr His Leu Thr Gln
   1 5
<210> 411
   <211> 7
   <212> PRT
   <213> Artificial Sequence
<220>
   <223> 24828 Finger 4
<400> 411
   Arg Ser Asp Asn Leu Ser Glu
   1 5
<210> 412
   <211> 7
   <212> PRT
   <213> Artificial Sequence
<220>
   <223> 24828 Finger 5
<400> 412
   Ala Ser Lys Thr Arg Lys Asn
   1 5
<210> 413
   <211> 7
   <212> PRT
   <213> Artificial Sequence
<220>
   <223> 24829 Finger 1
<400> 413
   Arg Ser Asp Thr Leu Ser Glu
   1 5
<210> 414
   <211> 7
   <212> PRT
   <213> Artificial Sequence
<220>
   <223> 24829 Finger 2
<400> 414
   Gln Ser His Asn Arg Thr Lys
   1 5
<210> 415
   <211> 7
   <212> PRT
   <213> Artificial Sequence
<220>
   <223> 24829 Finger 3
<400> 415
   Gln Ser Asp His Leu Thr Gln
   1 5
<210> 416
   <211> 7
   <212> PRT
   <213> Artificial Sequence
<220>
   <223> 24829 Finger 4
<400> 416
   Arg Ser Ser Asp Leu Ser Arg
   1 5
<210> 417
   <211> 7
   <212> PRT
   <213> Artificial Sequence
<220>
   <223> 24829 Finger 5
<400> 417
   Gln Ser Ser Asp Leu Ser Arg
   1 5
<210> 418
   <211> 7
   <212> PRT
   <213> Artificial Sequence
<220>
   <223> 24829 Finger 6
<400> 418
   Arg Ser Asp His Leu Thr Gln
   1 5
<210> 419
   <211> 7
   <212> PRT
   <213> Artificial Sequence
<220>
   <223> 24832 Finger 1
<400> 419
   Arg Ser Asp Ser Leu Ser Arg
   1 5
<210> 420
   <211> 7
   <212> PRT
   <213> Artificial Sequence
<220>
   <223> 24832 Finger 2
<400> 420
   Arg Lys Asp Ala Arg Ile Thr
   1 5
<210> 421
   <211> 7
   <212> PRT
   <213> Artificial Sequence
<220>
   <223> 24832 Finger 3
<400> 421
   Asp Arg Ser His Leu Ser Arg
   1 5
<210> 422
   <211> 7
   <212> PRT
   <213> Artificial Sequence
<220>
   <223> 24832 Finger 4
<400> 422
   Gln Ser Gly Asn Leu Ala Arg
   1 5
<210> 423
   <211> 7
   <212> PRT
   <213> Artificial Sequence
<220>
   <223> 24832 Finger 5
<400> 423
   Gln Ser Ser Asp Leu Ser Arg
   1 5
<210> 424
   <211> 7
   <212> PRT
   <213> Artificial Sequence
<220>
   <223> 24832 Finger 6
<400> 424
   Asp Arg Ser Ala Leu Ala Arg
   1 5
<210> 425
   <211> 7
   <212> PRT
   <213> Artificial Sequence
<220>
   <223> 24833 Finger 1
<400> 425
   Arg Ser Asp Asp Leu Ser Lys
   1 5
<210> 426
   <211> 7
   <212> PRT
   <213> Artificial Sequence
<220>
   <223> 24833 Finger 2
<400> 426
   Arg Ser Asp Thr Arg Lys Thr
   1 5
<210> 427
   <211> 7
   <212> PRT
   <213> Artificial Sequence
<220>
   <223> 24833 Finger 3
<400> 427
   Asp Arg Ser Asn Leu Ser Arg
   1 5
<210> 428
   <211> 7
   <212> PRT
   <213> Artificial Sequence
<220>
   <223> 24833 Finger 4
<400> 428
   Asp Arg Ser Asn Leu Trp Arg
   1 5
<210> 429
   <211> 7
   <212> PRT
   <213> Artificial Sequence
<220>
   <223> 24833 Finger 5
<400> 429
   Arg Ser Asp Ser Leu Ser Arg
   1 5
<210> 430
   <211> 7
   <212> PRT
   <213> Artificial Sequence
<220>
   <223> 24833 Finger 6
<400> 430
   Asn Asn Asp His Arg Lys Thr
   1 5
<210> 431
   <211> 694
   <212> DNA
   <213> artificial sequence
<220>
   <223> Event details: em02-5788-1-1 Left border flanking
<400> 431
   tcgagattgt gctgaagtaa accattttac ttcaaatcta tttttaacta tttactttta 60
   ttaaggagag aaactttgct gattaattca aattagtgat cattaagatt ccaaagattc 120
   cgatttagaa aagtcaaaga ttcaaagaac aagtctaggt cctcatggct catgttgcat 180
   ccgattcacc atccactcat ctttcatatc ttcctccact gtctctctag aaacaactca 240
   tttaatttag aaaactcctt tttcaattta gaaatattaa agtttatcac aatgtatcaa 300
   ttaaatatta tccgatgact cattcatagt caggaccttg ctgtctgtgt cgtccgtaat 360
   tattatttca atacaaaaca aatatatgtt cactcagaaa attacggcgc aatcatctaa 420
   ttttgtggac caaaataaat agcgtagctt cgagatttca aagttgtgtt caaatttaat 480
   tttgatttcc gttcctcgta tactctttta tgtatagaaa ataataatat ccactactag 540
   tagttgataa ctacattaca tatattaaaa ttatgatgtc acattgcgga cgtttttaat 600
   gtactgaatt aacgccgaat tgaattcgag ctcggtaccc ggggatcctc tagagtcgac 660
   ctgcaggcat gcaagcttag cttgagcttg gatc 694
<210> 432
   <211> 732
   <212> DNA
   <213> artificial sequence
<220>
   <223> em02-5788-1-1 Left border flanking
<400> 432
   cataaccacc atctcaaaca atagaacttc ctaagtgaag caatgacttc aaatctactt 60
   gaaggcatgg agtataagcc atgttccttt cagaggggac tgtacttctg tagattactt 120
   tccctcatta accagatctg gccggcctac ccagctttct tgtacacata gcgaccgagc 180
   tcgagccgaa gttcggtcgc tgtttcactg tttgggaaag catcagtaac gcagaagaca 240
   taattaaaaa ttaattatat atggtagttt ttctagattc tccactatac ctcattgtga 300
   ttgaaaaaca actatatata tatatatata tgtatttaaa attaagaaat cattaaatcg 360
   taccataatg cagaaaactt tataagttcc tattcttttg tcaagatgag tagatgacac 420
   atcatgtacc acataacata accataatgg tgcgatacca taccaaaact taatttagaa 480
   actaattaaa attttggtag tttaagattc ctcctaatgg ttgtcaaaaa aaaaaaaaga 540
   ttcctcctaa tacatggtag aatatatttt tgagttaaaa tgaaagtaaa atcttcaaat 600
   tagtcataag gaaattctaa aaaacatcga ctttctttat aaagatccca ttgtaatttt 660
   agatgattaa ttttatccca atccaattaa gaattgtaca catcggcctc tatatatatc 720
   aaacacctaa aa 732
<210> 433
   <211> 643
   <212> DNA
   <213> artificial sequence
<220>
   <223> Event details: ad58-5784-2-1 Left border flanking
<400> 433
   cgatttgcag ctataatcaa tcacacctta tcgttctttc aaagaaaaat cgaaagttgt      60
   aaactttatc agcctgtgta gtgattattt caatttgata aagaaaaaaa aaggcttagc     120
   tttatttggt tttttgttac aatcttgatt aattttagat tagcactctg attctagcgg     180
   aacatgagag tggttccatc aaacctcaga cagtgagcac agtggttgca gcaaaccatt     240
   tgggtgagag ctcttcagtt tctttgctat tagctggttc tggctcttct cttcaagaag     300
   ctgcttctca agctgcctct tgtcatcctt ctgtttccga ggtaactaac tacattcttc     360
   atttgtcctt ttttcttgtg ggtcttaaat gttygtgctt ttctttatag gtacttgttg     420
   ctgattcaga tagatttgag taccctttag ctgaaccttg ggctaaactg gttgactttg     480
   ttcgccaaca aagagattac tctcacatcc ttgccttcct ctagctcatt tggcaagaac     540
   atacttcctc tagacaactt aataacacat tgcggacgtt tttaatgtac tgaattaacg     600
   ccgaattgaa ttcgagctcg gtacccgggg atcctctaga gtc                       643
<210> 434
   <211> 653
   <212> DNA
   <213> artificial sequence
<220>
   <223> ad58-5784-2-1 Right border flanking
<400> 434
   cctgtcataa ccaccatctc aaacaataga acttcctaag tgaagcaatg acttcaaatc 60
   tacttgaagg catggagtat aagccatgtt cctttcagag gggactgtac ttctgtagat 120
   tactttccct cattaaccag atctggccgg cctacccagc tttcttgtac aaagtggtga 180
   taaactatcg ccggcctacc tcgcgttgct gctcttttag atgtctctcc tgttactgat 240
   gttgtcaaaa tcttaggatc caatgagttt atcaggtata cttctatcat gtattgcttg 300
   agattttgga gtgttagtaa agatttcaat aaaagaattt tttcaaacaa aattttgggg 360
   gcttgaagct aatgtttgga aatatgtaac ggagtttaaa tcttttggca ggcctatata 420
   tgcgggaaac gccttgtgta gagttcgcta tactggtgct ggtccttgtg tgttgactat 480
   tagaactaca tcttttcctg ttaccccaat aactgagtca aagaaagcta ctatctctca 540
   gattgatctc tcgaaattca aagaaggttt gtttagtatt attctcttgt gcatagcctt 600
   tttgcttttt ttttttttat aaaaaaagtt gagtatgctt attgcccatt gca 653
<210> 435
   <211> 450
   <212> DNA
   <213> artificial sequence
<220>
   <223> Event details: ad58-5898-10-1 Left border flanking
<400> 435
   taatttcatt ttcgtcattt tggtaaagta acaaaagaca gaatattggt tagtcgtgtt 60
   ggttaggaat aaaataaaga acgtggacat cgtggaataa aaatattcag acaaggaaac 120
   taacaataaa acagtaatga acatggttct gaatctcatc tttgtgtatc tccaatggaa 180
   tccaccgcca cgaatcagac tccttctcca agctccaccg tcgacgatga caatggcgac 240
   ggtgtctata ccgacgaatt taccaaacta cccccggacc cggggatcct ctagagtcaa 300
   caaattgacg cttagacaac ttaataacac attgcggacg tttttaatgt actgaattaa 360
   cgccgaattg aattcgagct cggtacccgg ggatcctcta gagtcgacct gcaggcatgc 420
   aagcttagct tgagcttgga tcagattgtc 450
<210> 436
   <211> 644
   <212> DNA
   <213> artificial sequence
<220>
   <223> ad58-5898-10-1 Right border flanking
<400> 436
   cggcctaccc agctttcttg tacaaagtgg tgataaacta tcagtgtttg attaaagata 60
   aaatttgatt tttcattaca taataatcca ttaattttca cgcacgtgga acccatttgg 120
   tgtacttcca cgtcctctaa gagagcactg acccactcat caaaaagata catctttata 180
   agccccttca tcgtcacaca gacacacact tccctctcaa ttattccata ttctcctaat 240
   ttctaattgt tacacctcaa cacattagat tcgtaccaaa caaaaacgat tagctcccaa 300
   gcctaagctt ttatttcctt ataatttttc ttgggtttct ctctataaag aatgcaaatg 360
   actgagagag gccgagccat gtggcacacg tccctagcct cggcattccg cacagctcta 420
   gcttgcacaa tcgttggtgc ggctacgctc tacggacccg agtggatcct ccgttttgtg 480
   gcattcccgg cgttttctta cgtcacggtc attctcatca ttacggacgc cacgctaggc 540
   gacacactac gtggctgctg gctagccctt tacgccacat gtcagagcgt tgcaccggct 600
   atcattacac taaggcttat aggaccagct cggctcacgg ccgg 644
<210> 437
   <211> 674
   <212> DNA
   <213> artificial sequence
<220>
   <223> lf31-6139-2-3 Left border flanking
<400> 437
   tttctttcca tcagttcttc ggcaccttcc tggctctgcg tctatctttc tttccatccc 60
   ggcccatctc gtacacattc cacccaacta cacaaacacg ttatagtctt ttacattatg 120
   accaaatcaa ccctaaagat acagccttta taaaaaatat aggggtcaaa gcaaagaaga 180
   gaaagtttgc ttacagtgtg gagaaaagaa gttggaagat gaggagtaag aagaagaaga 240
   gaagagaaag ggtcttctga tgaggaatag gagataaggt ggaactggaa tgtttgccgc 300
   taaatacttg aagacaagag cttgattttc aagctcttcc cattgtgact cagtgaaagg 360
   gatcctagtc gtcatgaaga gatagagaaa attgatgatg aaggaggtct gatgaagaga 420
   agagagagag agagatatta cacaaagagg gttgtattgc aaaattgaaa gtgtagagag 480
   agtagtaggt aagtttttat taataatgtt gttcacacct gcagtctgca gaatattgtg 540
   gtgtgaacaa attgacgctt agacaactta ataacacatt gcggacgttt ttaatgtact 600
   gaattaacgc cgaattgaat tcgagctcgg tacccgggga tcctctagag tcgacctgca 660
   ggcatgcaag ctta 674
<210> 438
   <211> 544
   <212> DNA
   <213> artificial sequence
<220>
   <223> [00213] lf31-6139-2-3 Right border flanking
<400> 438
   ttcaatctac ttgaaggcat ggagtataag ccatgttcct ttcagagggg actgtacttc      60
   tgtagattac tttccctcat taaccagatc tggccggcct acccagcttt cttgtacaaa     120
   gtggtgataa actatcagtg tttgaacata tatatacgca taatattctc agaacccgac     180
   ccattggttg actcggatca agatcgaccc gatccgaccc ggttaaaagc acgtcgtctc     240
   ctttggttcg ccccttatat tcgacgagtg agttcgattg gatcgctgtt tgtcatttct     300
   cactacctta agaaaaaaaa aaaggtgcgt ctctctctca cctttaccgc tcacttacct     360
   ctcagatctg acatcgattt tccaaatctt ctccaggtac tctctctcct ggccgttgac     420
   ggtcccgtcc cggccgtgga tctgatttcg ccgcgatctg aggtcaagca tggcggcagc     480
   taacgccccc atcacgatga aggaggtcct aacggtgagt cccgcctcca tttttagtaa     540
   cata                                                                  544
<210> 439
   <211> 569
   <212> DNA
   <213> artificial sequence
<220>
   <223> bm56-6315-1-1 Left border flanking
<400> 439
   tacatcgcga ttcatcctgg tttgattaga atgacgagga agttgtcata ttcccaaaca      60
   ggaaaattgg gatcgcctta tttgaaagtg ggataacttc ttcatcttaa ttcttatgag     120
   aattattcca cttcctggtg attctccact actttttgta tataaataca gcttcttaca     180
   tcgcgattca tcctgatttg attagaatga cgagaaagtt gtcatattcc caaacaggaa     240
   aactgggatc acctgatttg aaagtgggat aacttcttca tcctaattct tatgagattt     300
   attccacttc ctggtgattc tccacttctt tatgtatcca aatacatctt cttacatcgc     360
   gattcatcct ggtttgatta gaatgacgag gaagttgtca tattcccaaa catgaaaact     420
   gggaaagtgg gattgacgct tagacaactt aataacacat tgcggacgtt tttaatgtac     480
   tgaattaacg ccgaattgaa ttcgagctcg gtacccgggg atcctctaga gtcgacctgc     540
   aggcatgcaa gcttagcttg agcttggat                                       569
<210> 440
   <211> 685
   <212> DNA
   <213> artificial sequence
<220>
   <223> bm56-6315-1-1 Right border flanking
<400> 440
   ccaccatctc aaacaataga acttcctaag tgaagcaatg acttcaaatc tacttgaagg      60
   catggagtat aagccatgtt cctttcagag gggactgtac ttctgtagat tactttccct     120
   cattaaccag atctggccgg cctacccagc tttcttgtac aaagtgacga taaactatca     180
   gtctttcaaa gcgcatctat ggctagtcat cacggttttt aactgtttta cgaagtcgcg     240
   gagaggctcg tcttctccct aggataaact gcagaggtcg acatcggagg tttctcgatc     300
   tatgaacata gagtactgtt tgagaaactc cgaggcgagt tggcggaaac tccctataga     360
   gtttttcttt agacaagaga accactcaag cgctgcttcg cggaggttct cgacgaagag     420
   gcggcattcg ccggcatctc tttctctatc tttaaacttg gctcttccca tcgcgatctg     480
   gaaagcctgc aagtgtgcct taggatcggt tgtaccatcg taaggagcca ctttgacttt     540
   atcaggatcc gaaaccgtag tttcggtgat gcgggtggtg aagggcgttt ttcgagactc     600
   ctcgaggagt agggtgatat cgagtgcagt actagtagcg tgatgatttg ggatttcacc     660
   gctctaacct ccgcagccgt tttca                                           685
<210> 441
   <211> 734
   <212> DNA
   <213> artificial sequence
<220>
   <223> ad58-6372-1-1 Left border flanking
<400> 441
   ttttacagtg ttagaagaag tggatgaagc tgaaattgaa ttttcaaact cgttcagctt      60
   gactagaggt gggagagagt caaagcctcc catcaagtac caaaacatgg aatagaagac     120
   agtccgaggg agaggaaacc gtggccgacg aggccgtggc tcctatcatt aactagtgtc     180
   tttcttacta ttaatggttt attaagtctc agcctttgtt gtttcattgg tttgagattc     240
   actcatacat gaaacttgtt tcattccagc ttttccaaac tataagaata tttccaatct     300
   tatcttgtaa tagtttaagt tttaaattga aagcccttag ttcaaaaaac aaaaaaaaaa     360
   aattagccct tgaatttata tataatcacg acggccatat ttggcagcta cactgatatg     420
   ttttcaattg gctgacaggc cttgagcagg gtttgctggg tatattggta ggaagatgtg     480
   ttgcgaggtt gaagcctcat ttaggcaata taaacatgat cattagcgtt atgtcattag 540
   ttatacttat acgtagacta agtaacccac taaaggttgc tgattccttt tgtatcgact 600
   aacacattgc ggacgttttt aatgtactga attaacgccg aattgaattc gagctcggta 660
   cccggggatc ctctagagtc gacctgcagg catgcaagct tagcttgagc ttggatcaga 720
   ttgtcgtttc ccgc 734
<210> 442
   <211> 521
   <212> DNA
   <213> artificial sequence
<220>
   <223> ad58-6372-1-1 Right border flanking
<400> 442
   catgttcctt tcagagggga ctgtacttct gtagattact ttccctcatt aaccagatct 60
   ggccggccta cccagctttc ttgtacaaag tggtgataaa ctatcagtgt ttgactgaat 120
   tttaatttct aatttttgta aaaaatttgt ataacctcaa attattaaaa ggcggatttt 180
   attagaatta taactaaatt atctataact ccaaaatttt gacaatcaat catgtctata 240
   tctttatttt tttgctaaat tatcatgtct atatcttttc tttcttccaa acttacttga 300
   gactaaaagt ctttataaat tttgatagga gttccacaca caaacaaaaa caaaacaaat 360
   atttttcatc aagggatact tatttaacat cacggattca cagtttatta acaaaaatcc 420
   aaacaaagac tgaaagacag aagattcaat ctaacaatag tcggcaaaca ccagtgatta 480
   actaacgaaa taaattaaca agtggtcaga tcttcgggaa a 521
<210> 443
   <211> 450
   <212> DNA
   <213> artificial sequence
<220>
   <223> ad58-6620-4-1 Left border flanking
<400> 443
   taatttcatt ttcgtcattt tggtaaagta acaaaagaca gaatattggt tagtcgtgtt      60
   ggttaggaat aaaataaaga acgtggacat cgtggaataa aaatattcag acaaggaaac     120
   taacaataaa acagtaatga acatggttct gaatctcatc tttgtgtatc tccaatggaa     180
   tccaccgcca cgaatcagac tccttctcca agctccaccg tcgacgatga caatggcgac     240
   ggtgtctata ccgacgaatt taccaaacta cccccggacc cggggatcct ctagagtcaa     300
   caaattgacg cttagacaac ttaataacac attgcggacg tttttaatgt actgaattaa     360
   cgccgaattg aattcgagct cggtacccgg ggatcctcta gagtcgacct gcaggcatgc     420
   aagcttagct tgagcttgga tcagattgtc                                      450
<210> 444
   <211> 775
   <212> DNA
   <213> artificial sequence
<220>
   <223> ad58-6620-4-1 Right border flanking
<400> 444
   cataaccacc atctcaaaca atagaacttc ctaagtgaag caatgacttc aaatctactt      60
   gaaggcatgg agtataagcc atgttccttt cagaggggac tgtacttctg tagattactt     120
   tccctcatta accagatctg gccggcctac ccagctttct tgtacaaagt ggtgataaac     180
   tatcagtgtt tgaaataatc ggatatttaa ttttcttaga cagttcatta gtagttgatc     240
   ttaaacattc acgttttatt ttcttttctt ttcgaatgct agactctagt ttggtaccca     300
   taggattcga gttacatgaa gctattgaca ctgggagtgc ttcaagatct gtaagaggca     360
   aagattcaca aacagaacgt gatttcttgg atagtgatgt ggagattgtg ataagaacca     420
   gcatgagtat tacttttact gcccctgctg tggtgaagac atcaccaaaa cagtcaagct     480
   cgtgaagaaa tcagatattc aacccgcaaa aaaatctgac aatgcaaata aacctattga     540
   cactaagaat ggttcaagat ccgaagacaa gaagacaaaa aatttgtcct ggctccctgc     600
   ttatctccag aagctgtttc tttctgttta tggccacatc aaagacaaag gtaccctttc     660
   ttttggtgcc tttggtgtgg acaggtgtga ttgactttgg tttcttggca gattcaggca     720
   agatagaggt tgattcaaag tcaactaaca atgatcttgg taccaatagt gagga          775
<210> 445
   <211> 808
   <212> DNA
   <213> artificial sequence
<220>
   <223> ad58-6620-17-1 Left border flanking
<400> 445
   ccgccttgaa caaccgctcc gccgttgctc agtacattat cgaggttacc aaaaagttca      60
   atcctttatg cttgttttgg cgtgtgattc gttacgaatg agtaaaattg atttggtttt     120
   tttctttgaa cagcatggtg gagatgtgaa tgcgacggat catacggggc agactgcgtt     180
   gcattggagt gcggttcgtg gtgcggtgca agttgcggag cttttgcttc aagagggtgc     240
   aagggttgat gctacggata tgtatggata tcaggttcta accacttcct ctttctttgt     300
   ggagattgtc tttttgtttc aatgctagtc aacttctttc tttctttcac aaaactaagt     360
   agtattgctt gttttgttgt cgttgcattt tttcttatgg ctgtgttctg aggttcatgt     420
   aggtatataa gcacttcgta ctttgccact tgtttcattt aggcaacatt gtgcacatat     480
   ctaagtagtt ggtctttgta aaattagttt gtttgtcttc aaagtatatt gagcagtttc     540
   atgactcatt attcaaaggt ttgtctaaat tagagagaac tttcattttg cctggattta     600
   atcagcattt agaatgttta tagcgatatc atttttagtt gaaaaaatct caacaaattg     660
   acgcttagac aacttaataa cacattgcgg acgtttttaa tgtactgaat taacgccgaa     720
   ttgaattcga gctcggtacc cggggatcct ctagagtcga cctgcaggca tgcaagctta     780
   gcttgagctt ggatcagatt gtcgtttc                                        808
<210> 446
   <211> 578
   <212> DNA
   <213> artificial sequence
<220>
   <223> ad58-6620-17-1 Right border flanking
<400> 446
   gtataagcca tgttcctttc agaggggact gtacttctgt agattacttt ccctcattaa      60
   ccagatctgg ccggcctacc cagctttctt gtacaaagtg gtgataaact atcagtgtta     120
   gatccccgac cgaccgccca tcctggacgg cctcgtgcat gctgatgttg tcaaaatctt     180
   aggatccaat gagtttatca ggtatacttc tatcatgtat tgcttgagat tttggagtgt     240
   tagtaaagat ttcaataaaa gaattttttc aaacaaaatt ttgggggctt gaagctaatg      300
   tttggaaata tgtaacggag tttaaatctt ttggcaggcc tatatatgcg ggaaacgcct     360
   tgtgtagagt tcgctatact ggtgctggtc cttgtgtgtt gactattaga actacatctt     420
   ttcctgttac cccaataact gagtcaaaga aagctactat ctctcagatt gatctctcga     480
   aattcaaaga aggtttgttt agtattattc tcttgtgcat agcctttttg sttttttttt      540
   ttttataaaa aaagttgagt atgcttattg cccattgc                             578
<210> 447
   <211> 24
   <212> DNA
   <213> artificial sequence
<220>
   <223> primer sequence
<400> 447
   cttacatgct taggatcgga cttg 24
<210> 448
   <211> 22
   <212> DNA
   <213> artificial sequence
<220>
   <223> primer
<400> 448
   agttccagca ccagatctaa cg 22
<210> 449
   <211> 24
   <212> DNA
   <213> artificial sequence
<220>
   <223> primer
<400> 449
   ccctgagccc aagcagcatc atcg 24
<210> 450
   <211> 18
   <212> DNA
   <213> artificial sequence
<220>
   <223> primer
<400> 450
   cggagagggc gtggaagg 18
<210> 451
   <211> 22
   <212> DNA
   <213> artificial sequence
<220>
   <223> primer
<400> 451
   ttcgatttgc tacagcgtca ac 22
<210> 452
   <211> 22
   <212> DNA
   <213> artificial sequence
<220>
   <223> primer
<400> 452
   aggcaccatc gcaggcttcg ct 22
<210> 453
   <211> 22
   <212> DNA
   <213> artificial sequence
<220>
   <223> primer
<400> 453
   cttccactcc ttcctcctcg tc 22
<210> 454
   <211> 21
   <212> DNA
   <213> artificial sequence
<220>
   <223> primer
<400> 454
   gcgtcccaaa gggttgttga g 21
<210> 455
   <211> 22
   <212> DNA
   <213> artificial sequence
<220>
   <223> primer
<400> 455
   tctctactgg gcctgccagg gc 22
<210> 456
   <211> 28
   <212> DNA
   <213> Artificial Sequence
<220>
   <223> primer
<400> 456
   ccccgagacg ttgaaggcta agtacaaa 28
<210> 457
   <211> 24
   <212> DNA
   <213> artificial sequence
<220>
   <223> primer
<400> 457
   ttgcgctgac ggattctaca agga 24
<210> 458
   <211> 24
   <212> DNA
   <213> artificial sequence
<220>
   <223> primer
<400> 458
   tccatcagtc caaacagcag caga 24
<210> 459
   <211> 23
   <212> DNA
   <213> artificial sequence
<220>
   <223> primer
<400> 459
   catagcagtc tcacgtcctg gtc 23
<210> 460
   <211> 26
   <212> DNA
   <213> artificial sequence
<220>
   <223> primer
<400> 460
   ggaagctaag ccattacact gttcag 26
<210> 461
   <211> 23
   <212> DNA
   <213> artificial sequence
<220>
   <223> primer
<400> 461
   catagcagtc tcacgtcctg gtc 23
<210> 462
   <211> 20
   <212> DNA
   <213> artificial sequence
<220>
   <223> primer
<400> 462
   cctgatccgt tgacctgcag 20
<210> 463
   <211> 20
   <212> DNA
   <213> artificial sequence
<220>
   <223> primer
<400> 463
   gtgtgaggtg gctaggcatc 20
<210> 464
   <211> 26
   <212> DNA
   <213> artificial sequence
<220>
   <223> primer
<400> 464
   ggaagctaag ccattacact gttcag 26
<210> 465
   <211> 22
   <212> DNA
   <213> artificial sequence
<220>
   <223> primer
<400> 465
   agagaggaga cagagagaga gt 22
<210> 466
   <211> 23
   <212> DNA
   <213> artificial sequence
<220>
   <223> primer
<400> 466
   agacagcatc aagatttcac aca 23
<210> 467
   <211> 21
   <212> DNA
   <213> artificial sequence
<220>
   <223> primer
<400> 467
   caacggcgag cgtaatctta g 21
<210> 468
   <211> 22
   <212> DNA
   <213> artificial sequence
<220>
   <223> primer
<400> 468
   gttccctgga attgctgata gg 22
<210> 469
   <211> 20
   <212> DNA
   <213> artificial sequence
<220>
   <223> primer
<400> 469
   tgttggtgga agaggatacg 20
<210> 470
   <211> 18
   <212> DNA
   <213> artificial sequence
<220>
   <223> primer
<400> 470
   atcagcagca gcgatagc 18
<210> 471
   <211> 118
   <212> DNA
   <213> brassica napus
<400> 471
   gacatcaagt ggtacggcaa gtacctcaac aacccgctag gacgcacggt gatgctaacc        60
   gtccagttca agctcggctg gccgttgtac ttagccttca acgtctcggg aagacctt         118
<210> 472
   <211> 118
   <212> DNA
   <213> brassica napus
<400> 472
   gacatcaagt ggtacggcaa gtacctcaac aacccgctag gacgcacggt gatgctaacc        60
   gtccagttca cgctcggctg gccgttgtac ttagccttca acgtctctgg aagacctt         118
<210> 473
   <211> 118
   <212> DNA
   <213> brassica napus
<400> 473
   gacatcaagt ggtacggcaa gtacctcaac aaccctttgg gacgcacggt gatgttaacg 60
   gttcagttca ctctcggctg gcctttgtac ttagccttca acgtctcggg aagacctt 118
<210> 474
   <211> 118
   <212> DNA
   <213> brassica napus
<400> 474
   gacatcaagt ggtacggcaa gtacctcaac aaccctttgg gacgcacggt gatgttaacg 60
   gttcagttca ctctcggctg gccgttgtac ttagccttca acgtctcggg aagacctt 118
<210> 475
   <211> 20
   <212> DNA
   <213> brassica napus
<400> 475
   cagacatcaa gtggtacggc 20
<210> 476
   <211> 28
   <212> DNA
   <213> brassica napus
<400> 476
   acggcaagta cctcaacaac cctttggg 28
<210> 477
   <211> 28
   <212> DNA
   <213> brassica napus
<400> 477
   cgcaccgtga tgttaacggt tcagttca 28
<210> 478
   <211> 28
   <212> DNA
   <213> brassica napus
<400> 478
   cggcaagtac ctcaacaacc ctttggga 28
<210> 479
   <211> 28
   <212> DNA
   <213> brassica napus
<400> 479
   accgtgatgt taacggttca gttcactc 28
<210> 480
   <211> 20
   <212> DNA
   <213> brassica napus
<400> 480
   cttagcttca acgtctcggg 20

## Description

### TECHNICAL FIELD

The disclosure relates to the field of fluorescence activated cell sorting to generate plants. In a preferred embodiment, the disclosure describes FACS enrichment of edited, regenerable protoplasts to generate fertile edited plants.

### BACKGROUND

The Fluorescence Activated Cell Sorter (FACS) was invented in the late 1960s by Bonner, Sweet, Hulett, Herzenberg, and others to do flow cytometry and cell sorting of viable cells. Becton Dickinson Immunocytometry Systems introduced the commercial machines in the early 1970s. Fluorescence-activated cell sorting (FACS) is a specialized type of flow cytometry. It provides a method for sorting a heterogeneous mixture of biological cells into two or more containers, one cell at a time, based upon the specific light scattering and fluorescent characteristics of each cell. It is a useful scientific instrument, as it provides fast, objective and quantitative recording of fluorescent signals from individual cells as well as physical separation of cells of particular interest.

The cell suspension is entrained in the center of a narrow, rapidly flowing stream of liquid. The flow is arranged so that there is a large separation between cells relative to their diameter. A vibrating mechanism causes the stream of cells to break into individual droplets. The system is adjusted so that there is a low probability of more than one cell per droplet. Just before the stream breaks into droplets, the flow passes through a fluorescence measuring station where the fluorescent character of interest of each cell is measured. An electrical charging ring is placed just at the point where the stream breaks into droplets. A charge is placed on the ring based on the immediately prior fluorescence intensity measurement, and the opposite charge is trapped on the droplet as it breaks from the stream. The charged droplets then fall through an electrostatic deflection system that diverts droplets into containers based upon their charge. In some systems, the charge is applied directly to the stream, and the droplet breaking off retains charge of the same sign as the stream. The stream is then returned to neutral after the droplet breaks off.

A wide range of fluorophores can be used as labels in flow cytometry. Fluorophores, or simply "fluors," are typically attached to an antibody that recognizes a target feature on or in the cell; they may also be attached to a chemical entity with affinity for the cell membrane or another cellular structure. Each fluorophore has a characteristic peak excitation and emission wavelength, and the emission spectra often overlap. Consequently, the combination of labels which can be used depends on the wavelength of the lamp(s) or laser(s) used to excite the fluorochromes and on the detectors available.

Fluorescence-activated cell sorting (FACS) provides a rapid means of isolating large numbers of fluorescently tagged cells from a heterogeneous mixture of cells. Collections of transgenic plants with cell type-specific expression of fluorescent marker genes such as green fluorescent protein (GFP) are ideally suited for FACS-assisted studies of individual cell types.

It has been demonstrated that flow cytometric analysis and fluorescence activated cell sorting (FACS) of plant protoplasts is practicable, moreover, this technique has yielded valuable results in a number of different fields of research (Harkins and Galbraith, 1984; Galbraith *et al.,* 1995; Sheen *et al.,* 1995). For instance, FACS of protoplasts from *Arabidopsis* plants expressing tissue-specific fluorescent protein markers has been used to examine both basal and environmentally stimulated transcriptional profiles in particular cell types (Birnbaum *et al.,* 2003; Brady *et al.,* 2007; Gifford *et al.,* 2008; Dinneny *et al.,* 2008) and flow cytometry has been employed to analyze reactive oxygen species production and programmed cell death tobacco protoplasts (*Nicotiana tabacum*; Lin *et al.,* 2006). A broad selection of fluorescent tools is available to study a plethora of physiological parameters in plants, *e.g*., *cis*-regulatory elements fused to fluorescent proteins (Haseloff and Siemering, 2006), genetically-encoded molecular sensors (Looger *et al.,* 2005) or dye-based sensors (Haugland, 2002) can be used in combination with cytometry to measure diverse biological processes. However, there are certain inefficiencies with this process due to the sensitivities of the assays and thus there is room for improvement.

"Fluorescence Activated Cell Sorting of Plant Protoplasts", Bargmann et al, Journal of Visualized Experiments pp1-4, vol 36, 2010 discloses a method of separating green fluorescent protein positive cells using fluorescence activated cell sorting.

"Nurse culture of low numbers of Medicago and Nicotiana protoplasts using calcium alginate beads", Larkin et al, Plant Science, pp203-210, vol 58, no 2, 1 January 1988 discloses culturing of lucerne and tobacco in semi-solid droplets of calcium alginate.

### DISCLOSURE

In a first aspect, there is provided a method for generating a transgenic plant from a population of plant cells, the method comprising isolating a plant protoplast comprising a polynucleotide of interest, the method comprising separating the at least one sodium alginate-encapsulated plant protoplast comprising the polynucleotide of interest and a fluorescent marker from the remaining plant protoplasts in the population of plant protoplasts by fluorescence-activated cell sorting (FACS) and culturing the separated sodium alginate-encapsulated plant protoplast(s) comprising the polynucleotide of interest and the fluorescent marker to regenerate a transgenic plant.

A plant can be regenerated by isolating a plant protoplast comprises a polynucleotide of interest integrated into the genome of the plant protoplast by providing a population of plant protoplasts having at least one protoplast comprising a polynucleotide of interest and a fluorescent marker; wherein the plant protoplast is encapsulated by sodium alginate; recovering microcalli from the population of protoplasts comprising the polynucleotide of interest and the fluorescent marker wherein the at least one protoplast comprises the polynucleotide of interest and the fluorescent marker has been transformed with the polynucleotide of interest and a polynucleotide encoding the fluorescent marker; regenerating a plant from said microcalli; and culturing said plant.

In a second aspect, there is provided a method for producing a transgenic plant, the method comprising transforming a population of plant protoplasts with a nucleic acid molecule comprising a polynucleotide of interest and a fluorescent marker, and encapsulating the individual protoplasts of the population by sodium alginate, such tha the protoplasts of the population are each encapsulated separately; separating the at least one sodium alginate-encapsulated protoplast comprising the polynucleotide of interest and the fluorescent marker from the remaining plant protoplasts in the population by fluorescence-activated cell sorting (FACS); and culturing the separated sodium alginate-encapsulated protoplast(s) comprising the polynucleotide of interest and the fluorescent marker to regenerate a transgenic plant.

The foregoing and other features will become more apparent from the following detailed description of several embodiments, which proceeds with reference to the accompanying figures.

### BRIEF DESCRIPTION OF THE FIGURES

FIGS. 1A-1E: Shows a sequence alignment of FAD2 gene sequences, generated using ALIGNX®.
FIG. 2: Shows a phylogenetic tree of FAD2 gene sequences generated using JALVIEW® v 2.3 based on neighbor joining distances.
FIGS. 3A-3M': Shows a sequence alignment of FAD3 gene sequences, generated using ALIGNX®.
FIG. 4: Shows a phylogenetic tree of FAD3 gene sequences generated using JALVIEW® v 2.3 based on neighbor joining distances. The labeled sequences correspond as follows: FAD3A'/A" is described throughout this application as FAD3A'; Haplotype2 is described throughout the application as FAD3C'; Haplotype 1 is described throughout the application as FAD3C"; and, Haplotype 3 is described throughout the application as FAD3A".
FIG. 5: Shows a plasmid map of pDAB104010 which that is a representative Zinc Finger Nuclease expression cassette. The lay-out of this construct was similar for the other ZFN expression cassettes, wherein the Zinc Finger domains, 24828 and 24829, were exchanged with alternative Zinc Finger domains that are described above.
FIG. 6: is an example multiple line graph showing number of sequence reads per 10,000 sequence reads with deletions at the target ZFN site. The X axis on the graph denotes number of bases deleted, the Y axis denotes number of sequence reads and the Z axis denotes color-coded sample identity as described to the right of the graph. Specific example shown is for locus 1 of the FAD2 gene family that contains 3 target ZFN sites, A, B and C with the four gene family members and two control transfections assessed as control samples A and B.
FIG. 7: (A) Details of figure axis are as FIG. 6. The figure displays data from ZFN targeting locus 4 of the FAD2 gene family. The locus contains two ZFN sites and two requisite control transfections. FIG. 7: (B) Specific sequence context (SEQ ID NOs 471-480) surrounding the ZFN target site, identifying FAD2A and C containing tri-nucleotide repeats of C, T and G, leading to the observed increase in single base deletions through sequencing of the FAD2A and C loci.
FIG. 8: Shows a plasmid map of pDAS000130.
FIG. 9: Shows a plasmid map of pDAS000271.
FIG. 10: Shows a plasmid map of pDAS000272.
FIG. 11: Shows a plasmid map of pDAS000273.
FIG. 12: Shows a plasmid map of pDAS000274.
FIG. 13: Shows a plasmid map of pDAS000275.
FIG. 14: Shows a plasmid map of pDAS000031.
FIG. 15: Shows a plasmid map of pDAS000036.
FIG. 16: Shows a plasmid map of pDAS000037.

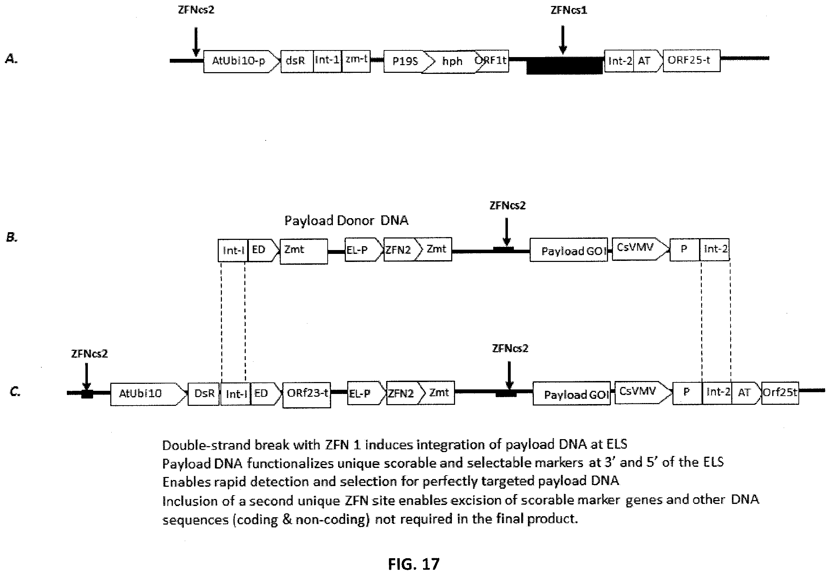

FIG. 17 illustrates an ETIP and payload nucleic acid configuration, as well as the product of the targeted Payload at the ETIP site in the plant cell genome.

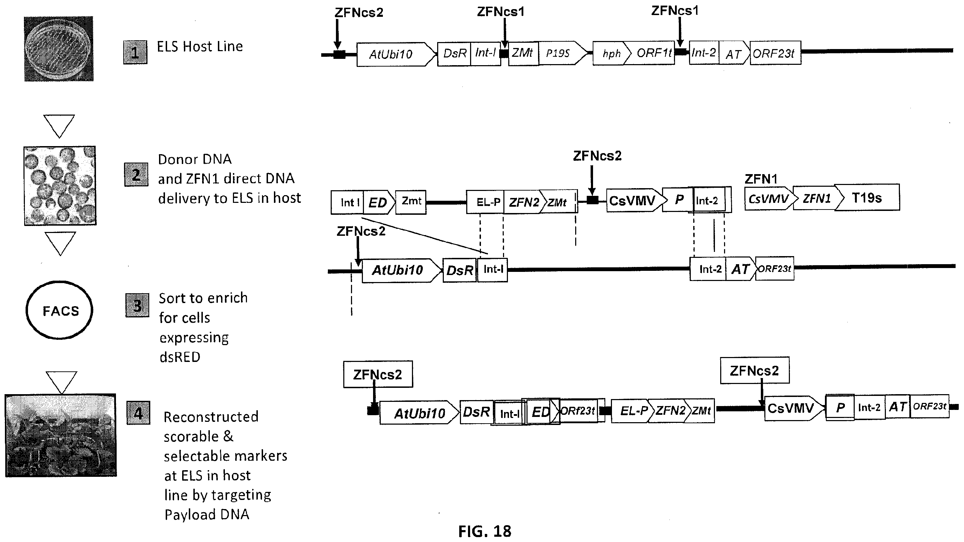

FIG. 18 illustrates transformation of protoplast followed by FACS selection of targeted Payload DNA at the ETIP in the host line using reconstruction of truncated scorable and selectable markers at both the 3' and 5' ends.
FIGS. 19A and 19B: Illustrates the homology directed repair of the ETIP canola event which results from the double stranded DNA cleavage of the genomic locus by the Zinc Finger Nuclease (pDAS000074 or pDAS000075) and the subsequent integration of the *Ds-red* donor (pDAS000068, pDAS000070, or pDAS000072) into the ETIP locus of the canola chromosome. The integration of the donor into the genomic locus results in a fully functional, highly expressing *Ds-red* transgene.
FIG. 20: Shows the FACS sorting of canola protoplasts and the calculated transfection efficiency of canola protoplasts that were transfected with pDAS000031 ("pDAS31"). In addition, the FACS sorting results of untransformed canola protoplasts are provided as a negative control.
FIG. 21: Shows the FACS sorting of canola protoplasts and the calculated transfection efficiency of canola ETIP protoplast events which were transfected with pDAS000064 / pDAS000074 (top graph) and pDAS000064 / pDAS000075 (bottom graph).
FIG. 22: Shows the FACS sorting of canola protoplasts and the calculated transfection efficiency of canola ETIP protoplast events which were transformed with pDAS000068 / pDAS000074 (top graph) and pDAS000068 / pDAS000075 (bottom graph).
FIG. 23: Shows the FACS sorting of canola protoplasts and the calculated transfection efficiency of canola ETIP protoplast events which were transformed with pDAS000070 / pDAS000074 (top graph) and pDAS000070 / pDAS000075 (bottom graph).
FIG. 24: Shows the FACS sorting of canola protoplasts and the calculated transfection efficiency of canola ETIP protoplast events which were transformed with pDAS000072 / pDAS000074 (top graph) and pDAS000072 / pDAS000075 (bottom graph).
FIG. 25: Shows a plasmid map of pDAS000074.
FIG. 26: Shows a plasmid map of pDAS000075.
FIG. 27: Shows a plasmid map of pDAS000064.
FIG. 28: Shows a plasmid map of pDAS000068.
FIG. 29: Shows a plasmid map of pDAS000070.
FIG. 30: Shows a plasmid map of pDAS000072.
FIG. 31: Is a schematic showing binding sites of transgene target primers and probe for transgene copy number estimation assay.
FIG. 32: Shows a SEQUENCHER® file showing FAD2A ZFN DNA recognition domain (bc12075_Fad2a-r272a2 and bc12075_Fad2a-278a2), and binding sites of ZFN specific primers (FAD2A.UnE.F1 and FAD2A.UnE.R1) and endogenous primers (FAD2A/2C.RB.UnE.F1 and FAD2A/2C.RB.UnE.R1).
FIG. 33: Shows a schematic showing binding sites of endogenous and transgene target primers used in the detection of transgene integration at FAD2A via perfect HDR.
FIG. 34: Is a schematic showing where Kpn1 restriction endonuclease sites would occur in a perfectly edited FAD2A locus, and where FAD2a 5', *hph* and FAD2A 3' Southern probes bind.
FIG. 35: Shows the location and size of Kpn1 fragments, FAD2A 5', hph, FAD2A 3' probes and expected outcomes of Southern analysis for plants that have integration of ETIP at FAD2A locus via HDR.

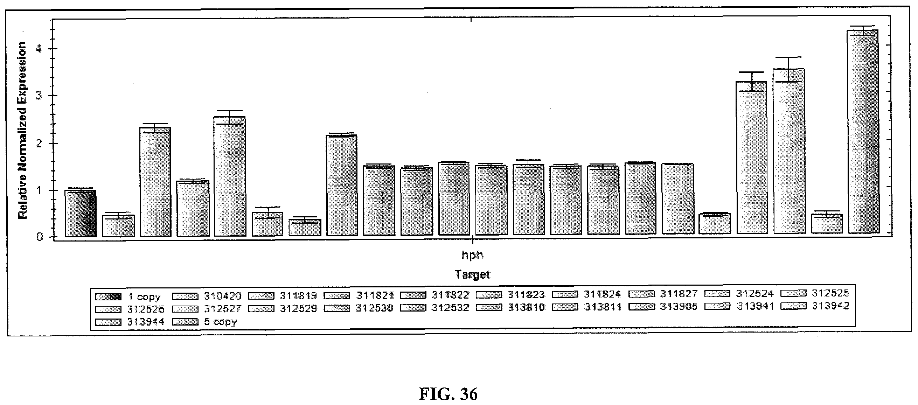

FIG. 36: Shows representative data output from copy number estimation qPCR. The left hand column represents data obtained from a known To transgenic plant with a single random transgene insert and is used as the calibrator sample to which all other samples are "normalized" against. The right hand column is a known To transgenic plant with 5 transgene integrations. The insert copy numbers for both plants was determined using Southern analysis. The remaining columns provide copy number estimates for the putative transgenic plants. The labels below the columns correspond with the columns in the graph and can be used to determine the estimated copy number for each transgenic plant. When using the software to estimate copy numbers, wild-type plants, non-transformed control plants, and plasmid only controls do not result in a copy number as they do not possess a Cq for both the *hph* and *HMG I*/*Y* target.

### MODE(S) FOR CARRYING OUT THE INVENTION

Transient transformation of protoplasts is a widely utilized tool in plant research that is swift and unproblematic. The technique can be used, for example, to monitor the regulation of promoter elements, to analyze gene expression or enzymatic activity in response to a variety of stimuli, to examine the roles of transcription factors or signal-transduction cascade components or to study the subcellular localization of proteins (Sheen, 2001; Yoo *et al.,* 2007). As opposed to stable transformation of plants (*Arabidopsis thaliana* being the most commonly used platform), which generally takes months and requires the use of a transfecting agent (usually *Agrobacterium tumefaciens*), transfection of protoplasts can be achieved in just one day and entails only raw DNA and either a chemical- or electroporation-based transfection method. Additionally, transient transformation analyses can overcome problems encountered with stable over-expression such as pleiotropic developmental effects or nonviability, when a cell-based assay is appropriate. However, due to the fact that protoplast transformation efficiency is never 100%, results can be convoluted by the non-transformed cells.

Transformation efficiencies are often low and variable (*e.g.* Cummins *et al.,* 2007; <10%) and depend on the employed method as well as properties of the protoplasts and DNA used. The present invention relates to the field of plant biotechnology, but can be used for all biological purposes. In particular, embodiments of the present invention relate to the generation of native or transgenic plant cell lines from a heterogeneous population of plant cells through flow cytometric sorting. Such plant cell line may either be a monocot or a dicot. As will be apparent for a skilled person, the invention also uses the plant cell line for the regeneration of whole fertile plants.

The present invention relates to FACS based sensitivity selection wherein there is better selection to select for successfully transformed cells. It has previously been reported that transformation of a population of plant cells such as a plant suspension culture frequently results in transgenic cultures that heterogeneous and inconsistent expression levels. The present invention is primarily concern with the provision of a plant based system. The ability to isolate and grow single cells has numerous possible applications. For example, methods outlined herein have utility in the improvement of processes related to the productivity of plant cell cultures. However, this application has broad applicability for all cells.

The present invention include the use of flow cytometric sorting, namely FACS technology to separate or isolate single, i.e. individualized protoplast that are prepared from a population of plant cells using materials and methods known in the art. These protoplasts can be transformed and are capable of 1) producing a fluorescent marker protein or polypeptide; 2) producing a desired product; and/or 3) surviving in the presence of a selection agent. Sorting criteria for FACS can be selected from the group comprising the genetic background (e.g., ploidy, aneuploidy), mutants, transgenics, gene exchange products, and fluorescence (autofluorescence (chloroplasts, metabolites), fluorescent proteins or enzyme mediation fluorescence). Any fluorescent protein may be used. A selection agent may or may not be used.

After separation or isolation of the single protoplasts by flow cytometric sorting, each single transformed protoplast is regenerated until the formation of a microcolony (microcallus) by co-cultivation. The plant source origin is not limited but is restricted to those lines, varieties and species whose protoplasts have the potential to regenerate until the formation of a microcolony or microcallus. The present invention will thus be applicable to all plant varieties and species for which a regeneration protocol has been established or will be provided. Thus, the present invention can be carried out with all plant varieties and species for which a regeneration portion has been established or will be provided for in the future.

The microcolony itself may be separated or removed from the feeder cell material and cultivated until the formation of a plant cell line.

Embodiments of the present invention can also include the generation of a callus tissue by 1) transferring the microcolony or microcallus to a solid cultivation medium and 2) cultivating the microcolony or microcallus in the presence of at least one selection agent until the formation of a transgenic callus tissue from which a transgenic plant cell line can be established by transferring the callus tissue to a liquid cultivation medium. The microcolony can also be removed or separated from the feeder cell material by mechanical means, i.e., by clone picking. In this case, no selection agent is needed and the cells comprised by the microcolony do not need to display resistance against any selection agent.

In some embodiments the cells can comprise a heterogeneous population of plant cells that are native or non-transgenic cells that, before being subjected to flow cytometric sorting and are stably or transiently transformed with at least one expression vector comprising at least one heterologous nucleic acid sequence which can be operably linked to a functional promoter, wherein said at least one heterologous nucleic acid sequence codes for a desired product. In additional embodiments the at least one heterologous nucleic acid sequence is operably linked to at least one functional promoter wherein the at least one heterologous nucleic acid sequence codes for a fluorescent marker protein or polypeptide and at least one heterologous nucleic acid sequence for resistance to a selection agent or for a desired product. Additional embodiments can include wherein the cells may additionally comprise a heterologous nucleic acid sequence that codes for a desired product to be accumulated in the transgenic plant cell line as provided.

In other embodiments the genome of the host cell can be expressed so that the recombinant protein or peptide can be modified by recombination, for example homologous recombination or heterologous recombination.

Any (transgenic) monoclonal or diclonal plant cell lines established can be treated or cultivated in the presence of precursors, inducers, hormones, stabilizers, inhibitors, RNAi/siRNA molecules, signaling compounds, enzymes and/or elicitors in addition to or instead of the vector suspension, for the production of recombinant proteins or metabolites.

Heterologous nucleic acids may encode genes of bacterial, fungal, plant or non-plant origin such as fusion proteins, and proteins of animal origin. Polypeptides produced may be utilized for producing polypeptides which can be purified therefrom for use elsewhere. Proteins that can be produced in a process of the invention include heterodimers, immunoglobulins, fusions antibodies and single chain antibodies. Furthermore, the above genes may be altered to produce proteins with altered characteristics.

Embodiments of the present invention include the ability to produce a large variety of proteins and polypeptides. These embodiments can also include methods for the production of at least one desired product selected from the group consisting of heterologous proteins or polypeptides, secondary metabolites, and markers. The method comprises to use the plant cell line as established according to the invention in order to produce and accumulate the at least one desired product which is subsequently obtained or isolated from the producing cells or from the cultivation medium.

Additional methods include methods of generating at least an extracellular heterologous protein comprising the steps of 1) stably introducing into a target cell comprised by the starting population of plant cells a first nucleic acid comprising the nucleotide sequence coding for the heterologous protein or desired product; 2) preparing protoplasts form plant suspension cells provided from said plant suspension culture, wherein the protoplasts are additionally transformed and capable of i) producing a fluorescent marker protein or polypeptide and ii) surviving in presence of a selection agent; 3) separating single transformed protoplasts by subjecting the preparation of protoplasts to FACS; 4) regenerating a separated single transformed protoplast until the formation of a microcolony or microcallus by co-cultivation in the presence of feeder cell material; 5) generating callus tissue by i) transferring the microcolony or microcallus to solid cultivation medium and ii) cultivating the microcolony or microcallus in the presence of at least one selection agent until the formation of a transgenic callus tissue; 6) establishing a transgenic plant cell line by transferring the callus tissue to liquid cultivation medium; 7) causing or permitting expression from the nucleic acid of the heterologous protein or desired product by providing appropriate cultivation conditions; and 8) harvesting the accumulated heterologous protein or desired product from the producing cells. Such isolation can be by entirely conventional means and may or may not entail partial or complete purification.

More than one gene may be used in each construct. Multiple vectors, each including one or more nucleotide sequences encoding heterologous protein of choice, may be introduced into the target cells as described herein or elsewhere. This can also be useful for producing multiple subunits of an enzyme.

The fluorescent marker protein or polypeptide can be a protein detectable by fluorescence such as GUS, fluorescent proteins such as GFP or DsRed, luciferase, etc. Preferably the reported is a non-invasive marker such as DsRed or GFP.

The techniques of this invention may be used to select for certain plants to be grown. Selection of a gene of interest may be handled in a number of ways. A large number of techniques are available for inserting DNA into a plant host cell. These techniques include transformation with T-DNA using Agrobacterium tumefaciens or Agrobacterium rhizogenes as transformation agent, fusion, injection, biolistics (microparticle bombardment), silicon carbide whiskers, aerosol beaming, PEG, or electroporation as well as other possible methods. If Agrobacteria are used for the transformation, the DNA to be inserted has to be cloned into special plasmids, namely either into an intermediate vector or into a binary vector. The intermediate vectors can be integrated into the Ti or Ri plasmid by homologous recombination owing to sequences that are homologous to sequences in the T-DNA. The Ti or Ri plasmid also comprises the vir region necessary for the transfer of the T-DNA. Intermediate vectors cannot replicate themselves in Agrobacteria. The intermediate vector can be transferred into Agrobacterium tumefaciens by means of a helper plasmid (conjugation). Binary vectors can replicate themselves both in E. coli and in Agrobacteria. They comprise a selection marker gene and a linker or polylinker which are framed by the right and left T-DNA border regions. They can be transformed directly into Agrobacteria (Holsters, 1978). The Agrobacterium used as host cell is to comprise a plasmid carrying a vir region. The vir region is necessary for the transfer of the T-DNA into the plant cell. Additional T-DNA may be contained. The bacterium so transformed is used for the transformation of plant cells. Plant explants can be cultivated advantageously with Agrobacterium tumefaciens or Agrobacterium rhizogenes for the transfer of the DNA into the plant cell. Whole plants can then be regenerated from the infected plant material (for example, pieces of leaf, segments of stalk, roots, but also protoplasts or suspension-cultivated cells) in a suitable medium, which may contain antibiotics or biocides for selection. The plants so obtained can then be tested for the presence of the inserted DNA. No special demands are made of the plasmids in the case of injection and electroporation. It is possible to use ordinary plasmids, such as, for example, pUC derivatives.

The transformed cells grow inside the plants in the usual manner. They can form germ cells and transmit the transformed trait(s) to progeny plants. Such plants can be grown in the normal manner and crossed with plants that have the same transformed hereditary factors or other hereditary factors. The resulting hybrid individuals have the corresponding phenotypic properties.

In some preferred embodiments of the invention, genes encoding proteins of interest are expressed from transcriptional units inserted into the plant genome. Preferably, said transcriptional units are recombinant vectors capable of stable integration into the plant genome and enable selection of transformed plant lines expressing mRNA encoding the proteins.

Once the inserted DNA has been integrated in the genome, it is relatively stable there (and does not come out again). It normally contains a selection marker that confers on the transformed plant cells resistance to a biocide or an antibiotic, such as kanamycin, G418, bleomycin, hygromycin, or chloramphenicol, inter alia. Plant selectable markers also typically can provide resistance to various herbicides such as glufosinate (e.g., PAT/bar), glyphosate (EPSPS), ALS-inhibitors (e.g., imidazolinone, sulfonylurea, triazolopyrimidine sulfonanilide, et al.), bromoxynil, HPPD-inhibitor resistance, PPO-inhibitors, ACC-ase inhibitors, and many others. The individually employed marker should accordingly permit the selection of transformed cells rather than cells that do not contain the inserted DNA. The gene(s) of interest are preferably expressed either by constitutive or inducible promoters in the plant cell. Once expressed, the mRNA is translated into proteins, thereby incorporating amino acids of interest into protein. The genes encoding a protein expressed in the plant cells can be under the control of a constitutive promoter, a tissue-specific promoter, or an inducible promoter.

Several techniques exist for introducing foreign recombinant vectors into plant cells, and for obtaining plants that stably maintain and express the introduced gene. Such techniques include the introduction of genetic material coated onto microparticles directly into cells (U.S. Pat. Nos. 4,945,050 to Cornell and 5,141,131 to DowElanco, now Dow AgroSciences, LLC). In addition, plants may be transformed using Agrobacterium technology, see U.S. Pat. Nos. 5,177,010 to University of Toledo; 5,104,310 to Texas A&M; European Patent Application 0131624B1; European Patent Applications 120516, 159418B1 and 176,112 to Schilperoot; U.S. Pat. Nos. 5,149,645, 5,469,976, 5,464,763 and 4,940,838 and 4,693,976 to Schilperoot; European Patent Applications 116718, 290799, 320500, all to Max Planck; European Patent Applications 604662 and 627752, and U.S. Pat. No. 5,591,616, to Japan Tobacco; European Patent Applications 0267159 and 0292435, and U.S. Pat. No. 5,231,019, all to Ciba Geigy, now Syngenta; U.S. Pat. Nos. 5,463,174 and 4,762,785, both to Calgene; and U.S. Pat. Nos. 5,004,863 and 5,159,135, both to Agracetus. Other transformation technology includes whiskers technology. See U.S. Pat. Nos. 5,302,523 and 5,464,765, both to Zeneca, now Syngenta. Other direct DNA delivery transformation technology includes aerosol beam technology. See U.S. Pat. No. 6,809,232. Electroporation technology has also been used to transform plants. See WO 87/06614 to Boyce Thompson Institute; U.S. Pat. Nos. 5,472,869 and 5,384,253, both to Dekalb; and WO 92/09696 and WO 93/21335, both to Plant Genetic Systems. Furthermore, viral vectors can also be used to produce transgenic plants expressing the protein of interest. For example, monocotyledonous plants can be transformed with a viral vector using the methods described in U.S. Pat. No. 5,569,597 to Mycogen Plant Science and Ciba-Geigy (now Syngenta), as well as U.S. Pat. Nos. 5,589,367 and 5,316,931, both to Biosource, now Large Scale Biology.

As mentioned previously, the manner in which the DNA construct is introduced into the plant host is not critical to this invention. Any method that provides for efficient transformation may be employed. For example, various methods for plant cell transformation are described herein and include the use of Ti or Ri-plasmids and the like to perform Agrobacterium mediated transformation. In many instances, it will be desirable to have the construct used for transformation bordered on one or both sides by T-DNA borders, more specifically the right border. This is particularly useful when the construct uses Agrobacterium tumefaciens or Agrobacterium rhizogenes as a mode for transformation, although T-DNA borders may find use with other modes of transformation. Where Agrobacterium is used for plant cell transformation, a vector may be used which may be introduced into the host for homologous recombination with T-DNA or the Ti or Ri plasmid present in the host. Introduction of the vector may be performed via electroporation, tri-parental mating and other techniques for transforming gram-negative bacteria which are known to those skilled in the art. The manner of vector transformation into the Agrobacterium host is not critical to this invention. The Ti or Ri plasmid containing the T-DNA for recombination may be capable or incapable of causing gall formation, and is not critical to said invention so long as the vir genes are present in said host.

In some cases where Agrobacterium is used for transformation, the expression construct being within the T-DNA borders will be inserted into a broad spectrum vector such as pRK2 or derivatives thereof as described in Ditta et al. (1980) and EPO 0 120 515. Included within the expression construct and the T-DNA will be one or more markers as described herein which allow for selection of transformed Agrobacterium and transformed plant cells. The particular marker employed is not essential to this invention, with the preferred marker depending on the host and construction used.

For transformation of plant cells using Agrobacterium, explants may be combined and incubated with the transformed Agrobacterium for sufficient time to allow transformation thereof. After transformation, the Agrobacteria are killed by selection with the appropriate antibiotic and plant cells are cultured with the appropriate selective medium. Once calli are formed, shoot formation can be encouraged by employing the appropriate plant hormones according to methods well known in the art of plant tissue culturing and plant regeneration. However, a callus intermediate stage is not always necessary. After shoot formation, said plant cells can be transferred to medium which encourages root formation thereby completing plant regeneration. The plants may then be grown to seed and said seed can be used to establish future generations. Regardless of transformation technique, the gene encoding a bacterial protein is preferably incorporated into a gene transfer vector adapted to express said gene in a plant cell by including in the vector a plant promoter regulatory element, as well as 3' non-translated transcriptional termination regions such as Nos and the like.

In addition to numerous technologies for transforming plants, the type of tissue that is contacted with the foreign genes may vary as well. Such tissue would include but would not be limited to embryogenic tissue, callus tissue types I, II, and III, hypocotyl, meristem, root tissue, tissues for expression in phloem, and the like. Almost all plant tissues may be transformed during dedifferentiation using appropriate techniques described herein.

As mentioned above, a variety of selectable markers can be used, if desired. Preference for a particular marker is at the discretion of the artisan, but any of the following selectable markers may be used along with any other gene not listed herein which could function as a selectable marker. Such selectable markers include but are not limited to aminoglycoside phosphotransferase gene of transposon Tn5 (Aph II) which encodes resistance to the antibiotics kanamycin, neomycin and G41; hygromycin resistance; methotrexate resistance, as well as those genes which encode for resistance or tolerance to glyphosate; phosphinothricin (bialaphos or glufosinate); ALS-inhibiting herbicides (imidazolinones, sulfonylureas and triazolopyrimidine herbicides), ACC-ase inhibitors (e.g., ayryloxypropionates or cyclohexanediones), and others such as bromoxynil, and HPPD-inhibitors (e.g., mesotrione) and the like.

In addition to a selectable marker, it may be desirous to use a reporter gene. In some instances a reporter gene may be used with or without a selectable marker. Reporter genes are genes that are typically not present in the recipient organism or tissue and typically encode for proteins resulting in some phenotypic change or enzymatic property. Examples of such genes are provided in Weising et al., 1988. Preferred reporter genes include the beta-glucuronidase (GUS) of the uidA locus of E. coli, the chloramphenicol acetyl transferase gene from Tn9 of E. coli, the green fluorescent protein from the bioluminescent jellyfish *Aequorea victoria,* and the luciferase genes from firefly *Photinus pyralis.* An assay for detecting reporter gene expression may then be performed at a suitable time after said gene has been introduced into recipient cells. A preferred such assay entails the use of the gene encoding beta-glucuronidase (GUS) of the uidA locus of *E. coli* as described by Jefferson et al. (1987), to identify transformed cells.

In addition to plant promoter regulatory elements, promoter regulatory elements from a variety of sources can be used efficiently in plant cells to express foreign genes. For example, promoter regulatory elements of bacterial origin, such as the octopine synthase promoter, the nopaline synthase promoter, the mannopine synthase promoter; promoters of viral origin, such as the cauliflower mosaic virus (35S and 19S), 35T (which is a re-engineered 35S promoter, see U.S. Pat. No. 6,166,302, especially Example 7E) and the like may be used. Plant promoter regulatory elements include but are not limited to ribulose-1,6-bisphosphate (RUBP) carboxylase small subunit (ssu), beta-conglycinin promoter, beta-phaseolin promoter, ADH promoter, heat-shock promoters, and tissue specific promoters. Other elements such as matrix attachment regions, scaffold attachment regions, introns, enhancers, polyadenylation sequences and the like may be present and thus may improve the transcription efficiency or DNA integration. Such elements may or may not be necessary for DNA function, although they can provide better expression or functioning of the DNA by affecting transcription, mRNA stability, and the like. Such elements may be included in the DNA as desired to obtain optimal performance of the transformed DNA in the plant. Typical elements include but are not limited to Adh-intron 1, Adh-intron 6, the alfalfa mosaic virus coat protein leader sequence, osmotin UTR sequences, the maize streak virus coat protein leader sequence, as well as others available to a skilled artisan. Constitutive promoter regulatory elements may also be used thereby directing continuous gene expression in all cells types and at all times (e.g., actin, ubiquitin, CaMV 35S, and the like). Tissue specific promoter regulatory elements are responsible for gene expression in specific cell or tissue types, such as the leaves or seeds (e.g., zein, oleosin, napin, ACP, globulin and the like) and these may also be used.

Promoter regulatory elements may also be active (or inactive) during a certain stage of the plant's development as well as active in plant tissues and organs. Examples of such include but are not limited to pollen-specific, embryo-specific, corn-silk-specific, cotton-fiber-specific, root-specific, seed-endosperm-specific, or vegetative phase-specific promoter regulatory elements and the like. Under certain circumstances it may be desirable to use an inducible promoter regulatory element, which is responsible for expression of genes in response to a specific signal, such as: physical stimulus (heat shock genes), light (RUBP carboxylase), hormone (Em), metabolites, chemical (tetracycline responsive), and stress. Other desirable transcription and translation elements that function in plants may be used. Numerous plant-specific gene transfer vectors are known in the art.

Plant RNA viral based systems can also be used to express bacterial protein. In so doing, the gene encoding a protein can be inserted into the coat promoter region of a suitable plant virus which will infect the host plant of interest. The protein can then be expressed thus providing protection of the plant from herbicide damage. Plant RNA viral based systems are described in U.S. Pat. No. 5,500,360 to Mycogen Plant Sciences, Inc. and U.S. Pat. Nos. 5,316,931 and 5,589,367 to Biosource.

Means of further increasing tolerance or resistance levels. It is shown herein that plants of the subject invention can be imparted with novel herbicide resistance traits without observable adverse effects on phenotype including yield. Such plants are within the scope of the subject invention. Plants exemplified and suggested herein can withstand 2x, 3x 4x and 5x typical application levels, for example, of at least one subject herbicide. Improvements in these tolerance levels are within the scope of this invention. For example, various techniques are known in the art, and can foreseeably be optimized and further developed, for increasing expression of a given gene.

One such method includes increasing the copy number of the subject genes (in expression cassettes and the like). Transformation events can also be selected for those having multiple copies of the genes.

Strong promoters and enhancers can be used to "supercharge" expression. Examples of such promoters include the preferred 35T promoter which uses 35S enhancers. 35S, maize ubiquitin, Arabidopsis ubiquitin, A.t. actin, and CSMV promoters are included for such uses. Other strong viral promoters are also preferred. Enhancers include 4 OCS and the 35S double enhancer. Matrix attachment regions (MARs) can also be used to increase transformation efficiencies and transgene expression, for example.

Shuffling (directed evolution) and transcription factors can also be used for embodiments according to the subject invention.

Variant proteins can also be designed that differ at the sequence level but that retain the same or similar overall essential three-dimensional structure, surface charge distribution, and the like. See e.g. U.S. Pat. No. 7,058,515; Larson et al., Protein Sci. 2002 11: 2804-2813, "Thoroughly sampling sequence space: Large-scale protein design of structural ensembles"; Crameri et al., Nature Biotechnology 15, 436-438 (1997), "Molecular evolution of an arsenate detoxification pathway by DNA shuffling"; Stemmer, W. P. C. 1994, DNA shuffling by random fragmentation and reassembly: in vitro recombination for molecular evolution, Proc. Natl. Acad. Sci. USA 91: 10747-10751; Stemmer, W. P. C. 1994, Rapid evolution of a protein in vitro by DNA shuffling, Nature 370: 389-391; Stemmer, W. P. C. 1995, Searching sequence space. Bio/Technology 13: 549-553; Crameri, A., Cwirla, S, and Stemmer, W. P. C. 1996, Construction and evolution of antibody-phage libraries by DNA shuffling, Nature Medicine 2: 100-103; and Crameri, A., Whitehorn, E. A., Tate, E. and Stemmer, W. P. C., 1996, Improved green fluorescent protein by molecular evolution using DNA shuffling, Nature Biotechnology 14: 315-319.

The activity of recombinant polynucleotides inserted into plant cells can be dependent upon the influence of endogenous plant DNA adjacent the insert. Thus, another option is taking advantage of events that are known to be excellent locations in a plant genome for insertions. See e.g. WO 2005/103266 A1, relating to cry1F and cry1Ac cotton events; FAD2, FAD3, wherein genes such as AAD1 or AAD12 or others can be substituted in those genomic loci in place of such inserts. Thus, targeted homologous recombination, for example, can be used according to the subject invention. This type of technology is the subject of, for example, WO 03/080809 A2 and the corresponding published U.S. application (USPA 20030232410), relating to the use of zinc fingers for targeted recombination. The use of recombinases (cre-10xand flp-frt for example) is also known in the art.

Computational design of 5' or 3' UTR most suitable for synthetic hairpins can also be conducted within the scope of the subject invention. Computer modeling in general, as well as gene shuffling and directed evolution, are discussed elsewhere herein. More specifically regarding computer modeling and UTRs, computer modeling techniques for use in predicting/evaluating 5' and 3' UTR derivatives of the present invention include, but are not limited to: MFold version 3.1 available from Genetics Corporation Group, Madison, Wis. (see Zucker et al., Algorithms and Thermodynamics for RNA Secondary Structure Prediction: A Practical Guide. In RNA Biochemistry and Biotechnology, 11-43, J. Barciszewski & B. F. C. Clark, eds., NATO ASI Series, Kluwer Academic Publishers, Dordrecht, NL, (1999); Zucker et al., Expanded Sequence Dependence of Thermodynamic Parameters Improves Prediction of RNA Secondary Structure. J. Mol. Biol. 288, 911-940 (1999); Zucker et al., RNA Secondary Structure Prediction. In Current Protocols in Nucleic Acid Chemistry, S. Beaucage, D. E. Bergstrom, G. D. Glick, and R. A. Jones eds., John Wiley & Sons, New York, 11.2.1-11.2.10, (2000)), COVE (RNA structure analysis using covariance models (stochastic context free grammar methods)) v. 2.4.2 (Eddy & Durbin, Nucl. Acids Res. 1994, 22: 2079-2088) which is freely distributed as source code and which can be downloaded by accessing the website genetics.wust1.edu/eddy/software/, and FOLDALIGN, also freely distributed and available for downloading at the website bioinf.au.dk. FOLDALIGN/ (see Finding the most significant common sequence and structure motifs in a set of RNA sequences. J. Gorodkin, L. J. Heyer and G. D. Stormo. Nucleic Acids Research, Vol. 25, no. 18 pp 3724-3732, 1997; Finding Common Sequence and Structure Motifs in a set of RNA Sequences. J. Gorodkin, L. J. Heyer, and G. D. Stormo. ISMB 5;120-123, 1997).

Embodiments of the subject invention can be used in conjunction with naturally evolved or chemically induced mutants (mutants can be selected by screening techniques, then transformed with other genes). Plants of the subject invention can be combined with various resistance genes and/or evolved resistance genes. Traditional breeding techniques can also be combined with the subject invention to powerfully combine, introgress, and improve selection of traits.

All references, including publications, patents, and patent applications, cited and discussed herein are provided solely for their disclosure prior to the filing date of the present application.

### EXAMPLES

The following Examples are provided to illustrate certain particular features and/or aspects. These Examples should not be construed to limit the disclosure to the particular features or aspects described.

### EXAMPLE 1: IDENTIFICATION OF PARALOGOUS FAD2 AND FAD3 TARGET SEQUENCES FROM A BACTERIAL ARTIFICIAL CHROMOSOME LIBRARY

### BAC CONSTRUCTION

A Bacterial Artificial Chromosome (BAC) library was sourced from a commercial vendor (Amplicon Express, Pullman, WA). The BAC library consisted of 110,592 BAC clones containing high molecular weight genomic DNA (gDNA) fragments isolated from *Brassica napus* L. var. DH10275. The gDNA was digested with either the *BamHI* or *HinDIII* restriction enzyme. Isolated gDNA fragments of about 135 Kbp were ligated into the pCC1BAC vector (Epicentre, Madison, WI) and transformed into *Escherichia coli* str. DH10B (Invitrogen). The BAC library was made up of an even number of BAC clones that were constructed using the two different restriction enzymes. As such, the *Hind III* constructed BAC library consisted of 144 individual 384-well plates. Likewise, the *BamHI* constructed BAC library consisted of 144 individual 384-well plates. A total of 110,592 BAC clones were isolated and arrayed into 288 individual 384-well plates. Each of the 288 individual 384 well plates were provided by the vendor as a single DNA extraction for rapid PCR based screening. The resulting BAC library covers approximately 15 Gbp of gDNA, which corresponds to a 12-fold genome coverage of *Brassica napus* L. var. DH10275genome (estimate of the *Brassica napus* L. genome is ca. 1.132 Gbp as described in Johnston et al. (2005) Annals of Botany 95:229-235).

### SEQUENCE ANALYSIS OF FAD2 CODING SEQUENCES ISOLATED FROM THE BAC LIBRARY

The constructed BAC library was used to isolate FAD2 gene coding sequences. Sequencing experiments were conducted to identify the specific gene sequences of four FAD2 gene paralogs from *Brassica napus* L. var. DH10275.

The FAD2 gene sequence was initially identified within the model species *Arabidopsis thaliana.* The gene sequence is listed in Genbank as Locus Tag: At3g12120. Comparative genomic relationships between the model plant species *Arabidopsis thaliana* and the diploid *Brassica rapa,* one of the progenitors of the tetraploid *Brassica napus,* have been previously described. (Schranz et al. (2006) Trends in Plant Science 11(11):535-542). With specific relation to the FAD2 gene the comparative analysis predicted that 3-4 copies of the gene may occur within the diploid *Brassica* genome. Additional genetic mapping studies were completed by Scheffler et al. (1997) Theoretical and Applied Genetics 94; 583-591. The results of these genetic mapping studies indicated that four copies of the FAD2 gene were present in *Brassica napus.*

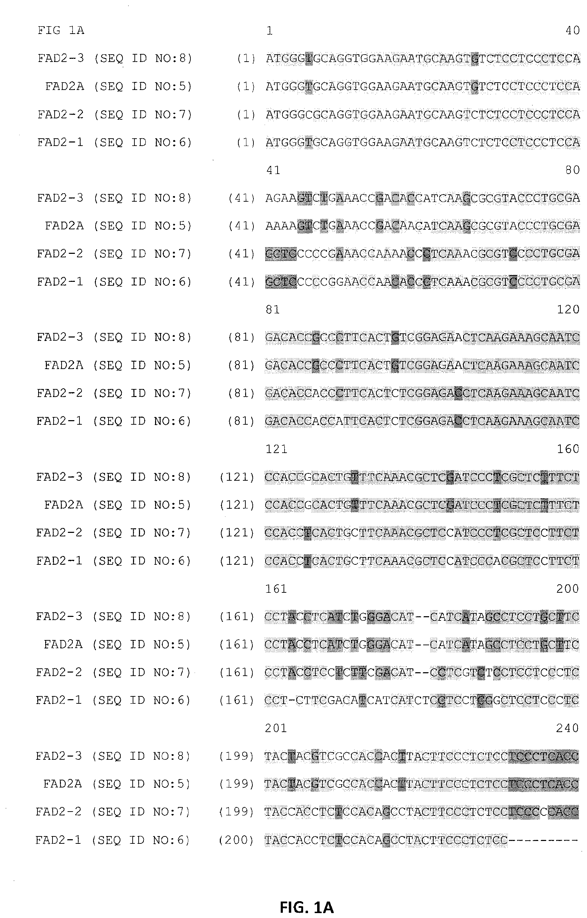

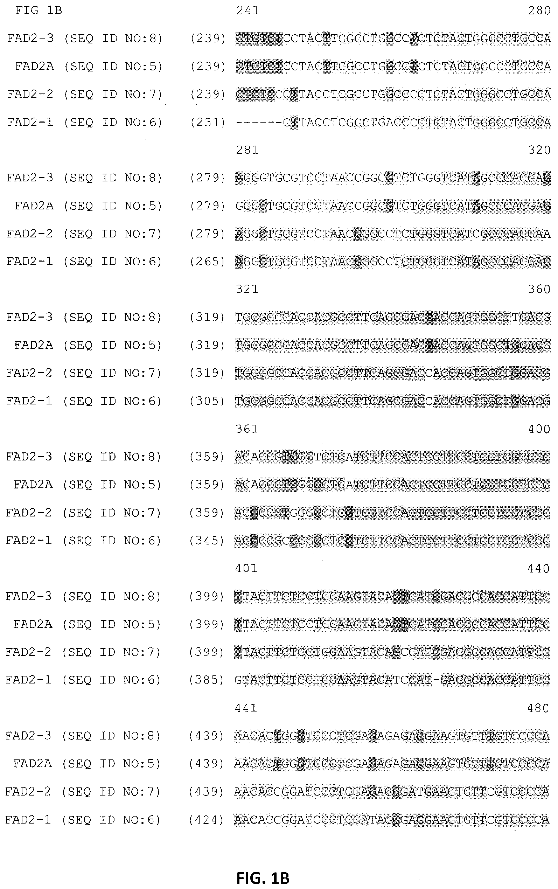

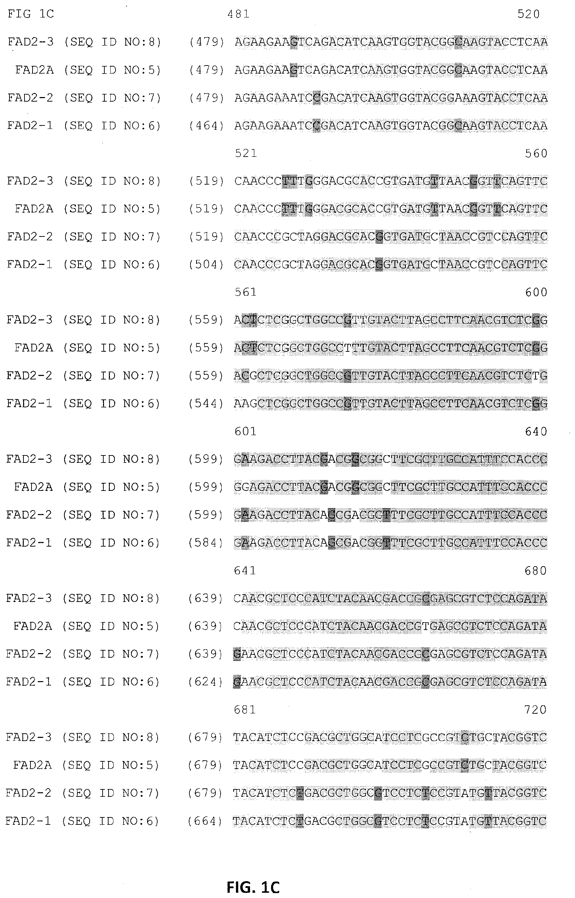

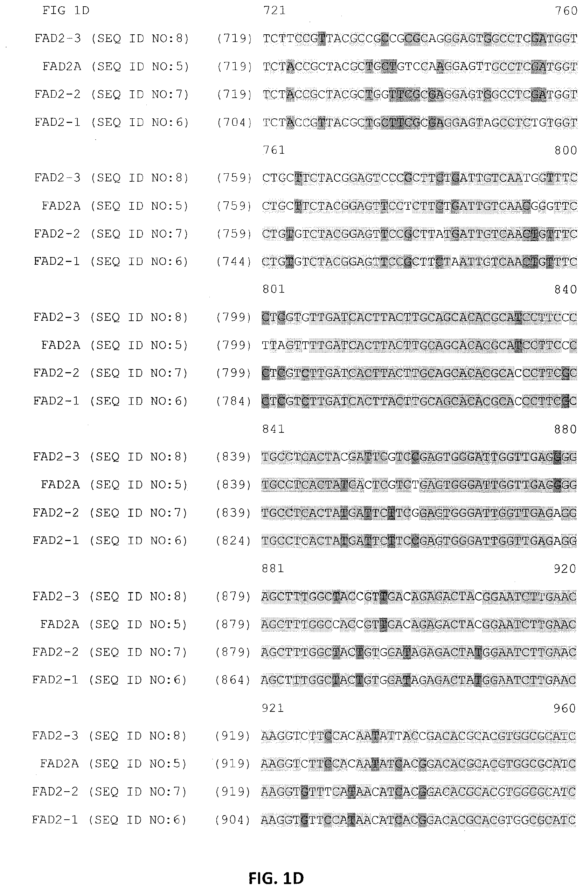

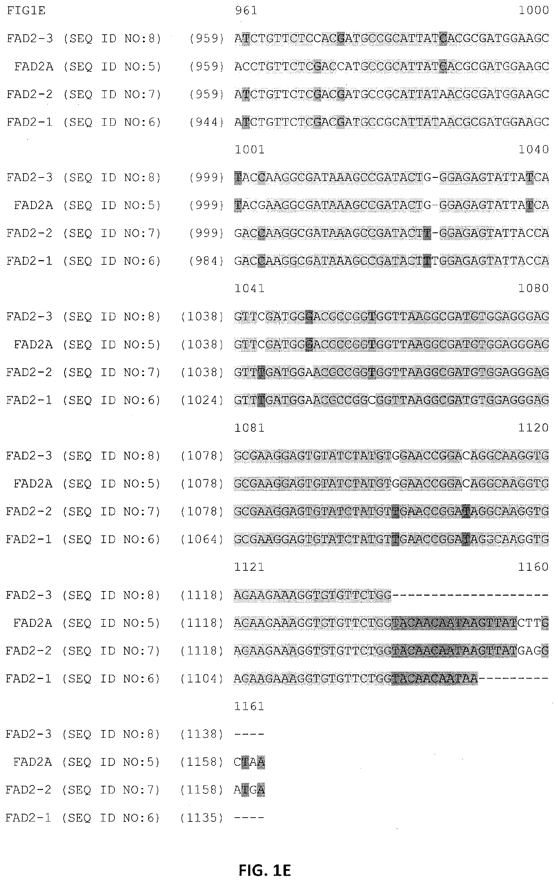

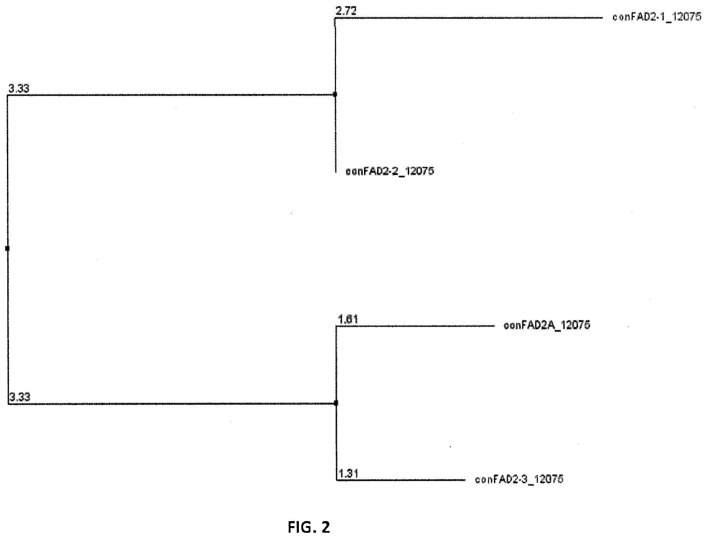

Sequencing analysis of the BAC library which was constructed from *B. napus* L. var. DH12075 resulted in the isolation of four BAC sequences (SEQ ID NO:1, SEQ ID NO:2, SEQ ID NO:3, and SEQ ID NO:4) from which the coding sequences for the FAD2A (SEQ ID NO:5), FAD2-1 (SEQ ID NO:6), FAD2-2 (SEQ ID NO:7), and FAD2-3(SEQ ID NO:8) genes were determined. The FAD2A, FAD2-1, FAD2-2, and FAD2-3 gene sequences were identified and genetically mapped. Sequence analysis of the four FAD2 genes was conducted using a sequence alignment program and a neighbor-joining tree using percentage of identity. The sequence alignment was made via the ALIGNX® program from the Vector NTI Advance 11.0 computer program (Life Technologies, Carlsbad, CA) and is shown in FIG. 1. ALIGNX® uses a modified **Clustal W algorithm** to generate multiple sequence alignments of either protein or nucleic acid sequences for similarity comparisons and for annotation. The neighbor-joining tree was created with JALVIEW v2.3® software and is shown in FIG. 2. (Waterhouse et al. (2009) Bioinformatics 25 (9) 1189-1191). As shown in FIG. 2, the analysis of the isolated sequences indicated that the FAD2A and FAD2-3 sequences shared high levels of sequence similarity and that, likewise, FAD2-1 and FAD2-2 shared high levels of sequence similarity. The four sequences can be categorized in two clades, wherein FAD2A and FAD2-3 comprise a first clade, and FAD2-1 and FAD2-2 comprise a second clade.

Next, the newly isolated FAD2 sequences from *Brassica napus* were used to BLAST genomic libraries isolated from a *Brassica rapa* genomic BAC library and *Brassica oleracea* shotgun genomic sequence reads. Both, *Brassica rapa* and *Brassica oleracea* are diploid progenitors of *Brassica napus* which is an amphidiploid species (AC genome, n = 19). *Brassica napus* derived from a recent hybridization event between *Brassica rapa* (A sub-genome, n = 10) and *Brassica oleracea* (C sub-genome, n = 9). The diploid progenitor sequences were compared to the four different FAD2 coding sequences isolated from *Brassica napus* using a BLASTn analysis. This sequence analysis identified specific, annotated gene sequences from *Brassica rapa* and *Brassica oleracea* which shared the highest sequence similarity to the newly discovered *Brassica napus* FAD2 sequences. Table 1 lists the newly identified FAD2 coding sequence and the corresponding progenitor reference sequence accession number and source organism.

**Table 1: FAD2 sequences from Brassica napus and the corresponding progenitor organism and related FAD sequence accession number.**

| **Isolated gene sequence** | **Progenitor organism and sequence accession number** | |
|---|---|---|
| FAD2A | *B.rapa* | Genbank Accession No: KBrB063G23 (A05) |
| FAD2-3 | *B.oleracea* | Genbank Accession No: GSS23580801* |
| FAD2-1 | *B.rapa* | Genbank Accession No: KBrB130I19 |
| FAD2-2 | *B.oleracea* | Genbank Accession No: GSS 17735412 |

| | | |
|---|---|---|
| *The Genbank sequence entry was edited | | |

The FAD2 genes exist in the *Brassica napus* genome as two copies of each gene per sub-genome. One copy of each gene is located on the A sub-genome, and likewise one copy of each gene is located on the C sub-genome. New naming conventions are described to indicate which sub-genome that each gene is located on. The high levels of sequence similarity between the four different FAD2 coding sequences isolated from the *Brassica napus* BAC genomic DNA library and the progenitor sequence data suggest that FAD2-3 is a duplicate of the FAD2 sequence from the C sub-genome and could be relabeled as FAD2C; FAD2-1 is a duplicate of the FAD2 sequence from the A sub-genome and could therefore be labeled as FAD2A'; and finally, FAD2-2 is a second copy that was duplicated from the FAD2 sequence of the C sub-genome and could be labeled as FAD2C'.

### SEQUENCE ANALYSIS OF FAD3 CODING SEQUENCES ISOLATED FROM THE BAC LIBRARY

The constructed BAC library was used to isolate FAD3 gene coding sequences. Sequencing experiments were conducted to identify the specific gene sequences of five FAD3 gene paralogs from *Brassica napus* L. var. DH10275.

The FAD3 gene sequence was initially identified within the model species *Arabidopsis thaliana.* The gene sequence is listed in Genbank as Locus Tag: At2g29980. Comparative genomic relationships between the model plant species *Arabidopsis thaliana* and the diploid *Brassica rapa,* one of the progenitors of the tetraploid *Brassica napus,* have been previously described. (Schranz et al. (2006) Trends in Plant Science 11(11):535-542). With specific relation to the FAD gene the comparative analysis predicted that 3-4 copies of the gene may occur within the diploid *Brassica* genome. Additional genetic mapping studies were completed by Scheffler et al. (1997) Theoretical and Applied Genetics 94; 583-591. The results of these genetic mapping studies indicated that six copies of the FAD3 gene were present in *Brassica napus.*

Previous sequencing efforts focused on the FAD3 genes from *Brassica napus* had identified and genetically mapped both A and C genome specific copies (Hu et al., (2006) Theoretical and Applied Genetics, 113(3): 497-507). A collection of EST sequences from seed specific cDNA libraries had previously been constructed and sequenced from the plant line DH12075 by Andrew Sharpe of Agriculture and Agri-food Canada, 107 Science Place, Saskatoon, Saskatchewan. As a collection of ESTs from the doubled haploid canola plant DH12075 full length gene sequences were not available, moreover the indications of sequence quality and confidence of correctly called nucleotides was also not available. Consequently, sequence variation between different FAD gene sequence reads could not be unequivocally attributed to different gene copies of the various paralogs of the FAD3 gene family, nor was the genomic sequence available. However, when a combined sequence analysis was performed with the ESTs as well as the two FAD3A and FAD3C full length gene sequences described in Hu *et al.,* (2006), ESTs that matched both of the genes were identified along with an additional 3 haplotypes. As a result, a total of six unique haplotypes of FAD3 were identified. Following the assembly of all available data for the various FAD3 haplotypes, high levels of exon sequence divergence in exon 1 was identified. The divergence of the FAD3 sequence in exon 1 was identified as an opportunity which could be utilized for the design of gene/allele specific PCR primers. In addition, exons were identified that were either minimally differentiated between haplotypes (*e.g*., exons 5, 6, 7 and 8 had 1-3 bp that varied between FAD3A and FAD3C) or that were devoid of sequence variation (*e.g*., exons 2 and 3).

Sequencing analysis of the BAC library which was constructed from *B. napus* L. var. DH12075 resulted in the isolation of six BAC sequences (SEQ ID NO:9, SEQ ID NO:10, SEQ ID NO:11, SEQ ID NO:12, SEQ ID NO:13, and SEQ ID NO:14) from which the coding sequences for the FAD3A (SEQ ID NO:15), FAD3A' (SEQ ID NO:16), FAD3A" (SEQ ID NO:17), FAD3C (SEQ ID NO:18), FAD3C" (SEQ ID NO:19), and FAD3C' (SEQ ID NO:20) genes were determined. The FAD3A, FAD3A', FAD3A", FAD3C, FAD3C", and FAD3C' gene sequences were identified and genetically mapped.

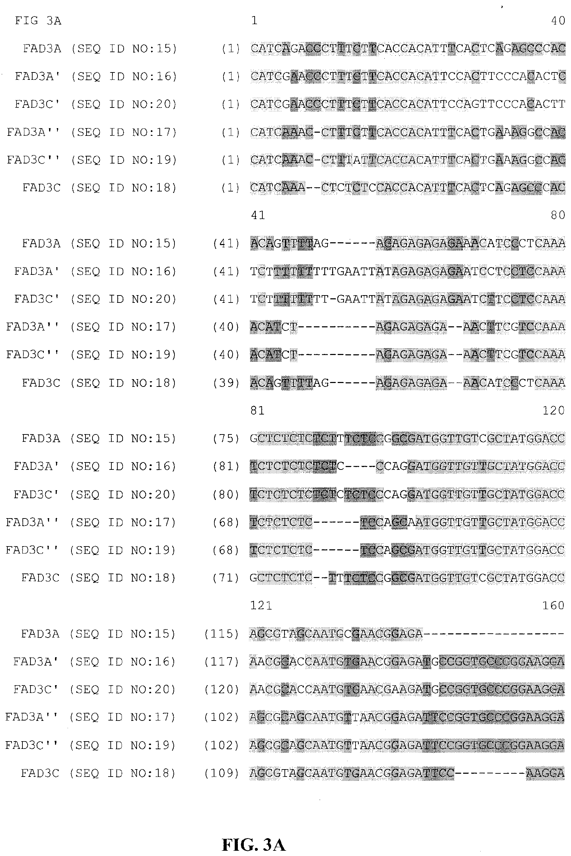

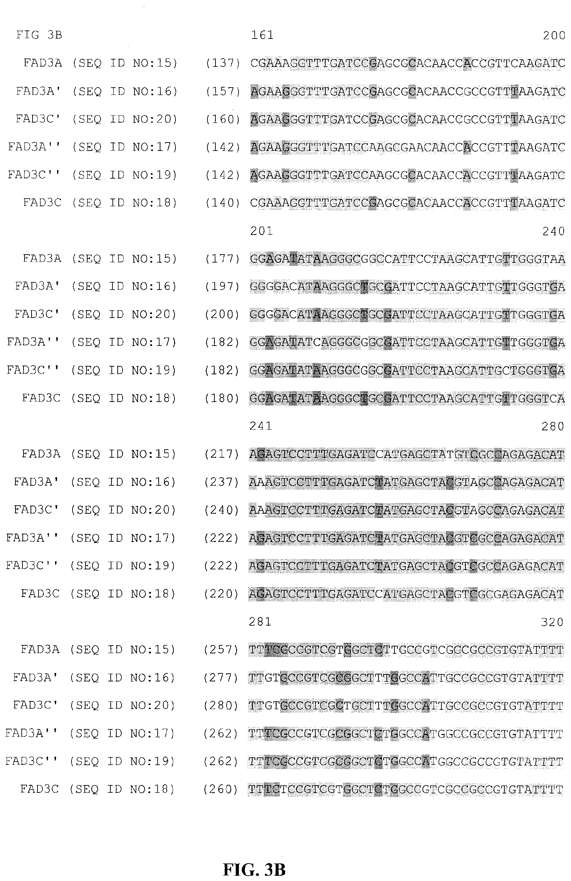

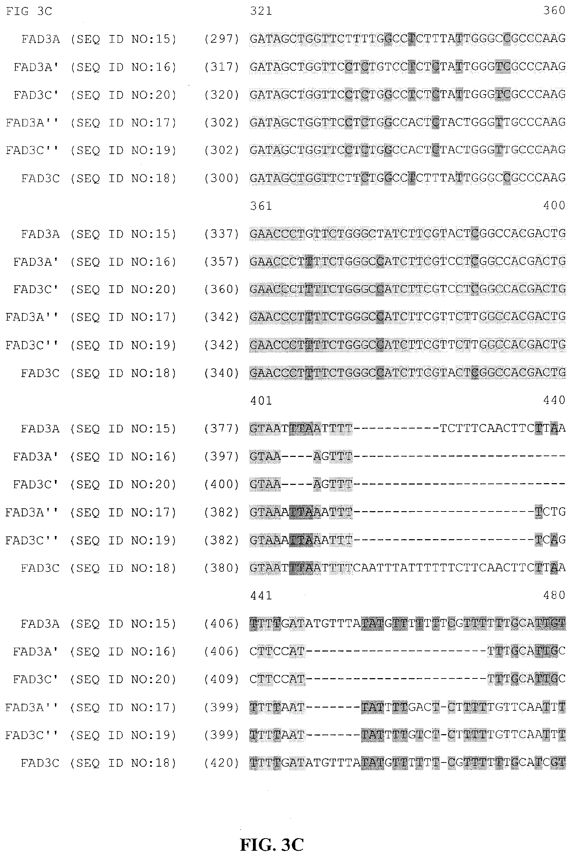

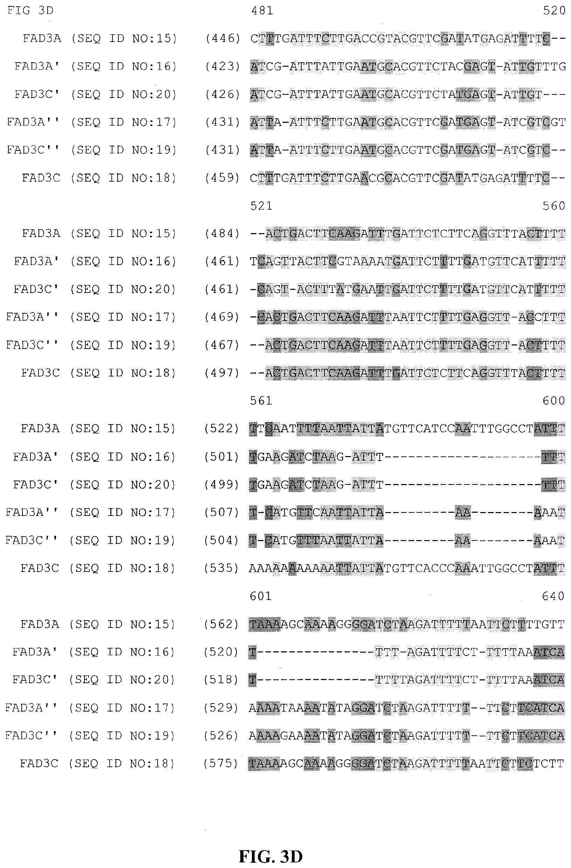

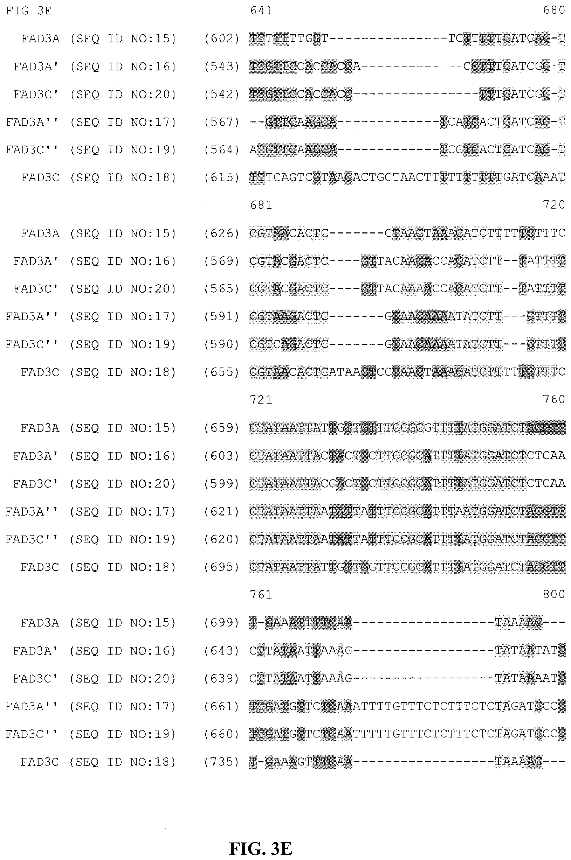

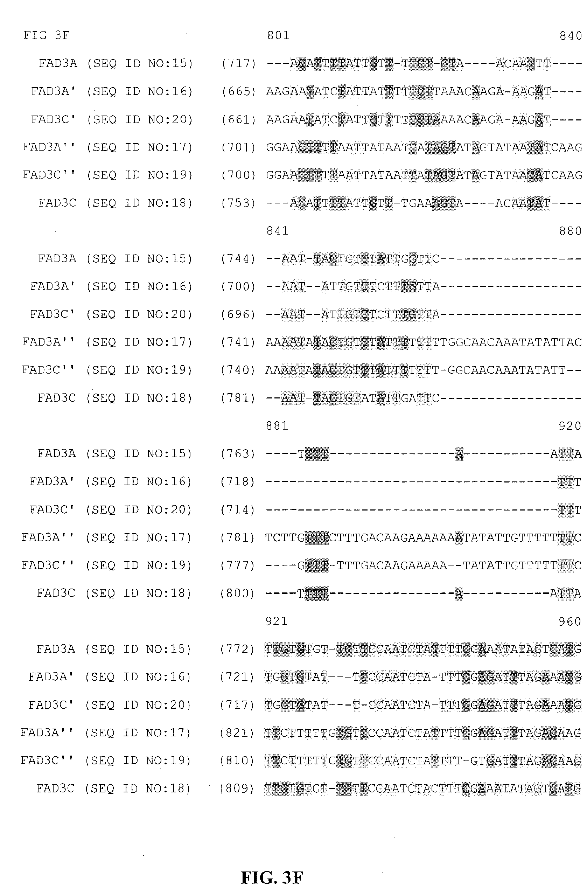

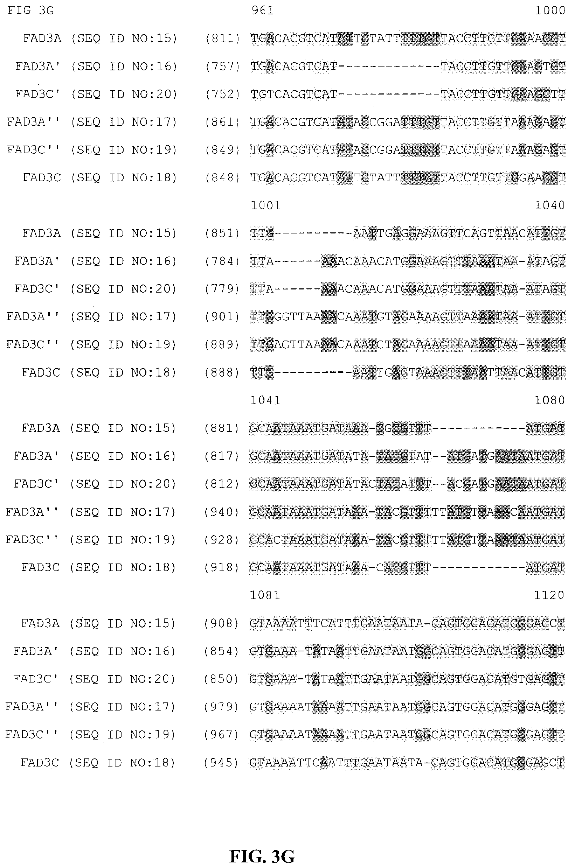

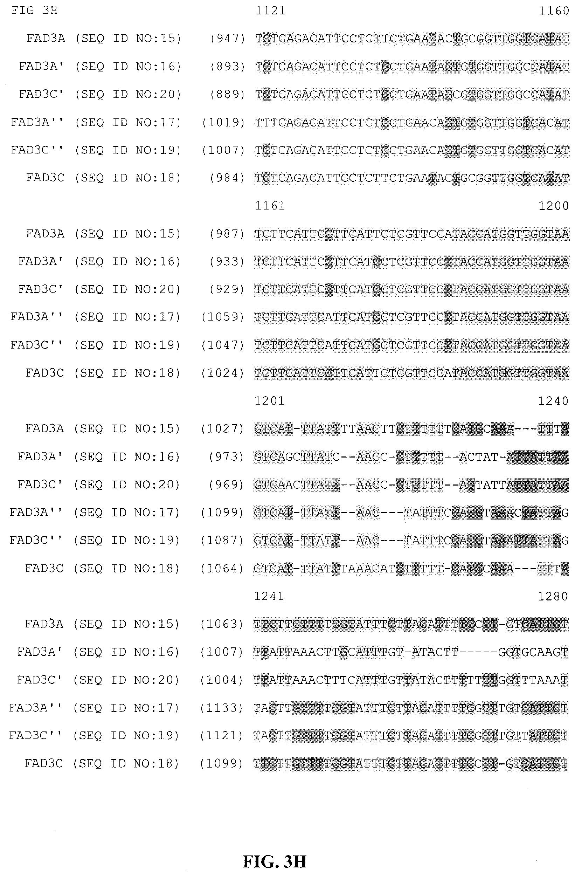

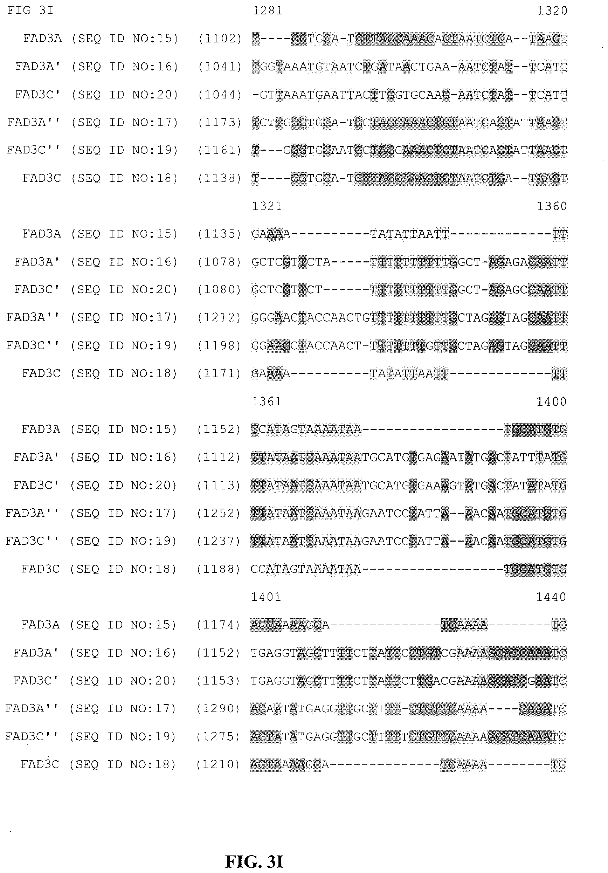

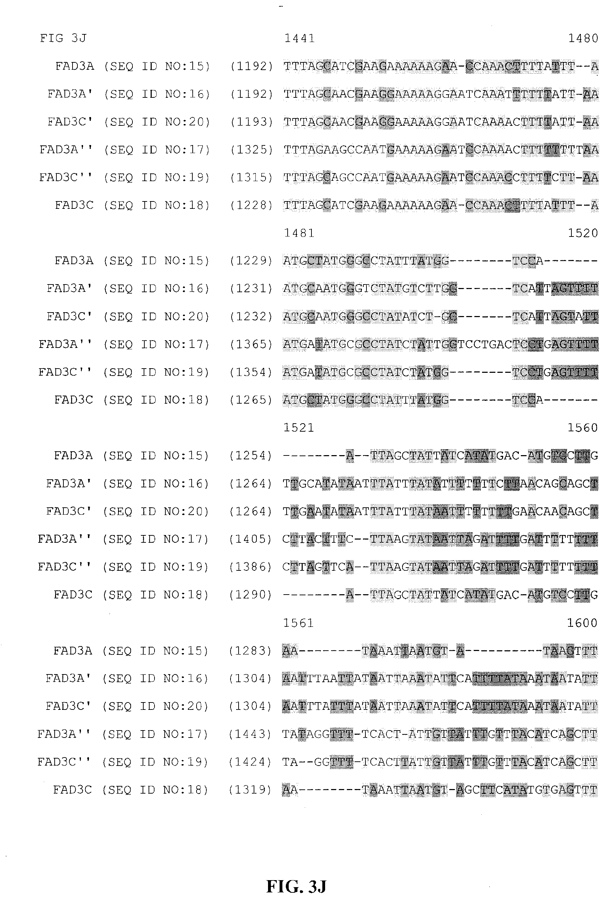

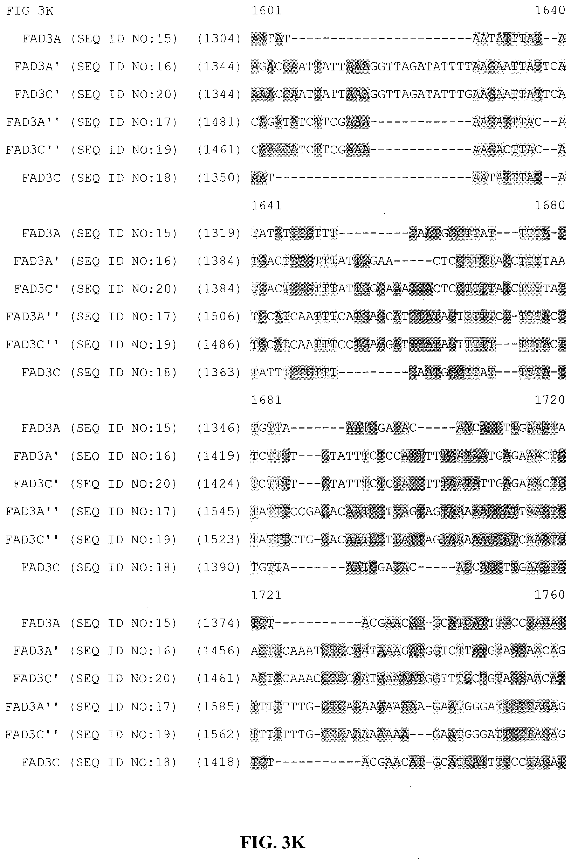

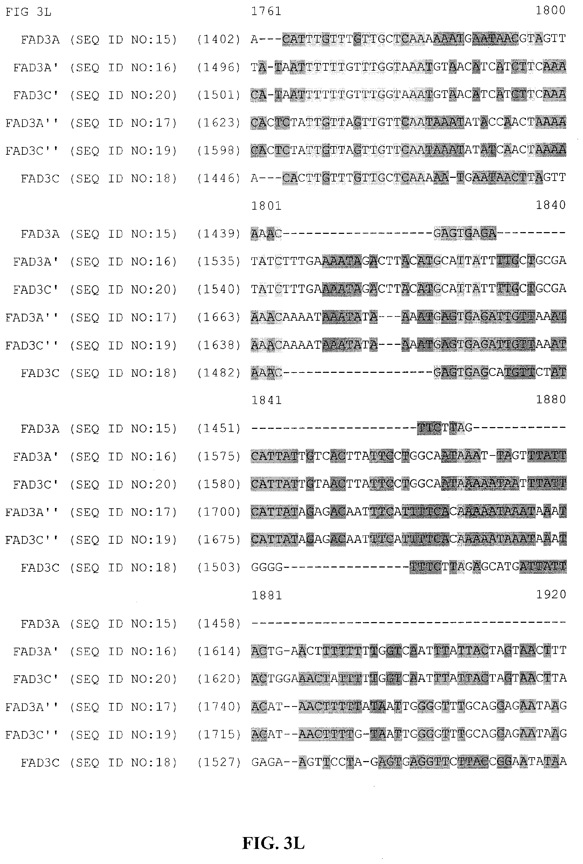

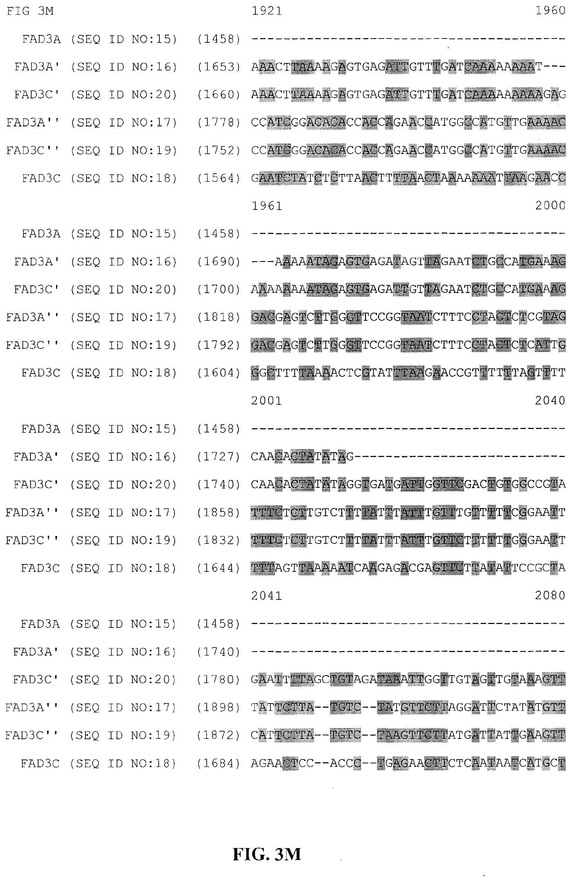

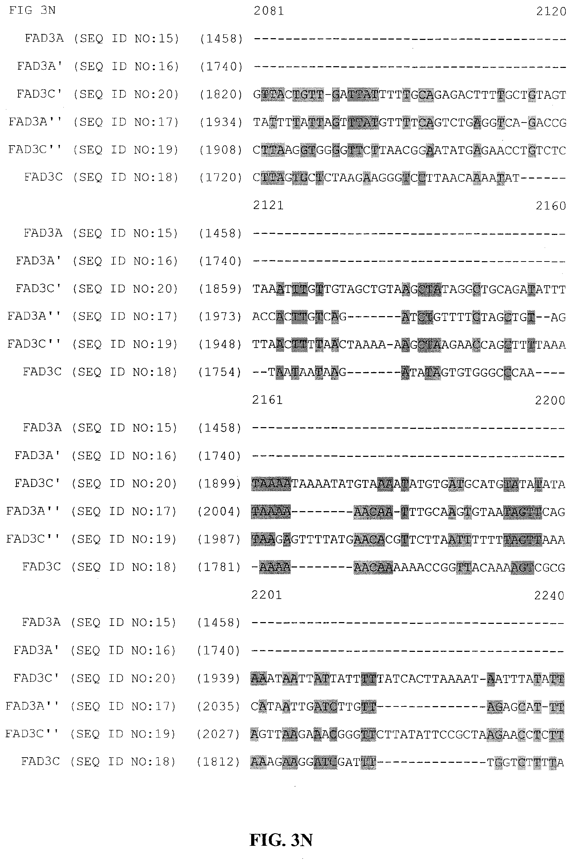

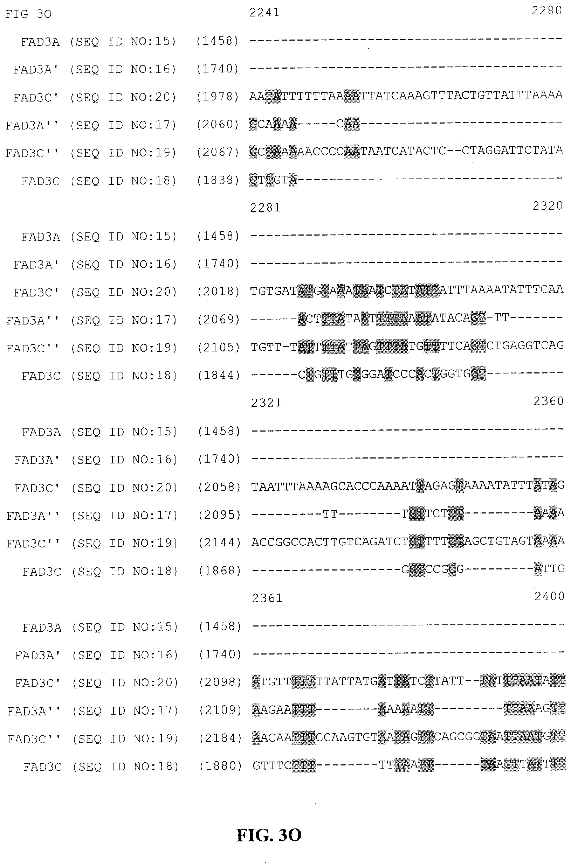

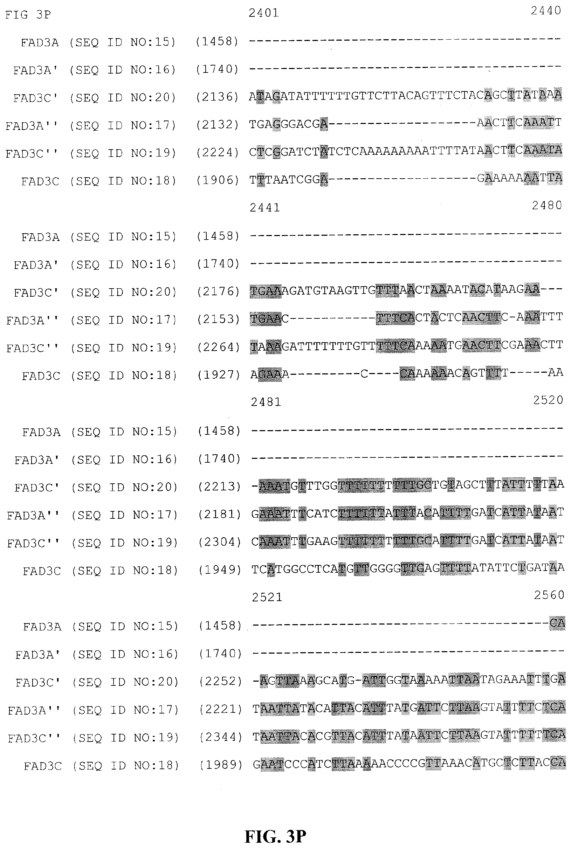

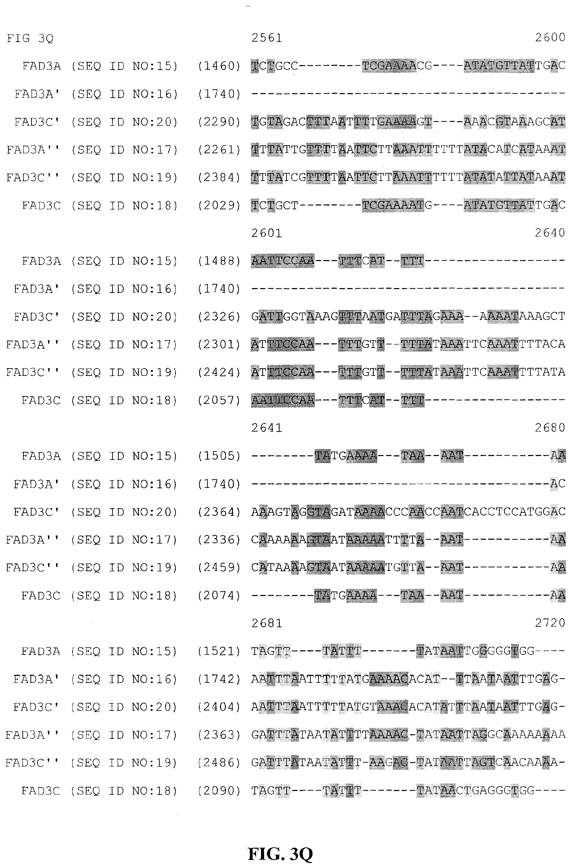

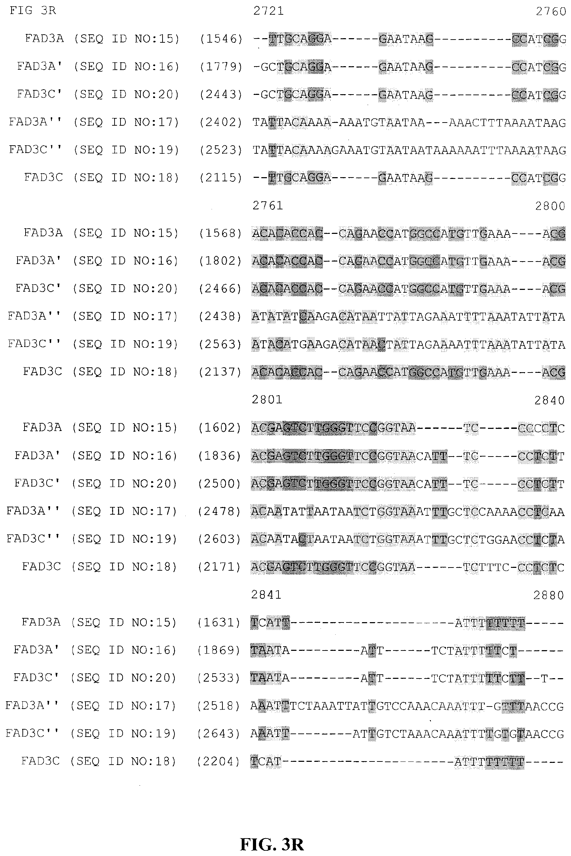

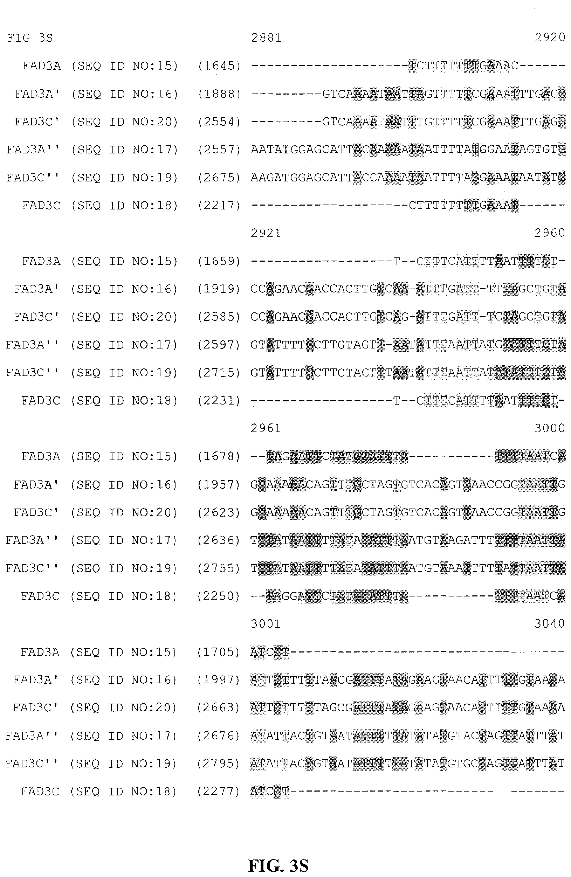

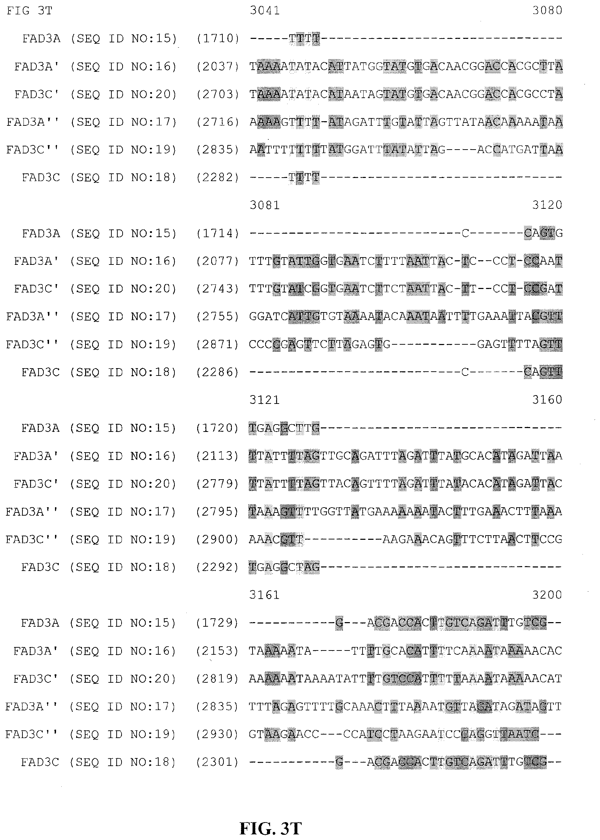

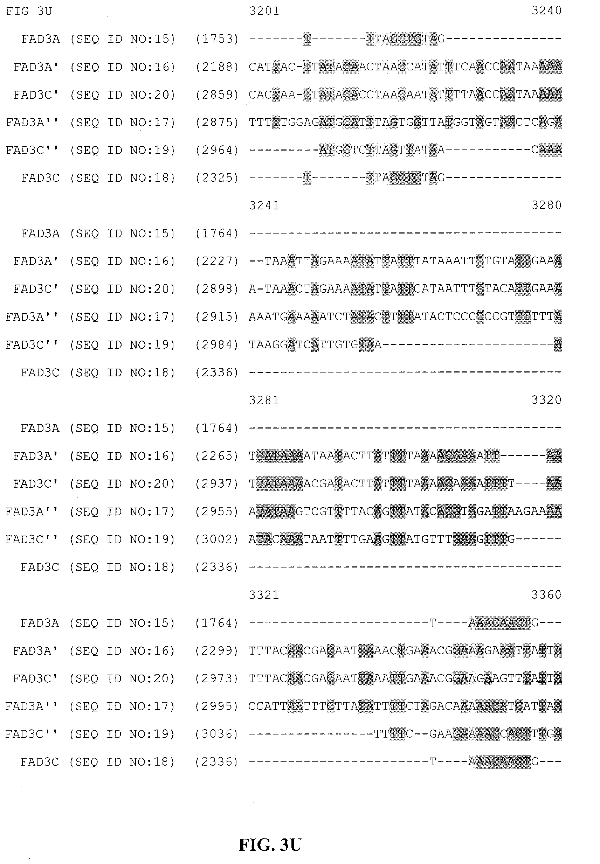

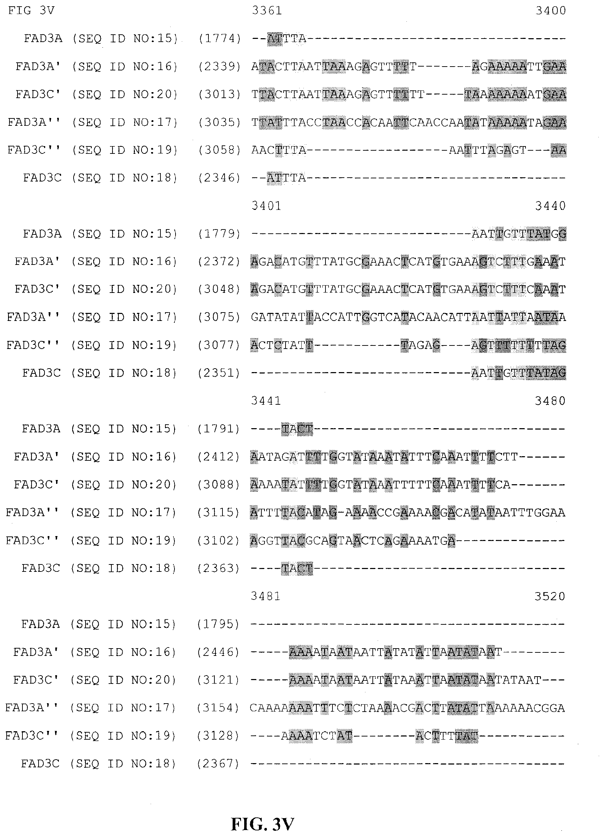

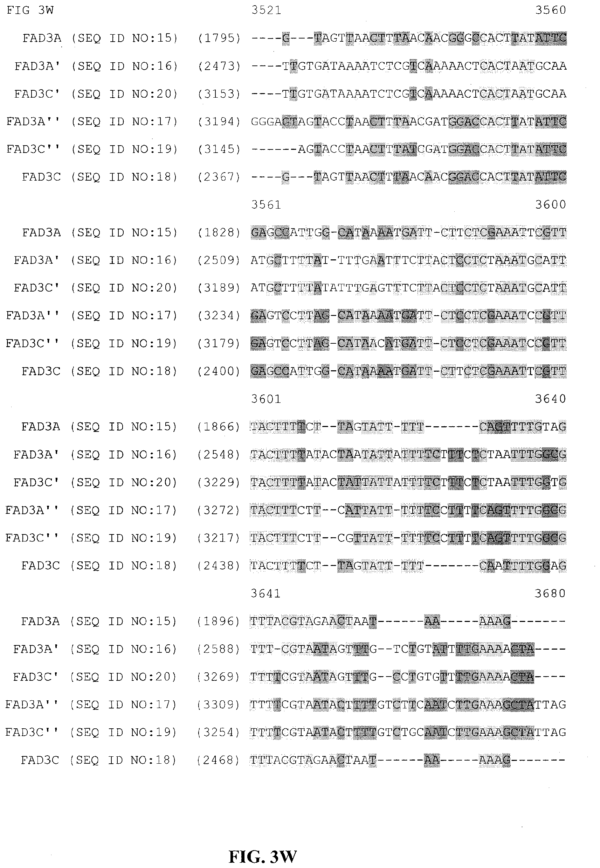

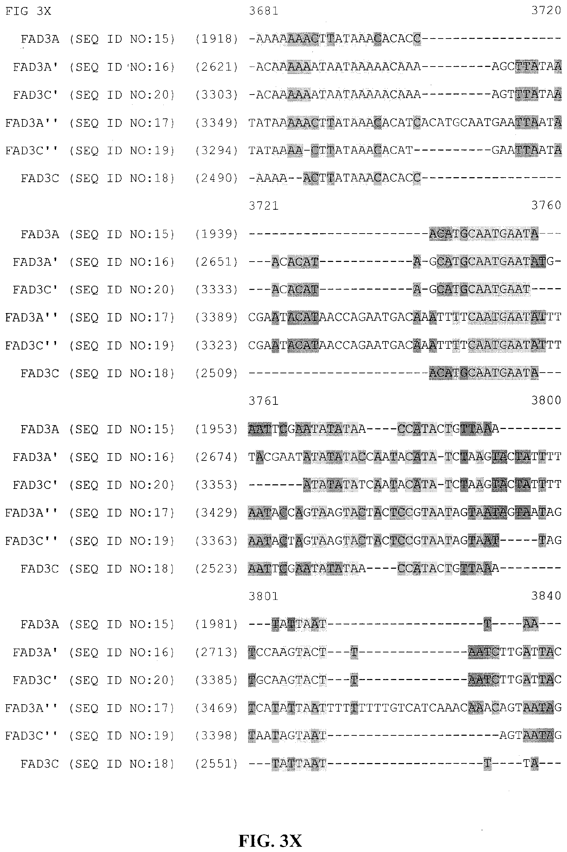

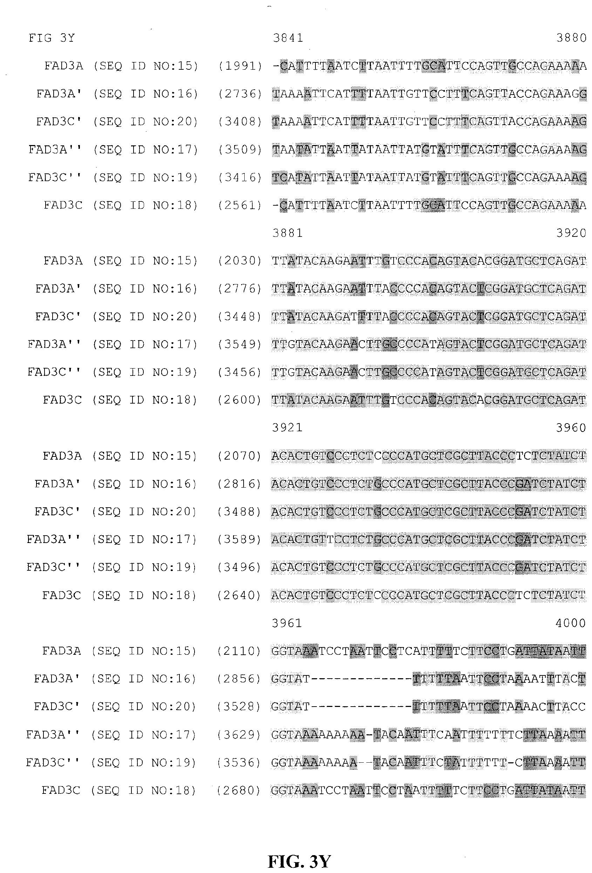

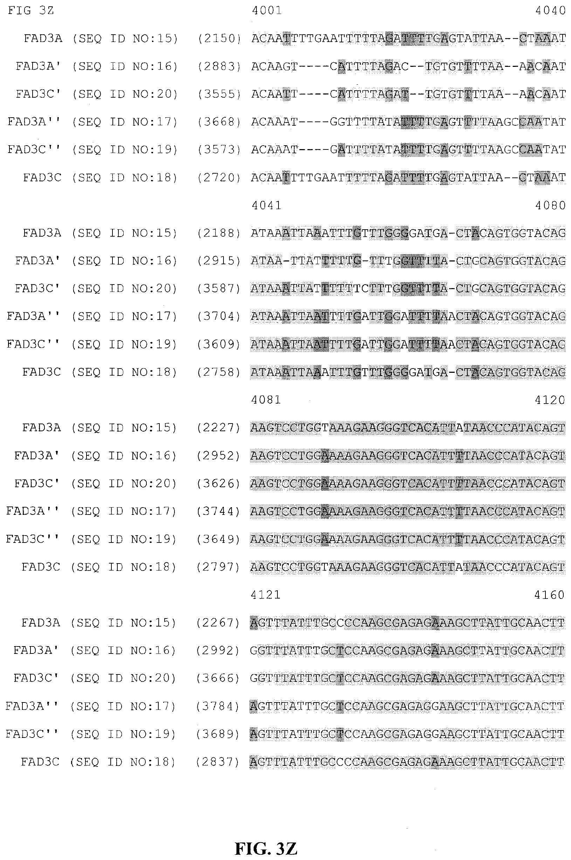

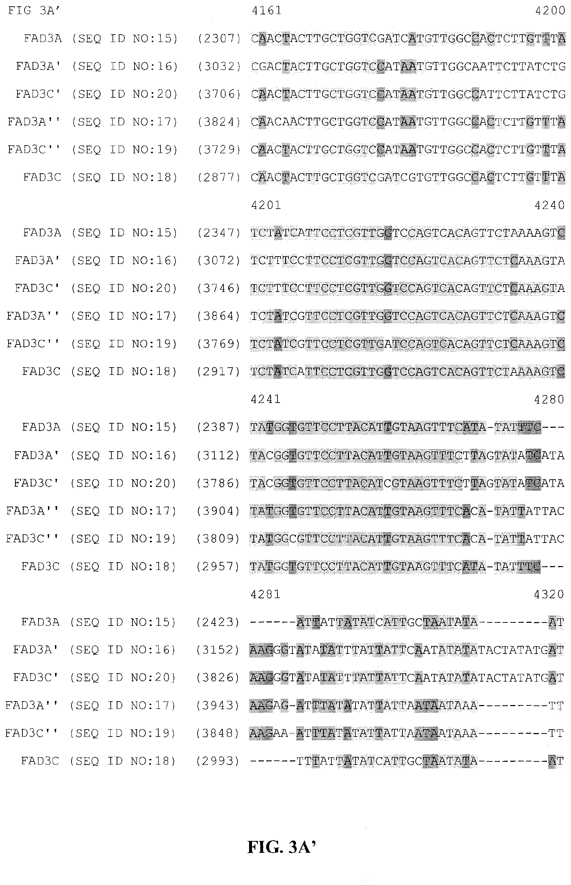

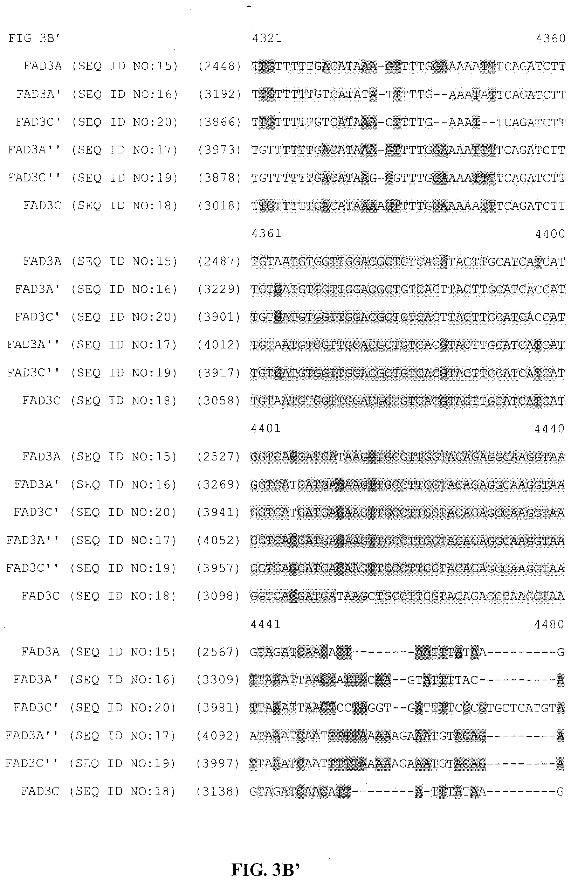

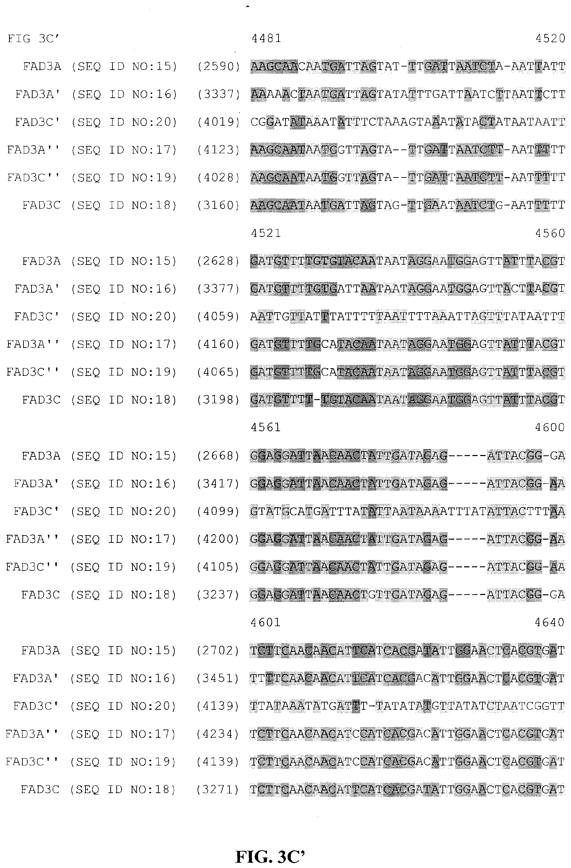

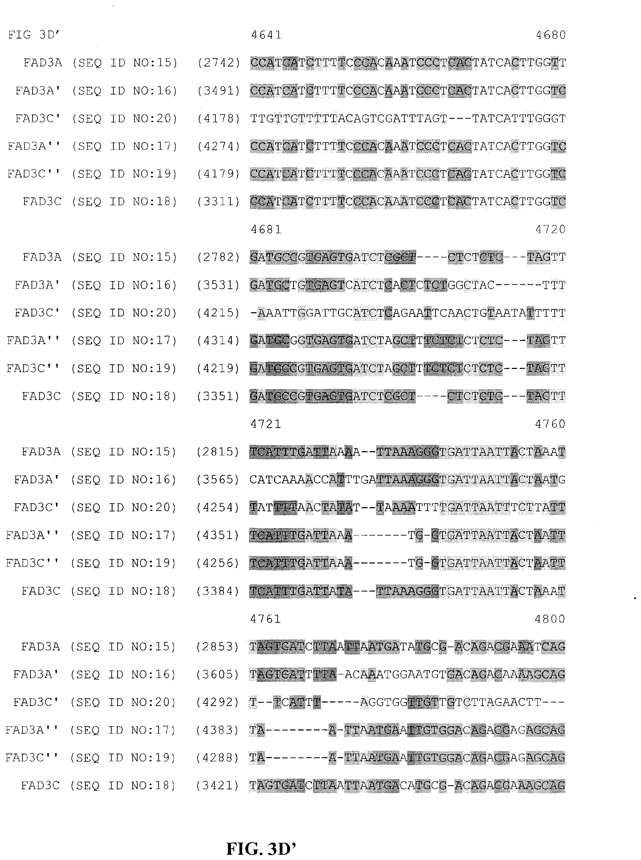

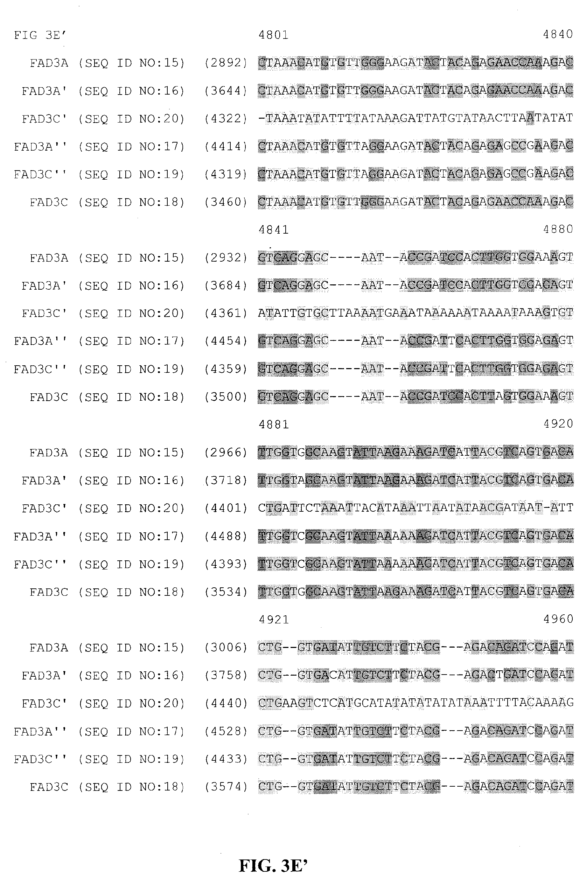

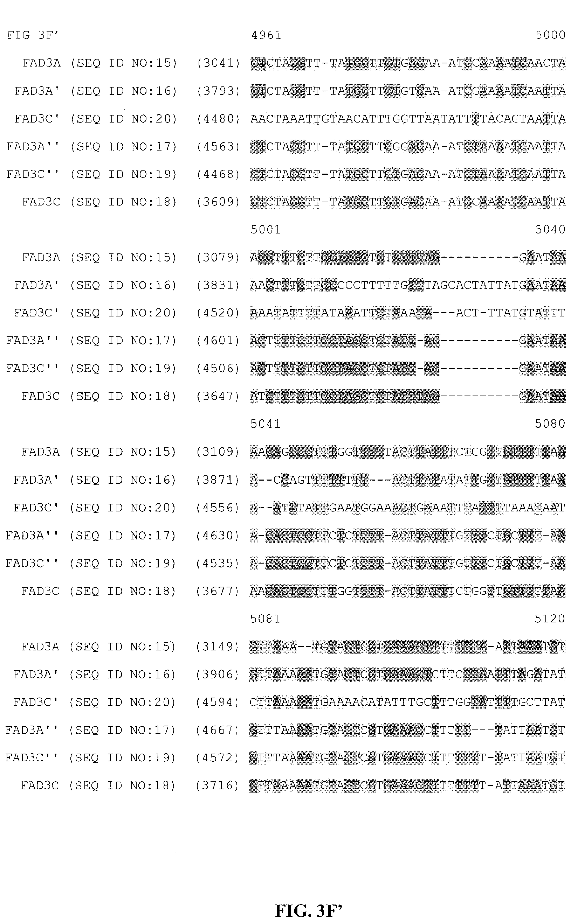

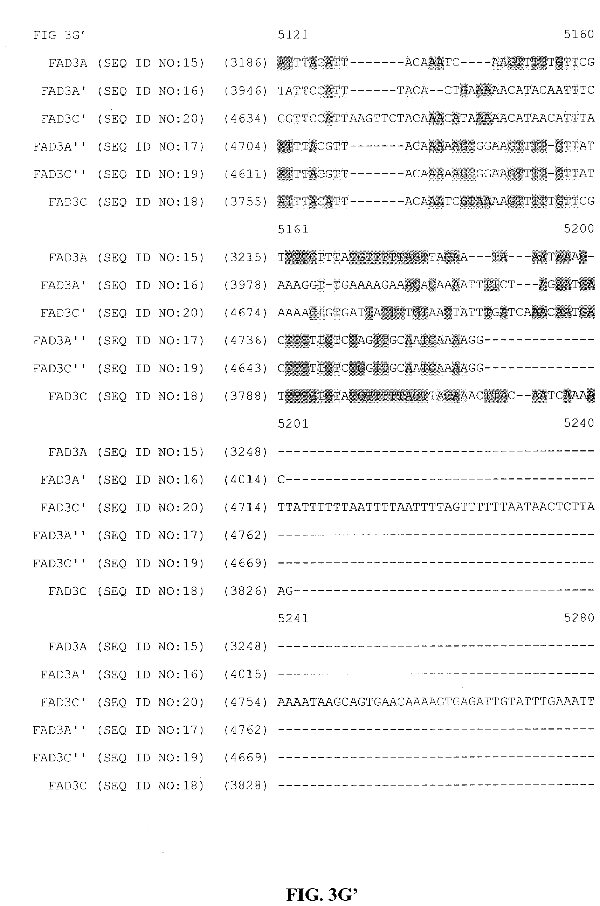

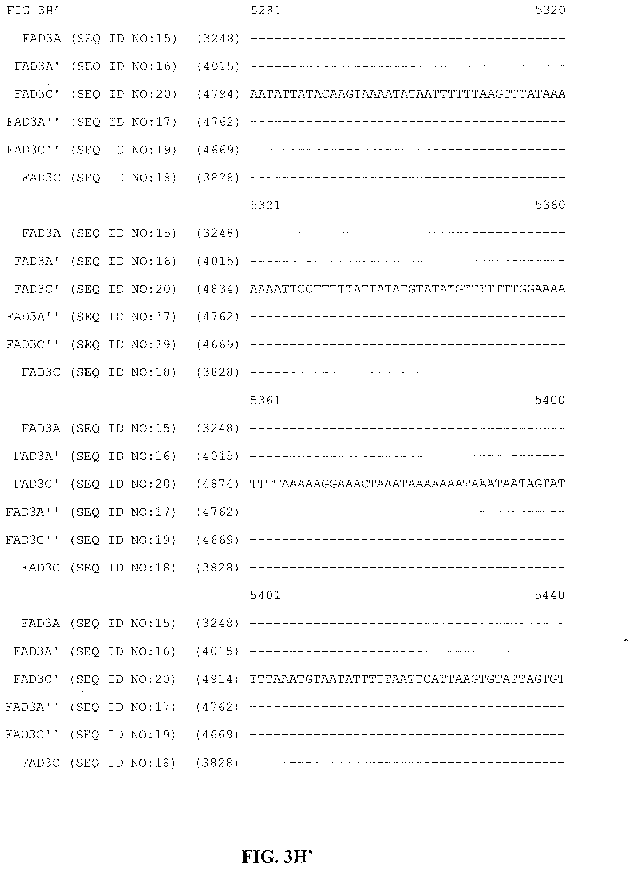

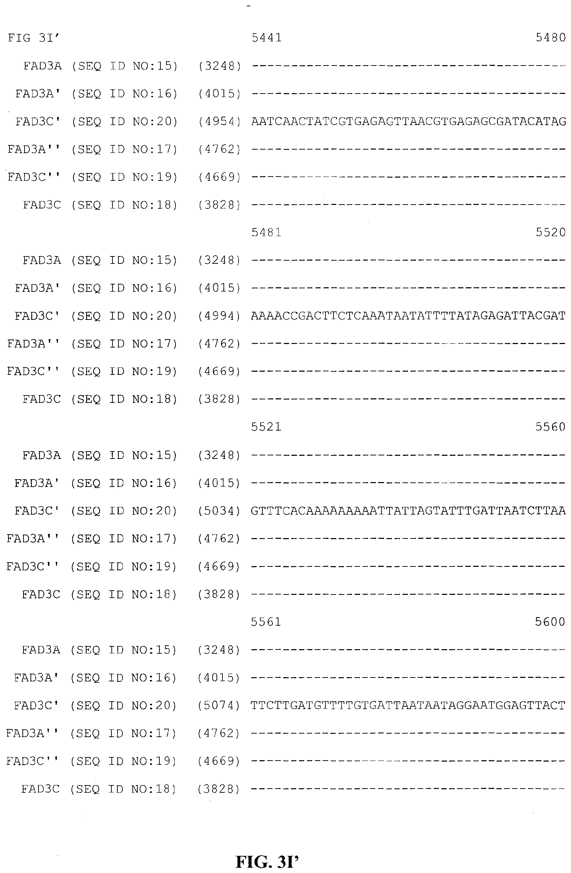

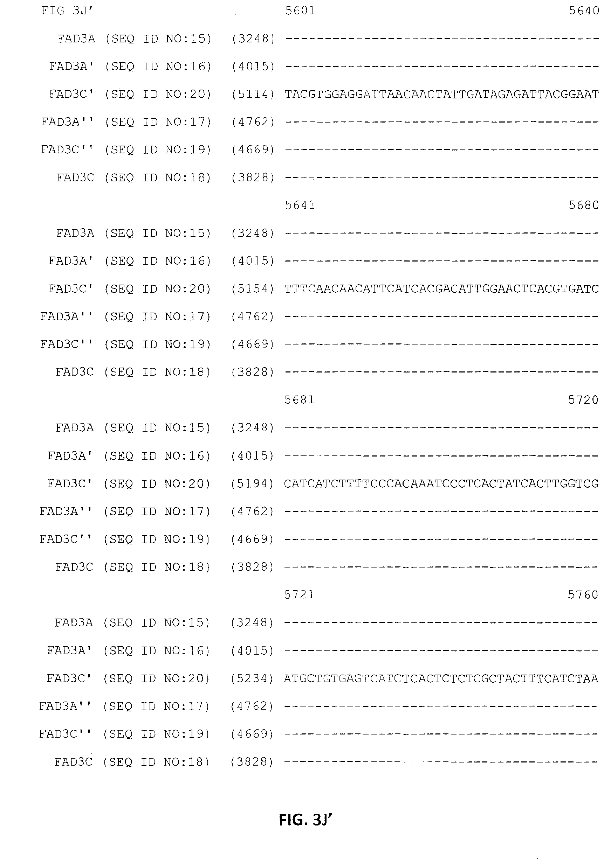

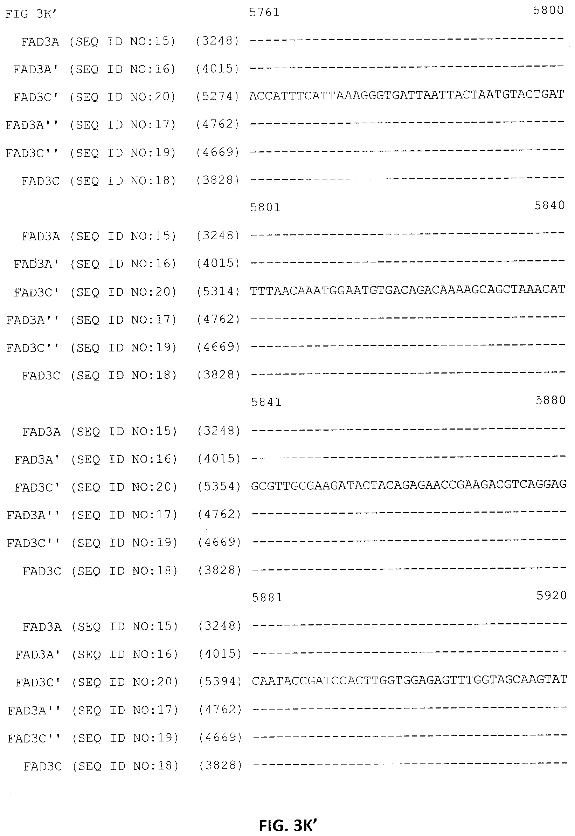

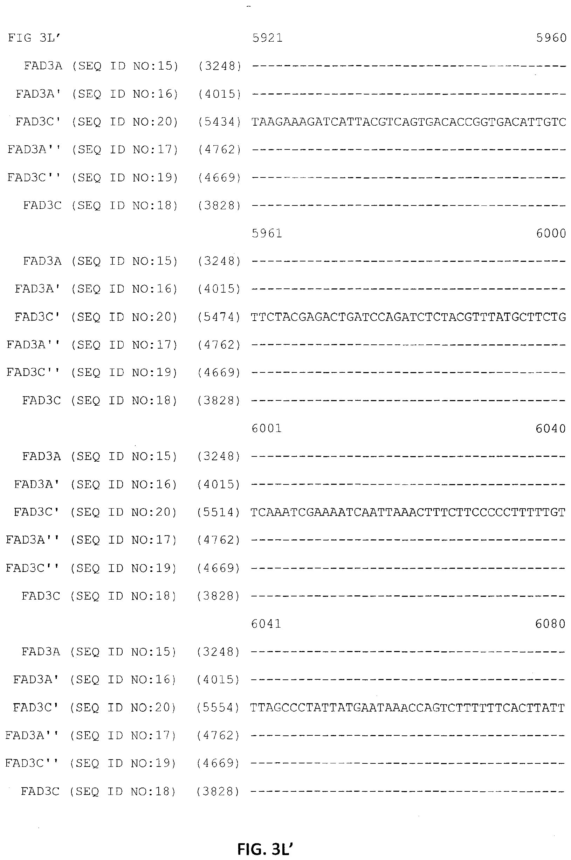

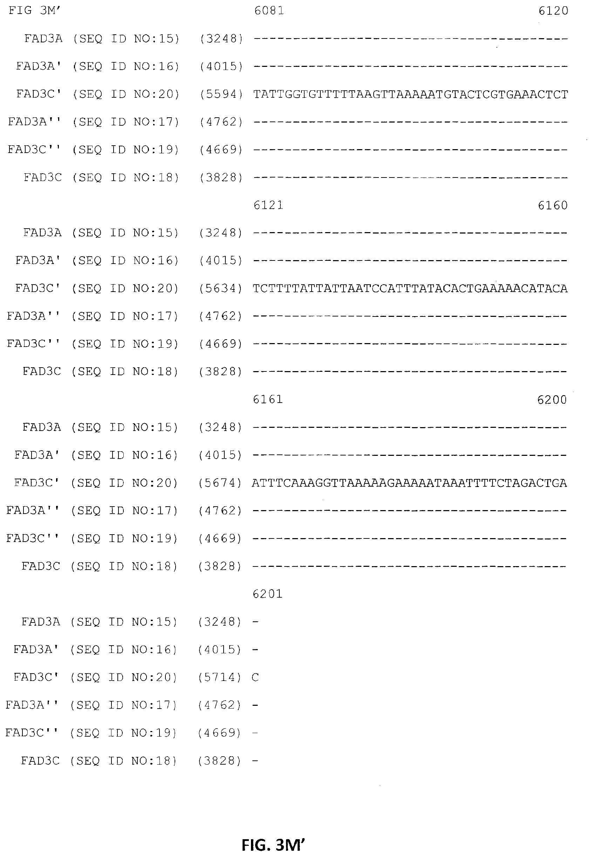

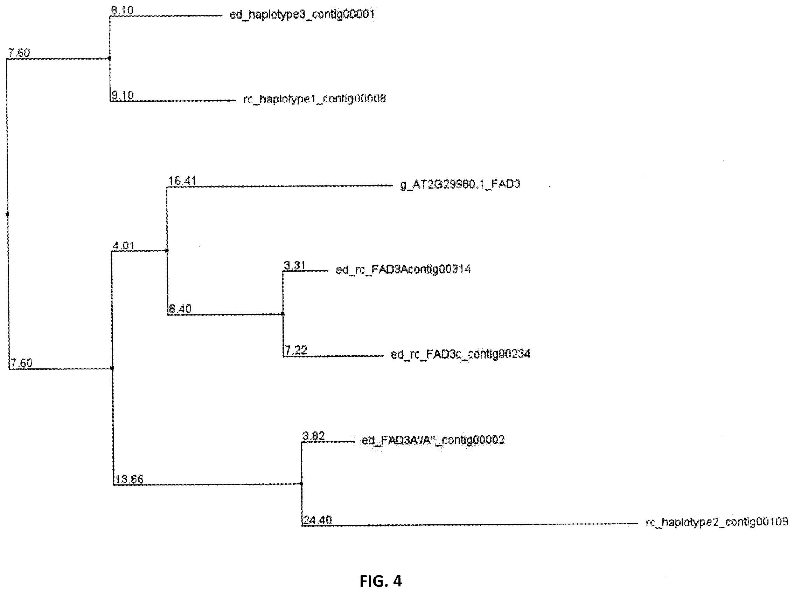

Sequence analysis of the six FAD3 genes was conducted using a sequence alignment program and a neighbor-joining tree using percentage of identity. The sequence alignment was made via the ALIGNX® program from the Vector NTI Advance 11.0 computer program (Life Technologies, Carlsbad, CA) and is shown in FIG. 3. ALIGNX® uses a modified **Clustal W algorithm** to generate multiple sequence alignments of either protein or nucleic acid sequences for similarity comparisons and for annotation. The neighbor-joining tree was created with JALVIEW v2.3® software and is shown in FIG. 4. (Waterhouse et al. (2009) Bioinformatics 25 (9) 1189-1191). The contigs identified as containing FAD3 genes were used as BLASTn queries against a database of *Arabidopsis thaliana* genes. The region of each of the 6 contigs containing the FAD3 gene was identified through comparison to the *Arabidopsis thaliana* FAD3 gene (Genbank Accession No: At2g29980). The FAD3 contigs were then orientated such that all FAD3 genes were in the 5' to 3' orientation. FAD3 contigs were trimmed to contain as many as 2 upstream (5') and 1 downstream (3') *Arabidopsis thaliana* genes where possible. Once orientated the complete coding region of the FAD3 genes were extracted from each contig and used to generate a Neighbor joining tree to display the relationship between the different FAD3 gene family members. The 6 FAD3 family members were aligned into 3 pairs of FAD3 genes (FIG. 4).

### PCR BASED SCREENING

A cohort of PCR primers were design to screen the aforementioned BAC library. The primers were designed as either universal primers, which would amplify all members of the gene family, or as gene specific primers for targeted allele amplification. The PCR primers were designed to be 20 bp long (+/- 1bp) and contain a G/C content of 50% (+/-8%). Table 2 and Table 3 lists the primers which were designed and synthesized. The clones of the BAC library were pooled and screened via the Polymerase Chain Reaction (PCR).

**Table 2: Primer sequences used for PCR amplification of FAD3 sequences.**

| **Primer Name:** | **SEQ ID NO:** | **Sequence:** |
|---|---|---|
| D_uni_F3_F1 | SEQ ID NO:21 | GAATAAGCCATCGGACACAC |
| D_spec_F3_F2 | SEQ ID NO:22 | ATGCGAACGGAGACGAAAGG |
| D_spec_F3_F3 | SEQ ID NO:23 | TGTTAACGGAGATTCCGGTG |
| D_spec_F3_F4 | SEQ ID NO:24 | GTAGCAATGTGAACGGAGAT |
| | | |
| D_uni_F3_R1 | SEQ ID NO:25 | CAGTGTATCTGAGCATCCG |
| D_spec_F3_R2 | SEQ ID NO:26 | GTGGCCGAGTACGAAGATAG |
| D_spec_F3_R3 | SEQ ID NO:27 | CAGTAGAGTGGCCAGAGGA |

**Table 3: PCR primer sequences designed for BAC library screening for FAD2 gene identification.**

| **Primer Name** | **SEQ ID NO:** | **Sequence** |
|---|---|---|
| D_UnivF2_F1 | SEQ ID NO:28 | ATGGGTGCAGGTGGAAGAATG |
| D_UnivF2_F2 | SEQ ID NO:29 | AGCGTCTCCAGATATACATC |
| D_UnivF2_R1 | SEQ ID NO:30 | ATGTATATCTGGAGACGCTC |
| D_UnivF2_R2 | SEQ ID NO:31 | TAGATACACTCCTTCGCCTC |
| D_SpecificF2_F3 | SEQ ID NO:32 | TCTTTCTCCTACCTCATCTG |
| D_SpecificF2_R3 | SEQ ID NO:33 | TTCGTAGCTTCCATCGCGTG |
| D_UnivF2_F4 | SEQ ID NO:34 | GACGCCACCATTCCAACAC |
| D_UnivF2_R4 | SEQ ID NO:35 | ACTTGCCGTACCACTTGATG |

A Two different sets of conditions were used for the polymerase chain reactions (PCR). The first series of PCR reactions contained: 1X PCR buffer (containing dNTPs); 1.5 mM MgCl₂; 200 µM of 0.25 U IMMOLASE® DNA polymerase (Bioline, London, UK); 250 nM of each primer; and, about 5-10 ng template DNA. A second series of PCR reactions were developed for the amplification of genomic DNA and contained: 5-10 ng of genomic DNA, 1X PCR buffer, 2 mM dNTPs, 0.4 µM forward and reverse primer, and 0.25 U IMMOLASE® DNA polymerase (Bioline, London, UK). Amplifications were pooled into a final volume of 13 µL and amplified using an MJ PTC200® thermocycler (BioRad, Hercules, CA) or an ABI 9700 GENE AMP SYSTEM® (Life Technologies, Carlsbad, CA). PCR based screening of specific plates was conducted using a 4 dimension screening approach based on the screening system described by Bryan et al (Scottish Crops Research Institute annual report: 2001-2002) with the above described PCR conditions. Following PCR based screening of pooled BAC libraries; the amplified PCR product was sequenced using a direct Sanger sequencing method. The amplified products were purified with ethanol, sodium acetate and EDTA following the BIGDYE® v3.1 protocol (Applied Biosystems) and electrophoresis was performed on an ABI3730xl® automated capillary electrophoresis platform.

Following PCR based screening and conformational Sanger sequencing, a collection of plates were identified that contained the various different FAD2 and FAD3 gene family members. A total of four unique FAD2 and FAD3 paralogous gene sequences were identified (Table 4 and Table 5). A total of two plates per each FAD2 and FAD3 paralogous gene sequence were chosen to undergo plate screening to identify the specific well and clone within the plate that contained the FAD2 and FAD3 gene (Table 4 and Table 5). The specific wells were identified for both of the plates and an individual clone was selected for each of the FAD2 and FAD3 gene family members.

**Table 4: Identification of the BAC clone plates that provided positive reaction with the detailed PCR primer combinations, along with two plate identities that were taken forward for clone identification within the plate.**

| **Gene Name** | **Primer Sets** | **Positive Plate Pools** | **Chosen Plates** | **Well Id** |
|---|---|---|---|---|
| FAD2A | F4+R1, F1+R1, F1+R4, F3+R3 | 8, 27, 30, 83, 109, 147, 180, 199, 209, 251, 288 | Plate 199 | L23 |
| | | | Plate 27 | D20 |
| FAD2-1 | F1+R4, F4+R1, F1+R1, F2+R2 | 12, 89, 123, 148, 269 | Plate 123 | N17 |
| | | | Plate 148 | B15 |
| FAD2-2 | F4+R1, F1+R1, F1+R4, F2+R2 | 24,44,46,47,80,91, 104, 110, 119, 121, 124, 248 | Plate 44 | H03 |
| | | | Plate 121 | A17 |
| FAD2-3 | F1+R4, F4+R1, F1+R1, F3+R3 | 8, 62, 113, 205, 276 | Plate 62 | I16 |
| | | | Plate 205 | K11 |

**Table 5: Identification of the BAC clone plates that provided positive reaction with the detailed PCR primer combinations, along with two plate identities that were taken forward for clone identification within the plate.**

| **Gene Name** | **Primer Sets** | **Positive Plate Pools** | **Chosen Plates** |
|---|---|---|---|
| FAD3A | F2+R2 | 16, 231 | Plate 16 |
| (FAD3A-1) | | | Plate 231 |
| FAD3C | F4+R2 | 18, 27, 136, 178, 211, 232 | Plate 18 |
| | | | Plate 27 |
| FAD3C" | F4+R2, F4+R3, F3+R3 | 23, 44, 53, 56, 77, 116, 158, 199, 209, 278, 280, 282, 283, 284, 286 | Plate 44 |
| (Haplotype1) | | | Plate 199 |
| FAD3A' | F4+R2 | 52, 121, 139 | Plate 121 |
| (FAD3A'/FAD3A") | | | Plate 139 |
| FAD3C' | F4+R2 | 144, 188, 235 | Plate 144 |
| (Haplotype2) | | | Plate 188 |
| FAD3A" | F4+R3 and F3+R3 | 69, 105, 106, 229, 242, 247, 248 | Plate 69 |
| (Haplotype3) | | | Plate 106 |

The single BAC clone, for each identified FAD gene family member, was further analysed via sequencing. The DNA was isolated for the BAC clone and was prepared for sequencing using a LARGE CONSTRUCT KIT® (Qiagen, Valencia, CA) following the manufacturer's instructions. The extracted BAC DNA was prepared for sequencing using GS-FLX® Titanium Technology (Roche, Indianapolis, IN) following manufacturer's instructions. Sequencing reactions were performed using a physically sectored GS-FLX TI® Pico-titer plate with the BACs pooled in pairs for optimal data output. The BACs were combined in pairs where the FAD2 gene was paired with a FAD3 gene. All generated sequence data was assembled by NEWBLER v2.0.01.14® (454 Life Sciences, Branford, CT). The assembled contigs were manually assessed for the presence of the corresponding FAD gene using SEQUENCHER v3.7® (GeneCodes, Ann Arbor, MI).

After the full genomic sequence of all four FAD2 and six FAD3 genes had been identified and fully characterized, zinc finger nucleases were designed to bind to the sequences for each specific gene family member.

### EXAMPLE 2: DESIGN OF ZINC FINGER BINDING DOMAINS SPECIFIC TO FAD2 GENES

Zinc finger proteins directed against DNA sequences encoding various functional sequences of the FAD2 gene locus were designed as previously described. *See, e.g.,* Urnov et al. (2005) Nature 435:646-651. Exemplary target sequence and recognition helices are shown in Table 6 and Table 8 (recognition helix regions designs) and Table 7 and Table 9 (target sites). In Table 8 and Table 9, nucleotides in the target site that are contacted by the ZFP recognition helices are indicated in uppercase letters; non-contacted nucleotides indicated in lowercase. Zinc Finger Nuclease (ZFN) target sites were designed to bind five target sites of FAD2A, and seven target sites of FAD3. The FAD2 and FAD3 zinc finger designs were incorporated into zinc finger expression vectors encoding a protein having at least one finger with a CCHC structure. *See,* U.S. Patent Publication No. 2008/0182332. In particular, the last finger in each protein had a CCHC backbone for the recognition helix. The non-canonical zinc finger-encoding sequences were fused to the nuclease domain of the type IIS restriction enzyme FokI (amino acids 384-579 of the sequence of Wah et al., (1998) Proc. Natl. Acad. Sci. USA 95:10564-10569) via a four amino acid ZC linker and an *opaque-2* nuclear localization signal derived from *Zea mays* to form FAD2A zinc-finger nucleases (ZFNs). Expression of the fusion proteins was driven by a relatively strong constitutive promoter such as a promoter derived from the Cassava Vein Mosaic Virus (CsVMV) promoter and flanked by the *Agrobacterium tumefaciens* ORF23 3' UnTranslated Region (AtuORF23 3'UTR v1). The self-hydrolyzing 2A encoding nucleotide sequence from *Thosea asigna* virus (Szymczak *et al.,* 2004) was added between the two Zinc Finger Nuclease fusion proteins that were cloned into the construct. Exemplary vectors are described below.

The optimal zinc fingers were verified for cleavage activity using a budding yeast based system previously shown to identify active nucleases. *See, e.g.,* U.S. Patent Publication No. 20090111119; Doyon et al. (2008) Nat Biotechnol. 26:702-708; Geurts et al. (2009) Science 325:433. Zinc fingers for the various functional domains were selected for *in-vivo* use. Of the numerous ZFNs that were designed, produced and tested to bind to the putative FAD genomic polynucleotide target sites, a ZFNs were identified as having *in vivo* activity at high levels, and selected for further experimentation. These ZFNs were characterized as being capable of efficiently binding and cleaving the unique FAD2 genomic polynucleotide target sites *in planta.*

**Table 6: FAD3 Zinc Finger Designs**

| **ZFP** | **F1** | **F2** | **F3** | **F4** | **F5** | **F6** |
|---|---|---|---|---|---|---|
| 27961 | RSDNLAR (SEQ ID NO: 178) | QKKDRSY (SEQ ID NO:179) | RSDNLAR (SEQ ID NO:180) | QRGNRNT (SEQ ID NO:181) | RSDHLSR (SEQ ID NO:182) | RNQDRTN (SEQ ID NO:183) |
| 27962 | DRSNLSR (SEQ ID NO:184) | RQDSRSQ (SEQ ID NO:185) | QSSDLSR (SEQ ID NO:186) | DRSALAR (SEQ ID NO:187) | TSGSLTR (SEQ ID NO:188) | N/A |
| 27973 | QSSDLSR (SEQ ID NO:189) | AASNRSK (SEQ ID NO:190) | TSGSLSR (SEQ ID NO:191) | RSDALAR (SEQ ID NO:192) | RSDVLST (SEQ ID NO:193) | WGRLRKL (SEQ ID NO:194) |
| 27974 | ERGTLAR (SEQ ID NO:195) | RSDDLTR (SEQ ID NO:196) | RSDHLSA (SEQ ID NO:197) | QHGALQT (SEQ ID NO:198) | TSGNLTR (SEQ ID NO:199) | QSGHLSR (SEQ ID NO:200) |
| 27987 | TSGSLTR (SEQ ID NO:201) | RSDHLSQ (SEQ ID NO:202) | CTRNRWR (SEQ ID NO:203) | RSDNLSE (SEQ ID NO:204) | ASKTRKN (SEQ ID NO:205) | N/A |
| 27990 | TSGSLSR (SEQ ID NO:206) | TSSNRAV (SEQ ID NO:207) | TSGNLTR (SEQ ID NO:208) | DRSALAR (SEQ ID NO:209) | RSDVLSE (SEQ ID NO:210) | RNFSLTM (SEQ ID NO:211) |
| 27991 | QSGDLTR (SEQ ID NO:212) | TSGSLSR (SEQ ID NO:213) | QSGNLAR (SEQ ID NO:214) | TSGSLSR (SEQ ID NO:215) | QSGSLTR (SEQ ID NO:216) | N/A |
| 27992 | DRSHLAR (SEQ ID NO:217) | TSGSLSR (SEQ ID NO:218) | TSSNRAV (SEQ ID NO:219) | TSGNLTR (SEQ ID NO:220) | DRSALAR (SEQ ID NO:221) | N/A |
| 28004 | QSGNLAR (SEQ ID NO:222) | HLGNLKT (SEQ ID NO:223) | RSDHLSQ (SEQ ID NO:224) | TARLLKL (SEQ ID NO:225) | QSGNLAR (SEQ ID NO:226) | QTSHLPQ (SEQ ID NO:227) |
| 28005 | RSDNLSV (SEQ ID NO:228) | TSGHLSR (SEQ ID NO:229) | TSGSLTR (SEQ ID NO:230) | RSDALST (SEQ ID NO:231) | DRSTRTK (SEQ ID NO:232) | N/A |
| 28021 | QNAHRKT (SEQ ID NO:233) | TSGNLTR (SEQ ID NO:234) | LKQMLAV (SEQ ID NO:235) | RSDNLSR (SEQ ID NO:236) | DNSNRKT (SEQ ID NO:237) | N/A |
| 28022 | RSDNLSV (SEQ ID NO:238) | QNANRIT (SEQ ID NO:239) | TSGSLSR (SEQ ID NO:240) | QSSVRNS (SEQ ID NO:241) | DRSALAR (SEQ ID NO:242) | N/A |
| 28023 | RSDNLSR (SEQ ID NO:243) | DNSNRKT (SEQ ID NO:244) | DRSNLTR (SEQ ID NO:245) | RSDVLSE (SEQ ID NO:246) | TRNGLKY (SEQ ID NO:247) | N/A |
| 28024 | RSDALAR (SEQ ID NO:248) | RSDVLSE (SEQ ID NO:249) | RSSDRTK (SEQ ID NO:250) | RSDNLSV (SEQ ID NO:251) | QNANRIT (SEQ ID NO:252) | N/A |
| 28025 | QSSDLSR (SEQ ID NO:253) | QSTHRNA (SEQ ID NO:254) | RSDNLAR (SEQ ID NO:255) | QRGNRNT (SEQ ID NO:256) | RSDHLSR (SEQ ID NO:257) | RNQDRTN (SEQ ID NO:258) |
| 28026 | DRSNLSR (SEQ ID NO:259) | RQDSRSQ (SEQ ID NO:260) | QSSDLSR (SEQ ID NO:261) | DRSALAR (SEQ ID NO:262) | TSGSLTR (SEQ ID NO:263) | N/A |
| 28035 | QSSDLSR (SEQ ID NO:264) | AASNRSK (SEQ ID NO:265) | TSGSLSR (SEQ ID NO:266) | RSDALAR (SEQ ID NO:267) | RSDTLSQ (SEQ ID NO:268) | QRDHRIK (SEQ ID NO:269) |
| 28036 | RSDDLTR (SEQ ID NO:270) | QSSDLRR (SEQ ID NO:271) | RSDHLSA (SEQ ID NO:272) | QHGALQT (SEQ ID NO:273) | TSGNLTR (SEQ ID NO:274) | QSGHLSR (SEQ ID NO:275) |
| 28039 | TSGSLSR (SEQ ID NO:276) | RSDALAR (SEQ ID NO:277) | RSDTLSQ (SEQ ID NO:278) | QRDHRIK (SEQ ID NO:279) | TSGNLTR (SEQ ID NO:280) | DRGDLRK (SEQ ID NO:281) |
| 28040 | DSSDRKK (SEQ ID NO:282) | TSGNLTR (SEQ ID NO:283) | DNYNRAK (SEQ ID NO:284) | DRSHLTR (SEQ ID NO:285) | RSDNLTT (SEQ ID NO:286) | N/A |
| 28051 | RSDNLSN (SEQ ID NO:287) | TSSSRIN (SEQ ID NO:288) | RSDNLSE (SEQ ID NO:289) | ASKTRKN (SEQ ID NO:290) | RSDALTQ (SEQ ID NO:291) | N/A |
| 28052 | RSDTLST (SEQ ID NO:292) | DRSSRIK (SEQ ID NO:293) | RSDDLSK (SEQ ID NO:294) | DNSNRIK (SEQ ID NO:295) | N/A | N/A |
| 28053 | QSSDLSR (SEQ ID NO:296) | QAGNLSK (SEQ ID NO:297) | QSGDLTR (SEQ ID NO:298) | TSGSLSR (SEQ ID NO:299) | QSGNLAR (SEQ ID NO:300) | N/A |
| 28054 | TSGSLSR (SEQ ID NO:301) | LRQTLRD (SEQ ID NO:302) | TSGNLTR (SEQ ID NO:303) | DRSALAR (SEQ ID NO:304) | RSDVLSE (SEQ ID NO:305) | RNFSLTM (SEQ ID NO:306) |
| 28055 | QSGDLTR (SEQ ID NO:307) | TSGSLSR (SEQ ID NO:308) | QSGNLAR (SEQ ID NO:309) | TSGSLSR (SEQ ID NO:310) | QSGSLTR (SEQ ID NO:311) | N/A |
| 28056 | DRSHLAR (SEQ ID NO:312) | TSGSLSR (SEQ ID NO:313) | LRQTLRD (SEQ ID NO:314) | TSGNLTR (SEQ ID NO:315) | DRSALAR (SEQ ID NO:316) | N/A |

**Table 7: Target Sites of FAD3 Zinc Fingers**

| **ZFP** | **Target Site (5' to 3')** | **SEQ ID NO:** |
|---|---|---|
| 27961 | cgCCGGAGAAAGAGAGAGAGctttgagg | SEQ ID NO:36 |
| 27962 | tgGTTGTCGCTATGGACcagcgtagcaa | SEQ ID NO:37 |
| 27969 | tcTCCGTTcGCATTGcTACGCTggtcca | SEQ ID NO:38 |
| 27970 | gaAAGGTTtGATCCGAGCGCAcaaccac | SEQ ID NO:39 |
| 27973 | ctTGAACGGTGGTTgTGCGCTcggatca | SEQ ID NO:40 |
| 27974 | tcGGAGATATAAGGGCGGCCattcctaa | SEQ ID NO:41 |
| 27987 | taGCCCAGAACAGGGTTccttgggcggc | SEQ ID NO:42 |
| 27988 | ctTCGTACTCGGCCACGactggtaattt | SEQ ID NO:43 |
| 27989 | ttGAAGTTGCAaTAAGCTttctctcgct | SEQ ID NO:44 |
| 27990 | acTTGCTGGTCGATCATGTTggccactc | SEQ ID NO:45 |
| 27991 | aaGTAGTTGAAGTTGCAataagctttct | SEQ ID NO:46 |
| 27992 | tgGTCGATCATGTTGGCcactcttgttt | SEQ ID NO:47 |
| 28004 | aaCGAGAATGAAGGAATGAAgaatatga | SEQ ID NO:48 |
| 28005 | atACCATGGTTGGTAAGtcatttatttt | SEQ ID NO:49 |
| 28021 | ccAACGAGgAATGATAGAtaaacaagag | SEQ ID NO:50 |
| 28022 | caGTCACAGTTcTAAAAGtctatggtgt | SEQ ID NO:51 |
| 28023 | tgTGACTGGACcAACGAGgaatgataga | SEQ ID NO:52 |
| 28024 | tcTAAAAGTCTATGGTGttccttacatt | SEQ ID NO:53 |
| 28025 | cgCCGGAGAAAGAGAGAGCTttgaggga | SEQ ID NO:54 |
| 28026 | tgGTTGTCGCTATGGACcagcgtagcaa | SEQ ID NO:55 |
| 28035 | ctTAAACGGTGGTTgTGCGCTcggatca | SEQ ID NO:56 |
| 28036 | tcGGAGATATAAGGGCTGCGattcctaa | SEQ ID NO:57 |
| 28039 | tcTCCGATctTAAACGGTGGTTgtgcgc | SEQ ID NO:58 |
| 28040 | atAAGGGCTGCGATTCCtaagcattgtt | SEQ ID NO:59 |
| 28051 | agATGGCCCAGAAAAGGgttccttgggc | SEQ ID NO:60 |
| 28052 | cgTACTCGGCCACGactggtaatttaat | SEQ ID NO:61 |
| 28053 | ttGAAGTTGCAaTAAGCTttctctcgct | SEQ ID NO:62 |
| 28054 | acTTGCTGGTCGATCGTGTTggccactc | SEQ ID NO:63 |
| 28055 | aaGTAGTTGAAGTTGCAataagctttct | SEQ ID NO:64 |
| 28056 | tgGTCGATCGTGTTGGCcactcttgttt | SEQ ID NO:65 |

**Table 8: FAD2 Zinc Finger Designs**

| **ZFP** | **F1** | **F2** | **F3** | **F4** | **F5** | **F6** |
|---|---|---|---|---|---|---|
| 24800 | RSDNLST (SEQ ID NO:317) | HSHARIK (SEQ ID NO:318) | HRSSLRR (SEQ ID NO:319) | RSDHLSE (SEQ ID NO:320) | QNANRIT (SEQ ID NO:321) | N/A |
| 24801 | DRSNLSR (SEQ ID NO:322) | HRSSLRR (SEQ ID NO:323) | TSGNLTR (SEQ ID NO:324) | MSHHLRD (SEQ ID NO:325) | DQSNLRA (SEQ ID NO:326) | N/A |
| 24794 | QSGNLAR (SEQ ID NO:327) | RSDNLSR (SEQ ID NO:328) | DNNARIN (SEQ ID NO:329) | DRSNLSR (SEQ ID NO:330) | RSDHLTQ (SEQ ID NO:331) | N/A |
| 24795 | RSDNLRE (SEQ ID NO:332) | QSGALAR (SEQ ID NO:333) | QSGNLAR (SEQ ID NO:334) | RSDVLSE (SEQ ID NO:335) | SPSSRRT (SEQ ID NO:336) | N/A |
| 24810 | RSDSLSR (SEQ ID NO:337) | RKDARIT (SEQ ID NO:338)** | RSDHLSA (SEQ ID NO:339)** | WSSSLYY (SEQ ID NO:340)** | NSRNLRN (SEQ ID NO:341)** | N/A |
| 24811 | DQSTLRN (SEQ ID NO:342) | DRSNLSR (SEQ ID NO:343) | DRSNLWR (SEQ ID NO:344) | DRSALSR (SEQ ID NO:345) | RSDALAR (SEQ ID NO:346) | N/A |
| 24814 | RSDALSR (SEQ ID NO:347) | DRSDLSR (SEQ ID NO:348) | RSDHLTQ (SEQ ID NO:349) | QSGALAR (SEQ ID NO:350) | QSGNLAR (SEQ ID NO:351) | N/A |
| 24815 | DRSNLSR (SEQ ID NO:352) | DSSARNT (SEQ ID NO:353) | DRSSRKR (SEQ ID NO:354) | QSGDLTR (SEQ ID NO:355) | LAHHLVQ (SEQ ID NO:356) | N/A |
| 24818 | RSDNLST (SEQ ID NO:357) | HSHARIK (SEQ ID NO:358) | TSGHLSR (SEQ ID NO:359) | RSDNLSV (SEQ ID NO:360) | IRSTLRD (SEQ ID NO:361) | N/A |
| 24819 | TSGHLSR (SEQ ID NO:362) | DRSNLSR (SEQ ID NO:363) | HRSSLRR (SEQ ID NO:364) | TSGNLTR (SEQ ID NO:365) | MSHHLRD (SEQ ID NO:366) | N/A |
| 24796 | RSDALSR (SEQ ID NO:367) | DRSDLSR (SEQ ID NO:368) | RSDHLTQ (SEQ ID NO:369) | QSGALAR (SEQ ID NO:370) | QSGNLAR (SEQ ID NO:371) | N/A |
| 24797 | RSAVLSE (SEQ ID NO:372) | TNSNRIT (SEQ ID NO:373) | LKQHLNE (SEQ ID NO:374) | QSGALAR (SEQ ID NO:375) | QSGNLAR (SEQ ID NO:376) | N/A |
| 24836 | DRSNLSR (SEQ ID NO:377) | QSGDLTR (SEQ ID NO:378) | QSGALAR (SEQ ID NO:379) | DRSNLSR (SEQ ID NO:380) | QRTHLTQ (SEQ ID NO:381) | N/A |
| 24837 | RSDNLSN (SEQ ID NO:382) | TNSNRIK (SEQ ID NO:383) | QSSDLSR (SEQ ID NO:384) | QSSDLRR (SEQ ID NO:385) | DRSNRIK (SEQ ID NO:386) | N/A |
| 24844 | RSANLAR (SEQ ID NO:387) | RSDNLTT (SEQ ID NO:388) | QSGELIN (SEQ ID NO:389) | RSADLSR (SEQ ID NO:390) | RSDNLSE (SEQ ID NO:391) | DRSHLAR (SEQ ID NO:392) |
| 24845 | DRSHLAR (SEQ ID NO:393) | RSDNLSE (SEQ ID NO:394) | SKQYLIK (SEQ ID NO:395) | ERGTLAR (SEQ ID NO:396) | RSDHLTT (SEQ ID NO:397) | N/A |
| 24820 | QSGALAR (SEQ ID NO:398) | QSGNLAR (SEQ ID NO:399) | DRSHLAR (SEQ ID NO:400) | DRSDLSR (SEQ ID NO:401) | RSDNLTR (SEQ ID NO:402) | N/A |
| 24821 | DRSHLAR (SEQ ID NO:403) | RSDNLSE (SEQ ID NO:404) | SKQYLIK (SEQ ID NO:405) | ERGTLAR (SEQ ID NO:406) | RSDHLTT (SEQ ID NO:407) | N/A |
| 24828 | DRSDLSR (SEQ ID NO:408) | RSDNLTR (SEQ ID NO:409) | QRTHLTQ (SEQ ID NO:410) | RSDNLSE (SEQ ID NO:411) | ASKTRKN (SEQ ID NO:412) | N/A |
| 24829 | RSDTLSE (SEQ ID NO:413) | QSHNRTK (SEQ ID NO:414) | QSDHLTQ (SEQ ID NO:415) | RSSDLSR (SEQ ID NO:416) | QSSDLSR (SEQ ID NO:417) | RSDHLTQ (SEQ ID NO:418) |
| 24832 | RSDSLSR (SEQ ID NO:419) | RKDARIT (SEQ ID NO:420) | DRSHLSR (SEQ ID NO:421) | QSGNLAR (SEQ ID NO:422) | QSSDLSR (SEQ ID NO:423) | DRSALAR (SEQ ID NO:424) |
| 24833 | RSDDLSK (SEQ ID NO:425) | RSDTRKT (SEQ ID NO:426 | DRSNLSR (SEQ ID NO:427) | DRSNLWR (SEQ ID NO:428) | RSDSLSR (SEQ ID NO:429) | NNDHRKT (SEQ ID NO:430) |

**Table 9: Target Sites of FAD2 Zinc Fingers**

| **ZFP** | **Plasmid No.** | **Target Site (5' to 3')** | **ZFP target/binding site present in SEQ ID Nos.** |
|---|---|---|---|
| 24800 | pDAB104001 | ccCAAAGGGTTGTTGAGgtacttgccgt | SEQ ID NO:66 |
| 24801 | pDAB104001 | cgCACCGTGATGTTAACggttcagttca | SEQ ID NO:67 |
| 24794 | pDAB104002 | taAGGGACGAGGAGGAAggagtggaaga | SEQ ID NO:68 |
| 24795 | pDAB104002 | ttCTCCTGGAAGTACAGtcatcgacgcc | SEQ ID NO:69 |
| 24810 | pDAB104003 | gtCGCTGAAGGcGTGGTGgccgcactcg | SEQ ID NO:70 |
| 24811 | pDAB104003 | caGTGGCTgGACGACACCgtcggcctca | SEQ ID NO:71 |
| 24814 | pDAB104004 | gaGAAGTAAGGGACGAGgaggaaggagt | SEQ ID NO:72 |
| 24815 | pDAB104004 | gaAGTACAGTCATCGACgccaccattcc | SEQ ID NO:73 |
| 24818 | pDAB104005 | tcCCAAAGGGTtGTTGAGgtacttgccg | SEQ ID NO:74 |
| 24819 | pDAB104005 | acCGTGATGTTAACGGTtcagttcactc | SEQ ID NO:75 |
| 24796 | pDAB104006 | gaGAAGTAAGGGACGAGgaggaaggagt | SEQ ID NO:76 |
| 24797 | pDAB104006 | tgGAAGTAcAGTCATCGAcgccaccatt | SEQ ID NO:77 |
| 24836 | pDAB104007 | gtAGAGACcGTAGCAGACggcgaggatg | SEQ ID NO:78 |
| 24837 | pDAB104007 | gcTACGCTGCTgTCCAAGgagttgcctc | SEQ ID NO:79 |
| 24844 | pDAB104008 | gaGGCCAGGCGAAGTAGGAGagagggtg | SEQ ID NO:80 |
| 24845 | pDAB104008 | acTGGGCCTGCCAGGGCtgcgtcctaac | SEQ ID NO:81 |
| 24820 | pDAB104009 | gaGAGGCCaGGCGAAGTAggagagaggg | SEQ ID NO:82 |
| 24821 | pDAB104009 | acTGGGCCTGCCAGGGCtgcgtcctaac | SEQ ID NO:83 |
| 24828 | pDAB104010 | agGCCCAGtAGAGAGGCCaggcgaagta | SEQ ID NO:84 |
| 24829 | pDAB104010 | ccAGGGCTGCGTCCTAACCGgcgtctgg | SEQ ID NO:85 |
| 24832 | pDAB104011 | taGTCGCTGAAGGCGTGGTGgccgcact | SEQ ID NO:86 |
| 24833 | pDAB104011 | agTGGCTGGACGACaCCGTCGgcctcat | SEQ ID NO:87 |

### EXAMPLE 3: EVALUATION OF ZINC FINGER NUCLEASE CLEAVAGE OF FAD2 GENES

### CONSTRUCT ASSEMBLY

Plasmid vectors containing ZFN expression constructs of the exemplary zinc finger nucleases, which were identified using the yeast assay, as described in Example 2, were designed and completed using skills and techniques commonly known in the art. Each zinc finger-encoding sequence was fused to a sequence encoding an opaque-2 nuclear localization signal (Maddaloni et al. (1989) Nuc. Acids Res. 17(18):7532), that was positioned upstream of the zinc finger nuclease.

Next, the opaque-2 nuclear localization signal::zinc finger nuclease fusion sequence was paired with the complementary opaque-2 nuclear localization signal: :zinc finger nuclease fusion sequence. As such, each construct consisted of a single open reading frame comprised of two opaque-2 nuclear localization signal: :zinc finger nuclease fusion sequences separated by the 2A sequence from *Thosea asigna* virus (Mattion et al. (1996) J. Virol. 70:8124-8127). Expression of the fusion proteins was driven by a relatively strong constitutive promoter such as a promoter derived from the Cassava Vein Mosaic Virus (CsVMV) promoter and flanked by the *Agrobacterium tumefaciens* ORF23 3' UnTranslated Region (AtuORF23 3'UTR).

The vectors were assembled using the IN-FUSION™ Advantage Technology (Clontech, Mountain View, CA). Restriction endonucleases were obtained from New England BioLabs (NEB; Ipswich, MA) and T4 DNA Ligase (Invitrogen) was used for DNA ligation. Plasmid preparations were performed using NUCLEOSPIN® Plasmid Kit (Macherey-Nagel Inc., Bethlehem, PA) or the Plasmid Midi Kit (Qiagen) following the instructions of the suppliers. DNA fragments were isolated using QIAquick Gel Extraction Kit™ (Qiagen) after agarose Tris-acetate gel electrophoresis. Colonies of all assembled plasmids were initially screened by restriction digestion of miniprep DNA. Plasmid DNA of selected clones was sequenced by a commercial sequencing vendor (Eurofins MWG Operon, Huntsville, AL). Sequence data were assembled and analyzed using the SEQUENCHER™ software (Gene Codes Corp., Ann Arbor, MI). Before delivery to *B. napus* protoplasts, Plasmid DNA was prepared from cultures of *E. coli* using the Pure Yield PLASMID MAXIPREP System® (Promega Corporation, Madison, WI) or PLASMID MAXI KIT® (Qiagen, Valencia, CA) following the instructions of the suppliers.

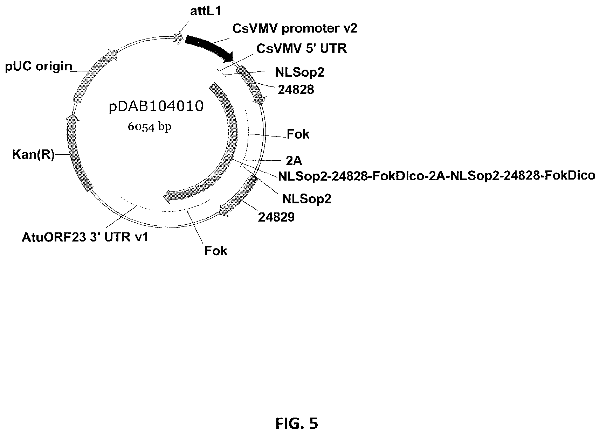

The resulting eleven plasmid constructs; pDAB104008 (containing the ZFN24845 and ZFN24844 construct), pDAB104009 (containing the ZFN24820 and ZFN24821 construct), pDAB104010 (containing the ZFN24828 and ZFN24829 construct) (FIG. 5), pDAB 104003 (containing the ZFN24810 and ZFN24811 construct), pDAB104011 (containing the ZFN24832 and ZFN24833 construct), pDAB104002 (containing the ZFN24794 and ZFN24795 construct), pDAB104006 (containing the ZFN24796 and ZFN24797 construct), pDAB104004 (containing the ZFN24814 and ZFN24815 construct), pDAB104001 (containing the ZFN24800 and ZFN24801 construct), pDAB104005 (containing the ZFN24818 and ZFN24819 construct), and pDAB104007 (containing the ZFN24836 and ZFN24837 construct) were confirmed via restriction enzyme digestion and via DNA sequencing. Table 10 lists the different constructs and the specific FAD sequence which each ZFN was designed to cleave and bind.

The resulting plasmid constructs; pDAB 107824 (ZFNs 28025-2A-28026), pDAB107815 (ZFNs 27961-2A-27962), pDAB107816 (ZFNs 27969-2A-27970), pDAB107817 (ZFNs 27973-2A-27974), pDAB 107825 (ZFNs 28035-2A-28036), pDAB107826 (ZFNs 28039-2A-28040), pDAB107818 (ZFNs 27987-2A-27988), pDAB107827 (ZFNs 28051-2A-28052), pDAB 107821 (ZFNs 28004-2A-28005), pDAB107819 (ZFNs 27989-2A-27990), pDAB 107828 (ZFNs 28053-2A-28054), pDAB107829 (ZFNs 28055-2A-28056), pDAB 107820 (ZFNs 27991-2A-27992), pDAB107822 (ZFNs 28021-2A-28022) and pDAB107823 (ZFNs 28023-2A-28024) were confirmed via restriction enzyme digestion and via DNA sequencing.

**Table 10: lists the Zinc Finger protein binding motif and the corresponding construct number. Each Zinc Finger was designed to bind and cleave the FAD2A which is described in the table.**

| **ZFN Design** | **Construct No.** | **Locus ID.** | **Target Cut Site in FAD2A Sequence** |
|---|---|---|---|
| 24844-2A-24845 | pDAB104008 | FAD2_ZFN_Locus1_F2A | 263-265 |
| 24820-2A-24821 | pDAB 104009 | FAD2_ZFN_Locus1_F2B | 265 |
| 24828-2A-24829 | pDAB104010 | FAD2_ZFN_Locus1_F2C | 275 |
| 24810-2A-24811 | pDAB 104003 | FAD2_ZFN_Locus2_F1D | 343-345 |
| 24832-2A-24833 | pDAB104011 | FAD2_ZFN_Locus2_F1E | 345-346 |
| 24794-2A-24795 | pDAB 104002 | FAD2_ZFN_Locus3_F2F | 402 |
| 24796-2A-24797 | pDAB 104006 | FAD2_ZFN_Locus3_F2G | 408 |
| 24814-2A-24815 | pDAB 104004 | FAD2_ZFN_Locus3_F2H | 408-410 |
| 24800-2A-24801 | pDAB 104001 | FAD2_ZFN_Locus4_F1J | 531 |
| 24818-2A-24819 | pDAB104005 | FAD2_ZFN_Locus4_F1K | 532-534 |
| 24836-2A-24837 | pDAB 104007 | FAD2_ZFN_Locus5_F1L | 724 |

### PREPARATION OF DNA FOR TRANSFECTION

Plasmid DNA of the above described vectors was sterilized by precipitation and washing in 100% (v/v) ethanol and dried in a laminar flow hood. The DNA pellet was suspended in 30 µL of sterile double-distilled water at a final concentration of 0.7 µg/µl for transfection into protoplast cells as described below. The preparation of the plasmid DNA was undertaken to result in supercoiled plasmid DNA for transient transfection and linearized plasmid DNA for stable transfection. The addition of carrier DNA (e.g. fish-sperm DNA) to the transforming plasmid was not required for the transient transfection of protoplast cells. For transient studies about 30 µg of plasmid DNA per 10⁶ protoplasts was used per transformation.

### TRANSFECTION

Transfection of *Brassica napus* L. var. DH10275 was completed as described in Spangenberg et al., (1986) Plant Physiology 66: 1-8, the media formulations are described in Spangenberg G. and Protrykus I. (1995) Polyethylene Glycol-Mediated Direct Gene Transfer in Tobacco Protoplasts. In: Gene Transfer to Plants. (Protrykus I. and Spangenberg G. Eds.) Springer-Verlag, Berlin. *Brassica napus* seeds were surface sterilized in 70% ethanol. The seeds were immersed in 12 mL of the 70% ethanol solution and mixed by gently rocking the cocktail for 10 minutes. The 70% ethanol solution was removed by decanting the solution and exchanged with a seed sterilization solution consisting of 1% w/v calcium hypochlorite and 0.1% v/v Tween-20. The seeds were immersed in the seed sterilization solution and mixed by gently rocking the cocktail for 25 minutes. The seed sterilization solution was decanted and the sterilized seeds were rinsed three times in 50 mL of sterile water. Finally, the seeds were transferred to a sterile 80 mm WHATMAN® filter paper disc (Fisher-Scientific, St. Louis, MO) that had been laid within a Petri dish and the seeds were lightly saturated with sterile water. The Petri dish was sealed with PARAFILM® (Fisher-Scientific, St. Louis, MO) and the plates were incubated at 25°C under complete darkness for one to two days. After signs of seedling emergence were observed from the seeds, the seedlings were transferred to Petri dish containing solidified GEM medium to encourage further seed germination. The seedlings were incubated on the GEM medium at 25°C for four to five days.

A volume of liquid PS medium (about 10 mL) was decanted into a sterile Petri dish. Using sterile forceps and a scalpel, an aerial portion of the four to five day old seedling in the 4-leaf stage of growth and development, was removed and discarded. Hypocotyl segments in lengths of 20-40 mm were determined to produce the highest population of small, cytoplasmic-rich protoplasts. The hypocotyl segments were aseptically excised and transferred to liquid PS medium. The excised hypocotyl segments were grouped together and cut transversely into 5-10 mm segments. Next, the hypocotyl segments were transferred to fresh PS medium and incubated at room temperature for 1 hour. The plasmolyzed hypocotyls were transferred to a Petri dish containing enzyme solution. Care was taken to immerse all of the hypocotyl segments into the solution. The Petri dishes were sealed with PARAFILM® and incubated overnight for sixteen to eighteen hours at 20 - 22°C with gentle rocking.

Protoplast cells were released from the hypocotyl segments. The overnight hypocotyl digests were gently agitated to release protoplasts into the enzyme solution. The Petri dish was angled slightly to aid the transfer of the digesting suspension which consisted of enzyme solution and plant debris. Using a 10 mL pipette the digesting suspension was transferred to a sterilized protoplast filtration (a filter of 100 micron mesh) unit to further separate the protoplasts from the plant debris. The filtration unit was tapped gently to release the excess liquid that had been caught in the sieve. The protoplast suspension, about 8 to 9 mL, was gently mixed and distributed into 14 mL sterile plastic round-bottomed centrifuge tubes. Each suspension was overlaid with 1.5 mL of W5 solution. The W5 solution was carefully dispensed over the protoplast suspension at an angle and dispensed drop-by-drop with minimal agitation. The addition of the W5 solution to the protoplast suspension resulted in the production of a protoplast rich interface. This interface was collected using a pipette. Next, the collected protoplasts were transferred into a new 14 mL centrifuge tube, and gently mixed. The yield or obtained protoplasts were determined using a hemocytometer to determine the number of protoplasts per milliliter. The method was repeated, wherein leaf tissue was digested to produce mesophyll protoplasts.

Next, W5 solution was added to a volume of 10 mL and the protoplasts were pelleted at 70 g, before removing the W5 solution. The remaining protoplast suspension was resuspended by gentle shaking. Each tube containing the protoplast suspension was filled with 5 mL of W5 solution and incubated at room temperature from one to four hours. The protoplast suspensions were pelleted at 70 g, and all of the W5 solution was removed. Next, 300 µL of transformation buffer was added to each of the pelleted protoplast suspensions which contained the isolated protoplasts. To each of the tubes, 10 µg of plasmid DNA was added to the protoplast suspensions. The plasmid DNA consisted of the Zinc Finger Nuclease constructs described above (*e.g.,* pDAB104010). Next, 300 µL of pre-warmed PEG 4000 solution was added to the protoplast suspension and the tubes were gently tapped. The protoplast suspensions and transformation mixture was allowed to incubate at room temperature for fifteen minutes without any agitation. An additional 10 mL of W5 solution was added to each tube in sequential aliquots of 1 mL, 1 mL, 1 mL, 2 mL, 2 mL, and 3 mL with gentle inversion of the tubes between each addition of W5 solution. The protoplasts were pelleted by spinning in a centrifuge at 70 g. All of the W5 solution was removed leaving a pure protoplast suspension.

Next, 0.5 mL of K3 medium was added to the pelleted protoplast cells and the cells were resuspended. The resuspended protoplast cells were placed in the center of a Petri dish and 5 mL of K3 and 0.6 mL Sea Plaque™ agarose (Cambrex, East Rutherford, NJ) in a 1:1 concentration. The Petri dishes were shaken in a single gentle swirling motion and left to incubate for 20-30 minutes at room temperature. The Petri dishes were sealed with PARAFILM® and the protoplasts were cultured for twenty-four hours in complete darkness. After the incubation in darkness, the Petri dishes were cultured for six days in dim light (5 µMol m⁻² s⁻¹ of Osram L36 W/21 Lumilux white tubes). After the culture step, a sterile spatula was used to divide the agarose containing the protoplasts into quadrants. The separated quadrants were placed into a 250 mL plastic culture vessel containing 20 mL of A medium and incubated on a rotary shaker at 80 rpm and 1.25 cm throw at 24 °C in continuous dim light for 14 days and then analyzed to determine the level of activity of each Zinc Finger Nuclease construct.

### GENOMIC DNA ISOLATION FROM CANOLA PROTOPLASTS

Transfected protoplasts were supplied in individual 1.5 or 2.0 mL microfuge tubes. The cells were pelleted at the base of the tube in a buffer solution. DNA extraction was carried out by snap freezing the cells in liquid nitrogen followed by freeze drying the cells, for about 48 hours in A LABCONCO FREEZONE 4.5® (Labconco, Kansas City, MO) at -40°C and about 133 x 10⁻³ mBar pressure. The lyophilized cells were subjected to DNA extraction using the DNEASY® (QIAGEN, Carlsbad, CA) plant kit following manufactures instructions, with the exception that tissue disruption was not required and the protoplast cells were added directly to the lysis buffer.

### TESTING OF FAD2A AND FAD3 ZFNs FOR GENOMIC DNA SEQUENCE CLEAVAGE IN CANOLA PROTOPLASTS

The design of the ZFN target sites for the FAD2A and FAD3 gene loci were clustered, so that multiple pairs of ZFN were design to overlap the target sites. The clustering of ZFN target sites enabled PCR primers to be designed that would amplify the surrounding genomic sequence from all FAD2A and FAD3 gene family members within a 100 bp window as to encapsulate all of the overlapping ZFN target sites. As such, the Illumina short read sequence technology could be used to assess the integrity of the target ZFN site of the transfected protoplasts. In addition, the PCR primers designed needed to include specific nucleotide bases that would attribute sequence reads to the specific gene member of the FAD2A and FAD3 family. Therefore, all of the PCR primers would be required to bind 5-10 nucleotides away from any ZFN target cut site as non-homologous end joining (NHEJ) activity is known to cause small deletions that could remove a priming site, inhibit amplification and therefore distort the assessment of NHEJ activity.

Primers were designed to bind to all of the ZFN target loci for the FAD2A and FAD3 gene families (Table 11) and were empirically tested for amplification of all gene family members through Sanger based sequencing of PCR amplification products. In several instances primers could not be developed that would distinguish all gene family members (Table 12 and Table 13), however in all instances the target gene sequences of FAD2A and FAD3, could be distinguished. Following PCR primer design custom DNA barcode sequences were incorporated into the PCR primers that were used to distinguish the different ZFN target loci and identify specific sequence reads to a transfection and ZFN (Tables 11, 12 and 13).

**Table 11: Primer sequences designed for FAD2 and FAD3 ZFN assessment of activity. Primers include custom barcodes, along with both requisite Illumina adaptor sequences for construction of Illumina library for sequencing-by-synthesis analysis. Purchased primer was the sum of all three columns presented.**

| **Locus ID** | **SEQ ID NO:** | **Illumina Adaptor Primer Sequence** | **Barcode** | **Locus Primer** |
|---|---|---|---|---|
| FAD2_ZFN_Locus1_F | 88 | | CGGG | |
| FAD2_ZFN_Locus1_F2A | 89 | | ACGTA | |
| FAD2_ZFN_Locus1_F2B | 90 | | CGTAC | |
| FAD2_ZFN_Locus1_F2C | 91 | | GTACG | |
| FAD2_ZFN_Locus2_F1D | 92 | | TACGT | |
| FAD2_ZFN_Locus2_F1E | 93 | | CTGAC | |
| FAD2_ZFN_Locus3_F2F | 94 | | TGACT | |
| FAD2_ZFN_Locus3_F2G | 95 | | GACTG | |
| FAD2_ZFN_Locus3_F2H | 96 | | ACTGA | |
| FAD2_ZFN_Locus4_F1J | 97 | | GCTAG | |
| FAD2_ZFN_Locus4_F1K | 98 | | CTAGC | |
| FAD2_ZFN_Locus5_F1L | 99 | | TAGCT | |
| FAD2_ZFN_Locus1_R1A | 100 | | ACGTA | |
| FAD2_ZFN_Locus1_R1B | 101 | | CGTAC | |
| FAD2_ZFN_Locus1_R1C | 102 | | GTACG | |
| FAD2_ZFN_Locus2_R1D | 103 | | TACGT | |
| FAD2_ZFN_Locus2_R1E | 104 | | CTGAC | |
| FAD2_ZFN_Locus3_R1F | 105 | | TGACT | |
| FAD2_ZFN_Locus3_R1G | 106 | | GACTG | |
| FAD2_ZFN_Locus3_R1H | 107 | | ACTGA | |
| FAD2_ZFN_Locus4_R1J | 108 | | GCTAG | |
| FAD2_ZFN_Locus4_R1K | 109 | | CTAGC | |
| FAD2_ZFN_Locus5_R1L | 110 | | TAGCT | |
| FAD3_ZFN_Locus1A_F3 | 111 | | ACGTA | |
| FAD3_ZFN_Locus1B_F3 | 112 | | CGTAC | |
| FAD3_ZFN_Locus2C_F1 | 113 | | CTGAC | |
| FAD3_ZFN_Locus3D_F1 | 114 | | TGACT | |
| FAD3_ZFN_Locus3E_F1 | 115 | | GACTG | |
| FAD3_ZFN_Locus3F_F1 | 116 | | ACTGA | |
| FAD3_ZFN_Locus4G_F1 | 117 | | GCTAG | |
| FAD3_ZFN_Locus4H_F1 | 118 | | CTAGC | |
| FAD3_ZFN_Locus5J_F1 | 119 | | TAGCT | |
| FAD3_ZFN_Locus6K_F1 | 120 | | TCAGT | |
| FAD3_ZFN_Locus6L_F1 | 121 | | CAGTC | |
| FAD3_ZFN_Locus6M_F1 | 122 | | AGTCA | |
| FAD3_ZFN_Locus6N_F1 | 123 | | GTCAG | |
| FAD3_ZFN_Locus7P_F3 | 124 | | GTACG | |
| FAD3_ZFN_Locus7Q_F3 | 125 | | TACGT | |
| FAD3_ZFN_Locus1A_R1 | 126 | | ACGTA | |
| FAD3_ZFN_Locus1B_R1 | 127 | | CGTAC | |
| FAD3_ZFN_Locus2C_R1 | 128 | | CTGAC | |
| FAD3_ZFN_Locus3D_R1 | 129 | | TGACT | |
| FAD3_ZFN_Locus3E_R1 | 130 | | GACTG | |
| FAD3_ZFN_Locus3F_R1 | 131 | | ACTGA | |
| FAD3_ZFN_Locus4G_R_uni | 132 | | GCTAG | |
| FAD3_ZFN_Locus4H_R_uni | 133 | | CTAGC | |
| FAD3_ZFN_Locus5J_R2 | 134 | | TAGCT | |
| FAD3_ZFN_Locus6K_R1 | 135 | | TCAGT | |
| FAD3_ZFN_Locus6L_R1 | 136 | | CAGTC | |
| FAD3_ZFN_Locus6M_R1 | 137 | | AGTCA | |
| FAD3_ZFN_Locus6N_R1 | 138 | | GTCAG | |
| FAD3_ZFN_Locus7P_R1 | 139 | | GTACG | |
| FAD3_ZFN_Locus7Q_R1 | 140 | | TACGT | |

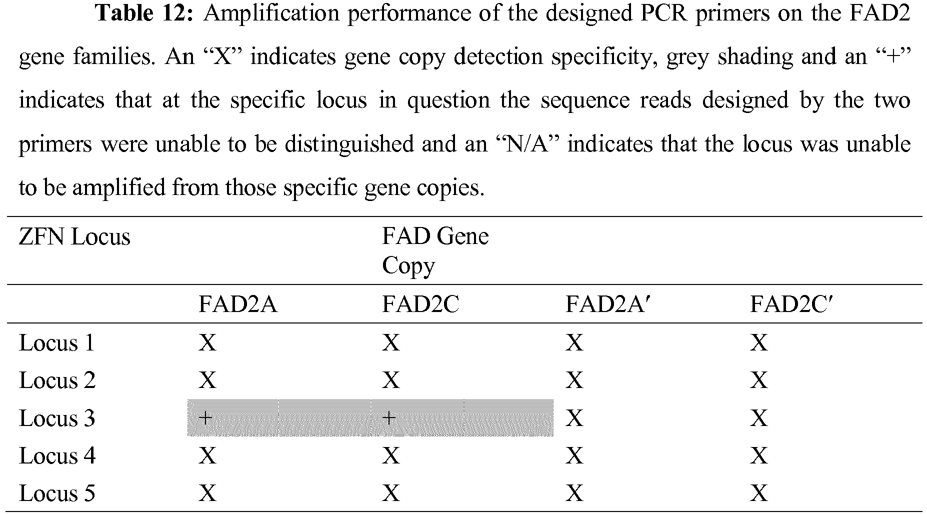

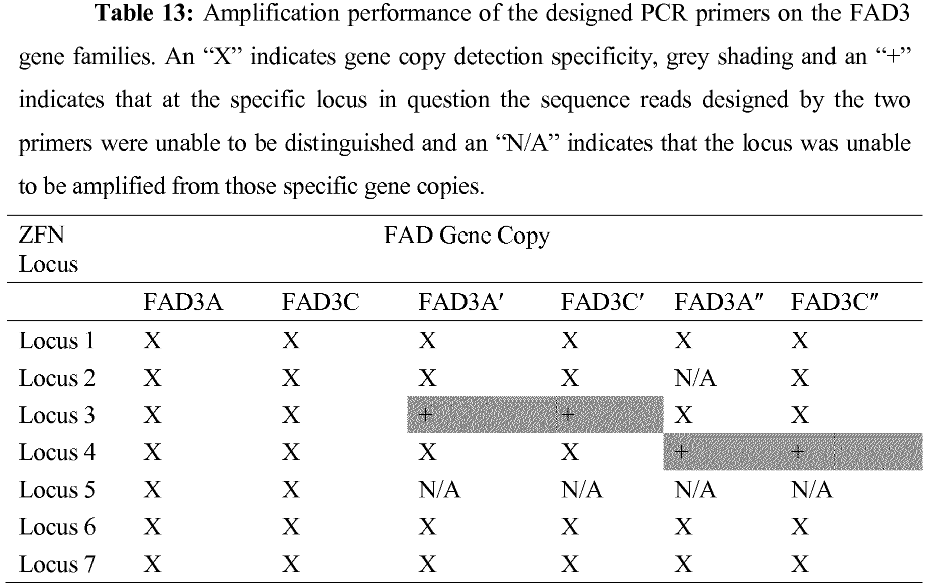

Following DNA extraction of canola protoplasts transfected with the ZFN, PCR amplification of the target ZFN loci was performed to generate the requisite loci specific DNA molecules in the correct format for Illumina based sequencing by synthesis technology. Each assay was optimised to work on 25 ng starting DNA (about 12,500 cell equivalents of the *Brassica napus* genome). Multiple reactions were performed, per sample to provide the coverage required to assess NHEJ efficiency and specificity at the appropriate level, about sixteen PCR reactions equivalent to 200,000 copies of the *Brassica napus* genome taken from individual protoplasts. PCR amplification master-mixes were made for all samples to be tested with the same assay and one reaction, performed in triplicate, was assayed using a quantitative PCR method that was used to determine the optimal number of cycles to perform on the target tissue, to ensure that PCR amplification had not become reagent limited and was still in an exponential amplification stage. The experimentation with the necessary negative control reactions, was performed in 96 well format using a MX3000P THERMOCYCLER® (Stratagene, LaJolla, CA). From the output gathered from the quantitative PCR platform, the relative increase in fluorescence was plotted from cycle-to-cycle and the cycle number was determined per assay that would deliver sufficient amplification, while not allowing the reaction to become reagent limited, in an attempt to reduce over cycling and the amplification of common transcripts or molecules. The unused master mix, remained on ice until the quantitative PCR analysis was concluded and the cycle number determined and was then aliquoted into the desired number of reaction tubes (about 16 per ZFN assay) and the PCR reaction was performed. Following amplification, samples for a single ZFN locus were pooled together and 200 µL of pooled product per ZFN was cleaned using the MINELUTE® PCR purification kit (Qiagen) following manufacturer's instructions. To enable the sample to be sequenced using the Illumina short read technology additional paired end primers were required to be attached by amplification onto the generated fragments. This was achieved by PCR amplification using primers that would be, in part complementary to the sequence added in the first round of amplification, but also contain the paired end sequence required. The optimal number of PCR cycles to perform, that would add the paired end sequences without over amplifying common fragments to the template was again determined using a sample pass through a quantitative PCR cycle analysis, as described previously. Following PCR amplification, the generated product was cleaned using a MINELUTE® column (Qiagen) following manufacturer's instructions and was resolved on a 2.5% agarose gel. DNA fragments visualised using SYBER SAFE® (Life Technologies, Carlsbad, CA) as bands of the correct size were gel extracted to remove any residual PCR generated primer-dimer or other spurious fragments, the DNA was extracted from the gel slice using a MINELUTE® gel extraction kit (Qiagen) following manufacturer's instructions. After completion of the gel extraction an additional clean up of the DNA was performed using AMPURE® magnetic beads (Beckman-Coulter, Brea, CA) with a DNA to bead ratio of 1:1.7. The DNA was then assessed for concentration using a quantitative PCR based library quantification kit for Illumina sequencing (KAPA) with a 1/40,000 and a 1/80,000 dilution and with the reaction being performed in triplicate. Based on the quantitative PCR results the DNA was diluted to a standard concentration of 2 nM and all libraries were combined for DNA sequencing. The samples were prepared for sequencing using a CBOT CLUSTER® generation kit (Illumina, San Diego, CA) and were sequenced on an ILLUMINA GA2x® with 100 bp paired-end sequencing reads following manufacturer's instructions.

### METHOD OF DATA ANALYSIS FOR DETECTION OF NON-HOMOLOGOUS END JOINING AT TARGET ZINC FINGER SITES

Following completion of the sequencing reaction and primary data calling performed using the Illumina bioinformatic pipeline for base calling, full analysis was performed to identify deleted bases at the target ZFN site in each instance. A custom PERL script was designed to extract and sort barcodes from DNA sequences computationally following a list of input sequences. The barcode had to match the reference sequence at a Phred score of greater than 30 to be accepted, to reduce misattributing sequence reads. After the sequence reads had been binned into the different barcode groups that had been used, a quality filter was passed across all sequences. The quality filter was a second custom developed PERL script. Sequence reads were excluded if there were more than three bases called as "N," or if the median Phred score was less than 20, or if there were 3 consecutive bases with a Phred score of less than 20, or if the sequence read was shorter than 40 bp in length. The remaining sequences were merged where both of the paired sequence reads were available using the NEXTGENE® (SoftGenetics, State College, PA) package. The remaining merged sequence reads were then reduced to a collection of unique sequence reads using a third custom PERL script with a count of the number of redundant sequences that had been identified recorded on the end of the remaining sequence identifier. The unique sequence reads were then aligned to the FAD2 and FAD3 reference sequence using the NEXTGENE® software that created a gapped FASTA aligned file.

Using the gapped FASTA file a conversion of the gapped base position number to the input reference was performed using a fourth custom PERL script. This enabled bases that discriminate the different gene family members (either homoeologous or paralogous sequence variation between the different gene family members) to be identified in the assembled data. Once the conversion of base numbering had been performed it was possible to generate haplotype reports for each unique sequence reads and assign the reads to specific gene family members. Once the reads had been grouped by gene a 10 bp window was identified and assessed that surrounded the ZFN target site. The number of sequences with deletions was recorded per gene along with the number of missing bases.

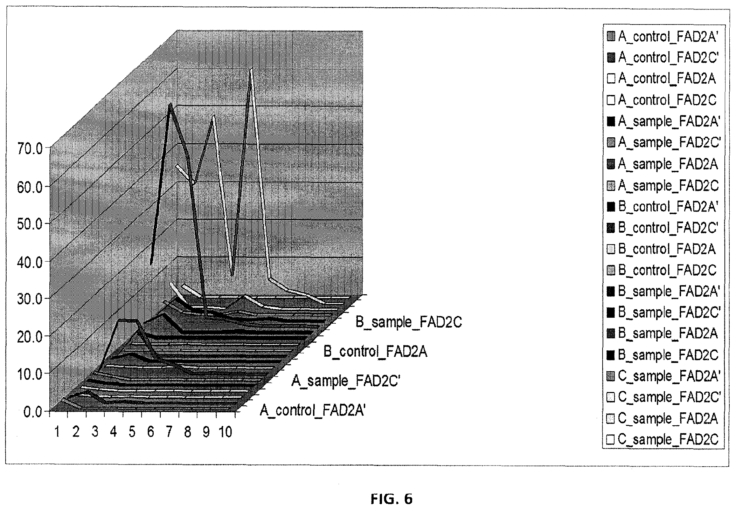

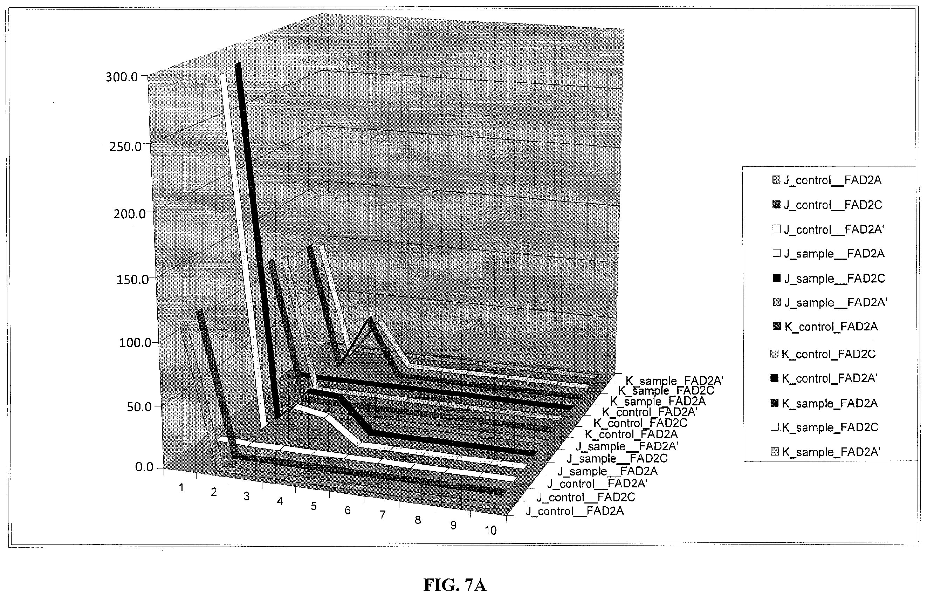

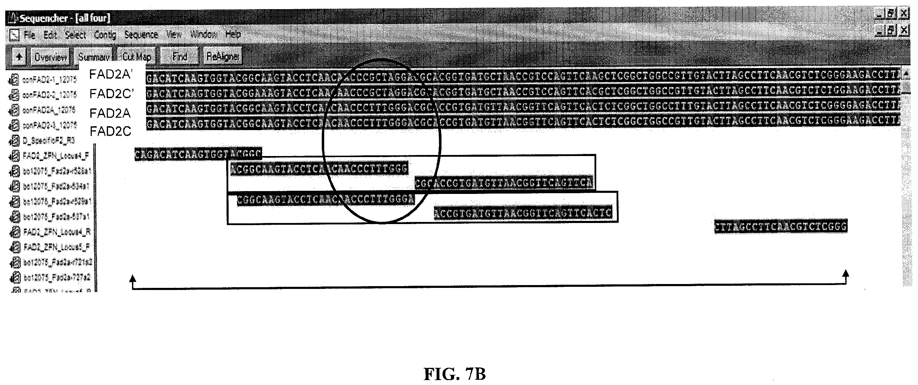

The data was then graphically displayed as a multiple line graph, with the number of sequences with 1 through 10 bases deleted at the target ZFN site per 10,000 sequence reads (FIG. 6). This analysis was performed for all ZFN transfections along with control transfections. In several instances, repeats in the native DNA sequence lead to an increase in sequencing error in the target ZFN site, such an error can be commonly seen as an increase in the prevalence of single base deletions that were reported in all samples, both transfected with ZFN or controls (FIG. 7).

From these results highest level of ZFN activity at a FAD2 target site, as determined by the greater activity of NHEJ, was identified at locus E. The ZFNs which were encoded on plasmid pDAB104010 (*i.e.,* ZFN24828 and 24829) were selected for *in planta* targeting of an Engineered Transgene Integration Platform (ETIP) given its characteristics of significant genomic DNA cleavage activity and minimal non-target activity.

### EXAMPLE 4: DNA CONSTRUCTS FOR ENGINEERED TRANSGENE INTEGRATION PLATFORM (ETIP) CANOLA PLANT LINES

The plasmid vector constructs described below were built using methods and techniques commonly known by one with skill in the art. The application of specific reagents and techniques described within this paragraph are readily known by those with skill in the art, and could be readily interchanged with other reagents and techniques to achieve the desired purpose of building plasmid vector constructs. The restriction endonucleases were obtained from New England BioLabs (NEB; Ipswich, MA). Ligations were completed with T4 DNA Ligase (Invitrogen, Carlsbad, CA). Gateway reactions were performed using GATEWAY® LR CLONASE® enzyme mix (Invitrogen) for assembling one entry vector into a single destination vector. IN-FUSION™ reactions were performed using IN-FUSION™ Advantage Technology (Clontech, Mountain View, CA) for assembling one entry vector into a single destination vector Plasmid preparations were performed using NUCLEOSPIN® Plasmid Kit (Macherey-Nagel Inc., Bethlehem, PA) or the Plasmid Midi Kit® (Qiagen) following the instructions of the suppliers. DNA fragments were isolated using QIAquick Gel Extraction Kit™ (Qiagen) after agarose Tris-acetate gel electrophoresis. Colonies of all assembled plasmids were initially screened by restriction digestion of miniprep DNA. Plasmid DNA of selected clones was sequenced by a commercial sequencing vendor (Eurofins MWG Operon, Huntsville, AL). Sequence data were assembled and analyzed using the SEQUENCHER™ software (Gene Codes Corp., Ann Arbor, MI).

### DIRECT-DELIVERY VECTORS FOR PRECISION INTEGRATION OF ETIP IN THE FAD2A LOCUS OF CANOLA

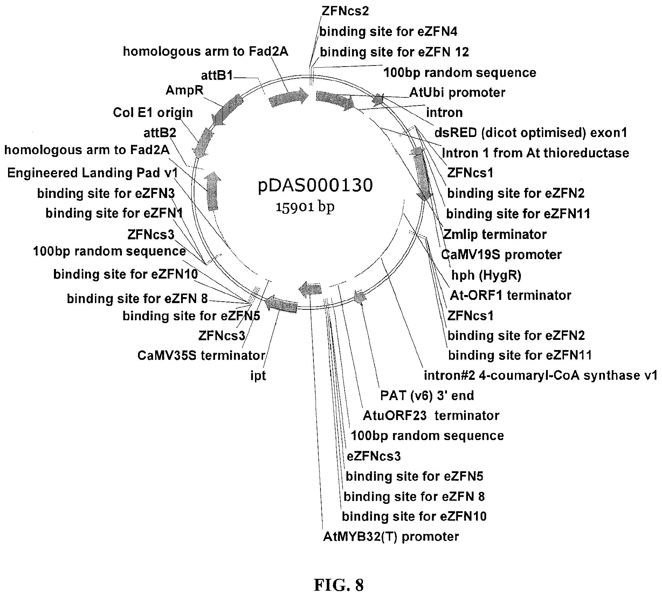

Standard cloning methods were used in the construction of the ETIP-containing vectors pDAS000130 (FIG. 8, T-strand insert as SEQ ID NO:141), for specific integration into the FAD2A gene *of B. napus.* This construct has been designed to be delivered into canola protoplasts with the Zinc Finger Nuclease construct pDAB1004010. The Zinc Finger Nuclease Construct will cleave the FAD2A locus and then the pDAS000130 construct will integrated within the canola genome via a homology directed repair mechanism. The ETIP consists of four expression cassettes (two incomplete) separated by additional ZFN recognition sequences and an Engineered Landing Pad (ELP) containing another ZFN recognition sequences. The additional ZFN recognition sequences are unique and have been designed to be targeted for the introduction of polynucleotide sequences within the ETIP and ELP transgene insertions. Similarly, the ZFN recognition sequences can be utilized for excision of polynucleotide sequences. The first gene expression cassette was an incomplete dsRED expression cassette and contained the promoter, 5' untranslated region and intron from the *Arabidopsis thaliana* Polyubiquitin 10 (AtUbi promoter) gene (Callis, et al., (1990) J. Biol. Chem., 265: 12486-12493) followed by 210 bp of a dsRed gene from the reef coral *Discosoma* sp. (Clontech, Mountain View, CA) codon-optimized for expression in dicot plants (ds RED (dicot optimized)exon 1) followed by an intron from the *Arabidopsis thaliana* thioreductase-like gene (Intron 1 from At thioreductase: Accession No: NC_00374) and the 3' untranslated region comprising the transcriptional terminator and polyadenylation site of the Zea mays Viviparous-1 (Vp1) gene (Zmlip terminator: Paek et al., (1998) Molecules and Cells , 8(3): 336-342). The second expression cassette contained the 19S promoter including 5' UTR from cauliflower mosaic virus (CaMV 19S: Cook and Penon (1990) Plant Molecular Biology 14(3): 391-405) followed by the *hph* gene from *E. coli*, codon-optimized for expression in dicots (hph(HygR): Kaster et al., (1983) Nucleic Acids Research 11(19): 6895-6911) and the 3'UTR comprising the transcriptional terminator and polyadenylation site of open reading frame 1 of *A. tumefaciens* pTi15955 (At-ORFl terminator: Barker et al., (1983) Plant Molecular Biology 2(6): 335-50). The third expression cassette was an incomplete PAT expression cassette and contained the first intron from Arabidopsis 4-coumaryl-CoA synthase (intron#2 4-coumaryl-CoA synthase v: Accession No: At3g21320/NC003074) followed by the last 256 bp of a synthetic, plant-optimized version of phosphinothricin acetyl transferase gene, isolated from *Streptomyces viridochromogenes*, which encodes a protein that confers resistance to inhibitors of glutamine synthetase comprising phophinothricin, glufosinate, and bialaphos (PAT(v6) 3'end: Wohlleben et al., (1988) Gene 70(1): 25-37). This cassette was terminated with the 3' UTR comprising the transcriptional terminator and polyadenylation sites of open reading frame 23 of *A. tumefaciens* pTi15955 (AtuORF23 terminator: Barker et al., (1983) Plant Molecular Biology 2(6): 335-50). The fourth Expression Cassette was the *ipt* gene cassette and contained a 588 bp truncated version of the promoter and 5' UTR from the Arabidopsis DNA-binding protein MYB32 gene (U26933) (AtMYB32(T) promoter: Li et al., (1999) Plant Physiology 121: 313) followed by the isopentyl transferase (*ipt*) gene from *A. tumefaciens* and the 35s terminator comprising the transcriptional terminator and polyadenylation sites from cauliflower mosaic virus (CaMV 35S terminator: Chenault et al., (1993) Plant Physiology 101 (4): 1395-1396). For delivery to FAD2A, each end of the ETIP sequence was flanked by 1kb of FAD2A genomic sequence from either side of the location of the double-stranded break induced by delivery of the ZFN encoded in pDAB104010 to the FAD2A gene of *B. napus.*

The ETIP sequence was synthesized by a commercial gene synthesis vendor (GeneArt, Life Technologies). The 1 kb segments of FAD2A genome sequence were amplified from genomic DNA purified from leaf tissue of *B. napus* DH12075 using a Qiagen DNEASY® plant mini kit (Qiagen, Hilden) following instructions supplied by the manufacturer. The 1 kb FAD2A sequences were ligated into the ETIP vector using T4 ligase (NEB, Ipswich, MA). Colonies of all assembled plasmids were initially screened by restriction digestion of miniprep DNA. Restriction endonucleases were obtained from New England BioLabs (NEB, Ipswich, MA) and Promega (Promega Corporation, WI). Plasmid preparations were performed using the QIAPREP *SPIN MINIPREP*® Kit (Qiagen) or the Pure Yield Plasmid MAXIPREP® system (Promega Corporation, WI) following the instructions of the suppliers. Plasmid DNA of selected clones was sequenced using ABI Sanger Sequencing and BIG DYE TERMINATOR® v3.1 cycle sequencing protocol (Applied Biosystems, Life Technologies). Sequence data were assembled and analyzed using the software (Gene Codes Corp., Ann Arbor, MI).

### DIRECT-DELIVERY VECTORS FOR PRECISION INTEGRATION OF ETIP IN THE FAD 3 LOCUS OF CANOLA

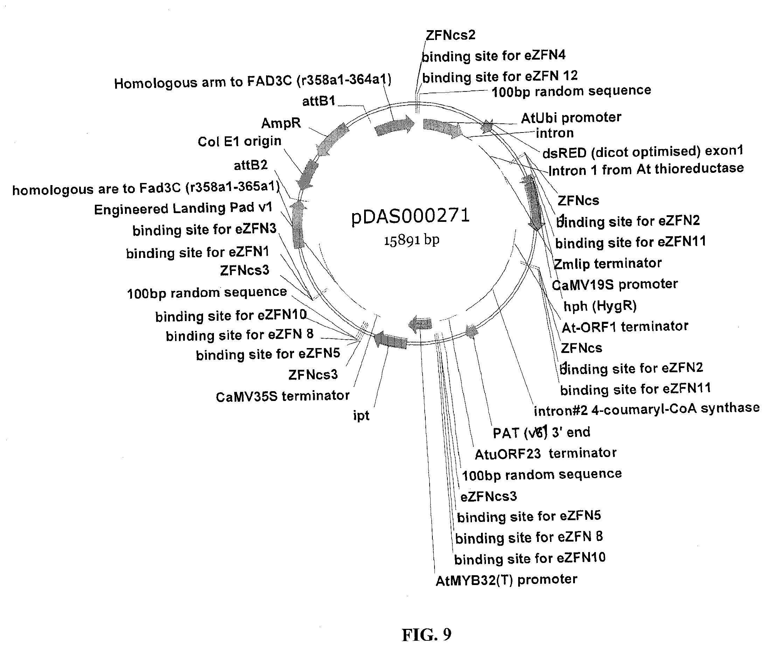

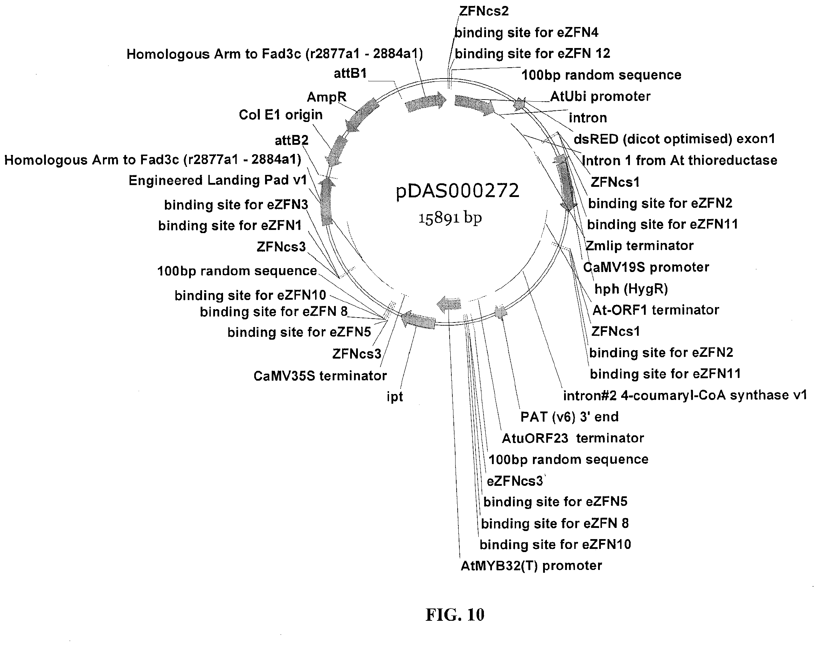

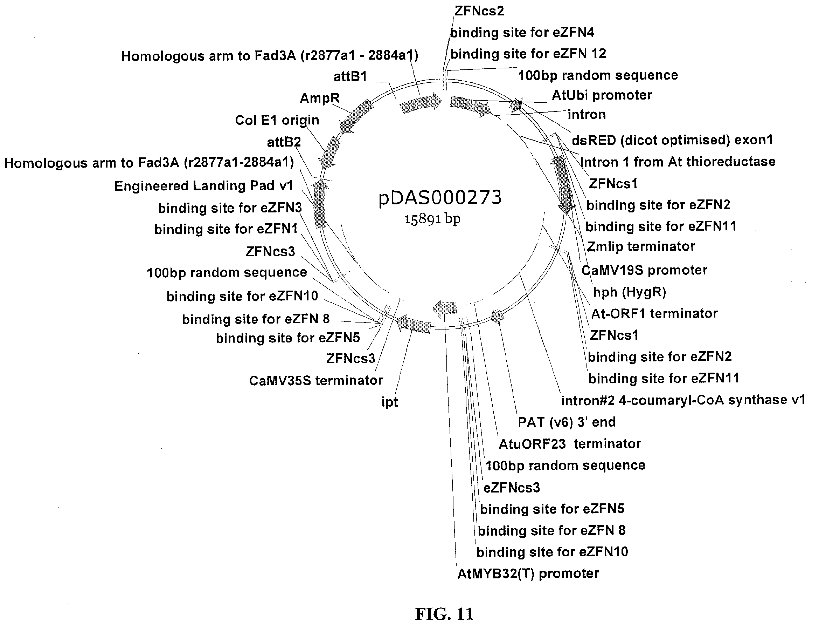

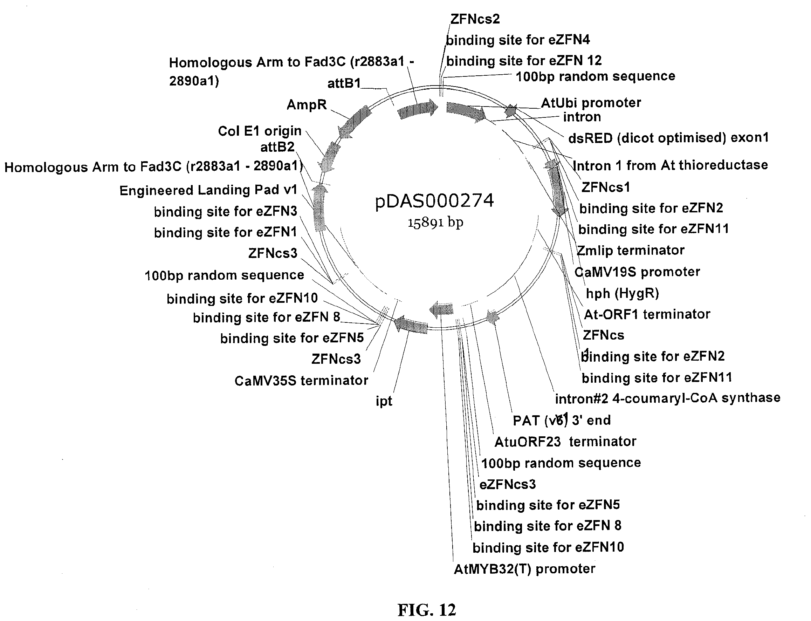

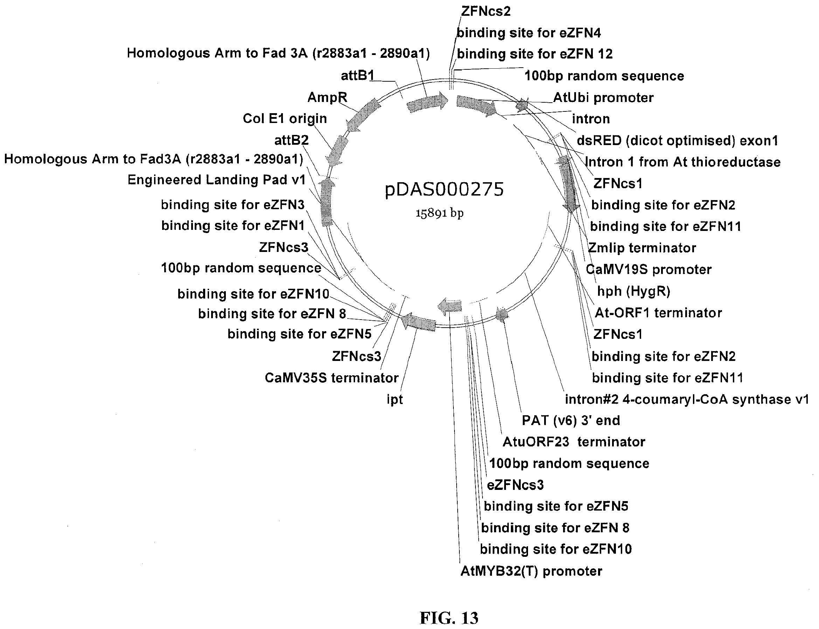

Standard cloning methods were used in the construction of the ETIP-containing vectors pDAS000271 (FIG. 9, T-strand insert as SEQ ID NO:142), pDAS000272 (FIG. 10, T-strand insert as SEQ ID NO:143), pDAS000273 (FIG. 11, T-strand insert as SEQ ID NO:144), pDAS000274 (FIG. 12, T-strand insert as SEQ ID NO:145), and pDAS000275 (FIG. 13, T-strand insert as SEQ ID NO:146) for specific integration into the FAD3A or FAD3C gene locus of *B. napus.* This construct has been designed to be delivered into canola protoplasts with the Zinc Finger Nuclease construct pDAB 107828 or pDAB107829. A specific Zinc Finger Nuclease Construct will cleave the FAD3A locus and then the pDAS000273 or pDAS275 construct will integrate within the canola genome via a homology directed repair mechanism. Likewise, a specific Zinc Finger Nuclease Construct will cleave the FAD3C locus and then the pDAS000271, pDAS000272 or pDAS000274 construct will integrate within the canola genome via a homology directed repair mechanism. The ETIP consists of four expression cassettes (two incomplete) separated by additional ZFN recognition sequences and an Engineered Landing Pad (ELP) containing another ZFN recognition sequences. The additional ZFN recognition sequences are unique and have been designed to be targeted for the introduction of polynucleotide sequences within the ETIP and ELP transgene insertions. Similarly, the ZFN recognition sequences can be utilized for excision of polynucleotide sequences. The first gene expression cassette was an incomplete dsRED expression cassette and contained the promoter, 5' untranslated region and intron from the *Arabidopsis thaliana* Polyubiquitin 10 (AtUbi promoter) gene (Callis, et al., (1990) J. Biol. Chem., 265: 12486-12493) followed by 210 bp of a dsRed gene from the reef coral *Discosoma* sp. (Clontech, Mountain View, CA) codon-optimized for expression in dicot plants (ds RED (dicot optimized)exon 1) followed by an intron from the *Arabidopsis thaliana* thioreductase-like gene (Intron 1 from At thioreductase: Accession No: NC_00374) and the 3' untranslated region comprising the transcriptional terminator and polyadenylation site of the Zea mays Viviparous-1 (Vp1) gene (Zmlip terminator: Paek et al., (1998) Molecules and Cells , 8(3): 336-342). The second expression cassette contained the 19S promoter including 5' UTR from cauliflower mosaic virus (CaMV 19S: Cook and Penon (1990) Plant Molecular Biology 14(3): 391-405) followed by the *hph* gene from *E. coli*, codon-optimized for expression in dicots (hph(HygR): Kaster et al., (1983) Nucleic Acids Research 11(19): 6895-6911) and the 3'UTR comprising the transcriptional terminator and polyadenylation site of open reading frame 1 of *A. tumefaciens* pTi15955 (At-ORFl terminator: Barker et al., (1983) Plant Molecular Biology 2(6): 335-50). The third expression cassette was an incomplete PAT expression cassette and contained the first intron from Arabidopsis 4-coumaryl-CoA synthase (intron#2 4-coumaryl-CoA synthase v: Accession No: At3g21320/NC003074) followed by the last 256 bp of a synthetic, plant-optimized version of phosphinothricin acetyl transferase gene, isolated from *Streptomyces viridochromogenes*, which encodes a protein that confers resistance to inhibitors of glutamine synthetase comprising phophinothricin, glufosinate, and bialaphos (PAT(v6) 3' end: Wohlleben et al., (1988) Gene 70(1): 25-37). This cassette was terminated with the 3' UTR comprising the transcriptional terminator and polyadenylation sites of open reading frame 23 of *A. tumefaciens* pTi15955 (AtuORF23 terminator: Barker et al., (1983) Plant Molecular Biology 2(6): 335-50). The fourth Expression Cassette was the *ipt* gene cassette and contained a 588 bp truncated version of the promoter and 5' UTR from the Arabidopsis DNA-binding protein MYB32 gene (U26933) (AtMYB32(T) promoter: Li et al., (1999) Plant Physiology 121: 313) followed by the isopentyl transferase (*ipt*) gene from *A. tumefaciens* and the 35s terminator comprising the transcriptional terminator and polyadenylation sites from cauliflower mosaic virus (CaMV 35S terminator: Chenault et al., (1993) Plant Physiology 101 (4): 1395-1396). For delivery to FAD3A or FAD3C, each end of the ETIP sequence was flanked by 1 kb of FAD3A or FAD3C genomic sequence from either side of the location of the double-stranded break induced by delivery of the ZFN encoded in FAD3A or FAD3c gene of *B. napus.*

The ETIP sequence was synthesized by a commercial gene synthesis vendor (GeneArt, Life Technologies). The 1 kb segments of FAD3A and FAD3C genome sequence were amplified from genomic DNA purified from leaf tissue of *B. napus* DH12075 using a Qiagen DNEASY® plant mini kit (Qiagen, Hilden) following instructions supplied by the manufacturer. The 1 kb FAD3A or FAD3C sequences were ligated into the ETIP vector using T4 ligase (NEB, Ipswich, MA). Colonies of all assembled plasmids were initially screened by restriction digestion of miniprep DNA. Restriction endonucleases were obtained from New England BioLabs (NEB, Ipswich, MA) and Promega (Promega Corporation, WI). Plasmid preparations were performed using the QIAPREP *Spin Miniprep*® Kit (Qiagen) or the Pure Yield Plasmid MAXIPREP System® (Promega Corporation, WI) following the instructions of the suppliers. Plasmid DNA of selected clones was sequenced using ABI Sanger Sequencing and BIG DYE TERMINATOR® v3.1 cycle sequencing protocol (Applied Biosystems, Life Technologies). Sequence data were assembled and analyzed using the software (Gene Codes Corp., Ann Arbor, MI).

### CONTROL VECTORS

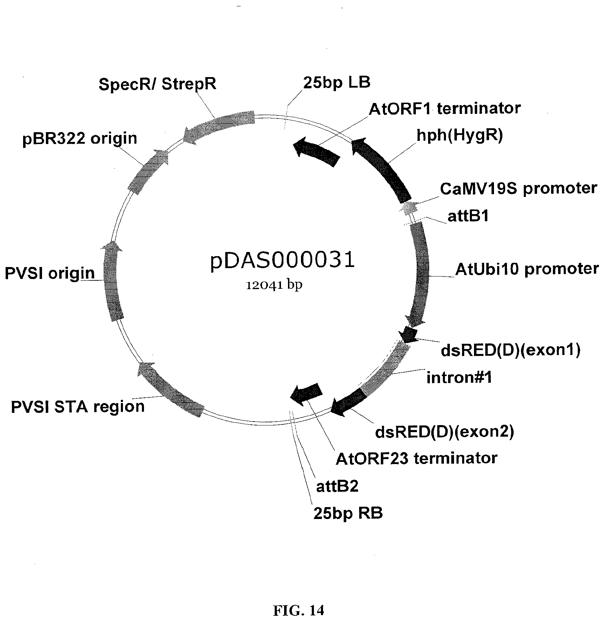

A control vector was used to develop a Fluorescence Activated Cell Sorting (FACS) cell based sorting method. Standard cloning methods were used in the construction of a control vector, pDAS000031 (FIG. 14: T-strand insert as SEQ ID NO:147) consisting of two gene expression cassettes. The first gene expression cassette contained the Cauliflower mosaic virus 19s promoter (CaMV 19S promoter; Shillito, et al., (1985) Bio/Technology 3; 1099-1103):: hygromycin resistance gene (hph(HygR);US Patent No. 4,727,028) :: and the *Agrobacterium tumefaciens* Open Reading Frame 1 3' UnTranslated Region (AtORF1 terminator; Huang et al., (1990) J. Bacteriol. 1990 172:1814-1822). The second gene expression cassette contained the *Arabidopsis thaliana* Ubiquitin 10 promoter (AtUbi10 promoter; Callis, et al., (1990) J. Biol. Chem., 265: 12486-12493):: dsRED (dsRED(D); US Patent No. 6,852,849) and an intron from Arabidopsis (intron #1; GenBank: AB025639.1) :: *Agrobacterium tumefaciens* Open Reading Frame 23 3' UnTranslated Region (AtORF23 terminator; US Patent No. 5,428,147) as an in-frame fusion with a *trans* orientation (*e*.*g*., head to head orientation). The plasmid vector was assembled using the IN-FUSION™ Advantage Technology (Clontech, Mountain View, CA).

### CONSTRUCTION OF BINARY VECTORS FOR RANDOM INTEGRATION OF ETIP IN CANOLA

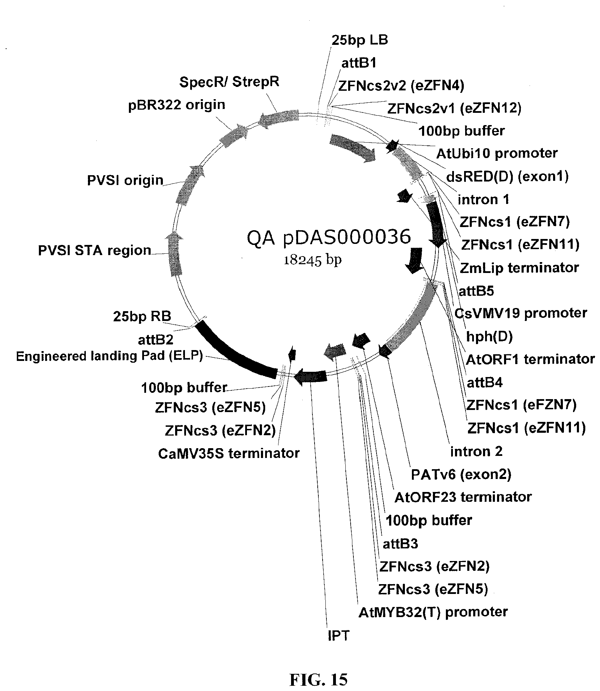

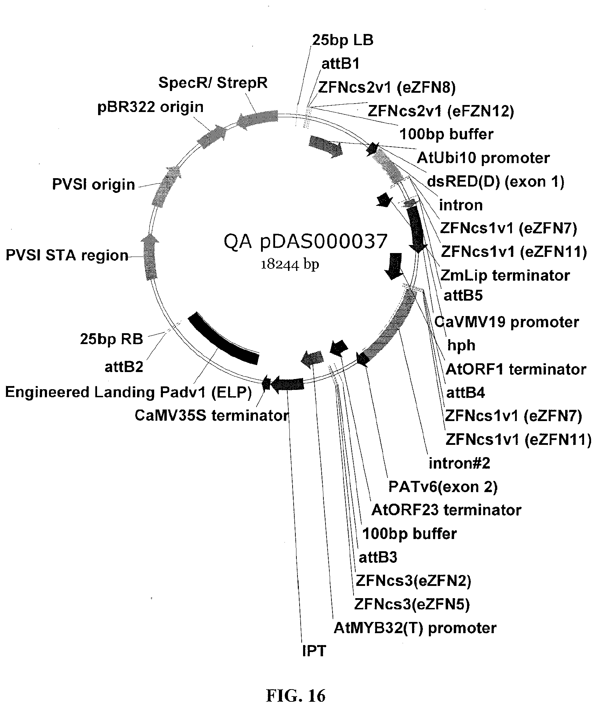

Two binary vectors were constructed for random integration of an ETIP T-Strand sequence within the genome of *Brassica napus.* Standard cloning methods were used in the construction of the ETIP-containing vectors pDAS000036 (FIG. 15, T-strand insert as SEQ ID NO:148) and pDAS000037 (FIG. 16, T-strand insert as SEQ ID NO:149). The ETIP vectors consist of four expression cassettes (two incomplete expression cassettes) separated by ZFN recognition sequences and an Engineered Landing Pad (ELP) containing further ZFN recognition sequences. The first gene expression cassette was an incomplete dsRED expression cassette and contained the promoter, 5' untranslated region and intron from the Arabidopsis thaliana polyubiquitin 10 (AtUbi10 promoter) gene (Norris et al., (1993) Plant Molecular Biology, 21(5): 895-906) followed by 210 bp of a dsRed gene from the reef coral *Discosoma* sp. (Clontech, Mountain View, CA) codon-optimized for expression in dicot plants followed by an intron from the Arabidopsis thioreductase-like gene (Accession No: NC_00374) and the 3' untranslated region (UTR) comprising the transcriptional terminator and polyadenylation site of the *Zea mays* Viviparous-1 (Vp1) gene (Zm Lip terminator: Paek et al., (1998) Molecules and Cells, 8(3): 336-342). The second expression cassette contained the 19S promoter including 5' UTR from cauliflower mosaic virus (CsVMV 19 promoter: Cook and Penon (1990) Plant Molecular Biololgy 14(3): 391-405) followed by the *hph* gene from *E. coli* codon-optimized for expression in dicots (hph(D): Kaster et al (1983) Nucleic Acids Research, 11(19): 6895-6911) and the 3'UTR comprising the transcriptional terminator and polyadenylation site of open reading frame 1 (ORF1) of *A. tumefaciens* pTi15955 (AtORF1 terminator: Barker et al., (1983) Plant Molecular Biology, 2(6): 335-50). The third expression cassette was an incomplete PAT expression cassette and contained the first intron from Arabidopsis 4-coumaryl-CoA synthase (Accession No: At3g21320 (NC003074)) followed by the last 256 bp of a synthetic, plant-optimized version of phosphinothricin acetyl transferase (PAT) gene, isolated from *Streptomyces viridochromogenes*, which encodes a protein that confers resistance to inhibitors of glutamine synthetase comprising phophinothricin, glufosinate, and bialaphos (PATv6(exon2); Wohlleben et al., (1988) Gene, 70(1): 25-37). This cassette was terminated with the 3' UTR comprising the transcriptional terminator and polyadenylation sites of open reading frames 23 (ORF23) of *A. tumefaciens* pTi15955 (AtORF23 terminator; Barker et al., (1983) Plant Molecular Biology, 2(6): 335-50). The fourth Expression Cassette was the *ipt* gene cassette and contained a 588 bp truncated version of the promoter and 5' UTR from the Arabidopsis DNA-binding protein MYB32 gene (U26933) (AtMYB32(T)promoter; Li et al., (1999) Plant Physiology, 121: 313) followed by the isopentyl transferase (*ipt*) gene from *A. tumefaciens* and the 35s terminator comprising the transcriptional terminator and polyadenylation sites from cauliflower mosaic virus (CaMV 35 S terminator; Chenault et al., (1993) Plant Physiology, 101 (4): 1395-1396).

The expression cassettes and ELP were synthesized with Multi-Gateway sites by a commercial gene synthesis vendor (GeneArt, Life Technologies). Entry clones were constructed of each expression cassette and ELP using BP clonase II enzyme mix™ (Invitrogen, Life Technologies) and the pDONR221 vector suite™ (Invitrogen, Life Technologies). The Entry clones were then used in a Multi-Gateway reaction with a Gateway-enabled binary vector using LR Clonase II Plus Enzyme mix™ (Invitrogen, Life Technologies). Colonies of all assembled plasmids were initially screened by restriction digestion of miniprep DNA. Restriction endonucleases were obtained from New England BioLabs (NEB; Ipswich, MA) and Promega (Promega Corporation, WI). Plasmid preparations were performed using the QIAprep *Spin Miniprep* Kit™ (Qiagen, Hilden) or the Pure Yield Plasmid Maxiprep System™ (Promega Corporation, WI) following the instructions of the suppliers. Plasmid DNA of selected clones was sequenced using ABI Sanger Sequencing and Big Dye Terminator v3.1 cycle sequencing protocol™ (Applied Biosystems, Life Technologies). Sequence data were assembled and analyzed using the software (Gene Codes Corporation, Ann Arbor, MI).

### EXAMPLE 5: GENERATION OF ETIP CANOLA PLANT LINES

### TRANSFORMATION OF Brassica napus

The ETIP constructs (pDAS000036, pDAS000037), the DS-Red control construct (pDAS000031), and the FAD2A, FAD3A, and FAD3C site specific constructs (pDAS000130, and pDAS000271-pDAS000275) and accompanying Zinc Finger Nuclease (pDAB104010, pDAB 10728, and pDAB 10729) described in Example 4. The binary vectors were transformed into *Agrobacterium tumefaciens* strain GV3101: PM90. Transformation of *Brassica napus* protoplast cells was completed using the transfection protocol described in Example 3 with some modification.

The modifications to the protocol included the use of sodium alginate instead of Sea Plaque™ agarose. The transfection experiments in which both the Zinc Finger Nuclease construct and the ETIP construct were co-delivered into *Brassica napus* protoplast cells were completed at DNA concentrations comprising a 5:1 molar ratio of plasmid DNA. The other ETIP and control plasmid constructs were transformed at concentrations of 30 µg of plasmid DNA. As such, pDAS000130 consisted of a concentration of 27.8 µg of plasmid DNA and pDAB104010 consisted of a concentration of 2.2 µg of plasmid DNA. The other ETIP and control plasmid constructs were transformed at concentrations of 30 µg of plasmid DNA.

Additional modifications to the protocol included the propagation of whole plants from the transformed protoplast cells in medium containing 1.5 mg/mL of hygromycin. The propagation of whole plants required that the A medium was replaced every two weeks and the growth of the protoplast-derived colonies was monitored. After the protoplast-derived colonies had grown to approximately 2-3 mm in diameter, the colonies were transferred into individual wells of a 12-well COSTAR® plate (Fisher Scientific, St. Louis, MO) containing solidified MS morpho medium. The plates were incubated for one to two weeks at 24 °C under continuous dim light until the calli had proliferated to a size of 8-10 mm in diameter. After the protoplast cells had reached a diameter of 1-2 cm in diameter, the protoplast cells were transferred to individual 250 mL culture vessels containing MS morpho medium. The vessels were incubated at 24 °C under 16 h light (20 µMol m⁻² s⁻¹ of Osram L36 W/21 Lumilux white tubes) and 8 h dark conditions. Within one to two weeks, multiple shoots were visible. The shoots were transferred into 250 mL culture vessels containing MS medium after they reached a length of 3-4 cm. The 250 mL culture vessels were incubated at 24 °C under 16 h light (20 µMol m⁻² s⁻¹ of Osram L36 W/21 Lumilux white tubes) and 8h dark conditions. The shoots were maintained in the culture vessels until they developed into plantlets at which time they were transferred to a greenhouse to grow to maturity.

### EXAMPLE 6: MOLECULAR CONFIRMATION OF INTEGRATION OF T-DNAS CONTAINING ETIPS IN CANOLA

Genomic DNA was extracted from leaf tissue of all putative transgenic plants using a DNEASY® 96 Plant DNA extraction kit™ or a DNEASY® Plant Mini Kit™ (Qiagen). The genomic DNA from each plant was analyzed by PCR using primers designed to amplify *vir*C from pTiC58 Forward (SEQ ID NO:150 CGAGAACTTGGCAATTCC) and pTiC58 Reverse (SEQ ID NO:151 TGGCGATTCTGAGATTCC) to test for persistence of *A.tumfaciens*, primers designed to amplify actin from *B. napus*; Actin Forward (SEQ ID NO:152 GACTCATCGTACTCTCCCTTCG) and Actin Reverse (SEQ ID NO:153 GACTCATCGTACTCTCCCTTCG) to check the quality of the genomic DNA. Primers were designed to amplify the *hph* gene; HPH Forward (SEQ ID NO:154 TGTTGGTGGAAGAGGATACG) and HPH Reverse (SEQ ID NO:155 ATCAGCAGCAGCGATAGC) encoded by the ETIP. Plants that did not give a product from *vir*C primers but from which products of the correct size were amplified with primers to actin and *hph* were classified as transgenic.

A second screen was completed, where gDNA from each transgenic plant was analyzed by PCR using five sets of primers designed to amplify the binary vector outside of the T-DNA region [(IF SEQ ID NO:156 ATGTCCACTGGGTTCGTGCC; 1R SEQ ID NO:157 GAAGGGAACTTATCCGGTCC) (2F SEQ ID NO:158 TGCGCTGCCATTCTCCAAAT; 2R SE ID NO:159 ACCGAGCTCGAATTCAATTC) (3F SEQ ID NO:160 CCTGCATTCGGTTAAACACC; 3R SEQ ID NO:161 CCATCTGGCTTCTGCCTTGC) (4F SEQ ID NO:162 ATTCCGATCCCCAGGGCAGT; 4R SEQ ID NO:163 GCCAACGTTGCAGCCTTGCT) (5F SEQ ID NO:164 GCCCTGGGATGTTGTTAAGT; 5R SEQ ID NO:165 GTAACTTAGGACTTGTGCGA)]. Plants from which PCR products of the correct and expected size were amplified with primer sets 3 and 4 were considered to have backbone integration.

DNA from plants with no backbone integration was purified from 20 g of leaf tissue using a modified CTAB method (Maguire et al,. (1994) Plant Molecular Biology Reporter, 12(2): 106-109). The isolated gDNA was digested with several restriction enzymes and 10 µg of gDNA was separated by electrophoresis on an agarose gel and transferred to membrane using a standard Southern blotting protocol. Membranes were probed using the DIG Easy Hyb System™ (Roche, South San Francisco, CA) following the manufacturer's instructions. Probes to each expression cassette to the ELP and to an endogenous control gene, actin, were amplified from the ETIP construct using the following primers: (IPT-F SEQ ID NO:166 TCTCTACCTTGATGATCGG; IPT-R SEQ ID NO:167 AACATCTGCTTAACTCTGGC; dsRED-F SEQ ID NO:168 ATGGCTTCATCTGAGAACG; dsRED-R SEQ ID NO:169 TTCCGTATTGGAATTGAGG; PAT-F SEQ ID NO:170 TTGCTTAAGTCTATGGAGGCG; PAT-R SEQ ID NO:171 TGGGTAACTGGCCTAACTGG; ELP-F SEQ ID NO:172 ATGATATGTAGACATAGTGGG; ELP-R SEQ ID NO:173 AGGGTGTAAGGTACTAGCC; Hph-F SEQ ID NO:174 TGTTGGTGGAAGAGGATACG; Hph-R SEQ ID NO:175 ATCAGCAGCAGCGATAGC; actin-F SEQ ID NO:176 GTGGAGAAGAACTACGAGCTACCC; actin-R SEQ ID NO:177 GACTCATCGTACTCTCCCTTCG).

The ETIP sequence was amplified and sequenced from all plants containing only a single copy of the ETIP. The sequence of each T-DNA insert was analyzed by direct sequencing of PCR products using the ABI3730xI™ (Applied Biosystems, Life Teachnologies). The T-DNA insert was amplified from genomic DNA, using Phusion Hot Start II Polymerase™ (Finnzymes, Thermo Fisher Scientific). The amplification reactions of the T-DNA were completed with multiple primer pairs to amplify overlapping sequences of approximately 2 Kbp in length. Each PCR product was sequenced with multiple primers to ensure complete coverage. The PCR reactions were treated with shrimp alkaline phosphatase and exonuclease I (Applied Biosystems, Life Technologies) to inactivate excess primer prior to the sequencing PCR reaction. The sequences flanking the T-DNA insert of each single copy ETIP line were identified by digestion of purified genomic DNA with eight restriction endonucleases followed by ligation of double-stranded adapters specific for the overhangs created by the restriction endonucleases. Following this ligation step a PCR was performed with a biotinylated primer to either the 3' or 5' end of the ETIP and a primer to each adapter. The PCR products were captures and cleaned on Ampure Solid Phase Reversible Immobilization (SPRI) beads™ (Agencourt Bioscience Corporation, Beckman Coulter Company). A nested PCR was performed and all products were sequenced using ABI Sanger Sequencing and Big Dye Terminator v3.1 cycle™ sequencing protocol (Applied Biosystems, Life Technologies). Sequence data were assembled and analyzed using the software (Gene Codes Corp., Ann Arbor, MI).

### SOUTHERN BLOT ANALYSIS

Specific restriction enzymes were selected to digest gDNA samples prior to Southern probing. The putative transgenic plants were analyzed by digesting the genomic DNA with *EcoR*I and *Swa*I. Next, the digested gDNA and uncut gDNA samples were probed with either polynucleotide fragments comprising PATv6, IPT or ELP gene elements as these polynucleotide probe fragments enabled differentiation of multiple inserts in *EcoR*I digests as well as in the *Swa*I digests. Identified single copy transgenic plant lines were then further analyzed with all six probes to identify the presence of all essential elements of the inserted vector.

Accordingly, 67 independent events transformed with ETIP-pDAS000036 were sampled and tested for the presence of the transgene (*hph*), and the presence of vector backbone. Of the 67 plants tested, 47 were found to have the transgene integrated within the genome. From the 47 transgenic plants, 17 of the plants were found to contain vector backbone (Table 14). The remaining 30 plants that contained no significant portion of vector backbone (absence of Ori or SpecR) were sampled for Southern analysis. As a general rule, the plants were screened initially with the IPT probe, and plant lines identified as putative single copy lines were further tested with probes comprising the dsRED, PAT, ELP and *hph* gene elements in order to confirm the presence of the whole cassette.

Likewise, 52 independent events transformed with ETIP-pDAS000037 and surviving in soil were sampled and tested for the presence of the transgene (*hph*), and the presence of vector backbone. Of the 52 plants tested, 48 were found to have the transgene integrated within the genome. From the 48 transgenic plants, 23 of the plants were found to contain vector backbone as well and 3 plants were not tested (Table 14). The remaining 22 plants that contained no significant portion of vector backbone (absence of Ori or SpecR) were sampled for Southern analysis. These transgenic plants were initially screened with the IPT probe, and the plant lines were identified as putative single copy lines, and were further tested with the dsRED, PAT, ELP, *hph* and actin probes in order to confirm results. Once the identification of 5 independent single copy lines were obtained, Southern analysis was terminated on the remaining plants. In total, 11 ETIP-pDAS000037 lines underwent Southern analysis.

**Table 14: Summary of +/- transgene and +/- vector backbone PCRs results**

| **Confirmation of transgene - Endpoint PCR** | | | | |
|---|---|---|---|---|
| | **Independent Events Surviving in Soil** | **Independent Events Sampled** | **Independent Events Tested** | **Independent Events Positive for Transgene** |
| pDAS000036 | 67 | 67 | 67 | 47 |
| pDAS000037 | 52 | 52 | 52 | 48 |

| **Presence of Backbone - Endpoint PCR** | | | | |
|---|---|---|---|---|
| | **Independent Events Tested** | | **Independent Events with no Ori or Spec^{R}** | |
| pDAS000036 | 47 | | 30 | |
| pDAS000037 | 48 | | 22 | |

### RESULTS OF ETIP TRANSGENIC CANOLA TRANSFORMED WITH

### PDAS000036 AND PDAS000037

The transgenic *Brassica napus* events which were produced via transformation of pDAS000036 and pDAS000037 resulted in the production of single copy, full length T-strand insertions. Three to four events for each plant were fully characterized, and were putatively mapped to specific chromosomes within the *Brassica napus* genome. Although a few single base-pair rearrangements occurred during the T-strand integration, the selected events contained full length expression cassettes which are capable of driving robust expression of the transgene. The selected T₀ events were grown to the T₁ stage of development. The T₁ were res-screened using the above described PCR assays to determine the zygosity of the integrated T-strand. Screened events were categorized as homozygous, hemizygous, or null.

The ETIP sequence was amplified and sequenced from all transgenic events containing only a single copy of the integrated ETIP sequence. The sequence of each T-DNA insert was analyzed by direct sequencing of PCR products. The T-DNA insert was amplified from genomic DNA, using Phusion Hot Start II Polymerase™ (Finnzymes, Thermo Fisher Scientific). Next, the T-DNA was amplified with multiple primer pairs to amplify overlapping sequences of approximately 2 Kb in length. Each PCR product was sequenced with multiple primers to ensure complete coverage. The PCR reactions were treated with Shrimp Alkaline Phosphotase and Exonuclease I (Applied Biosystems, Life Technologies) to inactivate excess primer prior to the sequencing PCR reaction.

The sequences flanking the T-DNA insert of each single copy ETIP line was identified by digestion of purified genomic DNA with eight restriction endonucleases followed by ligation of double-stranded adapters specific for the overhangs created by the restriction endonucleases. Following this step a PCR reaction was performed with a biotinylated primer to either the 3' or 5' end of the ETIP and a primer to each adapter. The PCR products were captured and cleaned on Ampure Solid Phase Reversible Immobilization™ (SPRI) beads (Agencourt Bioscience Corporation, Beckman Coulter Company). A nested PCR was performed and all products were sequenced using ABI Sanger Sequencing and Big Dye Terminator v3.1 cycle sequencing protocol (Applied Biosystems, Life Technologies). Sequence data were assembled and analyzed using the software (Gene Codes Corp., Ann Arbor, MI). Eight ETIP lines were identified and selected for flanking sequence analysis (Table 15). The left and right flanking sequences (also described as border or junction sequences) are provided as SEQ ID NO:431 - SEQ ID NO:446, the underlined sequences indicated plasmid vector, the non-underlined sequences indicate genomic flanking sequence.

**Table 15: Details of single copy events used in flanking sequence studies**

| **Plasmid Description** | **Event name** | **Barcode** | **Left Hand Border SEQ ID NO:** | **Right Hand SEQ ID NO:** |
|---|---|---|---|---|
| **pDAS000036** | em02-5788-1-1 | 228688 | 431 | 432 |
| | ad58-5784-2-1 | 232502 | 433 | 434 |
| | ad58-5898-10-1 | 237143 | 435 | 436 |
| **pDAS000037** | 1f31-6139-2-3 | 234576 | 437 | 438 |
| | bm56-6315-1-1 | 234703 | 439 | 440 |
| | ad58-6372-1-1 | 240653 | 441 | 442 |
| | ad58-6620-4-1 | 242268 | 443 | 444 |
| | ad58-6620-17-1 | 242293 | 445 | 446 |

**pDAS000036 Event details: em02-5788-1-1 Left border flanking (SEQ ID NO:431)**
**em02-5788-1-1 Left border flanking (SEQ ID NO:432)**
**pDAS000036 Event details: ad58-5784-2-1 Left border flanking (SEQ ID NO:433)**
**ad58-5784-2-1 Right border flanking (SEQ ID NO:434)**
**pDAS000036 Event details: ad58-5898-10-1 Left border flanking (SEQ ID NO:435)**
**ad58-5898-10-1 Right border flanking (SEQ ID NO:436)**
**pDAS000037 Event details: lf31-6139-2-3 Left border flanking (SEQ ID NO:437)**
**lf31-6139-2-3 Right border flanking (SEQ ID NO:438)**
**pDAS000037 Event details: bm56-6315-1-1 Left border flanking (SEQ ID NO:439)**
**bm56-6315-1-1 Right border flanking (SEQ ID NO:440)**
**pDAS000037 Event details: ad58-6372-1-1 Left border flanking (SEQ ID NO:441)**
**ad58-6372-1-1 Right border flanking (SEQ ID NO:442)**
**pDAS000037 Event details: ad58-6620-4-1 Left border flanking (SEQ ID NO:443)**
**ad58-6620-4-1 Right border flanking (SEQ ID NO:444)**
**pDAS000037 Event details: ad58-6620-17-1 Left border flanking (SEQ ID NO:445)**
**ad58-6620-17-1 Right border flanking (SEQ ID NO:446)**

### MAPPING OF ETIPS

For each transgenic event containing a single copy insertion of the ETIP, the flanking sequence was taken following manual assembly and used as the query in a local BLAST analysis. There were a total of eight plants that had single copy integrations identified by this process (Table 16 and Table 17). A collection of 595,478 genomic derived shotgun sequences from *Brassica oleracea* were downloaded from the NCBI GSS database and formatted as a nucleotide BLAST database. The flanking ETIP sequences were then BLASTn compared to the database and all matches were manually examined. The most significant sequence match to the flanking ETIP sequence from the *B. oleracea* database was then taken and aligned against the online *Brassica rapa* genome sequence (http://brassicadb.org/brad/blastPage.php) where the position in the genome that had the most significant sequence match was also retrieved. In instances where a only the 5' or 3' flanking sequences provided significant matches with the *B. oleracea* genome sequences, it was assumed that the unaligned or unmatched sequence had either; identified missing sequence from the database, or that there had been significant genome rearrangements generated during the integration of the ETIP. For the samples that generated significant BLASTn matches from the analysis the flanking ETIP sequence, the most significant *B. oleracea* GSS matching sequence along with the most significant matching sequence from the *B. rapa* genome, were then manually aligned in Sequencher™ v5.0 software (Gene Codes Corp., Ann Arbor, MI) for each of the eight single copy ETIP plants. The three sequences were then compared and the most similar sequence from either of the diploid *Brassica* species compared to the flanking ETIP was designated the genome that the ETIP was located in. For the majority of the samples significant variation did exist between the two diploid *Brassica* genome sequences and the *B. napus* derived flanking ETIP sequence showed a predominant association with one or other of the diploid sequences. There were instances however, where there was insufficient sequence variation between the diploids and a linkage group assignment may have been possible but a sub-genome assignment was not possible. The specific genome location was then predicted from the location from the *Brassica rapa* genome sequence. In instances where the ETIP was identified as being integrated into the *B. oleracea* C genome, the comparative synteny between the diploid Brassica genomes described in Parkin et al. (Genetics 2005, 171: 765-781) was used to extrapolate the genomic location into the *Brassica napus* C sub-genome. In addition the sequences identified were BLASTn compared to the *Arabidopsis thaliana* genomes coding sequences (TAIR 9 CDS downloaded from http://arabidopsis.org/index.jsp) and the identity of any gene sequences disrupted were identified, as well as a confirmation of genomic location following the *Arabidopsis Brassica* synteny described in Schranz et al. (Trend in Plant Science 2006, 11,11: 535-542).

**Table 16: BLAST search and predicted the location of these above sequences (predicted locations in Brassica napus genome).**

| **Event/Vector Name** | **One copy of each cassette detected by Southern** | **LB Flanking Sequence** | **RB Flanking Sequence** | **Predicted location** |
|---|---|---|---|---|
| **pDAS000036** | | | | |
| em02-5788-1-1 228688 | yes | yes | yes | A6 |
| ad58-5784-2-1 232502 | yes | yes | yes | A8 |
| ad58-5898-10-1 237143 | yes | yes | yes | C7 |

| **pDAS000037** | | | | |
|---|---|---|---|---|
| lf31-6139-2-3 234576 | yes | yes | yes | A5 |
| bm56-6315-1-1 234703 | yes | yes | yes | Genomic Repeat |
| ad58-6372-1-1 240653 | yes | yes | yes | A/C8 |
| ad58-6620-4-1 242268 | yes | yes | yes | C1 |
| ad58-6620-17-1 242293 | yes | yes | yes | A/C 3 or 8 |

**Table 17: description of single copy ETIP containing plant from the two constructs pDAS000036 and 37, BLASTn result to a Brassica oleracea genome sequence data base, potential disruption of gene sequence identified through Arabidopsis thaliana gene comparison and predicted genome location.**

| **pDAS000036** | **BLASTn match to the C genome** | **Predicted gene disrupted** | **Predicted Location** |
|---|---|---|---|
| em02-5788-1-1 228688 | Left border only value e-175 | At3g30775: proline oxidase | A6 |
| ad58-5784-2-1 232502 | Left border only value e-134 | Atlg50940: Electron transfer flavoprotein alpha | A8 |
| ad58-5898-10-1 237143 | Left border generated two significant matches at value 0 and e-107 | No significant match to *Arabidopsis* gene | C7 |
| | | | |

| **pDAS000037** | **BLASTn match to the C genome** | **Predicted gene disrupted** | **Predicted Location** |
|---|---|---|---|
| lf31-6139-2-3 234576 | Right border only value e-105 | At3g08530: Clathrin | A5 |
| bm56-6315-1-1 234703 | Both borders had large numbers of matches e value 0 | N/A | Genomic Repeat |
| ad58-6372-1-1 240653 | Left and right border value e-103 and -80 | No significant match to Arabidopsis genes | Equivocal location: subgenome A or C on linkage group 8 |
| ad58-6620-4-1 242268 | Left and right border value e-154 and e-48 | At4g27860: Vacuolar ion transporter | C1 |
| ad58-6620-17-1 242293 | Left and right border value e-167 and e-94 | Borders identified At5g20340 and At1g50930 | Equivocal location: potentially sub-genome A or C and linkage group 3 or 8 |

The homozygous events are used to produce protoplasts via the previously described method. The protoplasts are subsequently co-transformed with a Zinc Finger Nuclease that is designed to target a Zinc Finger binding site which is incorporated within the ETIP sequence and a donor plasmid which shares homology with specific regions of the ETIP. The Zinc Finger Nuclease cleaves the ETIP locus and the donor plasmid is integrated within the genome of *Brassica napus* cells via homology directed repair. As a result of the integration of the donor plasmid, the partial *DS-red* transgene is repaired to a full length *DS-red* transgene. The expression of the now fully operational *DS-red* transgene is used to sort protoplast cells with a FACS method. Putative transgenic plants are sorted using the FACS method described in Example 7 and the isolated protoplasts are regenerated into mature plants. The integration of the donor plasmid is confirmed within the ETIP-targeted plants using molecular confirmation methods. As such, the ETIP locus serves as a site-specific locus for gene targeted integration of a donor polynucleotide sequence.

The genomic targeting locations provide genomic locations that do not alter the plants normal phenotype. The resulting events, wherein a transgene is targeted within an ETIP present no agronomically meaningful unintended differences when the ETIP events are compared to the control plants. In addition, the protein expression levels of transgenes integrated within the ETIP locus are robustly expressed and consistent and stable across multiple genomic locations. The disclosed genomic sequences of SEQ ID NO:431 to SEQ ID NO:446 provide genomic locations within the brassica genome that are targetable for the integration of gene expression cassettes comprising a transgene.

### MOLECULAR CONFIRMATION OF FAD2A INTEGRATION OF ETIPS IN CANOLA

Genomic DNA was extracted from leaf tissue of all putative transgenic plants using a DNeasy Plant Mini Kit™ (Qiagen) following the manufacturer's instructions, with the exception that tissue was eluted in 80 µl of AE buffer. Thirty milligrams of young leaf tissue from regenerated plants was snap frozen in liquid nitrogen before being ground to a powder.

Molecular characterization of the FAD2A locus was performed using three independent assays. Assays were designed and optimized using the following controls; characterized transgenic events comprising a single randomly integrated transgene, characterized transgenic event with five randomly integrated transgenes, wild-type canola c.v. DH12075 plants and non-template control reactions. The results from the three following molecular analyses are considered together in order to provide evidence for integration of the ETIP at FAD2A via HDR.

### IDENTIFYING TRANSGENE INTEGRATION BY REAL-TIME POLYMERASE CHAIN REACTION

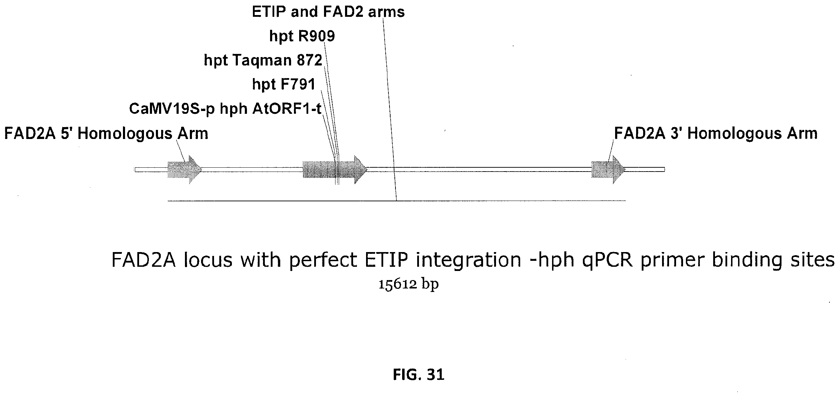

Four replicates of each plant were analyzed using primers specific to the *hph* (also described as *hpt*) target gene (SEQ ID NO:447, hpt F791 5' CTTACATGCTTAGGATCGGACTTG 3'; SEQ ID NO:448, hpt R909 5' AGTTCCAGCACCAGATCTAACG 3'; SEQ ID NO:449, hpt Taqman 872 5' CCCTGAGCCCAAGCAGCATCATCG 3' FAM) (FIG. 31) and reference gene encoding High Mobility Group protein I/Y *(HMG I*/*Y)* (SEQ ID NO:450, F 5' CGGAGAGGGCGTGGAAGG 3'; SEQ ID NO:451, R 5' TTCGATTTGCTACAGCGTCAAC 3'; SEQ ID NO:452, Probe 5' AGGCACCATCGCAGGCTTCGCT 3' HEX). The reactions were amplified using the following conditions: 95°C for 10 minutes followed by 40 cycles of 95°C for 30 seconds, 60°C for 1 minute, with amplification data being captured at the end of each annealing step. Copy number was calculated using the ΔCq method, where ΔCq = Cq(target gene) - Cq(reference gene). Livak, K.J. and T.D. Schmittgen, Analysis of relative gene expression data using real-time quantitative PCR and the 2(-Delta Delta C(T)) Method. Methods, 2001. 25(4): p. 402-8. Plants with amplification of *hph* and *HMG I*/*Y* and a copy number of 0.5 or more were considered transgenic, while plants with a copy number of ≥ 0.5 and ≤ 1.2 were scored as putatively single copy. Amplification was performed on a BioRad CFX96 Touch™ Real-Time PCR Detection System with FastStart Universal Probe Master (ROX), (Roche, Basel, Switzerland).

### DETECTION OF DISRUPTED FAD2A ZFN SITE

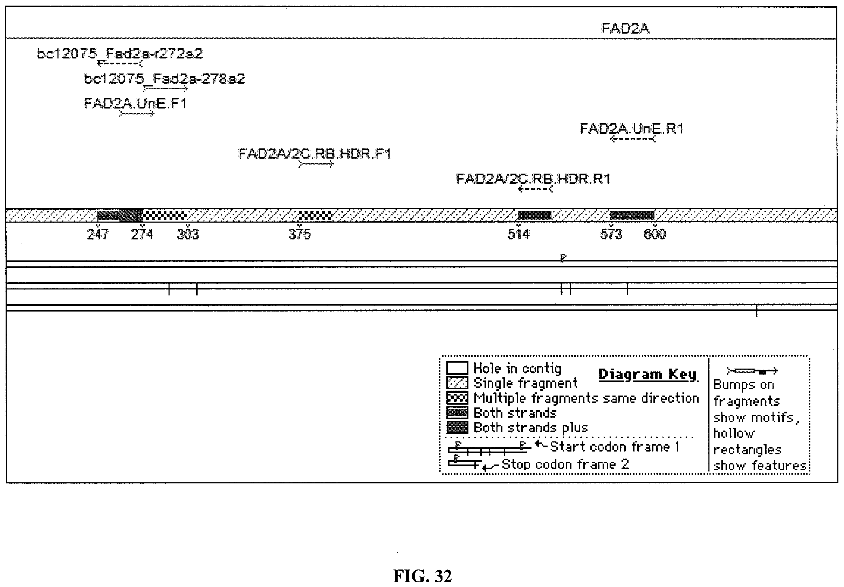

Each plant was analyzed for presence or absence of amplification of endogenous target in the disrupted locus test, which is a dominant assay. The assay is a SYBR® Green I qPCR assay and in singleplex, but with each reaction run simultaneously on the same PCR plate, targets an endogenous locus (FAD2A/2C.RB.UnE.F1, SEQ ID NO:453, 5' CTTCCACTCCTTCCTCCTCGT*C 3' and FAD2A/2C.RB.UnE.R1, 5' SEQ ID NO:454, GCGTCCCAAAGGGTTGTTGA*G 3') and the ZFN locus (locus at which the ZFN pDAB104010 binds and cuts the genome) (FAD2A.UnE.F1, SEQ ID NO:455, 5' TCTCTACTGGGCCTGCCAGGG*C 3' and FAD2A.UnE.R1, SEQ ID NO:456, 5' CCCCGAGACGTTGAAGGCTAAGTACAA*A 3') (FIG. 32). Both primer pairs were amplified using the following conditions: 98°C for 30 seconds followed by 35 cycles of (98°C for 10 seconds, 65°C for 20 seconds, 72°C for 90 seconds) then followed by 95°C for 10 seconds then a melt analysis from 50°C to 95°C with 0.5°C increments for 0.05 seconds and a plate read at each increment. The reaction conditions are listed in Table 18.

**Table 18: Single reaction reagent components and concentrations for PCR amplification.**

| **Reaction Components** | **Volume (µl)** |
|---|---|
| 10 mM dNTP | 0.40 |
| 5X Phusion HF Buffer | 4.00 |
| Phusion Hot Start II High-Fidelity DNA Polymerase (2U/µl) (Thermo Scientific) | 0.25 |
| Forward Primer 10 µM | 0.40 |
| Reverse Primer 10 µM | 0.40 |
| 1:10000 dilution of SYBR Green I dye (Invitrogen) | 1.00 |
| Molecular Biology Grade H₂O | 11.55 |
| Genomic DNA template (∼20 ng/µl) | 2.00 |
| Total Volume | 20.00 |

Plants that had amplification of the endogenous target but no amplification of the ZFN target, were scored as positive for the disrupted locus test and were considered to have a disrupted ZFN locus. This assay was considered to be positive when the ZFN binding site on both alleles at the FAD2A locus have been disrupted.

### PCR DETECTION OF TRANSGENE INTEGRATION AT FAD2A VIA HOMOLOGY DIRECTED REPAIR

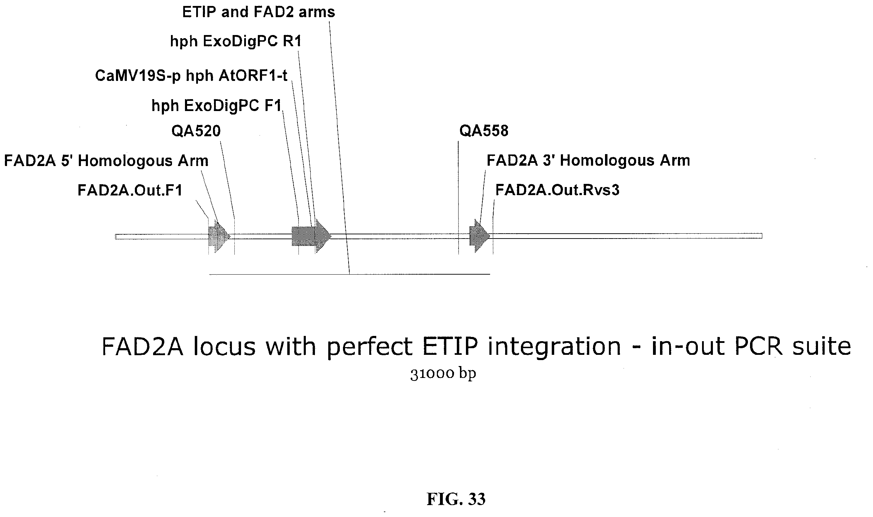

Each putative plant transformant was analyzed using endpoint with PCR primers designed to amplify the transgene target *hph (hph*_ExoDigPC_F1, SEQ ID NO:457, 5' TTGCGCTGACGGATTCTACAAGGA 3' and *hph*_ExoDigPC-R1, SEQ ID NO:458, 5' TCCATCAGTCCAAACAGCAGCAGA 3'), the FAD2A endogenous locus (FAD2A.Out.Fl, SEQ ID NO:459, 5' CATAGCAGTCTCACGTCCTGGT*C 3' and FAD2A.Out.Rvs3, SEQ ID NO:460, 5' GGAAGCTAAGCCATTACACTGTTCA*G 3'), the region spanning the 5' end of any transgene inserted into the FAD2A locus via HDR, upstream of the transgene into the FAD2 A locus (FAD2A.Out.F1, SEQ ID NO:461, 5' CATAGCAGTCTCACGTCCTGGT*C 3' and QA520, SEQ ID NO:462, 5' CCTGATCCGTTGACCTGCAG 3') and the region spanning the 3' end of any transgene inserted into the FAD2A locus via HDR, downstream of the transgene into the FAD2 A locus (QA558, SEQ ID NO:463, 5' GTGTGAGGTGGCTAGGCATC 3' and FAD2A.Out.Rvs3, SEQ ID NO:464, 5' GGAAGCTAAGCCATTACACTGTTCA*G 3') (FIG. 33). All primer pairs were amplified using the following conditions 98°C for 30 seconds followed by 35 cycles of (98°C for 10 seconds, 65°C for 20 seconds, 72°C for 90 seconds). Reaction reagent conditions are as described in **Table 19.**

**Table 19: Single reaction reagent components and concentrations for PCR amplification.**

| **Reaction Components** | **Volume (µl)** |
|---|---|
| 5x Phusion HF Buffer | 6.00 |
| 10 mM dNTPs | 0.60 |
| Forward Primer 10 µM | 0.60 |
| Reverse Primer 10 µM | 0.60 |
| Phusion Hot Start II High-Fidelity DNA Polymerase (2U/µl) (Thermo Scientific) | 0.25 |
| Molecular Biology Grade H₂O | 19.95 |
| Genomic DNA template (∼20 ng/µl) | 2.00 |
| Total Volume | 30.0 |

Amplification of the 5' transgene-genome flanking target and/or amplification of the 3' transgene-genome flanking target indicated a putative insertion event. It must be noted that due to the approximately 1,000 bp FAD2A homology arms in the pDAS000130 cassette (comprising polynucleotide sequences with 100% sequence identity to the FAD2A regions immediately upstream and downstream of the ZFN cut site), the PCR reactions were subject to false positive PCR product amplification due to PCR chimerism arising from amplification of off-target ETIP integration events. Amplification of the *hph* target confirmed transgene integration had occurred. Amplification of the FAD2A target suggests that the FAD2A locus is intact or contains only a partial insertion. Due to the size of the ETIP (11,462 bp for the ETIP cassettes or 13,472 bp including the FAD2A homologous arms and the ETIP cassettes) it is expected that the FAD2A primers would not amplify a product when an intact ETIP is integrated into the FAD2A locus.

### SOUTHERN DETECTION OF FAD2A EDITING

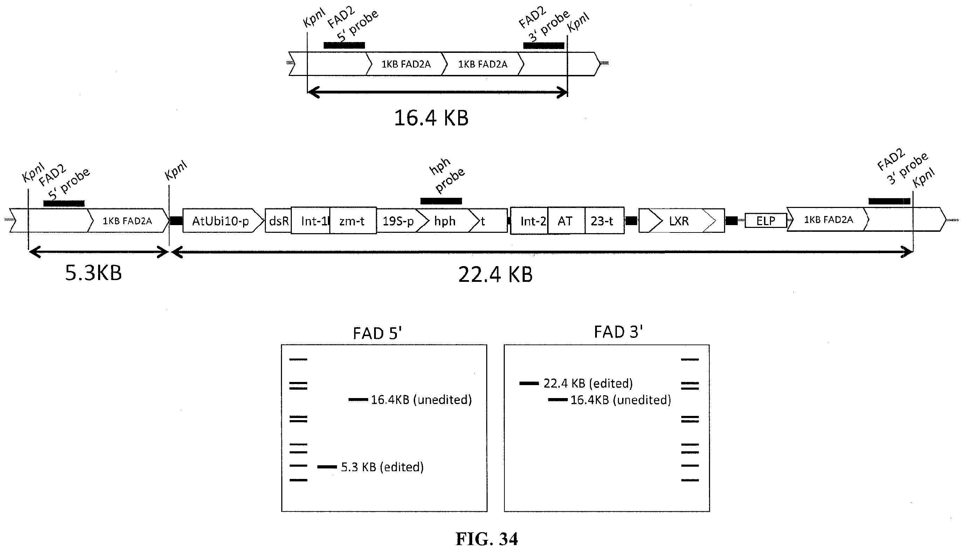

Plants that had amplification of either a 5' genome-transgene flanking target product and/or amplification of a 3' transgene-genome flanking target, or no amplification of the ZFN locus target, or both, were subject to Southern analysis for detection of transgene integration at the FAD2A locus. Genomic DNA was purified from 5 g of leaf tissue using a modified CTAB method (Maguire, T.L., G.G. Collins, and M. Sedgley A modified CTAB DNA extraction procedure for plants belonging to the family proteaceae. Plant Molecular Biology Reporter, 1994. 12(2): p. 106-109). Next, 12 µg of genomic DNA was digested with *Kpn1*-HF (New England BioLabs) and digestion fragments were separated by electrophoresis on a 0.8% agarose gel before transfer to membrane using a standard Southern blotting protocol. Primers to *FAD2A 5'* target region (F, SEQ ID NO:465, 5' AGAGAGGAGACAGAGAGAGAGT 3' and R, SEQ ID NO:466, 5' AGACAGCATCAAGATTTCACACA 3'), *FAD2A* 3' target region (F, SEQ ID NO:467, 5' CAACGGCGAGCGTAATCTTAG 3' and R, SEQ ID NO:468, 5' GTTCCCTGGAATTGCTGATAGG 3') and *hph* (F, SEQ ID NO:469, 5' TGTTGGTGGAAGAGGATACG 3' and R, SEQ ID NO:470, 5' ATCAGCAGCAGCGATAGC 3') were used to generate probes to detect the presence of the ETIP within the FAD2A locus using the DIG EASY HYB SYSTEM® (Roche, South San Francisco, CA) following the manufacturer's instructions (FIG. 34). Hybridization was performed at 42° C for *FAD2A* 5' region, 45°C for *FAD2A* 3' region and 42° C for detection of *hph.*

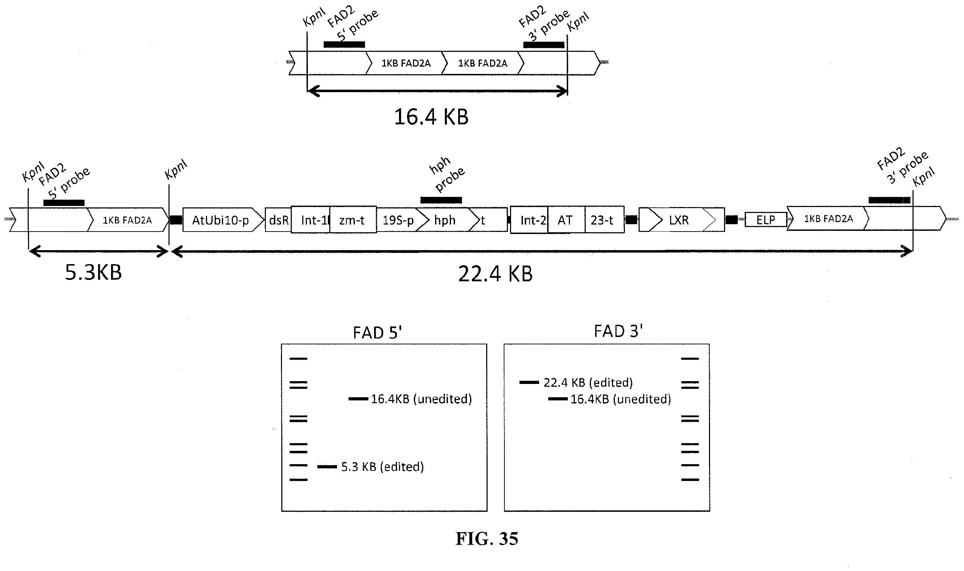

Membrane-bound genomic DNA was probed in a specific order; firstly *FAD2A* 5' sequences were probed, then the *FAD2A* 3' sequences were probe, and finally the *hph* sequences were probed (FIG. 35). The rational for this is as follows. The first probe (FAD2A 5') is the diagnostic probe, and if the ETIP has integrated into FAD2A via perfect HDR, a 5,321 bp fragment will be visible on the membrane. The resulting band size is easily differentiated during electroporation and will sit close to the 5,148 bp fragments in the DIG labeled Roche DNA MOLECULAR WEIGHT MARKER III® (Roche, Indianapolis, IN). The second probe of the membrane is with the FAD2A 3' probe and an edited plant will have a 22,433 bp fragment whereas an unedited plant will have a 16,468 bp fragment. The same 22,433 bp fragment identified with the FAD2A 3' probe should also be bound by and identified with the *hph* probe. These fragments are difficult to differentiate on a gel as they are extremely large and it may be difficult to determine any difference between a fragment occurring above or below the largest, 21,226 bp fragment in the DIG labeled Roche DNA MOLECULAR WEIGHT MARKER III®. As such, these probes provide evidence that may strengthen the identification of ETIP integration into FAD2A via homology directed repair (HDR), by visualization of a 5 kb fragment using the FAD2A 5' probe. The restriction enzyme, KpnI was the only suitable restriction endonuclease for use in this assay, as *KpnI* sites occurred in a single locus of the cut the ETIP cassette in a single locus, and was present in two sites of the FAD2A ZFN locus. One site was located upstream and the second site located downstream of the FAD2A homology arms. In addition, *KpnI* is not methylation sensitive, and is available as a recombinant enzyme with increased fidelity (New England Biolabs).

### RESULTS OF MOLECULAR AND SOUTHERN ANALYSIS

Following transfection, culturing, and selection the transgenic plants were transferred to soil. From this process, 139 plants survived and had tissue sampled for gDNA extraction and analysis. All 139 plants were analyzed for copy number estimation. Of these 139 plants, 56 were positive for the ETIP and 11 of the 56 positive plants had a putative single copy integration (FIG. 36) (Table 20). Of the 56 plants that were positive for ETIP integration, amplification of the FAD2A 5'-genome-transgene flanking sequence occurred in 7 plants. Amplification of the FAD2A 3'-transgene-genome flanking sequence did not occur in any of the 56 plants that were positive for ETIP integration. Additionally, of the 56 plants that were positive for transgene integration, 11 plants were positive for the disrupted locus qPCR test. Fourteen plants that were positive for amplification of the FAD2A 5' genome-transgene flanking sequence and/or positive for the disrupted locus qPCR test were subject to Southern analysis, with the 3 probes described above. Of the 14 plants advanced for Southern analysis, all of the plants showed partial integration within the FAD2A locus, but none of these plants showed evidence of a complete full-length integration of the ETIP at the FAD2A locus via HDR when probed with the FAD2A 5' probe, FAD2A 3' and *hph* probes. No bands that appeared to be i) larger than WT and ii) identical to bands observed for those samples when probed with FAD2A 3' probe (**Table 20**).

**Table 20: Overview of outcomes from analysis of ETIP integration.**

| **Number of plants surviving in soil** | **Number of plants sampled** | **Number of plants for which qPCR copy number analysis was completed** | **Number of plants positive for ETIP integration** | **Number of plants comprising a putative single copy insert** | **Number of ETIP/*FAD2* in-out 5' reaction** | **Number of ETIP/*FAD2* in-out 3' reactions** | **Number of locus disrupted qPCR tests** | **ETIP on-target (Southern)** |
|---|---|---|---|---|---|---|---|---|
| **139** | **139** | **139** | **56** | **11** | **7 (from 56)** | **0 (from 56)** | **9 (from 56)** | **0 (from 14)** |

### RESULTS OF ETIP TRANSGENIC CANOLA TRANSFORMED WITH PDAS000130 AND PDAB104010.

The transgenic *Brassica napus* events which are produced via transformation of pDAS000130 and pDAB104010 result in the integration of a single copy, full length T-strand insertion of the ETIP polynucleotide sequence from pDAS000130 within the FAD2A locus. Three to four events are fully characterized and confirmed to contain the integrated ETIP. The confirmation is completed using an in-out PCR amplification method, and further validated via Southern blot. The selected T₀ events are grown to the T₁ stage of development. The T₁ plants are re-screened to determine the zygosity of the integrated T-strand. Screened events are categorized as homozygous, hemizygous, or null.

The homozygous events are used to produce protoplasts via the previously described method. The protoplasts are subsequently co-transformed with a Zinc Finger Nuclease that is designed to target a Zinc Finger binding site which is incorporated within the ETIP sequence and a donor plasmid which shares homology with specific regions of the ETIP wherein the donor is integrated within the ETIP via an HDR mechanism. Likewise, the protoplasts are subsequently co-transformed with a Zinc Finger Nuclease that is designed to target a Zinc Finger binding site which is incorporated within the ETIP sequence and a donor plasmid which does not share homology with specific regions of the ETIP, wherein the donor is integrated within the ETIP via an non-homologous end joining mechanism. The Zinc Finger Nuclease cleaves the ETIP locus and the donor plasmid is integrated within the genome of *Brassica napus* cells via homology directed repair or non-homologous end joining. As a result of the integration of the donor plasmid, the partial *DS-red* transgene is repaired to a full length *DS-red* transgene. The expression of the now fully operational *DS-red* transgene is used to sort protoplast cells with a FACS method. Putative transgenic plants are sorted using the FACS method described in Example 7 and the isolated protoplasts are regenerated into mature plants. The integration of the donor plasmid is confirmed within the ETIP-targeted plants using molecular confirmation methods. As such, the ETIP locus serves as a site-specific locus for gene targeted integration of a donor polynucleotide sequence.

### RESULTS OF ETIP TRANSGENIC CANOLA TRANSFORMED WITH ZINC FINGER NUCLEASE AND PDAS000271-PDAS000275 ETIP CONSTRUCTS

The transgenic *Brassica napus* events which are produced via transformation of ETIP and Zinc Finger Nuclease constructs result in the integration of a single copy, full length T-strand insertion of the ETIP polynucleotide sequence from pDAS000273 or pDAS275 within the FAD3A locus, and from pDAS000271, pDAS000272 or pDAS000274 into the FAD3C locus. Three to four events are fully characterized and confirmed to contain the integrated ETIP. The confirmation is completed using an in-out PCR amplification method, and further validated via Southern blot. The selected T₀ events are grown to the T₁ stage of development. The T₁ plants are res-screened to determine the zygosity of the integrated T-strand. Screened events are categorized as homozygous, hemizygous, or null.

The homozygous events are used to produce protoplasts via the previously described method. The protoplasts are subsequently co-transformed with a Zinc Finger Nuclease that is designed to target a Zinc Finger binding site which is incorporated within the ETIP sequence and a donor plasmid which shares homology with specific regions of the ETIP. The Zinc Finger Nuclease cleaves the ETIP locus and the donor plasmid is integrated within the genome of *Brassica napus* cells via homology directed repair. As a result of the integration of the donor plasmid, the partial *DS-red* transgene is repaired to a full length *DS-red* transgene. The expression of the now fully operational *DS-red* transgene is used to sort protoplast cells with a FACS method. Putative transgenic plants are sorted using the FACS method described in Example 7 and the isolated protoplasts are regenerated into mature plants. The integration of the donor plasmid is confirmed within the ETIP-targeted plants using molecular confirmation methods. As such, the ETIP locus serves as a site-specific locus for gene targeted integration of a donor polynucleotide sequence.

### EXAMPLE 7: FACS BASED SORTING OF PROTOPLAST CELLS

*Brassica napus* protoplasts that were transfected with the DS-Red control construct, pDAS000031, were sorted via FACS-mediated cell sorting using a BD Biosciences Influx-Cell sorter™ (San Jose, CA). The protoplast cells were isolated and transfected as described in Example 3. After the cells had been transfected with pDAS000031, the cells were sorted using the FACS sorter with the conditions described in Table 21.

**Table 21: Conditions used for sorting protoplast cells transfected with pDAS000031.**

| **Parameters** | |
|---|---|
| **Drop frequency** | 6.1 KHz |
| **Nozzle diameter** | 200 µm |
| **Sheath pressure** | 4 psi |
| **Recovery media** | W5 media |
| **Culture conditions** | Bead type culture using sea-plaque agarose and sodium alginate |
| **Sort criteria** | Sorting based on chlorophyll autofluorescence, reporter gene expression (Ds-Red) |
| **Sort recovery (%)** | 50-75 |
| **Viability post sorting (%)** | >95 |

The protoplasts which expressed the *DS-red* transgene were sorted and isolated. The FACS isolated protoplasts were counted using the sorter. About 1x10⁵ to 1.8x10⁵ of cells were placed in a well of a 24-well micro titer plate on the first day after the FACS isolation. The cells were transferred to a bead culture for 5 to 20 days. Similar conditions were tested, wherein about 1x10⁴ of cells were placed in a well of a 2 or 4-well micro titer plate on the second day after the FACS isolation. The various conditions that were tested resulted in the recovery of cells at a viability or 95 - 98% of the total isolated protoplast cells. The FACS sorted protoplast cells were transferred to a bead culture for 3 - 20 days. The FACS sorted protoplast cells were regenerated into plants on media which contained 1.5 mg/mL of hygromycin using the above described protocol. The putative transgenic plants were confirmed to contain an intact T-strand insert from pDAS000031 via molecular conformation protocols.

### TARGETING OF ETIP LINES WITH ZFN MEDIATED HOMOLOGOUS RECOMBINATION OF DS-RED

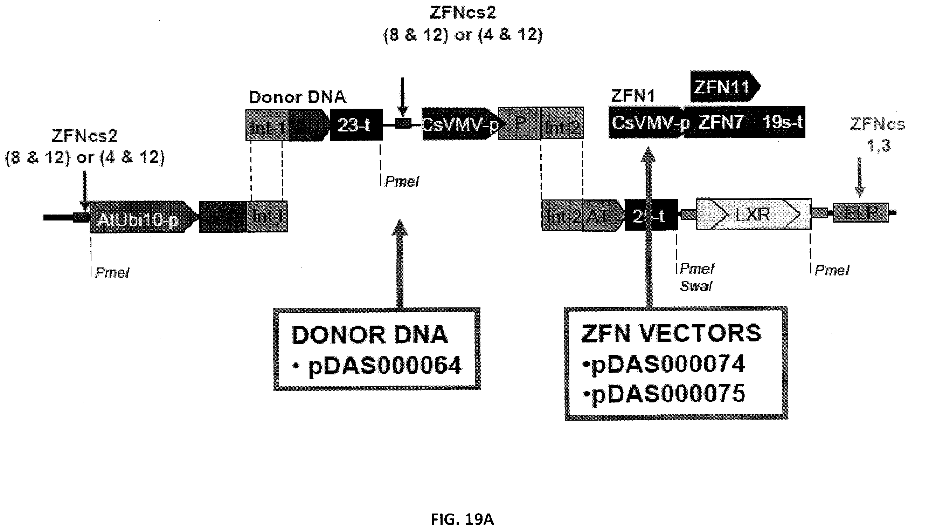

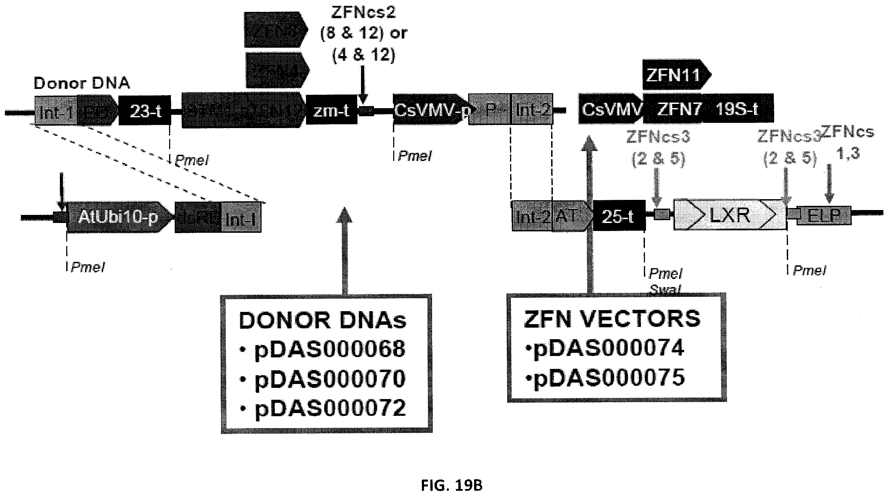

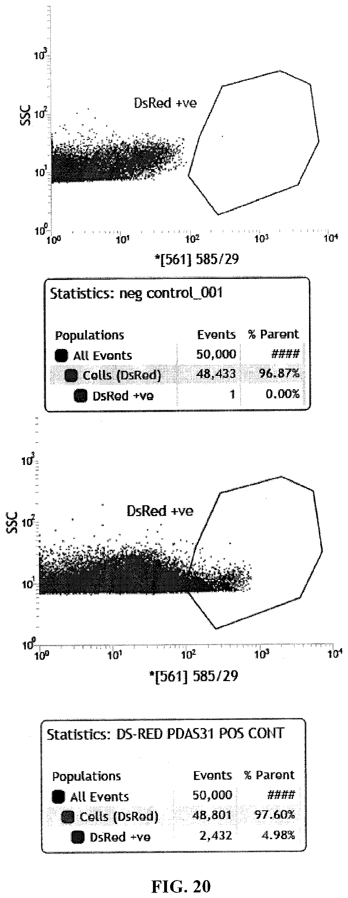

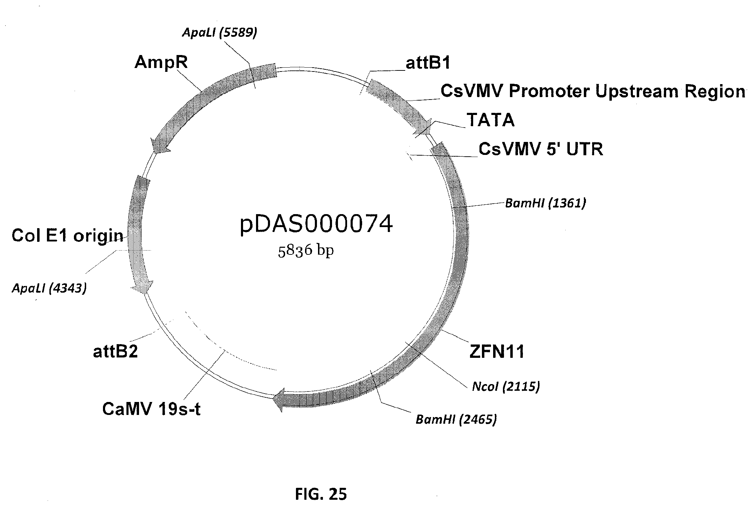

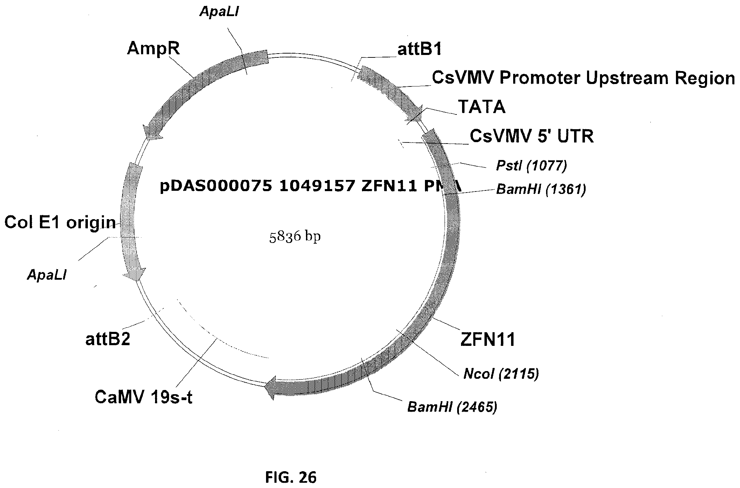

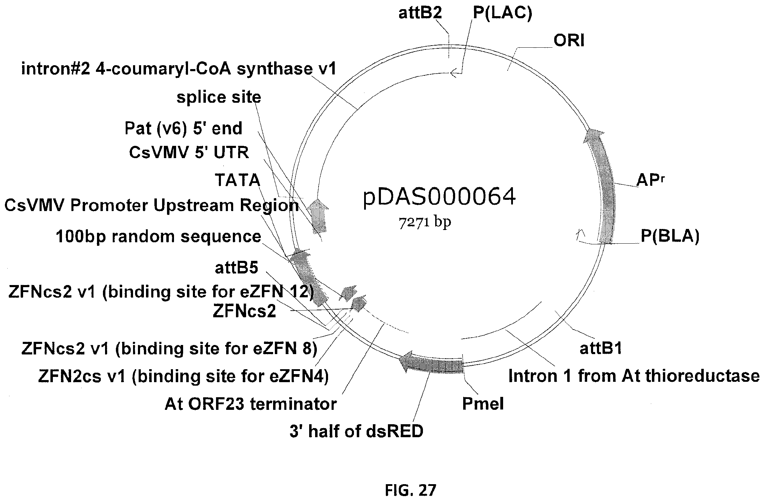

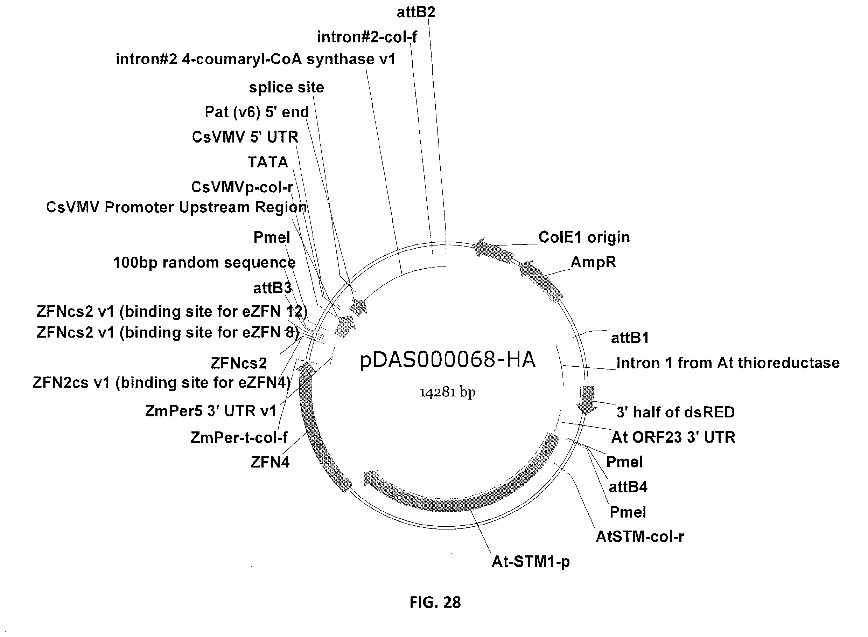

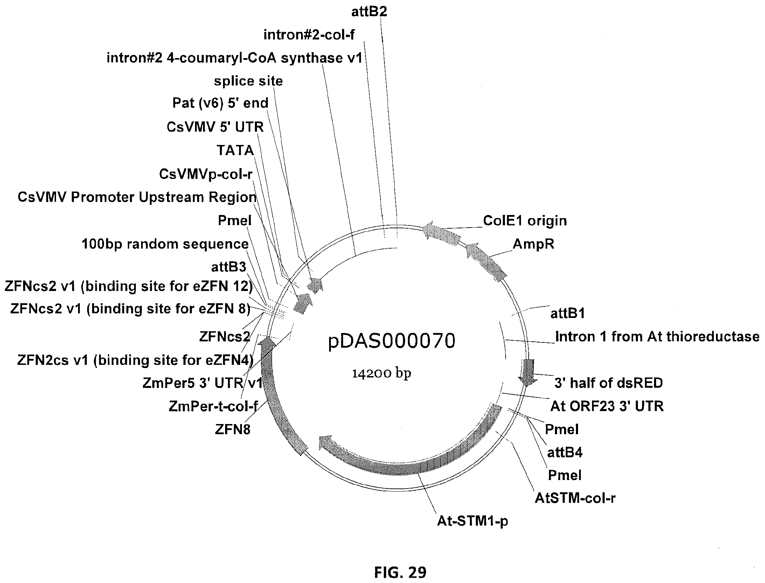

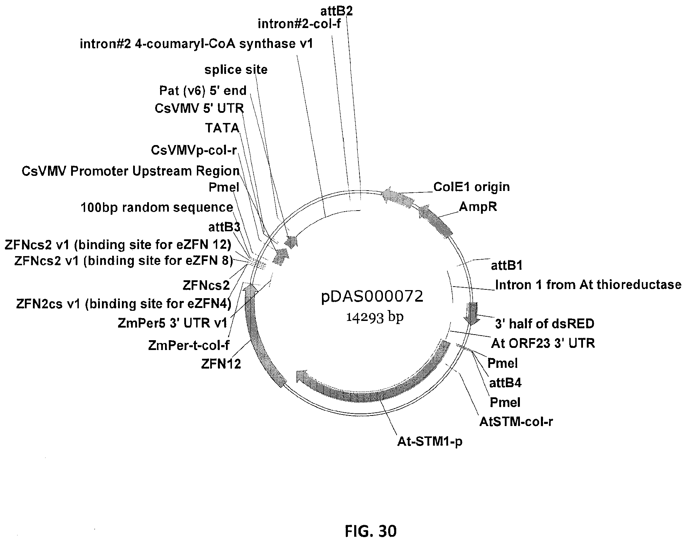

A canola line containing the T-strand insert from pDAS000036 was obtained and confirmed via molecular characterization to contain a full length, single copy of the T-strand. This canola event was labeled as pDAS000036 - 88 and was used to produce protoplasts via the previously described method. The protoplasts were isolated and ∼50,000 canola protoplast cells were subsequently co-transformed with a Zinc Finger Nuclease, either pDAS000074 (FIG. 25) or pDAS000075 (FIG. 26), that was designed to target the Zinc Finger binding sites incorporated within the ETIP sequence and a donor plasmid, pDAS000064, pDAS000068, pDAS000070, or pDAS000072 (FIG. 27, FIG. 28, FIG. 29, and FIG. 30, respectively), which shares homology with specific regions of the ETIP. FIG. 19 and FIG. 20 provide illustrations of the homology directed repair which results in the site-specific integration of the *Ds-red* transgene via Zinc Finger Nuclease mediated homologous recombination. The Zinc Finger Nuclease was designed to cleave the ETIP locus at a defined Zinc Finger binding sequence, thereby creating a double strand break within the genome. Next, the donor plasmid was integrated within the genome of *Brassica napus* protoplast cells via homology directed repair. The intron-1 and intron-2 regions of the donor plasmid share homology with the corresponding intron-1 and intron-2 regions of the ETIP locus. As a result of the integration of the donor plasmid, the partial *DS-red* transgene was repaired to a full length, highly expressing *DS-red* transgene. The expression of the fully operational *DS-red* transgene was used to sort protoplast cells with the above described FACS method. As such, the ETIP locus serves as a site-specific locus for targeted integration of a donor polynucleotide sequence. Finally, the isolated protoplasts can be sorted and regenerated into mature plants. The integration of the donor plasmid can be confirmed within the ETIP-targeted plants using molecular confirmation methods.

The donor plasmid DNA and ZFN plasmid DNA were mixed at various concentrations and used to transfect the canola protoplast cells containing Event pDAS000036 - 88, and the transgenic protoplast cells were sorted using the FACS transfection that was previously described. Table 22 describes the various transfection experiments and the DNA concentrations which were used for the transfection of the canola protoplasts containing Event pDAS000036 - 88. The ZFN and donor plasmid DNA was isolated and prepared for the transfections via the previously described methods.

**Table 22: Donor plasmids and Zinc Finger Nuclease constructs used for the ETIP targeting experiments. The DNA concentrations were used at the indicated ratio of donor to Zinc Finger Nuclease, for a total concentration of 30 micrograms of plasmid DNA per transfection.**

| REACTIONS | PLASMIDS | DONOR PLASMID DNA (µg) | ZFN PLASMID DNA (µg) | TOTAL (µg) |
|---|---|---|---|---|
| 1 | pDAS000074 | - | 30 | 30 |
| 2 | pDAS000075 | - | 30 | 30 |
| 3 | pDAS000064 + pDAS000074 | 26 | 4 | 30 |
| 4 | pDAS000064 + pDAS000075 | 26 | 4 | 30 |
| 5 | pDAS000068 + pDAS000074 | 28 | 2 | 30 |
| 6 | pDAS000068 + pDAS000075 | 28 | 2 | 30 |
| 7 | pDAS000070 + pDAS000074 | 28 | 2 | 30 |
| 8 | pDAS000070 + pDAS000075 | 28 | 2 | 30 |
| 9 | pDAS000072 + pDAS000074 | 28 | 2 | 30 |
| 10 | pDAS000072 + pDAS000075 | 28 | 2 | 30 |
| 11 | pDAS000064 | 30 | - | 30 |
| 12 | pDAS000068 | 30 | - | 30 |
| 13 | pDAS000070 | 30 | - | 30 |
| 14 | pDAS000072 | 30 | - | 30 |

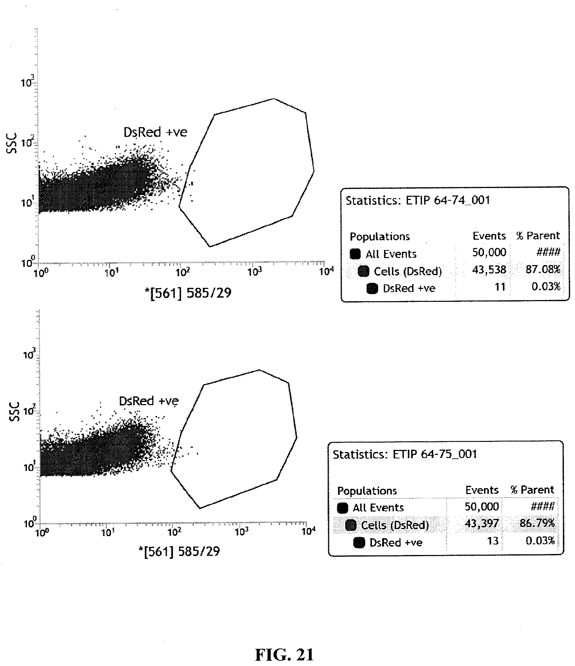

After the transfection experiments were completed the protoplasts were incubated at room temperature for 48 hours and sorted using the above described FACS protocol. Each experiment was sorted independently and Zinc Finger-mediated introgression of a transgene was confirmed via identification of individual events which expressed the *DS-red* transgene. FIGS. 21 - 24 show the results of the FACS sorting. As the results depicted in the graphs indicate, multiple events were produced which contained an intact fully integrated *DS-red* transgene. These multiple *Ds-Red* events were the result of Zinc Finger Nuclease mediated integration of the donor DNA construct within the ETIP genomic locus. This site-specific integration resulted in a highly expressing, complete copy of the *Ds-Red* transgene. The frequency of the *Ds-Red* transgene expression ranged from about 0.03 - 0.07% of the total canola protoplast cells (∼50,000). However, the frequency of transfection efficiency for the Zinc Finger Nuclease mediated integration of the donor DNA construct within the ETIP genomic locus was much higher and ranged from about 0.07 - 0.64%.

FIG. 21 shows the results of the transfections in which the donor plasmid and ZFN plasmid were co-transformed. The top graph, wherein donor, pDAS000064, and the Zinc Finger Nuclease, pDAS000074, were co-transformed at a ratio of 26 µg to 4 µg of plasmid DNA resulted in the Zinc Finger Nuclease mediated integration of the donor DNA construct within the ETIP genomic locus at a recombination frequency of about 0.03% of the ∼50,000 canola protoplast cells. In actuality, the recombination frequency is much higher. Of the ∼50,000 canola protoplast cells which were provided during the transfection experiment, only about 10 - 30% of these canola protoplast cells are actually transformed. As such, the actual Zinc Finger Nuclease mediated integration of the donor DNA construct within the ETIP genomic locus transfection efficiency ranges from about 0.22 - 0.07%. Similarly the bottom graph, wherein donor, pDAS000064, and the Zinc Finger Nuclease, pDAS000075, were co-transformed at a ratio of 26 µg to 4 µg of plasmid DNA resulted in the Zinc Finger Nuclease mediated integration of the donor DNA construct within the ETIP genomic locus at a recombination frequency of about 0.03% of the ∼50,000 canola protoplast cells. In actuality, the recombination frequency is much higher. Of the ∼50,000 canola protoplast cells which were provided during the transfection experiment, only about 10 - 30% of these cells are actually transfected. As such, the actual Zinc Finger Nuclease mediated integration of the donor DNA construct within the ETIP genomic locus transfection efficiency ranges from about 0.26 - 0.08%. The results of the zinc finger mediated homology directed repair are significantly greater than the negative control experiments, wherein only one protoplast of ∼50,000 was identified to have red fluorescence, thereby resulting in a recombination frequency of 0.00%, as shown in FIG. 20.

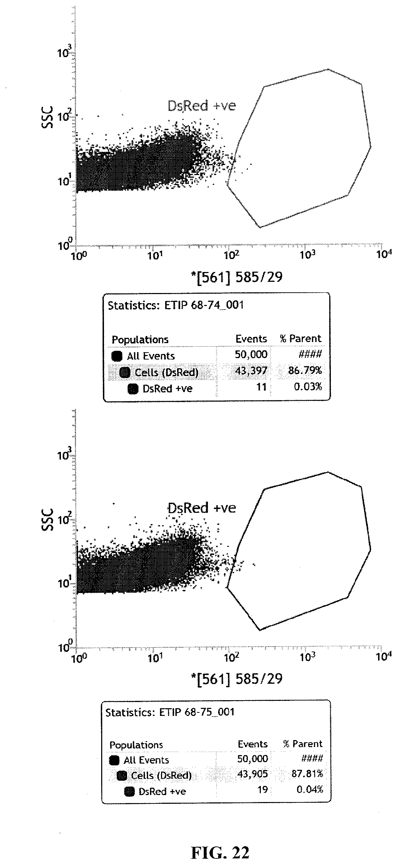

FIG. 22 shows the results of the transfections in which the donor plasmid and ZFN plasmid were co-transformed. The top graph, wherein donor, pDAS000068, and the Zinc Finger Nuclease, pDAS000074, were co-transformed at a ratio of 28 µg to 2 µg of plasmid DNA resulted in the Zinc Finger Nuclease mediated integration of the donor DNA construct within the ETIP genomic locus at a recombination frequency of about 0.03% of the ∼50,000 canola protoplast cells. In actuality, the recombination frequency is much higher. Of the ∼50,000 canola protoplast cells which were provided during the transfection experiment, only about 10 - 30% of these canola protoplast cells are actually transformed. As such, the actual Zinc Finger Nuclease mediated integration of the donor DNA construct within the ETIP genomic locus transfection efficiency ranges from about 0.22 - 0.07%. Similarly the bottom graph, wherein donor, pDAS000068, and the Zinc Finger Nuclease, pDAS000075, were co-transformed at a ratio of 28 µg to 2 µg of plasmid DNA resulted in the Zinc Finger Nuclease mediated integration of the donor DNA construct within the ETIP genomic locus at a recombination frequency of about 0.04% of the ∼50,000 canola protoplast cells. In actuality, the recombination frequency is much higher. Of the ∼50,000 canola protoplast cells which were provided during the transfection experiment, only about 10 - 30% of these cells are actually transfected. As such, the actual Zinc Finger Nuclease mediated integration of the donor DNA construct within the ETIP genomic locus transfection efficiency ranges from about 0.38 - 0.12%. The results of the zinc finger mediated homology directed repair are significantly greater than the negative control experiments, wherein only one protoplast of ∼50,000 was identified to have red fluorescence, thereby resulting in a recombination frequency of 0.00%, as shown in FIG. 20.

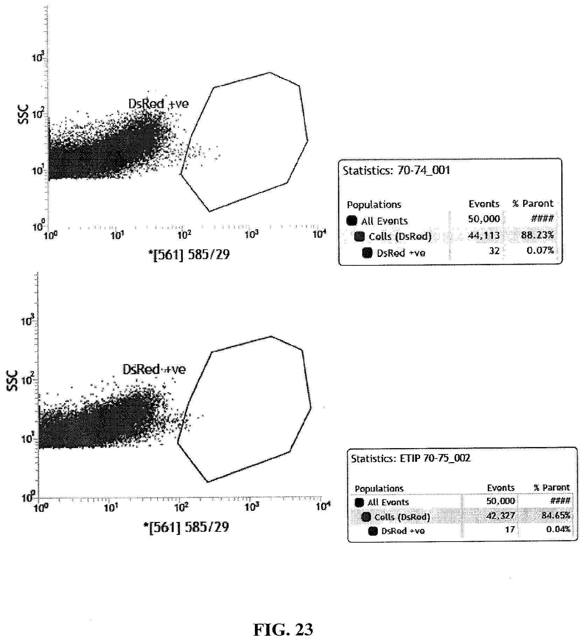

FIG. 23 shows the results of the transfections in which the donor plasmid and ZFN plasmid were co-transformed. The top graph, wherein donor, pDAS000070, and the Zinc Finger Nuclease, pDAS000074, were co-transformed at a ratio of 28 µg to 2 µg of plasmid DNA resulted in the Zinc Finger Nuclease mediated integration of the donor DNA construct within the ETIP genomic locus at a recombination frequency of about 0.07% of the ∼50,000 canola protoplast cells. In actuality, the recombination frequency is much higher. Of the ∼50,000 canola protoplast cells which were provided during the transfection experiment, only about 10 - 30% of these canola protoplast cells are actually transformed. As such, the actual Zinc Finger Nuclease mediated integration of the donor DNA construct within the ETIP genomic locus transfection efficiency ranges from about 0.64 - 0.21%. Similarly the bottom graph, wherein donor, pDAS000070, and the Zinc Finger Nuclease, pDAS000075, were co-transformed at a ratio of 28 µg to 2 µg of plasmid DNA resulted in the Zinc Finger Nuclease mediated integration of the donor DNA construct within the ETIP genomic locus at a recombination frequency of about 0.04% of the ∼50,000 canola protoplast cells. In actuality, the recombination frequency is much higher. Of the ∼50,000 canola protoplast cells which were provided during the transfection experiment, only about 10 - 30% of these cells are actually transfected. As such, the actual Zinc Finger Nuclease mediated integration of the donor DNA construct within the ETIP genomic locus transfection efficiency ranges from about 0.34 - 0.11%. The results of the zinc finger mediated homology directed repair are significantly greater than the negative control experiments, wherein only one protoplast of ∼50,000 was identified to have red fluorescence, thereby resulting in a recombination frequency of 0.00%, as shown in FIG. 20.

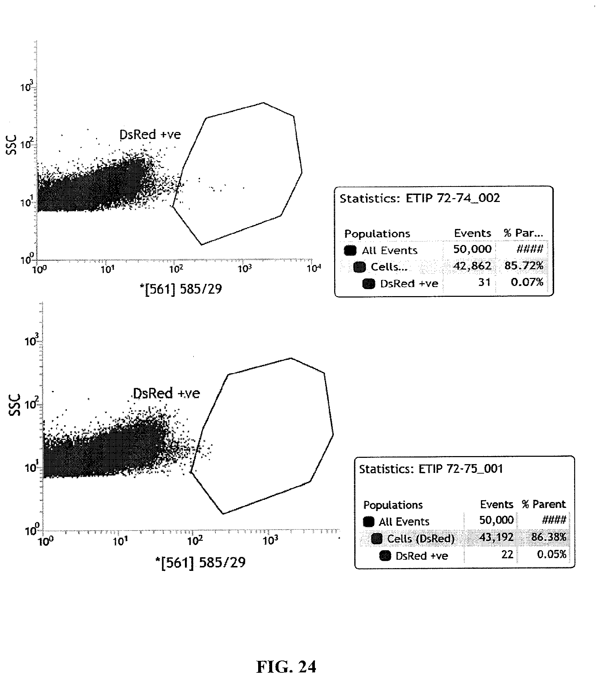

FIG. 24 shows the results of the transfections in which the donor plasmid and ZFN plasmid were co-transformed. The top graph, wherein donor, pDAS000072, and the Zinc Finger Nuclease, pDAS000074, were co-transformed at a ratio of 28 µg to 2 µg of plasmid DNA resulted in the Zinc Finger Nuclease mediated integration of the donor DNA construct within the ETIP genomic locus at a recombination frequency of about 0.07% of the ∼50,000 canola protoplast cells. In actuality, the recombination frequency is much higher. Of the ∼50,000 canola protoplast cells which were provided during the transfection experiment, only about 10 - 30% of these canola protoplast cells are actually transformed. As such, the actual Zinc Finger Nuclease mediated integration of the donor DNA construct within the ETIP genomic locus transfection efficiency ranges from about 0.62 - 0.20%. Similarly the bottom graph, wherein donor, pDAS000072, and the Zinc Finger Nuclease, pDAS000075, were co-transformed at a ratio of 28 µg to 2 µg of plasmid DNA resulted in the Zinc Finger Nuclease mediated integration of the donor DNA construct within the ETIP genomic locus at a recombination frequency of about 0.05% of the ∼50,000 canola protoplast cells. In actuality, the recombination frequency is much higher. Of the ∼50,000 canola protoplast cells which were provided during the transfection experiment, only about 10 - 30% of these cells are actually transfected. As such, the actual Zinc Finger Nuclease mediated integration of the donor DNA construct within the ETIP genomic locus transfection efficiency ranges from about 0.44 - 0.14%. The results of the zinc finger mediated homology directed repair are significantly greater than the negative control experiments, wherein only one protoplast of ∼50,000 was identified to have red fluorescence, thereby resulting in a recombination frequency of 0.00%, as shown in FIG. 20.

Selected explants were transferred and cultured upon regeneration media containing phosphothrinocin. After the culturing period the surviving explants were transferred to elongation medium and root induction medium for culturing and plant development. Whole plants that consisted of developed root and shoot structures were transferred into soil and further propagated in the greenhouse. The tissue culture process utilized media and culture conditions as previously described above. The results of plants produced from the tissue culturing process are shown in Table 23 below.

**Table 23: Results of tissue culturing process.**

| **Construct** | **No. of explants transferred to regeneration media: B2-2 PPT** | **No. of explants surviving in regeneration media: B2-2 PPT** | | **No. of shoots surviving in shoot elongation media: SEM- 2 PPT** | **No. of shoots surviving in RIM - 2 PPT** | **No. of rooted plants transferred to soil** |
|---|---|---|---|---|---|---|
| **pDAS000064+pDAS000074-I** | **4021** | **36** | | **74** | **1** | **--** |
| **pDAS000064+pDAS000074-II** | **1300** | **90** | | **13** | **1** | **1** |
| **pDAS000064+pDAS000074-III** | **1760** | **15** | | **36** | **2** | **--** |
| **pDAS000068+pDAS000074-I** | **1700** | **100** | | **4** | **8** | **2** |
| **pDAS000068+pDAS000074-II** | **1630** | **--** | | **29** | **15** | **--** |
| **pDAS000068+pDAS000074-III** | **2523** | **30** | | **11** | **1** | **--** |
| **pDAS000070+pDAS000074-I** | **2084** | **10** | | **34** | **1** | **--** |
| **pDAS000070+pDAS000074-II** | **4151** | **--** | | **88** | **7** | **--** |
| **pDAS000070+pDAS000074-III** | **1480** | **415** | | **14** | **0** | **--** |
| **pDAS000072+pDAS000074-I** | **1980** | **7** | | **19** | **16** | **--** |
| **pDAS000072+pDAS000074-II** | **1050** | **0** | | **0** | **0** | **--** |
| **pDAS000072+pDAS000074-III** | **1200** | **--** | | **2** | **0** | **--** |
| **pDAS000064+pDAS000074-I** | **556** | **--** | | **8** | **1** | **--** |
| **pDAS000064+pDAS000074-II** | **581** | **13** | | **7** | **--** | **--** |
| **pDAS000064+pDAS000074-III** | **1160** | **90** | | **17** | **1** | **--** |
| **pDAS000068+pDAS000074-I** | **516** | **0** | | **13** | **--** | **--** |
| **pDAS000068+pDAS000074-II** | **1725** | **55** | | **19** | **3** | **--** |
| **pDAS000068+pDAS000074-III** | **930** | **57** | | **0** | **--** | **--** |
| **pDAS000070+pDAS000074-I** | **600** | **8** | | **3** | **--** | **--** |
| **pDAS000070+pDAS000074-II** | **4410** | **1410** | | **360** | **3** | **--** |
| **pDAS000070+pDAS000074-III** | **2350** | **108** | | **51** | **8** | **--** |
| **pDAS000072+pDAS000074-I** | **1660** | **10** | | **19** | **3** | **1** |
| **pDAS000072+pDAS000074-II** | **175** | **--** | | **13** | **--** | **--** |
| **pDAS000072+pDAS000074-III** | **250** | **9** | | **2** | **--** | **--** |
| | | | | | | |

| **CONSTRUCTS** | **No. of protoplasts recovered** | **No. of explants transferred to regeneration media: B2-2 PPT** | **No. of explants surviving in regeneration media: B2-2 PPT** | **No. of shoots recovered on shoot elongation media: SEM- 2 PPT** | **No. of shoots transferred to RIM - 2 PPT** | **No. of rooted plants transferred to soil** |
|---|---|---|---|---|---|---|
| pDAS000064 + pDAS000074 | **3X10⁵** | -- | -- | -- | -- | -- |
| pDAS000068 + pDAS000074 | **1X10⁵** | **114** | **12** | **1** | -- | -- |
| pDAS000070 + pDAS000074 | **3X10⁵** | **478** | **391** | **--** | -- | -- |
| pDAS000072 + pDAS000074 | **3X10⁵** | **81** | **12** | **--** | -- | -- |
| pDAS000064 + pDAS000074 | **3X10⁵** | **38** | **7** | **--** | -- | -- |
| pDAS000068 + pDAS000074 | **3X10⁵** | -- | -- | -- | -- | -- |
| pDAS000070 + pDAS000074 | **1X10⁵** | **80** | **7** | **1** | -- | -- |
| pDAS000072 + pDAS000074 | **3X10⁵** | **7** | **3** | -- | -- | -- |

The FACS sorting method is directly applicable to screen any fluorescent transgene sequence and is used to isolate a proportion of any protoplast, herein *Brassica napus* protoplast cells that are targeted with a fluorescent transgene via homology mediated repair within a specific site in the ETIP region within a genomic locus.

While certain exemplary embodiments have been described herein, those of ordinary skill in the art will recognize and appreciate that many additions, deletions, and modifications to the exemplary embodiments may be made without departing from the scope of the following claims. In addition, features from one embodiment may be combined with features of another embodiment.

## Claims

1. A method for generating a transgenic plant from a population of plant protoplasts, the method comprising isolating a plant protoplast comprising a polynucleotide of interest, the method comprising:
separating the at least one sodium alginate-encapsulated plant protoplast comprising the polynucleotide of interest and a fluorescent marker from the remaining plant protoplasts in a population of plant protoplasts by fluorescence-activated cell sorting (FACS); and
culturing the separated sodium alginate-encapsulated plant protoplast(s) comprising the polynucleotide of interest and the fluorescent marker to regenerate a transgenic plant.

2. The method according to claim 1, wherein the fluorescent marker is a fluorescent polypeptide that is expressed from a polynucleotide in the sodium alginate-encapsulated plant protoplast.

3. The method according to claim 1 or claim 2, wherein the polynucleotide of interest encodes a polypeptide of interest.

4. The method according to claim 3, wherein the polypeptide of interest is a zinc finger nuclease.

5. The method according to any one of claims 1 to 4, wherein the population of plant protoplasts is obtained from a plant tissue.

6. The method according to any one of claims 1 to 5, comprising separating a plurality of sodium alginate-encapsulated protoplasts comprising the polynucleotide of interest and the fluorescent marker.

7. The method according to any one of claims 1 to 6, wherein the sodium alginate-encapsulated plant protoplast is from a monocot.

8. The method according to any one of claims 1 to 6, wherein the sodium alginate-encapsulated plant protoplast is from a dicot.

9. The method according to any one of claim 1 to 8, wherein the polynucleotide of interest and the polynucleotide encoding the fluorescent marker are integrated at a single site in the genome of the sodium alginate-encapsulated plant protoplast.

10. The method according to claim 9, wherein the polynucleotide of interest and the polynucleotide encoding the fluorescent marker are integrated in a site-specific manner by utilizing a zinc-finger nuclease.

11. A method for producing a transgenic plant, the method comprising:
transforming a population of plant protoplasts with a nucleic acid molecule comprising a polynucleotide of interest and a fluorescent marker; and
encapsulating the individual protoplasts of the population by sodium alginate, such that the protoplasts of the population are each encapsulated separately;
separating at least one sodium alginate-encapsulated protoplast comprising the polynucleotide of interest and the fluorescent marker from the remaining plant protoplasts in the population by fluorescence-activated cell sorting (FACS); and
culturing the separated sodium alginate-encapsulated protoplast(s) comprising the polynucleotide of interest and the fluorescent marker to regenerate a transgenic plant.

## Patentansprüche

1. Verfahren zum Erzeugen einer transgenen Pflanze aus einer Population von Pflanzenprotoplasten, wobei das Verfahren das Isolieren eines Pflanzenprotoplasten umfasst, der ein interessierendes Polynukleotid umfasst, wobei das Verfahren Folgendes umfasst:
Abtrennen des mindestens einen Natriumalginat-verkapselten Pflanzenprotoplasten, der das interessierende Polynukleotid und einen Fluoreszenzmarker umfasst, von den verbleibenden Pflanzenprotoplasten in einer Population von Pflanzenprotoplasten durch fluoreszenzaktivierte Zellsortierung (FACS); und
Kultivieren des/der abgetrennten Natriumalginat-verkapselten Pflanzenprotoplasten, der/die das interessierende Polynukleotid und den Fluoreszenzmarker umfasst/umfassen, um eine transgene Pflanze zu regenerieren.

2. Verfahren nach Anspruch 1, wobei der Fluoreszenzmarker ein fluoreszierendes Polypeptid ist, das aus einem Polynukleotid in dem Natriumalginat-verkapselten Pflanzenprotoplast exprimiert wird.

3. Verfahren nach Anspruch 1 oder Anspruch 2, wobei das interessierende Polynukleotid für ein interessierendes Polypeptid kodiert.

4. Verfahren nach Anspruch 3, wobei das interessierende Polypeptid eine Zinkfinger-Nuklease ist.

5. Verfahren nach einem der Ansprüche 1 bis 4, wobei die Population von Pflanzenprotoplasten aus einem Pflanzengewebe erhalten wird.

6. Verfahren nach einem der Ansprüche 1 bis 5, umfassend das Trennen einer Vielzahl von Natriumalginat-verkapselten Protoplasten, die das interessierende Polynukleotid und den Fluoreszenzmarker umfassen.

7. Verfahren nach einem der Ansprüche 1 bis 6, wobei der Natriumalginat-verkapselte Pflanzenprotoplast aus einer Monokotyle stammt.

8. Verfahren nach einem der Ansprüche 1 bis 6, wobei der Natriumalginat-verkapselte Pflanzenprotoplast aus einer Dikotyle stammt.

9. Verfahren nach einem der Ansprüche 1 bis 8, wobei das interessierende Polynukleotid und das für den Fluoreszenzmarker kodierende Polynukleotid auf einem einzelnen Ort im Genom des Natriumalginat-verkapselten Protoplasten integriert sind.

10. Verfahren nach Anspruch 9, wobei das interessierende Polynukleotid und das für den Fluoreszenzmarker kodierende Polynukleotid auf ortsspezifische Weise unter Verwendung einer Zinkfinger-Nuklease integriert werden.

11. Verfahren zum Herstellen einer transgenen Pflanze, wobei das Verfahren Folgendes umfasst:
Transformieren einer Population von Pflanzenprotoplasten mit einem Nukleinsäuremolekül, das ein interessierendes Polynukleotid und einen Fluoreszenzmarker umfasst; und
Verkapseln der einzelnen Protoplasten der Population durch Natriumalginat, sodass die Protoplasten der Population jeweils separat verkapselt sind;
Abtrennen mindestens eines Natriumalginat-verkapselten Protoplasten, der das interessierende Polynukleotid und den Fluoreszenzmarker umfasst, von den verbleibenden Pflanzenprotoplasten in der Population durch fluoreszenzaktivierte Zellsortierung (FACS); und
Kultivieren des/der abgetrennten Natriumalginat-verkapselten Protoplasten, der/die das interessierende Polynukleotid und den Fluoreszenzmarker umfasst/umfassen, um eine transgene Pflanze zu regenerieren.

## Revendications

1. Procédé pour générer une plante transgénique à partir d'une population de protoplastes de plante, le procédé comprenant isoler un protoplaste de plante comprenant un polynucléotide d'intérêt, le procédé comprenant :
séparer le au moins un protoplaste de plante encapsulé dans de l'alginate de sodium comprenant le polynucléotide d'intérêt et un marqueur fluorescent provenant des protoplastes de plante restants dans une population de protoplastes de plante par cytométrie en flux (FACS) ; et
cultiver le ou les protoplastes de plante encapsulés dans de l'alginate de sodium séparés comprenant le polynucléotide d'intérêt et le marqueur fluorescent pour régénérer une plante transgénique.

2. Procédé selon la revendication 1, dans lequel le marqueur fluorescent est un polypeptide fluorescent qui est exprimé à partir d'un polynucléotide dans le protoplaste de plante encapsulé dans de l'alginate de sodium.

3. Procédé selon l'une des revendications 1 ou 2, dans lequel le polynucléotide d'intérêt code pour un polypeptide d'intérêt.

4. Procédé selon la revendication 3, dans lequel le polypeptide d'intérêt est une nucléase à doigt de zinc.

5. Procédé selon l'une quelconque des revendications 1 à 4, dans lequel la population de protoplastes de plante est obtenue à partir d'un tissu végétal.

6. Procédé selon l'une quelconque des revendications 1 à 5, comprenant séparer une pluralité de protoplastes encapsulés dans de l'alginate de sodium comprenant le polynucléotide d'intérêt et le marqueur fluorescent.

7. Procédé selon l'une quelconque des revendications 1 à 6, dans lequel le protoplaste de plante encapsulé dans de l'alginate de sodium provient d'une monocotylédone.

8. Procédé selon l'une quelconque des revendications 1 à 6, dans lequel le protoplaste de plante encapsulé dans de l'alginate de sodium provient d'une dicotylédone.

9. Procédé selon l'une quelconque des revendications 1 à 8, dans lequel le polynucléotide d'intérêt et le polynucléotide codant pour le marqueur fluorescent sont intégrés dans un seul site du génome du protoplaste de plante encapsulé dans de l'alginate de sodium.

10. Procédé selon la revendication 9, dans lequel le polynucléotide d'intérêt et le polynucléotide codant pour le marqueur fluorescent sont intégrés d'une manière spécifique à un site à l'aide d'une nucléase à doigt de zinc.

11. Procédé de production d'une plante transgénique, le procédé comprenant :
transformer une population de protoplastes de plante avec une molécule d'acide nucléique comprenant un polynucléotide d'intérêt et un marqueur fluorescent ; et
encapsuler les protoplastes individuels de la population par de l'alginate de sodium, de telle manière que les protoplastes de la population sont chacun encapsulés séparément ;
séparer au moins un protoplaste encapsulé dans de l'alginate de sodium comprenant le polynucléotide d'intérêt et le marqueur fluorescent des protoplastes de plante restants dans la population par cytométrie en flux (FACS) ; et
cultiver le ou les protoplastes encapsulés dans de l'alginate de sodium séparés comprenant le polynucléotide d'intérêt et le marqueur fluorescent pour régénérer une plante transgénique.
